(19) Europäisches Patentamt
European Patent Office
Office européen des brevets

(11) EP 3 515 912 B1

(12) # FASCICULE DE BREVET EUROPEEN

(45) Date de publication et mention de la délivrance du brevet:
**14.10.2020 Bulletin 2020/42**

(21) Numéro de dépôt: **17783941.2**

(22) Date de dépôt: **26.09.2017**

(51) Int Cl.:
***C07D 471/04*** *(2006.01)* ***A61K 49/00*** *(2006.01)*
***A61K 51/00*** *(2006.01)*

(86) Numéro de dépôt international:
**PCT/FR2017/052592**

(87) Numéro de publication internationale:
**WO 2018/055316 (29.03.2018 Gazette 2018/13)**

(54) **COMPOSES POUR LEUR UTILISATION EN IMAGERIE ET NOTAMMENT POUR LE DIAGNOSTIC DE MALADIES NEURO-DEGENERATIVES**

VERBINDUNGEN ZUR VERWENDUNG IN DER BILDGEBUNG, INSBESONDERE ZUR DIAGNOSE VON NEURODEGENERATIVEN ERKRANKUNGEN

COMPOUNDS FOR USING IN IMAGING AND PARTICULARLY FOR THE DIAGNOSIS OF NEURODEGENERATIVE DISEASES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.09.2016 FR 1659033**
**10.03.2017 FR 1752001**

(43) Date de publication de la demande:
**31.07.2019 Bulletin 2019/31**

(73) Titulaires:
- **Centre National de la Recherche Scientifique 75794 Paris Cedex 16 (FR)**
- **Université d'Orléans 45100 Orleans Cedex 2 (FR)**
- **Université de Tours 37020 Tours Cedex 1 (FR)**
- **Centre Hospitalier Régional Universitaire de Tours 37044 Tours Cedex 9 (FR)**
- **INSERM (Institut National de la Santé et de la Recherche Médicale) 75654 Paris Cedex 13 (FR)**

(72) Inventeurs:
- **ROUTIER, Sylvain 54510 Tigy (FR)**
- **SUZENET, Franck 45650 Saint Jean Le Blanc (FR)**
- **CHALON, Sylvie 37540 Saint-Cyr-sur-Loire (FR)**
- **BURON, Frédéric 45100 Orléans (FR)**
- **VERCOUILLIE, Johnny 37400 Amboise (FR)**
- **MELKI, Ronald 78000 Versailles (FR)**
- **BOIARYNA, Liliana 44400 Rezé (FR)**
- **GUILLOTEAU, Denis 37540 Saint-Cyr-sur-Loire (FR)**
- **PIERI, Laura 91400 Orsay (FR)**

(74) Mandataire: **Monni, Richard**
**Cabinet LLR**
**11 boulevard de Sébastopol**
**75001 Paris (FR)**

(56) Documents cités:
**WO-A1-2011/119565 WO-A2-2010/111303**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 515 912 B1

## Description

L'invention concerne des composés pour leur utilisation en imagerie et notamment pour le diagnostic de maladies neuro-dégénératives.

L'agrégation de protéines anormales est une caractéristique commune à plusieurs affections neurodégénératives. La propagation de ces agrégats à partir d'une zone cérébrale initiale vers d'autres zones connectées anatomiquement apparaît comme un processus majeur de mort neuronale progressive. Parmi ces agrégats ou dépôts se trouvent, en particulier, les agrégats de protéines anormales de tau, béta-amyloïde, et d'alpha-synucléines.

Les pathologies concernées par ce type de dépôt sont directement reliées à la protéine concernée. Ainsi, les maladies neuro-dégénératives associées aux agrégats des protéines anormales de tau, beta-amyloïde et alpha-synucléine sont nommées respectivement taupathies, amyloidopathies et synucléinopathies. Plus particulièrement, ces maladies neuro-dégénératives comprennent les maladies d'Alzheimer et de Parkinson.

Les dépôts de protéine alpha-synucléine ($\alpha$-syn) sont, en particulier, des marqueurs majeurs des synucléinopathies et tout particulièrement de la maladie de Parkinson (MP). L'$\alpha$-syn est une protéine de 14 kDa localisée dans les neurones, au niveau du cytoplasme et des noyaux mais surtout des boutons synaptiques. Dans des conditions physiologiques, les rôles précis de cette protéine sont encore peu clairs (elle semble interagir avec les vésicules présynaptiques). Dans certaines conditions pathologiques, l'$\alpha$-syn adopte des conformations oligomériques et/ou fibrillaires. Des agrégats (ou corps de Lewy) peuvent alors se former et induire des altérations de la libération de neurotransmetteurs (en particulier la dopamine), et affecter la fonction mitochondriale, le transport vésiculaire, et certains processus de dégradation protéique, l'ensemble de ces processus pouvant aboutir à la mort neuronale.

Actuellement, le diagnostic de la MP repose essentiellement sur des critères cliniques qui commencent à être appuyés par des explorations en imagerie moléculaire en tomographie d'émission monophotonique (TEMP) ou en tomographie par émission de positrons (TEP) du transporteur de la dopamine dont la diminution est un index de cette maladie.

Cependant, les agrégats d'a-syn apparaissant dans les premiers stades de la MP, leur détection constitue un élément déterminant de diagnostic précoce. L'une des méthodes de choix dans ce cadre est l'imagerie moléculaire TEP ou TEMP, qui permet d'explorer in vivo des cibles telles que des récepteurs, transporteurs, ou des amas protéiques. Cette méthode requiert l'utilisation de traceurs, ou radiopharmaceutiques, émetteurs de rayonnements $\beta$+ ou $\gamma$ et spécifiques de la cible à explorer. A ce jour, aucun traceur TEP ou TEMP spécifique de l'a-syn n'est disponible.

C'est pourquoi l'un des buts de l'Invention est l'utilisation du composé de l'Invention comme agent d'imagerie in vivo. En particulier, un autre but de l'invention est de fournir un agent d'imagerie TEP ou TEMP permettant de détecter et quantifier les agrégats d'alpha-synucléine, de $\beta$-amyloïde ou de tau anormale in vivo.

Un autre but de l'Invention est de développer un traceur radiomarqué, par exemple au fluor-18 ($^{18}$F), pour une utilisation en clinique sur une large population, afin d'améliorer le diagnostic précoce, le suivi et le développement de traitements de synucléinopathies telles que la MP.

Un autre but de l'Invention est de fournir un composé ayant une affinité pour les fibres d'alpha-synucléine, de peptide $\beta$-amyloïde et/ou de Tau.

Un autre but de l'Invention est de fournir un composé pour son utilisation dans un procédé de diagnostic ou d'imagerie in vivo.

Un autre but de l'Invention est de fournir une composition pharmaceutique comprenant le composé de l'Invention.

Un autre but de l'Invention est de fournir un composé pour son utilisation comme médicament pharmaceutique ou radiopharmaceutique.

La présente Invention concerne ainsi un composé de formule I

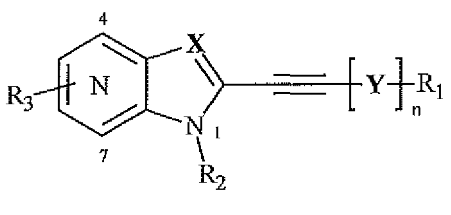

(I)

- **X étant choisi parmi** N ou C-$R_4$
- **Y étant choisi parmi** un alcène ou un alcyne, un aryle ou un hétéroaryle, un alcane comprenant de 1 à 7 atomes de carbone, n étant un entier égal à 0 ou 1 ;
- **$R_1$ étant choisi parmi** un aryle ou hétéroaryle choisis parmi les groupes phényle, pyrimidine, pyridine, thiophène, furane, triazole, oxazole, (aza)indole, (aza)indoline, (aza)benzimidazole éventuellement substitués sur une ou plusieurs positions par un groupe choisi parmi :

**Halogène, $NO_2$, CN, diméthyltriazène, triméthylammonium, aryliodonium,**

**$NR^aR^b$, $SO_2NR^aR^b$, $CONR^aR^b$, $NR^cSO_2NR^aR^b$, $NR^cCONR^aR^b$, $NR^aCOR^b$;**

$R^a$, $R^b$ et $R^c$ représentent indépendamment les uns des autres un H, un ($C_1$-$C_7$)alkyle , un aryle, un hétéroaryle, un ($C_3$-$C_7$)carbocyclyle, un ($C_1$-$C_7$)alkyle-aryle, ($C_1$-$C_7$)alkyle-hétéroaryle, ou $R^a$ et $R^b$ forment ensemble un ($C_3$-$C_7$)hétérocyclyle ;

**$Sn(Alkyle)_3$, Alkyle étant choisi parmi méthyle ou *n*-butyle ;**

**$B(OH)_2$, B(pinacol) ;**

**$R^d$, $CH_2R^d$** ;

$R^d$ représente un H, un ($C_1$-$C_7$)alkyle, un aryle, un hétéroaryle, un ($C_3$-$C_7$)hétérocyclyle, un ($C_3$-$C_7$)carbocyclyle, un ($C_1$-$C_7$)alkyle-aryle, un ($C_1$-$C_7$)alkyle-hétéroaryle, un ($C_1$-$C_7$)alkyle-($C_3$-$C_7$)hétérocyclyle, un $[(C_1\text{-}C_7)\text{Alkyle}]_n$-Z ou un $[(C_1\text{-}C_7)\text{Alkyle-Z}]_n$ Z étant un hétéroatome choisi parmi N, O ou S, en particulier choisi parmi $NR^aR^b$ ou $OR^e$, et n étant un entier compris de 1 à 7 ;

**$OR^e$, OAc, OTs, OTf, $SR^e$, $SO_2R^e$, $COR^e$, $NR^aSO_2R^c$, $NHCOOR^e$;**

$R^e$ représente un H, un ($C_1$-$C_7$)alkyle, un aryle, un hétéroaryle, un ($C_3$-$C_7$)hétérocyclyle, un ($C_3$-$C_7$)carbocyclyle, un ($C_1$-$C_7$)alkyle-aryle, un ($C_1$-$C_7$)alkyle-hétéroaryle ou un ($C_1$-$C_7$)alkyle-($C_3$-$C_7$)hétérocyclyle ;

- **$R_2$ étant choisi parmi :**

H, $SO_2Ph$, $COR^e$, $NR^cCONR^aR^b$, $COOR^e$, OH, $R^d$ ;

- **$R_3$ étant choisi parmi :**

Halogène, $NO_2$, CN, diméthyltriazène, triméthylammonium, aryliodonium,

$NR^aR^b$, $SO_2NR^aR^b$, $CONR^aR^b$, $NR^cSO_2NR^aR^b$, $NR^cCONR^aR^b$ , $NR^aCOR^b$;

$Sn(Alkyle)_3$, Alkyle étant choisi parmi méthyle ou *n*-butyle ;

$B(OH)_2$, B(pinacol) ;

$R^d$, $CH_2R^d$,

$OR^e$, OAc, OTs, OTf, O(hétéroaryle) notamment O(HOBt), $SR^e$, $SO_2R^e$, $COR^e$, $NR^aSO_2R^e$, $NHCOOR^e$;

- **$R_4$ étant choisi parmi :**

Halogène, $CH_2NR^aR^b$, $R^a$, $COOR^e$, CHO, $CH_2OR^e$ ;

dans lequel au moins l'un des substituants $R_1$, $R_2$, $R_3$ ou $R_4$ comprend éventuellement un radioélément choisi parmi $^{18}F$. $^{11}C$, $^{123}I$ et $^{125}I$.

Lorsqu'au moins l'un des substituants $R_1$ et $R_3$ est le triméthylammonium, le contre ion est choisi parmi $I^-$, $Cl^-$, $OTs^-$ et $OMs^-$.

Au sens de la présente invention, le terme « *aryliodonium* » est défini comme le cation d'un atome d'iode portant un groupement aryle. Des exemples, non limitatif, d'aryliodonium incluent le [triméthyl-2,4,6-phenyle]$I^+$, le [4-tertbutylphenyle]$I^+$ et le [2,6-diméthyl-4-tertbutylphenyle]$I^+$ dont le contre ion est le *para*-toluènesulfonate.

Au sens de l'Invention, l'expression « *($C_1$-$C_7$)alkyle* » désigne une chaine carbonée acyclique, saturée, linéaire ou branchée, comprenant 1 à 7 atomes de carbone. Des exemples de groupe ($C_1$-$C_7$)alkyle incluent les groupes méthyle, éthyle, propyle, butyle, pentyle, hexyle ou heptyle. La définition de propyle, butyle, pentyle, hexyle ou heptyle inclut tous les isomères possibles. Par exemple, le terme butyle comprend *n*-butyle, *iso*-butyle, *sec*-butyle et ter-butyle. Le ($C_1$-$C_7$)alkyle peut être substitué par un ou plusieurs groupes tels que halogène, hydroxyle, alkoxyle, amino, amido, cyano, aryle, trifluorométhyle, pentafluorosulfure, acide carboxylique ou ester carboxylique. De préférence, le groupe

$(C_1-C_7)$alkyle peut être substitué par un halogène, un hydroxyle ou un alkoxyle, ou plus particulièrement par un fluoro, un chloro, un hydroxyle ou un alkoxyle tel que les groupes OMs ou OTs, représentant respectivement les groupes méthanesulfonate et *para*-toluènesulfonate. En particulier le groupe $(C_1-C_7)$alkyle peut être de la forme $(CH_2)_n-CH_2F$, n étant compris de 0 à 6.

Ainsi, au sens de la présente invention, un groupe

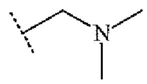

est donc compris dans la définition d'un groupe alkyle substitué par une amine, l'amine étant dans ce cas une (*N,N*-diméthyle)-amine.

Au sens de l'Invention, l'expression « *$(C_3-C_7)$carbocyclyle* » désigne un mono-, bi- ou tri-cycle saturé ou partiellement saturé, comprenant 3 à 7 atomes de carbone. Des exemples de $(C_3-C_7)$carbocyclyle incluent les groupes cyclopropyle, cyclobutyle, cyclopentyle, cyclohexyle ou cycloheptyle. Le cycloalkyle peut être substitué par un ou plusieurs groupes tels que fluoro, chloro, bromo, iodo, amino ou alkoxyle. Le « *$(C_3-C_7)$carbocyclyle* » peut également former un spirocycle avec un autre « *$(C_3-C_7)$carbocyclyle* » ou un « *$(C_3-C_7)$hétérocyclyle ».*

Au sens de l'Invention, l'expression « *$(C_3-C_7)$hétérocyclyle* » désigne un hétérocycle saturé comprenant 3, 4, 5, 6, ou 7 atomes dans le cycle dont 1, 2 ou 3 hétéroatomes, choisis parmi O, S ou N, remplacent respectivement 1, 2 ou 3 atomes de carbone. Des exemples de « *$(C_3-C_1)$hétérocyclyle »* incluent pyrrolidinyle, pipéridinyle, pipérazinyle et morpholinyle. L'hétérocyclyle peut être substitué par un ou plusieurs groupes tels que amine, alkoxyle, méthyle, cyclohexyle, cyclopentyle, aryle, halogène, ou $(C_1-C_3)$alkyle-halogène. Le « *$(C_3-C_7)$carbocyclyle* » peut également former un spirocycle avec un « *$(C_3-C_7)$carbocyclyle* » ou un autre « *$(C_3-C_7)$hétérocyclyle ».*

Au sens de l'Invention on entend par l'expression « *$(C_1-C_3)$alkyle-halogène* », une chaîne carbonée acyclique, saturée, linéaire ou branchée, comprenant 1 à 3 atomes de carbone, substituée sur une ou plusieurs positions par un groupement halogène tel que fluoro, chloro, bromo ou iodo.

De manière générale, au sens de la présente Invention, lorsqu'un groupe comprend deux ou plus sous-unités, leur attachement est noté « - ». Ainsi, on entend par les expressions « *$(C_1-C_7)$alkyle-aryle », « $(C_1-C_7)$alkyle-hétéroaryle* » ou « *$(C_1-C_7)$alkyle-$(C_3-C_7)$hétérocyclyle* », une chaine $(C_1-C_7)$alkyle telle que définie dans la présente Invention liée respectivement à un groupe aryle, hétéroaryle ou $(C_3-C_7)$hétérocyclyle tels que définis dans la présente Invention.

Au sens de l'Invention, l'expression « *$R^a$ et $R^b$ forment ensemble un $(C_3-C_7)$hétérocyclyle »* signifie que l'azote, $R^a$ et $R^b$ représentent ensemble un hétérocycle. Par exemple $R^a$ et $R^b$ peuvent être connectés entre eux *via* une chaine en $C_4$-alkyle, formant un cycle pyrrolidine avec l'atome d'azote par lequel ils sont reliés.

Le terme « *aryle* » désigne un mono-cycle aromatique comprenant de 5 à 6 atomes de carbone, pouvant être lui-même fusionné avec un second cycle saturé, insaturé ou aromatique. Le terme aryle inclut, sans restriction, le phényle. Le groupe aryle peut être substitué par un ou plusieurs groupes indépendamment choisis parmi les groupes alkyle, halogène, $(C_1-C_3)$alkyle-halogène, hydroxyle, alkoxy, amino, amido, nitro, cyano, trifluorométhyle, pentafluorosulfure, acide carboxylique ou ester carboxylique. Des exemples d'aryles substitués incluent, sans restrictions, le 2-, 3- ou 4-(*N,N*-diméthylamino)-phényle, 2-, 3- ou 4-cyanophényle, le 2-, 3- ou 4-nitrophényle, le 2-, 3- ou 4-fluoro-, chloro-, bromo- ou iodo-phényle, le 2-, 3- ou 4-méthoxyphényle.

Au sens de l'Invention, on entend par l'expression « *$(C_1-C_3)$alkyle-halogène* », une chaine carbonée acyclique, saturée, linéaire ou branchée, comprenant 1 à 3 atomes de carbone, substituée sur une ou plusieurs positions par un groupement halogène tel que fluoro, chloro, bromo ou iodo.

Au sens de la présente Invention, le terme « *hétéroaryle* » désigne un aryle mono- ou polycyclique tel que décrit ci-dessus, dans lequel un ou plusieurs atomes de carbone ont été remplacés par un ou plusieurs hétéroatomes choisis parmi N, O ou S. Le terme hétéroaryle inclut tous les isomères possibles. Des exemples d'hétéroaryles incluent les groupes furanyle, thiényle, pyrrolyle, *N*-alkyle pyrrolyle, indolyle, aza-indolyle, benzofuranyle, benzothiophényle, pyridyle, oxazolyle, thiazolyle, imidazolyle, pyrimidyle, pyrazinyle, triazolyle, *N*-alkyle triazolyle, thiadiazolyle, oxadiazolyle, tétrazolyle et N-alkyle tétrazolyle. Le groupe hétéroaryle peut être de plus substitué par un ou plusieurs groupes indépendamment choisis parmi les groupes alkyle, $(C_1-C_3)$alkyle-halogène, alkoxy, halogène, hydroxyle, amino, amido, nitro, cyano, trifluorométhyle, pentafluorosulfure, acide carboxylique ou ester carboxylique.

Au sens de la présente invention, le terme « *halogène* » représente un fluor, un chlore, un brome ou un iode.

Au sens de la présente invention, les termes « *alkoxyle* » et « *alkoxy* » désignent un hydroxyle ou une chaine alkyle dont l'un au moins des atomes de carbone est remplacé par un atome d'oxygène. La chaine alkyle peut, en outre, être substituée. En particulier, elle peut être substituée par un groupe aryle ou un halogène. Ainsi, des exemples d'alkoxyle au sens de l'invention comprennent OH, OMe, $O-(CH_2)_n-F$, $O-(CH_2)_n-O-(CH_2)_m-F$, n et m étant indépendamment l'un de l'autre compris de 0 à 6, $O-(CH_2)_n$-aryle, en particulier

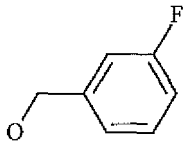

,

$O-(CH_2)_2-F$, $O-(CH_2)_2-O-(CH_2)_2-F$.

L'expression *« au moins l'un des substituants $R_1$, $R_2$, $R_3$ ou $R_4$ comprend éventuellement un radioélément choisi parmi $^{18}F$, $^{11}C$, $^{123}I$ et $^{124}I$»* signifie, au sens de l'invention, que l'un des atomes d'au moins l'un des substituants $R_1$, $R_2$, $R_3$ ou $R_4$ est éventuellement substitué par un radioélément.

Les molécules de la présente invention se lient à ces dépôts avec des affinités et des sélectivités diverses.

La présente Invention repose sur l'observation faite par les Inventeurs, de l'affinité des composés de formule I pour les fibres d'alpha-synucléine, β-amyloïde et/ou tau.

La présente Invention concerne également un composé de formule Ibis

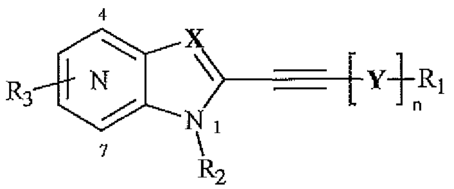

(Ibis)

- **X étant choisi parmi** N ou C-$R_4$
- **Y étant choisi parmi** un alcane, un alcène ou un alcyne comprenant de 1 à 7 atomes de carbone, ou un aryle ou un hétéroaryle, n étant un entier égal à 0 ou 1 ;
- **$R_1$ étant choisi parmi** un aryle ou hétéroaryle choisis parmi les groupes phényle, pyrimidine, pyridine, thiophène, furane, triazole, oxazole, (aza)indole, (aza)indoline, (aza)benzimidazole éventuellement substitués sur une ou plusieurs positions par un groupe choisi parmi :

**Halogène, $NO_2$, CN, diméthyltriazène,**

**$NR^aR^b$, $SO_2NR^aR^b$, $CONR^aR^b$, $NR^cSO_2NR^aR^b$, $NR^cCONR^aR^b$, $NR^aCOR^b$;**

$R^a$, $R^b$ et $R^c$ représentent indépendamment les uns des autres un H, un ($C_1$-$C_7$)alkyle , un aryle, un hétéroaryle, un ($C_3$-$C_7$)carbocyclyle, un ($C_1$-$C_7$)alkyle-aryle, ($C_1$-$C_7$)alkyle-hétéroaryle, ou $R^a$ et $R^b$ forment ensemble un ($C_3$-$C_7$)hétérocyclyle ;

**$R^d$, $CH_2R^d$ ;**

$R^d$ représente un H, un ($C_1$-$C_7$)alkyle, un aryle, un hétéroaryle, un ($C_3$-$C_7$)hétérocyclyle, un ($C_3$-$C_7$)carbocyclyle, un ($C_1$-$C_7$)alkyle-aryle, un ($C_1$-$C_7$)alkyle-hétéroaryle, un ($C_1$-$C_7$)alkyle-($C_3$-$C_7$)hétérocyclyle, un [($C_1$-$C_7$)Alkyle]$_n$-Z ou un [($C_1$-$C_7$)Alkyle-Z]$_n$ Z étant un hétéroatome choisi parmi N, 0 ou S, en particulier choisi parmi $NR^aR^b$ ou $OR^e$ et n étant un entier compris de 1 à 7 ;

**$OR^e$, $SR^e$, $SO_2R^e$, $COR^e$, $NR^aSO_2R^e$, $NHCOOR^e$;**

$R^e$ représente un H, un ($C_1$-$C_7$)alkyle, un aryle, un hétéroaryle, un ($C_3$-$C_7$)hétérocyclyle, un ($C_3$-$C_7$)carbocyclyle, un ($C_1$-$C_7$)alkyle-aryle, un ($C_1$-$C_7$)alkyle-hétéroaryle ou un ($C_1$-$C_7$)alkyle-($C_3$-$C_7$)hétérocyclyle ;

- **$R_2$ étant choisi parmi** :
H, $SO_2Ph$, $COR^e$, $NR^cCONR^aR^b$, $COOR^e$, OH, $R^d$ ;
- **$R_3$ étant choisi parmi :**

Halogène, $NO_2$, CN, diméthyltriazène,

$NR^aR^b$ $SO_2NR^aR^b$, $CONR^aR^b$, $NR^cSO_2NR^aR^b$, $NR^cCONR^aR^b$ , $NR^aCOR^b$;

$R^d$, $CH_2R^d$,

$OR^e$, $SR^e$, $SO_2R^e$, $COR^e$, $NR^aSO_2R^e$, $NHCOOR^e$;

- **$R_4$ étant choisi parmi :**
  Halogène, $CH_2NR^aR^b$, $R^a$, $COOR^e$, CHO, $CH_2OR^e$ ;

dans lequel au moins l'un des substituants $R_1$, $R_2$, $R_3$ ou $R_4$ comprend éventuellement un radioélément choisi parmi $^{18}F$, $^{11}C$, $^{123}I$ et $^{124}I$.

Les substituants triméthylammonium, aryliodonium, $Sn(Alkyle)_3$, $B(OH)_2$, B(pinacol), OAc, OMs, OTs, OTf et O(HOBt) correspondent à des intermédiaires de synthèse pour la formation des composés de formule Ibis.

La présente Invention repose sur l'observation faite par les Inventeurs, de l'affinité des composés de formule Ibis pour les fibres d'alpha-synucléine, β-amyloïde et/ou tau.

Selon un mode de réalisation, le composé de l'Invention, dans lequel au moins l'un des substituants $R_1$, $R_2$, $R_3$ ou $R_4$ comprend un radioélément choisi parmi $^{18}F$, $^{11}C$, $^{123}I$ et $^{124}I$, est de formule II.

Les substituants X, Y, $R_1$, $R_2$, $R_3$ et $R_4$ des composés de formule II ont la même définition que dans la formule I, à condition qu'au moins l'un des substituants $R_1$, $R_2$, $R_3$ ou $R_4$ comprenne un radioélément choisi parmi $^{18}F$, $^{11}C$, $^{123}I$ et $^{124}I$.

De préférence, le radioélément est le $^{18}F$ car sa demi-vie (110 minutes) est suffisament longue pour permettre une utilisation du composé radiomarqué, à la fois en préclinique et en clinique, dans un système d'imagerie TEP dédié, et situé à distance du cyclotron nécessaire à la production du radionucléide et donc du composé radiomarqué.

Selon un mode de réalisation, le composé de l'Invention, dans lequel au moins l'un des substituants $R_1$, $R_2$, $R_3$ ou $R_4$ comprend un radioélément choisi parmi $^{18}F$, $^{11}C$, $^{123}I$ et $^{124}I$, est de formule IIbis.

Selon un autre mode de réalisation, le composé de l'Invention, dans lequel aucun des substituants $R_1$, $R_2$, $R_3$ et $R_4$ ne comprend de radioélément, est de formule III.

Selon un mode de réalisation avantageux, le composé de l'Invention est de formule générale la ou IIa :

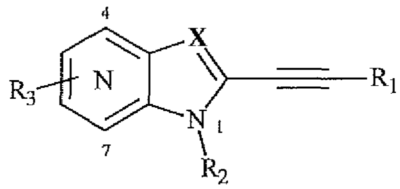

(Ia) ou (IIa)

X, $R_1$, $R_2$, $R_3$ étant tels que définis précédemment dans la formule I ou II respectivement. Selon un mode de réalisation avantageux, le composé de l'Invention est de formule générale Ib ou IIb :

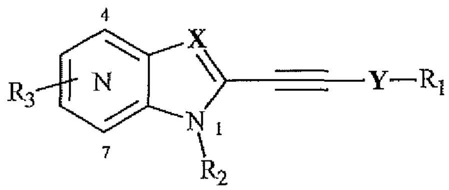

(Ib) ou (IIb)

Y étant choisi parmi un phényle, un thiényle ou un furanyle.
X, $R_1$, $R_2$, $R_3$ étant tels que définis précédemment dans la formule I ou II.

Selon un mode de réalisation avantageux, le composé de l'Invention est de formule générale I-1 :

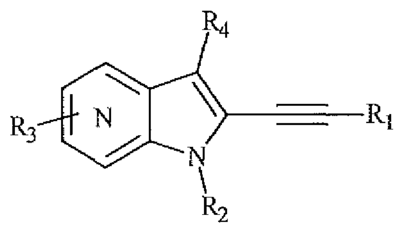

(I-1)

$R_1$, $R_2$, $R_3$ et $R_4$ étant tels que définis précédemment dans la formule I.

Selon un mode de réalisation avantageux, le composé de l'Invention est de formule générale II-1 :

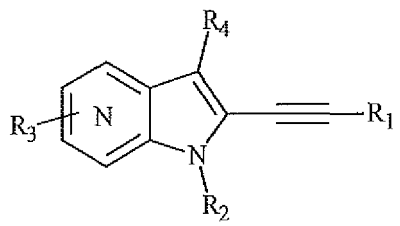

(II-1)

$R_1$, $R_2$, $R_3$ et $R_4$ étant tels que définis précédemment dans la formule I ou II ;
dans lequel au moins l'un des substituants $R_1$, $R_2$, $R_3$ ou $R_4$ comprend un radioélément choisi parmi $^{18}F$, $^{11}C$, $^{123}I$ et $^{124}I$.

Selon un mode de réalisation avantageux, le composé de l'Invention est de formule générale III-1 :

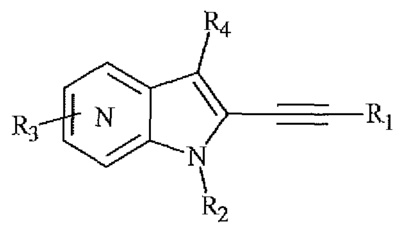

(III-1)

$R_1$, $R_2$, $R_3$ et $R_4$ étant tels que définis précédemment dans la formule I ;
dans lequel aucun des substituants $R_1$, $R_2$, $R_3$ et $R_4$ ne comprend de radioélément.

Selon un mode de réalisation avantageux, le composé de l'Invention est de formule générale I-1a ou II-1a:

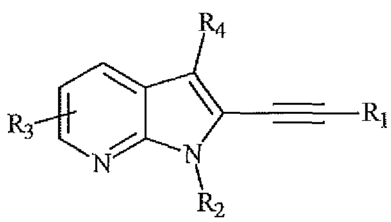

(I-1a) ou (II-1a)

$R_1$, $R_2$, $R_3$ et $R_4$ étant tels que définis précédemment dans la formule I ou II respectivement.

Selon un mode de réalisation avantageux, le composé de l'Invention est de formule générale I-1b ou II-1b:

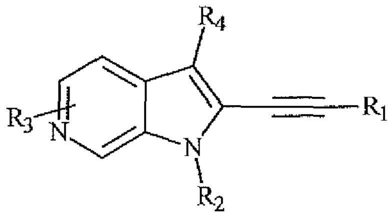

(I-1b) ou (II-1b)

$R_1$, $R_2$, $R_3$ et $R_4$ étant tels que définis précédemment dans la formule I ou II respectivement.

Selon un mode de réalisation avantageux, le composé de l'Invention est de formule générale I-1c ou II-Ic :

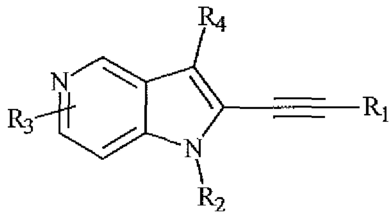

(I-1c) ou (II-1c)

$R_1$, $R_2$, $R_3$ et $R_4$ étant tels que définis précédemment dans la formule I ou II respectivement.

Selon un mode de réalisation avantageux, le composé de l'Invention est de formule générale I-1d ou II-1d :

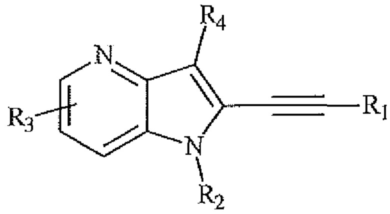

(I-1d) ou (II-1d)

$R_1$, $R_2$, $R_3$ et $R_4$ étant tels que définis précédemment dans la formule I ou II respectivement.

Selon un mode de réalisation avantageux, le composé de l'Invention est de formule générale I-1 ou II-1 :

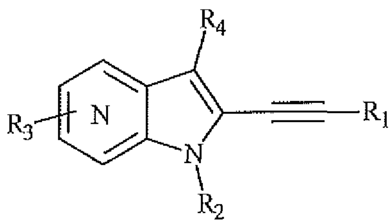

(I-1) ou (II-1)

$R_2$, $R_3$ et $R_4$ étant tels que définis précédemment dans la formule I ou II respectivement;

$R_1$ étant éventuellement marqué et choisi parmi

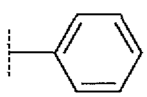 ; 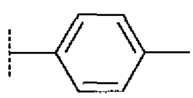 ; 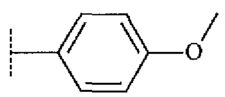 ; 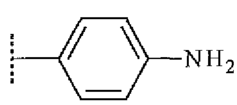

ou

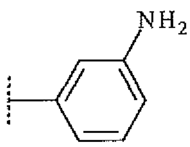 ; 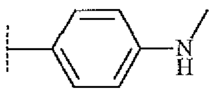 ; 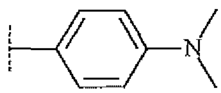 ou 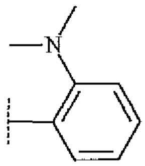

ou

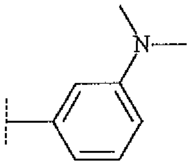 ; 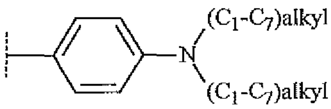 ou 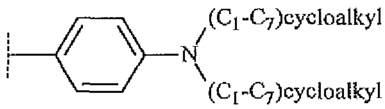 ,

notamment

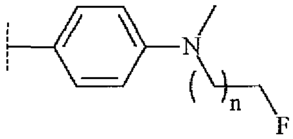 ,

n étant compris de 0 à 6 ;

- 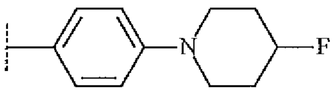 ; 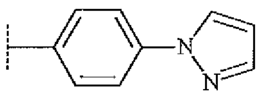 ; 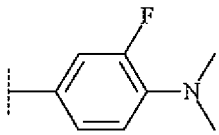

ou

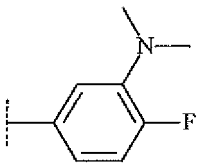 ; 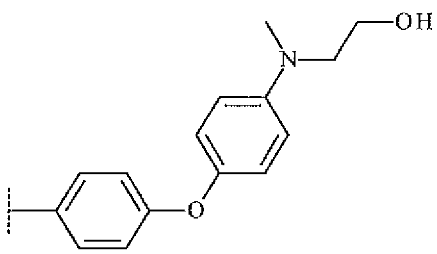 ;

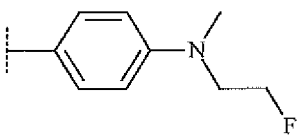 ; 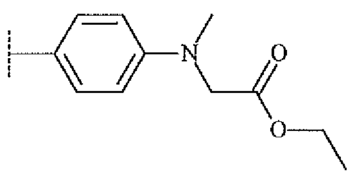 ;

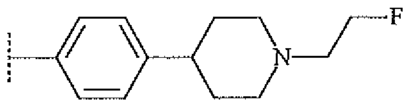 ; 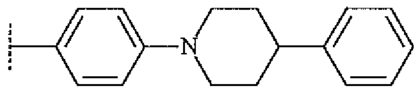 ;

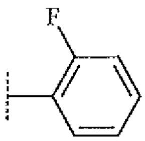 ; 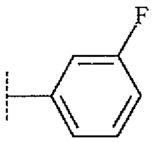 ; 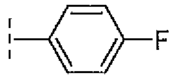 ;

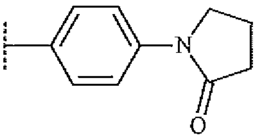 ; 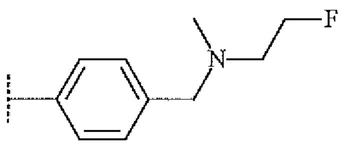 ; 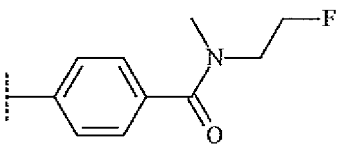 ;

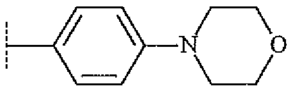 ; 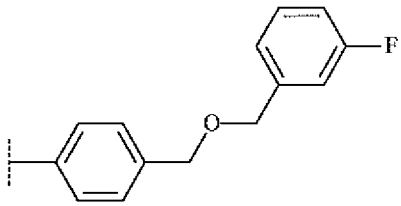 ;

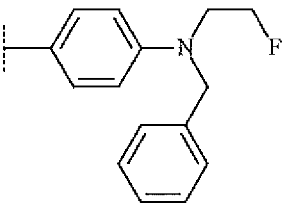 ; 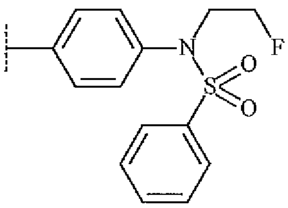 ; 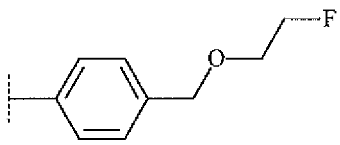 ;

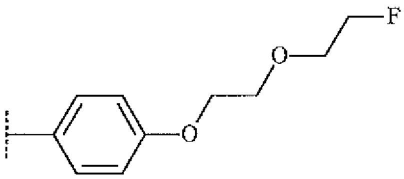 ; 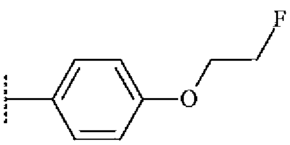 ;

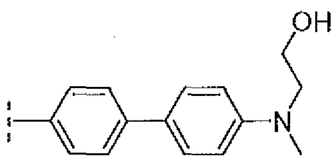 ; 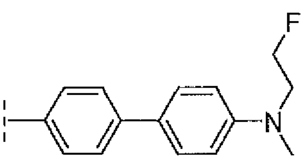 ; 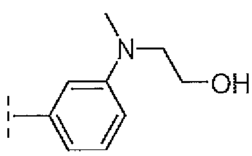 ;

- 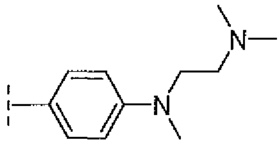 ; 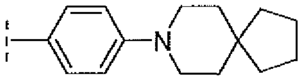 ; 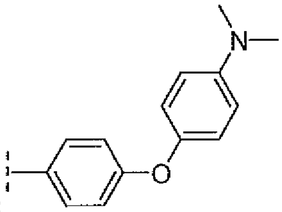 ;

- 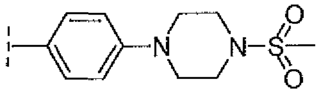 ; 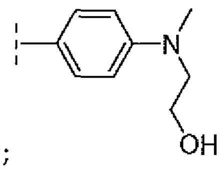 ; 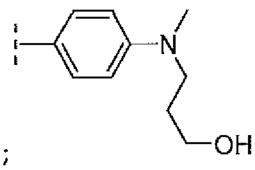 ;

- 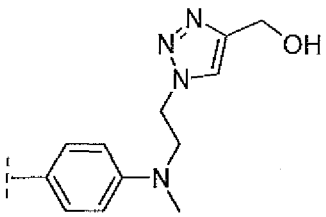 ; 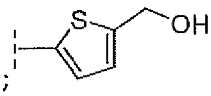 ; 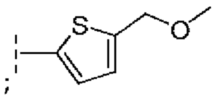 ; 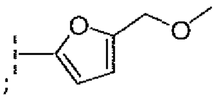 ;

- 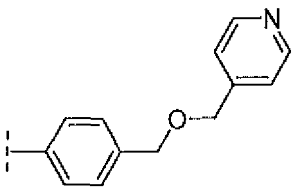 ; 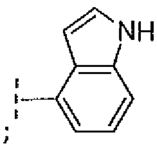 .

L'expression « *éventuellement marqué* » signifie que le substituant dont il est question comprend potentiellement un radioélément. Ainsi, les composés de formule II-1 comprennent un radioélément choisi parmi $^{18}F$, $^{11}C$, $^{123}I$ et $^{124}I$ sur au moins l'un des substituants $R_1$, $R_2$, $R_3$ ou $R_4$.

Selon un mode de réalisation avantageux, le composé de l'Invention est de formule générale I-1 ou II-1 :

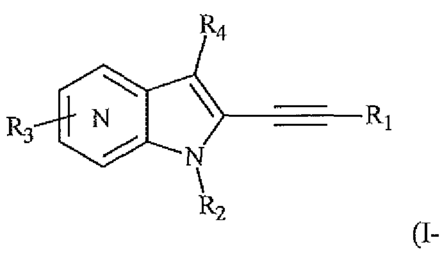

(I-1) ou (II-1)

$R_1$, $R_3$ et $R_4$ étant tels que définis précédemment dans la formule I ou II respectivement ;
$R_2$ étant éventuellement marqué et choisi parmi

- H;
- $(CH_2CH_2O)_n$-$CH_2CH_2F$, n étant un entier égal à 0, 1 ou 2 ;
- $SO_2Ph$ ;
- $COO^tBu$ ;
- $CH_2OCH_3$ ;
- $CH_2O(CH_2)_2OCH_3$;
- $COCH_3$;

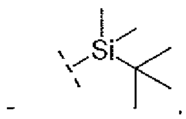

Les composés de formule II-1 comprennent un radioélément choisi parmi $^{18}F$, $^{11}C$, $^{123}I$ et $^{124}I$ sur au moins l'un des substituants $R_1$, $R_2$, $R_3$ ou $R_4$.

Selon un mode de réalisation avantageux, le composé de l'Invention est de formule générale I-1 ou II-1 :

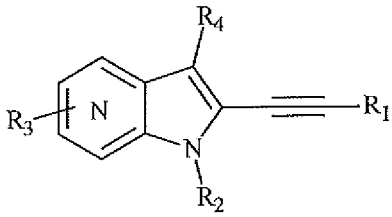

(I-1) ou (II-1)

$R_1$, $R_2$ et $R_4$ étant tels que définis précédemment dans la formule I ou II respectivement ;
$R_3$ étant éventuellement marqué et choisi parmi

- 6-Cl ;
- 6-F ;
- 5-F ;
- 4-F ;
- 6-(2-thiényle) ;
- 6-(3-thiényle) ;
- 6-(6-fluoro-3-pyridyle) ;
- 5-OMe ;
- 6-morpholino ;
- 5-morpholino ;
- 6-Br ;
- 4-Br ;
- 4-Bpin ;
- 5-Bpin.

Les composés de formule II-1 comprennent un radioélément choisi parmi $^{18}F$, $^{11}C$, $^{123}I$ et $^{124}I$ sur au moins l'un des substituants $R_1$, $R_2$, $R_3$ ou $R_4$.

Selon un mode de réalisation avantageux, le composé de l'Invention est de formule générale I-1 ou II-1 :

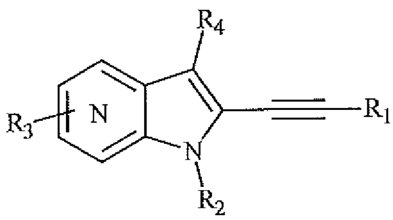

(I-1) ou (II-1)

$R_1$, $R_2$ et $R_3$ étant tels que définis précédemment dans la formule I ou II respectivement ;
$R_4$ étant choisi parmi

- H;
- $(CH_2)_n$-N-alkyl, n étant un entier égal à 0, 1 ou 2 .

Les composés de formule II-1 comprennent un radioélément choisi parmi $^{18}F$, $^{11}C$, $^{123}I$ et $^{124}I$ sur au moins l'un des substituants $R_1$, $R_2$ ou $R_3$.

Selon un mode de réalisation avantageux, le composé de l'Invention est de formule générale I-1a ou II-1a :

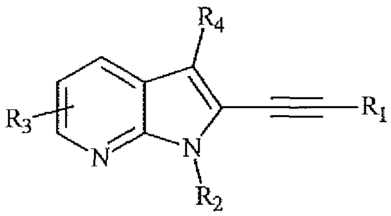

(I-1a) ou (II-1a)

$R_2$, $R_3$ et $R_4$ étant tels que définis précédemment dans la formule I ou II respectivement; $R_1$ étant éventuellement marqué et choisi parmi

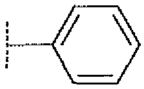 ; 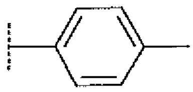 ; 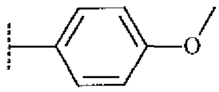 ; 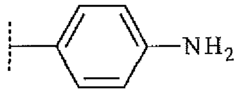

ou

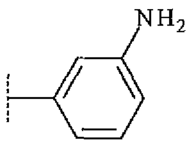 ; 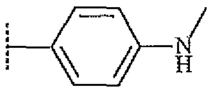 ; 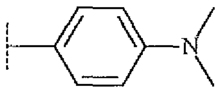 ou 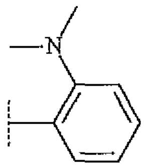

ou

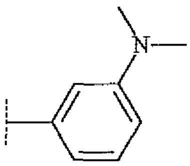 ; 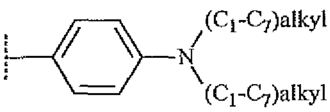 ou 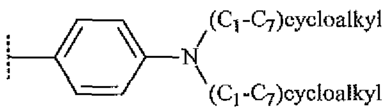 ,

notamment

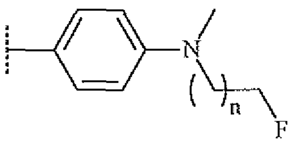 ,

n étant compris de 0 à 6 ;

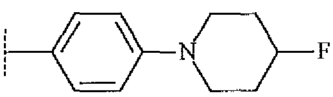 ; 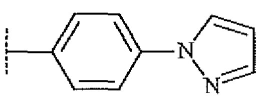 ; 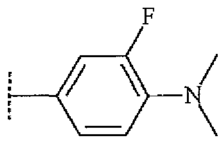

ou

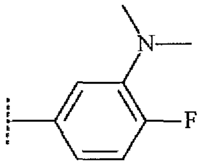 ; 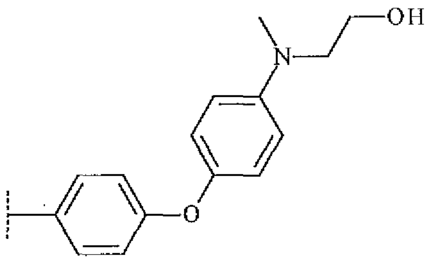 ; 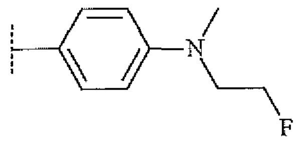 ;

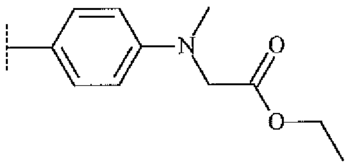 ; 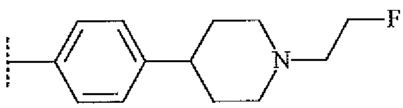 ;

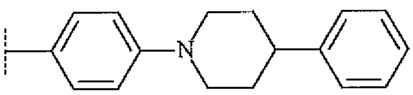 ; 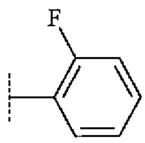 ; 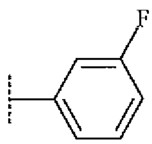 ; 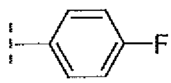 ;

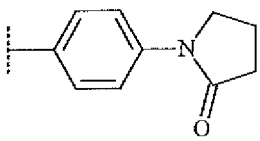 ; 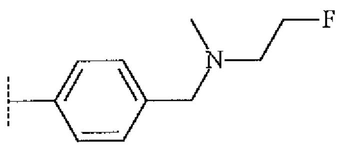 ; 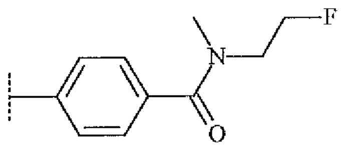 ;

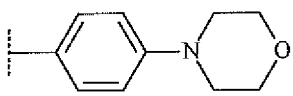 ; 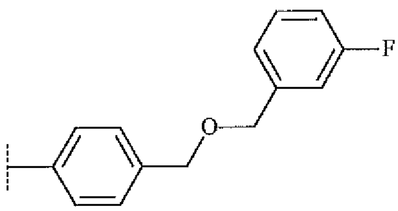 ;

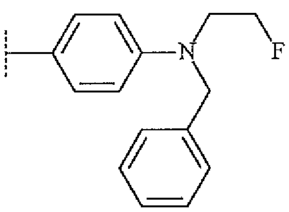 ; 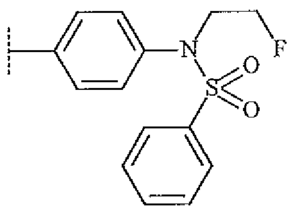 ; 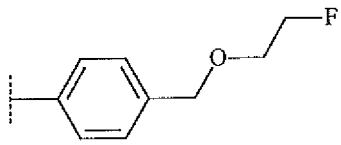 ;

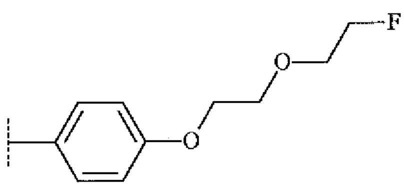 ; 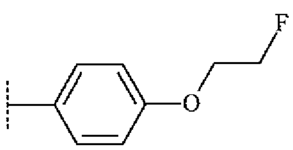 ;

- 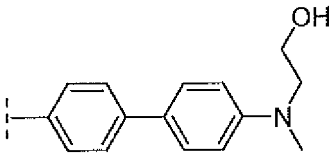 ; 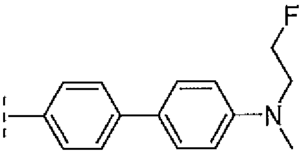 ; 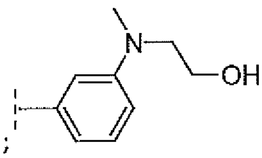 ;

- 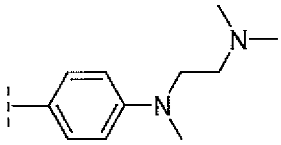 ; 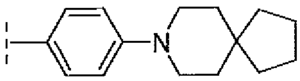 ; 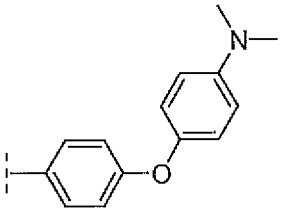 ;

- 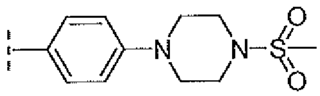 ; 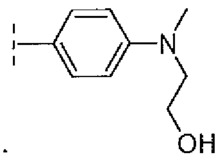 ; 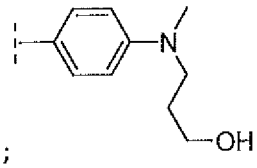 ;

- 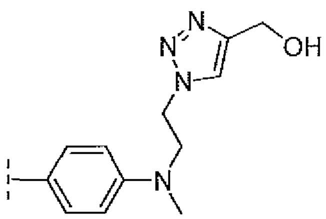 ; 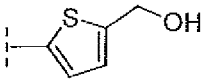 ; 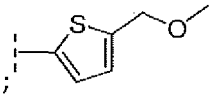 ; 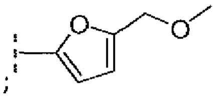 ;

- 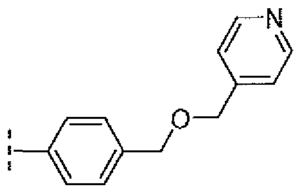 ; 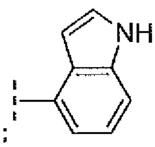 .

Les composés de formule II-1a comprennent un radioélément choisi parmi $^{18}F$, $^{11}C$, $^{123}I$ et $^{124}I$ sur au moins l'un des substituants $R_1$, $R_2$, $R_3$ ou $R_4$.

Selon un mode de réalisation avantageux, le composé de l'Invention est de formule générale I-1a ou II-1a :

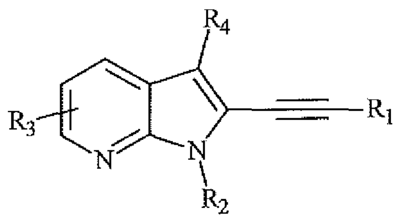

(I-1a) ou (II-1a)

$R_1$, $R_3$ et $R_4$ étant tels que définis précédemment dans la formule I ou II respectivement ;
$R_2$ étant éventuellement marqué et choisi parmi

- H;
- $(CH_2CH_2O)_n$-$CH_2CH_2F$, n étant un entier égal à 0, 1 ou 2 ;

- $SO_2Ph$ ;
- $COO^tBu$ ;
- $CH_2OCH_3$ ;
- $CH_2O(CH_2)_2OCH_3$
- $COCH_3$ ;
- 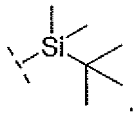

Les composés de formule II-1a comprennent un radioélément choisi parmi $^{18}F$, $^{11}C$, $^{123}I$ et $^{124}I$ sur au moins l'un des substituants $R_1$, $R_2$, $R_3$ ou $R_4$.

Selon un mode de réalisation avantageux, le composé de l'Invention est de formule générale I-1a ou II-1a :

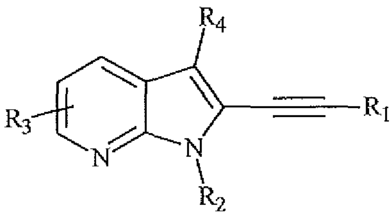

(I-1a) ou (II-1a)

$R_1$, $R_2$ et $R_4$ étant tels que définis précédemment dans la formule I ou II respectivement ;
$R_3$ étant éventuellement marqué et choisi parmi

- 6-Cl ;
- 6-F ;
- 5-F ;
- 4-F ;
- 6-(2-thiényle) ;
- 6-(3-thiényle) ;
- 6-(6-fluoro-3-pyridyle) ;
- 5-OMe
- 6-morpholino ;
- 5-morpholino ;
- 6-Br ;
- 4-Br ;
- 4-Bpin ;
- 5-Bpin.

Les composés de formule II-1a comprennent un radioélément choisi parmi $^{18}F$, $^{11}C$, $^{123}I$ et $^{124}I$ sur au moins l'un des substituants $R_1$, $R_2$, $R_3$ ou $R_4$.

Selon un mode de réalisation avantageux, le composé de l'Invention est de formule générale I-1a ou II-1a :

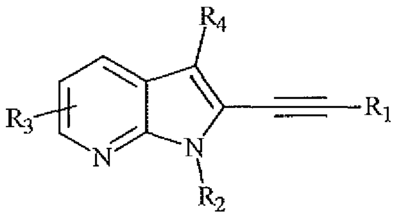

(I-1a) ou (II-1a)

$R_1$, $R_2$ et $R_3$ étant tels que définis précédemment dans la formule I ou II respectivement ;
$R_4$ étant choisi parmi

- H;

$(CH_2)_n$-*N*-alkyl, n étant un entier égal à 0, 1 ou 2 .

Les composés de formule II-1a comprennent un radioélément choisi parmi $^{18}F$, $^{11}C$, $^{123}I$ et $^{124}I$ sur au moins l'un des substituants $R_1$, $R_2$ ou $R_3$.

Selon un mode de réalisation avantageux, le composé de l'Invention est de formule générale I-1a ou II-1a :

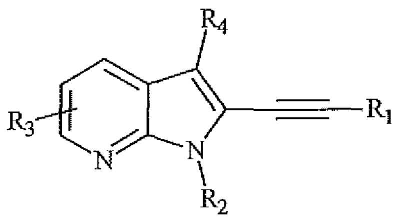

(I-1a) ou (II-1a)

$R_3$ et $R_4$ étant tels que définis précédemment dans la formule I ou II respectivement ;
$R_1$ étant éventuellement marqué et choisi parmi

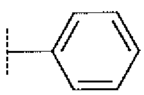 ; 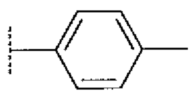 ; 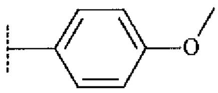 ; 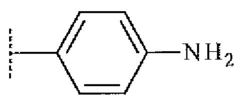

ou

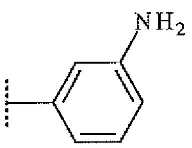 ; 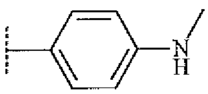 ; 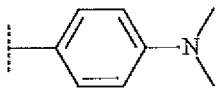 ou 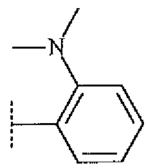

ou

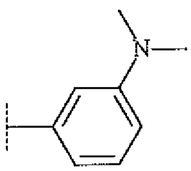 ; 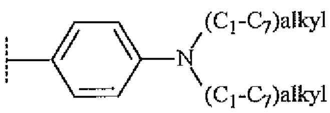 ou 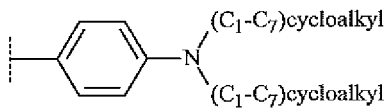 ,

notamment

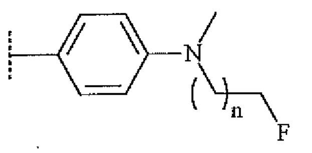 ,

n étant compris de 0 à 6 ;

- 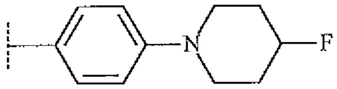 ; 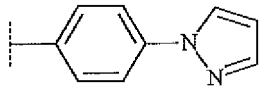 ; 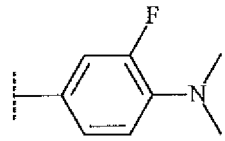

ou

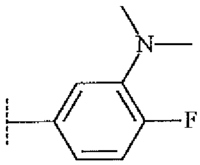 ; 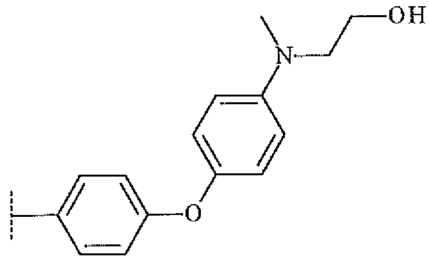 ; 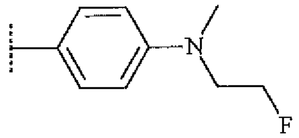 ;

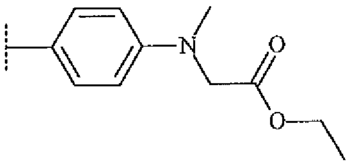 ; 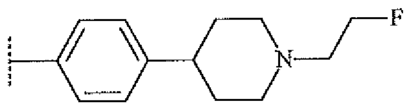 ;

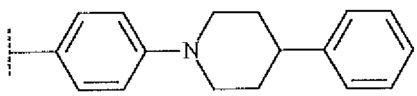 ; 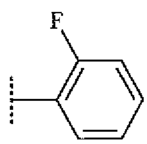 ; 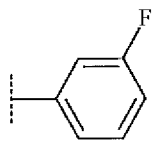 ; 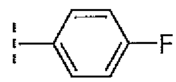 ;

- 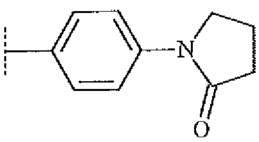 ; 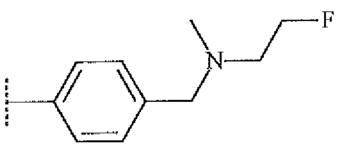 ; 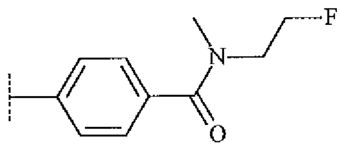 ;

- 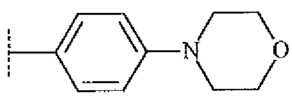 ; 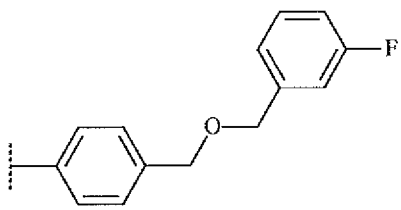 ;

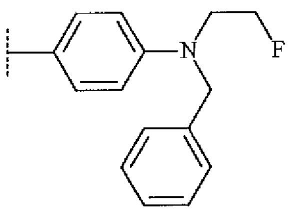 ; 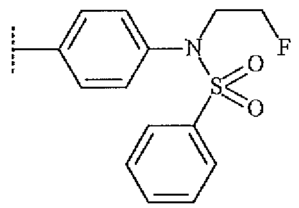 ; 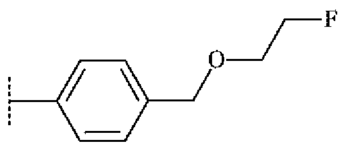 ;

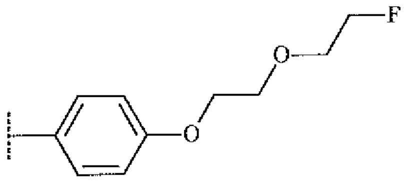 ; 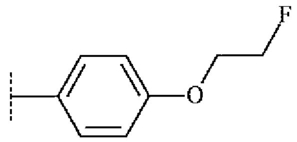 ;

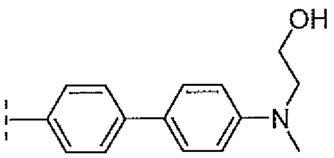 ; 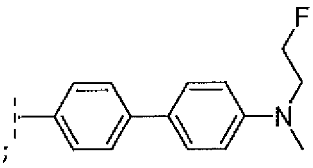 ; 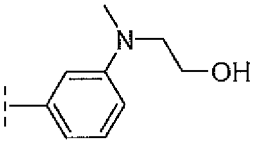 ;

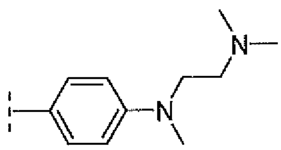 ; 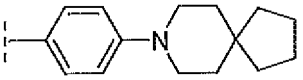 ; 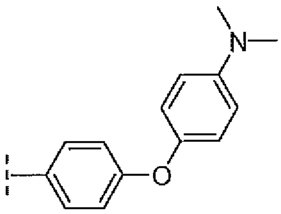 ;

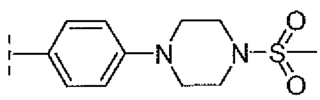 ; 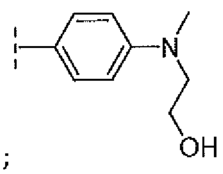 ; 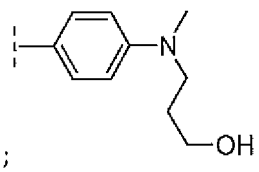 ;

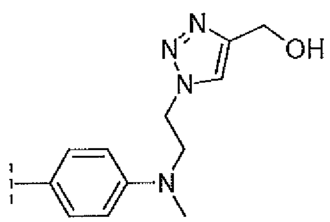 ; 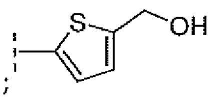 ; 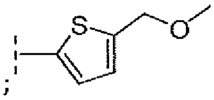 ; 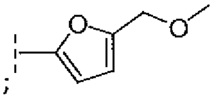 ;

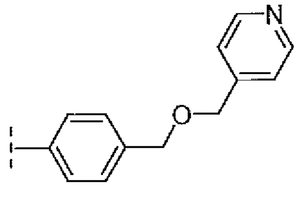 ; 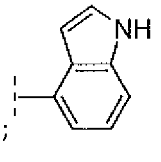 .

$R_2$ étant éventuellement marqué et choisi parmi

- H ;
- $(CH_2CH_2O)_n$-$CH_2CH_2F$, n étant un entier égal à 0, 1 ou 2 ;
- $SO_2Ph$ ;
- $COO^tBu$ ;
- $CH_2OCH_3$ ;
- $CH_2O(CH_2)_2OCH_3$
- $COCH_3$;
- 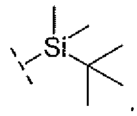

.

Les composés de formule II-1a comprennent un radioélément choisi parmi $^{18}F$, $^{11}C$, $^{123}I$ et $^{124}I$ sur au moins l'un des substituants $R_1$, $R_2$, $R_3$ ou $R_4$.

Selon un mode de réalisation avantageux, le composé de l'Invention est de formule générale I-1a ou II-1a :

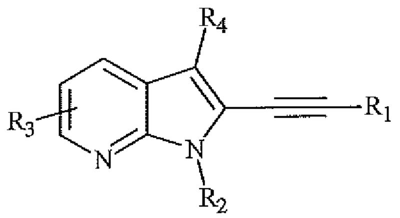

(I-1a) ou (II-1a)

$R_2$ et $R_4$ étant tels que définis précédemment dans la formule I ou II respectivement ;
$R_1$ étant éventuellement marqué et choisi parmi

- 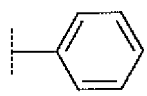 ; 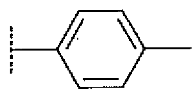 ; 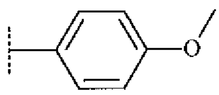 ; 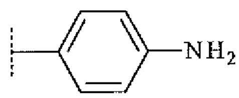

ou

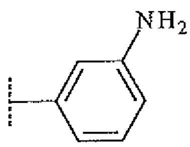 ; 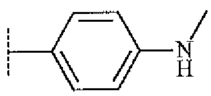 ; 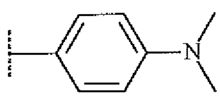 ou 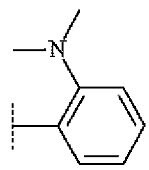

ou

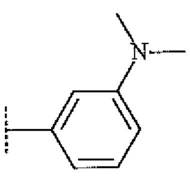 ; 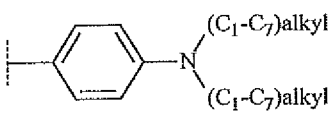 ou 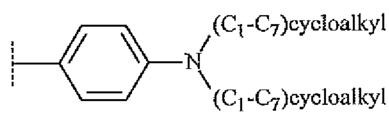 ,

notamment

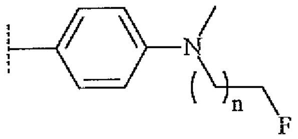,

n étant compris de 0 à 6 ;

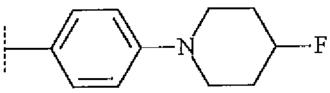 ; 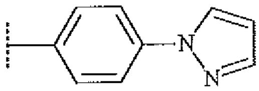 ; 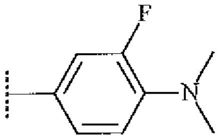

ou

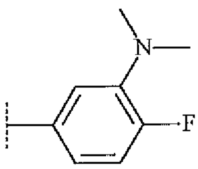 ; 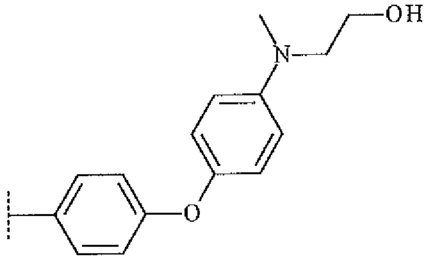 ; 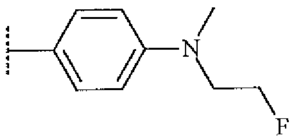 ;

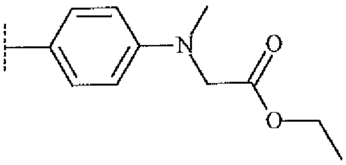 ; 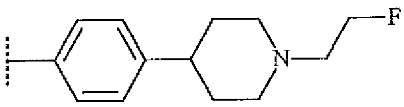 ;

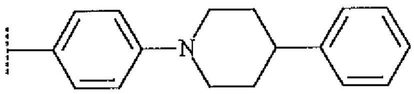 ; 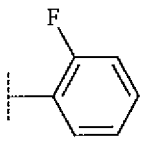 ; 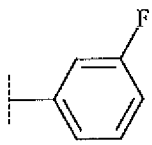 ; 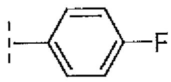 ;

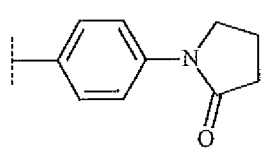 ; 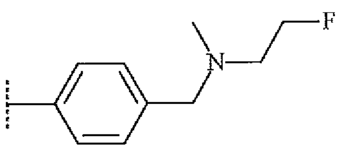 ; 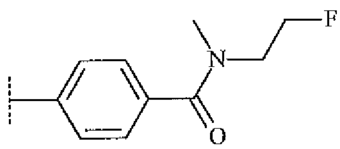 ;

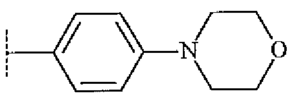 ; 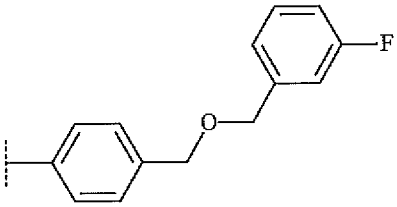 ;

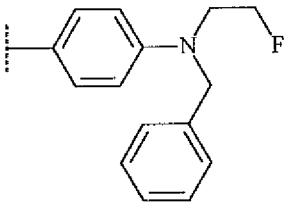 ; 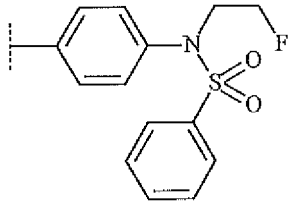 ; 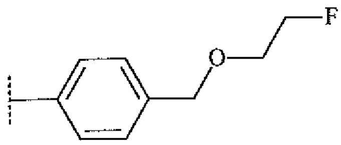 ;

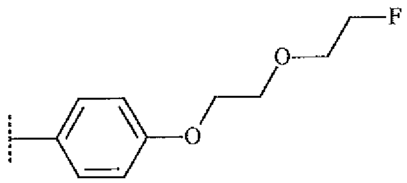 ; 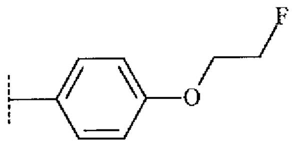 ;

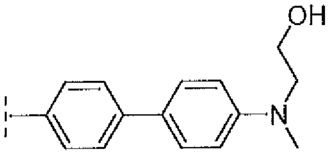 ; 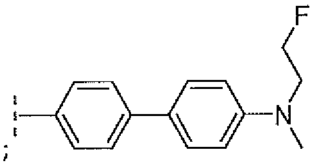 ; 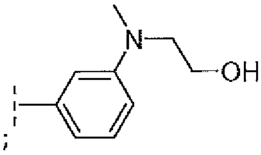 ;

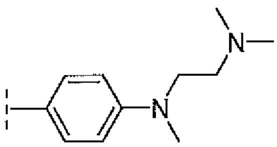 ; 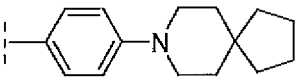 ; 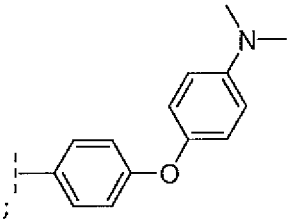 ;

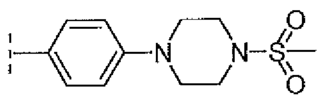 ; 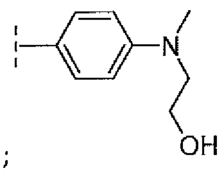 ; 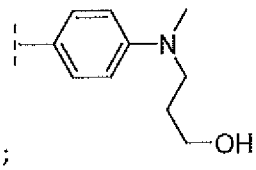 ;

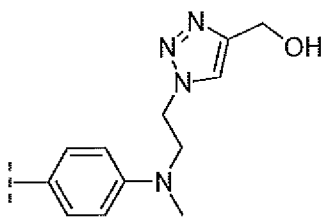 ; 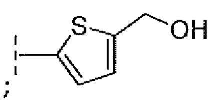 ; 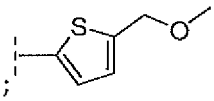 ; 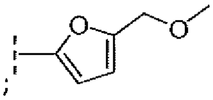 ;

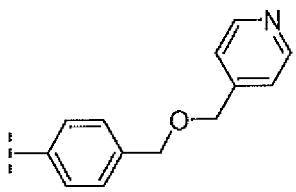 ; 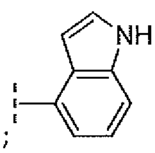 .

$R_3$ étant éventuellement marqué et choisi parmi

- 6-Cl ;
- 6-F ;
- 5-F ;
- 4-F ;
- 6-(2-thiényle) ;
- 6-(3-thiényle) ;
- 6-(6-fluoro-3-pyridyle) ;
- 5-OMe ;
- 6-morpholino ;
- 5-morpholino ;
- 6-Br ;
- 4-Br ;
- 4-Bpin ;
- 5-Bpin.

Les composés de formule II-1a comprennent un radioélément choisi parmi $^{18}F$, $^{11}C$, $^{123}I$ et $^{124}I$ sur au moins l'un des substituants $R_1$, $R_2$, $R_3$ ou $R_4$.

Selon un mode de réalisation avantageux, le composé de l'Invention est de formule générale I-1a ou II-1a :

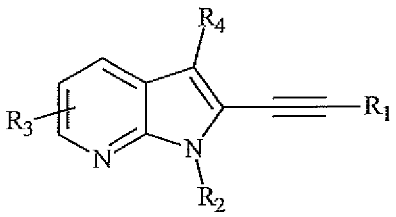

(I-1a) ou (II-1a)

$R_2$ et $R_3$ étant tels que définis précédemment dans la formule I ou II respectivement ;

$R_1$ étant éventuellement marqué et choisi parmi

- 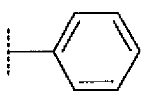 ; 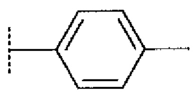 ; 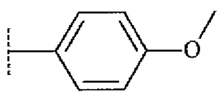 ; 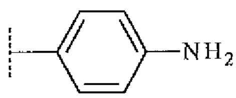

ou

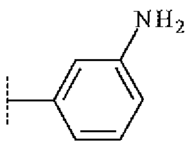 ; 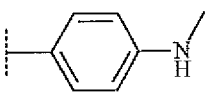 ; 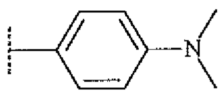 ou 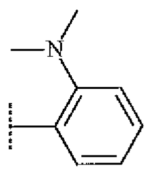

ou

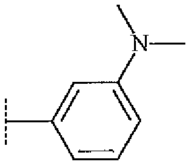 ; 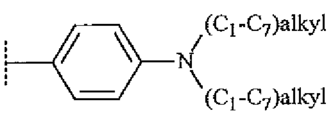 ou 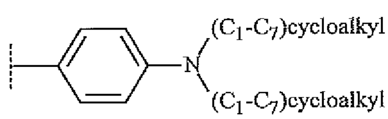 ,

notamment

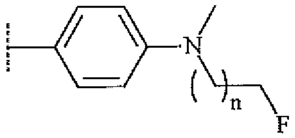,

n étant compris de 0 à 6 ;

- 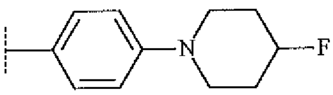 ; 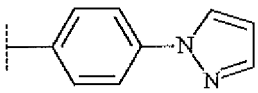 ; 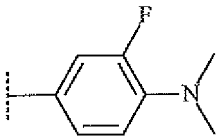

ou

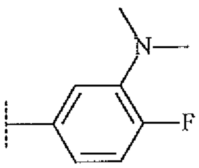 ; 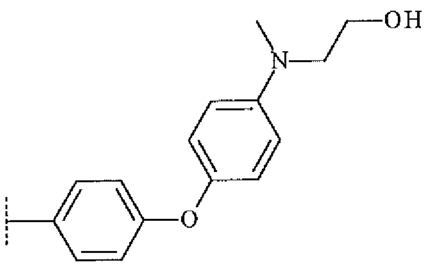 ; 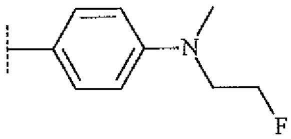 ;

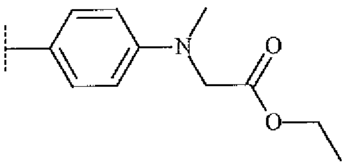 ; 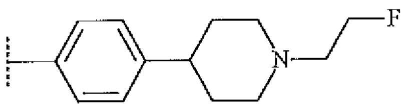 ;

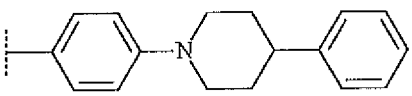 ; 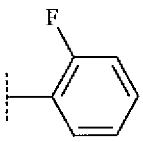 ; 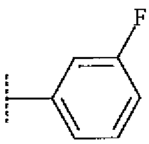 ; 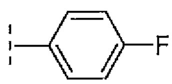 ;

- 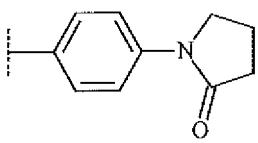 ; 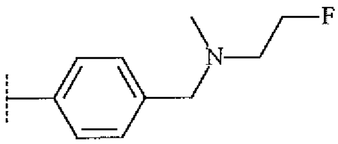 ; 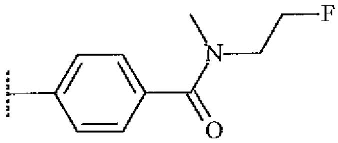 ;

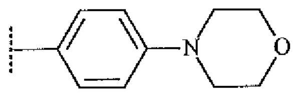 ; 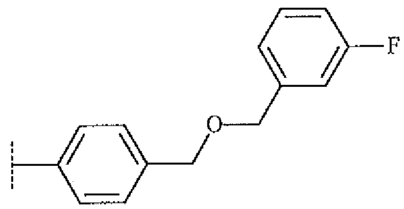 ;

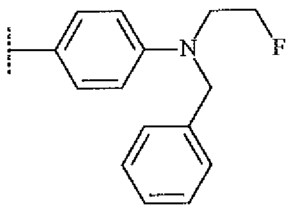 ; 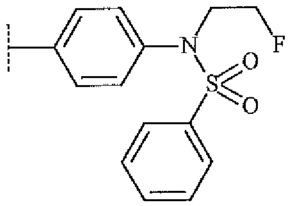 ; 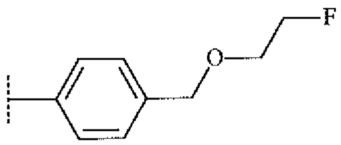 ;

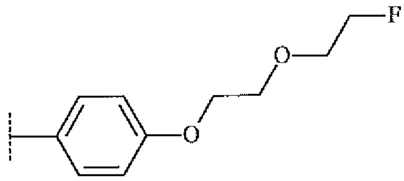 ; 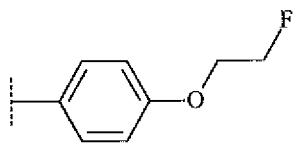 ;

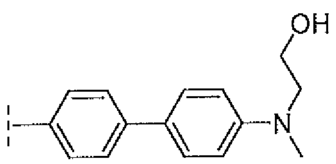 ; 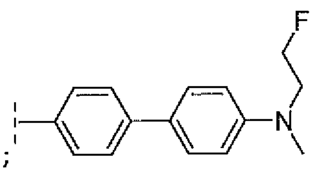 ; 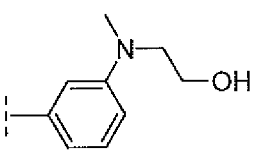 ;

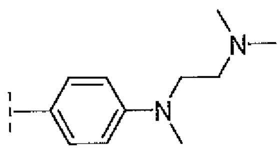 ; 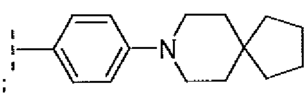 ; 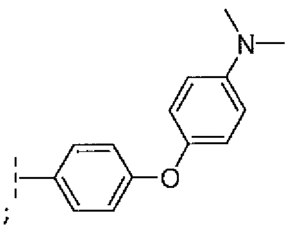 ;

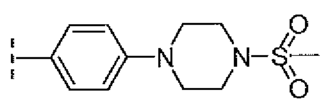 ; 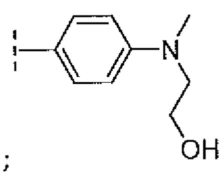 ; 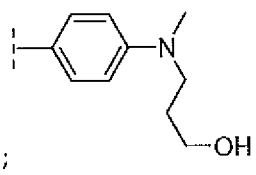 ;

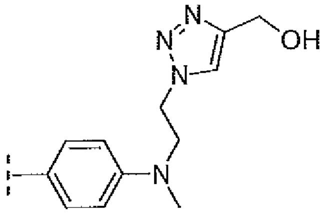 ; 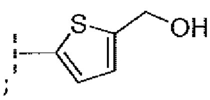 ; 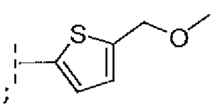 ; 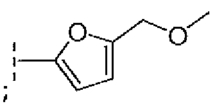 ;

- 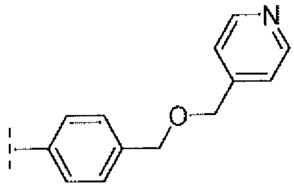 ; 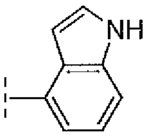 .

$R_4$ étant choisi parmi
- H ;
$(CH_2)_n$-*N*-alkyl, n étant un entier égal à 0, 1 ou 2.

Les composés de formule II-1a comprennent un radioélément choisi parmi $^{18}F$, $^{11}C$, $^{123}I$ et $^{124}I$ sur au moins l'un des substituants $R_1$, $R_2$ ou $R_3$.

Selon un mode de réalisation avantageux, le composé de l'Invention est de formule générale I-1a ou II-1a :

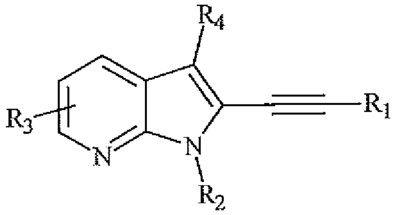

(I-1a) ou (II-1a)

$R_1$ et $R_4$ étant tels que définis précédemment dans la formule I ou II respectivement ;
$R_2$ étant éventuellement marqué et choisi parmi

- H;
- $(CH_2CH_2O)_n$-$CH_2CH_2F$, n étant un entier égal à 0, 1 ou 2 ;
- $SO_2Ph$ ;
- $COO^tBu$ ;
- $CH_2OCH_3$ ;
- $CH_2O(CH_2)_2OCH_3$
- $COCH_3$ ;
- 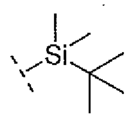 ;

$R_3$ étant éventuellement marqué et choisi parmi

- 6-Cl ;
- 6-F ;
- 5-F ;
- 4-F ;
- 6-(2-thiényle) ;
- 6-(3-thiényle) ;
- 6-(6-fluoro-3-pyridyle) ;
- 5-OMe ;
- 6-morpholino ;
- 5-morpholino ;
- 6-Br;
- 4-Br ;
- 4-Bpin ;
- 5-Bpin.

Les composés de formule II-1a comprennent un radioélément choisi parmi $^{18}F$, $^{11}C$, $^{123}I$ et $^{124}I$ sur au moins l'un des substituants $R_1$, $R_2$, $R_3$ ou $R_4$.

Selon un mode de réalisation avantageux, le composé de l'Invention est de formule générale I-1a ou II-1a :

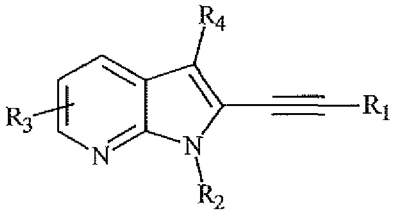

(I-1a) ou (II-1a)

$R_1$ et $R_3$ étant tels que définis précédemment dans la formule I ou II respectivement ;
$R_2$ étant éventuellement marqué et choisi parmi

- H ;
- $(CH_2CH_2O)_n$-$CH_2CH_2F$, n étant un entier égal à 0, 1 ou 2 ;
- $SO_2Ph$ ;
- COO*t*Bu ;
- $CH_3OCH_3$ ;
- $CH_2O(CH_2)_2OCH_3$
- $COCH_3$;
- 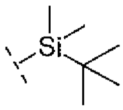 ;

$R_4$ étant choisi parmi

- H ;

$(CH_2)_n$-*N*-alkyl, n étant un entier égal à 0, 1 ou 2 .

Les composés de formule II-1a comprennent un radioélément choisi parmi $^{18}F$, $^{11}C$, $^{123}I$ et $^{124}I$ sur au moins l'un des substituants $R_1$, $R_2$ ou $R_3$.

Selon un mode de réalisation avantageux, le composé de l'Invention est de formule générale I-1a ou II-1a :

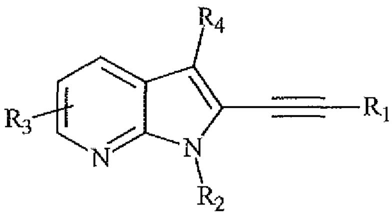

(I-1a) ou (II-1a)

$R_1$ et $R_2$ étant tels que définis précédemment dans la formule I ou II respectivement ;
$R_3$ étant éventuellement marqué et choisi parmi

- 6-Cl ;
- 6-F ;
- 5-F ;
- 4-F ;
- 6-(2-thiényle) ;
- 6-(3-thiényle) ;
- 6-(6-fluoro-3-pyridyle) ;
- 5-OMe ;

- 6-morpholino ;
- 5-morpholino ;
- 6-Br ;
- 4-Br ;
- 4-Bpin ;
- 5-Bpin.

$R_4$ étant choisi parmi

- H;

$(CH_2)_n$-*N*-alkyl, n étant un entier égal à 0, 1 ou 2.

Les composés de formule II-1a comprennent un radioélément choisi parmi $^{18}F$, $^{11}C$, $^{123}I$ et $^{124}I$ sur au moins l'un des substituants $R_1$, $R_2$ ou $R_3$.

Selon un mode de réalisation avantageux, le composé de l'Invention est de formule générale I-1a ou II-1a :

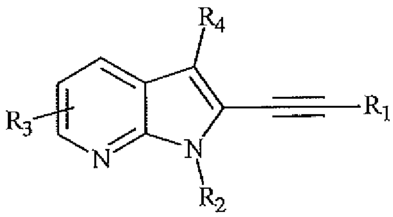

(I-1a) ou (II-1a)

$R_4$ étant tel que défini précédemment dans la formule I ou II respectivement ;
$R_1$ étant éventuellement marqué et choisi parmi

- 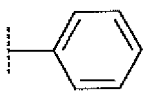 ; 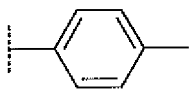 ; 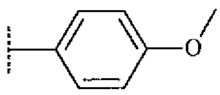 ; 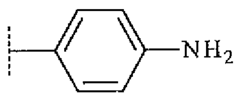

ou

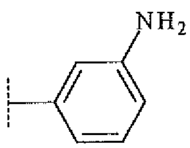 ; 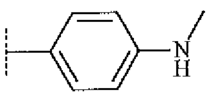 ; 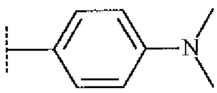 ou 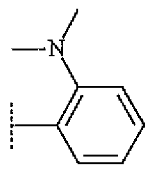

ou

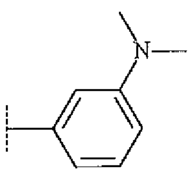 ; 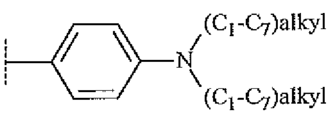 ou 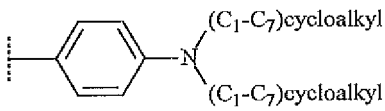 ,

notamment

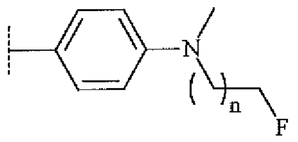 ,

n étant compris de 0 à 6 ;

- 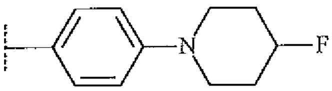 ; 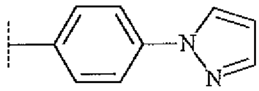 ; 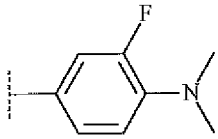

ou

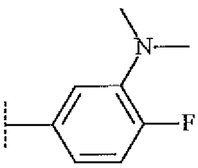 ; 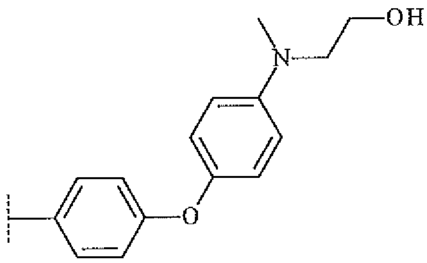 ; 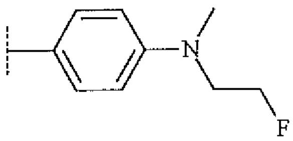 ;

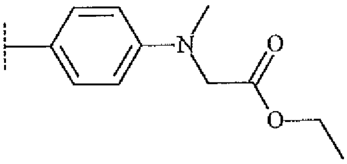 ; 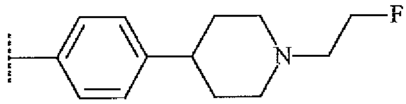 ;

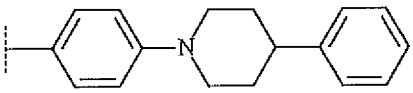 ; 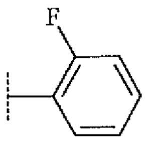 ; 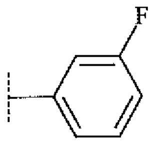 ; 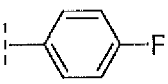 ;

- 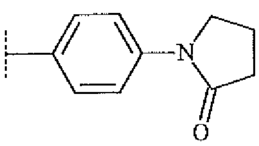 ; 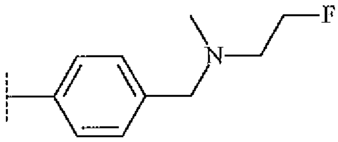 ; 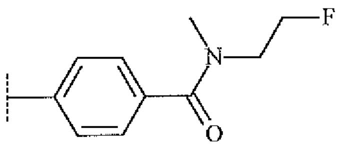 ;

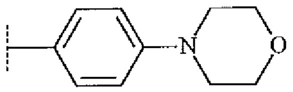 ; 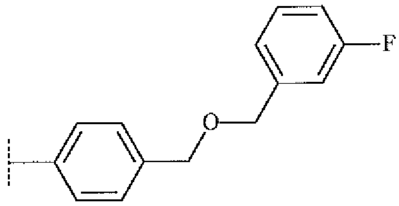 ;

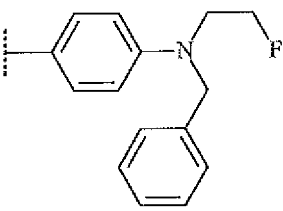 ; 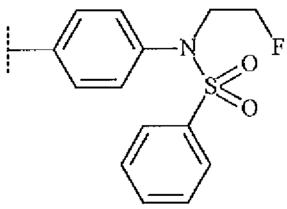 ; 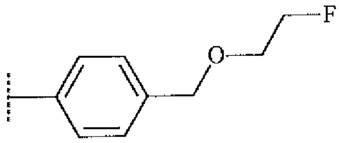 ;

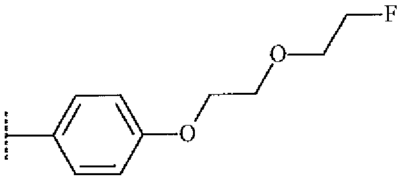 ; 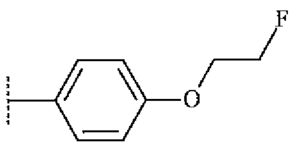 ;

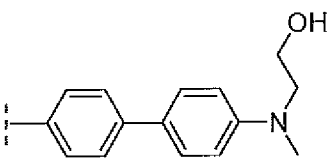 ; 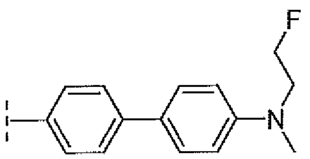 ; 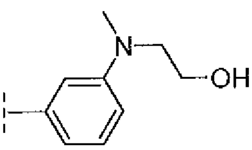 ;

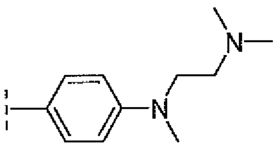 ; 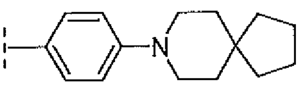 ; 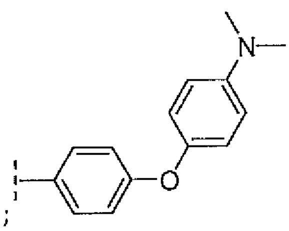 ;

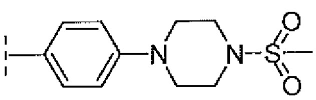 ; 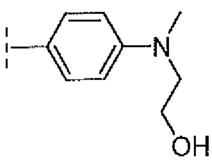 ; 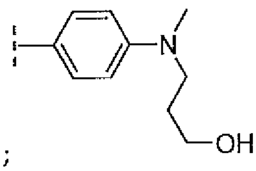 ;

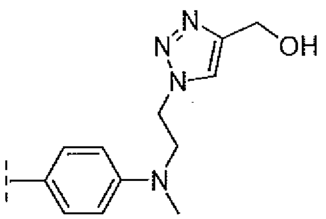 ; 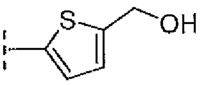 ; 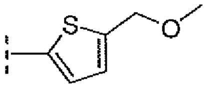 ; 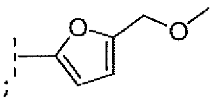 ;

- 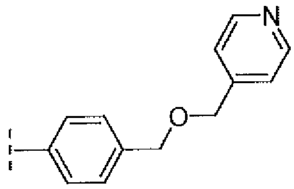 ; 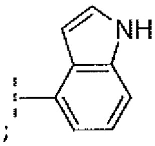 .

$R_2$ étant éventuellement marqué et choisi parmi

- H;
- $(CH_2CH_2O)_n$-$CH_2CH_2F$, n étant un entier égal à 0, 1 ou 2 ;
- $SO_2Ph$ ;
- $COO^tBu$ ;
- $CH_2OCH_3$ ;
- $CH_2O(CH_2)_2OCH_3$
- $COCH_3$ ;

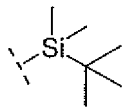 ;

$R_3$ étant éventuellement marqué et choisi parmi

- 6-Cl ;
- 6-F ;
- 5-F ;
- 4-F ;
- 6-(2-thiényle) ;
- 6-(3-thiényle) ;
- 6-(6-fluoro-3-pyridyle) ;
- 5-OMe ;
- 6-morpholino ;
- 5-morpholino ;
- 6-Br ;
- 4-Br ;
- 4-Bpin ;
- 5-Bpin.

Les composés de formule II-1a comprennent un radioélément choisi parmi $^{18}F$, $^{11}C$, $^{123}I$ et $^{124}I$ sur au moins l'un des substituants $R_1$, $R_2$, $R_3$ ou $R_4$.

Selon un mode de réalisation avantageux, le composé de l'Invention est de formule générale I-1a-1 ou II-1a-1 :

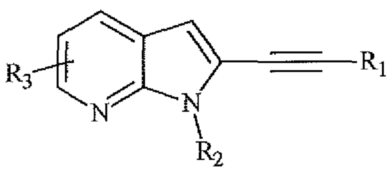

(I-1a-1) ou (II-1a-1)

$R_3$ étant tel que défini précédemment dans la formule I ou II respectivement;
$R_1$ étant éventuellement marqué et choisi parmi

- 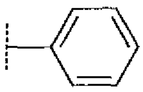 ; 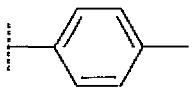 ; 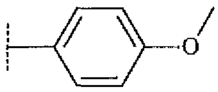 ; 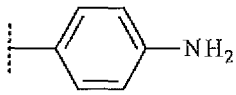

ou

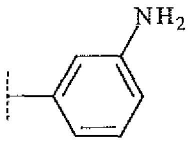 ; 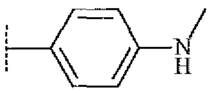 ; 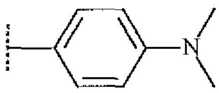 ou 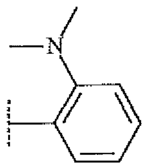

ou

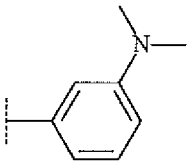 ; 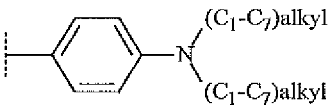 ou 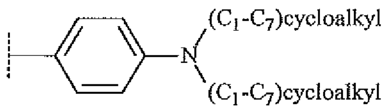 ,

notamment

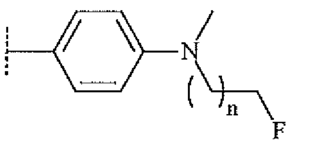 ,

n étant compris de 0 à 6 ;

- 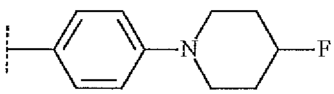 ; 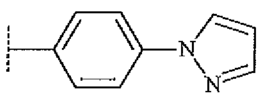 ; 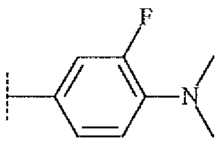

ou

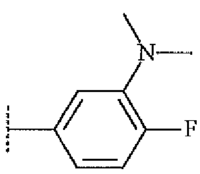 ; 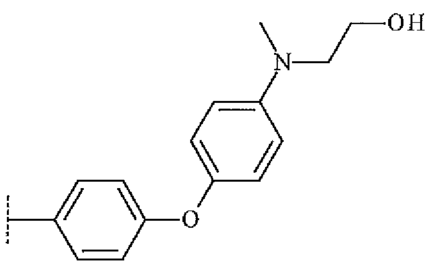 ; 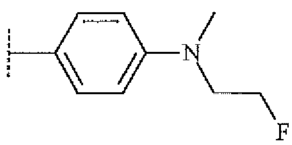 ;

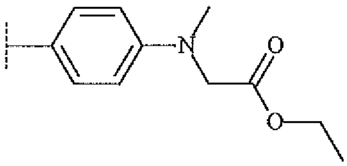;
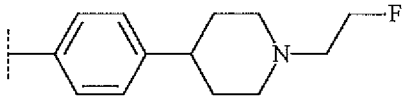;

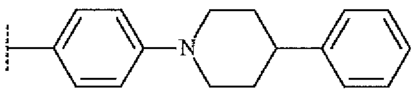;
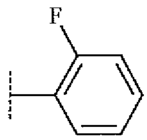;
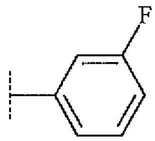;
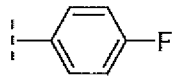;

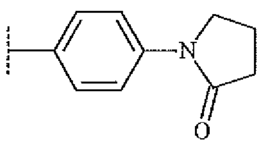;
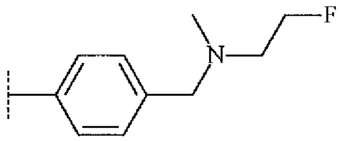;
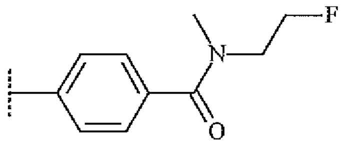;

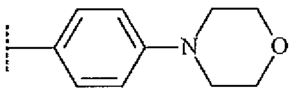;
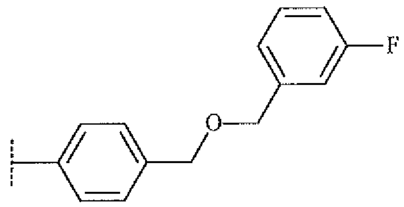;

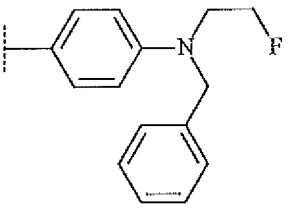;
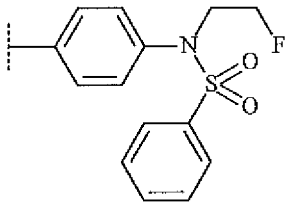;
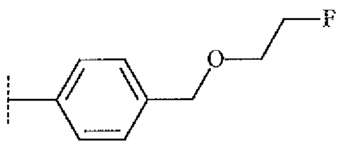;

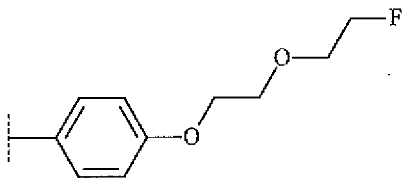;
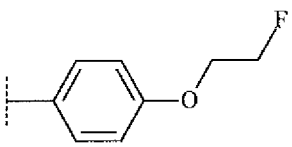;

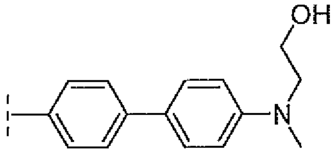;
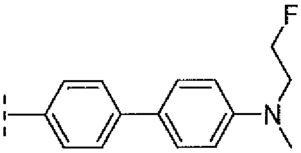;
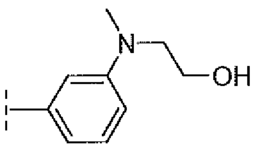;

- 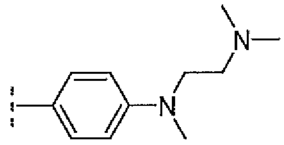 ; 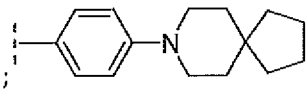 ; 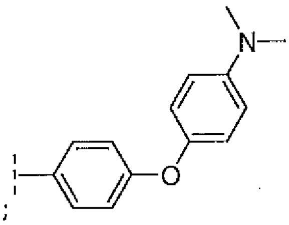 ;
- 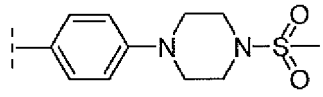 ; 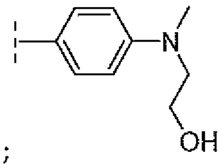 ; 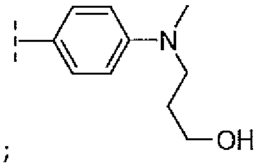 ;
- 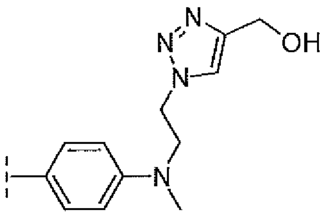 ; 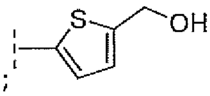 ; 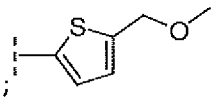 ; 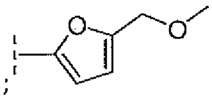 ;
- 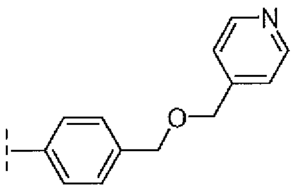 ; 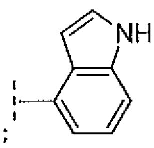 .

$R_2$ étant éventuellement marqué et choisi parmi

- H;
- $(CH_2CH_2O)_n$-$CH_2CH_2F$, n étant un entier égal à 0, 1 ou 2 ;
- $SO_2Ph$ ;
- $COO^tBu$ ;
- $CH_2OCH_3$ ;
- $CH_2O(CH_2)_2OCH_3$
- $COCH_3$ ;
- 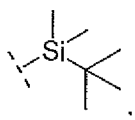 .

Les composés de formule II-1a-1 comprennent un radioélément choisi parmi $^{18}F$, $^{11}C$, $^{123}I$ et $^{124}I$ sur au moins l'un des substituants $R_1$, $R_2$ ou $R_3$.

Selon un mode de réalisation avantageux, le composé de l'Invention est de formule générale I-1a-1 ou II-1a-1 :

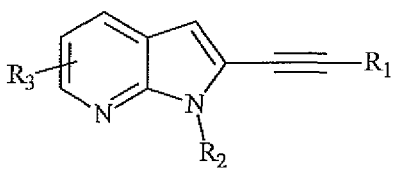

(I-1a-1) ou (II-1a-1)

$R_2$ étant tel que défini précédemment dans la formule I ou II respectivement ;
$R_1$ étant éventuellement marqué et choisi parmi

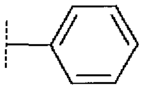 ; 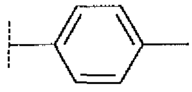 ; 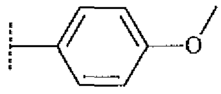 ; 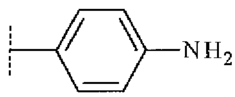

ou

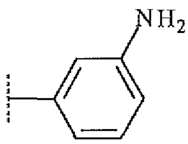 ; 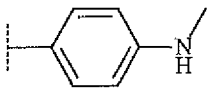 ; 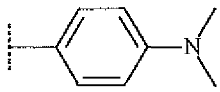 ou 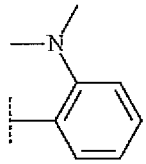

ou

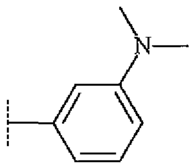 ; 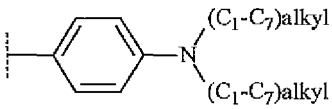 ou 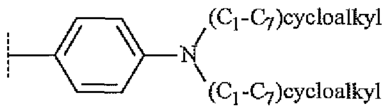 ,

notamment

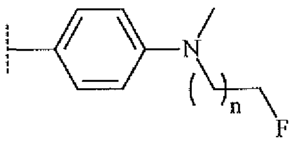 ,

n étant compris de 0 à 6 ;

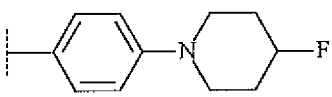 ; 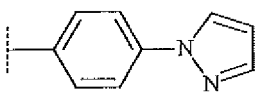 ; 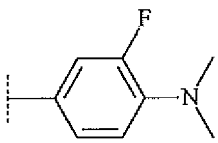

ou

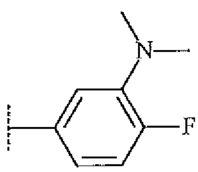 ; 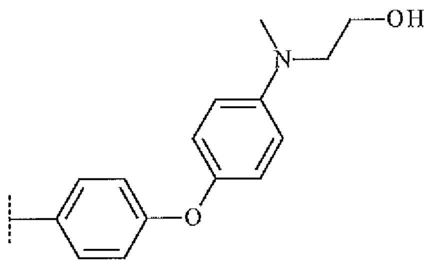 ; 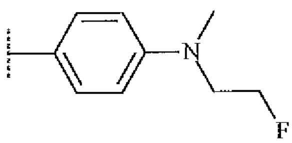 ;

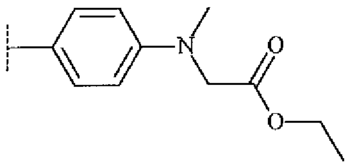 ; 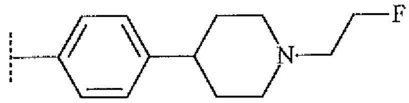 ;

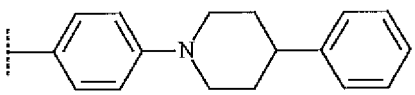 ; 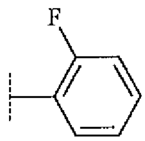 ; 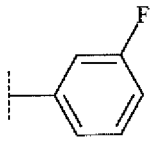 ; 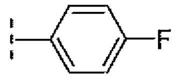 ;

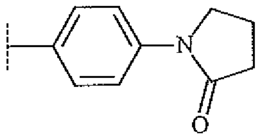 ; 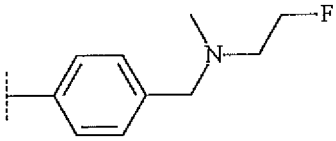 ; 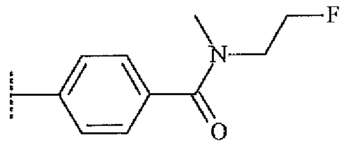 ;

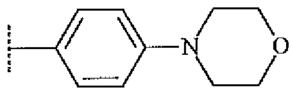 ; 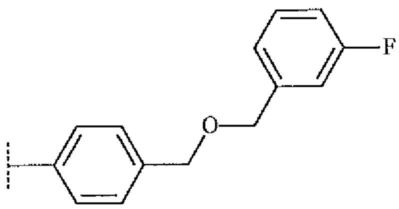 ;

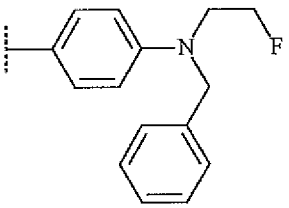 ; 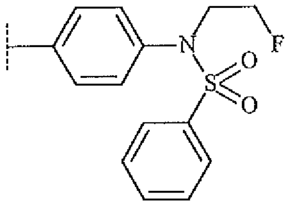 ; 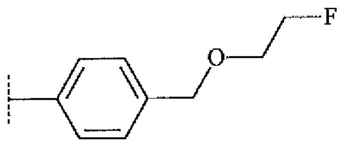 ;

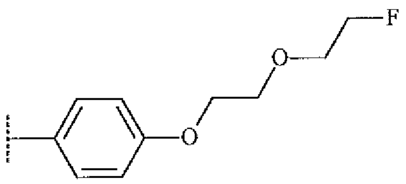 ; 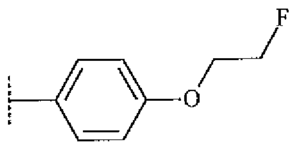 ;

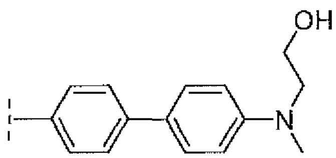 ; 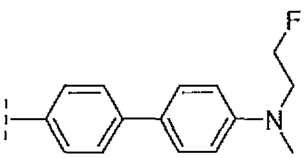 ; 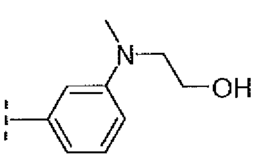 ;

- 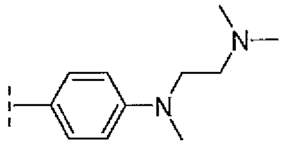 ; 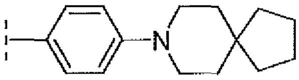 ; 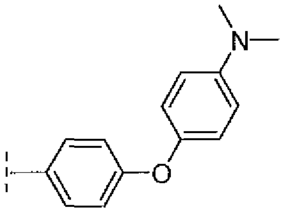 ;

- 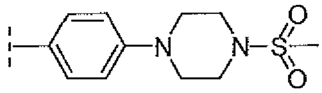 ; 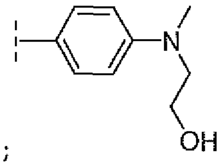 ; 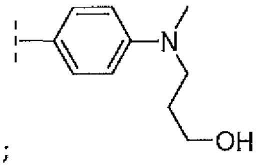 ;

- 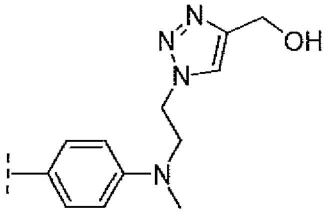 ; 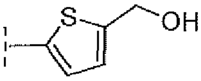 ; 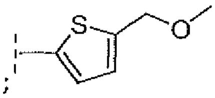 ; 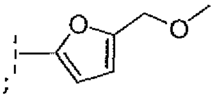 ;

- 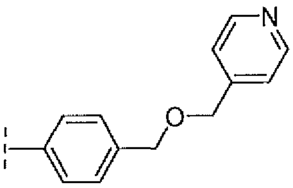 ; 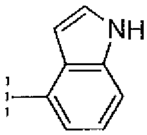 .

$R_3$ étant éventuellement marqué et choisi parmi

- 6-Cl ;
- 6-F ;
- 5-F ;
- 4-F ;
- 6-(2-thiényle) ;
- 6-(3-thiényle) ;
- 6-(6-fluoro-3-pyridyle) ;
- 5-OMe ;
- 6-morpholino ;
- 5-morpholino ;
- 6-Br ;
- 4-Br ;
- 4-Bpin ;
- 5-Bpin.

Les composés de formule II-Ia-1 comprennent un radioélément choisi parmi $^{18}F$, $^{11}C$, $^{123}I$ et $^{124}I$ sur au moins l'un des substituants $R_1$, $R_2$ ou $R_3$.

Selon un mode de réalisation avantageux, le composé de l'Invention est de formule générale I-Ia-1 ou II-1a-1 :

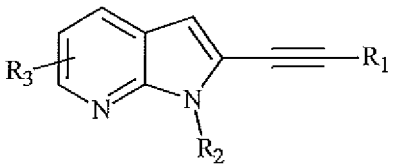

(I-1a-1) ou (II-1a-1)

$R_1$ étant tel que défini précédemment dans la formule I ou II respectivement ;
$R_2$ étant éventuellement marqué et choisi parmi

- H;
- $(CH_2CH_2O)_n$-$CH_2CH_2F$, n étant un entier égal à 0, 1 ou 2 ;
- $SO_2Ph$ ;
- $COO^tBu$ ;
- $CH_2OCH_3$;
- $CH_2O(CH_2)_2OCH_3$
- $COCH_3$;
- 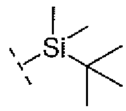 ;

$R_3$ étant éventuellement marqué et choisi parmi

- 6-Cl ;
- 6-F ;
- 5-F ;
- 4-F ;
- 6-(2-thiényle) ;
- 6-(3-thiényle) ;
- 6-(6-fluoro-3-pyridyle) ;
- 5-OMe ;
- 6-morpholino ;
- 5-morpholino ;
- 6-Br ;
- 4-Br ;
- 4-Bpin ;
- 5-Bpin.

Les composés de formule II-1a-1 comprennent un radioélément choisi parmi $^{18}F$, $^{11}C$, $^{123}I$ et $^{124}I$ sur au moins l'un des substituants $R_1$, $R_2$ ou $R_3$.

Selon un mode de réalisation avantageux, le composé de l'Invention est de formule générale I-1a-1 ou II-1a-1 :

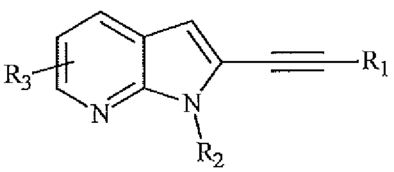

(I-1a-1) ou (II-1a-1)

$R_1$ étant éventuellement marqué et choisi parmi

- 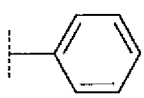 ; 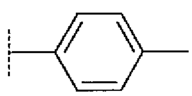 ; 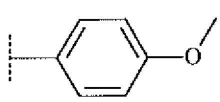 ; 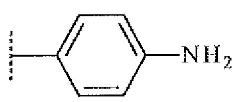

ou

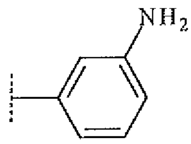
;
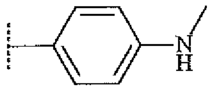
;
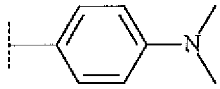
ou
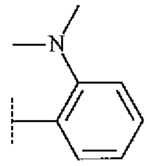

ou

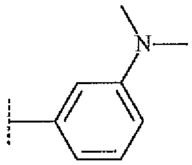
;
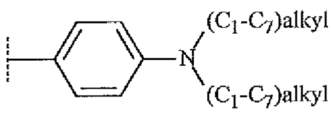
ou
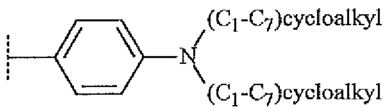
,

notamment

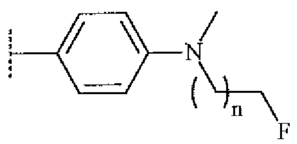
,

n étant compris de 0 à 6 ;

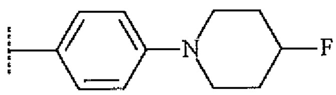
;
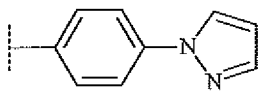
;
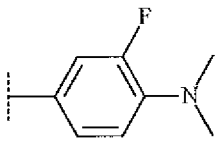

ou

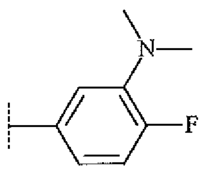
;
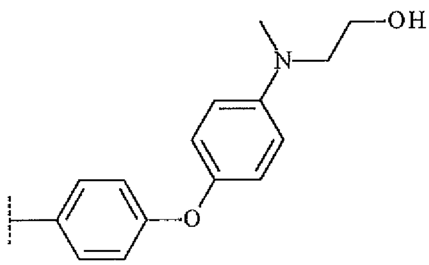
;
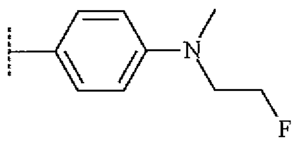
;

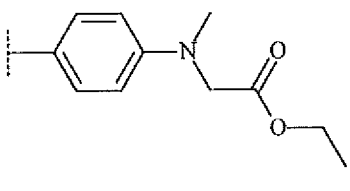
;
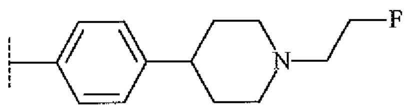
;

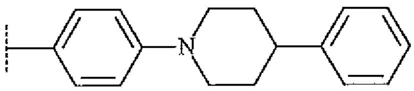 ; 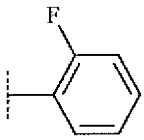 ; 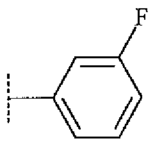 ; 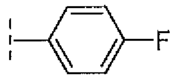 ;

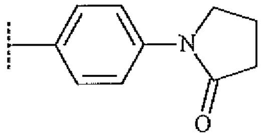 ; 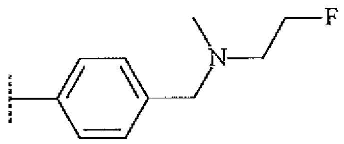 ; 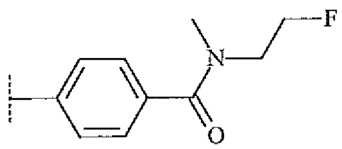 ;

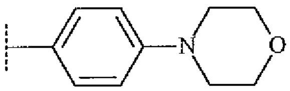 ; 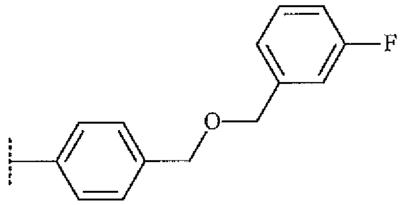 ;

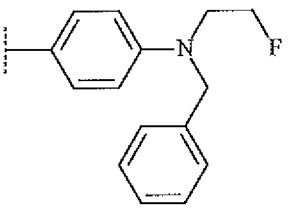 ; 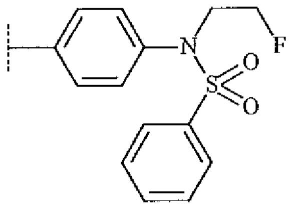 ; 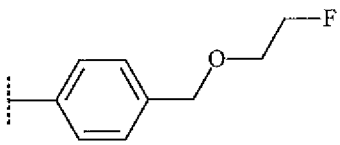 ;

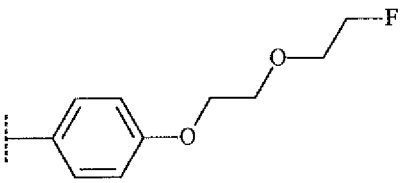 ; 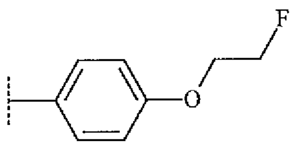 ;

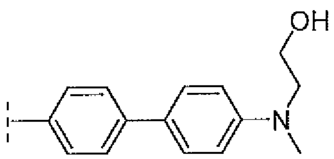 ; 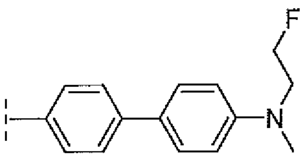 ; 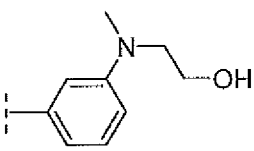 ;

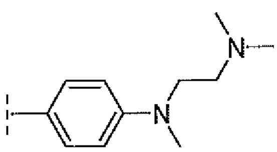 ; 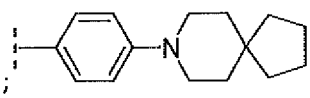 ; 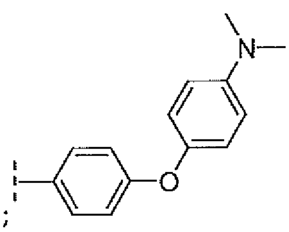 ;

- 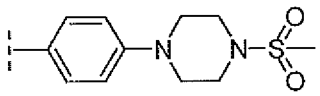 ; 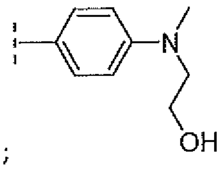 ; 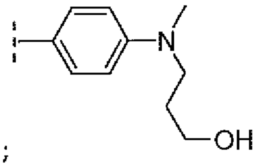 ;
- 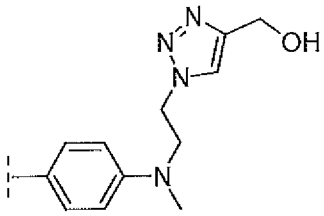 ; 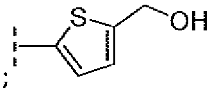 ; 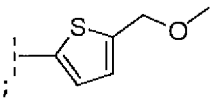 ; 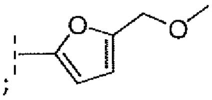 ;
- 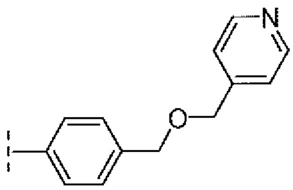 ; 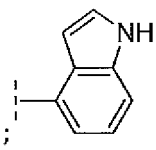 .

$R_2$ étant éventuellement marqué et choisi parmi

- H;
- $(CH_2CH_2O)_n$-$CH_2CH_2F$, n étant un entier égal à 0, 1 ou 2 ;
- $SO_2Ph$ ;
- COO*t*Bu ;
- $CH_2OCH_3$ ;
- $CH_2O(CH_2)_2OCH_3$
- $COCH_3$ ;
- 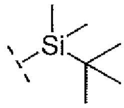 ;

$R_3$ étant éventuellement marqué et choisi parmi

- 6-Cl ;
- 6-F ;
- 5-F ;
- 4-F ;
- 6-(2-thiényle) ;
- 6-(3-thiényle) ;
- 6-(6-fluoro-3-pyridyle) ;
- 5-OMe ;
- 6-morpholino ;
- 5-morpholino ;
- 6-Br ;
- 4-Br ;
- 4-Bpin ;
- 5-Bpin.

Les composés de formule II-1a-1 comprennent un radioélément choisi parmi $^{18}F$, $^{11}C$, $^{123}I$ et $^{124}I$ sur au moins l'un des substituants $R_1$, $R_2$ ou $R_3$.

Selon un mode de réalisation avantageux, le composé de l'Invention est de formule générale I-1a-2 ou II-1a-2:

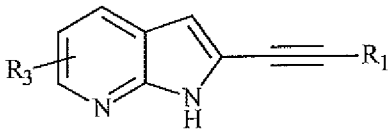

(I-1a-2) ou (II-1a-2)

$R_3$ étant tel que défini précédemment dans la formule I ou II respectivement;
$R_1$ étant éventuellement marqué et choisi parmi

- 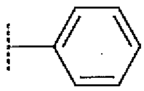 ; 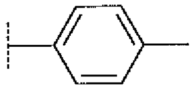 ; 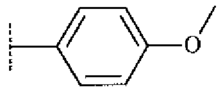 ; 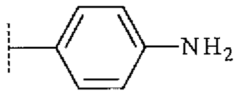

ou

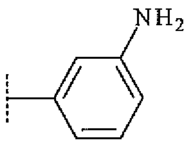 ; 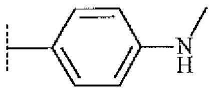 ; 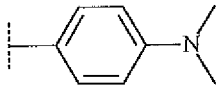 ou 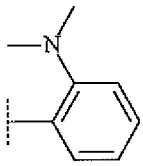

ou

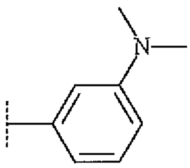 ; 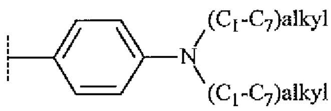 ou 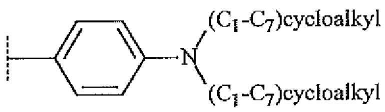 ,

notamment

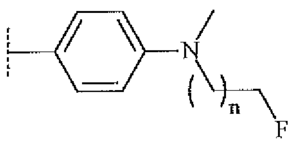 ,

n étant compris de 0 à 6 ;

- 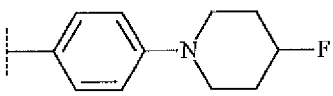 ; 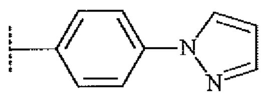 ; 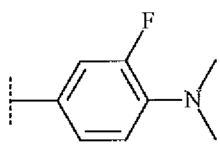

ou

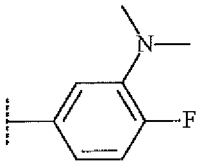 ; 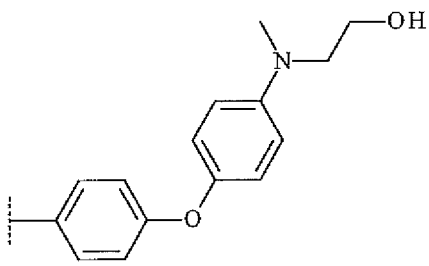 ; 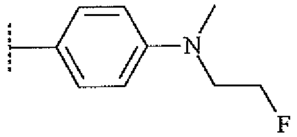 ;

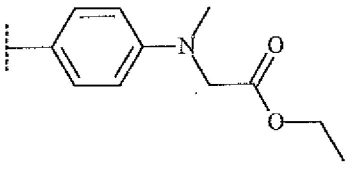 ; 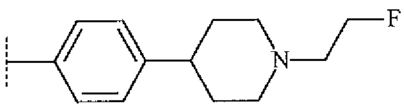 ;

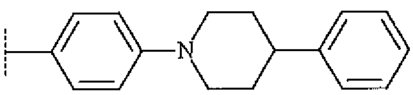 ; 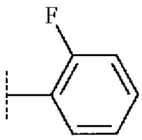 ; 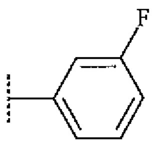 ; 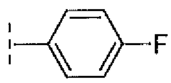 ;

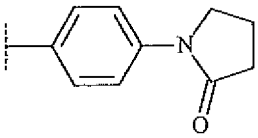 ; 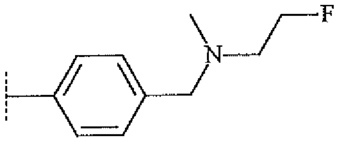 ; 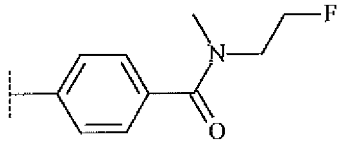 ;

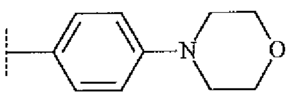 ; 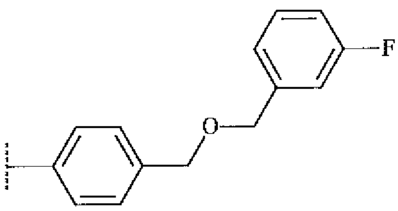 ;

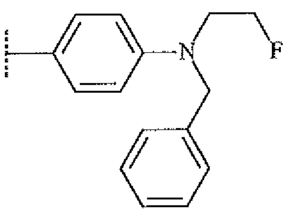 ; 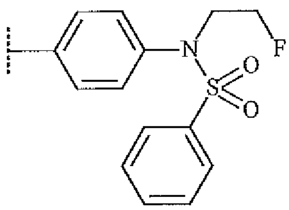 ; 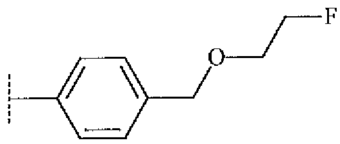 ;

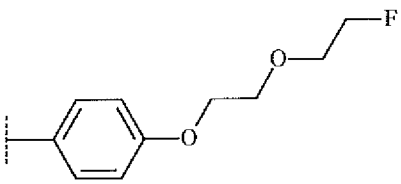 ; 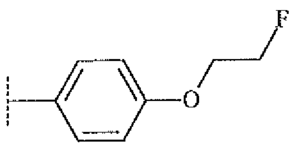 ;

- 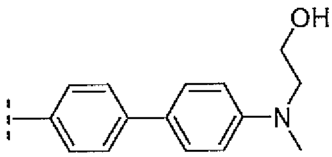 ; 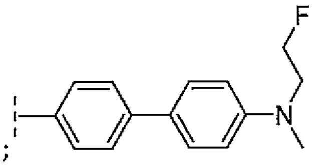 ; 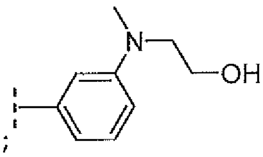 ;

- 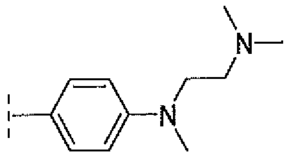 ; 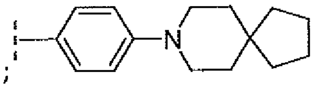 ; 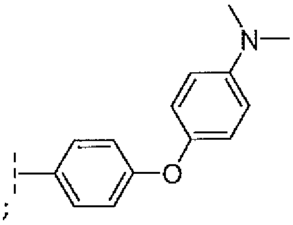 ;

- 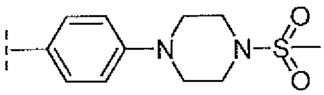 ; 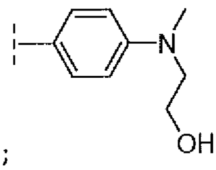 ; 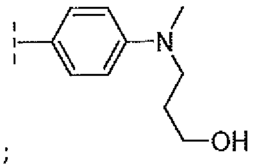 ;

- 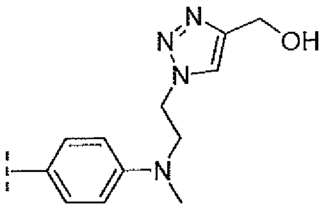 ; 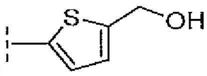 ; 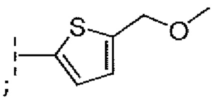 ; 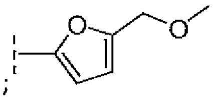 ;

- 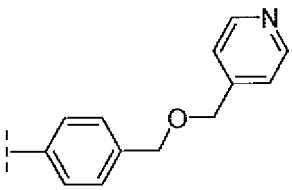 ; 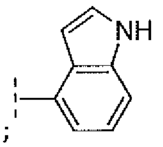 .

Les composés de formule II-1a-2 comprennent un radioélément choisi parmi $^{18}F$, $^{11}C$, $^{123}I$ et $^{124}I$ sur au moins l'un des substituants $R_1$ ou $R_3$.

Selon un mode de réalisation avantageux, le composé de l'Invention est de formule générale I-1a-2 ou II-1a-2 :

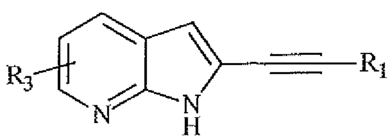

(I-1a-2) ou (II-1a-2)

$R_1$ étant tel que défini précédemment dans la formule I ou II respectivement ;
$R_3$ étant éventuellement marqué et choisi parmi

- 6-Cl ;
- 6-F ;
- 5-F ;
- 4-F ;
- 6-(2-thiényle) ;

- 6-(3-thiényle) ;
- 6-(6-fluoro-3-pyridyle) ;
- 5-OMe ;
- 6-morpholino ;
- 5-morpholino ;
- 6-Br ;
- 4-Br ;
- 4-Bpin ;
- 5-Bpin.

Les composés de formule II-1a-2 comprennent un radioélément choisi parmi $^{18}F$, $^{11}C$, $^{123}I$ et $^{124}I$ sur au moins l'un des substituants $R_1$ ou $R_3$.

Selon un mode de réalisation avantageux, le composé de l'Invention est de formule générale I-1a-2 ou II-1a-2 :

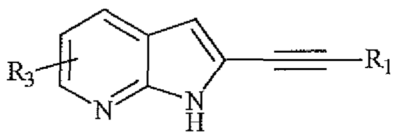

(I-1a-2) ou (II-1a-2)

$R_3$ étant éventuellement marqué et choisi parmi

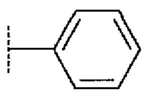 ; 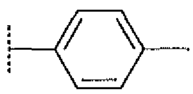 ; 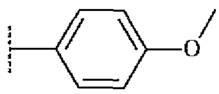 ; 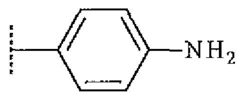

ou

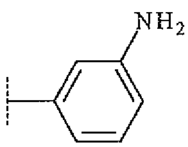 ; 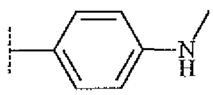 ; 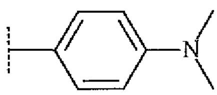 ou 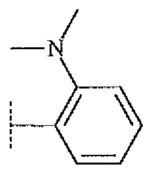

ou

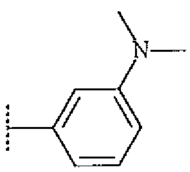 ; 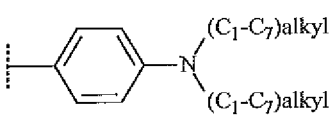 ou 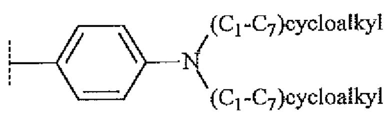 ,

notamment

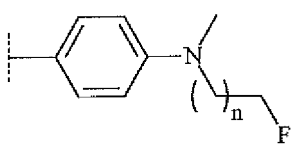 ,

n étant compris de 0 à 6 ;

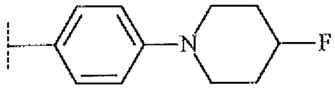 ; 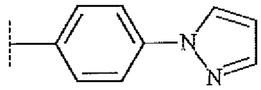 ; 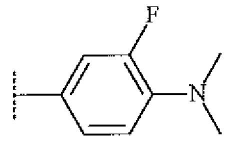

ou

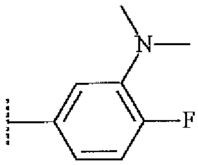 ; 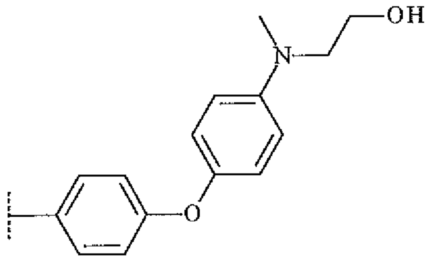 ; 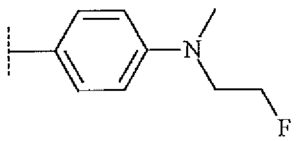 ;

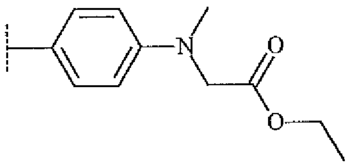 ; 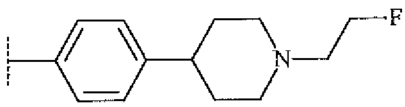 ;

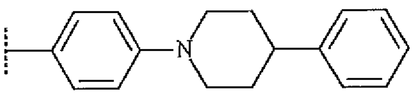 ; 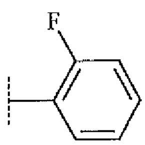 ; 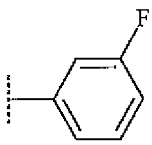 ; 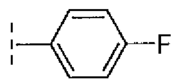 ;

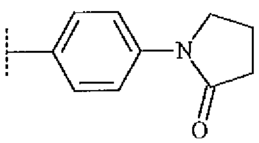 ; 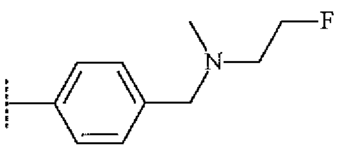 ; 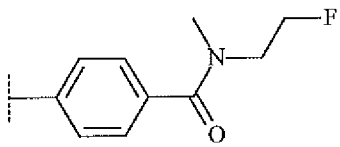 ;

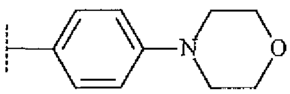 ; 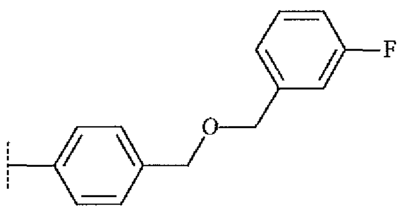 ;

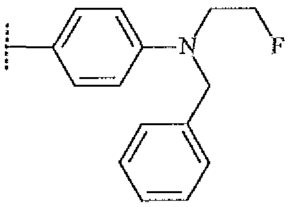 ; 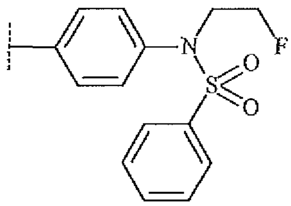 ; 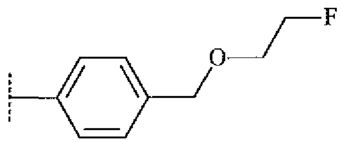 ;

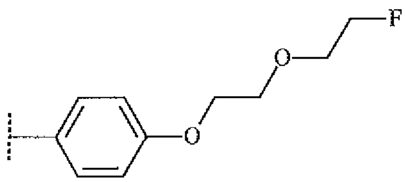 ; 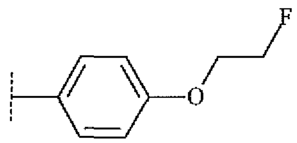 ;

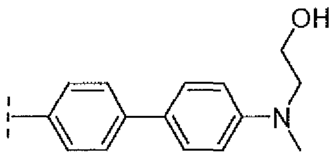 ; 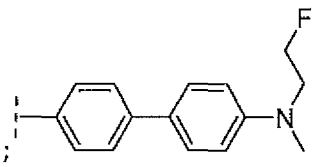 ; 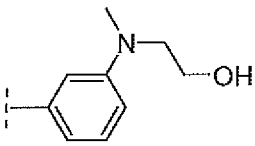 ;

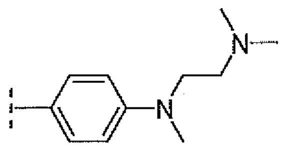 ; 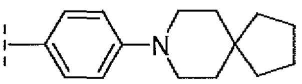 ; 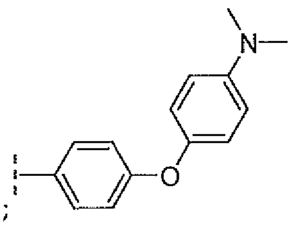 ;

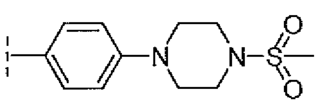 ; 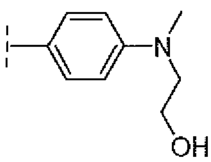 ; 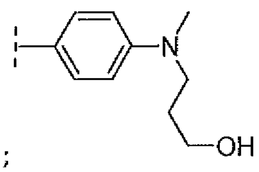 ;

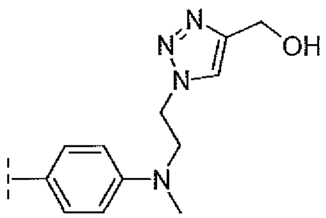 ; 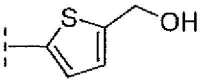 ; 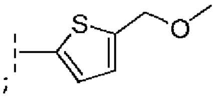 ; 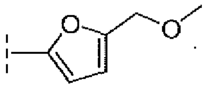 ;

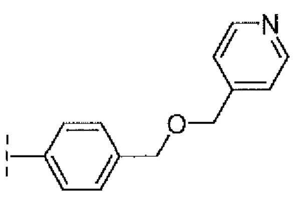 ; 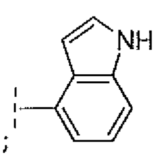 .

$R_3$ étant éventuellement marqué et choisi parmi

- 6-Cl ;
- 6-F ;
- 5-F ;
- 4-F ;
- 6-(2-thiényle) ;
- 6-(3-thiényle) ;
- 6-(6-fluoro-3-pyridyle) ;
- 5-OMe ;
- 6-morpholino ;
- 5-morpholino ;
- 6-Br ;
- 4-Br ;
- 4-Bpin ;
- 5-Bpin.

Les composés de formule II-1a-2 comprennent un radioélément choisi parmi $^{18}F$, $^{11}C$, $^{123}I$ et $^{124}I$ sur au moins l'un des substituants $R_1$ ou $R_3$.

Selon un mode de réalisation avantageux, le composé de l'Invention est de formule générale I-1a-2-A ou II-1a-2-A :

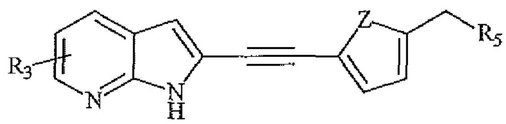

(I-1a-2-A) ou (II-1a-2-A)

$R_3$ étant tel que défini précédemment dans la formule I ou II respectivement ;
Z étant un O ou un S ;
$R_5$ étant éventuellement marqué et choisi parmi

- $NR^aR^b$, $R^a$ et $R^b$ étant tels que définis précédemment dans la formule I ou II respectivement ;
- $OR^e$, $R^e$ étant tel que défini précédemment dans la formule I ou II respectivement.

Les composés de formule II-1a-2-A comprennent un radioélément choisi parmi $^{18}F$, $^{11}C$, $^{123}I$ et $^{124}I$ sur au moins l'un des substituants $R_5$ ou $R_3$.

Dans le cadre des formules (I-1a-2-A) et (II-1a-2-A), le groupe $R_1$ des formules I et II respectivement est un hétéroaryle substitué par un alkyle, ledit alkyle étant lui-même substitué par une amine ou un alkoxyle. En particulier, dans le cas présent, l'hétéroaryle est choisi parmi un thiophènyle ou un furanyle, substitué par un alkyle en $C_1$, lui-même substitué par une amine ou un alkoxyle.

Selon un autre mode de réalisation avantageux, le composé de l'Invention est de formule générale I-1a-2-A ou II-1a-2-A :

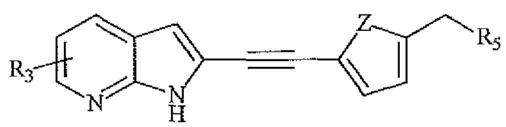

(I-1a-2-A) ou (II-1a-2-A)

$R_3$ étant tel que défini précédemment dans la formule I ou II respectivement ;
Z étant un O ;
$R_5$ étant éventuellement marqué et choisi parmi

- $NR^aR^b$ ;
- $OR^e$.

Les composés de formule II-1a-2-A comprennent un radioélément choisi parmi $^{18}F$, $^{11}C$, $^{123}I$ et $^{124}I$ sur au moins l'un des substituants $R_3$ ou $R_3$.

Selon un autre mode de réalisation avantageux, le composé de l'Invention est de formule générale I-1a-2-A

ou II-1a-2-A :

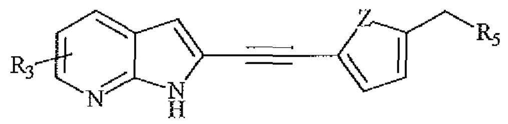

(I-1a-2-A) ou (II-1a-2-A)

$R_3$ étant tel que défini précédemment dans la formule I ou II respectivement ;
Z étant un S ;
$R_5$ étant éventuellement marqué et choisi parmi

- $NR^aR^b$ ;
- $OR^e$.

Les composés de formule II-1a-2-A comprennent un radioélément choisi parmi $^{18}F$, $^{11}C$, $^{123}I$ et $^{124}I$ sur au moins l'un des substituants $R_5$ ou $R_3$.

Selon un mode de réalisation plus avantageux, le composé de l'Invention est de formule générale I-1a-2-A ou II-1a-2-A :

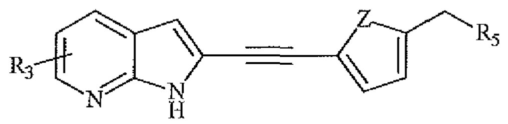

(I-1a-2-A) ou (II-1a-2-A)

$R_3$ étant tel que défini précédemment dans la formule I ou II respectivement ;
Z étant un O ou un S ;
$R_5$ étant éventuellement marqué et choisi parmi

- $N(CH_3)_2$ ;
- 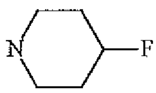 ;
- OH
- $OCH_3$.

Les composés de formule II-1a-2-A comprennent un radioélément choisi parmi $^{18}F$, $^{11}C$, $^{123}I$ et $^{124}I$ sur au moins l'un des substituants $R_5$ ou $R_3$.

Au sens de l'invention, le groupe

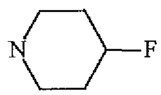

est une amine $NR^aR^b$dans laquelle $R^a$ et $R^b$ forment ensemble un $(C_3-C_7)$hétérocyclyle, ledit $(C_3-C_7)$hétérocyclyle étant dans le cas présent un $C_5$-hétérocyclyle substitué par un halogène, le fluor.

Selon un autre mode de réalisation plus avantageux, le composé de l'Invention est de formule générale I-1a-2-A ou II-1a-2-A :

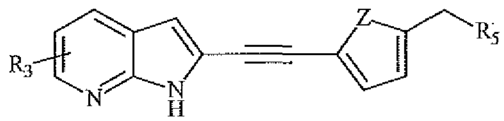

(I-1a-2-A) ou (II-1a-2-A)

$R_3$ étant tel que défini précédemment dans la formule I ou II respectivement ;
Z étant un O ;
$R_5$ étant éventuellement marqué et choisi parmi

- $N(CH_3)_2$ ;
- 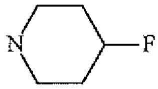 ;
- OH
- $OCH_3$.

Les composés de formule II-1a-2-A comprennent un radioélément choisi parmi $^{18}F$, $^{11}C$, $^{123}I$ et $^{124}I$ sur au moins l'un des substituants $R_5$ ou $R_3$.

Selon un autre mode de réalisation plus avantageux, le composé de l'Invention est de formule générale I-1a-2-A ou II-1a-2-A :

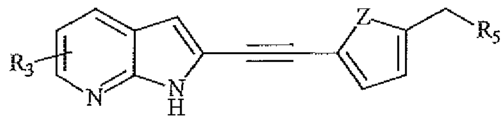

(I-1a-2-A) ou (II-1a-2-A)

$R_3$ étant tel que défini précédemment dans la formule I ou II respectivement ;
Z étant un S ;
$R_5$ étant éventuellement marqué et choisi parmi

- $N(CH_3)_2$ ;

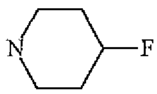 ;

- OH
- $OCH_3$.

Les composés de formule II-1a-2-A comprennent un radioélément choisi parmi $^{18}F$, $^{11}C$, $^{123}I$ et $^{124}I$ sur au moins l'un des substituants $R_5$ ou $R_3$.

Selon un mode de réalisation particulièrement avantageux, le composé de l'Invention de formule I est choisi parmi :

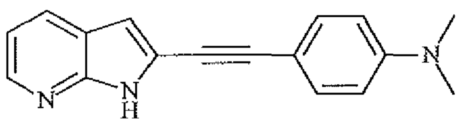

**22a**

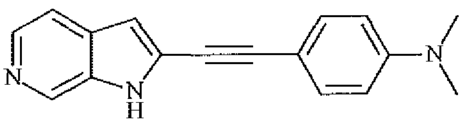

**22b**

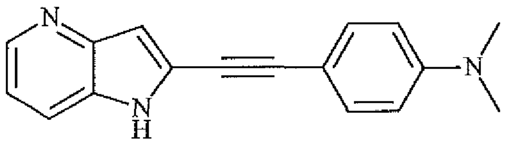

22c

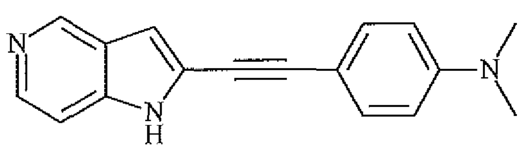

22d

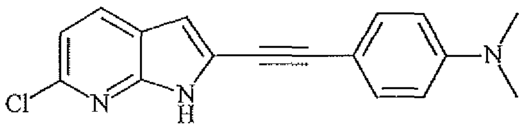

22e

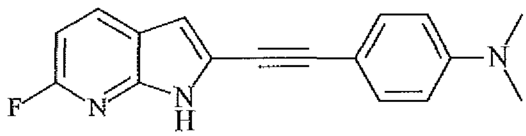

22f

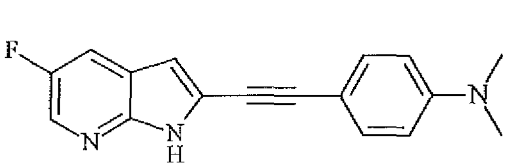

22g

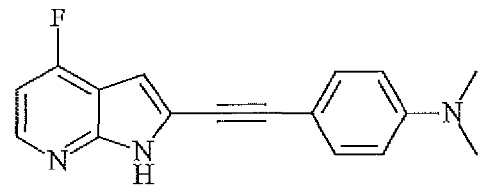

22h

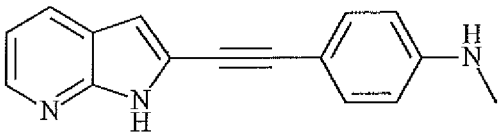

22i

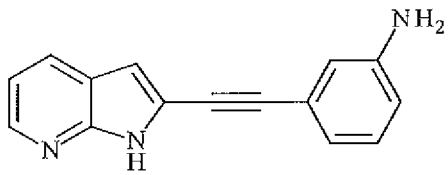

22j

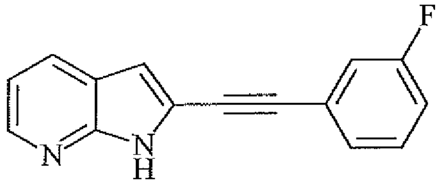

22k

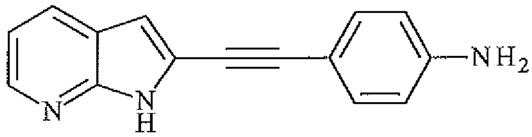

22L

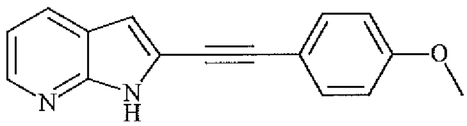

22m

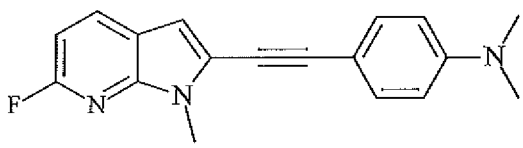

22n

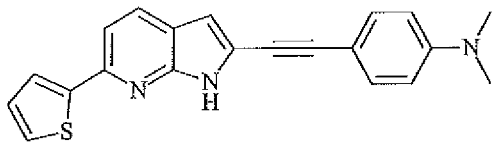

25a

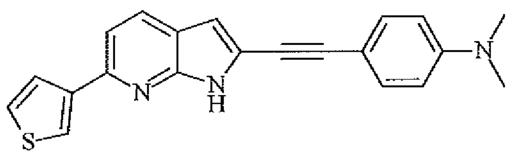

25b

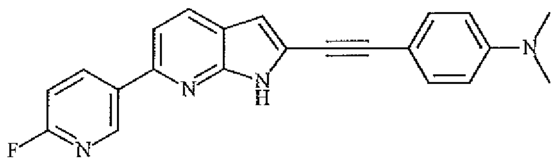

25c

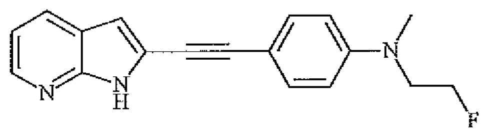

28

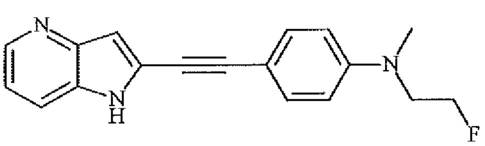 35

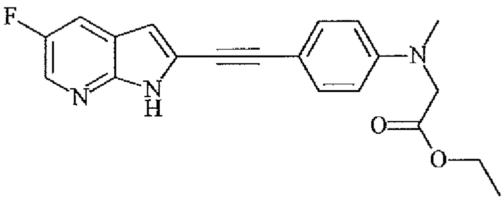 37

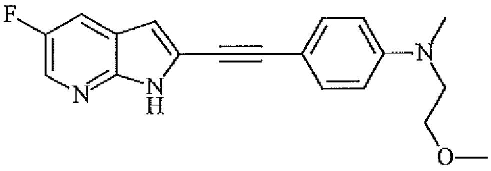 38

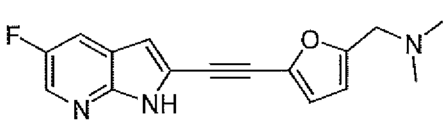 131

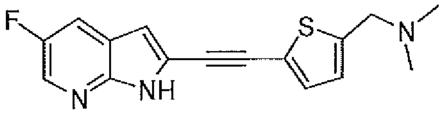 133

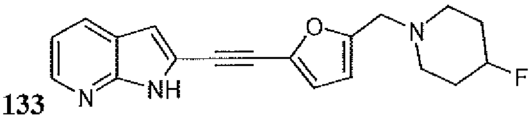 128

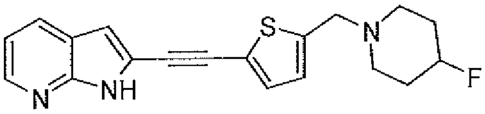 124

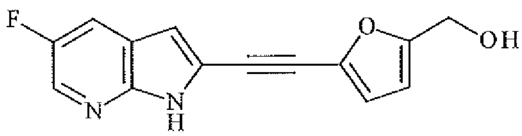 120

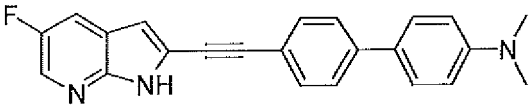 116

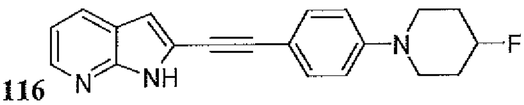 113

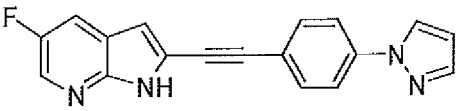 108

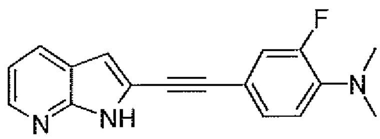 103

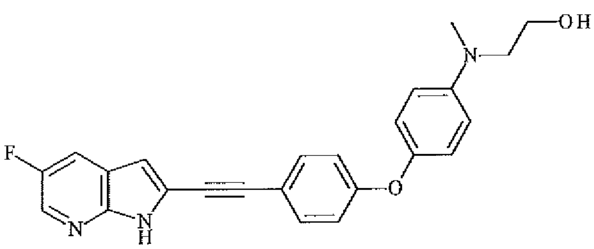 97

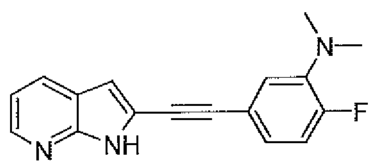 93

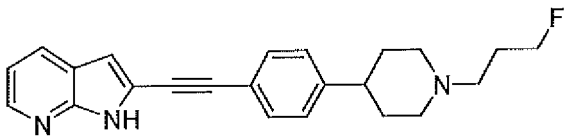 85

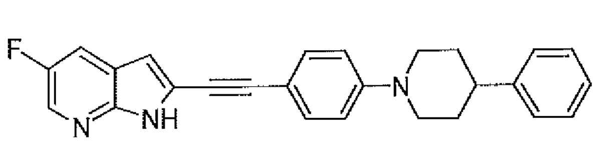
90

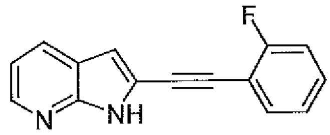
84

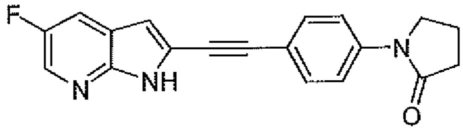
79

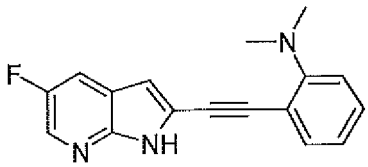
76

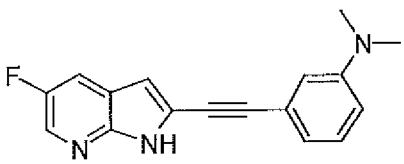
74

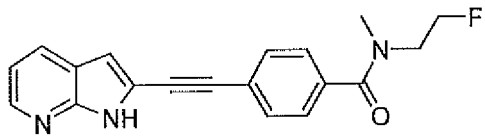
71

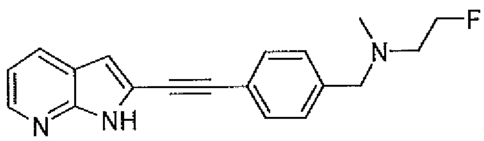
67

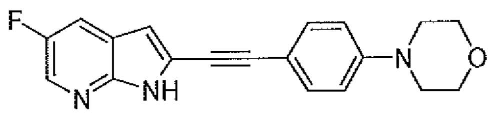
64

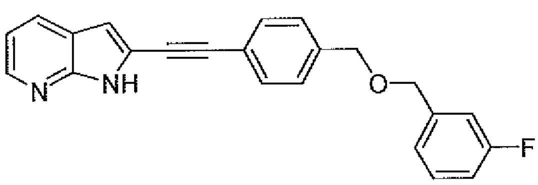
61

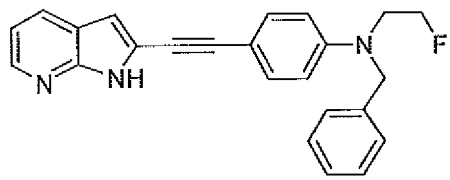
52

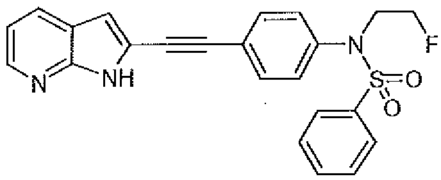
57

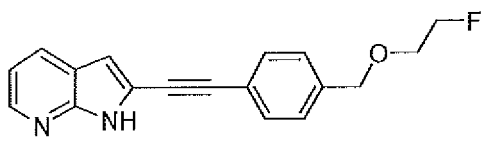
44

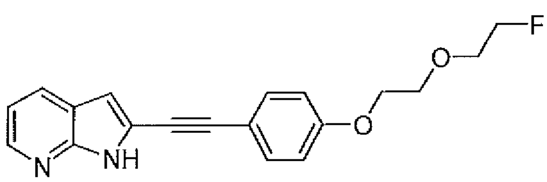
48

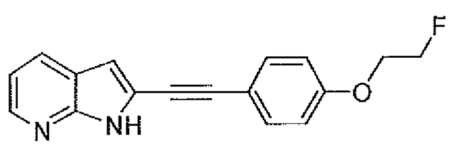
43

Selon un autre mode de réalisation particulièrement avantageux, le composé de l'Invention de formule I est choisi parmi :

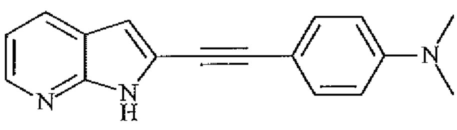
22a

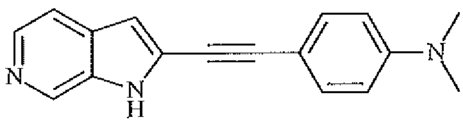
22b

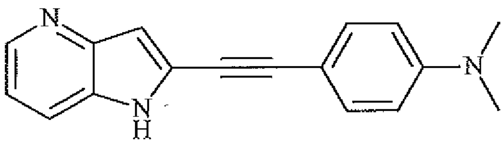
22c

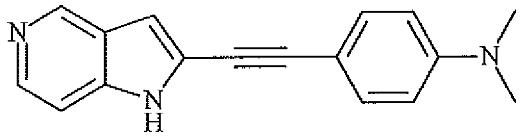
22d

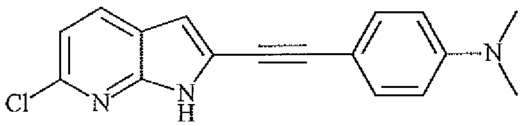
22e

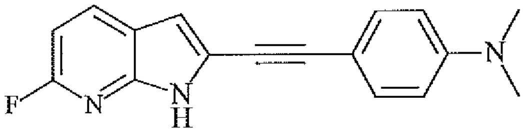
22f

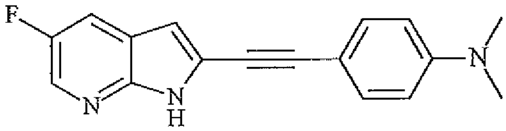
22g

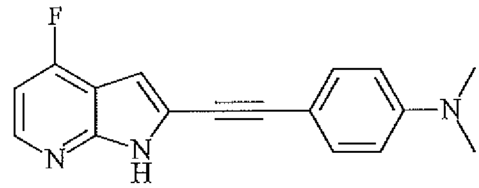
22h

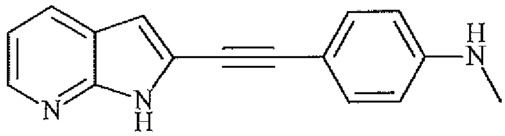
22i

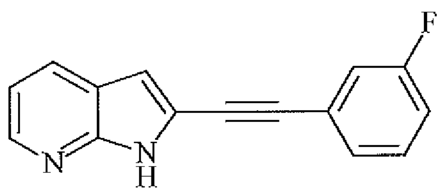
22k

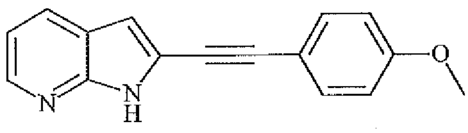
22m

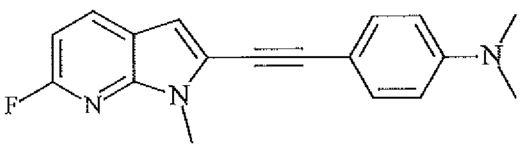
22n

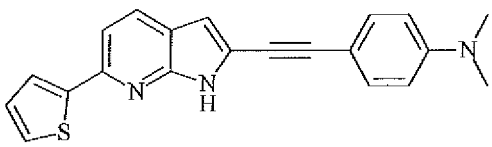
25a

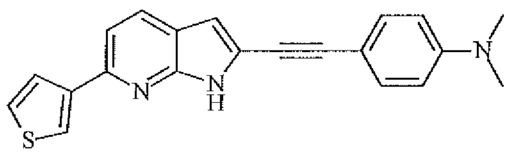
25b

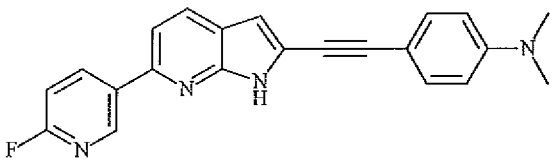
25c

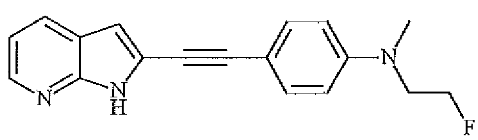
28

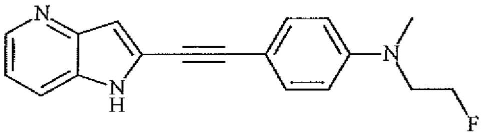
35

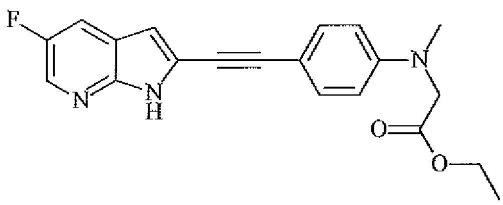
37

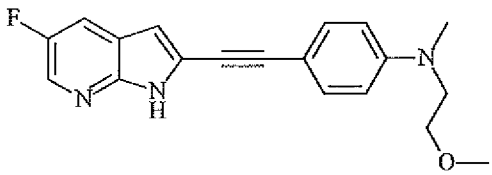
38

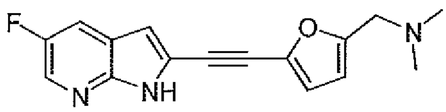
131

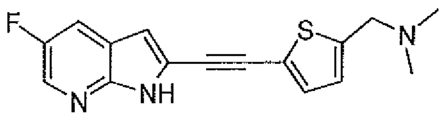
133

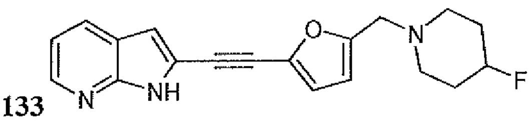
128

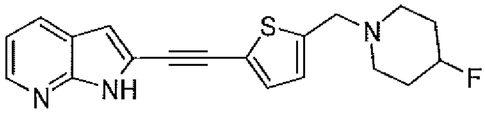
124

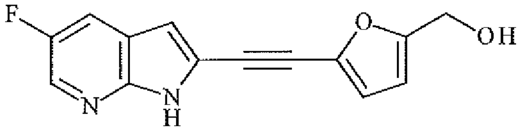
120

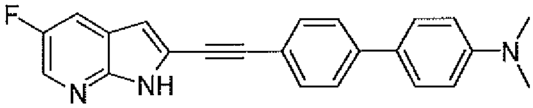
116

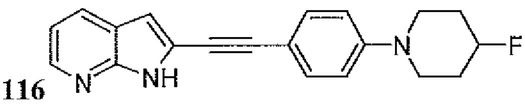
113

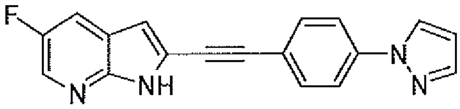
108

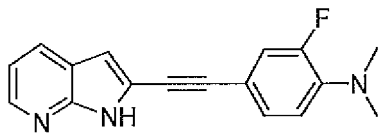
103

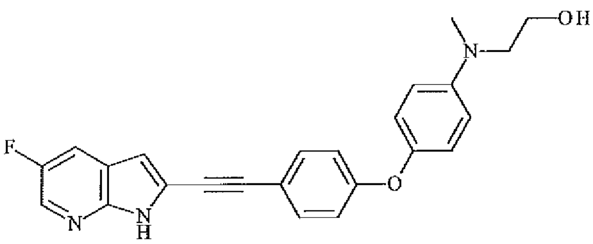
97

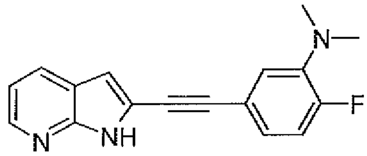
93

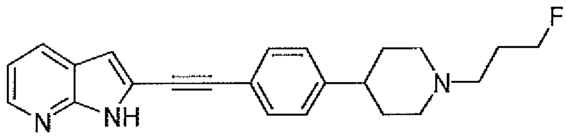
85

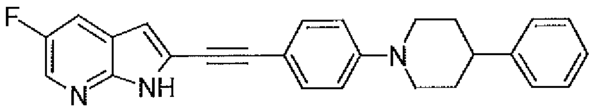
90

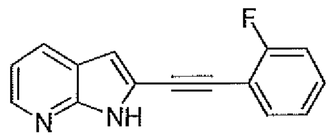
84

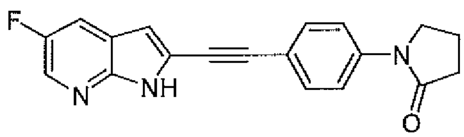
79

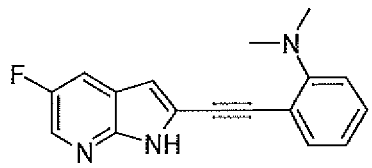
76

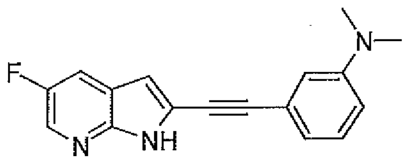
74

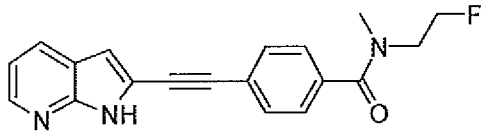
71

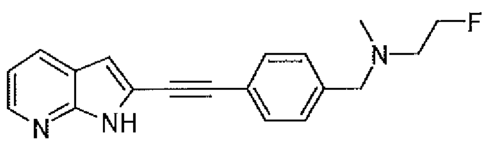
67

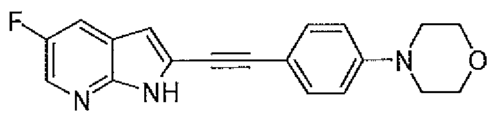
64

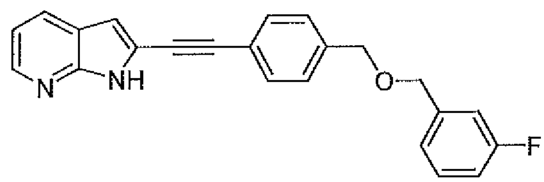
61

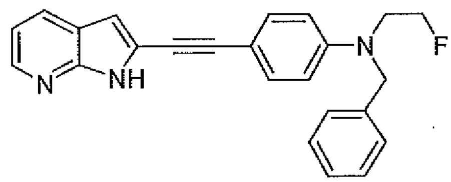
52

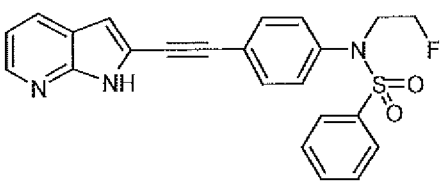
57

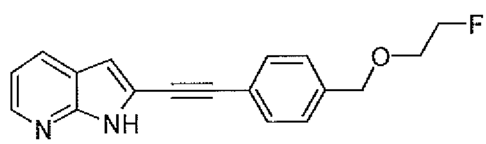
44

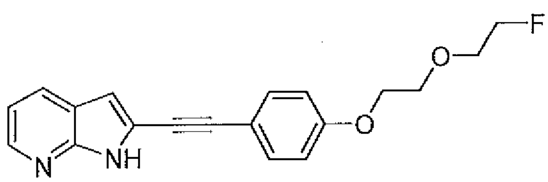
48

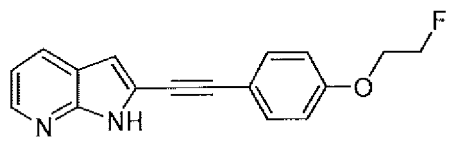
43

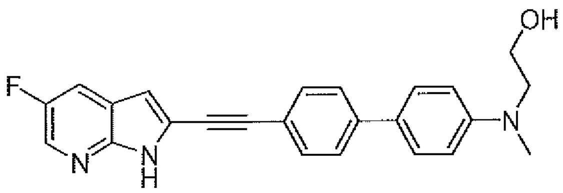

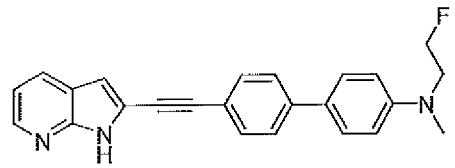

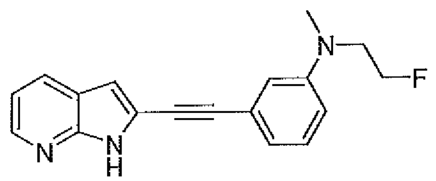

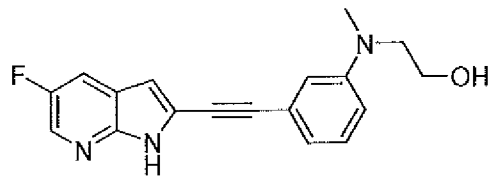

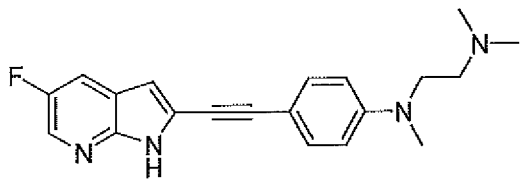

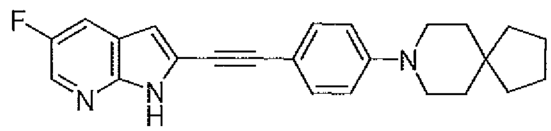

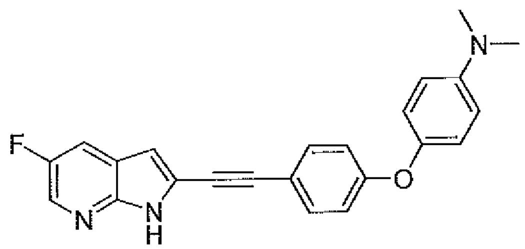

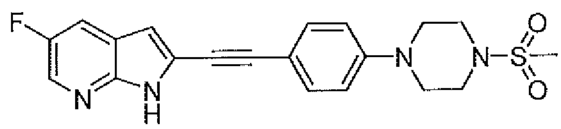

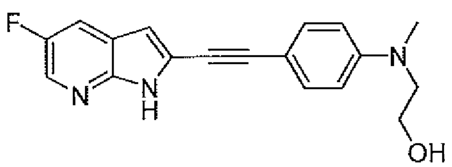

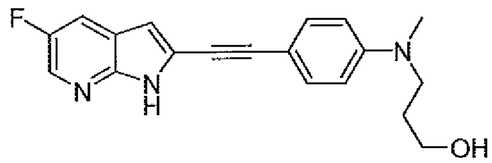

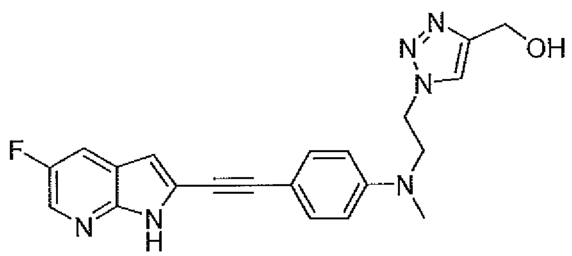

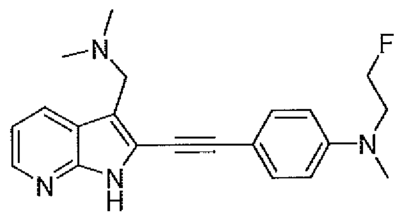

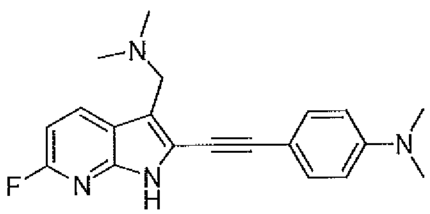

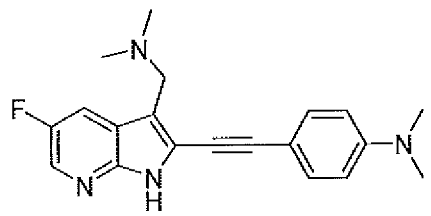

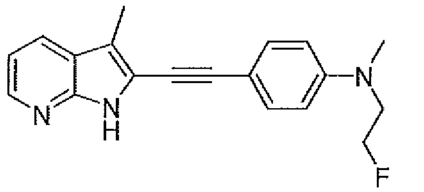

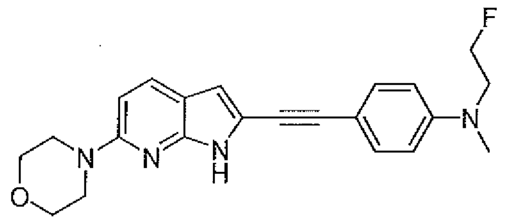

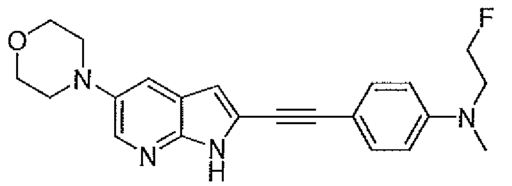

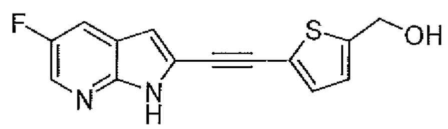

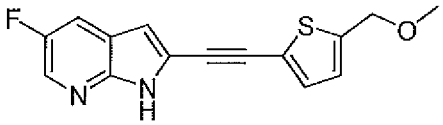

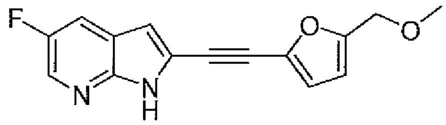

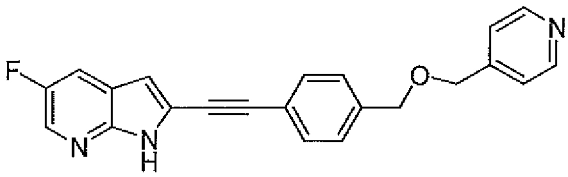

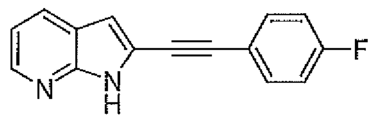

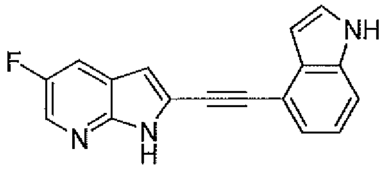

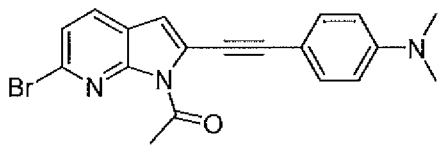

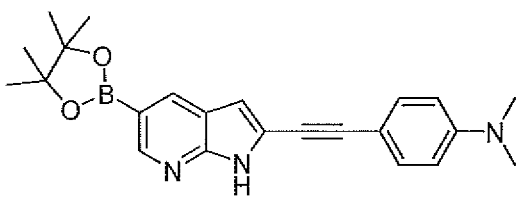

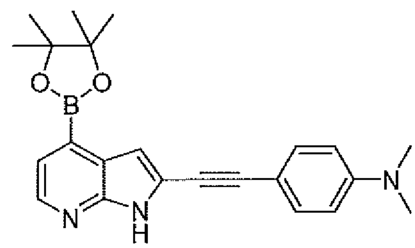

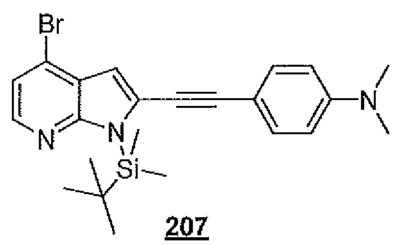

**207**

Selon un mode de réalisation particulièrement avantageux, le composé de l'Invention de formule II est:

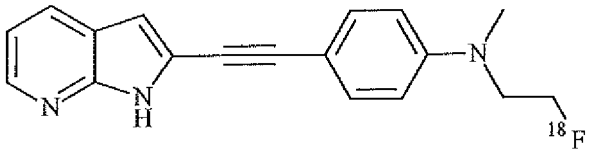

[$^{18}$F]-28

Selon un mode de réalisation, le composé de l'Invention est choisi parmi

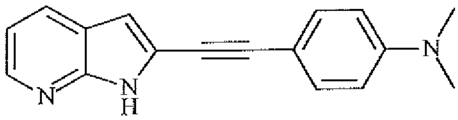

22a

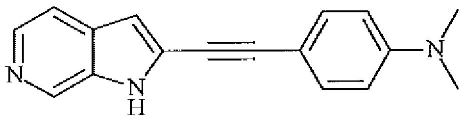

22b

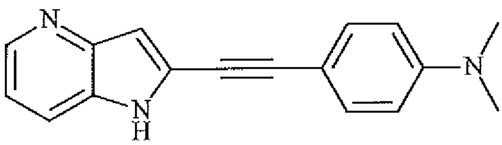

22c

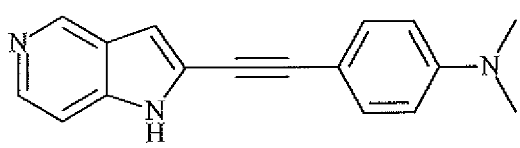

22d

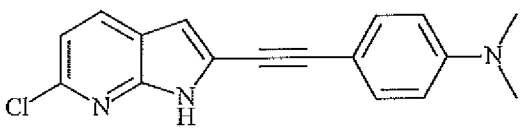

22e

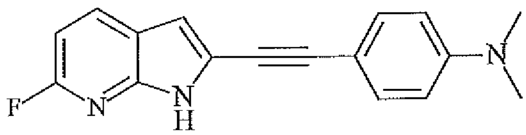

22f

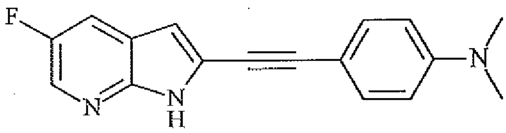

22g

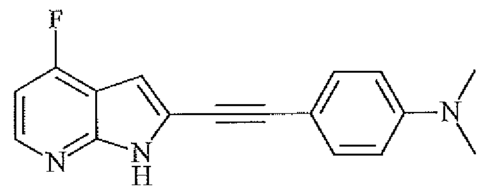

22h

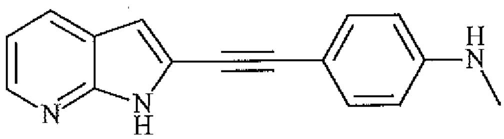

22i

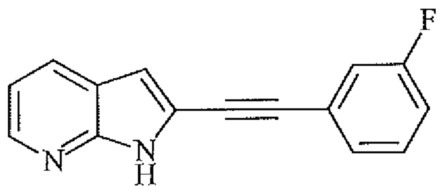

22k

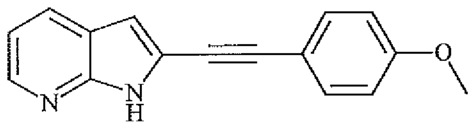

22m

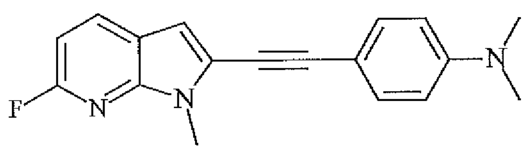

22n

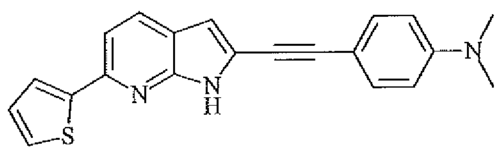

25a

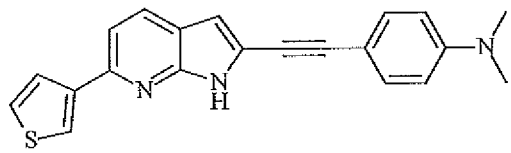

25b

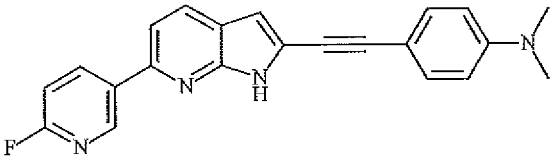
25c

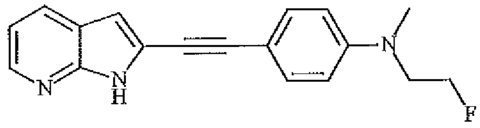
28

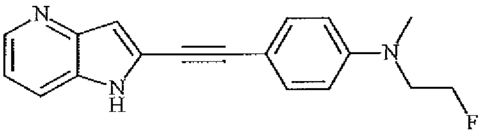
35

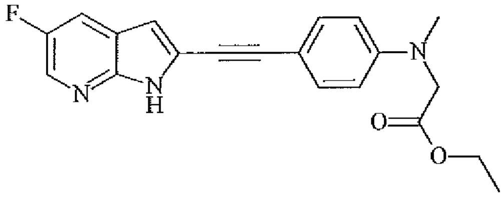
37

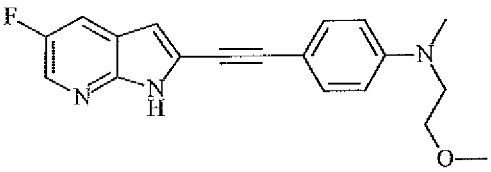
38

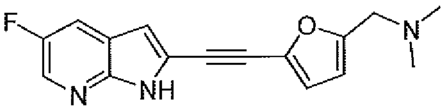
131

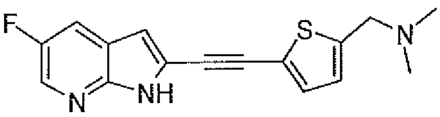
133

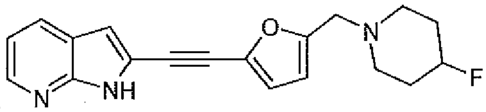
128

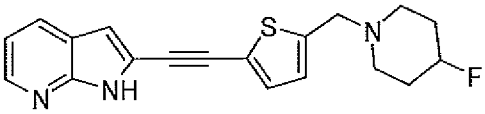
124

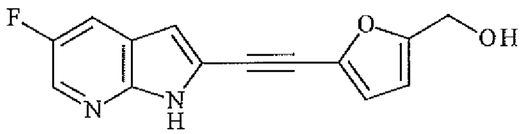
120

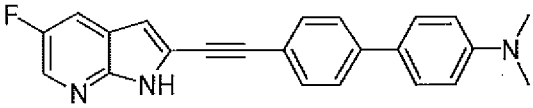
116

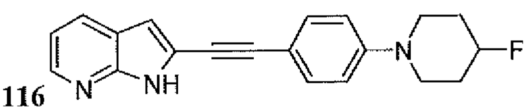
113

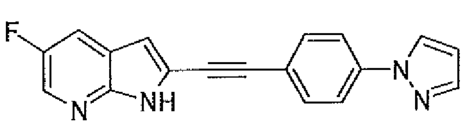
108

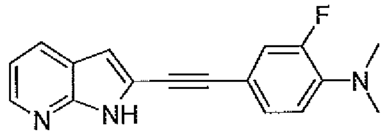
103

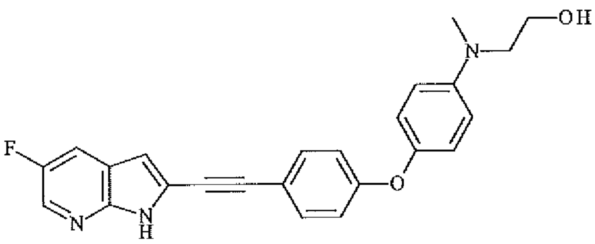

97

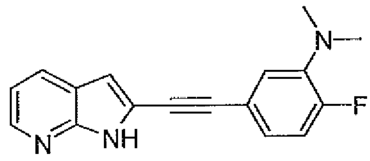

93

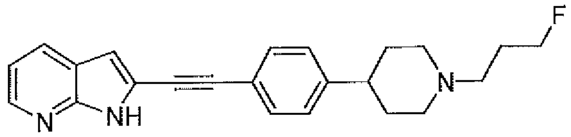

85

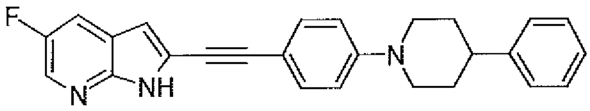

90

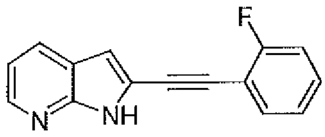

84

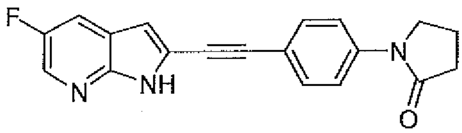

79

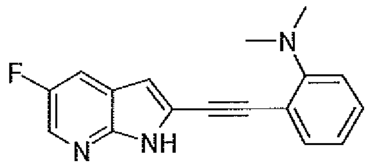

76

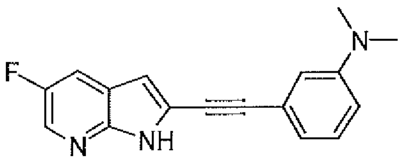

74

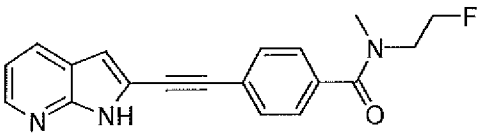

71

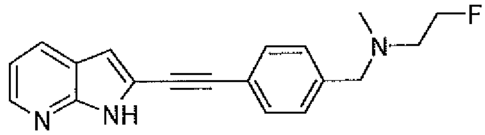

67

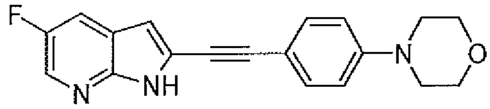

64

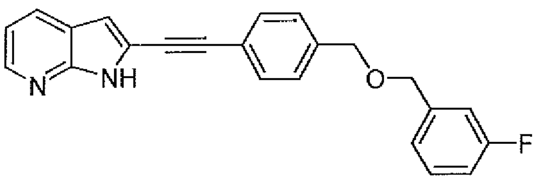

61

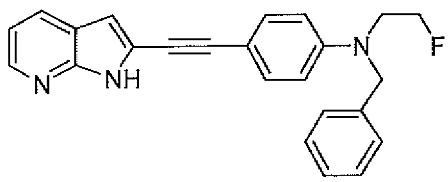

52

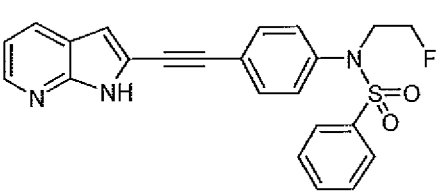

57

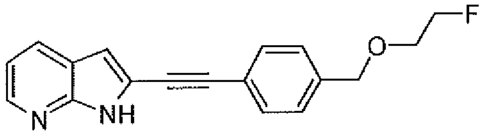

44

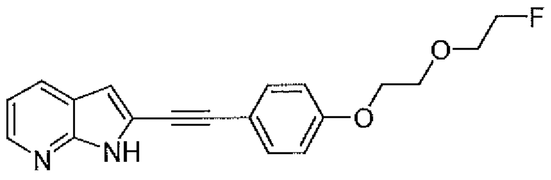 48 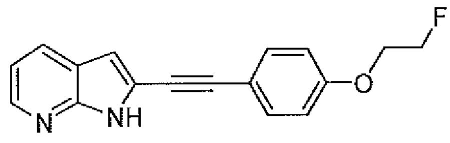 43

à l'état radiomarqué ;
notamment, ledit composé de formule II étant :

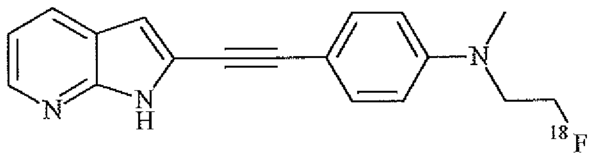

$[^{18}F]$-28

Selon un autre mode de réalisation avantageux, le composé de l'Invention est de formule générale I-2 :

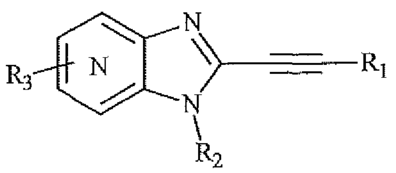

(I-2)

$R_1$, $R_2$ et $R_3$ étant tels que définis précédemment dans la formule I.

Selon un mode de réalisation avantageux, le composé de l'Invention est de formule générale II-2 :

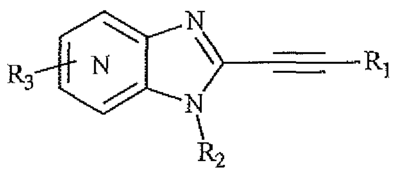

(II-2)

$R_1$, $R_2$ et $R_3$ étant tels que définis précédemment dans la formule II ;
dans lequel au moins l'un des substituants $R_1$, $R_2$ ou $R_3$ comprend un radioélément choisi parmi $^{18}F$, $^{11}C$, $^{123}I$ et $^{124}I$.

Selon un mode de réalisation avantageux, le composé de l'Invention est de formule générale III-2 :

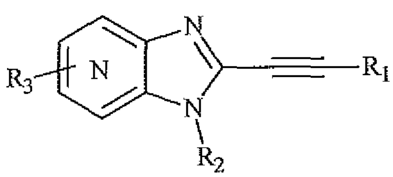

(III-2)

$R_1$, $R_2$ et $R_3$ étant tels que définis précédemment dans la formule III ;
dans lequel les substituants $R_1$, $R_2$ et $R_3$ ne comprennent pas de radioélément.

Selon un mode de réalisation avantageux, le composé de l'Invention est de formule générale I-2a ou II-2a :

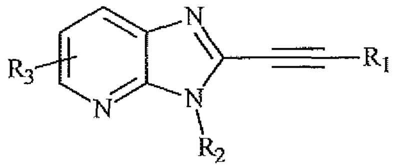

(I-2a) ou (II-2a)

$R_1$, $R_2$ et $R_3$ étant tels que définis précédemment dans la formule I ou II respectivement.

Les composés de formule II-2a comprennent un radioélément choisi parmi $^{18}F$, $^{11}C$, $^{123}I$ et $^{124}I$ sur au moins l'un des substituants $R_1$, $R_2$ ou $R_3$.

Selon un mode de réalisation avantageux, le composé de l'Invention est de formule générale I-2a ou II-2a :

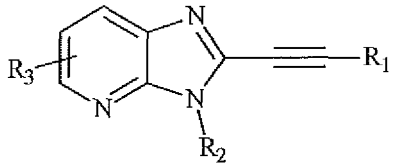

(I-2a) ou (II-2a)

$R_2$ et $R_3$ étant tels que définis précédemment dans la formule I ou II respectivement ;
$R_1$ étant éventuellement marqué et choisi parmi

- phényle ;
- *p*-$N(CH_3)_2$-phényle ;
- *p*-$N(CH_3)(CH_2CH_2F)$-phényle.

Les composés de formule II-2a comprennent un radioélément choisi parmi $^{18}F$, $^{11}C$, $^{123}I$ et $^{124}I$ sur au moins l'un des substituants $R_1$, $R_2$ ou $R_3$.

Selon un mode de réalisation avantageux, le composé de l'Invention est de formule générale I-2a ou II-2a:

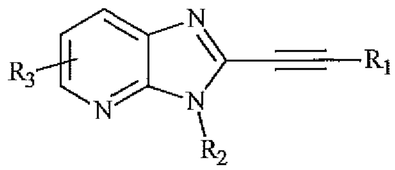

(I-2a) ou (II-2a)

$R_1$ et $R_3$ étant tels que définis précédemment dans la formule I ou II respectivement ;
$R_2$ étant éventuellement marqué et choisi parmi

- H;
- $(CH_2CH_2O)_n$-$CH_2CH_2F$, n étant un entier égal à 0, 1 ou 2 ;
- $SO_2Ph$ ;
- COO*t*Bu ;
- $CH_2OCH_3$ ;
- $CH_2O(CH_2)_2OCH_3$
- $COCH_3$ ;
- 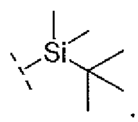.

Les composés de formule H-2a comprennent un radioélément choisi parmi $^{18}F$, $^{11}C$, $^{123}I$ et $^{124}I$ sur au moins l'un des substituants $R_1$, $R_2$ ou $R_3$.

Selon un mode de réalisation avantageux, le composé de l'Invention est de formule générale I-2a ou II-2a :

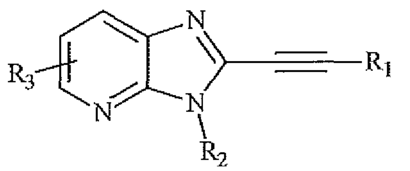

(I-2a) ou (II-2a)

$R_3$ étant tel que défini précédemment dans la formule I ou II respectivement ;
$R_1$ étant éventuellement marqué et choisi parmi

- phényle ;
- *p*-$N(CH_3)_2$-phényle ;
- *p*-$N(CH_3)(CH_2CH_2F)$-phényle ;

$R_2$ étant éventuellement marqué et choisi parmi

- H;
- $(CH_2CH_2O)_n$-$CH_2CH_2F$, n étant un entier égal à 0, 1 ou 2 ;
- $SO_2Ph$ ;
- COO'Bu ;
- $CH_2OCH_3$ ;
- $CH_2O(CH_2)_2OCH_3$
- $COCH_3$ ;

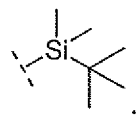

Les composés de formule II-2a comprennent un radioélément choisi parmi $^{18}F$, $^{11}C$, $^{123}I$ et $^{124}I$ sur au moins l'un des substituants $R_1$, $R_2$ ou $R_3$.

L'Invention concerne également un composé de formule III pour la préparation de composés de formule II, IIbis, IIa, IIb, II-1, II-2, II-1a, II-1b, II-1c, II-1d, II-1a-1, II-1a-2, II-1a-2-A, ou II-2a.

L'Invention concerne également un composé, tel que défini précédemment dans les formules II, IIbis, IIa, IIb, II-1, II-2, II-1a, II-1b, II-1c, II-1d, II-1a-1, II-1a-2, II-1a-2-A, ou II-2a, dans lequel au moins l'un des substituants $R_1$, $R_2$, $R_3$ ou $R_4$ comprend un radioélément choisi parmi $^{18}F$, $^{11}C$, $^{123}I$ et $^{124}I$, pour son utilisation dans un procédé de diagnostic ou d'imagerie in vivo d'un sujet atteint d'une maladie neuro-dégénérative, dans lequel ledit procédé de diagnostic ou d'imagerie comprend l'administration dudit composé.

Selon un mode de réalisation, l'invention concerne le composé de formule II, IIbis, IIa, IIb, II-1, II-2, II-1a, II-1b, II-1c, II-1d, II-1a-1, II-1a-2, II-1a-2-A, ou II-2a pour son utilisation dans un procédé de diagnostic ou d'imagerie in vivo d'un sujet atteint d'une maladie neuro-dégénérative, ladite maladie neuro-dégénérative étant une amyloïdopathie, une alpha-synucléinopathie ou une tauopathie.

Au sens de la présente Invention, l'expression « *maladie neuro-dégénérative* » se réfère à un groupe de pathologies progressives liées à un dysfonctionnement métabolique au sein du tissu nerveux, conduisant à la mort des neurones et à la destruction du système nerveux.

Par « *amyloïdopathie* » est entendu, au sens de l'Invention, une maladie neuro-dégénérative présentant une agrégation en fibres de peptides β-amyloïdes anormaux dans le cerveau.

Par « *alpha-synucléionopathie* » est entendu, au sens de l'Invention, une maladie neuro-dégénérative présentant une agrégation de protéine α-synucléine en fibres dans le cerveau. En particulier, les alpha-synucléinopathies concernées par l'invention sont la maladie de Parkinson, la démence à corps de Lewy et l'atrophie multi-systématisée.

Par « *tauopathie* » est entendu, au sens de l'Invention, une maladie neuro-dégénérative présentant une agrégation en fibres de protéines tau anormales dans le cerveau.

Les composés concernés présentent une affinité pour au moins deux des protéines β-amyloïde, α-synucléine et tau fibrillaires mais ne sont pas sélectifs vis-à-vis de l'une d'elles.

Le terme « *affinité* », au sens de l'Invention, se rapporte à la capacité d'un composé de l'invention à se lier aux protéines β-amyloïde, α-synucléine et tau fibrillaires. L'affinité est déterminée indirectement par la valeur de la constante d'inibition

$K_i$ obtenue par mesures de compétition avec d'autres ligands, notamment la thioflavine T, ou directement, et plus précisément, par la mesure de $K_d$. Au sens de l'Invention, un composé est considéré avoir une bonne affinité pour la protéine fibrillaire considérée lorsque la valeur de $K_i$ est inférieure ou égale à 50 nM et/ou la valeur de $K_d$ est inférieure ou égale à 20 nM. Au-dessus d'un seuil de $K_d$ de 20 nM le composé est considéré comme ayant une faible affinité pour la protéine fibrillaire considérée. Autrement dit, l'affinité est alors insuffisante pour que le composé soit potentiellement utilisé comme radiotraceur sélectif de la cible protéique.

Un composé est dit « *sélectif* » pour l'une des protéines β-amyloïde, α-synucléine ou tau fibrillaires, au sens de l'Invention, dès lors qu'il présente une bonne affinité pour cette protéine et que son affinité pour cette protéine soit au moins deux fois plus forte que celle pour au moins l'une des deux autres protéines.

A titre d'exemple, selon un mode de réalisation, et selon les analyses de Ki issues du tableau 5,
des composés sélectifs d'a-syn sont **22a, 22b, 22e, 22f, 22g, 22h**
des composés sélectifs de Tau sont **22a, 22b, 22c, 22e.**
A titre d'exemple, selon un autre mode de réalisation, et selon les analyses de Kd issues des tableaux 6 et 7,
des composés sélectif de Tau sont **22a, 22b, 22c, 28**
des composés sélectifs d'a-syn sont **22a, 22b, 22d, 22f, 25a, 25b, 25c**
un composé sélectif de Aβ est **28.**

Selon un mode de réalisation, l'invention concerne le composé de formule II, IIbis, IIa, IIb, II-1, II-2, II-1a, II-1b, II-1c, II-1d, II-1a-1, II-1a-2, II-1a-2-A, ou II-2a pour son utilisation dans un procédé de diagnostic ou d'imagerie in vivo d'un sujet atteint d'une maladie neuro-dégénérative, ladite maladie neuro-dégénérative étant une amyloïdopathie.

Selon un mode de réalisation, l'invention concerne le composé de formule II, IIbis, IIa, IIb, II-1, II-2, II-1a, II-1b, II-1c, II-1d, II-1a-1, II-1a-2, II-1a-2-A, ou II-2a pour son utilisation dans un procédé de diagnostic ou d'imagerie in vivo d'un sujet atteint d'une maladie neuro-dégénérative, ladite maladie neuro-dégénérative étant une alpha-synucléinopathie.

Selon un mode de réalisation, l'invention concerne le composé de formule II, IIbis, IIa, IIb, II-1, II-2, II-1a, II-1b, II-1c, II-1d, II-1a-1, II-1a-2, II-1a-2-A, ou II-2a pour son utilisation dans un procédé de diagnostic ou d'imagerie in vivo d'un sujet atteint d'une maladie neuro-dégénérative, ladite maladie neuro-dégénérative étant une tauopathie.

Selon un mode de réalisation, l'invention concerne le composé de formule II, IIbis, IIa, IIb, II-1, II-2, II-1a, II-1b, II-1c, II-1d, II-1a-1, II-1a-2, II-1a-2-A, ou II-2a pour son utilisation dans un procédé de diagnostic ou d'imagerie in vivo d'un sujet atteint d'une maladie neuro-dégénérative, dans lequel le procédé d'imagerie in vivo est la tomographie par émission de positrons ou la tomographie d'emission monophotonique.

La tomographie par émission de positrons (TEP) et la tomographie d'emission monophotonique (TEMP) sont des techniques d'imagerie médicale non invasives utilisées pour le diagnostic de certaines maladies, le suivi de l'évolution de ces maladies et l'évaluation de l'efficacité des traitements. Les images sont obtenues par injection dans l'organisme d'un radiotraceur (molécule radioactive), de l'enregistrement son parcours dans l'organisme grâce à des caméras TEP ou TEMP et un traitement informatique des données.

L'exploration par imagerie de l'α-syn représente un progrès majeur par exemple dans le diagnostic de la maladie de Parkinson au cours de laquelle des agrégats de cette protéine apparaissent dans les stades précoces. Par ailleurs, ce type d'exploration permet d'évaluer les effets de traitements de la maladie de Parkinson visant à agir sur les agrégats d'α-syn.

L'Invention concerne également l'utilisation du composé de formule II, IIbis, IIa, IIb, II-1, II-2, II-1a, II-1b, II-1c, II-1d, II-1a-1, II-1a-2, II-1a-2-A, ou II-2a, dans lequel au moins l'un des substituants $R_1$, $R_2$, $R_3$ ou $R_4$ comprend un radioélément choisi parmi $^{18}F$, $^{11}C$, $^{123}I$ et $^{124}I$, comme marqueur pour le diagnostic in vivo d'une maladie neuro-dégénérative.

L'Invention concerne également l'utilisation du composé de formule II, IIbis, IIa, IIb, II-1, II-2, II-1a, II-1b, II-1c, II-1d, II-1a-1, II-1a-2, II-1a-2-A, ou II-2a, dans lequel au moins l'un des substituants $R_1$, $R_2$, $R_3$ ou $R_4$ comprend un radioélément choisi parmi $^{18}F$, $^{11}C$, $^{123}I$ et $^{124}I$, comme marqueur pour le diagnostic in vivo ou in vitro d'une maladie neuro-dégénérative.

L'invention concerne également l'utilisation du composé de formule II, IIbis, IIa, IIb, II-1, II-2, II-1a, II-1b, II-1c, II-1d, II-1a-1, II-1a-2, II-1a-2-A, ou II-2a, dans lequel au moins l'un des substituants $R_1$, $R_2$, $R_3$ ou $R_4$ comprend un radioélément choisi parmi $^{18}F$, $^{11}C$, $^{123}I$ et $^{124}I$, comme agent d'imagerie in vivo d'un sujet atteint d'une maladie neuro-dégénérative.

Selon un mode de réalisation avantageux, dans l'utilisation du composé de formule II, IIbis, IIa, IIb, II-1, II-2, II-1a, II-1b, II-1c, II-1d, II-1a-1, II-1a-2, II-1a-2-A, ou II-2a de l'Invention comme agent d'imagerie in vivo, l'imagerie in vivo est la tomographie par émission de positrons ou tomographie d'emission monophotonique.

Au sens de la présente invention, l'expression « *dérivés radiomarqués TEP* » signifie que le composé considéré comprend au moins un radioélément choisi parmi $^{18}F$, $^{11}C$, ou $^{124}I$, en substitution d'un élément non marqué. Par exemple, le composé **[$^{18}$F]-28** est un dérivé radiomarqué au $^{18}F$ du composé 28. Au sens de la présente invention, l'expression « *dérivés radiomarqués TEMP* » signifie que le composé considéré comprend au moins un radioélément

comme $^{123}I$ en substitution d'un élément non marqué.

L'Invention concerne également une composition pharmaceutique ou radiopharmaceutique qui comprend comme substance active le composé de formule I, II, III, Ibis, IIbis, Ia, Ib, IIa, IIb, I-1, I-2, II-1, II-2, III-1, III-2, I-1a, I-1b, I-1c, I-1d, II-1a, II-1b, II-1c, II-1d, I-1a-1, I-1a-2, I-1a-2-A, II-1a-1, II-1a-2, II-1a-2-A, I-2a ou II-2a décrit précédemment, éventuellement en association avec un support biocompatible.

En particulier, la composition radiopharmaceutique comprend comme substance active un composé marqué de formule II, IIbis, IIa, IIb, II-1, II-2, II-1a, II-1b, II-1c, II-1d, II-1a-1, II-1a-2, ou II-2a. L'Invention concerne également un composé de formule I, II, III, Ibis, IIbis, Ia, Ib, IIa, IIb, I-1, I-2, II-1, II-2, III-1, III-1, I-1a, I-1b, I-1c, I-1d, II-1a, II-1b, II-1c, II-1d, I-1a-1, I-1a-2, I-1a-2-A, II-1a-1, II-1a-2, II-1a-2-A, I-2a ou II-2a tel que décrit précédemment pour son utilisation comme médicament pharmaceutique ou radiopharmaceutique, éventuellement en association avec un support biocompatible.

Selon un mode de réalisation, le composé de l'invention pour son utilisation comme médicament pharmaceutique ou radiopharmaceutique est administrable par voie parentérale, notamment par voie intraveineuse, à une dose de l'ordre de 3 MBq/kg de poids corporel de l'individu. La dose sera déterminée en fonction de l'individu.

L'activité de la dose administrée est au minimum de 200 MBq afin d'être observable dans le cerveau mais au maximum de 450 MBq pour des raisons de radioprotection des patients.

L'Invention concerne également une méthode pour diagnostiquer ou surveiller une maladie neuro-dégénérative chez un sujet humain ou animal comprenant :

une étape d'administration d'un composé de formule II, IIbis, IIa, IIb, II-1, II-2, II-1a, II-1b, II-1c, II-1d, II-1a-1, II-1a-2, II-1a-2-A ou II-2a comprenant un radioélément à un sujet humain ou animal ;
une étape d'imagerie du cerveau humain ou animal par la tomographie par émission de positrons ou tomographie d'emission monophotonique.

Le cas animal permet notamment de vérifier la fixation in vivo du traceur sur la cible. L'animal est ainsi utilisé comme modèle. Il s'agit, par exemple, d'un animal auquel des agrégats ont été injectés dans le cerveau, ou d'un animal génétiquement modifié qui exprime dans son cerveau des agrégats humains.

La quantification in vivo de l'aggrégation des protéines β-amyloïde, α-synucléine et/ou tau fibrillaires chez un patient est bénéfique non seulement pour un diagnostic précoce des pathologies en résultant mais également pour suivre l'évolution de ces pathologies et évaluer les traitements.

Les méthodes diagnostiques adaptées pour une utilisation des ligands des protéines β-amyloïde, α-synucléine et/ou tau fibrillaires de l'Invention sont la TEP et la TEMP. La TEP et la TEMP utilisent des molécules biologiquement actives, préalablement marquées avec un isotope à courte durée de vie émetteur de positrons, ou des emétteurs gamma à des concentrations de l'ordre du micromolaire ou nanomolaire. Les caractéristiques physiques des isotopes et les sélectivités moléculaires des molécules marquées, combinées avec la haute efficacité de détection des scanners utilisés pour ces methodes scintigtaphiques apportent une sensibilité pour les mesures in vivo indicatrices de concentrations qui est, de plusieurs ordres de magnitude, plus élevée que d'autres méthodes d'imagerie.

L'administration à un sujet du composé marqué avec un isotope émetteur de positrons ou emétteurs gamma a lieu par voie intraveineuse. Le sujet est scanné et des sections tomographiques axiales de la région cérébrale dans laquelle le composé marqué s'est accumulé sont obtenues. L'accumulation du composé peut être reliée au métabolisme cérébral, au flux sanguin ou à la concentration des sites de liaison grâce à des modèles mathématiques appropriés. Ainsi, l'utilisation d'un composé marqué avec un isotope émetteur de positrons ayant une forte affinité et sélectivité pour l'une des protéines β-amyloïde, α-synucléine et/ou tau fibrillaires permet la quantification du niveau d'aggrégation de ces protéines et leur localisation. Cette approche permet non seulement d'améliorer le diagnostic des maladies neurodégénératives telles que la MP, mais également de fournir un outil capable de suivre l'évolution de ces maladies et l'efficacité du traitement, et d'améliorer la compréhension de la progession de ces maladies.

Selon un mode de réalisation de l'Invention, dans la méthode de l'Invention la maladie neuro-dégénérative est une synucléinopathie, une amyloïdopathie ou une tauopathie.

Selon un mode de réalisation de l'Invention, dans la méthode de l'Invention la maladie neuro-dégénérative est une synucléinopathie.

Selon un mode de réalisation de l'Invention, dans la méthode de l'Invention la maladie neuro-dégénérative est une amyloïdopathie.

Selon un mode de réalisation de l'Invention, dans la méthode de l'Invention la maladie neuro-dégénérative est une tauopathie.

Selon un mode de réalisation de l'Invention, dans la méthode de l'Invention le radioélément est choisi parmi $^{18}F$, $^{11}C$, $^{123}I$ et $^{124}I$.

## FIGURES

Figure la schématise la préparation de synthons radiomarqué au carbone11 1a) à partir du dioxyde de carbone radiomarqué au carbone11 et 1b) à partir du méthane radiomarqué au carbone11.

Figure 2 représente les fonctionnalités chimiques radiomarquées au carbone11 accessibles par des réactions de carbonylation.

Figure 3 représente les chromatogrammes de contrôle qualité du composé [$^{18}$F]**28** avec une double détection, A) signal radioactif et B) ultraviolet à 360 nm.

Figure 4 représente l'image obtenue après l'expérience décrite dans l'exemple 19.6 en imagerie TEP/CT, il s'agit de la coupe transversale au niveau de la tête d'un rat après injection i.v. du traceur et de la somme des acquisitions entre 50 et 180 min post-injection.

## EXEMPLES

### Procédures générales

**Exemple 1. Procédure A (Protection des azaindoles avec un groupement benzènesulfonyle)**

**Exemple 1.1**

**A1** : A une solution de NaH (2 éq.) dans du THF sous argon et à 0°C a été additionnée lentement une solution d'azaindole (1 éq.) dans du THF (1 M). Le mélange est agité à température ambiante pendant 30 min. De retour à 0°C, le chlorure de benzènesulfonyle (1.2 éq.) a été ajouté goutte-à-goutte. Après une nuit d'agitation à température ambiante, le mélange a été versé dans un becher rempli avec de la glace. Une fois le milieu revenu à la température ambiante, les phases ont été séparées. La phase aqueuse a été extraite à l'acétate d'éthyle. Les phases organiques réunies ont été lavées au NaCl, séchées sur $MgSO_4$ et concentrées sous pression réduite. Le produit protégé a été isolé soit par filtration et lavage de précipité par de l'éther diéthylique, soit par colonne de flash chromatographie sur gel de silice.

**Exemple 1.2**

**A2** : A une solution d'azaindole (1 éq.) dans de la 2-butanone (0.1 M) ont été ajoutés le carbonate de potassium (4 éq.) et le chlorure de benzènesulfonyle (1.5 éq.). Le mélange est agité à 80°C durant 2 heures. Le mélange est concentré sous pression reduite, puis de l'eau et de l'acétate d'éthyle ont été ajoutés. Après séparation des phases, la phase aqueuse a été extraite à l'acétate d'éthyle (3 fois). Les phases organiques réunies ont été lavées au NaCl, séchées sur $MgSO_4$ et concentrées sous pression réduite. Le produit protégé a été isolé soit par filtration et lavage de précipité à l'éther diéthylique, soit par colonne de chromatographie flash sur gel de silice.

**Exemple 2. Procédure B (Iodation en position C-2 des azaindoles)**

**Exemple 2.1**

**B1** (E. Desarbre, S. Coudret, C. Meheust, J.-Y. Merour, Tetrahedron, 1997, 53 (10), 3637-3648 ; B. Joseph, H. Da Costa, J.-Y. Merour, S. Leonce, Tetrahedron, 2000, 56, 3189-3196) : Sous atmosphère d'argon, à une solution de phénylsulfonylazaindole (1 éq.) et TMEDA (1.05 éq.) dans du THF (0.1 M), refroidie à -25°C, a été additionnée une solution de LDA dans du THF (2 M, 2 éq.) goutte-à-goutte. Après 30 min, une solution d'iode (1 M, 2 éq.) dans du THF a été ajoutée lentement *via* une cannule au milieu réactionnel. La réaction est suivie par RMN $^{1}$H, une fois le produit de départ consommé, l'addition d'eau et extraction avec de l'acétate d'éthyle (3 fois) sont réalisées. La phase organique rassemblée est séchée sur $MgSO_4$, puis concentrée sous pression réduite et purifiée par colonne de chromatographie flash sur gel de silice.

**Exemple 2.2**

**B2:** Sous atmosphère d'argon, à une solution de phénylsulfonylazaindole (1 éq.) et TMEDA (1.1 éq.) dans du

THF (0.1M), refroidie à -70°C, a été additionnée une solution de LDA (2M dans du THF, 1,7 éq.) goutte-à-goutte. Après 30 min, une solution d'iode (2.2 éq.) dans THF (1M) a été ajoutée lentement *via* une cannule au milieu réactionnel. La réaction est suivie par RMN $^1H$. Une fois le produit de départ consommé, de l'eau est additionnée et la phase aqueuse est extraite avec de l'acétate d'éthyle (3 fois). La phase organique rassemblée est séchée ensuite sur $MgSO_4$, puis concentrée sous pression réduite et purifiée par colonne de chromatographie flash sur gel de silice.

**Exemple 3 : Procédure C (Couplage de Sonogashira)**

**Exemple 3.1 C1 :**

Du catalyseur $[Pd(PPh_3)_4]$ (5 % mol) a été ajouté à une solution dégazée de dérivé iodé azaindolique, (1.0 éq.), d'alcyne (1.2 éq.), de CuI (10 % mol), dans un mélange de $Et_3N$/THF (1:1) à la concentration de 0.1 M. Le mélange est chauffé à 50-60°C pendant 4 h sous atmosphère d'argon. Après un retour à la température ambiante, le mélange est ensuite versé dans une solution aqueuse (10%) de $NH_4Cl$. Après séparation, la phase aqueuse est extraite avec de l'acétate d'éthyle (3 fois 20 mL) et les phases organiques réunies ont ensuite été lavées avec du NaCl, séchées sur $MgSO_4$, filtrées et concentrées sous pression réduite, avant d'être purifiées par colonne de chromatographie flash sur gel de silice.

**Exemple 3.1 C2**

Du catalyseur $[Pd(PPh_3)_2Cl_2]$ (5 % mol) a été ajouté à une solution dégazée de THF (C = 0.1 M) de dérivé iodé azaindolique, (1.0 éq.), d'alcyne (1.2 éq.), de CuI (10 % mol) et $Et_3N$ (2.2 éq). Le mélange est agité à température ambiante pendant 16 h sous atmosphère d'argon. Ensuite, le mélange réactionnel est concentré sous pression réduite, avant d'être purifiées par colonne de chromatographie flash sur gel de silice.

**Exemple 4. Procédure D (desulfonylation des azaindoles)** (C. Chaulet, C. Croix, J. Basset, M.-D. Pujol, M.-C. Viaud-Massuard, *Synlett.* **2010**, *10,* 1481-1484)

A une solution de dioxane contenant le dérivé d'azaindole (1 éq.) protégé par un groupement benzene sulfonyle, a été ajouté le *tert*-butoxide de sodium (1.5 éq.). Le mélange est chauffé à 80°C durant 18 heures. Le milieu est concentré sous pression réduite et le résidu a été dissous dans de l'acétate d'éthyle et de l'eau. Après décantation, la phase aqueuse a été extraite avec de l'acétate d'éthyle (3 fois). Les phases organiques réunies ont été séchées sur $MgSO_4$ et concentrées sous pression réduite, avant de purifier le brut réactionnel par colonne de chromatographie flash sur gel de silice.

**Exemple 5. Procédure E (protection des azaindole avec un Boc)**

Une solution d'azaindole (1 éq.), du dicarbonate de di-*tert*-butyle (1.5 éq.) et de la 4- DMAP (0,2 éq.) dans du THF (0.2 M) est agitée à température ambiante durant une nuit. Le mélange est concentré sous pression réduite, puis purifié par colonne de chromatographie flash sur gel de silice.

**Exemple 6. Procédure F (déprotection du Boc)**

A une solution de dichlorométhane (0.05 M) refroidie à 0°C, contenant le produit azaindolique protégé par le groupement Boc (1 éq.), a été additionné du TFA goutte-à-goutte (rapport TFA/dichlorométhane = 1/2). L'agitation à 0°C est effectuée avec un suivi CCM. A la fin de la réaction, le milieu réactionnel est neutralisé avec une solution aqueuse saturée en $NaHCO_3$. Après décantation, la phase aqueuse est extraite avec du dichlorométhane (2 fois). Les phases organiques réunies ont été séchées sur $MgSO_4$ et concentrées sous pression réduite, avant de purifier le brut réactionnel par colonne de chromatographie flash sur gel de silice.

**Exemple 7. Procédure G (Couplages de Suzuki)**

**Exemple 7.1 G1** : Un composé halogéné (1 éq.) est dissous dans du dioxane sous argon, puis l'acide boronique (1.2 éq.), du $Cs_2CO_3$ (1.2 éq.), ainsi que du $[Pd_2(dba)_3]$ (5 mol %) et son ligand P(*t*-Bu)$_3$ (10 mol %) ont été ajoutés. Le milieu réactionnel est agité et irradié sous microonde à 100°C pendant 1 heure. Après refroidissement, le mélange est concentré sous pression réduite pour ensuite purifier le résidu par colonne de chromatographie flash.

**Exemple 7.2 G2 :** Un composé halogéné (1 éq.) est dissous dans un mélange DME/$H_2O$ (2/1) sous argon, puis l'acide boronique (1.2 éq.), du $Na_2CO_3$ (2 éq.) et du $[Pd(PPh_3)_4]$ (5 mol %) ont été ajoutés. Le milieu réactionnel

est agité et irradié sous microonde à 120°C pendant 30 min. Après refroidissement, l'eau est ajoutée et le composé extrait avec du dichlorométhane (3 fois). La phase organique a été séchée sur $MgSO_4$ et concentrée sous pression réduite, avant de purifier le résidu par colonne de chromatographie flash.

**Préparation de dérivés acétylèniques des azaindoles**

Schéma général :

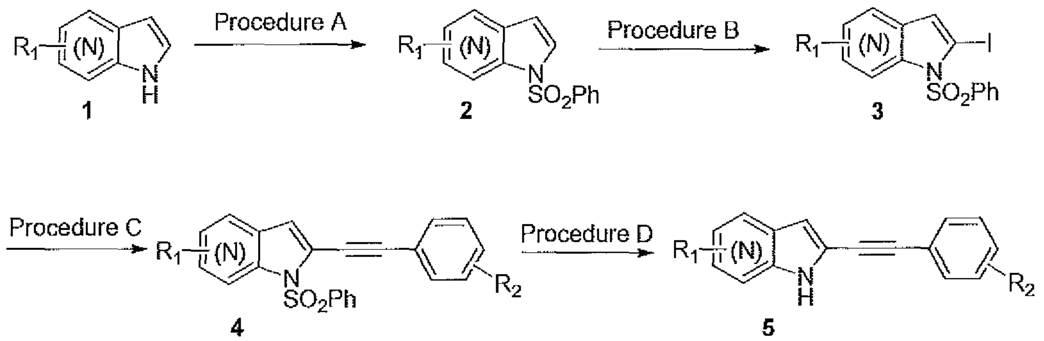


1 2 3 4 5

**Exemple 8. Synthèse d'azaindoles de départ**

Parmi les différents azaindoles, certains ont été synthétisés selon les méthodes de préparation décrites dans la littérature. C'est notamment le cas du 4-azaindole (**1a**), 6-azaindole (**1b**), 6-chloro-7-azaindole (**1c**) et 6-fluoro-7-azaindole (**1d**).

**Tableau 1. Azaindoles de départ**

| | | | |
|---|---|---|---|
| 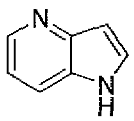 **1a** Rendement global 78% | 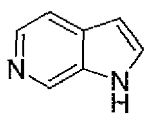 **1b** Rendement global 50% | 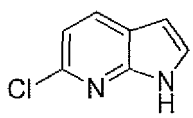 **1c** Rendement global 33% | 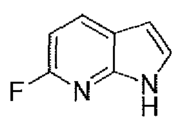 **1d** Rendement global 5% |

**Exemple 8.1 Préparation du 4-azaindole 1a**

Le 4-azaindole a été préparé à partir du 4-amino-2-bromopyridine selon une séquence de 2 étapes :

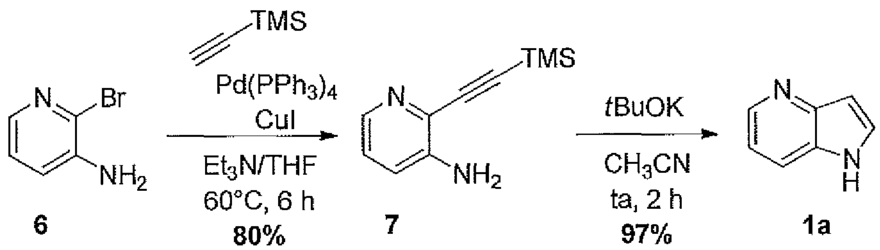


6 7 1a

Préparation du 2-((triméthylsilyl)éthynyl)pyridin-3-amine 7

Le produit **7** est obtenu à partir de 3-amino-2-bromopyridine **6** selon la **procédure C** en agitant le mélange à 60°C pendant 6 h et est isolé avec un rendement de 80% par colonne de chromatographie flash (acétate d'éthyle/éther de pétrole = 5/95).

Préparation du 4-azaindole **Ia**

Le produit **7** (1.96 g, 10.3 mmol, 1 éq.) a été engagé dans une réaction de cyclisation en présence de la solution 1M dans du THF du *tert*-butoxide de potassium (10.3 mL, 10.3 mmol, 1 éq.) dans le $CH_3CN$ (15 mL) à température ambiante durant 2 heures. Le mélange est ensuite dilué avec de l'acétate d'éthyle et hydrolysé avec de l'eau. Après décantation, la phase organique est lavée avec une solution aqueuse saturée de NaCl, séchée sur $MgSO_4$ et concentrée sous pression réduite. Le 4-azaindole Ia a été isolé avec un rendement de 97% par colonne de chromatographie flash sur gel de silice (acétate d'éthyle/éther de pétrole = 1/1).

**Exemple 8.2 Préparation du 6-azaindole 1b**

Le 6-azaindole a été préparé à partir de la 3-amino-4-méthylpyridine par une séquence en 3 étapes décrite par Hands (D. Hands, B. Bishop, M. Cameron, J. S. Edwards, I. F. Cottrell, S. H. B. Wright, *Synthesis,* **1996**, 7, 877-882).

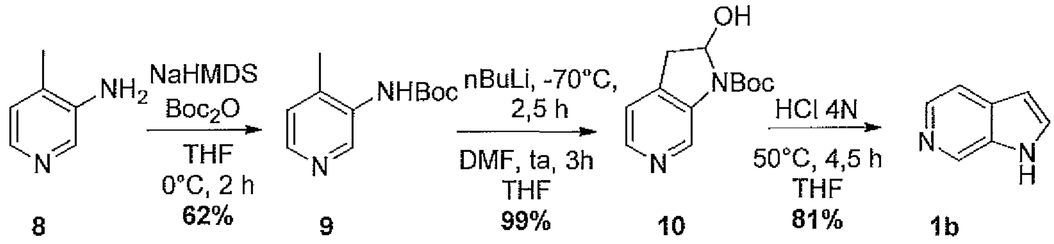


Préparation du *tert*-butyl ('4-méthylpyridin-3-yl)carbamate **9**

A une solution de 3-amino-4-méthylpyridine (2 g, 18.5 mmol, 1 éq.) dans le THF (35 mL) sous argon à 0°C, une solution de 1M NaHMDS/THF (40.7 mL, 40.7 mmol, 2.2 éq.) a été additionnée lentement. Ensuite du $Boc_2O$ (4.8 g, 22.2 mmol, 1.2 éq.) est ajouté et le bain de glace enlevé. Le mélange est agité 1h30 à température ambiante, puis le solvant est évaporé et le résidu a été repris dans du dichlorométhane pour être lavé avec une solution aqueuse d'HCl (50 mL), puis avec une solution saturée de NaCl. La phase organique a été sechée sur $MgSO_4$ et concentrée sous pression réduite, avant d'isoler 9 avec un rendement de 62% (2.4 g) après une colonne de chromatographie flash sur gel de silice (acétate d'éthyle/éther de pétrole = 30/70).

Préparation du *tert*-butyl 2-hydroxy-2,3-dihydro-1*H*-pyrrolo[2,3-c]pyridine-1-carboxylate **10**

A une solution du *tert*-butyl (4-méthylpyridin-3-yl)carbamate **9** (2.4 g, 11.5 mmol, 1 éq.) dans le THF (100 mL) refroidie à -70°C, a été additionnée une solution 2.2 M de *n*BuLi/hexanes (11.5 mL, 25.4 mmol, 2.2 éq.) goutte-à-goutte. Après l'ajout, la température est remontée à -30°C et maintenue à cette température en agitant le mélange durant 2h30. Ensuite le DMF (1.34 mL, 17.3 mmol, 1.5 éq.) est ajouté et le mélange est laissé remonter à température ambiante. Après 3 heures d'agitation à température ambiante, l'eau (100 mL) est additionnée et le produit extrait par de l'acétate d'éthyle (100 mL×3). Les phases organiques réunies ont été lavées avec une solution saturée de NaCl, séchées sur $MgSO_4$ et concentrées sous pression réduite, avant de purifier **10** par colonne de chromatographie flash sur gel de silice (acétate d'éthyle/éther de pétrole = 1/1) pour fournir le produit souhaité avec un rendement de 99% (2.68 g).

Préparation du 6-azaindole **1b**

A une solution du *tert*-butyl 2-hydroxy-2,3-dihydro-1*H*-pyrrolo[2,3-*c*]pyridine-1-carboxylate **10** (2.68 g, 11.3 mmol, 1 éq.) dans le THF (45 mL) a été ajoutée une solution 4N de HCl dans le dioxane (15.6 mL, 62.4 mmol, 5.5 éq.). Le mélange est agité à 50°C durant 4h30. Le mélange réactionnel est neutralisé avec une solution aqueuse 5M de NaOH (15.8 mL, 79.4 mmol, 7 éq.) et la phase aqueuse est extraite avec l'acétate d'éthyle (3 fois). Les phases organiques combinées sont sechées sur $MgSO_4$ et concentrées sous pression réduite pour ensuite être purifiées par colonne de chromatographie flash sur gel de silice (le solvant utilisé: acétate d'éthyle) le produit souhaité **1b** avec un rendement de 81% (1.09 g).

**Exemple 8.3 Préparation du 6-chloro-7-azaindole 1c**

Le 6-chloro-7-azaindole **Ie** a été préparé par une séquence en 3 étapes décrite par Minakata (S. Minakata, M. Komatsu, Y. Ohshiro, Synthesis 1992, 7, 661-663):

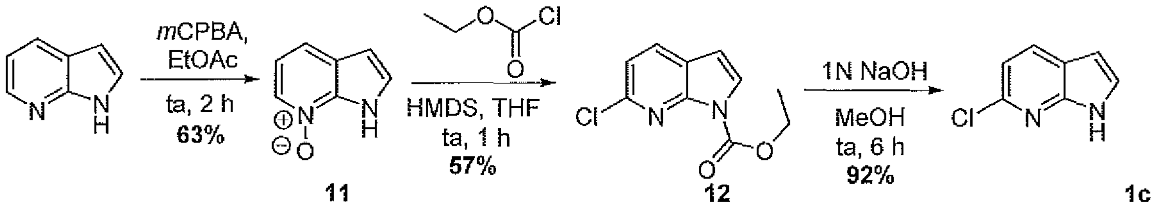


Préparation de *N*-oxide **11**

A une solution de 7-azaindole (2 g, 16.9 mmol, 1 éq.) dans de l'acétate d'éthyle (60 mL) à 0°C, *m*CPBA (6.3 g, 25.6 mmol, 1.5 éq.) a été ajouté. Le milieu est agité à température ambiante pendant 2 heures. Le mélange réactionnel est ensuite mis dans un bain de glace et le précipité formé est isolé par filtration et lavé avec l'éther diéthylique. Ensuite l'eau est ajoutée à ce solide jaunâtre et le milieu basifié avec une solution saturée de $K_2CO_3$ jusqu'à l'obtention d'un pH = 9. Le mélange est mis au réfrigérateur et filtré à froid le lendemain. Le filtrat est concentré de nouveau et remis au froid pour répéter l'opération et donner après groupement des moissons, un solide blanc avec un rendement de 63% (1.43 g).

Préparation du l'éthyl 6-chloro-1*H*-pyrrolo[2,3-bjpycidine-1-carbmylate **12**

A une solution de **11** (3.05g, 22.7 mmol, 1 éq.) et HMDS (3.67 g, 22.7 mmol, 1 éq.) dans du THF, le chloroformate de méthyle (5.42 mL, 56.8 mmol, 2.5 éq.) est ajouté goutte-à-goutte. Le milieu est ajouté pendant 1 heure à température ambiante, puis concentré sous pression réduite. Le residu obtenu est dissous dans l'acétate d'éthyle, la phase organique est lavée avec une solution saturée de $NaHCO_3$, séchée sur $MgSO_4$ et concentrée sous pression réduite, avant de purifier le résidu par colonne de chromatographie flash sur gel de silice (acétate d'éthyle/éther de pétrole = 1/1) et fournir le produit souhaité **12** avec un rendement de 99% (2.68 g).

Préparation du 6-chloro-7-azaindole **1c**

A une solution de 12 (0.302 g, 1.4 mmol, 1 éq.) dans du MeOH (25 mL) est ajoutée une solution 1 N de NaOH (11 mL). Le mélange est agité à température ambiante durant 6 heures, puis neutralisé avec une solution saturée de $NaHCO_3$ et concentré sous pression réduite. Le précipité est ensuite lavé à l'eau, filtré et séché sous pression réduite pour donner le avec un rendement de 92% (0.2 g).

**Exemple 8.4 Préparation du 6-fluoro-7-azaindole 1d**

Le 6-fluoro-7-azaindole **1d** a été préparé par une méthode en 5 étapes au départ de la 2-amino-6-fluoropyridine (A. Stoit, H. K.A.C. Coolen, M. A.W. Van Der Neut, C. G. Kruse, Préparation of azaindoles with a combination of partial nicotinic acetylcholine receptor agonism and dopamine reuptake inhibitory activity PCT Int. Appl. 2008, WO 2008003736 A1 Jan 10, 2008).

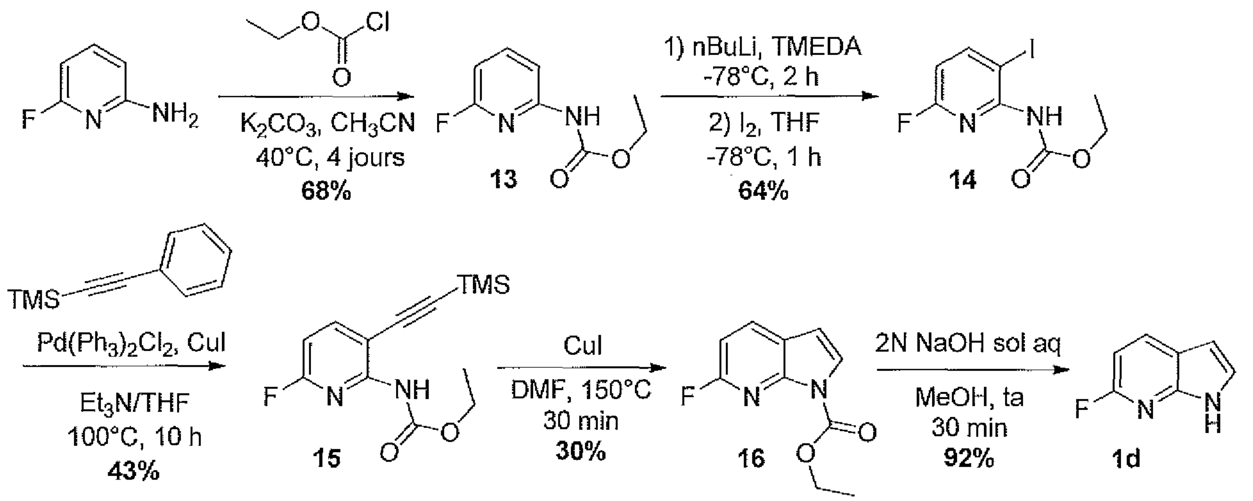

Préparation de l'éthyl(6-fluoropyridin-2-yl)carbamate **13**

A une solution contenant de la 2-amino-6-fluoropyridine (5 g, 44.6 mmol, 1 éq.) sont ajoutés du $K_2CO_3$ (18.5 g, 133.8 mmol, 3 éq.) dans du $CH_3CN$ (110 mL) et du chloroformate d'éthyle (4.3 mL, 44.6 mmol, 1 éq.). Le mélange est agité à 40°C pendant 4 jours. Ensuite une portion supplémentaire de chloroformate d'éthyle (4.3 mL, 44.6 mmol, 1 éq.) est additionnée et le mélange est agité à 40°C pendant 2 jours. Au retour à la température ambiante, de l'acétate d'éthyle est ajouté et la phase organique est lavée avec une solution saturée de $NaHCO_3$, séchée sur $MgSO_4$, filtrée et concentrée sous pression réduite. Le résidu est ensuite purifié par colonne de chromatographie flash sur gel de silice (acétate d'éthyle/éther de pétrole = 10/90) pour fournir le produit souhaité 13 avec un rendement de 68% (5.62 g). **RMN $^1$H** (400 MHz, $CDCl_3$, 20°C) $\delta$ 7.83 (dd, *J* = 8.0 Hz, *J* = 2.0 Hz, 1H), 7.77 (m, 1H), 7.45 (ls, 1H), 6.59 (m, 1H), 4.25 (q, *J* = 8.0 Hz, 2H), 1.32 (t, *J* = 8.0 Hz, 3H).

Préparation de l'éthyl (6-fluoro-3-iodopyridin-2-yl)carbamate **14**

A une solution of **13** (5.6 g, 30.4 mmol, 1 éq.) dans du THF (80 mL), de la TMEDA (11.3 mL, 76.0 mmol, 2.5 éq.) est ajoutée et le mélange a été refroidi à -78°C sous argon. Puis une solution de n-BuLi (1.6 M dans le THF, 50 mL, 79.1 mmol, 2.6 éq.) a été additionnée lentement. Le mélange est agité à - 78°C pendant 2 heures. Ensuite une solution d'iode (18 g, 70.9 mmol, 2.3 éq.) dans du THF (20 mL) a été ajoutée *via* une canule. Après 1 heure d'agitation à -78°C, une solution aqueuse saturée en $Na_2S_2O_3$ est ajoutée. Au retour à température ambiante, de l'acétate d'éthyle est ajouté et la phase organique séparée est lavée avec une solution aqueuse saturée de $NaHCO_3$, séchée sur $MgSO_4$ et concentrée sous pression réduite, avant de purifier le résidu par colonne de chromatographie flash sur gel de silice (acétate d'éthyle/éther de pétrole = 10/90) et fournir le produit souhaité **14** avec un rendement de 64% (6.03 g). **RMN $^1$H** (400 MHz, $CDCl_3$, 20°C) $\delta$ 8.07 (t, *J* = 8.0 Hz, 1H), 7.19 (1s, 1H), 6.49 (dd, *J* = 8.0 Hz, *J* = 3.0 Hz, 1H), 4.29 (q, *J* = 8.0 Hz, 2H), 1.35 (t, *J* = 8 Hz, 3H).

Préparation de l'éthyl (6-fluoro-3-((triméthylsilyl)éthynyl)pyridin-2-yl)carbamate **15**

Du $[Pd(PPh_3)_2]Cl_2$ (0.68 g, 0.97 mmol, 0.05 éq.) a été ajouté à une solution dégazée contenant le dérivé **14** (6.03 g, 19.5 mmol, 1 éq.), de l'allyltriméthylsilane (4.9 mL, 35.0 mmol, 1,2 éq.), du CuI (0.37 g, 1.95 mmol, 0.1 éq.), dans un mélange de 20 mL de $Et_3N$/DMF (1:1). Le mélange est chauffé à 100°C pendant 10 heures sous atmosphère d'argon. Après un retour à température ambiante, le mélange est ensuite versé dans une solution d'acétate d'éthyle et d'$H_2O$. Après décantation, la phase organique est lavée avec $H_2O$, séchée sur $MgSO_4$ et concentrée sous pression réduite, avant de purifier le résidu par colonne de chromatographie flash sur gel de silice (acétate d'éthyle/éther de pétrole = 5/95) et fournir **15** avec un rendement de 43% (2.32 g). **RMN $^1$H** (250 MHz, $CDCl_3$, 20°C) $\delta$ 7.76 (t, *J* = 8.0 Hz, 1H), 7.64 (1s, 1H), 6.56 (dd, *J* = 8.0 Hz, *J* = 3.0 Hz, 1H), 4.29 (q, *J* = 8.0 Hz, 2H), 1.34 (t, *J* = 8.0 Hz, 3H), 0.3 (s, 9H).

Préparation de l'éthyl 6-flaoro-1*H*-pyrrolo[2,3-*b*]pyridine-1-carboxylate **16**

Un mélange de dérivé **15** (2.32 g, 8.3 mmol, 1 éq.) et de CuI (3.15 g, 16.5 mmol, 2 éq.) dans du DMF (40 mL) a été dégazé sous argon durant 30 minutes. Ensuite, le milieu réactionnel a été chauffé à 150°C durant 30 minutes. Après refroidissement, le milieu est dilué avec de l'acétate d'éthyle et filtré. Le résidu est lavé avec de l'$H_2O$, séché sur $MgSO_4$, filtré et concentré sous pression réduite, avant d'être purifié par colonne de chromatographie flash sur gel de silice (acétate d'éthyle/éther diéthylique = 1/1) pour fournir **16** avec un rendement de 30% (0.52 g). **RMN $^1$H** (250 MHz, $CDCl_3$, 20°C) $\delta$ 7.95 (t, *J* = 8.0 Hz, 1H), 7.69 (d, *J* = 4.0 Hz, 1H), 6.87 (dd, *J* = 8.0 Hz, *J* = 2.0 Hz, 1H), 6.57 (d, *J* = 4.0 Hz, 1H), 4.55 (q, *J*= 8.0 Hz, 2H), 1.49 (t, *J* = 8.0 Hz, 3H).

Préparation de 6-fluoro-7-azaindole **1d**

A une solution de **16** (1.3 g, 6.2 mmol, 1 éq.) dans le MeOH (25 mL) est additionnée une solution aqueuse 2N de NaOH (10 mL). Le mélange est agité pendant 30 minutes à température ambiante, puis de l'acétate d'éthyle est ajouté. Après décantation, la phase organique est lavée avec une solution à 5% de $NaHCO_3$, séchée sur $MgSO_4$, filtrée et concentrée sous pression réduite pour fournir **1d** avec un rendement de 99% (0.84 g). **RMN $^1$H** (400 MHz, $CDCl_3$, 20°C) $\delta$ 9.6 (1s, 1H), 7.95 (t, *J* = 8.0 Hz, 1H), 7.29 (m, 1H), 6.75 (dd, *J* = 8.0 Hz, *J* = 2.0 Hz, 1H), 6.53 (m, 1H).

**Exemple 9.Synthèse des N-benzènesulfonyle azaindole**

**Exemple 9.1 Synthèse de dérivés de *N*-benzènesulfonyle azaindole décrits dans la littérature** Les azaindoles commerciaux et ceux préparés ci-dessus ont été protégés par le groupement benzènesulfonyle selon l'une de

deux méthodes générales alternatives (**A1** et **A2**). Le tableau 2 ci-dessous inclut les composés 1-(phénylsulfonyl)-1*H*-pyrrolo[2,3-*b*]pyridine **2a** (S.-F. Liu, Q. Wu, H. L. Schmider, H. Aziz, N.-X. Hu, Z. Popovic, S. Wang, J. Am. Chem. Soc. 2000, 122, 3671-3678), 1-(phénylsulfonyl)-1*H*-pyrrolo[3,2-*c*]pyridine **2b** (M. Lefoix, J.-P. Daillant, S. Routier, J.-Y. Mérour, I. Gillaizeau, G. Coudert, Synthesis, 2005, 20,3581-3588), 1-(phénylsulfonyl)-1*H*-pyrrolo[3,2-*b*]pyridine **2c** (D. C. Brookings, R. J. Cubbon, J. M. Davis, B. J. Langham. Préparation of pyrrolo[3,2-b]pyridines as p38 kinase inhibitors. PCT Int. Appl., 2004031188, 15 Apr 2004), 1-(phénylsulfonyl)-1*H*-pyrrolo[2,3-c]pyridine **2d** (Y. Horiguchi, H. Imoto, M. A. Wolf. Préparation of 6-azaindoles as IκB kinase inhibitors for treating diabetes and inflammatory diseases. PCT Int. Appl., 2005097129, 20 Oct 2005), 6-chloro-1-(phénylsulfonyl)-1*H*-pyrrolo[2,3-*b*]pyridine **2e** (Y.-S. Tung, M. S. Coumar, Y.-S. Wu, H.-Y. Shiao, J.-Y. Chang, J.-P. Liou, P. Shukla, C.-W. Chang, C.-Y. Chang, C.-C. Kuo, T.-K. Yeh, C.-Y. Lin, J.-S. Wu, S.-Y. Wu, C.-C. Liao, H.-P. Hsieh, J Med. Chem. 2011, 54, 3076-3080), 6-fluoro-1-(phénylsulfonyl)-1*H*-pyrrolo[2,3-*b*]pyridine **2f** (A. R. Stoit, A. P. den Hartog, H. Mons, S. van Schaik, N. Barkhuijsen, C. Stroomer, H. K. A. C. Coolen, J. H. Reinders, T. J. P. Adolfs, M. van der Neut, H. Keizer, C. G. Kruse, Bioorg. Med. Chem. Lett. 2008, 18, 188-193), 5-fluoro-1-(phénylsulfonyl)-1*H*-pyrrolo[2,3-*b*]pyridine **2g** (S. J. Berthel, L. Chen, W. L. Corbett, L. C. Feng, N.-E. Haynes, R. F. Kester, S. S. So, J. Wright Tilley. Azaindole derivatives as glucokinase activators and their preparation and use in the treatment of metabolic disorders. U.S. Pat. Appl. Publ., 20110144105, 16 Jun 2011), 6-bromo-1-(phénylsulfonyl)-1*H*-pyrrolo[2,3-*b*]pyridine **2i** (A. Stoit, H. K.A.C. Coolen, M. A.W. Van Der Neut, C. G. Kruse, Azaindole derivatives with a combination of partial nicotinic acetylcholine receptor agonism and dopamine reuptake inhibition, US 2008/0009514 A1 Jan 10, 2008), 5-bromo-1-(phénylsulfonyl)-1*H*-pyrrolo[2,3-*b*]pyridine **2j** (N. Liu, Y Wang, G. Huang, C. Ji. W. Fan, H. Li, Y. Cheng, H. Tian, Bioorg. Chem. 2016, 65, 146-158) et 4-chloro-1-(phénylsulfonyl)-1*H*-pyrrolo[2,3-*b*]pyridine **2k** (H.-Q. Dong, K. Foreman, A.-H. Li, M. J. Mulvihill, B. Panicker, A. G. Steinig, K. M. Stolz, Q. Weng, M. Jin, B. Volk, J. Wang, T. Wang, J. D. Beard, Pyrrolopyridine kinase inhibiting compounds, US 2007/0129364 A1 Jun. 7, 2007).

**Tableau 2. *N*-benzènesulfonyle azaindoles**

| | | | |
|---|---|---|---|
| 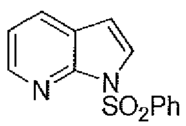 **2a, 90%** *Procédure A1* | 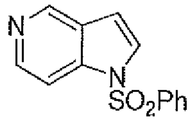 **2b, 92%** *Procédure A2* | 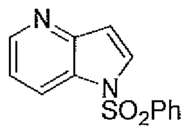 **2c, 79%** *Procédure A2* | 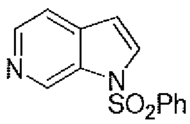 **2d, 85%** *Procédure A2* |
| 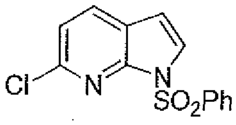 **2e, 96%** *Procédure A2* | 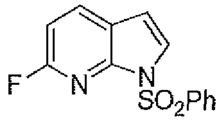 **2f, 89%** *Procédure A1* | 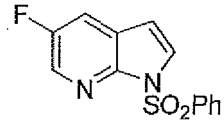 **2g, 80%** *Procédure A1* | 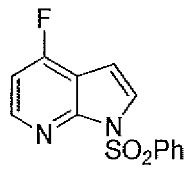 **2h, 98%** *Procédure A1* |
| 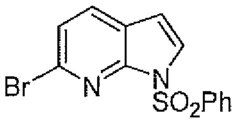 **2i, 97%** *Procédure A1* | 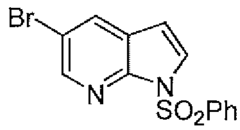 **2j, 91%** *Procédure A1* | 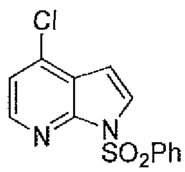 **2k, 96%** *Procédure A1* | |

**Exemple 10. Synthèse de dérivés iodés d'azaindoles**

Les différents dérivés iodés ont été préparés par les **procédures** générales adéquates. Les composés 2-iodo-1-(phénylsulfonyl)-1*H*-pyrrolo[2,3-*b*]pyridine **3a**,2-iodo-1-(phénylsulfonyl)-1*H*-pyrrolo[3,2-*c*]pyridine **3b,** ont été obtenus avec des bons rendements de 83 et 60% respectivement.

**Tableau 3. Dérivés iodés d'azaindoles obtenus selon la procédure A, B ou B et D**

| | | | |
|---|---|---|---|
| 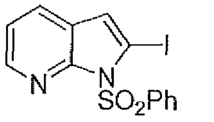 **3a, 83%** *Procédure B1* | 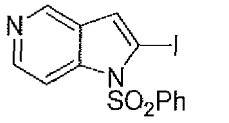 **3b, 60%** *Procédure B1* | 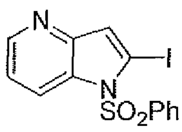 **3c, 70%** *Procédure B1* | 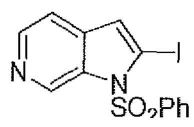 **3d, 67%** *Procédure A2* |

(suite)

| | | | |
|---|---|---|---|
| 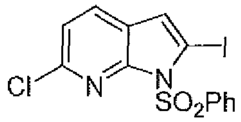<br>**3e, 65%** *Procédure B2* | 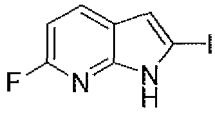<br>**18f 56%** sur 2 étapes *Procédures B2/D* | 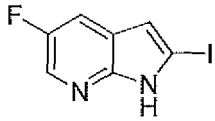<br>**18g 48%** sur 2 étapes *Procédures B2/D* | 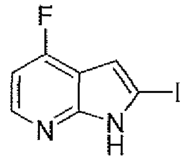<br>**18h 73%** sur 2 étapes *Procédure B2/D* |
| 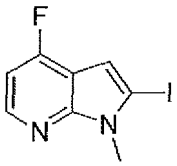<br>**18i, 90%** | 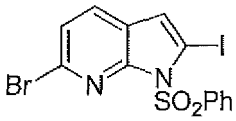<br>**3j, 93%** *Procédure B2* | 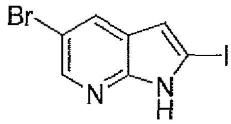<br>**18k 3%** sur 2 étapes *Procédures B1/D* | 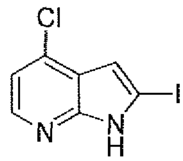<br>**18l 58%** sur 2 étapes *Procédure B1/D* |

Le 2-iodo-1-(phénylsulfonyl)-1*H*-pynolo[3,2-*b*]pyridine **3c** a été préparé selon la procédure B1, cependant, sous les mêmes conditions son isomère 2-iodo-1-(phénylsulfonyl)-1*H*-pyrrolo[2,3-*c*]pyridine **3d** a conduit à la formation d'un mélange non-séparable du produit souhaité **3d** et un produit di-iodé **17d.** Le mélange a été ensuite isolé par extraction et engagé dans une réaction de désulfonylation selon la **procédure D** pour fournir le produit **18d** avec un rendement de 47% sur 2 étapes. Ce dernier a été protégé selon la **procédure A2** afin d'obtenir le produit souhaité **3d.**

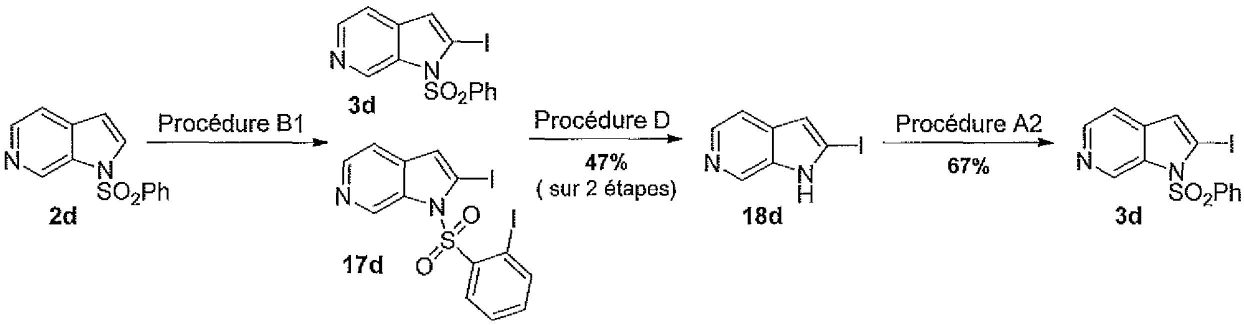


Le dérivé iodé du 6-chloro-7-azaindole **3e** a été préparé selon la **procédure B1** avec un rendement modéré de 46%, ce dernier a pu être amélioré jusqu'à 65% en utilisant la **procédure B2.**

En revanche, son analogue bromé **3j** a été obtenu avec un excellent rendement de 93% selon la **procédure B2.**

La **procédure B2** au départ des composés **2f-h** fournit les dérivés fluorés du 7-azaindole **3f-h** en mélange avec avec des produits di-iodés **17f-h** et **19f-h.** Après application de la **procédure D** de désulfonylation, les dérivés **18f-h** ont été isolés seuls et utilisés directement dans les réactions de couplage de Sonogashira.

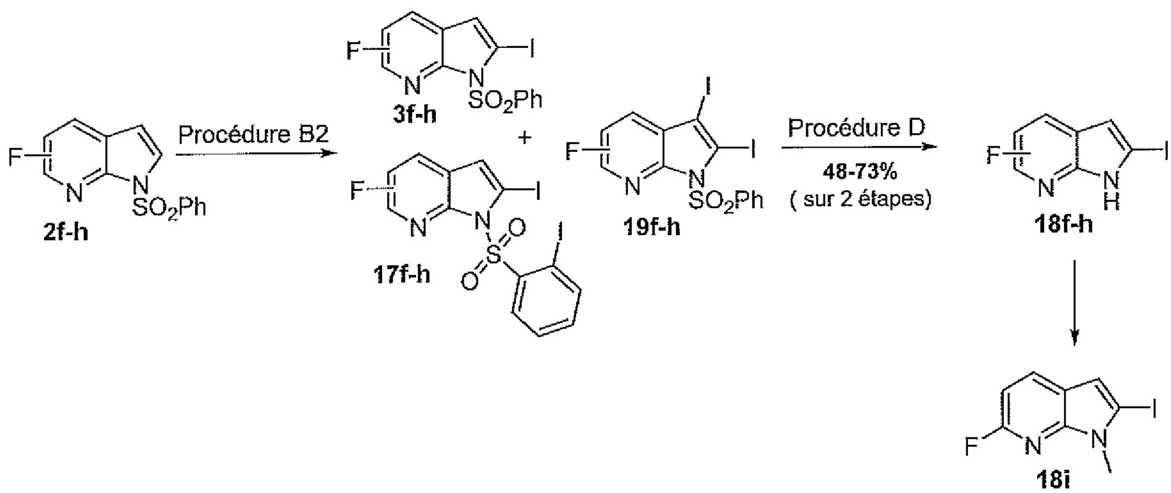


La désulfonylation des azaindoles de type **3a** été achevée selon la **procédure D,** les produits de type **18** ont alors subi une étape de protection par un groupement protecteur Boc générant **20 (procédure E).**

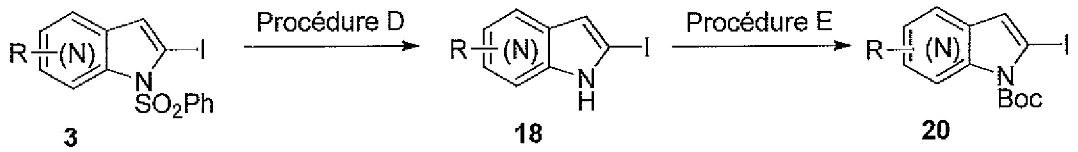


**3** **18** **20**

R = H, Cl

**Tableau 4. Dérivés iodés d'azaindoles obtenus selon les procédures D ou E.**

| | | | |
|---|---|---|---|
| 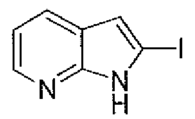 | 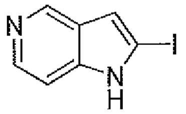 | 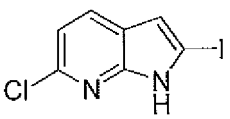 | 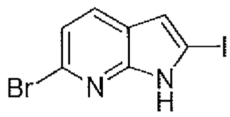 |
| **18a, 85%** *Procédure D* | **18b, 65%** *Procédure D* | **18e, 61%** *Procédure D* | **18j, 87%** *Procédure D* |
| 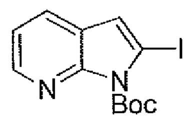 | 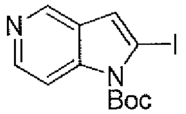 | 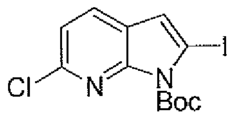 | 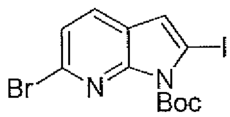 |
| **20a, 94%** *Procédure E* | **20b, 88%** *Procédure E* | **20e, 99%** *Procédure E* | **20j, 99%** *Procédure E* |

**Exemple 10.1 2-Iodo-1-(phénylsulfonyl)-1*H*-pyrrolo[3,2-*b*]pyridine 3c.**

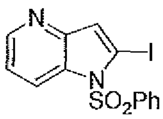

$C_{13}H_9IN_2O_2S$ MW: 384,19 g.mol$^{-1}$

Le composé a été préparé selon la **procédure B1** et purifié par colonne de chromatographie flash sur gel de silice (acétate d'éthyle/éther de pétrole = 25/75). Solide jaune (70%), **Pf** 138-140°C, $\boldsymbol{R_f}$ = 0.27 (acétate d'éthyle/éther de pétrole = 30/70). **Infrarouge** (ν, cm$^{-1}$, neat) 3704, 3061, 2360, 1566, 1502, 1479, 1450, 1405, 1375, 1268, 1213, 1191, 1166, 1125, 1090, 998, 802, 781, 756, 724, 682, 668, 584, 558, 524, 509. **RMN $^1$H** (250 MHz, $CDCl_3$, 20°C) δ 8.59 - 8.45 (m, 2H), 7.95 - 7.83 (m, 2H), 7.61 (m, 1H), 7.52 - 7.40 (m, 2H), 7.21 (m, 2H). **RMN $^{13}$C** (63 MHz, $CDCl_3$, 20°C) δ 149.1 (Cq), 146.6 (CH), 137.9 (Cq), 134.5 (CH), 132.3 (Cq), 129.4 (2×CH), 127.3 (2xCH), 124.8 (CH), 122.5

(CH), 119.3 (CH), 81.0 (Cq). **HRMS (+ESI)** calculée pour $C_{13}H_{10}IN_2O_2S$ (M+H+) : 384.9502, trouvée: 384.9503.

**Exemple 10.2 2-Iodo-1-(phénylsulfonyl)-1*H*-pyrrolo[2,3-*c*]pyridine 3d.**

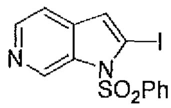

$C_{13}H_9IN_2O_2S$ MW: 384,19 g.mol-1

Le composé a été préparé selon la **procédure A2** et purifié par colonne de chromatographie flash sur gel de silice (acétate d'éthyle/éther de pétrole = 30/70). Solide jaune (67%), **Pf** 149-151°C, $R_f$ = 0.34 (acétate d'éthyle/éther de pétrole = 1/1). **Infrarouge** (v, cm-1, neat) 3091, 2919, 2842, 2735, 1639, 1612, 1592, 1540, 1480, 1450, 1416, 1379, 1319, 1272, 1205, 1170, 1124, 1109, 1091, 1049, 1031, 1014, 996, 899, 834, 819, 755, 725, 684, 668, 607, 559, 514. **RMN $^1$H** (250 MHz, $CDCl_3$, 20°C) δ 9.53 (t, *J* = 0.9 Hz, 1H), 8.39 (d, *J* = 5.3 Hz, 1H), 8.03 - 7.86 (m, 2H), 7.61 (m, 1H), 7.55 - 7.41 (m, 2H), 7.35 (dd, *J* = 5.3 Hz, *J* = 1.1 Hz, 1H), 7.00 (d, *J* = 0.8 Hz, 1H). **RMN $^{13}$C** (63 MHz, $CDCl_3$, 20°C) δ 142.8 (CH), 137.7 (Cq), 137.2 (CH), 136.7 (Cq), 135.6 (Cq), 134.5 (CH), 129.4 (2×CH), 127.4 (2×CH), 122.4 (CH), 113.7 (CH), 81.9 (Cq). **HRMS (+ESI)** calculée pour $C_{13}H_{10}IN_2O_2S$ (M+H+) : 384.9502, trouvée: 384.9507.

**Exemple 10.3 6-Chloro-2-iodo-1-(phénylsulfonyl)-1*H*-pyrrolo[2,3-*b*]pyridine 3e.**

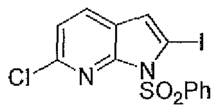

$C_{13}H_8ClIN_2O_2S$ MW: 418,64 g.mol-1

Le composé a été préparé selon la **procédure B2** et purifié par colonne de chromatographie flash sur gel de silice (acétate d'éthyle/éther de pétrole = 10/90). Solide blanc (46%), **Pf** 176-178°C, $R_f$ = 0.39 (acétate d'éthyle/éther de pétrole = 10/90). **Infrarouge** (v, cm-1, neat) 3115, 2954, 1907, 1570, 1486, 1435, 1374, 1336, 1277, 1240, 1207, 1177, 1126, 1089, 1012, 928, 825, 756, 724, 684, 631, 588, 558, 545, 521. **RMN $^1$H** (250 MHz, $CDCl_3$, 20°C) δ 8.32 - 8.19 (m, 2H), 7.70 - 7.41 (m, 4H), 7.14 (d, *J* = 8.2 Hz, 1H), 6.95 (s, 1H). **RMN $^{13}$C** (63 MHz, $CDCl_3$, 20°C) δ 1477 (Cq), 146.1 (Cq), 138.1 (Cq), 134.4 (CH), 129.6 (CH), 129.0 (2×CH), 128.5 (2xCH), 122.2 (Cq), 119.7 (CH), 119.4 (CH), 75.9 (Cq). **HRMS (+ESI)** calculée pour $C_{13}H_9ClIN_2O_2S$ (M+H+) : 418.9113, trouvée: 418.9111.

**Exemple 10.4 6-Bromo-2-iodo-1-(phénylsulfonyl)-1*H*-pyrrolo[2,3-*b*]pyridine 3j**

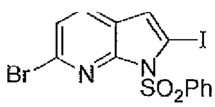

$C_{13}H_8BrIN_2O_2S$ MW: 463,09 g.mol-1

Le composé a été préparé selon la **procédure B2** et purifié par colonne de chromatographie sur gel de silice sous pression (le rapport de solvants utilisé : acétate d'éthyle/éther de pétrole = 10/90). Solide blanc (93%), **Pf** 205-207°C, $R_f$ = 0.43 (acétate d'éthyle/éther de pétrole = 10/90). **Infrarouge** (v, cm-1, neat) 3383, 2359, 1635, 1588, 1567, 1485, 1449, 1430, 1370, 1333, 1275, 1235, 1207, 1175, 1129, 1114, 1087, 1011, 918, 826, 755, 723, 697, 684, 623, 585, 557, 537, 502. **RMN $^1$H** (250 MHz, $CDCl_3$, 20°C) δ 8.35 - 8.01 (m, 2H), 7.69 - 7.37 (m, 4H), 7.25 (m, 1H), 6.91 (s, 1H). **RMN $^{13}$C** (63 MHz, $CDCl_3$, 20°C) δ 148.0 (Cq), 138.1 (Cq), 136.1 (Cq), 134.4 (CH), 129.4 (CH), 129.0 (2xCH), 128.7 (2xCH), 123.3 (CH), 122.4 (Cq), 119.5 (CH), 76.0 (Cq). **HRMS (+ESI)** calculée pour $C_{13}H_9BrIN_2O_2S$ (M+H+) : 464.8587, trouvée: 464.8581.

**Exemple 10.5 2-Iodo-1*H*-pyrrolo[2,3-*b*]pyridine 18a.**

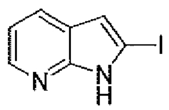

$C_7H_5IN_2$ MW: 244,03 g.mol$^{-1}$

Le composé a été préparé selon la **procédure D** et purifié par colonne de chromatographie flash sur gel de silice (acétate d'éthyle/éther de pétrole = 10/90). Solide blanc (85%), **Pf** 189-191°C, $R_f$ = 0.43 (acétate d'éthyle/éther de pétrole = 20/80). **Infrarouge** (ν, cm$^{-1}$, neat) 2790, 1580, 1481, 1431, 1403, 1336, 1308, 1275, 911, 812, 762, 622, 510. **RMN $^1$H** (400 MHz, DMSO-$d_6$, 20°C) δ 12.20 (s, 1H), 8.13 (dd, *J* = 4.7 Hz, *J* = 1.6 Hz, 1H), 7.86 (dd, *J* = 7.8 Hz, *J* = 1.6 Hz, 1H), 7.02 (dd, *J* = 7.8 Hz, *J* = 4.7 Hz, 1H), 6.70 (s, 1H). **RMN $^{13}$C** (101 MHz, DMSO-$d_6$, 20°C) δ 151.0 (Cq), 142.9 (CH), 126.9 (CH), 122.1 (Cq), 116.3 (CH), 110.0 (CH), 81.1 (Cq). **HRMS (+ESI)** calculée pour $C_7H_6$IN2 (M+H$^+$) : 244.9570, trouvée: 244.9570.

**Exemple 10.6 2-Iodo-1*H*-pyrrolo[3,2-*c*]pyridine 18b.**

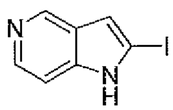

$C_7H_5IN_2$ MW: 244,03 g.mol$^{-1}$

Le composé a été préparé selon la **procédure D** et isolé par précipitation dans l'$Et_2O$ avec une filtration. Solide blanc (65%), **Pf** 247-249°C, $R_f$ = 0.19 (acétate d'éthyle/éther de pétrole = 30/70). **Infrarouge** (ν, cm$^{-1}$, neat) 3349, 3120, 3080, 3044, 2930, 2770, 2733, 2690, 2644, 1668, 1606, 1574, 1504, 1447, 1423, 1324, 1292, 1248, 1201, 1182, 1168, 1101, 1028, 948, 908, 838, 808, 796, 753, 629, 604, 537, 522, 518, 514, 511. **RMN $^1$H** (400 MHz, DMSO-$d_6$, 20°C) δ 12.08 (s, 1H), 8.73 (s, 1H), 8.09 (d, *J* = 5.6 Hz, 1H), 7.29 (d, *J* = 5.6 Hz, 1H), 6.81 (s, 1H). **RMN $^{13}$C** (101 MHz, DMSO-$d_6$, 20°C) δ $^{13}$C NMR (101 MHz, DMSO) δ 142.3 (Cq), 141.6 (CH), 140.6 (CH), 127.0 (Cq), 110.3 (CH), 106.3 (CH), 81.5 (Cq). **HRMS (+ESI)** calculée pour $C_7H_6IN_2$ (M+H$^+$) : 244.9570, trouvée: 244.9568.

**Exemple 10.7 2-Iodo-1*H*-pyrrolo[3,2-*b*]pyridine 18c.**

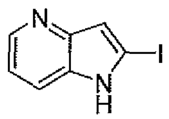

$C_7H_5IN_2$ MW: 244,03 g.mol$^{-1}$

Le composé a été préparé selon la **procédure D** et purifié par colonne de chromatographie flash sur gel de silice (acétone/dichlorométhane = 3/97). Solide blanc (73%), **Pf** 192-194°C, $R_f$ = 0.31 (acétone /dichlorométhane = 2/98). **Infrarouge** (ν, cm$^{-1}$, neat) 3052, 2950, 2871, 2644, 1613, 1563, 1504, 1481, 1432, 1393, 1352, 1309, 1282, 1195, 1118, 908, 769, 622, 578, 512, 502. **RMN $^1$H** (400 MHz, DMSO-$d_6$, 20°C) δ 11.91 (s, 1H), 8.26 (dd, *J* = 4.6 Hz, *J* = 1.4 Hz, 1H), 7.68 (dd, *J* = 8.3 Hz, *J* = 1.4 Hz, 1H), 7.03 (dd, *J* = 8.3 Hz, *J* = 4.6 Hz, 1H), 6.80 (s, 1H). **RMN $^{13}$C** (101 MHz, DMSO-$d_6$, 20°C) δ 147.7 (Cq), 143.2 (CH), 132.0 (Cq), 117.8 (CH), 116.8 (CH), 111.6 (CH), 84.6 (Cq). **HRMS (+ESI)** calculée pour $C_7H_6IN_2$ (M+H$^+$) : 244.9570, trouvée: 244.9573.

**Exemple 10.82-Iodo-1*H*-pyrrolo[2,3-*c*]pyridine 18d**

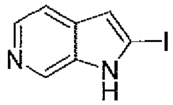

$C_7H_5IN_2$ MW: 244,04 g.mol$^{-1}$

Le composé a été préparé selon la **procédure B1** suivie de la **procédure D** et purifié par colonne de chromatographie flash sur gel de silice (acétate d'éthyle). Solide jaune (47% sur 2 étapes), **Pf** 210-212°C, $\boldsymbol{R_f}$ = 0.26 (acétate d'éthyle/éther de pétrole = 1/1). **Infrarouge** (v, cm$^{-1}$, neat) 2583, 1611, 1516, 1433, 1400, 1297, 1218, 1155, 1031, 910, 817, 630, 598, 512. **RMN $^1$H** (400 MHz, DMSO-$d_6$, 20°C) δ 12.21 (large s, 1H), 8.64 (s, 1H), 8.04 (m, 1H), 7.45 (d, *J* = 5.3 Hz, 1H), 6.73 (s, 1H). **RMN $^1$H $^{13}$C** (101 MHz, DMSO-$d_6$, 20°C) δ 138.5 (Cq), 136.7 (Cq), 134.0 (Cq), 133.4 (CH), 113.5 (CH), 110.5 (2×CH). **HRMS (+ESI)** calculée pour $C_7H_5IN_2$ (M+H$^+$) : 244.9570, trouvée: 244.9573.

**Exemple 10.9 6-Chloro-2-iodo-1*H*-pyrrolo[2,3-*b*]pyridine 18e.**

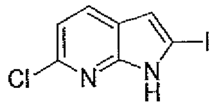

$C_7H_4ClIN_2$ MW: 278,48 g.mol$^{-1}$

Le composé a été préparé selon la **procédure D** et purifié par colonne de chromatographie flash sur gel de silice (acétate d'éthyle/éther de pétrole = 3/97). Solide blanc (61%), **Pf** 208-210°C, $\boldsymbol{R_f}$ = 0.40 (acétate d'éthyle/éther de pétrole = 3/97). **Infrarouge** (v, cm$^{-1}$, neat) 3103, 3024, 2938, 2852, 2806, 1596, 1575, 1474, 1409, 1387, 1332, 1285, 1265, 1124, 1111, 1092, 965, 933, 812, 752, 690, 630, 607, 517, 506. **RMN $^1$H** (400 MHz, DMSO-$d_6$, 20°C) δ 12.44 (s, 1H), 7.92 (d, *J* = 8.2 Hz, 1H), 7.09 (d, *J* = 8.2 Hz, 1H), 6.76 (s, 1H). **RMN $^{13}$C** (101 MHz, DMSO-$d_6$, 20°C) δ 149.6 (Cq), 143.5 (Cq), 130.1 (CH), 121.0 (Cq), 116.2 (CH), 110.4 (CH), 81.8 (Cq). **HRMS (+ESI)** calculée pour $C_7H_5ClIN_2$ (M+H$^+$) : 278.9181, trouvée: 278.9177.

**Exemple 10.10 6-Fluoro-2-iodo-1*H*-pyrrolo[2,3-*b*]pyridine 18f.**

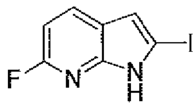

$C_7H_4FIN_2$ MW: 262,02 g.mol$^{-1}$

Le composé a été préparé selon la séquence des **procédures B2** et **D** et purifié par colonne de chromatographie flash sur gel de silice (acétate d'éthyle/éther de pétrole = 3/97). Solide blanc (56% sur 2 étapes), **Pf** 172-174°C, $\boldsymbol{R_f}$ **=** 0.34 (acétate d'éthyle/éther de pétrole = 5/95). **Infrarouge** (v, cm$^{-1}$, neat) 3134, 1589, 1424, 1343, 1272, 1199, 1105, 995, 811, 757, 614. **RMN $^1$H** (250 MHz, DMSO-$d_6$, 20°C) δ 12.34 (s, 1H), 8.02 (t, *J*= 8.3 Hz, 1H), 6.79 (d, *J*= 8.3 Hz, 1H), 6.76 (s, 1H). **RMN $^{13}$C** (63 MHz, DMSO-$d_6$, 20°C) δ 161.3 (Cq), 147. 0 (d, *J* = 18.8 Hz, Cq), 132.2 (d, *J* = 9.4 Hz, CH), 120.2 (Cq), 110.5 (CH), 101.6 (d, *J* = 38.9 Hz, CH), 79.4 (Cq). **RMN $^{19}$F** (376 MHz, DMSO-$d_6$, 20°C) δ - 76.0. **HRMS (+ESI)** calculée pour $C_7R_5FIN_2$ (M+H$^+$) : 262.9476, trouvée: 262.9475.

**Exemple 10.11 5-Fluoro-2-iodo-1*H*-pyrrolo[2,3-*b*]pyridine 18g.**

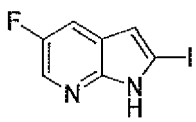

$C_7H_4FIN_2$ MW: 262,02 g.mol$^{-1}$

Le composé a été préparé selon la séquence des **procédures B2** et **D** et purifié par colonne de chromatographie flash sur gel de silice (acétate d'éthyle/éther de pétrole = 5/95). Solide blanc (48% sur 2 étapes), **Pf** 199-201°C, $\boldsymbol{R_f}$ **=** 0.32 (acétate d'éthyle/éther de pétrole = 10/90). **Infrarouge** (v, $cm^{-1}$, neat) 3105, 3038, 2952, 2911, 2863, 2786, 2738, 2709, 2361, 1759, 1609, 1586, 1565, 1500, 1425, 1387, 1321, 1290, 1237, 1194, 1093, 1009, 978, 897, 877, 802, 760, 630, 590, 541, 505. **RMN $^1$H** (250 MHz, DMSO-$d_6$, 20°C) δ 12.38 (s, 1H), 8.13 (m, 1H), 7.77 (dd, *J* = 9.3 Hz, *J* = 2.4 Hz, 1H), 6.71 (m, 1H). **RMN $^{13}$C** (101 MHz, DMSO-$d_6$, 20°C) δ 155.3 (d, *J* = 239.7 Hz, Cq), 147.9 (Cq), 131.0 (d, *J* = 29.0 Hz, CH), 122.0 (d, *J* = 7.3 Hz, Cq), 112.5 (d, *J* = 20.9 Hz, CH), 110.0 (d, *J* = 4.0 Hz, CH), 83.9 (Cq). **RMN $^{19}$F** (376 MHz, DMSO-$d_6$, 20°C) δ -139.0. **HRMS (+ESI)** calculée pour $C_7H_5FIN_2$ (M+H$^+$) : 262.9476, trouvée: 262.9473.

**Exemple 10.12 4-fluoro-2-iodo-1*H*-pyrrolo[2,3-*b*]pyridine 18h**

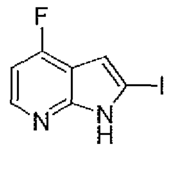

$C_7H_4FIN_2$ MW: 262,02 g.mol$^{-1}$

Le composé a été préparé selon la sequence des **procédures B2** et **D** et purifié par colonne de chromatographie flash sur gel de silice (acétate d'éthyle/éther de pétrole = 20/80). Solide blanc (73% sur 2 étapes), **Pf** 218-220°C, $\boldsymbol{R_f}$ **=** 0.26 (acétate d'éthyle/éther de pétrole = 20/80). **Infrarouge** (v, $cm^{-1}$, neat) 2724, 1628, 1570, 1511, 1479, 1432, 1395, 1336, 1311, 1273, 1253, 1113, 1049, 874, 806, 746, 616, 511. **RMN $^1$H** (400 MHz, DMSO-$d_6$, 20°C) δ 12.57 (s, 1H), 8.14 (s, 1H), 6.92 (d, *J* = 4.0 Hz, 1H), 6.78 (s, 1H). **RMN $^{13}$C** (101 MHz, DMSO-$d_6$, 20°C) δ 159.67 (d, *J* = 260.5 Hz, Cq), 154.4 (d, *J* = 11.4Hz, Cq), 145.0 (d, *J* = 6.1 Hz, CH), 110.9 (d, *J* = 18.0 Hz, Cq), 105.6 (CH), 102.8 (d, *J* = 14.8 Hz, CH), 81.6 (Cq). **RMN $^{19}$F** (376 MHz, DMSO-$d_6$, 20°C) δ -112.3. **HRMS (+ESI)** calculée pour $C_7H_5FIN_2$ (M+H$^+$) : 262.9476, trouvée: 262.9475.

**Exemple 10.13 6-Fluoro-2-iodo-1-méthyl-1*H*-pyrrolo[2,3-*b*]pyridine 18i**

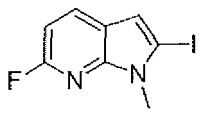

$C_8H_6FIN_2$

MW: 276,05 g.mol$^{-1}$

Le composé a été préparé selon la **procédure C** et purifié par colonne de chromatographie flash sur gel de silice (acétate d'éthyle/éther de pétrole = 1/99). Solide blanc (90%), **Pf** 94-96°C, $\boldsymbol{R_t}$ = 0.27 (acétate d'éthyle/éther de pétrole = 5/95). **Infrarouge** (v, $cm^{-1}$, neat) 3111, 1602, 1574, 1472, 1441, 1404, 1333, 1301, 1265, 1230, 1125, 1091, 1041, 973, 820, 777, 748, 690, 580, 564, 516, 506, 503. **RMN $^1$H** (400 MHz, $CDCl_3$, 20°C) δ 7.84 (m, 1H), 6.79 (s, 1H), 6.66 (dd, *J* = 8.4 Hz, *J* = 1.1 Hz, 1H), 3.78 (s, 3H). **RMN $^{13}$C** (101 MHz, $CDCl_3$, 20°C) δ 159.9 (d, *J* = 237.5 Hz, Cq), 145.1 (d, *J* = 17.5 Hz, Cq), 131.7 (d, *J* = 9.4 Hz, CH), 119.5 (d, *J* = 2.9 Hz, Cq), 110.3 (CH), 101.6 (d, *J* = 38.8 Hz, CH), 83.4 (d, *J* = 4.1 Hz, Cq), 32.6 ($CH_3$). **RMN $^{19}$F** (376 MHz, $CDCl_3$, 20°C) δ -75.0. **HRMS (+ESI)** calculée pour $C_8H_7FIN_2$ (M+H$^+$) : 276.9633, found: 276.9633.

**Exemple 10.14 6-Bromo-2-iodo-1*H*-pyrrolo[2,3-*b*]pyridine 18j**

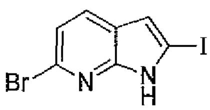

$C_7H_4BrIN_2$

MW: 322,93 g.mol$^{-1}$

Le composé a été préparé selon la **procédure D** et purifié par colonne de chromatographie sur gel de silice

sous pression (le rapport de solvants utilisé : acétate d'éthyle/éther de pétrole = 5/95). Solide blanc (81%), **Pf** 238-240°C, $\boldsymbol{R_f}$ = 0.24 (acétate d'éthyle/éther de pétrole = 5/95). **Infrarouge** (v, $cm^{-1}$, neat) 3898, 3851, 3819, 3800, 3749, 3742, 3731, 3687, 3674, 3668, 3646, 3627, 3565, 3099, 3019, 2930, 2844, 2798, 2588, 2216, 1912, 1595, 1567, 1473, 1406, 1382, 1330, 1282, 1263, 1206, 1120, 1098, 964, 953, 920, 813, 751, 670, 621, 603, 536, 524, 517, 506. **RMN $^1$H** (400 MHz, DMSO-$d_6$, 20°C) δ 12.46 (s, 1H), 7.83 (d, *J* = 8.2 Hz, 1H), 7.21 (d, *J* = 8.2 Hz, 1H), 6.76 (s, 1H). **RMN $^{13}$C** (101 MHz, DMSO-$d_6$, 20°C) δ 150.1 (Cq), 134.0 (Cq), 129.9 (CH), 121.2 (Cq), 119.7 (CH), 110.5 (CH), 81.9 (Cq). **HRMS (+ESI)** calculée pour $C_7H_5BrIN_2$ (M+H$^+$) : 324.8655, trouvée: 324.8652.

**Exemple 10.15 5-Bromo-2-iodo-1*H*-pyrrolo[2,3-*b*]pyridine 18k**

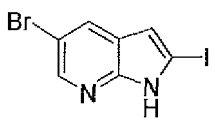

$C_7H_4BrIN_2$

MW: 322,93 g.mol$^{-1}$

Le composé a été préparé selon la sequence des **procédures B1** et **D** et purifié par colonne de chromatographie sur gel de silice sous pression (le rapport de solvants utilisé : (méthanol /dichlorométhane = 5/95). Solide blanc (3% sur 2 étapes), **Pf** 253-255°C, $\boldsymbol{R_f}$ = 0.57 (méthanol /dichlorométhane = 5/95). **Infrarouge** (v, $cm^{-1}$, neat) 2687, 1567, 1467, 1416, 1390, 1326, 1278, 1229, 924, 882, 805, 753, 683. **RMN $^1$H** (400 MHz, DMSO-$d_6$, 20°C) δ 12.46 (s, 1H), 8.19 (s, 1H), 8.11 (s, 1H), 6.69 (s, 1H). **RMN $^{13}$C** (101 MHz, DMSO-$d_6$, 20°C) δ 149.4 (Cq), 142.8 (CH), 128.8 (CH), 123.8 (Cq), 111.6 (Cq), 109.7 (CH), 83.8 (Cq). **HRMS (+ESI)** calculée pour $C_7H_5BrIN_2$ (M+H$^+$) : 322.8675, trouvée: 322.8675.

**Exemple 10.16 4-Chloro-2-iodo-1*H*-pyrrolo[2,3-b]pyridine 181**

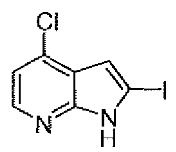

$C_7H_4ClIN_2$

MW: 278,48 g.mol$^{-1}$

Le composé a été préparé selon la séquence des **procédures B1** et **D** et purifié par précipitation de l'$Et_2O$. Solide blanc (58% sur 2 étapes), **Pf** 231-233°C, **Infrarouge** (v, $cm^{-1}$, neat) 2722, 1602, 1568, 1480, 1428, 1387, 1326, 1307, 1265, 1183, 961, 905, 850, 801, 740, 664, 615, 522, 510, 508. **RMN $^1$H** (250 MHz, DMSO-$d_6$, 20°C) δ 12.60 (s, 1H), 8.10 (d, *J* = 5.2 Hz, 1H), 7.16 (d, *J* = 5.2 Hz, 1H), 6.75 (s, 1H). **RMN $^{13}$C** (63 MHz, DMSO-$d_6$, 20°C) δ 151.1 (Cq), 143.2 (CH), 132.0 (Cq), 120.5 (Cq), 115.7 (CH), 107.5 (CH), 82.5 (Cq). **HRMS (+ESI)** calculée pour $C_7H_5ClIN_2$ (M+H$^+$) : 278.9181, trouvée: 278.9184.

**Exemple 10.17 *tert*-Butyl 2-iodo-1*H*-pyrrolo[2,3-*b*]pyridine-1-carboxylate 20a.**

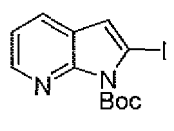

$C_{12}H_{13}IN_2O_2$

MW: 344,15 g.mol$^{-1}$

Le composé a été préparé selon la **procédure E** et purifié par colonne de chromatographie flash sur gel de silice (acétate d'éthyle/éther de pétrole = 10/90). Solide blanc (99%), **Pf** 74-76°C, $\boldsymbol{R_f}$ = 0.40 (acétate d'éthyle/éther de pétrole = 20/80). **Infrarouge** (v, $cm^{-1}$, neat) 3067, 2981, 1725, 1572, 1504, 1460, 1392, 1369, 1345, 1324, 1304, 1252, 1206, 1154, 1132, 1106, 1066, 1044, 912, 839, 805, 765, 665, 611, 589, 517, 511. **RMN $^1$H** (400 MHz, $CDCl_3$, 20°C) δ 8.36 (dd, *J* = 4.8 Hz, *J* = 1.6 Hz, 1H), 7.72 (dd, *J* = 7.8 Hz, *J* = 1.6 Hz, 1H), 7.08 (dd, *J* = 7.8 Hz, *J* = 4.8 Hz, 1H), 6.87 (s, 1H), 1.70 (s, 9H). **RMN $^{13}$C** (101 MHz, $CDCl_3$, 20°C) δ 149.7 (Cq), 147.7 (Cq), 144.7 (CH), 127.3 (CH), 123.3 (Cq),

118.6 (CH), 118.0 (CH), 85.8 (Cq), 77.0 (Cq), 28.2 (3×$CH_3$). **HRMS (+ESI)** calculée pour $C_{12}H_{14}IN_2O_2$(M+H$^+$) : 345.0095, trouvée: 345.0094.

**Exemple 10.18 *tert*-Butyl 2-iodo-1*H*-pyrrolo[3,2-*c*]pyridine-1-carboxylate 20b.**

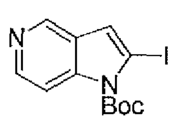

$C_{12}H_{13}IN_2O_2$

MW: 344,15 g.mol$^{-1}$

Le composé a été préparé selon la **procédure E** et purifié par colonne de chromatographie flash sur gel de silice (acétate d'éthyle/éther de pétrole = 20/80). Solide blanc (88%), **Pf** 82-84°C, ***R*$_f$** = 0.19 (acétate d'éthyle/éther de pétrole = 20/80). **Infrarouge** (ν, cm$^{-1}$, neat) 3041, 2983, 1733, 1592, 1504, 1479, 1447, 1432, 1394, 1367, 1331, 1316, 1271, 1254, 1178, 1150, 1056, 914, 844, 816, 768, 746, 644, 595, 544, 521, 505. **RMN $^1$H** (250 MHz, $CDCl_3$, 20°C) δ 8.66 (d, *J* = 1.0 Hz, 1H), 8.27 (dd, *J* = 5.9 Hz, *J* = 1.0 Hz, 1H), 7.81 (dd, *J* = 5.9 Hz, *J* = 0.9 Hz, 1H), 6.92 (t, *J* = 0.9 Hz, 1H), 1.62 (s, 9H). **RMN $^{13}$C** (63 MHz, $CDCl_3$, 20°C) δ 148.6 (Cq), 144.0 (CH), 142.0 (CH), 141.6 (Cq), 127.6 (Cq), 120.0 (CH), 110.2 (CH), 86.5 (Cq), 28.3 (3×$CH_3$). **HRMS (+ESI)** calculée pour $C_{12}H_{14}IN_2O_2$(M+H$^+$) : 345.0095, trouvée: 345.0093.

**Exemple 10.19 *tert*-Butyl 6-chloro-2-iodo-1*H*-pyrrolo[2,3-*b*]pyridine-1-carboxylate 20e.**

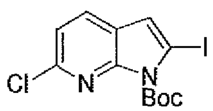

$C_{12}H_{12}ClIN_2O_2$

MW: 378,59 g.mol$^{-1}$

Le composé a été préparé selon la **procédure E** et purifié par colonne de chromatographie flash sur gel de silice (acétate d'éthyle/éther de pétrole = 1/99). Solide blanc (99%), **Pf** 81-83°C, ***R*$_f$** = 0.16 (acétate d'éthyle/éther de pétrole = 1/99). **Infrarouge** (ν, cm$^{-1}$, neat) 2986, 2973, 2929, 1746, 1592, 1568, 1493, 1474, 1457, 1436, 1392, 1367, 1352, 1311, 1276, 1251, 1157, 1127, 1101, 1068, 1037, 937, 844, 817, 772, 749, 738, 712, 640, 627, 589, 551, 517, 509, 503. **RMN $^1$H** (400 MHz, $CDCl_3$, 20°C) δ 7.69 (d, *J* = 8.2 Hz, 1H), 7.15 (d, *J* = 8.2 Hz, 1H), 6.91 (s, 1H), 1.72 (s, 9H). **RMN $^{13}$C** (101 MHz, $CDCl_3$, 20°C) δ 147.8 (Cq), 147.6 (Cq), 146.1 (Cq), 129.4 (CH), 121.9 (Cq), 119.1 (CH), 117.6 (CH), 86.1 (Cq), 77.1 (Cq), 28.1 (3×$CH_3$). **HRMS (+ESI)** calculée pour $C_7H_5ClIN_2$ (M+H$^+$) : 378.9705, trouvée: 378.9704.

**Exemple 10.20 *tert*-butyl 6-bromo-2-iodo-1*H*-pyrrolo[2,3-*b*]pyridine-1-carboxylate 20j**

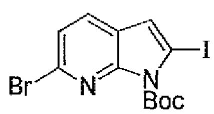

$C_{12}H_{12}BrIN_2O_2$

MW: 423,04 g.mol$^{-1}$

Le composé a été préparé selon la **procédure E** et purifié par colonne de chromatographie sur gel de silice sous pression (le rapport de solvants utilisé : acétate d'éthyle/éther de pétrole = 1/99). Solide orange (81%), **Pf** 69-71°C, ***R*$_f$** = 0.38 (acétate d'éthyle/éther de pétrole = 1/99). **Infrarouge** (ν, cm$^{-1}$, neat) 2972, 1746, 1588, 1561, 1489, 1431, 1390, 1367, 1349, 1308, 1275, 1250, 1156, 1136, 1096, 1065, 924, 843, 816, 771, 749, 734, 695, 517, 505, 503. **RMN $^1$H** (400 MHz, $CDCl_3$, 20°C) δ 7.60 (d, *J* = 8.1 Hz, 1H), 7.29 (d, *J* = 8.1 Hz, 1H), 6.89 (s, 1H), 1.72 (s, 9H). **RMN $^{13}$C** (101 MHz, $CDCl_3$, 20°C) δ 148.0 (Cq), 147.7 (Cq), 136.3 (Cq), 129.1 (CH), 122.6 (Cq), 122.1 (CH), 117.7 (CH), 86.1 (Cq), 77.2 (Cq), 28.1 (3×$CH_3$). **HRMS (+ESI)** calculée pour $C_{12}H_{13}BrIN_2O_2$ (M+H$^+$) : 424.9180, trouvée: 424.9178.

**Exemple 11. Synthèse de composés finaux et leurs analogues protégés**

Les produits finaux de type **22** ont été préparés par 3 méthodes différentes :

soit par le couplage de Sonogashira à partir de dérivés iodés protégés par un benzènesulfonyle de type **3** et avec une déprotection du produit intermédiaire **21** (méthode 1) ;

soit par le couplage de Sonogashira directement à partir de dérivés iodés non-protégés de type **18** (méthode 2) ;

soit par le couplage de Sonogashira à partir de dérivés iodés protégés par un *tert*-butoxycarbonyle de type **20** et avec une déprotection de produit intermédiaire **23** (méthode 3)

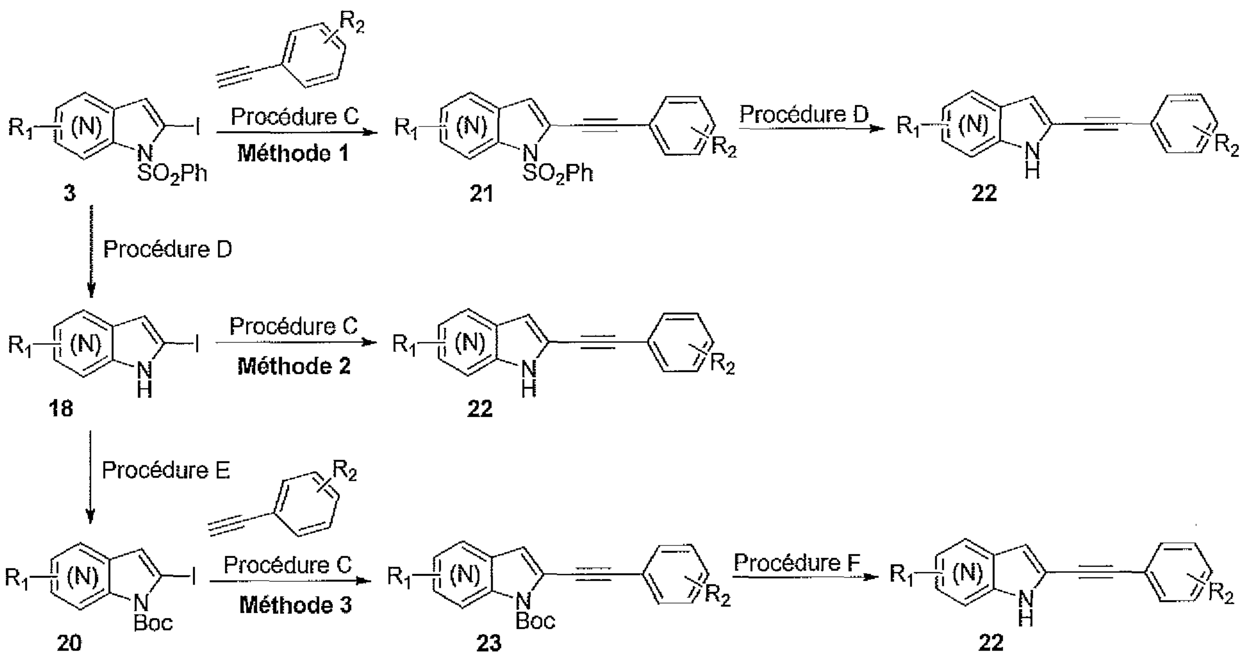


**Exemple 11.1 *N,N*-Diméthyl-4-((1-(phénylsulfonyl)-1*H*-pyrrolo[2,3-*b*]pyridin-2-yl)éthynyl)aniline 21a.**

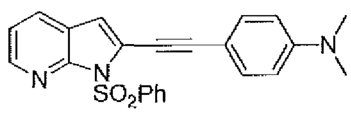

$C_{23}H_{19}N_3O_2S$ MW: 401,48 g.mol$^{-1}$

Le composé a été préparé selon la **procédure C2** et purifié par colonne de chromatographie flash sur gel de silice (acétate d'éthyle/éther de pétrole = 10/90). Solide jaune (62%), **Pf** 185-187°C, ***R*$_f$ =** 0.18 (acétate d'éthyle/éther de pétrole = 10/90). **Infrarouge** (ν, cm$^{-1}$, neat) 2918, 2194, 1732, 1605, 1578, 1545, 1517, 1474, 1447, 1398, 1374, 1311, 1267, 1226, 1183, 1128, 1114, 1089, 1071, 1054, 1036, 997, 941, 871, 829, 811, 726, 683, 661, 626. **RMN $^1$H** (250 MHz, $CDCl_3$, 20°C) δ 8.47 (dd, *J* = 4.8 Hz, *J* = 1.6 Hz, 1H), 8.28 - 8.17 (m, 2H), 7.73 (dd, *J* = 7.9 Hz, *J* = 1.6 Hz, 1H), 7.57-7.48 (m, 3H), 7.47 - 7.37 (m, 2H), 7.16 (dd, *J* = 7.9 Hz, *J* = 4.8 Hz, 1H), 6.75 (s, 1H), 6.73 - 6.63 (m, 2H), 3.02 (s, 6H). **RMN $^{13}$C** (63 MHz, $CDCl_3$, 20°C) δ 150.7 (Cq), 148.3 (CH), 145.3 (Cq), 139.2 (Cq), 133.8 (CH), 132.9 (2xCH), 129.0 (2×CH), 128.6 (CH), 127.8 (2×CH), 122.6 (Cq), 121.8 (Cq), 119.4 (CH), 111.8 (2×CH), 111.5 (CH), 108.7 (Cq), 99.7 (Cq), 78.6 (Cq), 40.15 (2×$CH_3$). **HRMS (+ESI)** calculée pour $C_{23}H_{20}N_3O_2S$ (M+H$^+$) : 402.1271, trouvé: 402.1274.

**Exemple 11.2 *N,N*-Diméthyl-4-((1-phénylsulfonyl)-1*H*-pyrrolo[3,2-*b*]pyridin-2-yl)éthynyl)aniline 21c.**

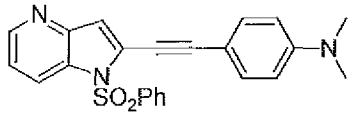

$C_{23}H_{19}N_3O_2S$ MW: 401,48 g.mol-1

Le composé a été préparé selon la **procédure C2** et purifié par colonne de chromatographie flash sur gel de silice (acétate d'éthyle/éther de pétrole = 30/70). Huile brune (98%), ***R*f** = 0.22 (acétate d'éthyle/éther de pétrole = 30/70). **Infrarouge** (ν, $cm^{-1}$, neat) 2924, 2200, 1733, 1605, 1566, 1549, 1516, 1446, 1405, 1367, 1268, 1225, 1169, 1127, 1089, 1043, 945, 816, 727, 685, 583, 559, 506, 503. **RMN $^1$H** (400 MHz, $CDCl_3$, 20°C) δ 8.56 (dd, *J* = 4.7 Hz, *J* = 1.1 Hz, 1H), 8.51 (d, *J* = 8.4 Hz, 1H), 8.01-7.96 (m, 2H), 7.60 - 7.51 (m, 3H), 7.43 (m, 2H), 7.29 (m, 1H), 7.04 (m, 1H), 6.72 (d, *J* = 9.0 Hz, 2H), 3.06 (s, 6H). **RMN $^{13}$C** (101 MHz, $CDCl_3$, 20°C) δ 150.8 (Cq), 147.3 (Cq), 146.9 (CH), 138.4 (Cq), 134.2 (CH), 133.0 (2×CH), 130.3 (Cq), 129.2 (2×CH), 127.2 (2×CH), 125.3 (Cq), 121.7 (CH), 119.5 (CH), 115.4 (CH), 111.8 (2×CH), 108.2 (Cq), 101.3 (Cq), 78.2 (Cq), 40.1 (2×$CH_3$). **HRMS (+ESI)** calculée pour $C_{23}H_{20}N_3O_2S$ (M+H$^+$) : 402.1271, found: 402.1271.

**Exemple 11.3 *N,N*-Diméthyl-4-((1-(phénylsulfonyl)-1*H*-pyrrolo[2,3-*c*]pyridin-2-yl)éthynyl)aniline 21d.**

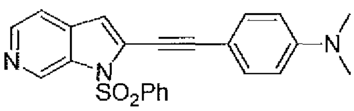

$C_{23}H_{19}N_3O_2S$ MW: 401,48 g.mol-1

Le composé a été préparé selon la **procédure C** et purifié par colonne de chromatographie flash sur gel de silice (acétate d'éthyle/éther de pétrole = 30/70). Solide marron (80%), **Pf** 168-170°C, ***R*f** = 0.34 (acétate d'éthyle/éther de pétrole = 30/70). **Infrarouge** (ν, $cm^{-1}$, neat) 3058, 2205, 1679, 1603, 1536, 1446, 1428, 1351, 1272, 1224, 1183, 1123, 1088, 1050, 836, 817, 729, 680, 654, 574, 556, 512. **RMN $^1$H** (250 MHz, $CDCl_3$, 20°C) δ 9.55 (s, 1H), 8.43 (d, *J* = 5.1 Hz, 1H), 8.12 - 7.96 (m, 2H), 7.64 - 7.50 (m, 3H), 7.47-7.37 (m, 3H), 6.79 (s, 1H), 6.72 (d, *J* = 8.9 Hz, 2H), 3.05 (s, 6H). **RMN $^{13}$C** (63 MHz, $CDCl_3$, 20°C) δ 150.9 (Cq), 142.9 (Cq), 138.3 (Cq), 136.7 (CH), 134.8 (Cq), 134.2 (CH), 133.1 (2xCH), 129.3 (2×CH), 127.3 (2×CH), 125.2 (Cq), 114.9 (CH), 113.2 (CH), 111.7 (2×CH), 107.9 (Cq), 101.0 (Cq), 78.0 (Cq), 40.1 (2×$CH_3$). **HRMS (+ESI)** calculée pour $C_{23}H_{20}N_3O_2S$ (M+H$^+$) : 402.1271, trouvée: 402.1273.

**Exemple 11.4 4-((6-chloro-1-(phénylsulfonyl)-1*H*-pyrrolo[2,3-*b*]pyridin-2-yl)éthynyl)-*N,N*-diméthylaniline 21e.**

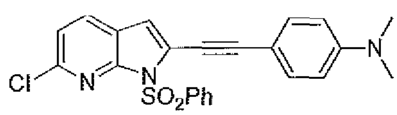

$C_{23}H_{18}ClN_3O_2S$ MW: 435,93 g.mol-1

Le composé a été préparé selon la **procédure C** et purifié par colonne de chromatographie flash sur gel de silice (acétate d'éthyle/éther de pétrole = 5/95). Solide marron (99%), **Pf** 195-197°C, ***R*f** = 0.41 (acétate d'éthyle/éther de pétrole = 10/90). **Infrarouge** (ν, $cm^{-1}$, neat) 2204, 1608, 1567, 1544, 1439, 1379, 1361, 1312, 1279, 1246, 1180, 1111, 1093, 1042, 812, 736, 686, 617, 577, 561, 515, 506, 503. **RMN $^1$H** (250 MHz, $CDCl_3$, 20°C) δ 8.22 (m, 2H), 7.63 (dd, *J* = 8.2 Hz, *J* = 1.1 Hz, 1H), 7.57 - 7.38 (m, 5H), 7.13 (dd, *J* = 8.2 Hz, *J* = 1.1 Hz, 1H), 6.74 - 6.55 (m, 3H), 2.99 (s, 6H). **RMN $^{13}$C** (101 MHz, $CDCl_3$, 20°C) δ δ 150.7 (Cq), 146.7 (Cq), 146.6 (Cq), 138.8 (Cq), 134.1 (CH), 133.0 (2×CH), 130.5 (CH), 129.0 (2×CH), 128.2 (2×CH), 123.0 (Cq), 120.1 (Cq), 119.9 (CH), 111.8 (2×CH), 110.7 (CH), 108.6 (Cq), 100.0 (Cq), 78.3 (Cq), 40.2 (2×$CH_3$). **HRMS (+ESI)** calculée pour $C_{23}H_{19}ClN_3O_2S$ (M+H$^+$) : 436.0881, trouvée: 436.0881.

**Exemple 11.5 3-((1-(phénylsulfonyl)-1*H*-pyrrolo[2,3-*b*]pyridin-2-yl)éthynyl)aniline 21f.**

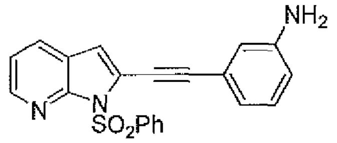

$C_{21}H_{15}N_3O_2S$ MW: 373,43 g.mol$^{-1}$

Le composé a été préparé selon la **procédure C** et purifié par colonne de chromatographie flash sur gel de silice (acétate d'éthyle/éther de pétrole = 10/90). Solide marron (94%), **Pf** 156-158°C, $R_f$ = 0.32 (acétate d'éthyle/éther de pétrole = 10/90). **Infrarouge** (v, cm$^{-1}$, neat) 3431, 3325, 3228, 3061, 2360, 2210, 1637, 1598, 1575, 1486, 1447, 1394, 1377, 1333, 1262, 1176, 1127, 1088, 1033, 993, 916, 860, 822, 782, 764, 724, 683, 570, 553, 509. **RMN $^1$H** (400 MHz, $CDCl_3$, 20°C) $\delta$ 8.52 (dd, *J* = 4.8 Hz, *J* = 1.6 Hz, 1H), 8.25 (dd, *J* = 8.4 Hz, *J* = 1.4 Hz, 2H), 7.80 (dd, *J* = 7.9 Hz, *J* = 1.6 Hz, 1H), 7.58 (m, 1H), 7.48 (dd, *J* = 8.5 Hz, *J* = 7.1 Hz, 2H), 7.21 (ddd, *J* = 7.8 Hz, *J* = 6.3 Hz, *J* = 1.4 Hz, 2H), 7.09 (dt, *J* = 7.7 Hz, *J* = 1.2 Hz, 1H), 7.02 (t, *J* = 1.9 Hz, 1H), 6.87 (s, 1H), 6.76 (ddd, *J* = 7.9 Hz, *J* = 2.4 Hz, *J* = 1.0 Hz, 1H), 3.79 (s, 2H). **RMN $^{13}$C** (101 MHz, $CDCl_3$, 20°C) $\delta$ 148.5 (Cq), 146.6 (Cq), 146.1 (CH), 139.2 (Cq), 134.1 (CH), 129.6 (CH), 129.2 (2×CH), 129.1 (CH), 128.0 (2×CH), 123.2 (Cq), 122.1 (CH), 121.9 (Cq), 121.6 (Cq), 119.7 (CH), 117.8 (CH), 116.3 (CH), 113.1 (CH), 98.2 (Cq), 79.7 (Cq). **HRMS (+ESI)** calculée pour $C_{21}H_{16}N_3O_2S$ (M+H$^+$) : 374.0958, trouvée: 374.0957.

**Exemple 11.6 4-((1*H*-Pyrrolo[2,3-*b*]pyridin-2-yl)éthynyl)-*N,N*-diméthylaniline 22a.**

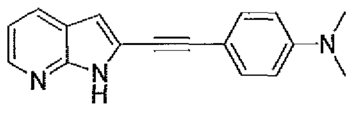

$C_{17}H_{15}N_3$ MW: 261,32 g.mol$^{-1}$

Le composé a été préparé selon la **procédure E** et purifié par colonne de chromatographie flash sur gel de silice (acétate d'éthyle/éther de pétrole = 15/85). Solide jaune (92%), **Pf** 229-231°C, $R_f$ = 0.36 (acétate d'éthyle/éther de pétrole = 30/70). **Infrarouge** (v, cm$^{-1}$, neat) 3055, 2886, 2796, 2198, 1603, 1584, 1538, 1509, 1433, 1405, 1354, 1326, 1281, 1223, 1186, 1165, 1118, 1061, 976, 945, 918, 807, 763, 699, 645, 625. **RMN $^1$H** (400 MHz, DMSO-$d_6$, 20°C) $\delta$ 12.07 (s, 1H), 8.20 (m, 1H), 7.92 (dd, *J* = 7.8 Hz, *J* = 1.7 Hz, 1H), 7.39 (d, *J* = 8.4 Hz, 2H), 7.08 (dd, *J* = 7.8 Hz, *J* = 4.6 Hz, 1H), 6.84 - 6.46 (m, 3H), 2.97 (s, 6H). **RMN $^{13}$C** (100 MHz, DMSO-$d_6$, 20°C) $\delta$ 150.8 (Cq), 148.8 (Cq), 144.3 (CH), 132.9 (2×CH), 128.4 (CH), 120.6 (Cq), 120.2 (Cq), 116.6 (CH), 112.4 (2×CH), 108.0 (Cq), 105.4 (CH), 94.9 (Cq), 80.5 (Cq), 40.1 (2×$CH_3$). **HRMS (+ESI)** calculée pour $C_{17}H_{16}N_3$ (M+H$^+$) : 262.1339, trouvée: 262.1340.

**Exemple 11.7 4-((1*H*-Pyrrolo[2,3-*c*]pyridin-2-yl)éthynyl)-*N,N*-diméthylaniline 22b.**

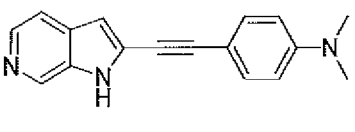

$C_{17}H_{15}N_3$ MW: 261,32 g.mol$^{-1}$

Le composé a été préparé selon la **procédure D** et purifié par colonne de chromatographie flash sur gel de silice (acétate d'éthyle/éther de pétrole = 20/80). Solide jaune (88%), **Pf** 242-244°C, $R_f$ = 0.27 (acétate d'éthyle/éther de pétrole = 30/70). **Infrarouge** (v, cm$^{-1}$, neat) 2669, 2207, 1609, 1573, 1535, 1361, 1303, 1224, 1187, 906, 811, 598, 515. **RMN $^1$H** (250 MHz, DMSO-$d_6$, 20°C) $\delta$ 12.08 (large s, 1H), 8.67 (s, 1H), 8.10 (d, *J* = 5.5 Hz, 1H), 7.49 (dd, *J* = 5.5 Hz, *J* = 1.0 Hz, 1H), 7.45 - 7.36 (m, 2H), 6.79-6.71 (m, 3H), 2.98 (s, 6H). **RMN $^{13}$C** (63 MHz, DMSO-$d_6$, 20°C) $\delta$ 151.0(Cq), 138.6 (CH), 134.4 (CH), 133.0 (2×CH), 132.2 (Cq), 123.6(Cq), 112.4 (2×CH), 107.5(Cq), 105.8 (CH), 95.1(Cq), 80.1(Cq), 40.1 (2×$CH_3$). **HRMS (+ESI)** calculée pour $C_{17}H_{16}N_3$ (M+H$^+$) : 262.1339, trouvée: 262.1338.

**Exemple 11.8 4-((1*H*-Pyrrolo[3,2-*b*]pyridin-2-yl)éthynyl)-*N,N*-diméthylaniline 22c.**

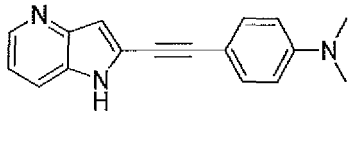

$C_{17}H_{15}N_3$ MW: 261,32 g.mol$^{-1}$

Le composé a été préparé selon la **procédure E** et purifié par colonne de chromatographie flash sur gel de silice (acétate d'éthyle/éther de pétrole = 30/70). Solide orange (87%), **Pf** 191-193°C, $\boldsymbol{R_f}$ = 0.24 (acétate d'éthyle/éther de pétrole = 30/70). **Infrarouge** (ν, cm$^{-1}$, neat) 3064, 2889, 2806, 2703, 2199, 1673, 1606, 1570, 1537, 1512, 1406, 1355, 1286, 1223, 1186, 1122, 943, 913, 814, 778, 623, 558, 514. **RMN $^1$H** (400 MHz, DMSO-$d_6$, 20°C) δ 11.82 (s, 1H), 8.35 (dd, *J* = 4.6 Hz, *J* = 1.2 Hz, 1H), 7.69 (d, *J* = 8.1 Hz, 1H), 7.40 (t, *J* = 8.9 Hz, 2H), 7.14 (dd, *J* = 8.2 Hz, *J* = 4.6 Hz, 1H), 6.81 (d, *J* = 0.9 Hz, 1H), 6.75 (d, *J* = 8.9 Hz, 2H), 2.97 (s, 6H). **RMN $^{13}$C** (101 MHz, DMSO-$d_6$, 20°C) δ 150.9 (Cq), 145.9 (Cq), 143.8 (CH), 132.9 (2×CH), 129.7 (Cq), 123.2 (Cq), 118.4 (CH), 117.8 (CH), 112.4 (2×CH), 107.8 (Cq), 106.8 (CH), 95.4 (Cq), 80.4 (Cq), 40.1 (2×$CH_3$). **HRMS (+ESI)** calculée pour $C_{17}H_{16}N_3$ (M+H$^+$) : 262.1339, trouvée: 262.1342.

**Exemple 11.9 4-((1*H*-Pyrrolo[3,2-*c*]pyridin-2-yl)éthynyl)-*N,N*-diméthylaniline 22d.**

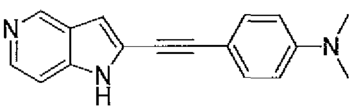

$C_{17}H_{15}N_3$ MW: 261,32 g.mol$^{-1}$

Le composé a été préparé selon la **procédure F** et purifié par colonne de chromatographie flash sur gel de silice (acétate d'éthyle). Solide orange (47%), Pf 250-252°C, $\boldsymbol{R_f}$ = 0.22 (acétate d'éthyle). **Infrarouge** (ν, cm$^{-1}$, neat) 2899, 2623, 2203, 1605, 1543, 1517, 1369, 1292, 1233, 1182, 814, 762, 519, 506. **RMN $^1$H** (400 MHz, DMSO-$d_6$, 20°C) δ 11.99 (s, 1H), 8.80 (s, 1H), 8.19 (d, *J* = 5.7 Hz, 1H), 7.43 - 7.36 (m, 2H), 7.29 (dt, *J* = 5.8 Hz, *J* = 1.0 Hz, 1H), 6.84 (s, 1H), 6.78 - 6.68 (m, 2H), 2.97 (s, 6H). **RMN $^{13}$C** (101 MHz, DMSO-$d_6$, 20°C) δ 150.86, 143.51 (CH), 141.58 (CH), 139.88, 132.91 (2×CH), 125.03, 121.04, 112.37 (2×CH), 107.83, 106.77 (CH), 105.86 (CH), 94.44, 80.12, 40.12 (2×$CH_3$). **HRMS (+ESI)** calculée pour $C_{17}H_{16}N_3$ (M+H$^+$) : 262.1339, trouvée: 262.1340.

**Exemple 11.10 4-((6-Chloro-1*H*-pyrrolo[2,3-*b*]pyridin-2-yl)éthynyl)-*N,N*-diméthylaniline 22e.**

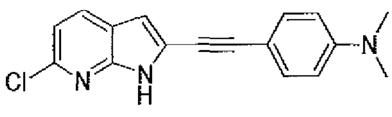

$C_{17}H_{14}ClN_3$

MW: 295,76 g.mol$^{-1}$

Le composé a été préparé selon la **procédure D** (32%) ou par la **procédure F** (35%) et purifié par colonne de chromatographie flash sur gel de silice (acétate d'éthyle/éther de pétrole = 5/95). Solide jaune. **Pf** 241-243°C, $\boldsymbol{R_f}$ = 0.32 (acétate d'éthyle/éther de pétrole = 10/90). **Infrarouge** (ν, cm$^{-1}$, neat) 2895, 2121, 1600, 1514, 1440, 1360, 1224, 1160, 942, 815, 510. **RMN $^1$H** (250 MHz, DMSO-$d_6$, 20°C) δ 12.27 (s, 1H), 7.93 (d, *J* = 8.2 Hz, 1H), 7.42 - 7.25 (m, 2H), 7.09 (d, *J* = 8.2 Hz, 1H), 6.75 - 6.64 (m, 3H), 2.92 (s, 6H). **RMN $^{13}$C** (63 MHz, DMSO-$d_6$, 20°C) δ 150.9 (Cq), 147.5 (Cq), 144.6 (Cq), 132.9 (2×CH), 131.5 (CH), 121.2 (Cq), 119.2 (Cq), 116.5 (CH), 112.4 (2×CH), 107.7 (Cq), 105.7 (CH), 95.4 (Cq), 80.1 (Cq), 40.1 (2×$CH_3$). **HRMS (+ESI)** calculée pour $C_{17}H_{15}ClN_3$ (M+H$^+$) : 296.0949, trouvée: 296.0946.

**Exemple 11.11 4-(6-Fluoro-1*H*-pyrrolo[2,3-*b*]pyridin-2-yl)éthynyl)-*N,N*-diméthylaniline 22f.**

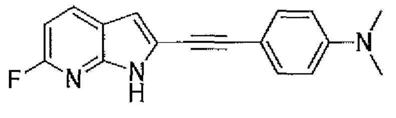

$C_{17}H_{14}FN_3$

MW: 279,31 g.mol$^{-1}$

Le composé a été préparé selon la **procédure C** et purifié par colonne de chromatographie flash sur gel de silice (acétate d'éthyle/éther de pétrole = 10/90). Solide jaune (62%), **Pf** 232-234°C, $\boldsymbol{R_f}$ = 0.33 (acétate d'éthyle/éther de pétrole = 10/90). **Infrarouge** (v, cm$^{-1}$, neat) 3157, 3072, 2893, 2205, 1605, 1541, 1511, 1432, 1273, 1224, 1183, 809, 763, 507. **RMN $^1$H** (400 MHz, DMSO-$d_6$, 20°C) δ 12.23 (s, 1H), 8.08 (t, *J* = 8.2 Hz, 1H), 7.38 (d, *J* = 8.3 Hz, 2H), 6.84 (d, *J* = 8.3 Hz, 1H), 6.78 - 6.57 (m, 3H), 2.97 (s, 6H). **RMN $^{13}$C** (63 MHz, DMSO-$d_6$, 20°C) δ 160.6 (d, *J* = 233.8 Hz, Cq), 150.8 (Cq), 145.2 (d, *J* = 19.3 Hz, Cq), 133.7 (d, *J* = 9.5 Hz, CH), 132.9 (2×CH), 120.2 (d, *J* = 4.5 Hz, Cq), 118.1 (d, *J* = 2.7 Hz, Cq), 112.4 (2×CH), 107.9 (Cq), 105.9 (CH), 102.04 (d, *J* = 38.8 Hz, CH), 94.7 (Cq), 80.1 (Cq), 40.1 (2×$CH_3$). **RMN $^{19}$F** (376 MHz, DMSO-$d_6$, 20°C) δ -74.40. **HRMS (+ESI)** calculée pour $C_{17}H_{15}FN_3$ (M+H$^+$) : 280.1245, trouvée: 280.1243.

**Exemple 11.12 4-((5-Fluoro-1*H*-pyrrolo[2,3-*b*]pyridin-2-yl)éthynyl)-*N,N*-diméthylaniline 22g.**

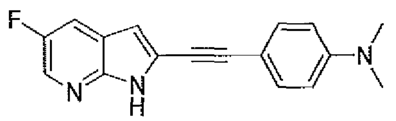

$C_{17}H_{14}FN_3$

MW: 279,31 g.mol$^{-1}$

Le composé a été préparé selon la **procédure C** et purifié par colonne de chromatographie flash sur gel de silice (acétate d'éthyle/éther de pétrole = 15/85). Solide jaune (68%), **Pf** 255-257°C, $\boldsymbol{R_f}$ = 0.25 (acétate d'éthyle/éther de pétrole = 20/80). **Infrarouge** (v, cm$^{-1}$, neat) 3120, 2987, 2897, 2801, 2360, 2209, 1609, 1583, 1541, 1503, 1447, 1399, 1362, 1345, 1293, 1230, 1189, 1151, 1109, 1066, 976, 944, 875, 817, 761, 559, 519, 509. **RMN $^1$H** (400 MHz, DMSO-$d_6$, 20°C) δ 12.23 (s, 1H), 8.20 (m, 1H), 7.80 (dd, *J* = 9.5 Hz, *J* = 2.8 Hz, 1H), 7.39 (d, *J* = 8.4 Hz, 2H), 6.75 (d, *J* = 8.4 Hz, 2H), 6.67 (m, 1H), 2.98 (s, 6H). **RMN $^{13}$C** (101 MHz, DMSO, 20°C) δ 155. 7 (d, *J* = 239.5 Hz, Cq), 150.9 (Cq), 145.6 (Cq), 132.9 (2×CH), 132.4 (d, *J* = 29.0 Hz, CH), 122.9 (Cq), 120.3 (d, *J* = 7.5 Hz, Cq), 113.7 (d, *J* = 20.7 Hz, CH), 112.4 (2xCH), 107.7 (Cq), 105.4 (d, *J* = 4.5 Hz, CH), 95.5 (Cq), 80.2 (Cq), 40.1 (2×$CH_3$). **RMN $^{19}$F** (376 MHz, DMSO-$d_6$, 20°C) δ -138.76. **HRMS (+ESI)** calculée pour $C_{17}H_{15}FN_3$ (M+H$^+$) : 280.1245, trouvée: 280.1243.

**Exemple 11.13 4-((4-Fluoro-1*H*-pyrrolo[2,3-*b*]pyridin-2-yl)éthynyl)-*N,N*-diméthylaniline 22h.**

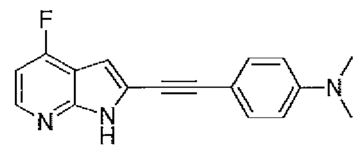

$C_{17}H_{14}FN_3$

MW: 279,31 g.mol$^{-1}$

Le composé a été préparé selon la **procédure C** et purifié par colonne de chromatographie flash sur gel de silice (acétate d'éthyle/éther de pétrole = 20/80). Solide blanc (73%), **Pf** 248-250°C, $\boldsymbol{R_f}$ = 0.31 (acétate d'éthyle/éther de pétrole = 20/80). **Infrarouge** (v, cm$^{-1}$, neat) 3066, 2900, 2803, 2359, 2201, 1604, 1540, 1510, 1441, 1362, 1345, 1326, 1299, 1274, 1250, 1231, 1185, 1170, 1078, 1058, 980, 946, 881, 796, 753, 618, 594, 530, 510. **RMN $^1$H** (400 MHz, DMSO-$d_6$, 20°C) δ 12.45 (s, 1H), 8.25 (dd, *J* = 8.2 Hz, *J* = 5.3 Hz, 1H), 7.40 (d, *J* = 8.3 Hz, 2H), 6.98 (dd, *J* = 10.3 Hz, J=5.4 Hz, 1H), 6.82 - 6.67 (m, 3H), 2.97 (s, 6H). **RMN $^{13}$C** (101 MHz, DMSO-$d_6$, 20°C) δ 161.2 (d, *J* = 260.9 Hz, Cq), 152.0 (d, *J* = 11.4 Hz, Cq), 150.92 (Cq), 146.3 (d, *J* = 6.1 Hz, CH), 133.0 (2xCH), 120.9 (Cq), 112.4 (2×CH), 109.2 (d, *J* = 18.1 Hz, Cq), 107.6 (Cq), 102.9 (d, *J* = 14.6 Hz, CH), 100.9 (CH), 95.2 (Cq), 79.8 (Cq), 40.1 (2×$CH_3$). **RMN $^{19}$F** (376 MHz, DMSO-$d_6$, 20°C) δ -111.96. **HRMS (+ESI)** calculée pour $C_{17}H_{15}FN_3$ (M+H$^+$) : 280.1245, trouvée: 280.1246.

**Exemple 11.14 4-((1*H*-pyrrolo[2,3-*b*]pyridin-2-yl)éthynyl)-*N*-méthylaniline 22i.**

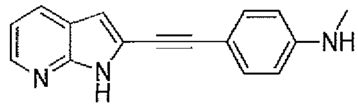

$C_{16}H_{13}N_3$

MW: 247,29 g.mol$^{-1}$

Le composé a été préparé selon la **procédure C** et purifié par colonne de chromatographie flash sur gel de silice (acétate d'éthyle/éther de pétrole = 20/80). Solide beige (87%), **Pf** 208-210°C, $R_f$ = 0.19 (acétate d'éthyle/éther de pétrole = 20/80). **Infrarouge** (ν, cm$^{-1}$, neat) 3409, 3056, 2195, 1602, 1532, 1509, 1478, 1431, 1406, 1358, 1324, 1282, 1177, 1157, 1123, 917, 823, 802, 764, 625, 550, 542, 527, 519, 512, 506. **RMN $^1$H** (400 MHz, DMSO-$d_6$, 20°C) δ 12.04 (s, 1H), 8.23 (d, *J* = 3.6 Hz, 1H), 7.91 (d, *J* = 7.6 Hz, 1H), 7.31 (d, *J* = 8.5 Hz, 2H), 7.07 (dd, *J* = 7.7 Hz, *J* = 3.6 Hz, 1H), 6.69 (d, *J* = 1.1 Hz, 1H), 6.57 (d, *J* = 8.5 Hz, 2H), 6.23 (d, *J* = 4.9 Hz, 1H), 2.72 (d, *J* = 4.9 Hz, 3H). **RMN $^{13}$C** (101 MHz, DMSO-$d_6$, 20°C) δ 150.9 (Cq), 148.7 (Cq), 144.2 (CH), 133.0 (2×CH), 128.3 (CH), 120.7 (Cq), 120.2 (Cq), 116.6 (CH), 112.0 (2×CH), 107.6 (Cq), 105.3 (CH), 95.2 (Cq), 80.0 (Cq), 29.7 ($CH_3$). **HRMS (+ESI)** calculée pour $C_{16}H_{14}N_3$ (M+H$^+$) : 248.1182, trouvée: 248.1183.

**Exemple 11.15 3-((1*H*-pyrrolo[2,3-*b*]pyridin-2-yl)éthynyl)aniline 22j.**

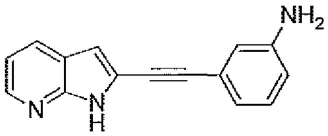

$C_{15}H_{11}N_3$

MW: 233,27 g.mol$^{-1}$

Le composé a été préparé selon la **procédure E** et purifié par colonne de chromatographie flash sur gel de silice (acétate d'éthyle/éther de pétrole = 30/70). Solide blanc (67%), **Pf** 196-198°C, $R_f$ = 0.14 (acétate d'éthyle/éther de pétrole = 30/70). **Infrarouge** (ν, cm$^{-1}$, neat) 3465, 3373, 3201, 3075, 3053, 2975, 2895, 2817, 2746, 1601, 1579, 1535, 1488, 1448, 1430, 1407, 1361, 1312, 1283, 1190, 1169, 1115, 992, 916, 852, 801, 767, 735, 681, 634, 621, 570, 526, 513, 503. **RMN $^1$H** (250 MHz, DMSO-$d_6$, 20°C) δ 12.17 (s, 1H), 8.28 (d, *J* = 3.8 Hz, 1H), 7.95 (d, *J* = 7.7 Hz, 1H), 7.11 (d, *J* = 7.8 Hz, 2H), 6.91 - 6.45 (m, 4H), 5.31 (s, 2H). **RMN $^{13}$C** (63 MHz, DMSO-$d_6$, 20°C) δ 149.4 (Cq), 148.8 (Cq), 144.7 (CH), 129.8 (CH), 128.8 (CH), 122.3 (Cq), 20.0 (Cq), 119.7 (Cq), 119.1 (CH), 116.8 (CH), 116.4 (CH), 115.4 (CH), 106.4 (CH), 94.2 (Cq), 81.2 (Cq). **HRMS (+ESI)** calculée pour $C_{15}H_{12}N_3$ (M+H$^+$) : 234.1026, trouvée: 234.1025.

**Exemple 11.16 2-((3-fluorophényl)éthynyl)-1*H*-pyrrolo[2,3-*b*]pyridine 22k**

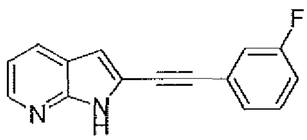

$C_{15}H_9FN_2$

MW: 236,24 g.mol$^{-1}$

Le composé a été préparé selon la **procédure C** et purifié par colonne de chromatographie sur gel de silice flash (acétate d'éthyle/éther de pétrole = 20/80). Solide blanc (64%), **Pf** 220-222°C, $R_f$ = 0.24 (acétate d'éthyle/éther de pétrole = 20/80). **Infrarouge** (ν, cm$^{-1}$, neat) 3056, 2817, 1607, 1577, 1481, 1434, 1406, 1360, 1283, 1174, 1112, 917, 807, 767, 676, 572, 517, 507. **RMN $^1$H** (400 MHz, DMSO-$d_6$, 20°C) δ 12.26 (s, 1H), 8.30 (dd, *J* = 4.6 Hz, *J* = 1.4 Hz, 1H), 7.97 (dd, *J* = 7.9 Hz, *J* = 1.4 Hz, 1H), 7.51 (dd, *J* = 14.1 Hz, *J* = 7.7 Hz, 1H), 7.46 - 7.39 (m, 2H), 7.37 - 7.28 (m, 1H), 7.11 (dd, *J* = 7.9 Hz, *J* =4.7 Hz, 1H), 6.86 (s, 1H). **RMN$^{13}$C** (101 MHz, DMSO-$d_6$, 20°C) δ 162.4 (d, *J* = 244.9 Hz), 148.8 (Cq), 145.2 (CH), 131.5 (d, *J* = 8.9 Hz, CH), 129.1 (CH), 128.2 (d, *J* = 2.9 Hz, CH), 124.1 (d, *J* = 9.7 Hz, Cq), 119.9 (Cq), 118.8 (Cq), 118.3 (d, *J* = 23.1 Hz, CH), 117.0 (d, *J* = 21.1 Hz, CH), 116.9 (CH), 107.3 (CH), 91.9 (d, *J* = 3.4 Hz, Cq), 83.6 (Cq). **RMN $^{19}$F** (376 MHz, DMSO-$d_6$, 20°C) δ -112.1. **HRMS (+ESI)** calculée pour $C_{15}H_{10}FN_2$ (M+H$^+$) :

237.0823, found: 237.0821.

**Exemple 11.17 4-((1*H*-pyrrolo[2,3-*b*]pyridin-2-yl)éthynyl)aniline 221**

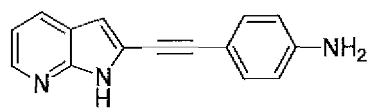

$C_{15}H_{11}N_3$

MW: 233,27 g.mol-1

Le composé a été préparé selon la **procédure C** et purifié par colonne de chromatographie sur gel de silice flash (acétate d'éthyle/éther de pétrole = 40/60). Solide jaune (87%), **Pf** 244-246°C, $R_f$ = 0.19 (acétate d'éthyle/éther de pétrole = 40/60). **Infrarouge** (v, $cm^{-1}$, neat) 3471, 3375, 3052, 2879, 2199, 1616, 1603, 1585, 1538, 1503, 1405, 1356, 1299, 1278, 1175, 917, 818, 761, 626, 568, 514, 511, 507, 503. **RMN $^1$H** (400 MHz, DMSO-$d_6$, 20°C) δ 12.04 (s, 1H), 8.23 (dd, *J* = 4.6 Hz, *J* = 1.4 Hz, 1H), 7.90 (m, 1H), 7.24 (d, *J* = 8.5 Hz, 2H), 7.07 (dd, *J* = 7.9 Hz, *J* = 4.7 Hz, 1H), 6.68 (d, *J* = 1.7 Hz, 1H), 6.59 (d, *J* = 8.5 Hz, 2H), 5.65 (s, 2H). **RMN$^{13}$C** (101 MHz, DMSO-$d_6$, 20°C) δ 150.3 (Cq), 148.7 (Cq), 144.2 (CH), 133.0 (2xCH), 128.3 (CH), 120.6 (Cq), 120.1 (Cq), 116.6 (CH), 114.1 (2×CH), 107.7 (Cq), 105.2 (CH), 95.2 (Cq), 79.7 (Cq). **HRMS** (+ESI) calculée pour $C_{15}H_{12}N_3$ (M+H$^+$) : 234.1026, found: 234.1025.

**Exemple 11.18 2-((4-méthoxyphényl)éthynyl)-1*H*-pyrrolo[2,3-*b*]pyridine 22m**

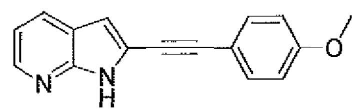

$C_{16}H_{12}N_2O$

MW: 248,28 g.mol-1

Le composé a été préparé selon la **procédure C** et purifié par colonne de flash chromatographie sur gel de silice (acétate d'éthyle/éther de pétrole = 30/70). Solide jaune (83%), **Pf** 193-195°C, $R_f$ = 0.26 (acétate d'éthyle/éther de pétrole = 30/70). Infrarouge (v, $cm^{-1}$, neat) 3054, 2976, 2832, 1600, 1583, 1536, 1501, 1436, 1404, 1358, 1280, 1242, 1171, 1107, 1027, 917, 812, 768, 723, 625, 562, 537, 507, 503. **RMN $^1$H** (400 MHz, DMSO-$d_6$, 20°C) δ 12.15 (s, 1H), 8.27 (dd, *J* = 4.7 Hz, *J* = 1.4 Hz, 1H), 7.95 (dd, *J* = 7.9 Hz, *J* = 1.2 Hz, 1H), 7.53 (d, *J* = 8.8 Hz, 2H), 7.09 (dd, *J* = 7.9 Hz, *J* = 4.7 Hz, 1H), 7.03 (d, *J* = 8.8 Hz, 2H), 6.78 (d, *J* = 1.6 Hz, 1H), 3.82 (s, 3H). **RMN $^{13}$C** (101 MHz, DMSO-$d_6$, 20°C) δ 160.3 (Cq), 148.8 (Cq), 144.7 (CH), 133.4 (2×CH), 128.7 (CH), 120.0 (Cq), 119.8 (Cq), 116.8 (CH), 115.0 (2xCH), 114.0 (Cq), 106.2 (CH), 93.3 (Cq), 81.3 (Cq), 55.8 ($CH_3$). **HRMS (+ESI)** calculée pour $C_{16}H_{13}N_2O$ (M+H$^+$) : 249.1022, found: 249.1021.

**Exemple 11.19 4-((6-fluoro-1-méthyl-1*H*-pyrrolo[2,3-*b*]pyridin-2-yl)éthynyl)-*N,N*-diméthylaniline 22n**

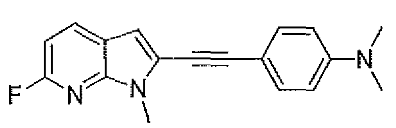

$C_{18}H_{16}FN_3$

MW: 293,34 g.mol-1

Le composé a été préparé selon la **procédure C** et purifié par colonne de chromatographie flash sur gel de silice (acétate d'éthyle/éther de pétrole = 2/98). Solide grisâtre (72%), **Pf** 194-196°C, $R_f$ = 0.24 (acétate d'éthyle/éther de pétrole = 4/96). **Infrarouge** (v, $cm^{-1}$, neat) 2896, 2202, 1606, 1577, 1539, 1505, 1445, 1406, 1358, 1316, 1278, 1230, 1191, 1092, 985, 947, 804, 760, 581, 517, 506, 502. **RMN $^1$H** (400 MHz, $CDCl_3$, 20°C) δ 7.90 (t, *J* = 8.0 Hz, 1H), 7.46 (d, *J* = 8.1 Hz, 2H), 6.84-6.65 (m, 4H), 3.90 (s, 3H), 3.04 (s, 6H). **RMN $^{13}$C** (101 MHz, $CDCl_3$, 20°C) δ 160.9 (d, *J* = 237.3 Hz, Cq), 150.6 (Cq), 144.8 (d, *J* = 18.0 Hz, Cq), 132.8 (2×CH), 132.8 (CH), 123.5 (d, *J* = 4.8 Hz, Cq), 117.6 (d, *J* = 2.7 Hz, Cq), 111.9 (2×CH), 108.8 (Cq), 104.5 (CH), 101.7 (d, *J* = 38.8 Hz, CH), 97.6 (Cq), 78.3 (Cq), 40.3 (2×$CH_3$), 29.5 ($CH_3$). **RMN $^{19}$F** (376 MHz, $CDCl_3$, 20°C) δ -74.2. **HRMS (+ESI)** calculée pour $C_{18}H_{17}FN_3$ (M+H$^+$) : 294.1401, found: 294.1401.

**Exemple 11.20 *tert*-Butyl 2-((4-(diméthylamino)phényl)éthynyl)-1*H*-pyrrolo[2,3-*b*]pyridine-1-carboxylate 23a.**

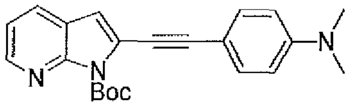

$C_{22}H_{23}N_3O_2$
MW: 361,44 g.mol$^{-1}$

Le composé a été préparé selon **la procédure C2** et purifié par colonne de chromatographie flash sur gel de silice (acétate d'éthyle/éther de pétrole = 15/85). Solide jaune (91%), **Pf** 188-190°C, $R_f$ = 0.39 (acétate d'éthyle/éther de pétrole = 20/80). **Infrarouge** (ν, cm$^{-1}$, neat) 3064, 2982, 2906, 2194, 1738, 1604, 1572, 1542, 1515, 1444, 1399, 1363, 1253, 1142, 1114, 1089, 974, 942, 840, 826, 813, 788, 773, 751, 697, 517, 504. **RMN $^1$H** (250 MHz, $CDCl_3$, 20°C) δ 8.53 (dd, *J* = 4.8Hz, *J* = 1.6 Hz, 1H), 7.83 (dd, *J* = 7.8 Hz, *J* = 1.6 Hz, 1H), 7.50 7.39 (m, 2H), 7.19 (dd, *J* = 7.8 Hz, *J* = 4.8 Hz, 1H), 6.82 (s, 1H), 6.76 - 6.62 (m, 2H), 3.03 (s, 6H), 1.71 (s, 9H). **RMN$^{13}$C** (63 MHz, $CDCl_3$, 20°C) δ 150.4 (Cq), 148.6 (Cq), 148.1 (Cq), 145.7 (CH), 132.7 (2×CH), 128.3 (CH), 122.0 (Cq), 121.6 (Cq), 118.8 (CH), 111.8 (2×CH), 111.6 (CH), 109.2 (Cq), 97.1 (Cq), 84.6 (Cq), 79.7 (Cq), 40.1 (2×$CH_3$), 28.2 (3×$CH_3$). **HRMS (+ESI)** calculée pour $C_{22}H_{24}N_3O_2$ (M+H$^+$) : 362.1863, trouvée: 362.1862.

**Exemple 11.21 *tert*-Butyl 2-((4-(diméthylamino)phényl)éthynyl)-1*H*-pyrrolo[3,2-*c*]pyridine-1-carboxylate 23b.**

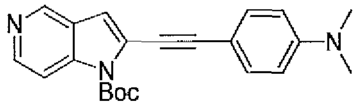

$C_{22}H_{23}N_3O_2$
MW: 361,44 g.mol$^{-1}$

Le composé a été préparé selon la **procédure C** et purifié par colonne de chromatographie flash sur gel de silice (acétate d'éthyle/éther de pétrole = 10/90). Solide jaune (99%), Pf 162-164°C, $R_f$ = 0.44 (acétate d'éthyle/éther de pétrole = 10/90). **Infrarouge** (ν, cm$^{-1}$, neat) 2983, 2205, 1733, 1604, 1549, 1517, 1456, 1338, 1317, 1247, 1149, 1092, 814, 507. **RMN $^1$H** (400 MHz, $CDCl_3$, 20°C) δ 8.82 (s, 1H), 8.45 (d, *J* = 5.8 Hz, 1H), 8.00 (d, *J* = 5.8 Hz, 1H), 7.43 (d, *J* = 7.9 Hz, 2H), 6.93 (s, 1H), 6.67 (d, *J* = 8.0 Hz, 2H), 3.00 (s, 6H), 1.69 (s, 9H). **RMN $^{13}$C** (101 MHz, $CDCl_3$, 20°C) δ 150.4 (Cq), 149.1 (Cq), 144.6 (CH), 143.2 (CH), 140.3 (Cq), 132.8 (2×CH), 125.4 (Cq), 122.8 (Cq), 112.9 (CH), 111.7 (2×CH), 110.3 (CH), 109.1 (Cq), 97.3 (Cq), 85.1 (Cq), 79.3 (Cq), 40.1 (2×$CH_3$), 28.2 (3×$CH_3$). **HRMS (+ESI)** calculée pour $C_{22}H_{24}N_3O_2$ (M+H$^+$): 362.1863, trouvée: 362.1862.

**Exemple 11.22 *tert*-Butyl 6-chloro-2-((4-(diméthylamino)phényl)éthynyl)-1*H*-pyrrolo[2,3-*b*]pyridine-1-carboxylate 23e.**

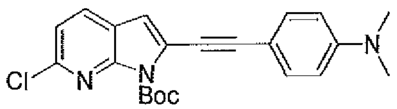

$C_{22}H_{22}ClN_3O_2$
MW: 395,88 g.mol$^{-1}$

Le composé a été préparé selon la **procédure C** et purifié par colonne de chromatographie flash sur gel de silice (acétate d'éthyle/éther de pétrole = 10/90). Solide jaune (93%), **Pf** 160-162°C, $R_f$ = 0.38 (acétate d'éthyle/éther de pétrole = 20/80). **Infrarouge** (ν, cm$^{-1}$, neat) 2923, 2853, 2196, 1740, 1611, 1567, 1546, 1517, 1439, 1397, 1368, 1334, 1306, 1268, 1245, 1186, 1152, 1140, 1110, 944, 881, 842, 813, 770, 744, 722, 602, 529. **RMN$^1$H** (250 MHz, $CDCl_3$, 20°C) δ 7.71 (d, *J* = 8.3 Hz, 1H), 7.40 (d, *J* = 9.0 Hz, 2H), 7.17 (d, *J* = 8.2 Hz, 1H), 6.75 (s, 1H), 6.64 (d, *J* = 9.0 Hz, 2H), 2.99 (s, 6H), 1.67 (s, 9H). **RMN $^{13}$C** (101 MHz, $CDCl_3$, 20°C) δ 150.5 (Cq), 147.6 (Cq), 147.0 (Cq), 146.9 (Cq), 132.8 (2×CH), 130.3 (CH), 122.6 (Cq), 120.1 (Cq), 119.3 (CH), 111.7 (2xCH), 110.9 (CH), 108.9 (Cq), 97.8 (Cq), 85.0

(Cq), 79.3 (Cq), 40.1 (2×$CH_3$), 28.2 (3×$CH_3$). **HRMS (+ESI)** calculée pour $C_{22}H_{23}ClN_3O_2$ (M+H+) : 396.1473, trouvée: 396.1474.

**Exemple 11.23 *tert*-Butyl 2-((4-(méthylamino)phényl)éthynyl)-1*H*-pyrrolo[2,3-*b*]pyridine-1-carboxylate 23i.**

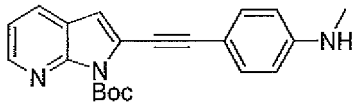

$C_{21}H_{21}N_3O_2$
MW: 347,41 g.mol-1

Le composé a été préparé selon la **procédure C** et purifié par colonne de chromatographie flash sur gel de silice (acétate d'éthyle/éther de pétrole = 20/80). Solide jaune (91%), **Pf** 166-168°C, ***R***$_f$ = 0.34 (acétate d'éthyle/éther de pétrole = 20/80). **Infrarouge** (v, $cm^{-1}$, neat) 3368, 3066, 2983, 2936, 2815, 2198, 1742, 1607, 1575, 1545, 1521, 1472, 1404, 1367, 1339, 1308, 1251, 1177, 1154, 1112, 1087, 876, 847, 826, 772, 640, 599, 561, 532, 515. **RMN $^1$H** (400 MHz, $CDCl_3$, 20°C) δ 8.53 (d, *J* = 3.9 Hz, 1H), 7.83 (d, *J* = 7.7 Hz, 1H), 7.40 (d, *J* = 8.3 Hz, 2H), 7.19 (dd, *J* = 7.5 Hz, *J* = 4.9 Hz, 1H), 6.81 (s, 1H), 6.59 (d, *J* = 8.3 Hz, 2H), 4.02 (ls, 1H), 2.89 (s, 3H), 1.70 (s, 9H). **RMN $^{13}$C** (101 MHz, $CDCl_3$, 20°C) δ 149.6 (Cq), 148.6 (Cq), 148.1 (Cq), 145.7 (CH), 132.9 (2×CH), 128.3 (CH), 122.0 (Cq), 121.6 (Cq), 118.8 (CH), 112.0 (2×CH), 111.6 (CH), 110.2 (Cq), 97.0 (Cq), 84.6 (Cq), 79.5 (Cq), 30.3 ($CH_3$), 28.2 (3×$CH_3$). **HRMS (+ESI)** calculée pour $C_{21}H_{22}N_3O_2$ (M+H+) : 348.1707, trouvée: 348.1705.

**Exemple 11.24 *tert*-butyl 6-bromo-2-((4-(diméthylamino)phényl)éthynyl)-1*H*-pyrrolo[2,3-*b*]pyridine-1-carboxylate 23j**

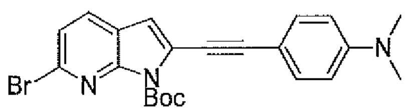

$C_{22}H_{22}BrN_3O_2$
MW: 440,33 g.mol-1

Le composé a été préparé selon la **procédure C** et purifié par colonne de chromatographie sur gel de silice sous pression (le rapport de solvants utilisé : acétate d'éthyle/dichlorométhane/éther de pétrole = 3/7/90). Solide jaune (68%), **Pf** 170-172°C, ***R***$_f$ = 0.27 (acétate d'éthyle/dichlorométhane/éther de pétrole = 3/7/90). **Infrarouge** (v, $cm^{-1}$, neat) 2983, 2200, 1752, 1606, 1561, 1542, 1434, 1394, 1367, 1353, 1332, 1306, 1240, 1153, 1137, 1103, 875, 832, 815, 802, 768, 518, 502. **RMN $^1$H** (400 MHz, $CDCl_3$, 20°C) δ 7.64 (d, *J* = 8.2 Hz, 1H), 7.46 - 7.39 (m, 2H), 7.33 (d, *J* = 8.2 Hz, 1H), 6.75 (s, 1H), 6.70 - 6.57 (m, 2H), 3.01 (s, 6H), 1.69 (s, 9H). **RMN $^{13}$C** (101 MHz, $CDCl_3$, 20°C) δ 150.5 (Cq), 147.6 (Cq), 147.2 (Cq), 137.0 (Cq), 132.8 (2×CH), 130.0 (CH), 122.9 (CH), 122.5 (Cq), 120.3 (Cq), 111.7 (2×CH), 110.9 (CH), 108.9 (Cq), 98.0 (Cq), 84.9 (Cq), 79.3 (Cq), 40.1 (2×$CH_3$), 28.2 (3×$CH_3$). **HRMS (+ESI)** calculée pour $C_{22}H_{23}BrN_3O_2$ (M+H+) : 440.0968, found: 440.0967.

**Exemple 12. Synthèse de composés finaux fonctionnalisés sur la position C-6 de 7-azaindoles**

Pour préparer les composés fonctionnalisés sur la position 6, un couplage de type Suzuki a été effectué à partir du composé **23e** pour obtenir le composé de type **24** qui a ensuite été déprotégé selon la **procédure F.**

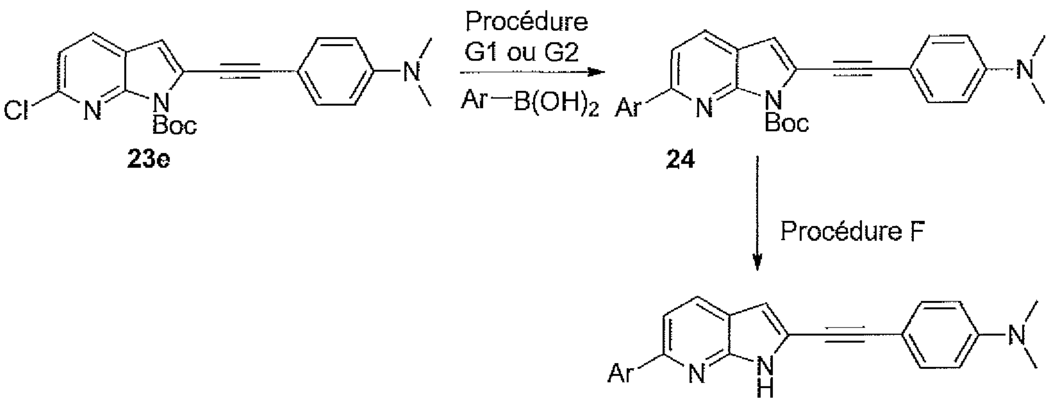


23e

24

25

**Exemple 12.1 *tert*-Butyl 2-((4-(diméthylamino)phényl)éthynyl)-6-(thiophèn-2-yl)-1*H*-pyrrolo[2,3-*b*]pyridine-1-carboxylate 24a.**

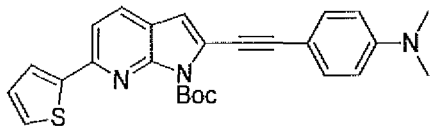

$C_{26}H_{23}N_3O_2S$
MW: 443,56 g.mol$^{-1}$

Le composé a été préparé selon **la procédure G2** et purifié par colonne de chromatographie flash sur gel de silice (acétate d'éthyle/éther de pétrole = 5/95). Solide jaune (37%), **Pf** 173-175°C, $\boldsymbol{R}_f$ = 0.42 (acétate d'éthyle/éther de pétrole = 10/90). **Infrarouge** (v, cm$^{-1}$, neat) 2927, 2197, 1744, 1593, 1365, 1258, 1154, 829, 696, 508. **RMN $^1$H** (400 MHz, $CDCl_3$, 20°C) δ 7.79 (d, *J* = 8.1 Hz, 1H), 7.64 (m, 1H), 7.59 (d, *J* = 8.2 Hz, 1H), 7.48 (d, *J* = 7.7 Hz, 2H), 7.38 (d, *J* = 4.8 Hz, 1H), 7.12 (m, 1H), 6.79 (s, 1H), 6.70 (d, *J* = 7.7 Hz, 2H), 3.03 (s, 6H), 1.80 (s, 9H). **RMN $^{13}$C** (101 MHz, $CDCl_3$, 20°C) δ 150.4 (Cq), 148.4 (Cq), 147.8 (Cq), 146.0 (Cq), 132.9 (2×CH), 128.6 (CH), 127.9 (CH), 127.0 (CH), 126.4 (Cq), 124.3 (CH), 122.4 (Cq), 120.4 (Cq), 114.6 (CH), 111.8 (2xCH), 111.2 (CH), 109.4 (Cq), 97.9 (Cq), 84.3 (Cq), 79.9 (Cq), 40.2 (2×$CH_3$), 28.4 (3×$CH_3$). **HRMS (+ESI)** calculée pour $C_{26}H_{26}N_3O_2S$ (M+H$^+$) : 444.1740, trouvée: 444.1742.

**Exemple 12.2 *N,N*-Diméthyl-4-((6-(thiophèn-2-yl)-1*H*-pyrrolo[2,3-*b*]pyridin-2-yl)éthynyl)aniline 25a.**

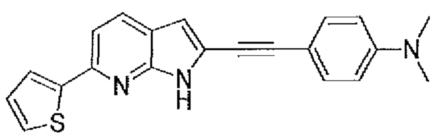

$C_{21}H_{17}N_3S$
MW: 343,44 g.mol$^{-1}$

Le composé a été préparé selon la **procédure F** et purifié par colonne de chromatographie flash sur gel de silice (acétate d'éthyle/éther de pétrole = 10/90). Solide jaune (38%), **Pf** 231-233°C, $R_f$ = 0.55 (acétate d'éthyle/éther de pétrole = 20/80). **Infrarouge** (v, cm$^{-1}$, neat) 3066, 1597, 1535, 1416, 1278, 821, 705. **RMN $^1$H** (250 MHz, DMSO-$d_6$, 20°C) δ 12.16 (s, 1H), 7.95 (d, *J* = 8.3 Hz, 1H), 7.77 (dd, *J* = 3.7 Hz, *J* = 1.1 Hz, 1H), 7.67 (d, *J* = 8.3 Hz, 1H), 7.58 (dd, *J* = 5.1 Hz, *J* = 1.1 Hz, 1H), 7.39 (d, *J* = 8.9 Hz, 2H), 7.16 (dd, *J* = 5.1 Hz, *J* = 3.7 Hz, 1H), 6.75 (d, *J* = 8.9 Hz, 2H), 6.71 (d, *J* = 2.0 Hz, 1H), 2.98 (s, 6H). **RMN $^{13}$C** (63 MHz, DMSO-$d_6$, 20°C) δ 150.8 (Cq), 148.3 (Cq), 147.0 (Cq), 146.1 (Cq), 132.6 (2×CH), 129.2 (CH), 128.8 (CH), 127.7 (CH), 124.9 (CH), 120.9 (Cq), 119.4 (Cq), 112.8 (CH), 112.4 (2×CH), 108.0 (Cq), 105.9 (CH), 95.3 (Cq), 80.6 (Cq), 40.1 (2×$CH_3$). **HRMS (+ESI)** calculée pour $C_{21}H_{18}N_3S$ (M+H$^+$) : 344.1216, trouvée: 344.1216.

**Exemple 12.3 *tert*-butyl 2-((4-(diméthylamino)phényl)éthynyl)-6-(thiophèn-3-yl)-1*H*-pyrrolo[2,3-*b*]pyridine-1-carboxylate 24b.**

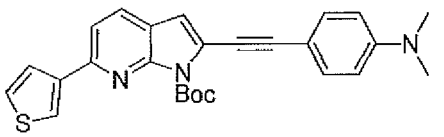

$C_{26}H_{25}N_3O_2S$
MW: 443,56 g.mol$^{-1}$

Le composé a été préparé selon la **procédure G1** et purifié par colonne de chromatographie flash sur gel de silice (acétate d'éthyle/éther de pétrole = 10/90). Solide jaune (57%), **Pf** 163-165°C, $R_f$ = 0.29 (acétate d'éthyle/éther de pétrole = 20/80). **Infrarouge** (ν, cm$^{-1}$, neat) 1743, 1607, 1257, 1154, 769, 507. **RMN $^1$H** (250 MHz, $CDCl_3$, 20°C) δ 7.98 (dd, *J* = 3.0 Hz, *J* = 1.2 Hz, 1H), 7.86 - 7.78 (m, 2H), 7.57 (d, *J* = 8.2 Hz, 1H), 7.52 - 7.44 (m, 2H), 7.41 (dd, *J* = 5.1 Hz, *J* = 3.0 Hz, 1H), 6.81 (s, 1H), 6.74 - 6.65 (m, 2H), 3.03 (s, 6H), 1.77 (s, 9H). **RMN $^{13}$C** (63 MHz, $CDCl_3$, 20°C) δ 150.3 (Cq), 149.4 (Cq), 148.3 (Cq), 148.2 (Cq), 143.0 (Cq), 132.8 (2×CH), 128.7 (CH), 126.6 (CH), 125.9 (CH), 123.0 (CH), 122.3 (Cq), 120.1 (Cq), 115.9 (CH), 111.8 (2×CH), 111.4 (CH), 109.4 (Cq), 97.6 (Cq), 84.1 (Cq), 80.0 (Cq), 40.2 (2×$CH_3$), 28.4 (3×$CH_3$). **HRMS (+ESI)** calculée pour $C_{26}H_{26}N_3O_2S$ (M+H$^+$) : 444.1740, trouvée: 444.1738.

**Exemple 12.4 *N,N*-Diméthyl-4-((6-(thiophèn-3-yl)-1*H*-pyrrolo[2,3-*b*]pyridin-2-yl)éthynyl)aniline 25b.**

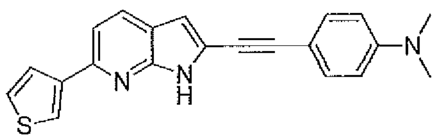

$C_{21}H_{17}N_3S$
MW: 343,44 g.mol$^{-1}$

Le composé a été préparé selon la **procédure F** et purifié par colonne de chromatographie flash sur gel de silice (acétate d'éthyle/éther de pétrole = 3/97). Solide jaune (66%), **Pf** 220-222°C, $R_f$ = 0.46 (acétate d'éthyle/éther de pétrole = 10/90). **Infrarouge** (ν, cm$^{-1}$, neat) 3103, 2211, 1768, 1596, 1536, 1501, 1422, 1328, 1269, 1163, 1138, 1054, 935, 864, 821, 769, 691, 603, 564, 520. **RMN $^1$H** (250 MHz, DMSO-$d_6$, 20°C) δ 12.09 (s, 1H), 8.11 (d, *J* = 1.7 Hz, 1H), 7.95 (d, *J* = 8.2 Hz, 1H), 7.79 (d, *J* = 4.1 Hz, 1H), 7.63 (m, 2H), 7.39 (d, *J* = 8.8 Hz, 2H), 6.75 (d, *J* = 8.9 Hz, 2H), 6.71 (d, *J* = 1.8 Hz, 1H), 2.98 (s, 6H). **RMN $^{13}$C** (63 MHz, DMSO-$d_6$, 20°C) δ 150.8 (Cq), 148.6 (Cq), 148.1 (Cq), 143.2 (Cq), 132.9 (2×CH), 129.2 (CH), 127.2 (CH), 126.9 (CH), 123.2 (CH), 120.8 (Cq), 119.1 (Cq), 114.2 (CH), 112.4 (2xCH), 108.1 (Cq), 105.7 (CH), 95.1 (Cq), 80.7 (Cq), 40.1 (2×$CH_3$). **HRMS (+ESI)** calculée pour $C_{21}H_{18}N_3S$ (M+H$^+$) : 344.1216, trouvée: 344.1216.

**Exemple 12.5 *tert*-Butyl 2-((4-(diméthylamino)phényl)éthynyl)-6-(6-fluoropyridin-3-yl)-1*H*-pyrrolo[2,3-*b*]pyridine-1-carboxylate 24c.**

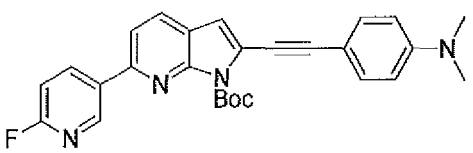

$C_{27}H_{25}FN_4O_2$
MW: 456,51 g.mol$^{-1}$

Le composé a été préparé selon la **procédure G1** et purifié par colonne de chromatographie flash sur gel de silice (acétate d'éthyle/éther de pétrole = 10/90). Solide jaune (46%), **Pf** 168-170°C, $R_f$ = 0.49 (acétate d'éthyle/éther de pétrole = 20/80). **Infrarouge** (ν, cm$^{-1}$, neat) 2925, 2196, 1740, 1612, 1567, 1546, 1439, 1398, 1369, 1335, 1306, 1269, 1245, 1154, 1110, 841, 813, 770, 508. **RMN $^1$H** (250 MHz, $CDCl_3$, 20°C) δ 8.91 (d, *J* = 2.4 Hz, 1H), 8.58 (td, *J* = 8.4, 2.4 Hz, 1H), 7.87 (d, *J* = 8.2 Hz, 1H), 7.62 (d, *J* = 8.2 Hz, 1H), 7.45 (d, *J* = 8.9 Hz, 2H), 7.03 (dd, *J* = 8.5 Hz, *J* = 2.9 Hz, 1H), 6.81 (s, 1H), 6.67 (d, *J* = 8.9 Hz, 2H), 3.01 (s, 6H), 1.74 (s, 9H). **RMN $^{13}$C** (63 MHz, $CDCl_3$, 20°C) δ 163.8 (d,

*J* = 240.1 Hz, Cq), 150.5 (Cq), 149.0 (Cq), 148.3 (Cq), 148.1 (Cq), 146.1 (d, *J* = 15.2 Hz, CH), 139.9 (d, *J* = 8.1 Hz, CH), 133.7 (d, *J* = 4.7 Hz, Cq), 132.9 (2×CH), 129.0 (CH), 123.3 (Cq), 121.0 (Cq), 115.4 (CH), 111.8 (2xCH), 111.0 (CH), 109.4 (d, *J* = 37.4 Hz, CH), 98.3 (Cq), 84.4 (2×Cq), 79.7 (Cq), 40.1 (2×$CH_3$), 28.3 (3×$CH_3$). **RMN $^{19}$F** (376 MHz, $CDCl_3$, 20°C) δ -72.03. **HRMS (+ESI)** calculée pour $C_{27}H_{25}FN_4O_2$ (M+H+) : 457.2034, trouvée: 457.2036.

**Exemple 12.6 4-((6-(6-Fluoropyridin-3-yl)-1*H*-pyrrolo[2,3-*b*]pyridin-2-yl)éthynyl)-*N,N*-diméthylaniline 25c**

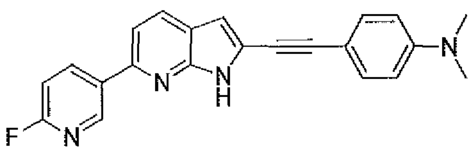

$C_{22}H_{17}FN_4$
MW: 356,40 g.mol-1

Le composé a été préparé selon la **procédure F** et purifié par colonne de chromatographie flash sur gel de silice (acétate d'éthyle/éther de pétrole = 10/90). Solide jaune (68%), **Pf** 251-253°C, $\boldsymbol{R_f}$ **=** 0.19 (acétate d'éthyle/éther de pétrole = 10/90). **Infrarouge** (ν, cm$^{-1}$, neat) 3140, 2899, 2197, 1610, 1540, 1470, 1415, 1371, 1291, 1273, 1187, 1140, 1065, 1019, 948, 814, 761, 699, 645, 567, 520. **RMN $^1$H** (250 MHz, , 20°C) δ 12.24 (s, 1H), 8.94 (d, *J* = 2.4 Hz, 1H), 8.65 (td, *J* = 8.4 Hz, *J* = 2.7 Hz, 1H), 8.06 (d, *J* = 8.3 Hz, 1H), 7.77 (d, *J* = 8.3 Hz, 1H), 7.41 (d, *J* = 8.9 Hz, 2H), 7.31 (dd, *J* = 8.7 Hz, *J* = 2.7 Hz, 1H), 6.83 - 6.68 (m, 3H), 2.98 (s, 6H). **RMN$^{13}$C** (63 MHz, DMSO-*d*$_6$, 20°C) δ 159.8 (d, *J* = 222.0 Hz, Cq), 150.9 (Cq), 148.8 (Cq), 147.6 (Cq), 146.0 (d, *J* = 15.7 Hz, CH), 140.4 (d, *J* = 8.0 Hz, CH), 134.2 (d, *J* = 4.5 Hz, Cq), 132.9 (2xCH), 129.5 (CH), 121.7 (Cq), 120.0 (Cq), 113.9 (CH), 112.4 (2xCH), 110.0 (d, *J* = 37.7 Hz, CH), 107.9 (Cq), 105.6 (CH), 95.5 (Cq), 80.5 (Cq), 40.1 (2×$CH_3$). **RMN $^{19}$F** (376 MHz, DMSO-*d*$_6$, 20°C) δ -70.25. **HRMS (+ESI)** calculée pour $C_{22}H_{18}FN_4$ (M+H+) : 357.1510, trouvée: 357.1507.

**Exemple 13. Synthèse de composés finaux alkyles fluorés**

Les dérivés de 7-azaindole, possédant un groupement fluoroéthyle sur l'amine de la *N*-méthylaniline ont été obtenus par 2 méthodes différentes.

Méthode 1

La première méthode consiste à effectuer une substitution nucléophile de l'amine secondaire du produit intermédiaire **23i.** Le produit **26** a été obtenu à hauteur de 31%. L'alcool **26** a été ensuite transformé en dérivé fluoré **27** en présence de DAST avec un rendement de 27%. Ce dernier a été déprotégé par une solution de TFA dans le $CH_2Cl_2$ pour donner le produit final **28** avec un rendement de 26 %.

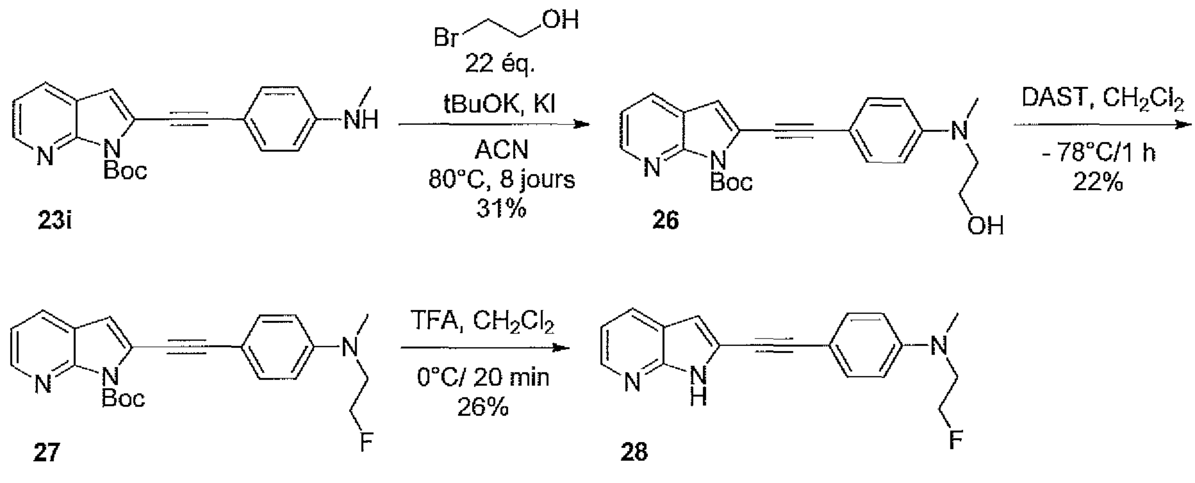


**23i** **26** **27** **28**

Pour préparer le radiomarquage au $^{18}$F, le dérivé *O*-tosylé **29** a été préparé à partir de **26**.

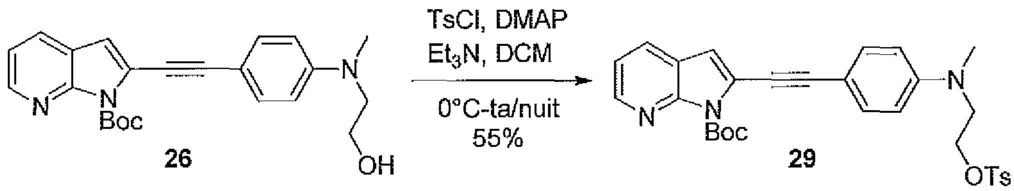


26

29

**Exemple 13.1** ***tert*-Butyl-2-((4-((2-hydroxyéthyl)(méthyl)amino)phényl)éthynyl)-1*H*-pyrrolo[2,3-*b*]pyridine-1-carboxylate 26.**

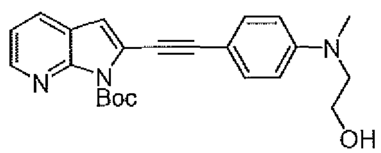

$C_{23}H_{25}N_3O_3$
MW: 391,46 $g.mol^{-1}$

A une solution contenant du dérivé **23i** (0.6 g, 1.73 mmol, 1 éq.), du 2-bromoéthanol (0.55 mL, 7.77 mmol, 4.5 éq.) et du KI (0.06 g, 0.35 mmol, 0.2 éq.) dans l'acétonitrile (30 mL), est ajoutée une solution de *t*BuOK (1M dans le THF, 3.8 mL, 3.80 mmol, 2.2 éq.). Le mélange est agité au reflux durant 8 jours (suivi CCM), en ajoutant 4 éq. de 2-bromoéthanol toutes les 24 heures. A la fin de la réaction l'eau est ajoutée au mélange réactionnel, les phases sont séparées et la phase aqueuse est extraite avec l'acétate d'éthyle. Les phases organiques réunies ont été lavées avec l'eau, une solution saturée de NaCl et séchées sur $MgSO_4$ avant d'être concentrées sous pression réduite. Le composé a été purifié par colonne de chromatographie sur gel de silice flash (acétate d'éthyle/éther de pétrole = 50/50 + 1% MeOH). Solide jaune (31%), **Pf** 154-156°C, $R_f$ = 0.22 (acétate d'éthyle/éther de pétrole = 40/60). **Infrarouge** (ν, $cm^{-1}$, neat) 3386, 2980, 2926, 2206, 1747, 1603, 1546, 1517, 1473, 1408, 1384, 1343, 1305, 1249, 1194, 1154, 1117, 1085, 980, 847, 831, 814, 776, 746, 643, 525. **RMN $^1H$** (250 MHz, $CDCl_3$, 20°C) δ 8.50 (dd, *J* = 4.8 Hz, *J* = 1.7 Hz, 1H), 7.81 (dd, *J* = 7.8 Hz, *J* = 1.7 Hz, 1H), 7.46 - 7.34 (m, 2H), 7.17 (dd, *J* = 7.8 Hz, *J* = 4.8 Hz, 1H), 6.80 (s, 1H), 6.79 - 6.64 (m, 2H), 3.85 (t, *J* = 5.7 Hz, 2H), 3.54 (t, *J* = 5.7 Hz, 2H), 3.04 (s, 3H), 1.82 (ls, 1H), 1.68 (s, 9H). **RMN $^{13}C$** (63 MHz, $CDCl_3$, 20°C) δ 149.8 (Cq), 148.6 (CH), 148.1 (Cq), 145.7 (Cq), 132.8 (2xCH), 128.3 (CH), 121.9 (Cq), 121.6 (Cq), 118.8 (CH), 112.1 (2xCH), 111.7 (CH), 109.9 (Cq), 96.9 (Cq), 84.6 (Cq), 79.8 (Cq), 60.2 ($CH_2$), 54.8 ($CH_2$), 38.9 ($CH_3$), 28.2 (3×$CH_3$). **HRMS (+ESI)** calculée pour $C_{23}H_{26}N_3O_3$ ($M+H^+$) : 392.1969, trouvée: 392.1968.

**Exemple 13.2** ***tert*-Butyl-2-((4-(méthyl(2-(tosyloxy)éthyl)amino)phényl)éthynyl)-1*H*-pyrrolo[2,3-*b*]pyridine-1-carboxylate 29.**

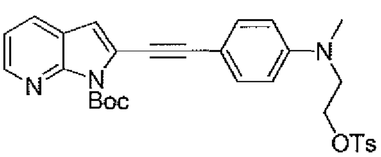

$C_{30}H_{31}N_3O_5S$

MW: 545,65 $g.mol^{-1}$

A une solution de TsCl (0.12 g, 0.63 mmol, 1.2 éq.), de triéthylamine (0.15 mL, 1.05 mmol, 2 éq.) et de DMAP (0.013 g, 0.11 mmol, 0,2 éq.) dans du THF (11 mL), refroidie à 0°C, a été ajoutée une solution de **26** (0.21 g, 0.53 mmol, 1 éq.) dans le THF (6 mL). Le mélange est agité à température ambiante durant une nuit, puis concentré sous pression réduite et purifié par colonne de chromatographie flash sur gel de silice (acétate d'éthyle/éther de pétrole = 30/70). Solide jaune (55%), **Pf** 171-173°C, $R_f$ = 0.38 (acétate d'éthyle/éther de pétrole = 40/60). **Infrarouge** (ν, $cm^{-1}$, neat) 2986, 2360, 2198, 1749, 1605, 1544, 1514, 1407, 1348, 1306, 1253, 1187, 1170, 1114, 1092, 1010, 972, 900, 804, 776, 663, 556, 508. **RMN $^1H$** (250 MHz, $CDCl_3$, 20°C) δ 8.48 (dd, *J* = 4.8, 1.7 Hz, 1H), 7.78 (dd, *J* = 7.8 Hz, *J* = 1.7 Hz, 1H), 7.73 - 7.52 (m, 2H), 7.39 - 7.26 (m, 2H), 7.30 - 7.17 (m, 2H), 7.14 (dd, *J* = 7.8 Hz, *J* = 4.8 Hz, 1H), 6.78 (s, 1H), 6.56 - 6.42 (m, 2H), 4.16 (t, *J* = 5.8 Hz, 2H), 3.62 (t, *J* = 5.8 Hz, 2H), 2.90 (s, 3H), 2.39 (s, 3H), 1.66 (s, 9H). **RMN $^{13}C$** (101 MHz, $CDCl_3$, 20°C) δ 148.6 (Cq), 148.3 (Cq), 148.1 (Cq), 145.8 (CH), 145.0 (Cq), 132.8 (2×CH), 132.6 (Cq), 129.8 (2×CH),

128.3 (CH), 127.8 (2×CH), 121.9 (Cq), 121.5 (Cq), 118.9 (CH), 111.8 (CH), 111.6 (2×CH), 110.0 (Cq), 96.7 (Cq), 84.6 (Cq), 79.9 (Cq), 66.7 ($CH_2$), 51.0 ($CH_2$), 39.0 ($CH_3$), 28.2 (3×$CH_3$), 21.7 ($CH_3$). **HRMS (+ESI)** calculée pour $C_{30}H_{32}N_3O_5S$ (M+H$^+$) : 546.2057, trouvée: 546.2053.

**Exemple 13.3 *tert*-Butyl-2-((4-((2-fluoroéthyl)(méthyl)amino)phényl)éthynyl)-1*H*-pyrrolo[2,3-*b*]pyridine-1-carboxylate 27.**

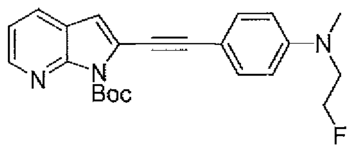

$C_{23}H_{24}FN_3O_2$

MW: 393,45 g.mol$^{-1}$

A une solution contenant du dérivé **26** (0.115 g, 0.29 mmol, 1 éq.) dans le dichlorométhane (3 mL) et refroidie à -78°C, est additionné du DAST (0.077 mL, 0.59 mmol, 2 éq.) goutte-à-goutte. Le milieu réactionnel est agité 1 h à -78°C. Après le retour à température ambiante, de l'eau et du dichlorométhane sont ajoutés et les phases sont séparées. La phase aqueuse est extraite par du dichlorométhane (2 fois), les phases organiques réunies sont séchées sur $MgSO_4$ et concentrées sous pression réduite. Le composé est purifié par colonne de chromatographie flash sur gel de silice (acétate d'éthyle/éther de pétrole = 20/80). Solide blanc (22%), **Pf** 151-153°C, $R_f$ = 0.31 (acétate d'éthyle/éther de pétrole = 20/80). **Infrarouge** (ν, cm$^{-1}$, neat) 2979, 2204, 1742, 1605, 1573, 1544, 1517, 1355, 1306, 1253, 1189, 1154, 1115, 1089, 1041, 979, 813, 774, 527, 510, 508, 504. **RMN $^1$H** (250 MHz, $CDCl_3$, 20°C) δ 8.48 (dt, *J* = 4.8 Hz, *J* = 1.6 Hz, 1H), 7.78 (dt, *J* = 7.9 Hz, *J* = 1.6 Hz, 1H), 7.44 - 7.34 (m, 2H), 7.15 (ddd, *J* = 7.8 Hz, *J* = 4.8 Hz, *J* = 1.4 Hz, 1H), 6.78 (s, 1H), 6.66 (d, *J* = 8.8 Hz, 2H), 4.69 (t, *J* = 5.1 Hz, 1H), 4.50 (t, *J* = 5.1 Hz, 1H), 3.72 (t, *J* = 5.2 Hz, 1H), 3.62 (t, *J* = 5.2 Hz, 1H), 3.04 (m, 3H), 1.66 (s, 9H). **RMN $^{13}$C** (101 MHz, $CDCl_3$, 20°C) δ 149.1 (Cq), 148.7 (Cq), 148.2 (Cq), 145.9 (CH), 133.0 (2×CH), 128.5 (CH), 122.1 (Cq), 121.7 (Cq), 119.0 (CH), 111.9 (2xCH), 111.8 (CH), 109.9 (Cq), 96.9 (Cq), 84.7 (Cq), 81.8 (d, *J* = 170.3 Hz, $CH_2$), 80.0 (Cq), 52.5 (d, *J* = 21.2 Hz, $CH_2$), 39.1 ($CH_3$), 28.3 (3×$CH_3$). $^{19}$F NMR (235 MHz, $CDCl_3$, 20°C) δ -222.2. **HRMS (+ESI)** calculée pour $C_{23}H_{25}FN_3O_2$ (M+H$^+$) : 394.1925, trouvée: 394.1925.

**Exemple 13.4 4-((1*H*-Pyrrolo[2,3-*b*]pyridin-2-yl)éthynyl)-*N*-(2-fluoroéthyl)-*N*-méthylaniline 28.**

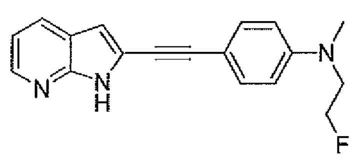

$C_{18}H_{16}FN_3$

MW: 293,34 g.mol$^{-1}$

Le composé a été préparé selon la **procédure F** (26%) ou selon la **procédure C** (83% voir la méthode 2, décrite ci-dessous) et purifié par colonne de chromatographie flash sur gel de silice (acétate d'éthyle/éther de pétrole = 10/90). Solide jaune (83%), **Pf** 242-244°C, $R_f$ = 0.28 (acétate d'éthyle/éther de pétrole = 20/80). **Infrarouge** (ν, cm$^{-1}$, neat) 3113, 3057, 2977, 2892, 2808, 2360, 2202, 1601, 1536, 1510, 1432, 1405, 1378, 1353, 1324, 1278, 1236, 1214, 1187, 1137, 1076, 1042, 1012, 977, 916, 810, 766, 692, 624, 552, 515. **RMN $^1$H** (400 MHz, DMSO-$d_6$, 20°C) δ 12.07 (s, 1H), 8.24 (m, 1H), 7.90 (m, 1H), 7.39 (d, *J* = 8.4 Hz, 2H), 7.08 (dd, *J* = 7.9 Hz, *J* = 4.6 Hz, 1H), 6.78 (d, *J* = 8.4 Hz, 2H), 6.71 (d, *J* = 1.7 Hz, 1H), 4.66 (t, *J* = 5.0 Hz, 1H), 4.54 (t, *J* = 5.0 Hz, 1H), 3.76 (t, *J* = 5.1 Hz, 1H), 3.69 (t, *J* = 5.1 Hz, 1H), 3.00 (s, 3H). **RMN $^{13}$C** (101 MHz, DMSO-$d_6$, 20°C) δ 149.7 (Cq), 148.8 (Cq), 144.3 (CH), 133.0 (2×CH), 128.4 (CH), 120.5 (Cq), 120.2 (Cq), 116.6 (CH), 112.4 (2×CH), 108.3 (Cq), 105.5 (CH), 94.7 (Cq), 82.4 (d, *J* = 166.2 Hz, $CH_2$), 80.5 (Cq), 51.9 (d, *J* = 19.8 Hz, $CH_2$), 38.9 ($CH_3$). **RMN$^{19}$F** (235 MHz, DMSO-$d_6$) δ -221.2 (tt, *J* = 47.5 Hz, *J* = 26.2 Hz). **HRMS (+ESI)** calculée pour $C_{18}H_{17}FN_3$ (M+H$^+$) : 294.1401, trouvée: 294.1401.

**Exemple 14. Synthèse de composés finaux alkyles fluorés**

Méthode 2:

Le composé **30,** obtenu par une amination réductrice d'éthynylaniline, est engagé dans une alkylation avec le bromoacétate d'éthyle pour donner le dérivé **31,** qui est ensuite réduit avec $LiAlH_4$ pour fournir l'aminoalcool **32.** Ce dernier est transformé en tosylate **33** et ensuite substitué avec du fluor en présence de TBAF, pour donner l'alcyne **34,** qui est utilisé pour le couplage de Sonogashira afin d'obtenir le produit souhaité **28** ou son analogue **35.**

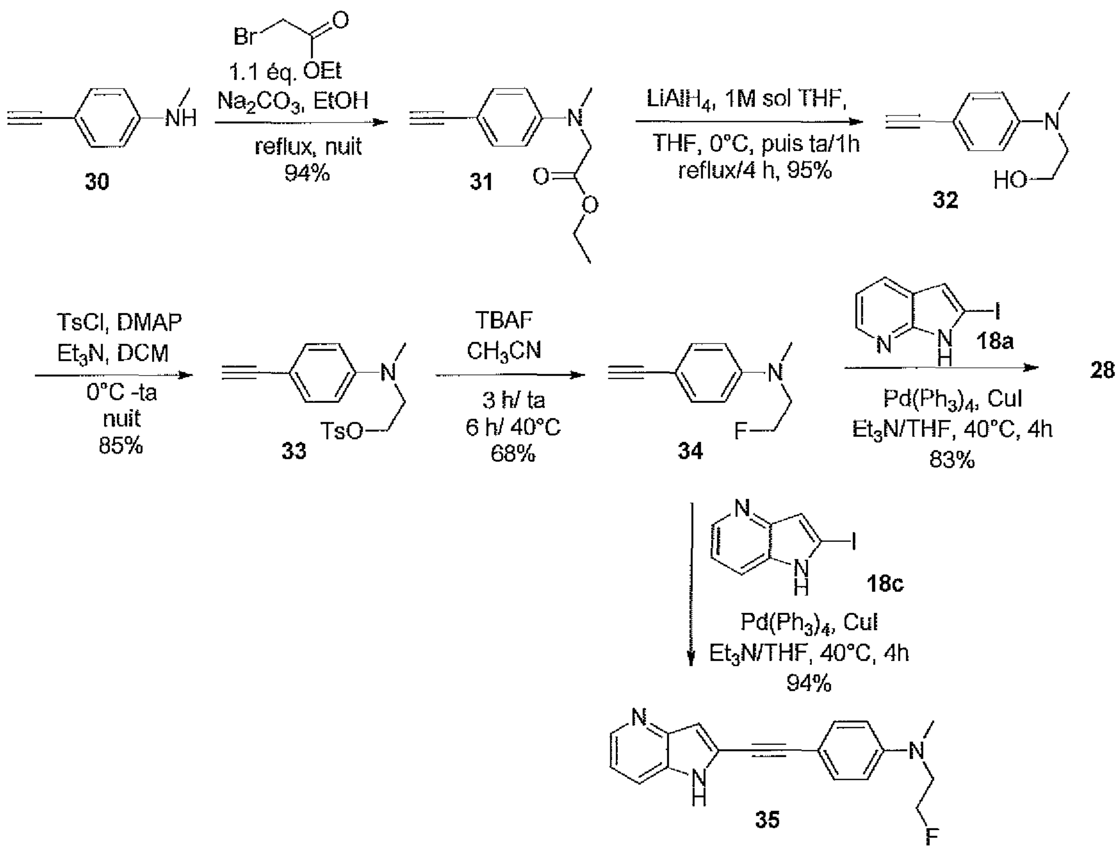

30 31 32 33 34 18a 28 18c 35

Par la suite, les alcynes **31** et **36** (analogue méthylé de **32**) ont été utilisés pour exemplifier la série de 5-fluoro-1*H*-pyrrolo[2,3-*b*]pyridine. Ainsi, les produits **37** et **38,** issus de couplage de Sonogashira avec le dérivé **18g** ont été obtenus avec les rendements de 93% et 80% respectivement.

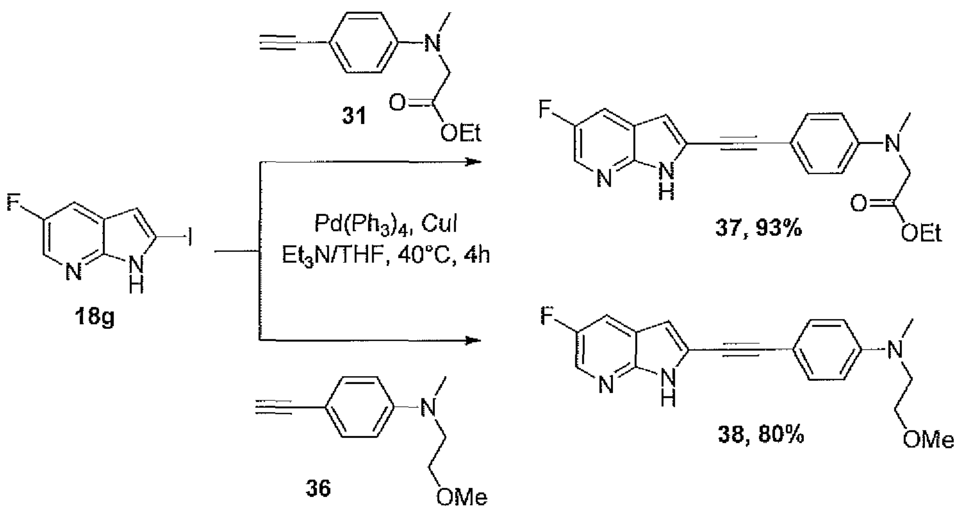

31 18g 36 **37, 93%** **38, 80%**

**Exemple 14.1 Ethyl 2-((4-éthynylphényl)(méthyl)amino)acétate 31.**

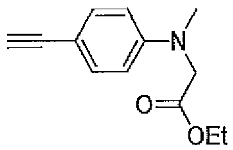

$C_{13}H_{15}NO_2$

MW: 217,26 g.mol$^{-1}$

La 4-éthynyl-*N*-méthylaniline (2.05 g, 15.6 mmol, 1 éq.) et du $Na_2CO_3$ (2.49 g, 23.4 mmol, 1.5 éq.) sont dissous dans de l'EtOH (30 mL), puis le bromoacétate d'éthyle (1.83 mL, 17.19 mmol, 1.1 éq.) est additionné lentement. Le milieu réactionnel est chauffé au reflux durant 1 nuit. Après refroidissement, l'eau (70 mL) et l'acétate d'éthyle (40 mL) sont ajoutés et le composé extrait avec de l'acétate d'éthyle (3 fois). La phase organique a été séchée sur $MgSO_4$ et concentrée sous pression réduite, avant d'être purifiée par colonne de chromatographie flash sur gel de silice (acétate d'éthyle/éther de pétrole = 5/95). Liquide transparent (3.18 g, 94%), $\boldsymbol{R_f}$ = 0.34 (acétate d'éthyle/éther de pétrole = 5/95). **Infrarouge** (ν, cm$^{-1}$, neat) 3282, 2981, 2099, 1741, 1607, 1515, 1476, 1370, 1250, 1181, 1115, 1026, 946, 816, 662, 538, 505. **RMN $^1$H** (400 MHz, $CDCl_3$, 20°C) δ 7.42 - 7.34 (m, 2H), 6.65 - 6.56 (m, 2H), 4.20 (q, *J* = 7.1 Hz, 2H), 4.08 (s, 2H), 3.09 (s, 3H), 2.99 (s, 1H), 1.26 (t, *J* = 7.1 Hz, 3H). **RMN $^{13}$C** (101 MHz, $CDCl_3$, 20°C) δ 170.5 (Cq), 149.0 (Cq), 133.3 (2×CH), 111.8 (2×CH), 110.0 (Cq), 84.6 (Cq), 75.0 (CH), 61.1 ($CH_2$), 54.2 ($CH_2$), 39.5 ($CH_3$), 14.2 ($CH_3$). **HRMS (+ESI)** calculée pour $C_{13}H_{16}NO_2$ ($M+H^+$) : 218.1176, trouvée: 218.1175.

**Exemple 14.2 2-((4-Ethynylphényl)(méthyl)amino)éthanol 32.**

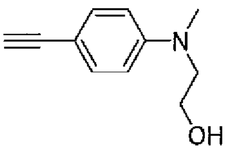

$C_{11}H_{13}NO$

MW: 175,23 g.mol$^{-1}$

A une solution de **31** (0.518 g, 2.38 mmol, 1 éq.) dans du THF (2.4 mL) est ajoutée lentement une solution 1M/THF de $LiAlH_4$ (4.8 mL, 4.76 mmol, 2 éq.). Le mélange est agité à température ambiante durant 1 heure, puis pendant 4 heures au reflux. Après refroidissement à 0°C, ont été ajoutés l'eau, une solution aqueuse saturée de NaCl et de l'$Et_2O$. La suspension est filtrée sur célite et lavée avec de l'$Et_2O$. Le filtrat est séparé en 2 phases et la phase aqueuse est lavée 3 fois avec de l'$Et_2O$. Les phases organiques réunies ont été séchées sur $MgSO_4$ et concentrées sous pression réduite. Le produit brut est purifié par colonne de chromatographie flash sur gel de silice (acétate d'éthyle/éther de pétrole = 20/80). Solide blanc (0.36 g, 85%), **Pf** 73-75°C, $\boldsymbol{R_f}$ = 0.36 (acétate d'éthyle/éther de pétrole = 30/70). **Infrarouge** (ν, cm$^{-1}$, neat) 3293, 3271, 2907, 2871, 2094, 1606, 1518, 1454, 1378, 1349, 1268, 1222, 1176, 1125, 1059, 977, 908, 852, 814, 803, 644, 581, 524, 509. **RMN $^1$H** (250 MHz, $CDCl_3$, 20°C) δ 7.37 - 7.28 (m, 2H), 6.70 - 6.60 (m, 2H), 3.79 (q, *J* = 5.6 Hz, 2H), 3.48 (t, *J* = 5.6 Hz, 2H), 2.97 (s, 3H), 2.95 (s, 1H), 1.62 (ls, 1H). **RMN $^{13}$C** (63 MHz, $CDCl_3$, 20°C) δ 149.8 (Cq), 133.3 (2×CH), 112.0 (2×CH), 109.4 (Cq), 84.6 (Cq), 75.0 (CH), 60.2 ($CH_2$), 54.8 ($CH_2$), 38.8 ($CH_3$). **HRMS (+ESI)** calculée pour $C_{11}H_{14}NO$ ($M+H^+$) : 176.1070, trouvée: 176.1071.

**Exemple 14.3 2-((4-Ethynylphényl)(méthyl)amino)ethyl 4-méthylbenzènesulfonate 33.**

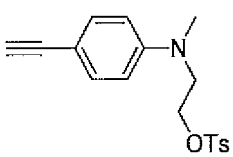

$C_{18}H_{19}NO_3S$

MW: 329,41 g.mol$^{-1}$

A une solution de TsCl (0.30 g, 1.55 mmol, 1.2 éq.), de triéthylamine (0.36 mL, 2.58 mmol, 2 éq.) et de DMAP

(0.032 g, 0.26 mmol, 0,2 éq.) dans du THF (27 mL), refroidie à 0°C, a été ajoutée une solution de **32** (0.23 g, 1.29 mmol, 1 éq.) dans du THF (15 mL). Le mélange est agité à température ambiante durant une nuit, puis concentré sous pression réduite et purifié par colonne de chromatographie flash sur gel de silice (acétate d'éthyle/éther de pétrole = 10/90). Solide blanc (85%), **Pf** 99-101°C, $R_f$ = 0.38 (acétate d'éthyle/éther de pétrole = 20/80). **Infrarouge** (ν, $cm^{-1}$, neat) 3278, 2991, 2920, 2099, 1608, 1519, 1351, 1293, 1268, 1240, 1217, 1172, 1145, 1093, 1078, 1015, 959, 918, 898, 842, 817, 777, 692, 658, 578, 556, 523. **RMN $^1$H** (250 MHz, $CDCl_3$, 20°C) δ 7.75 - 7.53 (m, 2H), 7.36 - 7.07 (m, 4H), 6.55 - 6.18 (m, 2H), 4.14 (t, *J* = 5.8 Hz, 2H), 3.59 (t, *J* = 5.8 Hz, 2H), 2.95 (s, 1H), 2.87 (s, 3H), 2.39 (s, 3H). **RMN $^{13}$C** (63 MHz, $CDCl_3$, 20°C) δ 148.2 (Cq), 145.0 (Cq), 133.3 (2×CH), 132.5 (Cq), 129.8 (2×CH), 127.8 (2×CH), 111.5 (2×CH), 109.5 (Cq), 84.5 (Cq), 75.1 (Cq), 66.7 ($CH_2$), 50.9 ($CH_2$), 39.0 ($CH_3$), 21.6 ($CH_3$). **HRMS (+ESI)** calculée pour $C_{18}H_{20}NO_3S$ ($M+H^+$) : 330.1158, trouvée: 330.1158.

**Exemple 14.4 4-Ethynyl-*N*-(2-fluoroéthyl)-*N*-méthylaniline 34.**

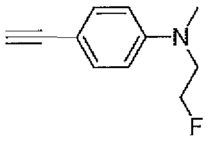

$C_{11}H_{12}FN$

MW: 177,22 g.mol$^{-1}$

A une solution de 33 (0.21 g, 0.65 mmol, 1 éq.) dans du $CH_3CN$ (5 mL) a été ajoutée une solution 1M/THF de TBAF (1.30 mL, 1.29 mmol, 2 éq.). Le mélange est agité à température ambiante durant une nuit, puis 4 heures à 40°C avant d'être concentré sous pression réduite et purifié par colonne de chromatographie flash sur gel de silice (acétate d'éthyle/éther de pétrole = 5/95). Liquide transparent (68%), $R_f$ = 0.39 (acétate d'éthyle/éther de pétrole = 5/95). **Infrarouge** (ν, $cm^{-1}$, neat) 3291, 2896, 2098, 1606, 1515, 1479, 1373, 1267, 1209, 1179, 1133, 1075, 1040, 1006, 979, 907, 846, 816, 845, 537, 520. **RMN $^1$H** (250 MHz, $CDCl_3$, 20°C) δ 7.39 - 7.27 (m, 2H), 6.67 - 6.45 (m, 2H), 4.66 (t, *J* = 5.2 Hz, 1H), 4.47 (t, *J* = 5.2 Hz, 1H), 3.68 (t, *J* = 5.2 Hz, 1H), 3.58 (t, *J* = 5.2 Hz, 1H), 3.00 (s, 3H), 2.96 (s, 1H). **RMN $^{13}$C** (63 MHz, $CDCl_3$, 20°C) δ 148.9 (Cq), 133.4 (2×CH), 111.6 (2×CH), 109.3 (Cq), 84.6 (CH), 81.6 (d, *J* = 170.1 Hz, $CH_2$), 75.0 (CH), 52.4 (d, *J* = 21.2 Hz, $CH_2$), 39.0 ($CH_3$). **RMN $^{19}$F** (235 MHz, $CDCl_3$, 20°C) δ -50.0. **HRMS (+ESI)** calculée pour $C_{11}H_{13}FN$ ($M+H^+$) : 178.1027, trouvée: 178.1026.

**Exemple 14.5 4-((1*H*-Pyrrolo[3,2-*b*]pyridin-2-yl)éthynyl)-*N*-(2-fluoroéthyl)-*N*-méthylaniline 35**

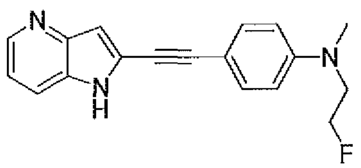

$C_{18}H_{16}FN_3$

MW: 293,34 g.mol$^{-1}$

Le composé a été préparé selon la **procédure C** et purifié par colonne de chromatographie sur gel de silice sous pression (acétate d'éthyle/éther de pétrole = 20/80). Solide jaune (94%), **Pf** 232-234°C, $R_f$ = 0.24 (acétate d'éthyle/éther de pétrole = 20/80). **Infrarouge** (ν, $cm^{-1}$, neat) 3066, 2952, 2890, 2820, 2703, 2202, 1606, 1571, 1538, 1513, 1405, 1376, 1354, 1285, 1247, 1189, 1124, 1040, 982, 913, 815, 776, 623, 583, 557, 529, 515, 506. **RMN $^1$H** (400 MHz, DMSO-$d_6$, 20°C) δ 11.83 (s, 1H), 8.35 (d, *J* = 4.6 Hz, 1H), 7.69 (m, 1H), 7.50 - 7.31 (m, 2H), 7.14 (dd, *J* = 8.2 Hz, *J* = 4.6 Hz, 1H), 6.89 - 6.69 (m, 3H), 4.67 (t, *J* = 4.9 Hz, 1H), 4.55 (t, *J* = 4.9 Hz, 1H), 3.76 (t, *J* = 5.0 Hz, 1H), 3.70 (t, *J* = 5.0 Hz, 1H), 3.00 (s, 3H). **RMN $^{13}$C** (101 MHz, DMSO-$d_6$, 20°C) δ 149.7 (Cq), 145.9 (Cq), 143.8 (CH), 133.0 (2×CH), 129.7 (Cq), 123.1 (Cq), 118.4 (CH), 117.8 (CH), 112.4 (2×CH), 108.1 (Cq), 106.8 (CH), 95.2 (Cq), 82.4 (d, *J* = 166.2 Hz, $CH_2$), 80.4 (Cq), 51.9 (d, *J* = 19.8 Hz, $CH_2$), 38.8 ($CH_3$). **RMN $^{19}$F** (376 MHz, DMSO-$d_6$, 20°C) δ 15.8. **HRMS (+ESI)** calculée pour $C_{18}H_{17}FN_3$ ($M+H^+$) : 294.1401, trouvée: 294.1404.

**Exemple 14.6 4-Ethynyl-*N*-(2-méthoxyéthyl)-*N*-méthylaniline 36.**

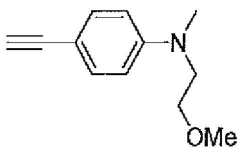

$C_{12}H_{15}NO$
MW: 189,26 g.mol$^{-1}$

A une solution de **32** (0.25 g, 1.43 mmol, 1 éq.) dans THF (3 mL) est ajouté lentement une solution 1M/THF de *t*BuOK (2.85 mL, 2.85 mmol, 2 éq.). Le mélange est agité sous ultrason durant 5 minutes, puis refroidi à 0°C et l'iodure de méthyle est additionné goutte à goutte. Le milieu est agité à la température ambiante pendant 16 h, puis concentré sous vide, le résidu est repris dans l'acétate d'éthyle, le précipité insoluble est filtré. Au filtrat est ajoutée l'eau et les phases sont séparées. La phase aqueuse est extraite avec acétate d'éthyle (2 fois). Les phases organiques réunies ont été lavées avec une solution saturée de NaCl, séchées sur $MgSO_4$ et concentrées sous pression réduite. Le produit est purifié par colonne de chromatographie sur gel de silice sous pression (le rapport de solvants utilisé : acétate d'éthyle/éther de pétrole = 5/95). Liquide transparent (0.22 g, 81%), $\boldsymbol{R_f}$ **=** 0.21 (acétate d'éthyle/éther de pétrole = 2/98). **Infrarouge** (ν, cm$^{-1}$, neat) 3286, 2877, 2097, 1606, 1515, 1451, 1375, 1274, 1193, 1179, 1110, 1067, 1018, 815, 644, 530, 514, 506, 503. **RMN $^1$H** (400 MHz, $CDCl_3$, 20°C) δ δ 7.38 (d, *J* = 9.0 Hz, 2H), 6.65 (d, *J* = 9.0 Hz, 2H), 3.56 (t, *J* = 3.1 Hz, 4H), 3.37 (s, 3H), 3.02 (s, 3H), 3.00 (s, 1H). **RMN $^{13}$C** (101 MHz, $CDCl_3$, 20°C) δ 149.3 (Cq), 133.3 (2×CH), 111.5 (2×CH), 108.7 (Cq), 84.8 (Cq), 74.8 (CH), 70.1 ($CH_2$), 59.1 ($CH_3$), 52.1 ($CH_2$), 38.9 ($CH_3$). **HRMS (+ESI)** calculée pour $C_{12}H_{16}NO$ (M+H$^+$) : 190.1226, trouvée: 190.1225.

**Exemple 14.7 2-((4-((5-Fluoro-1*H*-pyrrolo[2,3-*b*]pyridin-2-yl)éthynyl)phényl) (méthyl)amino)acetate d'éthyle 37**

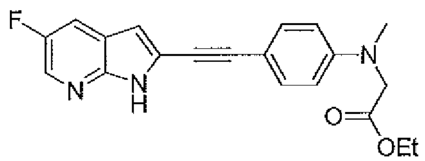

$C_{20}H_{18}FN_3O_2$
MW: 351,38 g.mol$^{-1}$

Le composé a été préparé selon la **procédure C** et purifié par colonne de chromatographie sur gel de silice sous pression (acétate d'éthyle/éther de pétrole = 15/85). Solide blanc (93%), **Pf** 186-188°C, $\boldsymbol{R_f}$ = 0.24 (acétate d'éthyle/éther de pétrole = 10/90). **Infrarouge** (ν, cm$^{-1}$, neat) 2984, 2208, 1729, 1608, 1585, 1540, 1508, 1372, 1295, 1254, 1190, 1154, 1109, 1030, 948, 871, 805, 765, 526, 514, 506, 503. **RMN $^1$H** (400 MHz, $CDCl_3$, 20°C) δ 10.59 (s, 1H), 8.25 (t, *J* = 2.3 Hz, 1H), 7.60 (dd, *J* = 8.9 Hz, *J* = 2.6 Hz, 1H), 7.52 - 7.42 (m, 2H), 6.80 - 6.49 (m, 3H), 4.23 (q, *J* = 7.1 Hz, 2H), 4.12 (s, 2H), 3.14 (s, 3H), 1.28 (t, *J* = 7.1 Hz, 3H). **RMN $^{13}$C** (101 MHz, $CDCl_3$, 20°C) δ 170.4 (Cq), 155.9 (d, *J* = 241.9 Hz), 149.3 (Cq), 145.0 (Cq), 133.0 (2×CH), 131.9 (d, *J* = 29.8 Hz, CH), 122.0 (Cq), 120.9 (d, *J* = 7.3 Hz, Cq), 114.0 (d, *J* = 21.0 Hz, CH), 112.0 (2xCH), 109.8 (Cq), 105.4 (d, *J* = 4.4 Hz, CH), 95.3 (Cq), 79.3 (Cq), 61.2 ($CH_2$), 54.2 ($CH_2$), 39.5 ($CH_3$), 14.2 ($CH_3$). **RMN $^{19}$F** (376 MHz, $CDCl_3$, 20°C) δ - 138.3. **HRMS (+ESI)** calculée pour $C_{20}H_{19}FN_3O_2$ (M+H$^+$) : 352.1456, trouvée: 352.1457.

**Exemple 14.8 4-((5-Fluoro-1*H*-pyrrolo[2,3-*b*]pyridin-2-yl)éthynyl)-*N*-(2-méthoxyéthyl)-*N*-méthylaniline 38**

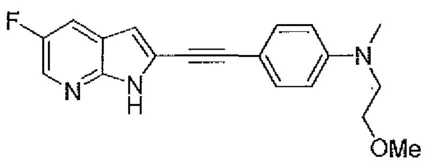

$C_{19}H_{18}FN_3O$
MW: 323,37 g.mol$^{-1}$

Le composé a été préparé selon la **procédure C** et purifié par colonne de chromatographie sur gel de silice sous pression (acétate d'éthyle/éther de pétrole = 20/80). Solide blanc (80%), **Pf** 160-162°C, $\boldsymbol{R_f}$ = 0.35 (acétate d'éthyle/éther de pétrole = 30/70). **Infrarouge** (ν, cm$^{-1}$, neat) 3066, 2882, 2203, 1602, 1534, 1507, 1372, 1346, 1293, 1218,

1189, 1155, 1106, 1019, 984, 895, 876, 821, 760, 553, 514, 506, 502. **RMN $^1$H** (400 MHz, $CDCl_3$, 20°C) δ 10.46 (s, 1H), 8.22 (t, *J* = 2.3 Hz, 1H), 7.57 (dd, *J* = 8.9 Hz, *J* = 2.6 Hz, 1H), 7.47 - 7.41 (m, 2H), 6.74 - 6.67 (m, 2H), 6.65 (d, *J* = 2.0 Hz, 1H), 3.58 (s, 4H), 3.37 (s, 3H), 3.04 (s, 3H). **RMN $^{13}$C** (101 MHz, $CDCl_3$, 20°C) δ 155.9 (d, *J* = 241.7 Hz, Cq), 149.5 (Cq), 145.1 (Cq), 133.0 (2xCH), 131.9 (d, *J* = 29.7 Hz, CH), 123.1 (Cq), 120.9 (d, *J* = 7.2 Hz, Cq), 113.9 (d, *J* = 20.9 Hz, CH), 111.7 (2×CH), 108.4 (Cq), 105.2 (d, *J* = 4.4 Hz, CH), 95.7 (Cq), 79.1 (Cq), 70.1 ($CH_2$), 59.1 ($CH_3$), 52.1 ($CH_2$), 38.9 ($CH_3$). **RMN $^{19}$F** (376 MHz, $CDCl_3$, 20°C) δ -138.4. **HRMS (+ESI)** calculée pour $C_{19}H_{19}FN_3O$ ($M+H^+$) : 324.1507, trouvée: 324.1507.

**Exemple 15. Production des ligands fluorés marqués au $^{18}$F par substitution nucléophile**

Étape : production des ions fluorures et exaltation de leur réactivité

Les ions [$^{18}$F] sont produits par transmutation nucléaire $^{18}O(p,n)^{18}F$. La solution d'ion [$^{18}$F] est passée sur une cartouche échangeuse d'anion QMA® pour fixer les ions fluorures. Cette cartouche a été préalablement conditionnée avec 10 mL de $K_2CO_3$ puis rincée avec 10 mL d'eau. Les ions [$^{18}$F] sont élués de la cartouche QMA par une solution aqueuse de $K_2CO_3$ et de Kyptofix$_{2.2.2}$ dissout dans du MeCN. Cette solution est transférée dans un réacteur puis séchée à deux reprises par distillation azéotropique avec ajout à chaque fois d'1 mL de MeCN. A la fin du séchage le complexe avec les ions fluorures est prêt pour l'étape suivante de radiofluoration.

Etape : substitution nucléophile (aliphatique, aromatique hétéroaromatique)

Les synthèses sont réalisées au départ de composés (hetéro)aromatiques aryliodoniums, de (hétéroaryl)nitrés électroniquement appauvris, d'(hétéro)aryltriméthylammoniums, d'esters ou acides (héréro)aromatiques boroniques mais aussi de composés aliphatiques halogénés (Cl, Br, I), triflates ou mésylates. Le précurseur solubilisé dans un solvant (MeCN, DMSO, DMF ou autres) est ajouté dans le réacteur contenant le complexe avec les ions fluorures. Pour les substitutions aliphatiques le MeCN est principalement utilisé mais si nécessaire des solvants avec des points d'ébullition plus élevés peuvent être utilisés comme pour les substitutions (hétéro)aromatiques qui requièrent généralement des solvants comme le DMSO ou le DMF. Le milieu réactionnel est chauffé entre 5 et 30 min en mode thermique ou par microonde puis la solution est ramenée à température ambiante. Celle-ci est diluée avec de l'eau et potentiellement hydrolysée, cela dépendant de la nature du précurseur utilisé (élimination d'un groupement protecteur). L'approche précédemment décrite est directe, une approche indirecte en fonction de l'accessibilité à la molécule, au précurseur peut être envisagée. A titre d'exemple, le [$^{18}$F]fluoroéthyltosylate ([$^{18}$F] FETos) est préparé à partir d'éthylène ditosylate puis une réaction de N, O ou S alkylation sur le précurseur conduit au produit souhaité.

Etape : purification et formulation

Le milieu réactionnel brut est passé au travers d'une cartouche de pré-purification puis rincée avec de l'eau. Le radiotraceur est élué de la cartouche par du MeCN puis la solution est chargée sur une colonne semi-préparative. Le produit radiomarqué d'intérêt est collecté et dissout dans de l'eau puis piégé sur une cartouche. Celle-ci est rincée puis le radiopharamceutique est élué par de l'éthanol injectable et la formulation est complétée par ajout de NaCl 0,9% de manière à ne pas avoir plus de 10% d'éthanol, en masse, dans la solution du médicament radiopharmaceutique. La solution est passée sur un filtre stérilisant et répartie dans des flacons stériles et apyrogènes afin d'être dispensée.

**Exemple 16. Production des ligands radiomarqués au carbone-11**

Étape : production des synthons réactifs marqués au Carbone-11

Les ions [$^{11}$C] sont produits par transmutation nucléaire $^{11}C(p,\alpha)^{15}N$. Cette transmutation se fait en présence de quelques ppm d'hydrogène ou d'oxygène pour conduire respectivement à la formation de méthane ou de dioxyde de carbone radiomarqué au carbone 11. Ces deux synthons produits vont ouvrir la voie à la production de nombreux autres synthons radiomarqués au carbone 11 et qui vont servir d'intermédiaires réactionnels pour une incorporation à la molécule d'intérêt. Parmi les plus courants on peut citer l'iodure de méthyle ou le triflate de méthyle.

La figure la représente la préparation de synthons à partir du dioxyde de carbone radiomarqué au carbone 11 tandis que la figure 1b représente la préparation de synthons à partir du méthane radiomarqué au carbone 1.

Etape : incorporation du synthon à la molécule d'intérêt.

L'iodure de méthyle ou le triflate de méthyle radiomarqués au carbone 11 peuvent être utilisés dans des

réactions courantes de O, N et S alkylation. Les solvants utilisés sont : MeCN, DMSO, DMF ou NMP. Des réactions de couplages avec des organométalliques peuvent être utilisées pour ces synthons radiomarqués : Stille, Suzuki, Sonogashira. Par ailleurs, le $[^{11}C]CO_2$ peut faire l'objet de l'attaque d'un réactif de Grignard ou être transformé en réactif de Grignard. Il peut également être réduit en monoxyde de carbone, $[^{11}C]CO$, et donner accès à de très nombreuses fonctions chimiques. La figure 2 représente les fonctionnalités chimiques accessibles par des réactions de carbonylation. Les réactions de marquage au Carbone 11 sont par conséquent la plupart du temps multiétapes.

Etape : purification et formulation

Le milieu réactionnel brut est passé au travers d'une cartouche de pré-purification puis rincée avec de l'eau. Le radiotraceur est élué de la cartouche par du MeCN puis la solution est chargée sur une colonne semi-préparative. Le produit radiomarqué d'intérêt est collecté puis dissout dans de l'eau et piégé sur une cartouche. Celle-ci est rincée puis le radiopharamceutique est élué par de l'éthanol injectable et la formulation est complétée par ajout de NaCl 0,9% de manière à ne pas avoir plus de 10% d'éthanol, en masse, dans la solution du médicament radiopharmaceutique. La solution est passée au travers d'un filtre stérilisant et répartie dans des flacons stériles et apyrogènes afin d'être dispensée.

**Exemple 17. Production des ligands radiomarqués à l'iode-123**

La solution commerciale de $Na^{123}I$ est acidifiée par une solution éthanolique d'acide chrlorhydrique ou sulfurique puis sont ajoutées :

- une solution éthanolique de précurseur aromatique ou hétéroaromatique stannylé ($SnBu_3$ ou $SnMe_3$)

- une solution d'oxydant (Chloramine T, eau oxygénée ou iodo-bead)

Le mélange est ensuite agité à température ambiante pendant quelques minutes (2-30 min). Puis la solution est rendue basique par ajout d'une solution de $NH_4OH$ ou $NaHCO_3$. Le milieu réactionnel est ensuite purifié par HPLC, le produit radiomarqué d'intérêt est collecté et dissout dans de l'eau puis piégé sur une cartouche. Celle-ci est rincée puis le radiopharmaceutique est élué par de l'éthanol injectable et la formulation est complétée par ajout de NaCl 0,9% de manière à ne pas avoir plus de 10% d'éthanol, en masse, dans la solution du médicament radiopharmaceutique. La solution est passée au travers d'un filtre stérilisant et répartie dans des flacons stériles et apyrogènes afin d'être dispensée.

Une approche similaire peut être appliquée pour des radiomarquages avec d'autres isotopes de l'iode (124, 125, 131).

**Exemple 18. Radiosynthèse**

**Exemple 18.1**

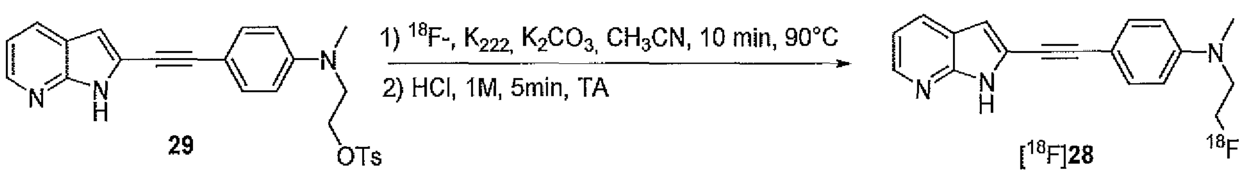


**29** $[^{18}F]$**28**

La radiosynthèse du 28 est réalisée à l'aide d'un automate FXFNpro (GE Healthcare). Les ions $[^{18}F]$ sont produits par transmutation nucléaire $^{18}O(p,n^{18}F$ à l'aide d'un cyclotron PETtrace (GE Healthcare). L'activité produite est transférée vers l'automate de radiosynthèse puis piégée sur une cartouche échangeuse d'anion QMA® (Waters) pour fixer les ions fluorures. Cette cartouche a été préalablement conditionnée avec 10 mL de $K_2CO_3$ puis rincée avec 10 mL d'eau. Les ions $[^{18}F]$ sont élués de la cartouche QMA par une solution aqueuse de $K_2CO_3$ et de Kyptofix$_{2.2.2}$ dissout dans du MeCN (7,2 mg de $K_{2.2.2}$ + 715 $\mu$l MeCN et 3,8 mg de $K_2CO_3$ + 285 $\mu$l $H_2O$). Cette solution est transférée dans un réacteur puis séchée à deux reprises par distillation azéotropique avec ajout à chaque fois d'1 mL de MeCN. A la fin du séchage le complexe avec les ions fluorures est prêt pour l'étape suivante de radiofluoration. 2 mg de précurseur **29** présentant la fonction tosyle dissous dans 700 $\mu$L de MeCN sont ajoutés aux ions fluorures. La réaction est portée à 90 °C pendant 10 min. la solution est ensuite ramenée à température ambiante avant d'ajouter 500 $\mu$L de HCl 1M. La réaction est agitée 5 min et ce toujours à température ambiante avant d'être quenchée par l'ajout de 8 mL

d'eau. Le mélange est ensuite pré-purifié sur une cartouche *t*C18plus (Waters) préalablement conditionnée. La cartouche est rincée par 5 mL d'eau avant d'en éluer le produit brut d'intérêt par 2 mL de MeCN auxquels sont ajoutés 2 mL d'acétate d'ammonium 0.1 M.

Les 4 mL récoltés sont ensuite chargés dans une boucle HPLC et injectés sur une colonne semi-préparative Zorbax Eclipse XDB-C18 9,4x250 mm 5μ (Agilent) avec comme phase mobile un mélange MeCN/Acétate d'ammonium 0.1M 50/50 à un débit de 5 mL/min. Dans ces conditions, le produit d'intérêt, le [$^{18}$F]**28**, est collecté avec un temps de rétention de l'ordre de 23 min. Le produit collecté est dissout dans 25 mL d'eau puis piégé sur une cartouche *t*C18light (Waters) préalablement conditionnée. La cartouche est rincée par 5 mL d'eau. Le [$^{18}$F]**28** est élué de la cartouche par 1 mL d'éthanol injectable, la formulation est complétée par l'ajout de 9 mL de NaCl 0,9%.

Pour une future injection à l'homme la solution mère obtenue doit faire l'objet d'une répartition aseptique selon les BPF, avec double filtration stérilisante, et remplissage des flacons dans un environnement adapté sous flux laminaire de classe A.

Le [$^{18}$F]**28** est obtenu en 85 ± 5 min avec un rendement de 32 ± 3 % (corrigé de la décroissance). L'activité spécifique déterminée à partir de la courbe de calibration avec le standard froid est de l'ordre de 128 GBq/μmole. La figure 3 représente un chromatogramme de contrôle qualité du composé [$^{18}$F]**28**.

**Exemple 19. Etudes biologiques**

Il s'agit d'études visant à évaluer l'affinité et la sélectivité de nouveaux composés pour les fibres d'α-synucléine. Pour identifier une meilleure sélectivité/affinité, la fixation des molécules synthétisée aux fibres d'alpha-synucléine est comparée à leur fixation à des fibres du peptide β-amyloïde (Aβ1-42) et/ou de Tau. Elles sont réalisées par étude de liaison in vitro sur des préparations de fibres synthétiques d'α-synucléine (α-syn), de peptide β-amyloïde (Aβ1-42) ou de Tau selon deux méthodes : 1/ compétition entre le nouveau composé et la thioflavine T (ThT) et mesure de la fluorescence provenant de la ThT liée, 2/ liaison directe du nouveau composé et mesure de la fluorescence provenant de la fraction liée.

**Exemple 19.1 Expression et purification des protéines recombinantes α-syn, Aβ1-42 et Tau**

Les protéines α-syn et Aβ1-42 natives sont exprimées chez *E. Coli* (souche BL21(DE3), (Stratagene, La Jolla, CA)) et purifiées selon des méthodes décrites (Ghee M., Melki R., Michot N., Mallet J. PA700, the regulatory complex of the 26S proteasome, interferes with α-synuclein assembly. FEBS J. 2005, 272:4023-4033 ; Walsh DM, Thulin E, Minogue AM, Gustavsson N, Pang E, Teplow DB, Linse S. A facile method for expression and purification of the Alzheimer's disease-associated amyloid beta-peptide. FEBS J. 2009, 276:1266-1281). La concentration d'α-syn est déterminée par mesure de l'absorbance à 280 nm avec un coefficient d'extinction molaire à 5960 $M^{-1}cm^{-1}$. Pour l'Aβ1-42, la concentration est déterminée par méthode fluorimétrique avec le réactif fluorescamine (Sigma) qui réagit avec les amines primaires pour former un produit fluorescent (Udenfriend S, Stein S, Böhlen P, Dairman W, Leimgruber W, Weigele M. Fluorescamine: a reagent for assay of amino acids, peptides, proteins, and primary amines in the picomolar range. Science 1972, 178:871-872).

La protéine Tau humaine (isoforme h2N4RTau) est exprimée dans le vecteur pET11d chez *E. Coli* (souche BL21(DE3), (Stratagene, La Jolla, CA)) et purifiée selon la méthode décrite (Barghorn S, Biernat J, Mandelkow E. (2005) Methods Mol Biol. 299:35-51). La concentration de Tau purifiée est déterminée par mesure de l'absorbance à 280 nm en utilisant un coefficient d'extinction molaire de 7450 $M^{-1}cm^{-1}$.

**Exemple 19.2 Production et caractérisation des fibres α-syn, Aβ1-42 et Tau**

Pour obtenir l'assemblage en fibres, l'α-syn est incubée dans du Tris-HCl 50 mM pH7.5, KCl 150 mM à 37°C sous agitation continue pendant 4 jours. L'Aβ1-42 lyophilisée est dissoute dans du 1,1,1,3,3,3-Hexafluoro-2-propanol (HFIP) à 0.2 mg/ml et incubée pendant 24 h à 37°C afin d'obtenir la solubilisation complète. L'HFIP est évaporé sous courant d'azote afin d'obtenir un produit sec. Pour démarrer l'assemblage en fibres, le peptide sec est re-suspendu dans du PBS pH7.4, 10% (v/v) DMSO et incubé à 37°C pendant 3 jours sans agitation.

Le processus d'assemblage est suivi par mesure de liaison de la Thioflavine T (ThT) aux fibres (LeVine, H 3rd. Thioflavine T interaction with synthetic Alzheimer's disease beta-amyloid peptides: détection of amyloid aggregation in solution. Protein Sci. 1993, 2: 404-410). A intervalles réguliers, des aliquots protéiques de 10 μl sont prélevés et mélangés avec 400 μl de ThT (10 μM) dans l'eau. La fluorescence de la ThT est mesurée à l'aide d'un Cary Eclipse Spectrofluorometer (Varian Inc., Palo Alto, USA) (excitation: 440 nm, émission : 480 nm). Les fibres protéiques sont systématiquement observées par microscopie électronique (Jeol 1400). Les images sont enregistrées avec une caméra CCD (Gatan Orius). Pour finir, le pourcentage d'a-syn et Aβ1-42 agrégé en fibres est contrôlé sur le culot après centrifugation à 40000*xg* pendant 20 min et détermination de la concentration de protéine soluble/peptide restant dans le surnageant.

La protéine Tau dans un tampon PBS contenant 1 mM de DTT est assemblée en fibre en présence d'un douzième d'équivalent molaire d'héparine à 37°C sous agitation.

**Exemple 19.3 Inhibition in vitro de la liaison de la ThT et détermination du Ki**

Des solutions fraîches des composés à tester (2.5 mM dans DMSO) sont diluées dans du PBS pH7.4, à des concentrations entre 0.5 nM et 5 μM en présence de fibres d'a-syn (200 nM), d'Aβ1-42 (500 nM) ou de Tau (200 nM) et de ThT 500 nM dans un volume final de 2 ml. Les échantillons sont incubés pendant 1h à température ambiante (RT) pour atteindre l'équilibre. La liaison de la ThT aux fibres d'a-syn, d'Aβ1-42 ou de Tau est mesurée par fluorescence (excitation 440 nm, émission 480 nm). L'inhibition de la liaison de la ThT par les concentrations croissantes de chaque composé testé est mesurée par réduction de la fluorescence. En raison de la superposition partielle entre les spectres d'émission de certains composés et de la ThT, les composés sont aussi incubés avec des fibres d'α-syn, d'Aβ1-42 ou de Tau en absence de ThT. Pour chaque concentration, les valeurs de fluorescence en absence de ThT sont soustraites de celles en présence de ThT. Les résultats sont exprimés en pourcentage de la valeur maximale de fluorescence de la ThT mesurée en absence de compétiteur (100% liaison). A partir de chaque courbe, la valeur d'$IC_{50}$ est mesurée en utilisant l'équation suivante :

$$Y = \min + (\max - \min) / 1 + (Y/IC_{50})^{slope}$$

Le Ki est ensuite déterminé selon la méthode de Cheng et Prusoff (Cheng Y, Prusoff WH. Relationship between the inhibition constant (K1) and the concentration of inhibitor which causes 50 per cent inhibition (150) of an enzymatic reaction. Biochem Pharmacol 1973, 22:3099-3108).

$$Ki = IC_{50} / (1 + [ThT]/Kd_{ThT})$$

L'équation prend en compte la concentration de ThT utilisée (500 nM) et son affinité ($Kd_{ThT}$) pour les fibres d'a-syn, d'Aβ1-42 et/ou de Tau, à savoir 700 nM, 500 nM, et 350 nM, respectivement.

Les résultats obtenus sont rassemblés dans le tableau 5.

**Tableau 5**

**Tableau 5. Mesure Ki vs Thioflavine T**

| Référence | Structure | Ki Aβ (nM) | Ki αsyn (nM) | Ki Tau (nM) |
|---|---|---|---|---|
| *AV45* | | 2.2±1 | 76.6144 | 30.6±9 |
| *FDDNP* | | 4.6±3 | 25.1±5 | 9.5±1 |
| **22a** | 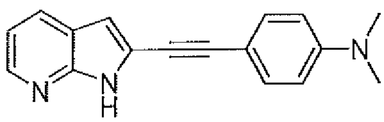 | 24.5±9 | 4.7±2 | 4.61±0.2 |
| **22b** | 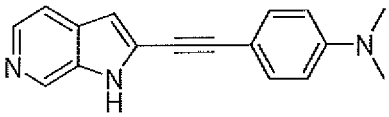 | 91.5±11 | 28.7±8 | 8.4±2 |
| **22c** | 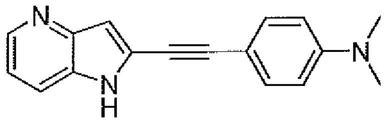 | 21.3±7 | 26.0±16 | 12.0±1 |

(suite)

| Référence | Structure | Ki Aβ (nM) | Ki αsyn (nM) | Ki Tau (nM) |
|---|---|---|---|---|
| **22d** | 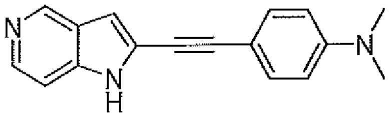 | | | |
| **22e** | 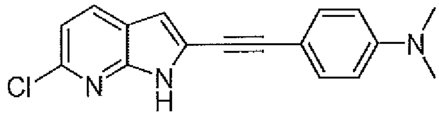 | 909±143 | 49.9±12 | 25.1±8 |
| **22f** | 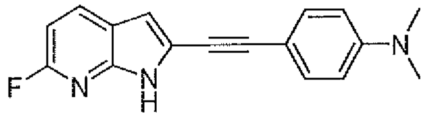 | 349±256 | 14.8±7 | |
| **22g** | 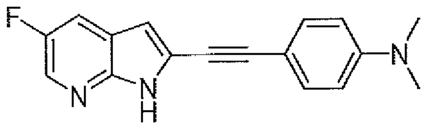 | 318.2±95 | 19.0±8 | |
| **22h** | 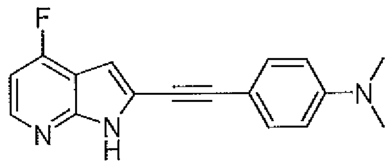 | 244.1±84 | 20.7±9 | |
| **22i** | 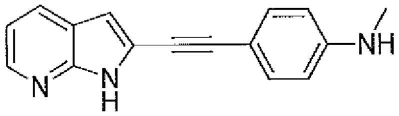 | | | |
| **22j** | 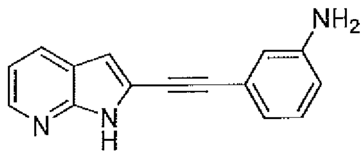 | | | |
| **22k** | 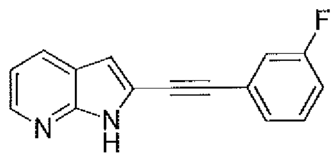 | | | |
| **22L** | 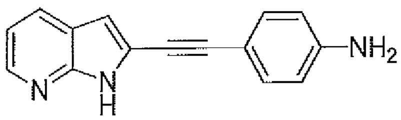 | | | |
| **22m** | 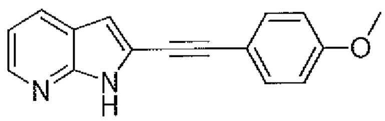 | | | |
| **22n** | 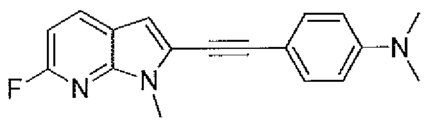 | | | |

(suite)

| Référence | Structure | Ki Aβ (nM) | Ki αsyn (nM) | Ki Tau (nM) |
|---|---|---|---|---|
| **25a** | 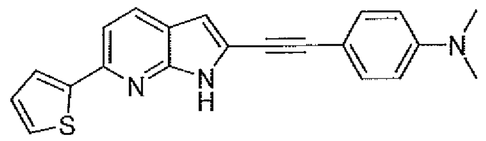 | | | |
| **25b** | 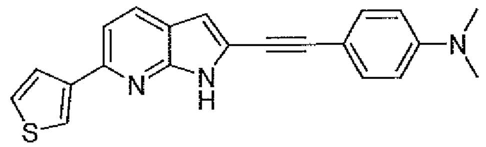 | | | |
| **25c** | 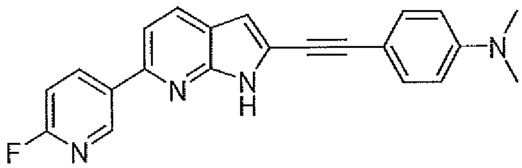 | | | |
| **28** | 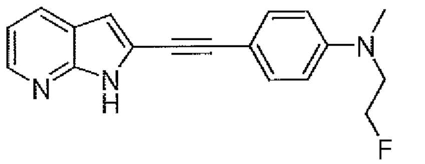 | | | |
| **35** | 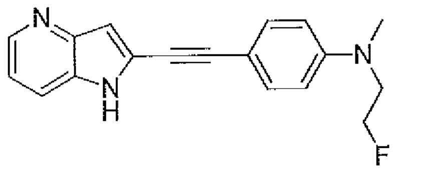 | | | |
| **37** | 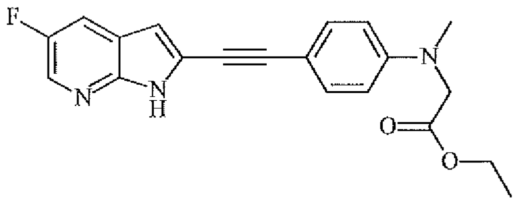 | | | |
| **38** | 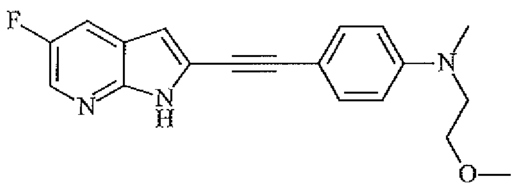 | | | |

**Exemple 19.4 Détermination directe du Kd et Bmax in vitro par fluorescence**

Pour certains des composés, l'$IC_{50}$ n'a pas pu être déterminée car leurs spectres d'émission étaient confondus avec celui de la ThT. Pour ces composés ainsi que pour ceux (série azaindole) qui montraient des compétitions importantes avec la ThT, des mesures directes en fluorescence ont été effectuées afin de déterminer leur affinité (Kd) et la densité de sites de liaison (Bmax) aux fibres.

Des solutions fraîches de chaque composé testé (2.5 mM dans DMSO) sont diluées dans du PBS pH = 7.4 à des concentrations entre 0.5 nM et 5 μM en présence ou absence de fibres d'a-syn (200 nM), d'Aβ1-42 (500 nM) ou de Tau (200 nM) dans un volume final de 2 ml. Après 1h d'incubation à RT, la liaison de chaque composé aux fibres d'a-syn, d'Aβ1-42 ou de Tau est mesurée par fluorescence grâce aux changements spectraux des composés lors de leur liaison aux fibres, en utilisant les longueurs d'onde d'excitation/émission appropriées (celles donnant la plus haute

variation de fluorescence lors de la liaison aux fibres). Pour chaque concentration de composé ajouté aux fibres, les fractions liées et libres sont mesurées par fluorescence selon l'équation suivante :

$$F_m = F_B \text{ x } B + F_F \text{ x } (T\text{-}B)$$

Où $F_m$ est la fluorescence mesurée en présence de fibres à une concentration T du composé rajouté aux fibres, $F_B$ est la fluorescence spécifique du composé lié, $F_F$ est la fluorescence spécifique du composé libre, et B est la concentration du composé lié aux fibres pour une concentration T donnée (Bell, J.E., "Fluorescence; Solution Studies", Spectrometry in Biochemistry, vol. I, Bell, J.E., ed., CRC Press, Inc., Boca Raton, FL, pp. 155-194 (1981)).

La valeur de $F_F$ est déterminée par la mesure de la fluorescence de concentrations connues de composés en absence de fibres. La valeur de $F_B$ est déterminée par la mesure de la fluorescence maximale de concentrations connues de composés en présence de concentrations saturantes des fibres d'a-syn, d'Aβ1-42, ou de Tau.

Ainsi à chaque point, la concentration de composé lié B est déterminée selon l'équation suivante :

$$B = (F_m - F_F \text{ x } T) / (F_B - F_F)$$

Les valeurs de B ainsi déterminées versus les concentrations des fractions libres (T-B) sont analysées selon l'équation de Michaelis-Menten. L'affinité (Kd) et la densité de sites de liaison au sein des fibres d'a-syn, Aβ1-42 ou Tau (Bmax) sont déterminées par analyse de Scatchard, qui permet aussi de mettre en évidence les multiples classes de sites.

Les résultats obtenus sont rassemblés dans les tableaux 6 et 7.

**Tableau 6. Mesure Kd & Bmax αsyn vs Aβ**

| Référence | Structure | Aβ Kd1 nM | Aβ Bmaxi nmol/μmol prot (nbre sites/monomère) | αsyn Kd1 nM | αsyn Bmax1 nmol/μmol prot (nbre sites/monomère) |
|---|---|---|---|---|---|
| *AV45* | | 1.68±0.2 | 3.33±0.2(1/300) | 24.3±20 | 27.9+19(1/35) |
| *FDDNP* | | 4.2±1 | 10.2±1(1/100) | 16.1±6 | 39.5±10(1/25) |
| **22a** | 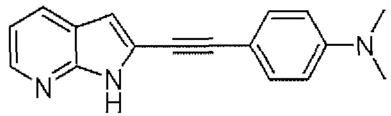 | 11.8±3 | 3.04±0.6(1/300) | 0.53±0.2 | 69.5±20(1/14) |
| **22b** | 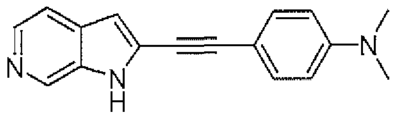 | 5.2±3 | 2.3±0.8(1/400) | 1.4±1 | 80.0±42(1/15) |
| **22c** | 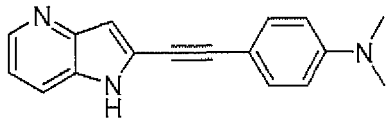 | 2.9±2 | 1.44±0.6(1/700) | 3.25±0.2 | 82.5±5(1/12) |
| **22d** | 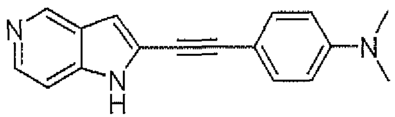 | 3.6±2 | 2.4±0.8(1/400) | 1.0±0.5 | 6.0±1(1/170) |
| **22f** | 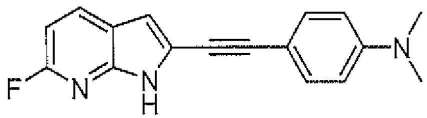 | 16.8±2 | 21.6±3(1/50) | 0.79±0.4 | 49.0±15(1/20) |
| **25a** | 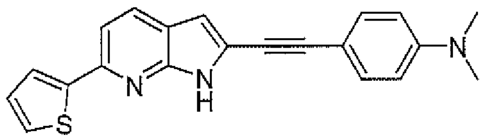 | 4.9±0.6 | 5.8±0.32(1/170) | 0.49±0.2 | 53.5±10(1/20) |

(suite)

| Référence | Structure | Aβ Kd1 nM | Aβ Bmaxi nmol/μmol prot (nbre sites/monomère) | αsyn Kd1 nM | αsyn Bmax1 nmol/μmol prot (nbre sites/monomère) |
|---|---|---|---|---|---|
| **25b** | 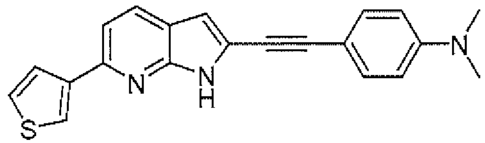 | 4.27±0.6 | 11.0±1(1/90) | 0.73±0.3 | 69.6±23(1/14) |
| **25c** | 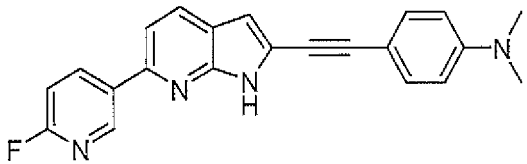 | 3.1±1 | 11.0±2(1/90) | 1.27±0.4 | 130±30(1/8) |
| **28** | 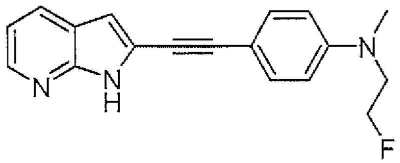 | 1.2±1 | 1.8±0.6(1/600) | 2.4±1 | 141.5±45(1/7) |

**Tableau 7. Mesure Kd & Bmax αsyn vs Tau**

| Référence | Structure | Tau Kd1 nM | Tau Bmax1 nmol/μmol prot (nbre sites/monomère) | αsyn Kd1 nM | αsyn Bmax1 nmol/μmol prot (nbre sites/monomère) |
|---|---|---|---|---|---|
| *AV45* | | 19.6±5 | 92.0±20(1/11) | 24.3±20 | 27.9±19(1/35) |
| *FDDNP* | | 34.9±6 | 138.5±16(1/7) | 16.1±6 | 39.5±10(1/25) |
| **22a** | 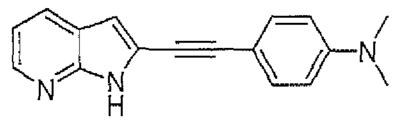 | 0.44±0.1 | 93.0±15(1/10) | 0.53±0.2 | 69.5±20(1/14) |
| **22b** | 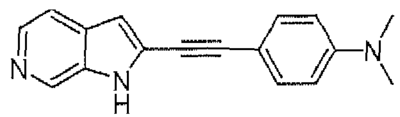 | 0.49±0.2 | 37.1+10(1/30) | 1.4±1 | 80.0±42(1/15) |
| **22c** | 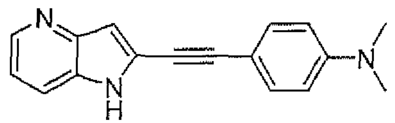 | 0.13±0.05 | 26.5±5(1/40) | 3.25±0.2 | 82.5±5(1/12) |
| **22d** | 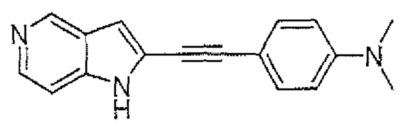 | 2.4±0.9 | 12.5±5(1/80) | 1.0±0.5 | 6.0±1(1/170) |
| **25a** | 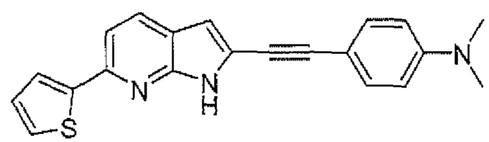 | 1.67±0.5 | 70.4±4(1/10) | 0.49±0.2 | 53.5±10(1/20) |
| **28** | 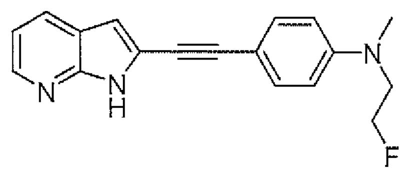 | 0.97±0.4 | 46.5±5(1/20) | 2.4±1 | 141.5±45(1/7) |

**Exemple 19.5 Evaluation in vivo d'un traceur d'α-syn marqué au fluor-18**

L'objectif est de déterminer si le traceur se lie in vivo aux fibres d'α-syn après injection intra-veineuse.

Modèle animal : rat mâle adulte de souche Wistar.

Préparation des fibres : les fibres d'α-syn produites selon le protocole décrit à l'exemple 19.2 sont centrifugées à 16000*xg* pendant 20 min pour deux fois et resuspendues dans du PBS pH 7.4.

Préparation des animaux : les fibres sont implantées par chirurgie stéréotaxique dans le striatum droit de l'animal anesthésié (Maia et al. Synapse 2012, 66 :573-83)

Etude in vivo : l'animal porteur de fibres reçoit une injection intra-veineuse du traceur à tester. Dès le moment de l'injection, l'imagerie est réalisée à l'aide d'un système dédié (eXplore VISTA/CT) qui enregistre l'accumulation intra-cérébrale de la radioactivité pendant 70 minutes. L'analyse des images obtenues permet de quantifier la liaison du traceur dans le striatum implanté par rapport au côté témoin (Sérrière et al. Nucl. Med. Biol. 2014, 41 :103-13 ; Sérrière et al. Neurobiol. Aging 2015, 36 :1639-52).

**Exemple 19.6 Evaluation in vivo d'un traceur d'a-syn marqué au fluor-18**

L'objectif est de déterminer si le traceur possède les propriétés de passage de la barrière hémato-encéphalique (BHE) dans des quantités suffisantes pour être employé comme traceur d'imagerie TEP. Par ailleurs la fixation osseuse peut être qualifiée, ce qui reflète le degré de défluoration du traceur in vivo.

Modèle animal : rat mâle adulte de souche Wistar.

Etude in vivo : l'animal sain reçoit une injection intra-veineuse du traceur à tester (molécule **28**). Dès le moment de l'injection, l'imagerie est réalisée à l'aide d'un système dédié (TEP/CT) qui enregistre l'accumulation intra-cérébrale de la radioactivité (Sérrière et al. Nucl. Med. Biol. 2014, 41 :103-13 ; Sérrière et al. Neurobiol. Aging 2015, 36 :1639-52). A l'issue de l'imagerie, l'animal est euthanasié ; le cerveau ainsi qu'un fragment d'os (fémur) sont prélevés, pesés et leur radioactivité est mesurée. Cette méthode permet de quantifier précisément l'accumulation du traceur dans les tissus. Les résultats sont exprimés en pourcentage de la dose de traceur injecté / g de tissu (%DI/g)

Résultats :

**Passage de la BHE (≈0.4%DI/g)**

**Faible liaison à l'os (0.3%DI/g)**

**Exemple 20. Procédure H (Couplage de Buchwald)**

Sous argon, $Pd_2dba_3$ (0.05 éq.) et BINAP (0.075 mmol) sont ajoutés à une solution de toluène anhydre dégazée de concentration 0.3 M contenant l'amine (1.0 éq.), le 1,4-dibromobenzène (1.0 éq.) et t-BuONa (1.2 éq.). Au bout de 16h à 80°C, le solvant est évaporé sous pression réduite. Ensuite, le mélange réactionnel est concentré sous pression réduite, avant d'être purifié par colonne de chromatographie flash sur gel de silice.

**Exemple 21. Procédure I (Préparation des alcynes vrais)**

**Exemple 21.1 I1**

Sous argon, $Pd(PPh_3)_2Cl_2$ (0.05 éq.) et CuI (0.1 mmol) sont ajoutés à une solution de pipéridine dégazée de concentration 0.1 M contenant l'éthynyltrimethylsilane (4.0 éq.), le dérivé bromé (1.0 éq.) et triphénylphosphine (0.2 éq.). Au bout de 16h à 85°C, le solvant est évaporé sous pression réduite. Ensuite, le mélange réactionnel est purifié par colonne de chromatographie flash sur gel de silice. Puis, l'alcyne intermédiaire est mis en réaction avec $K_2CO_3$ (1.5 éq.) dans le méthanol (0.03M). Au bout de 3h à température ambiante, le solvant est évaporé sous pression réduite. Le brut est solubilisé dans 10mL d'acétate d'éthyle. Puis la phase organique est lavée avec de la saumure (3 X 10 mL). Ensuite la phase organique est séchée avec $MgSO_4$, filtrée sur coton et évaporée sous pression réduite. Pour finir, le brut est purifié par colonne de chromatographie flash sur gel de silice avec comme éluant (acétate d'éthyle/éther de pétrole) pour conduire à l'alcyne vrai.

**Exemple 21.2. I2**

Du catalyseur [$Pd(PPh_3)_2Cl_2$] (0.025 éq.) a été ajouté à une solution dégazée du bromé **40** (1.0 éq.), Ethynyltrimethylsilane (1.4 éq.), de CuI (0.05 éq.), dans un mélange THF/$Et_3N$ (1/1). Le mélange est agité à température ambiante pendant 20 h sous atmosphère d'argon. Ensuite, le mélange réactionnel est concentré sous pression réduite, avant d'être purifié par colonne de chromatographie flash sur gel de silice avec comme éluant (acétate d'éthyle/éther de pétrole = 2/98) pour obtenir l'intermédiaire sylilé qui est mis en réaction avec du KOH (2.0 éq.) dans le méthanol. Au bout de 10 min d'agitation à température ambiante, du dichlorométhane ainsi que de l'eau sont ajoutés. Les phases organiques sont extraites 3 fois au dichlorométhane. Elles sont réunies, séchées sur $MgSO_4$, filtrées sur coton puis évaporées à sec pour conduire à l'alcyne attendu.

**Exemple 22. Procédure J (Désylilation)**

A 0°C et sous argon, une solution de TBAF dans le THF (C = 1 M, 1.5 éq.) est ajouté goutte à goutte au milieu réactionnel contenant le composé sylilé (1.0 éq.) dilué dans le THF. Au bout de 2 h à température ambiante, de l'eau (20 mL) et de l'acétate d'éthyle (20 mL) sont ajoutés successivement. Les phases organiques sont extraites 3 fois avec de l'acétate d'éthyle (20 mL). Puis, elles sont réunies, séchées avec du $MgSO_4$, filtrées sur coton et évaporées sous pression réduite. Pour finir, le brut est purifié par colonne de chromatographie flash sur gel de silice avec comme éluant (méthanol/dichlorométhane) pour conduire à l'alcool primaire.

**Exemple 23. Procédure K (Amination réductrice)**

L'aldéhyde (1.0 éq.) est mis en réaction avec une amine (6.0 éq.) en présence de $NaBH(OAc)_3$ (10 éq.) et de $Et_3N$ (11 éq.) dans le DCE. Au bout de 4h à température ambiante, le mélange est évaporé à sec puis 10 mL d'une solution de $NaHCO_3$ saturée et 10 mL d'acétate d'éthyle sont ajoutés. La phase organique est extraite avec de l'acétate d'éthyle (3 x 10 mL). Les phases organiques sont réunies et lavées avec une solution de NaCl saturée puis séchées sur $MgSO_4$, filtrées sur coton et évaporées à sec. Le brut est purifié par colonne de chromatographie flash sur gel de silice pour conduire au composé attendu.

**Exemple 24.2-{2-[4-(2-fluoroethoxy)phenyl]ethynyl}-1*H*-pyrrolo[2,3-*b*]pyridine 43**

Le 2-{2-[4-(2-fluoroethoxy)phenyl]ethynyl}-1*H*-pyrrolo[2,3-*b*]pyridine a été synthétisé en 3 étapes. Dans un premier temps, l'alcyne correspondant a été préparé en 2 étapes et puis nous avons effectué la réaction de Sonogashira avec l'azaindole pour obtenir le composé final.

**Exemple 24.1 Préparation du 1-ethynyl-4-(2-fluoroéthoxy)benzene 39 :**

L'alcyne **39** a été préparé en trois étapes en partant du 4-iodophénol **40.** La première étape a été décrite par Hirose et al. (Chemical Communications, 2009, 39 p. 5832 - 5834) et pour les deux dernières étapes nous avons utilisé la méthode décrite par Ouach et al. (European Journal of Medicinal Chemistry, 2016, vol. 107, p. 153 - 164).

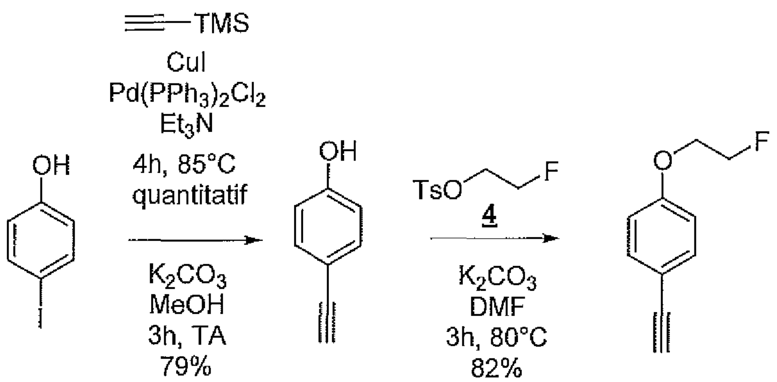


**40** **41** **39**

Du catalyseur [$Pd(PPh_3)_2Cl_2$] (39 mg, 0.055 mmol, 0.03 éq.) a été ajouté à une solution dégazée du iodé **39** (400 mg, 1.82 mmol, 1.0 éq.), ethynyltrimethylsilane (0.386 mL, 2.73 mmol, 1.5 éq.), de CuI (10.4 mg, 0.055 mmol, 0.03

éq.), dans 6 mL de $Et_3N$. Le mélange est agité à 85°C ambiante pendant 4 h sous atmosphère d'argon. Ensuite, le mélange réactionnel est concentré sous pression réduite, avant d'être purifié par colonne de chromatographie flash sur gel de silice avec comme éluant (acétate d'éthyle/éther de pétrole = 20/80) pour obtenir 200 mg de l'intermédiaire silylé qui est mis en réaction avec du $K_2CO_3$ (351 mg, 2.4 éq.) dans 15 mL de méthanol. Au bout de 3h d'agitation à température ambiante, la solution est filtrée sur coton. Après évaporation à sec, le brut est purifié par colonne de chromatographie flash sur gel de silice avec comme éluant (acétate d'éthyle/éther de pétrole = 30/70) pour obtenir l'alcyne **41** sous forme d'un solide marron avec un rendement de 79% sur 2 étapes.

Celui-ci est mis en réaction avec le dérivé tosylé **42** en présence de $K_2CO_3$ dans le DMF. Au bout de 3h à 80°C, le mélange réactionnel est refroidi et 10 mL d'$H_2O$ est introduit. La phase organique est extraite avec de l'acétate d'éthyle (3 x 10 mL). Puis les phases organiques sont réunies, séchées avec du $MgSO_4$ et filtrées sur coton. Après évaporation sous pression réduite, le brut est purifié par colonne de chromatographie flash sur gel de silice avec comme éluant (acétate d'éthyle/éther de pétrole = 15/85) pour obtenir l'alcyne **39** sous forme d'une huile incolore avec un rendement de 82%.

**Exemple 24.2 2-{2-[4-(2-fluoroethoxy)phenyl]ethynyl}-1*H*-pyrrolo[2,3-*b*]pyridine 43**

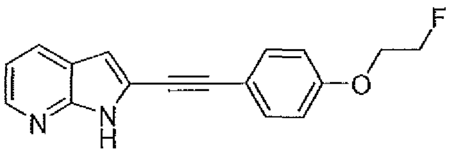

$C_{17}H_{13}FN_2O$

M W: 280,30 gmol-1

Le composé a été préparé selon la **procédure C1** et purifié par colonne de chromatographie flash sur gel de silice (acétate d'éthyle/éther de pétrole = 30/70). Solide beige (38%), **Pf** 250-252°C, $R_f$ = 0.20 (acétate d'éthyle/éther de pétrole = 30/70). **Infrarouge** (ν, $cm^{-1}$, neat) 3122, 3054, 2978, 2883, 2790, 1603, 1582, 1534, 1498, 1277, 1249, 1175, 1078, 1051, 921. **RMN $^1$H** (400 MHz, DMSO-$d_6$, 20°C) δ 12.11 (s, 1H), 8.22 (s, 1H), 7.90 (d, *J* = 7.9 Hz, 1H), 7.49 (d, *J* = 8.6 Hz, 2H), 7.10 - 6.95 (m, 4H), 6.74 (s, 1H), 4.82 (t, *J* = 6.2 Hz, 1H), 4.62 (t, J = 6.2 Hz, 1H), 4.32 (t, *J* = 5.7 Hz, 1H), 4.20 (t, *J* = 5.7 Hz, 1H). **RMN $^{13}$C** (63 MHz, DMSO-$d_6$, 20°C) δ 159.2 (Cq), 144.7 (CH), 133.5 (2xCH), 128.7 (CH), 120.0 (Cq), 119.7 (Cq), 116.8 (CH), 115.6 (2 x CH), 114.4 (Cq), 106.2 (CH), 93.2 (Cq), 82.6 (d, *J* = 151.0 Hz, $CH_2$), 81.4 (Cq), 67.8 (d, *J* = 18.8 Hz, $CH_2$). **RMN $^{19}$F** (376 MHz, DMSO-$d_6$, 20°C) δ - 222.2. **HRMS (+ESI)** calculée pour $C_{17}H_{13}FN_2O$ ($M+H^+$) : 281.1084, trouvée: 281.1085.

**Exemple 25 : 2-(2-{4-[2-fluoroethoxy)methyl]phenyl}ethynyl)-1*H*-pyrrolo[2,3-*b*]pyridine 44**

Le 2-(2-{4-[(2-fluoroethoxy)methyl]phenyllethynyl)-1*H*-pyrrolo[2,3-*b*]pyridine **44** a été synthétisé en 2 étapes. Dans un premier temps, l'alcyne correspondant **45** a été préparé en 1 étape et puis nous avons effectué la réaction de Sonogashira avec l'azaindole pour obtenir le composé final.

**Exemple 25.1 Synthèse du 1-ethynyl-4-((2-fluoroethoxy)methyl)benzene 45** (US2015/196672 A1)

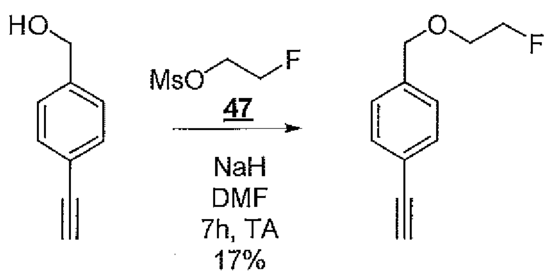


**46** **45**

Sous argon, 100 mg d'alcyne **46** (0.756 mmol, 1.0 éq.) est solubilisé dans 5 mL de DMF. A 0°C, 51 mg de NaH (50% dans de l'huile minérale, 1.06 mmol, 1.4 éq.) est ajouté par portion puis au bout de 5 minutes, 118 mg de mésyle

47 (0.832 mmol, 1.1 éq.) est ajouté goutte à goutte. Au bout de 7h à température ambiante, 10 ml d'$H_2O$ sont ajoutés puis la phase organique est extraite avec l'acétate d'éthyle (3 x 10 mL). Ensuite les phases organiques sont réunies, séchées et filtrées sur coton. Après évaporation sous pression réduite, le brut est purifié par colonne de chromatographie flash sur gel de silice avec comme éluant (acétate d'éthyle/éther de pétrole = 5/45) pour obtenir l'alcyne **45** sous forme d'une huile incolore avec un rendement de 17%. **RMN $^1$H** (250 MHz, $CDCl_3$) $\delta$ 7.44 (d, *J* = 8.2 Hz, 2H), 7.28 (d, *J* = 8.2 Hz, 2H), 4.68 - 4.61 (m, 1H), 4.56 (s, 2H), 4.50 - 4.42 (m, 1H), 3.77 - 3.72 (m, 1H), 3.65 - 3.60 (m, 1H), 3.03 (s, 1H). Numéro CAS : 1562413-13-1.

**Exemple 25.2 2-(2-{4-[(2-fluoroethoxy)methoxy)methyl]phenyl}ethynyl)-1*H*-pyrrolo[2,3-*b*]pyridine 44**

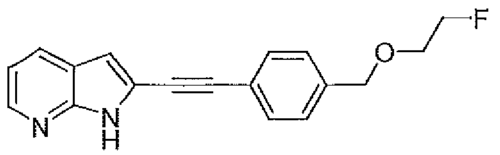

$C_{18}H_{15}FN_2O$

MW: 294,33 gmol-1

Le composé a été préparé selon la **procédure C1** et purifié par colonne de chromatographie flash sur gel de silice (acétate d'éthyle/éther de pétrole = 30/70). Solide jaune (57%), **Pf** 192-194°C, $R_f$ = 0.18 (acétate d'éthyle/éther de pétrole = 30/70). **Infrarouge** (ν, $cm^{-1}$, neat) 3122, 3054, 2978, 2883, 2790, 1603, 1582, 1534, 1498, 1277, 1249, 1175, 1078, 1051, 921. **RMN $^1$H** (400 MHz, $CDCl_3$, 20°C) $\delta$ 10.81 (s, 1H), 8.39 (s, 1H), 7.93 (s, 1H), 7.58 (s, 2H), 7.40 (s, 2H), 7.12 (s, 1H), 6.78 (s, 1H), 4.73 - 4.51 (m, 4H), 3.76 (d, *J* = 29.5 Hz, 2H). **RMN$^{13}$C** (101 MHz, $CDCl_3$, 20°C) $\delta$ 148.4 (Cq), 144.0 (CH), 138.8 (Cq), 131.7 (2xCH), 129.1 (CH), 127.7 (2xCH), 121.6 (Cq), 120.6 (Cq), 119.8 (Cq), 116.6 (CH), 106.6 (CH), 93.4 (Cq), 83.9 (d, *J* = 169.4 Hz, $CH_2$) 81.5 (Cq), 72.9 ($CH_2$), 69.6 (d, *J* = 19.7 Hz, $CH_2$). **RMN $^{19}$F** (376 MHz, DMSO-$d_6$, 20°C) $\delta$ -221.4. **HRMS (+ESI)** calculée pour $C_{18}H_{15}FN_2O$ (M+H$^+$): 295.1241, trouvée: 295.1242.

**Exemple 26: 2-(2-{4-[2-(2-fluoroethoxy)ethoxy]phenyl}ethynyl)-1*H*-pyrrolo[2,3-*b*]pyridine 48**

Le 2-(2-{4-[2-(2-fluoroethoxy)ethoxy]phenyl}ethynyl)-1H-pyrrolo[2,3-b]pyridine **48** a été synthétisé en 3 étapes. Dans un premier temps, l'alcyne correspondant **49** a été préparé en 2 étapes à partir du tosylé **50** et puis nous avons effectué la réaction de Sonogashira avec l'azaindole pour obtenir le composé final.

**Exemple 26.1 Synthèse de 1-ethynyl-4-[2-(2-fluoroethoxy)ethoxy]benzene 49 :**

L'alcyne **49** est obtenu en 3 étapes en partant du ditosylé **50** qui a été préparer selon la méthode de Herbert (Organic Letters, 2013, 15, 13 p. 3334- 3337).

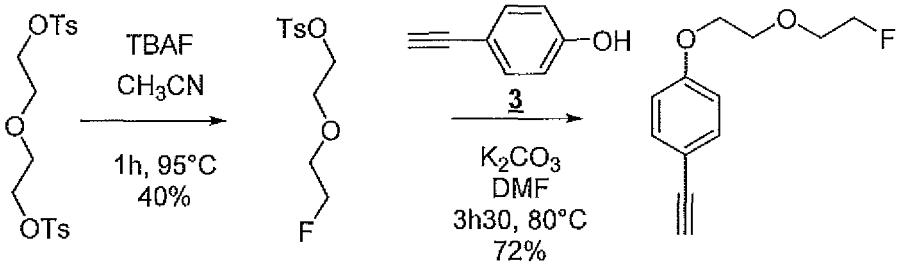


**50** **51** **49**

Synthèse du fluoré **51:**

Sous argon, le composé **50** (300 mg, 0.724 mmol, 1.0 éq.) est solubilisé dans 17 mL de $CH_3CN$. Ensuite, le TBAF (0.796 mL, 0.796 mmol, 1.1 éq.) est ajouté goutte à goutte. Au bout d'1h à 95°C, le mélange réactionnel est refroidi à température ambiante et évaporé sous pression réduite. Le brut est purifié par colonne de chromatographie flash sur gel de silice avec comme éluant (acétate d'éthyle/éther de pétrole = 2/4) pour obtenir le fluoré **51** sous forme d'une huile incolore avec un rendement de 40%. **RMN $^1$H** (400 MHz, $CDCl_3$) $\delta$ 7.83 (d, *J* = 8.3 Hz, 2H), 7.37 (d, *J* = 8.3 Hz, 2H), 4.61 - 4.52 (m, 1H), 4.47 - 4.43 (m, 1H), 4.24 - 4.14 (m, 2H), 3.78 - 3.70 (m, 3H), 3.68 - 3.63 (m, 1H), 2.47 (s, 3H).

Numéro CAS : 1118567-11-5

Synthèse de l'alcyne **49** :

L'alcyne **41** (43.4 mg, 0.367 mmol, 1.0 éq.) et le fluoré **51** (107.0 mg, 0.408 mmol, 1.1 éq.) sont mis en présence de $K_2CO_3$ (101.3 mg, 0.734 mmol, 2.0 éq.) dans 3 mL de DMF. Au bout de 3h à 80°C, le mélange réactionnel est refroidi et 10 mL d'$H_2O$ est introduit. La phase organique est extraite avec de l'acétate d'éthyle (3 x 10 mL). Puis les phases organiques sont réunies, séchées avec du $MgSO_4$ et filtrées sur coton. Apres evaporation sous pression reduite, le brut est purifié par colonne de chromatographie flash sur gel de silice avec comme éluant (acétate d'éthyle/éther de pétrole = 5/45) pour obtenir l'alcyne **49** sous forme d'une huile incolore avec un rendement de 72%. **Rf** = 0.65 (acétate d'éthyle/éther de pétrole = 20/80). **Infrarouge** (v, cm-1, neat) 3286, 2889, 2359, 2105, 1604, 1571, 1505, 1454, 1356, 1287, 1245, 1171, 1134, 1046, 927, 873. **RMN $^1$H** (250 MHz, $CDCl_3$, 20°C) δ 7.39 (d, *J* = 8.8 Hz, 2H), 6.83 (d, *J* = 8.8 Hz, 2H), 4.70 - 4.40 (m, 2H), 4.15 - 4.08 (m, 2H), 3.90 - 3.80 (m, 3H), 3.76 - 3.68 (m, 1H), 2.97 (s, 1H). **RMN $^{13}$C** (63 MHz, $CDCl_3$, 20°C) δ 159.0 (Cq), 133.6 (2 x CH), 114.6 (2 x CH), 114.5 (Cq), 83.1 (d, *J* = 169.1 Hz, $CH_2$), 83.6 (Cq), 75.9 (CH), 70.6 (d, *J* = 19.7 Hz, $CH_2$), 69.8 ($CH_2$), 67.5 ($CH_2$). **RMN $^{19}$F** (235 MHz, $CDCl_3$, 20°C) δ -223.0. **HRMS** (+ESI) calculée pour $C_{12}H_{13}FO_2$ (M+H+) : 209.0972, trouvée: 209.09714.

**Exemple 26.2 2-(2-{4-[2-(2-fluoroethoxy)ethoxy]phenyl}ethynyl)-1*H*-pyrrolo[2,3-*b*]pyridine 48**

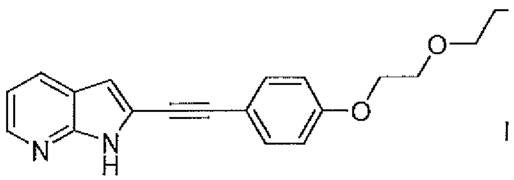

$C_{19}H_{17}FN_2O_2$

M W: 324,36 gmol-1

Le composé a été préparé selon la **procédure C1** et purifié par colonne de chromatographie flash sur gel de silice (acétate d'éthyle/éther de pétrole = 10/90). Solide jaunâtre (75%), **Pf** 156-158°C, $\boldsymbol{R_f}$ = 0.33 (acétate d'éthyle/éther de pétrole = 10/90). **Infrarouge** (v, $cm^{-1}$, neat) 3122, 3054, 2978, 2883, 2790, 1603, 1582, 1534, 1498, 1277, 1249, 1175, 1078, 1051, 921 . **RMN $^1$H** (400 MHz, $CDCl_3$, 20°C) δ 9.97 (s, 1H), 8.35 (d, *J* = 4.7 Hz, 1H), 7.90 (dd, *J* = 7.8, 1.5 Hz, 1H), 7.51 (d, *J* = 8.7 Hz, 2H), 7.10 (dd, *J* = 7.9, 4.7 Hz, 1H), 6.94 (d, *J* = 8.7 Hz, 2H), 6.73 (s, 1H), 4.73 - 4.47 (m, 2H), 4.19 (m, 2H), 3.92 (m, 3H), 3.83 (m, 2H). **RMN $^{13}$C** (63 MHz, $CDCl_3$, 20°C) 159.6 (Cq), 148.7 (Cq), 144.4 (CH), 138.2 (Cq), 133.6 (2 x CH), 129.3 (CH), 120.5 (Cq) 116.9 (CH), 115.3 (2 x CH), 115.12 (Cq), 106.8 (CH), 92.8 (Cq), 83.7 (d, *J* = 177.2 Hz, $CH_2$), 80.7 (Cq), 71.1 (d, *J* = 19.7 Hz, $CH_2$), 70.4 ($CH_2$), 67.7 ($CH_2$). **RMN $^{19}$F** (235 MHz, $CDCl_3$, 20°C) δ.-223.0. **HRMS (+ESI)** calculée pour $C_{19}H_{17}FN_2O_2$ (M+$H^+$) : 325.1326, trouvée: 325.1347.

**Exemple 27. 4-1(1*H*-pyrrolo[2,3-*b*]pyridin-2-yl)ethynyl)-*N*-benzyl-*N*-(2-fluoroethyl)aniline 52**

Le 4-((1*H*-pyrrolo[2,3-*b*]pyridin-2-yl)ethynyl)-*N*-benzyl-*N*-(2-fluoroethyl)aniline a été synthétisé en 5 étapes à partir de la benzylamine **54.** Dans un premier temps, l'alcyne correspondant **53** a été préparé en 4 étapes à partir de la benzylamine **54** et puis nous avons effectué la réaction de Sonogashira avec l'azaindole pour obtenir le composé final.

**Exemple 27.1 Synthèse de *N*-benzyl-4-ethynyl-*N*-(2-fluoroethyl)aniline 53:**

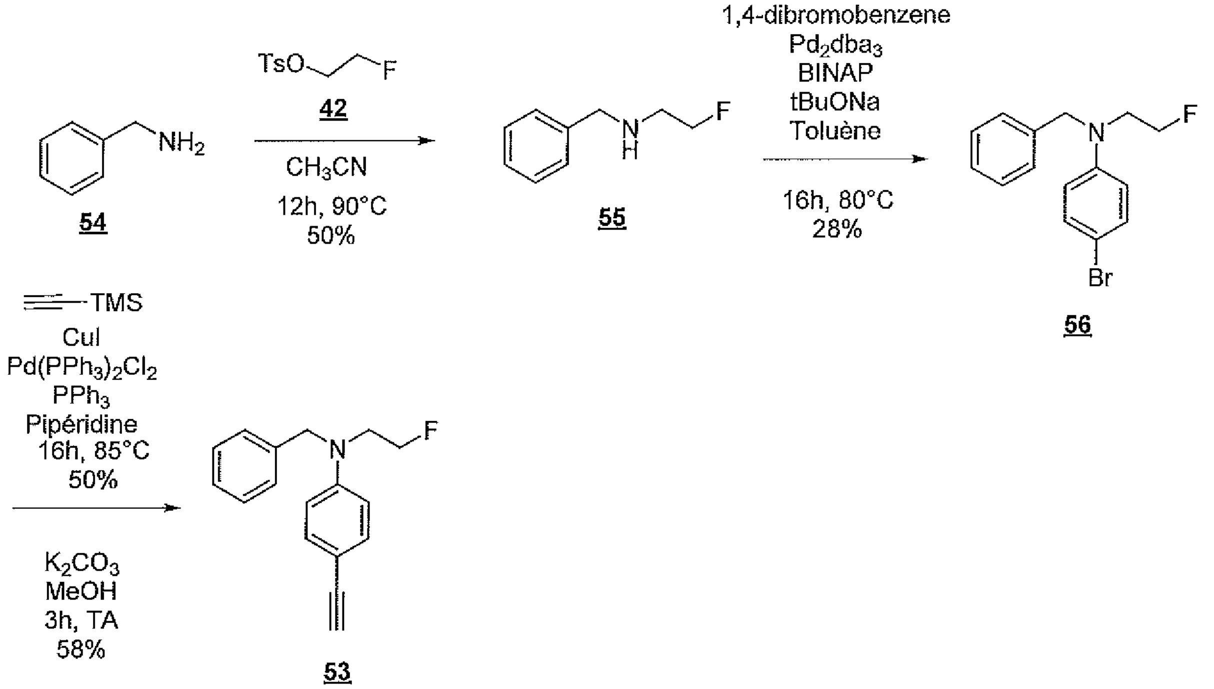


54 55 56 53

Synthèse de l'amine **55** :

Sous argon, 130 mg du composé **42** (0.985 mmol, 1.0 éq.) est solubilisé dans 3 mL de $CH_3CN$. Après l'ajout de 0.40 mL de benzylamine **54** (2.38 mmol, 4.0 éq.), le mélange réactionnel est chauffé à 90°C pendant 12h. Après refroidissement du milieu réactionnel, 10 mL d'une solution saturée de $NaHCO_3$ est ajoutée. Ensuite, la phase organique est extraite avec du DCM (3 x 10 mL). Puis les phases organiques sont réunies, séchées avec du $MgSO_4$ et filtrées sur coton. Après évaporation sous pression réduite, le brut est purifié par colonne de chromatographie flash sur gel de silice avec comme éluant (méthanol/dichlorométhane /$NH_4OH$ = 2/48/0.5) pour obtenir l'amine **55** sous forme d'une huile incolore avec un rendement de 50%. **RMN $^1$H** (250 MHz, $CDCl_3$) δ 7.31 (m, 5H), 4.69 - 4.32 (m, 2H), 3.82 (s, 2H), 3.02 - 2.73 (m, 2H). Numéro CAS : 122974-04-3.

Synthèse de l'amine **56** :

Le composé **56** a été préparé selon la **procédure D** et purifié par colonne de chromatographie flash sur gel de silice (acétate d'éthyle/éther de pétrole = 1/49). Huile incolore (28%), $\boldsymbol{R_f}$ = 0.50 (acétate d'éthyle/éther de pétrole = 5/45). **Infrarouge** (ν, $cm^{-1}$, neat) 3028, 2951, 2359, 1591, 1493, 1451, 1386, 1352, 1228, 1193, 1173, 1101, 1080, 1046, 1003, 912, 882. **RMN $^1$H** (250 MHz, $CDCl_3$, 20°C) δ 7.39 - 7.19 (m, 7H), 6.66 - 6.57 (m, 2H), 4.71 (t, *J* = 5.3 Hz, 1H), 4.66 - 4.55 (m, 3H), 3.76 (dt, *J* = 23.5, 5.3 Hz, 2H). **RMN $^{13}$C** (63 MHz, $CDCl_3$, 20°C) δ 147.25 (Cq), 137.89 (Cq), 131.9 (2 x CH), 128.7 (2 x CH), 127.09 (Cq), 126.4 (2 x CH), 114.2 (2 x CH), 108.9 (Cq), 81.6 (d, *J* = 21.5 Hz, $CH_2$), 55.0 ($CH_2$), 51.3 (d, *J* = 21.5 Hz, $CH_2$). **RMN $^{19}$F** (235 MHz, $CDCl_3$, 20°C) δ. -221.7. **HRMS (+ESI)** calculée pour $C_{15}H_{15}BrFN$ ($M+H^+$) : 308.0444, trouvée: 308.0445.

Synthèse de l'alcyne vrai **53** :

L'alcyne **11** a été préparé selon la **procédure** E et purifié par colonne de chromatographie flash sur gel de silice (acétate d'éthyle/éther de pétrole = 2/48). Huile incolore (29% sur les deux étapes), $\boldsymbol{R_f}$ = 0.50 (acétate d'éthyle/éther de pétrole = 2/48). **Infrarouge** (ν, $cm^{-1}$, neat) 3028, 2951, 2359, 1591, 1493, 1451, 1386, 1352, 1228, 1193, 1173, 1101, 1080, 1046, 1003, 912, 882. **RMN $^1$H** (400 MHz, $CDCl_3$, 20°C) δ 7.37 - 7.10 (m, 7H), 6.68 - 6.54 (m, 2H), 4.62 (s, 2H), 4.61 (dt, *J* = 47.1, 5.3 Hz, 2H), 3.74 (dt, *J* = 23.3, 5.3 Hz, 2H), 2.93 (s, 1H), 4.76 - 4.58 (m, 3H). **RMN $^{13}$C** (101 MHz, $CDCl_3$, 20°C) δ 147.3 (Cq), 137.8 (Cq), 133.4 (2 x CH), 128.7 (2 x CH), 127.09 (Cq), 126.9 (2 x CH), 112.0 (2 x CH), 108.98 (Cq), 84.1 (Cq), 81.5 (d, *J* = 21.5 Hz, $CH_2$), 75.0 (CH), 54.0 ($CH_2$), 50.9 (d, *J* = 21.5 Hz, $CH_2$). **RMN $^{19}$F** (376 MHz, $CDCl_3$, 20°C) δ. -221.7. **HRMS (+ESI)** calculée pour $C_{17}H_{16}FN$ ($M+H^+$) : 254.1339, trouvée: 254.1344.

Exemple 27.2 4-((1*H*-pyrrolo[2,3-*b*]pyridin-2-yl)ethynyl)-*N*-benzyl-*N*-(2-fluoroethyl)aniline 52

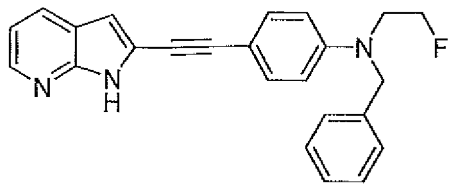

$C_{24}H_{20}FN_3$

M W: 369,44 gmol-1

Le composé a été préparé selon la **procédure C1** et purifié par colonne de chromatographie flash sur gel de silice (acétate d'éthyle/éther de pétrole = 30/70). Solide jaune (66%), **Pf** 184-186°C, $R_f$ = 0.20 (acétate d'éthyle/éther de pétrole = 30/70). **Infrarouge** (v, $cm^{-1}$, neat) 3054, 2885, 2201, 1601, 1538, 1508, 1401, 1355, 1279, 1236, 1189, 1050, 811. **RMN $^1$H** (250 MHz, DMSO-*d6*, 20°C) δ 12.06 (s, 1H), 8.23 (m, 1H), 7.91 (d, *J* = 7.9 Hz, 1H), 7.42 - 7.25 (m, 3H), 7.23 (t, *J* = 7.6 Hz, 2H), 7.07 (dd, *J* = 7.6, 4.7 Hz, 1H), 6.82 - 6.64 (m, 2H), 4.70 (s, 1H), 4.66 (dt, *J* = 47.8, 5.2 Hz, 3H), 3.85 (dt, *J* = 25.5, 5.2 Hz, 2H). **RMN $^{13}$C** (63 MHz, DMSO-$d_6$, 20°C) δ 150.5 (Cq), 148.5 (Cq), 144.2 (CH), 138.7 (Cq), 133.1 (2 x CH), 129.2 (2 x CH), 128.6 (CH), 127.5 (CH), 126.9 (2 x CH), 120.4 (Cq), 120.2 (Cq), 116.6 (CH), 112.8 (2 x CH), 108.4 (Cq), 105.2 (CH), 94.3 (Cq), 83.7 (Cq), 80.8 (d, *J* = 168.8 Hz, $CH_2$), 53.7 ($CH_2$). **RMN $^{19}$F** (235 MHz, DMSO-$d_6$, 20°C) δ. -221.7. **HRMS (+ESI)** calculée pour $C_{24}H_{20}FN_3$ ($M+H^+$) : 370.1714, trouvée: 370.1413.

Exemple 28 *N*-(4-((1*H*-pyrrolo[2,3-*b*]pyridin-2-yl)ethynyl)phenyl)-*N*-(2-fluoroethyl)benzenesulfonamide 57

Le *N*-(4-((1*H*-pyrrolo[2,3-*b*]pyridin-2-yl)ethynyl)phenyl)-*N*-(2-fluoroethyl)benzenesulfonamide **57** a été synthétisé en 3 étapes à partir de l'alcyne **59.** Dans un premier temps, l'alcyne correspondant **58** a été préparé en 2 étapes et puis nous avons effectué la réaction de Sonogashira avec l'azaindole pour obtenir le composé final.

Exemple 28.1 Synthèse du N-(4-ethynylphenyl)-N-(2-fluoroethyl)benzenesulfonamide 58 :

La synthèse de l'alcyne **58** est obtenue en 2 étapes en partant de l'alcyne **59.** L'alcyne **60** est préparé en utilisant les conditions de Sakai et al. (Macromolecules, 2012, 45, 20 8221 - 8227,7).

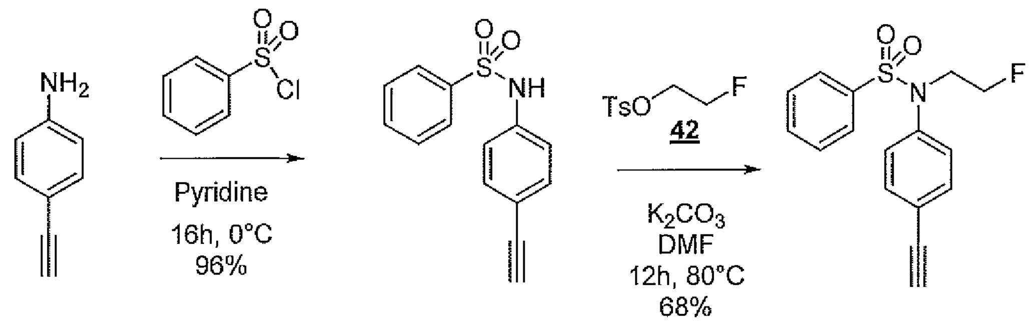


59 60 58

Synthèse de l'alcyne **60** :

Sour argon, l'alcyne **59** (100 mg, 0.854 mmol, 1.05 éq.) est solubilisé dans la pyridine. A 0°C, le chlorure de benzenesulfonyle (0.10 mL, 0.811 mmol, 1.0 éq.) est additionné goutte à goutte. Ay bout de 16h à 0°C, 10 ml d'une solution de HCl à 1M est additionné doucement. Ensuite, la phase organique est extraite avec de l'acétate d'éthyle (3 x 10 mL). Puis les phases organiques sont réunies, séchées avec du $MgSO_4$ et filtrées sur coton. Après évaporation sous pression réduite, l'alcyne **60** est obtenu sans purification avec un rendement de 96% sous forme d'un solide jaunâtre. **RMN $^1$H** (250 MHz, $CDCl_3$) δ 7.83 - 7.73 (m, 2H), 7.58 - 7.31 (m, 5H), 7.02 (d, J = 8.6 Hz, 2H), 6.57 (si, 1H), 3.05 (s, 1H). Numéro CAS : 383147-75-9.

Synthèse du *N*-(4-ethynylphenyl)-*N*-(2-fluoroethyl)benzenesulfonamide **58** :

L'alcyne **60** (50.0 mg, 0.194 mmol, 1.0 éq.) et le fluoré **42** (51 mg, 0.233 mmol, 1.2 éq.) sont mis en présence de $K_2CO_3$ (53.5 mg, 0.328 mmol, 2.0 éq.) dans 2 mL de DMF. Au bout de 12h à 80°C, le mélange réactionnel est refroidi à température ambiante. Après évaporation sous pression réduite, le brut est purifié par colonne de chromatographie flash sur gel de silice avec comme éluant (acétate d'éthyle/éther de pétrole = 20/80) pour obtenir l'alcyne **58** sous forme d'une huile incolore avec un rendement de 68%. **Rf** = 0.50 (acétate d'éthyle/éther de pétrole = 20/80). **Infrarouge** (n, cm-1, neat) 3285, 2959, 2360, 1601, 1500, 1446, 1347, 1265, 1228, 1164, 1080, 1046, 1017, 940, 903, 842. **RMN $^1$H** (250 MHz, $CDCl_3$, 20°C) δ 7.66 - 7.42 (m, 7H), 7.11 - 6.97 (m, 2H), 4.53 (dt, *J* = 46.8, 5.3 Hz, 2H), 4.51 - 4.43 (m, 1H), 4.62 - 4.55 (m, 1H), 3.90 (dt, *J* = 22.1, 5.3 Hz, 2H), 3.14 (s, 1H). **RMN $^{13}$C** (63 MHz, $CDCl_3$, 20°C) δ 139.7 (Cq), 138.1 (Cq), 133.0 (3 x CH), 129.0 (2 x CH), 128.9 (2 x CH), 127.6 (2 x CH), 122.2 (Cq), 82.6 (Cq), 81.3 (d, *J* = 172.4 Hz), 78.6 (CH), 51.1 (d, *J* = 22.7 Hz). **RMN $^{19}$F** (235 MHz, $CDCl_3$, 20°C) δ -223.0. **HRMS** (+ESI) calculée pour $C_{16}H_{14}FNO_2S$ ($M+H^+$) : 304.0802, trouvée: 304.0802.

**Exemple 28.2 *N*-(4-((1*H*-pyrrolo[2,3-*b*]pyridin-2-yl)ethynyl)phenyl)-*N*-(2-fluoroethyl)benzenesulfonamide 57**

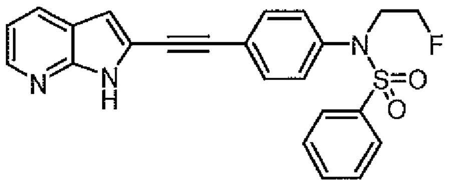

$C_{23}H_{18}FN_3O_2S$
M W: 419,47 gmol-1

Le composé a été préparé selon la **procédure C1** et purifié par colonne de chromatographie flash sur gel de silice (acétate d'éthyle/éther de pétrole = 30/70). Solide jaune (51%), **Pf** 202-204°C, $\boldsymbol{R_f}$ = 0.33 (acétate d'éthyle/éther de pétrole = 10/90). **Infrarouge** (v, $cm^{-1}$, neat) 3124, 3058, 2962, 2890, 2358, 2219, 1581, 1537, 1496, 1446, 1329, 1286, 1229, 1155, 1123, 1084, 1050, 1017, 956, 937, 912, 836, 808, 762. **RMN $^1$H** (250 MHz, DMSO-$d_6$, 20°C) δ 12.25 (s, 1H), 8.32 - 8.27 (m, 1H), 7.98 (d, *J* = 7.9 Hz, 1H), 7.75 - 7.71 (m, 1H), 7.64 - 7.60 (m, 4H), 7.57 (d, *J* = 8.2 Hz, 2H), 7.17 (d, *J* = 8.2 Hz, 2H), 7.14 - 7.09 (m, 1H), 6.85 (s, 1H), 4.53 - 4.48 (m, 1H), 4.42 - 4.37 (m, 1H), 4.01 - 3.97 (m, 1H), 3.93 (m, 1H). **RMN $^{13}$C** (63 MHz, DMSO-$d_6$ 20°C) δ 206.9 (Cq), 148.8 (Cq), 145.1 (CH), 139.7 (Cq), 137.9 (Cq), 133.9 (CH), 132.5 (2xCH), 129.9 (2xCH), 129.3 (CH), 129.02 (2xCH), 127.7 (CH), 121.6 (CH), 119.9 (Cq), 119.1 (Cq), 116.9 (CH), 107.0 (CH), 92.34 (Cq), 83.7 (Cq), 81.7 (d, *J* = 168.8 Hz, $CH_2$), 51.0 (d, *J* = 20.2 Hz, $CH_2$), **RMN $^{19}$F** (235 MHz, DMSO-$d_6$, 20°C) δ. -222.0. **HRMS (+ESI)** calculée pour $C_{23}H_{18}FN_3O_2S$ ($M+H^+$) : 420.1176, trouvée: 420.1174.

**Exemple 29 2-((4-(((3-fluorobenzyl)oxy)methyl)phenyl)ethynyl)-1*H*-pyrrolo[2,3-*b*]pyridine 61**

Le 2-((4-(((3-fluorobenzyl)oxy)methyl)phenyl)ethynyl)-1*H*-pyrrolo[2,3-*b*]pyridine a été synthétisé en 2 étapes à partir de l'alcyne **46**. Dans un premier temps, l'alcyne correspondant **62** a été préparé en 1 étape et puis nous avons effectué la réaction de Sonogashira avec l'azaindole pour obtenir le composé final.

Exemple 29.1 Synthèse du **1-(((4-ethynylbenzyl)oxy)methyl)-3-fluorobenzene 62 :**

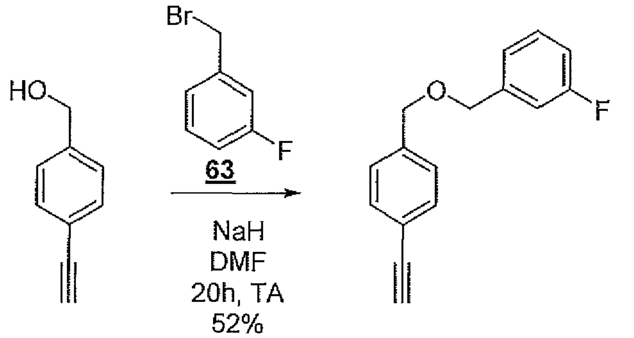


46 62

Sous argon, 100 mg d'alcyne **46** (0.756 mmol, 1.1 éq.) est solubilisé dans 2 mL de DMF. A 0°C, 48 mg de NaH (50% dans de l'huile minérale, 1.02 mmol, 1.5 éq.) est ajouté par portion puis au bout de 1h à température ambiante, 0.084 mL de bromé **63** (0.68 mmol, 1.0 éq.) est ajouté goutte à goutte. Au bout de 20h à température ambiante, 10 ml d'$H_2O$ sont ajoutés puis la phase organique est extraite avec l'acétate d'éthyle (3 x 10 mL). Ensuite les phases organiques sont réunies, séchées et filtrées sur coton. Après évaporation sous pression réduite, le brut est purifié par colonne de chromatographie flash sur gel de silice avec comme éluant (acétate d'éthyle/éther de pétrole = 2/48) pour obtenir 93 mg de l'alcyne **62** sous forme d'une huile incolore avec un rendement de 52%. , $R_f$ = 0.80 (acétate d'éthyle/éther de pétrole = 10/90). **Infrarouge** (v, $cm^{-1}$, neat) 3291, 2858, 1698, 1607, 1590, 1507, 1487, 1449, 1412, 1359, 1267, 1204, 1174, 1138, 1094, 1070, 1017, 946, 859. **RMN $^1$H** (250 MHz, $CDCl_3$, 20°C) 7.49 (d, *J* = 7.9 Hz, 2H), 7.32 (d, *J* = 7.9 Hz, 3H), 7.10 (m, 2H), 6.99 (m, 1H), 4.55 (2 x s, 4H), 3.07 (s, 1H). **RMN $^{13}$C** (63 MHz, $CDCl_3$ 20°C) δ 162.4 (d, *J* = 246.4 Hz, Cq), 140.7 (d, *J* = 7.9 Hz, Cq), 138.8 (Cq), 132.2 (2 x CH), 129.9 (d, *J* = 7.9 Hz, CH), 127.5 (2 x CH), 123.0 (d, *J* = 2.9 Hz, CH), 121.45 (Cq), 114.6 (d, *J* = 12.8 Hz, CH), 114.38 (d, *J* = 12.8Hz, CH), 83.5 (Cq), 77.2 (CH), 71.8 ($CH_2$), 71.5 (d, *J* = 2.0 Hz, $CH_2$). **RMN $^{19}$F** (235 MHz, DMSO-$d_6$, 20°C) δ. -113.4. **HRMS (+ESI)** calculée pour $C_{16}H_{13}FO$ ($M+H^+$) : 241.1023, trouvée: 241.1024.

**Exemple 29.2 2-((4-(((3-fluorobenzyl)oxy)methyl)phenyl)ethynyl)-1*H*-pyrrolo[2,3-*b*]pyridine 61**

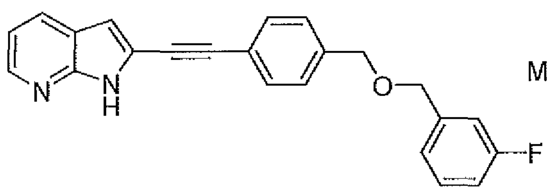

M

$C_{23}H_{17}FN_2O$
MW: 356,40 gmol-1

Le composé a été préparé selon la **procédure C1** et purifié par colonne de chromatographie flash sur gel de silice (acétate d'éthyle/éther de pétrole = 30/70). Solide jaune (58%), **Pf** 192-194°C, $R_f$ = 0.20 (acétate d'éthyle/éther de pétrole = 30/70). **Infrarouge** (v, $cm^{-1}$, neat) 3057, 2978, 2893, 2848, 1607, 1584, 1488, 1447, 1433, 1406, 1357, 1282, 1258, 1205, 1141, 1111, 1062, 1007, 934. **RMN $^1$H** (250 MHz, DMSO-$d_6$, 20°C) δ 12.23 (s, 1H), 8.29 (s, 1H), 7.97 (d, *J* = 9.8 Hz, 1H), 7.58 (s, 2H), 7.46 (d, *J* = 7.9 Hz, 3H), 7.20 (m, 2H), 7.12 (d, *J* = 7.9 Hz, 2H), 6.84 (s, 1H), 4.60 (s, 4H). **RMN $^{13}$C** (63 MHz, DMSO-$d_6$ 20°c δ 160.6 (d, *J* = 276 Hz, Cq), 148.81 (Cq), 144.9 (CH), 141.8 (d, *J* = 8.3 Hz, Cq), 139.9 (Cq), 131.8 (2 x CH), 130.8 (d, *J* = 8.3 Hz, CH), 128.9 (CH), 128.2 (2 x CH), 123.8 (d, *J* = 2 Hz, CH), 121.1 (Cq), 119.9 (Cq), 119.4 (Cq), 116.8 (CH), 114.7 (d, *J* = 20.9 Hz, CH), 114.4 (d, *J* = 20.9 Hz, CH), 106.8 (CH), 93.1 (Cq), 82.6 (Cq), 71.5 ($CH_2$), 71.3(d, *J* = 2 Hz, $CH_2$). **RMN $^{19}$F** (235 MHz, DMSO-$d_6$, 20°C) δ. -113.4. **HRMS (+ESI)** calculée pour $C_{23}H_{17}FN_2O$ ($M+H^+$) 357.1397, trouvée: 357.1398.

**Exemple 30. 4-[4-[2-(5-fluoro-1*H*-pyrrolo[2,3-*b*]pyridin-2-yl)ethynyl]phenyl]morpholine 64**

Le 4-[4-[2-(5-fluoro-1*H*-pyrrolo[2,3-*b*]pyridin-2-yl)ethynyl]phenyl]morpholine a été synthétisé en 4 étapes à partir du dibromobenzène. Dans un premier temps, l'alcyne correspondant **65** a été préparé en 3 étapes et puis nous avons effectué la réaction de Sonogashira avec l'azaindole pour obtenir le composé final.

**Exemple 30.1 Préparation du 4-(4-ethynylphenyl)morpholine 65**

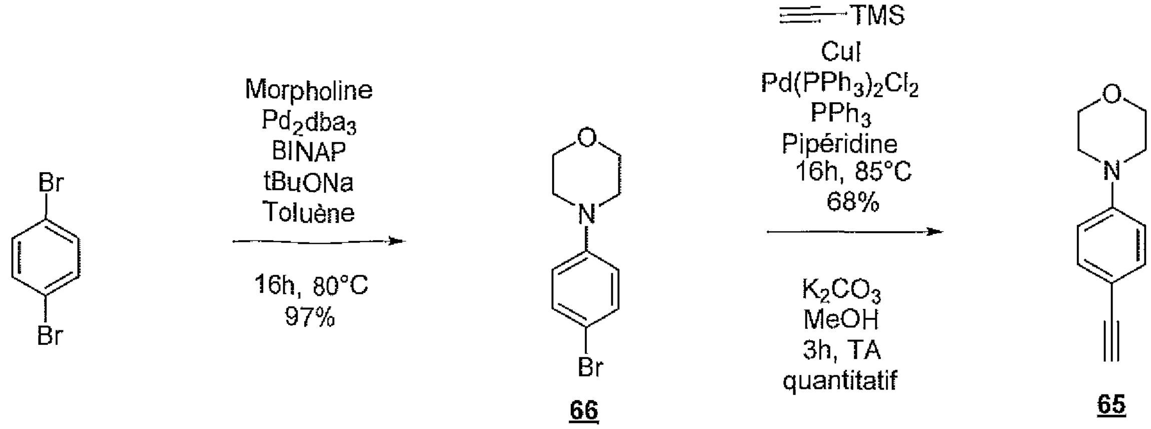


66 65

Synthèse du Bromé **66** :

Le composé 66 a été préparé selon la **procédure D** et purifié par colonne de chromatographie flash sur gel de silice (acétate d'éthyle/éther de pétrole = 1/49). Huile incolore (97%). **RMN $^1$H** (250 MHz, $CDCl_3$) δ 7.40 - 7.22 (m, 2H), 6.82 - 6.68 (m, 2H), 3.90 - 3.72 (m, 4H), 3.17 - 3.03 (m, 4H). Numéro CAS : 30483-75-1.

Synthèse de l'alcyne vrai **65**:

L'alcyne 65 a été préparé selon la **procédure E** et purifié par colonne de chromatographie flash sur gel de silice (acétate d'éthyle/éther de pétrole = 2/48). Huile incolore (68% sur les deux étapes). **RMN $^1$H** (250 MHz, $CDCl_3$) δ 7.47 - 7.38 (m, 4H), 6.88 - 6.80 (m, 4H), 3.91 - 3.84 (m, 4H), 3.25 - 3.18 (m, 4H), 3.01 (s, 1H). Numéro CAS : 41876-72-6.

**Exemple 30.2 4-[4-[2-(5-fluoro-1*H*-pyrrolo[2,3-*b*]pyridin-2-yl)ethynyl]phenyl]morpholine 64**

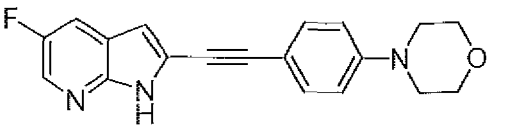

$C_{19}H_{16}FN_3O$
M W: 321,36 gmol-1

Le composé a été préparé selon la **procédure C1** et purifié par colonne de chromatographie flash sur gel de silice (acétate d'éthyle/éther de pétrole = 40/60). Solide jaune (42%), **Pf** >260°C, ***R*$_f$** = 0.33 (acétate d'éthyle/éther de pétrole = 40/60). **Infrarouge** (v, cm$^{-1}$, neat) 3114, 3057, 2964, 2852, 2209, 1604, 1586, 1536, 1507, 1448, 1394, 1381, 1341, 1285, 1264, 1242, 1222, 1197, 1157, 1122, 1053, 922. **RMN $^1$H** (250 MHz, DMSO-$d_6$, 20°C) δ 12.26 (s, 1H), 8.23 (s, 1H), 7.81 (m, 1H), 7.43 (d, *J* = 8.7 Hz, 2H), 6.99 (d, *J* = 8.7 Hz, 2H), 6.73 (s, 1H), 3.85 - 3.58 (m, 4H), 3.24 - 3.04 (m, 4H). **RMN** $^{13}$C (63 MHz, DMSO-$d_6$ 20°C) δ 155.8 (d, *J* = 239.2 Hz, Cq), 151.64 (Cq), 145.61 (Cq), 132.9 (2 x CH), 132.7 (d, *J* = 29.0 Hz, CH), 122.6 (Cq), 120.6 (d, *J* = 7.5 Hz, Cq), 114.9 (2 x CH), 113.8 (d, *J* = 20.9 Hz, CH), 110.9 (Cq), 105.8 (d, *J* = 4.4 Hz, CH), 94.8 (Cq), 80.6 (Cq), 66.4 (2 x $CH_2$), 47.7 (2 x $CH_2$). **RMN $^{19}$F** (235 MHz, DMSO-$d_6$, 20°C) δ. -138.4. **HRMS (+ESI)** calculée pour $C_{19}H_{16}FN_3O$ (M+H$^+$): 322.1350, trouvée: 322.1354.

**Exemple 31. *N*-(4-((*1H*-pyrrolo[2,3-*b*]pyridin-2-yl)ethynyl)benzyl)-2-fluoro-*N*-methylethan-1-amine 67**

Le *N*-(4-((1*H*-pyrrolo[2,3-*b*]pyridin-2-yl)ethynyl)benzyl)-2-fluoro-*N*-methylethan-1-amine a été synthétisé en 2 étapes à partir l'alcyne 69. Dans un premier temps, l'alcyne correspondant 68 a été préparé en 1 étape et puis nous avons effectué la réaction de Sonogashira avec l'azaindole pour obtenir le composé final.

**Exemple 31.1 Synthèse du *N*-[(4-ethynylphenyl)methyl]-2-fluoro-*N*-methyl-ethanamine 68**

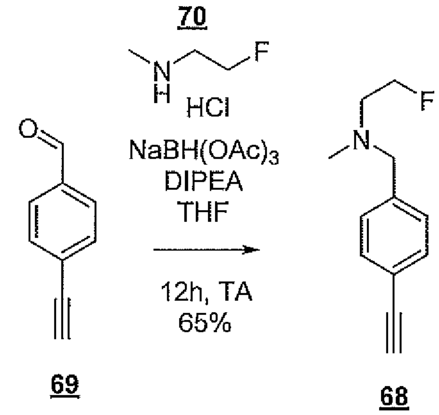


Sous Argon, 100 mg d'aldéhyde **69** (0.77 mmol, 1.0 éq.) et 98.5 mg d'amine **70** (0.845 mmol, 1.1 éq.) est solubilisé dans 5 mL de THF. Après l'ajout de 0.536 mL de DIPEA (3.08 mmol, 4.0 éq.) et 386.4 mg de $NaBH(OAc)_3$, le mélange réactionnel est agité pendant 12h à température ambiante. Ensuite, 15 mL d'une solution saturée de $NaHCO_3$ est additionné et la phase organique est extraite avec de l'acétate d'éthyle (3 x 15 mL). Ensuite les phases organiques sont réunies, séchées et filtrées sur coton. Après évaporation sous pression réduite, le brut est purifié par colonne de chromatographie flash sur gel de silice avec comme éluant (acétate d'éthyle/éther de pétrole = 5/45) pour obtenir 95 mg de l'alcyne **68** sous forme d'une huile incolore avec un rendement de 65%. , $R_f$ = 0.40 (acétate d'éthyle/éther de pétrole = 5/45). **Infrarouge** (v, $cm^{-1}$, neat) 3289, 2952, 2794, 2359, 2107, 1682, 1504, 1455, 1410, 1364, 1017, 846, 825. **RMN $^1$H** (250 MHz, $CDCl_3$, 20°C) δ 7.40 (d, *J* = 8.2 Hz, 2H), 7.24 (d, *J* = 8.2 Hz, 2H), 4.51 (dt, *J* = 47.6, 5.0 Hz, 2H), 3.53 (s, 2H), 3.01 (s, 1H), 2.68 (dt, *J* = 27.0, 5.0 Hz, 2H), 2.25 (s, 3H). **RMN $^{13}$C** (63 MHz, $CDCl_3$ 20°C) δ 139.8 (Cq), 132.1 (2 x CH), 128.8 (2 x CH), 120.8 (Cq), 83.8 (Cq), 82.4 (d, *J* = 158.6 Hz, $CH_2$), 76.45 (CH), 62.3 ($CH_2$), 56.8 (d, *J* = 20.1 Hz, $CH_2$), 42.7 ($CH_3$). **RMN $^{19}$F** (235 MHz, $CDCl_3$, 20°C) δ. -219.2. **HRMS (+ESI)** calculée pour $C_{12}H_{14}FN$ ($M+H^+$) : 192.1183, trouvée: 192.1185.

**Exemple 31.2 *N*-(4-((1H-pyrrolo[2,3-*b*]pyridin-2-yl)ethynyl)benzyl)-2-fluoro-*N*-methylethan-1-**amine **67**

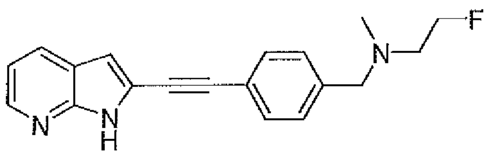

$C_{19}H_{18}FN_3$
MW: 307,37 gmol-1

Le composé a été préparé selon la **procédure C1** et purifié par colonne de chromatographie flash sur gel de silice (acétate d'éthyle/éther de pétrole = 40/60). Solide jaune (60%), **Pf** 160-162C, $R_f$ = 0.18 (acétate d'éthyle/éther de pétrole = 40/60). **Infrarouge** (v, $cm^{-1}$, neat) 3055, 2951, 2779, 1583, 1536, 1497, 1433, 1405, 1359, 1283, 1115, 1014, 879. **RMN $^1$H** (250 MHz, $CDCl_3$, 20°C) δ 11.78 (s, 1H), 8.41 (m, 1H), 7.93 (m, 1H), 7.56 (d, *J* = 8.0 Hz, 2H), 7.38 (d, *J* = 8.0 Hz, 2H), 7.20 - 7.05 (m, 1H), 6.78 (s, 1H), 4.58 (dt, *J* = 47.6, 5.0 Hz, 2H), 3.62 (s, 2H), 2.76 (dt, *J* = 27.1, 5.0 Hz, 2H), 2.33 (s, 3H). **RMN $^{13}$C** (63 MHz, $CDCl_3$ 20°C) δ 148.6 (Cq), 143.6 (CH), 139.9 (Cq), 131.5 (2 x CH), 129.1 (2 x CH), 121.1 (Cq), 120.8 (Cq), 120.1 (Cq), 116.5 (CH), 106.3 (CH), 93.4 (Cq), 83.8 (Cq), 82.5 (d, *J* = 167.6 Hz, $CH_2$), 62.3 ($CH_2$), 57.8 (d, *J* = 20.0 Hz, $CH_2$), 42.7 ($CH_3$). **RMN $^{19}$F** (235 MHz, $CDCl_3$, 20°C) δ. -219.1. **HRMS (+ESI)** calculée pour $C_{19}H_{18}FN_3$ ($M+H^+$) : 308.1557, trouvée: 308.1557.

**Exemple 32. 4-((1*H*-pyrrolo[2,3-*b*]pyridin-2-yl)ethynyl)-*N*-(2-fluoroethyl)-*N*-methylbenzamide 71**

Le 4-((1*H*-pyrrolo[2,3-*b*]pyridin-2-yl)ethynyl)-*N*-(2-fluoroethyl)-*N*-methylbenzamide a été synthétisé en 2 étapes à partir l'alcyne **73**. Dans un premier temps, l'alcyne correspondant **72** a été préparé en 1 étape et puis nous avons

effectué la réaction de Sonogashira avec l'azaindole pour obtenir le composé final.

**Exemple 32.1 Synthèse du 4-ethynyl-*N*-(2-fluoroethyl)-*N*-methyl-benzamide 72**

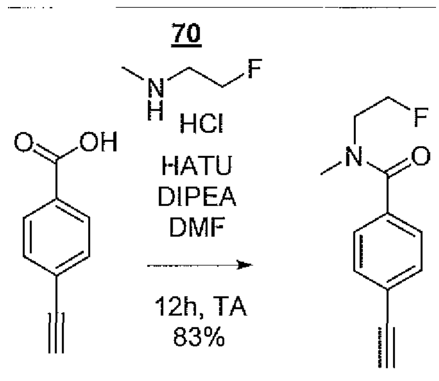


73 72

Sous Argon, 100 mg de l'acide 73 (0.684 mmol, 1.0 éq.) et 85 mg d'amine 70 (0.752 mmol, 1.1 éq.) est solubilisé dans 5 mL de DMF. Après l'ajout de 0.571 mL de DIPEA (3.28 mmol, 4.8 éq.) et 312 mg de HATU (0.821 mmol, 1.2 éq.), le mélange réactionnel est agité pendant 12h à température ambiante. Ensuite, 15 mL d'$H_2O$ est additionné et la phase organique est extraite avec de l'acétate d'éthyle (3 x 15 mL). Ensuite les phases organiques sont réunies, séchées et filtrées sur coton. Après évaporation sous pression réduite, le brut est purifié par colonne de chromatographie flash sur gel de silice avec comme éluant (acétate d'éthyle/éther de pétrole = 30/70) pour obtenir 95 mg de l'alcyne 72 sous forme d'une huile incolore avec un rendement de 83%. , $R_f$ = 0.25 (acétate d'éthyle/éther de pétrole = 30/70). **Infrarouge** (ν, $cm^{-1}$, neat) 3290, 3225, 2929, 2359, 1625, 1508, 1482, 1400, 1303, 1177, 1074, 1036, 1017, 979, 847, 764, 668. **RMN $^1$H** (250 MHz, $CDCl_3$, 20°C) δ 7.53 (d, *J* = 7.9 Hz, 2H), 7.38 (d, *J* = 7.2 Hz, 2H), 4.60 (m, 2H), 3.92 - 3.46 (m, 2H), 3.11 (m, 4H). **RMN $^{13}$C** (63 MHz, $CDCl_3$ 20°C) δ 168.9 (Cq), 136.2 (Cq), 132.2 (2 x CH), 127.0 (2 x CH), 123.6 (Cq), 83.8 (d, *J* = 167.6 Hz, $CH_2$), 82.8 (Cq), 78.6 (CH), 48.4 (d, *J* = 20 Hz, $CH_2$), 39.4 ($CH_3$). **RMN $^{19}$F** (235 MHz, $CDCl_3$, 20°C) δ. -222.2. **HRMS (+ESI)** calculée pour $C_{12}H_{12}FN$ (M+H$^+$) : 206.0975, trouvée: 206.0976.

**Exemple 32.2 4-((1*H*-pyrrolo[2,3-*b*lpyridin-2-yl)ethynyl)-*N*-(2-fluoroethyl-*N*-methylbenzamide 71**

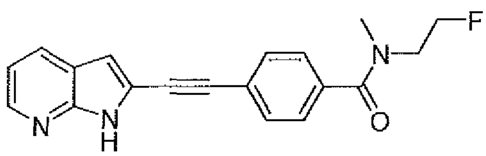

$C_{19}H_{16}FN_3O$
M W: 321,36 gmol-1

Le composé a été préparé selon la **procédure C1** et purifié par colonne de chromatographie flash sur gel de silice (acétate d'éthyle/éther de pétrole = 10/90). Solide jaune (34%), **Pf** 192-194°C, $R_f$ = 0.33 (acétate d'éthyle/éther de pétrole = 10/90). **Infrarouge** (ν, $cm^{-1}$, neat) 3055, 2951, 2779, 1583, 1536, 1497, 1433, 1405, 1359, 1283, 1115, 1014, 879. **RMN $^1$H** (250 MHz, $CDCl_3$, 20°C) δ 8.40 (d, *J* = 4.7 Hz, 1H), 7.95 (d, *J* = 7.8 Hz, 1H), 7.63 (d, *J* = 7.9 Hz, 2H), 7.47 (d, *J* = 7.9 Hz, 2H), 7.13 (dd, *J* = 7.8, 4.7 Hz, 1H), 6.81 (s, 1H), 4.74 (d, *J* = 47.8 Hz, 2H), 3.86 (d, *J* = 28.1 Hz, 2H), 3.13 (s, 3H). **RMN $^{13}$C DEPT** (63 MHz, $CDCl_3$ 20°C) δ 144.2 (CH), 131.6 (2 x CH), 129.2 (CH), 127.3 (2 x CH), 116.7 (CH), 107.1 (CH), 84.8 (d, *J* = 167.6 Hz, $CH_2$), 83.0 (Cq), 48.6 (d, *J* = 20.0 Hz, $CH_2$), 39.6 ($CH_3$). **RMN $^{19}$F** (235 MHz, $CDCl_3$, 20°C) δ. -222.5. **HRMS (+ESI)** calculée pour $C_{19}H_{16}FN_3O$ (M+H$^+$) : 322.1350, trouvée: 322.1352.

**Exemple 33. 3-((5-fluoro-1*H*-pyrrolo[2,3-*b*]pyridin-2-yl)ethynyl)-*N,N*-dimethylaniline 74**

Le 3-((5-fluoro-1*H*-pyrrolo[2,3-*b*]pyridin-2-yl)ethynyl)-*N,N*-dimethylaniline a été synthétisé en 3 étapes à partir du 3-bromoaniline. Dans un premier temps, l'alcyne correspondant 75 a été préparé en 2 étapes et puis nous avons

effectué la réaction de Sonogashira avec l'azaindole pour obtenir le composé final.

**Exemple 33.1 Synthèse du 3-ethynyl-*N,N*-dimethylaniline 75 :**

Pour synthétiser l'alcyne **75**, nous utilisons la méthode de Fang et coll. (Journal of Chemical Research, 2015 , 39, 8,487 - 491).

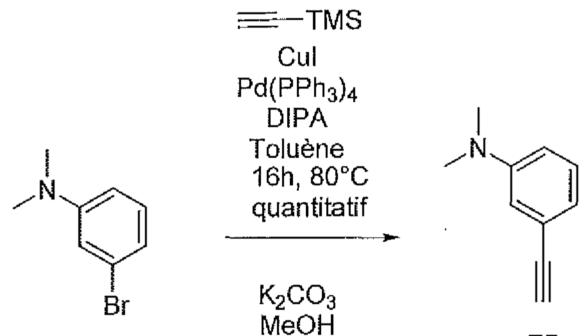


**75**

Du catalyseur $[Pd(PPh_3)_4]$ (29 mg, 0.025 mmol, 5 % mol) a été ajouté à une solution dégazée de dérivé bromé 3-bromo-*N,N*-diméthylaniline (100 mg, 0.5 mmol, 1.0 éq.), d'éthynyltrimethylsilane (0.083 mL, 0.6 mmol, 1.2 éq.), de CuI (5 mg, 0.025 mmol, 5 % mol), dans un mélange de diisopropylamine/Toluène (1:3) à la concentration de 0.1 M. Le mélange est chauffé à 80°C pendant 16 h sous atmosphère d'argon. Après un retour à la température ambiante, le mélange est concentré sous pression réduite, avant d'être purifiées par colonne de chromatographie flash sur gel de silice avec comme éluant (acétate d'éthyle/éther de pétrole = 1/49) pour obtenir 100 mg de l'alcyne intermédiaire sous forme d'une huile incolore avec un rendement quantitatif. Celui-ci est mis en réaction avec du $K_2CO_3$ (103 mg, 0.745, 1.5 éq.) dans le méthanol. Au bout de 3h d'agitation à température ambiante, le solvant est évaporé. Ensuite, le brut est solubilisé dans 10mL d'acétate d'éthyle. Puis la phase organique est lavé avec de la saumure (3 x 10 mL). Ensuite la phase organique est séchée avec du $MgSO_4$ filtrée sur coton et évaporée sous pression réduite. L'alcyne **75** est obtenu avec un rendement de 64% sans purification. **RMN $^1$H** (250 MHz, $CDCl_3$) δ 7.22 - 7.11 (m, 1H), 6.93 - 6.82 (m, 2H), 6.72 (m, 1H), 3.48 (s, 6H), 3.3 (s, 1H). Numéro CAS : 52324-05-7.

**Exemple 33.2 3-((5-fluoro-1*H*-pyrrolo[2,3-*b*]pyridin-2-yl)ethynyl)-*N,N*-dimethylaniline 74**

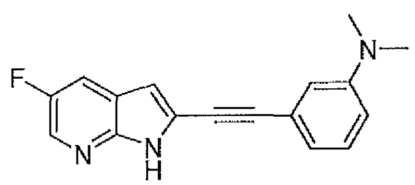

$C_{17}H_{14}FN_3$
M W: 279,32 gmol-1

Le composé a été préparé selon la **procédure C2** et purifié par colonne de chromatographie flash sur gel de silice (DCM = 100). Solide blanc (27%), **Pf** 226-228°C, **Rf** = 0.25 (dichlorométhane = 100). **Infrarouge** (v, $cm^{-1}$, neat) 3063, 2890, 2805, 1595, 1494, 1360, 1343, 1296, 1224, 1143, 767. **RMN $^1$H** (250 MHz, DMSO-$d_6$, 20°C) δ 11.91 (s, 1H), 7.83 (dd, *J* = 2.8, 1.8 Hz, 1H), 7.41 (dd, *J* = 8.0, 2.8 Hz, 1H), 6.82 (t, *J* = 8.0 Hz, 1H), 6.48 - 6.32 (m, 4H), 2.50 (s, 6H). **RMN $^{13}$C** (63 MHz, DMSO-$d_6$ 20°C) δ 155.8 (d, *J* = 239.5 Hz, Cq), 150.7 (Cq), 145.6 (Cq), 133.1 (d, *J* = 29.2 Hz, CH), 129.9 (CH), 122.1 (d, *J* = 17.9 Hz, Cq), 120.1 (d, *J* = 7.5 Hz, Cq), 119.4 (CH), 114.8 (CH), 114.1 (d, *J* = 20.8 Hz, CH), 113.9 (CH), 106.5 (d, *J* = 4.5 Hz, CH), 94.9 (Cq), 81.1 (Cq), 40.5 (2 x $CH_3$). **RMN $^{19}$F** (235 MHz, DMSO-$d_6$, 20°C) δ. -138.5. HRMS (+ESI) calculée pour $C_{17}H_{14}FN_3$ $(M+H^+)$ : 280.1244, trouvée: 280.1245.

**Exemple 34 2-((5-fluoro-1*H*-pyrrolo[2,3-*b*]pyridin-2-yl)ethynyl)-*N,N*-dimethylaniline 76**

Le 2-((5-fluoro-1*H*-pyrrolo[2,3-*b*]pyridin-2-yl)ethynyl)-*N,N*-dimethylaniline a été synthétisé en 4 étapes à partir

du 2-iodoaniline. Dans un premier temps, l'alcyne correspondant 77 a été préparé en 3 étapes et puis nous avons effectué la réaction de Sonogashira avec l'azaindole pour obtenir le composé final.

**Exemple 34.1 Synthèse de 2-ethynyl-*N,N*-dimethylaniline 77** :

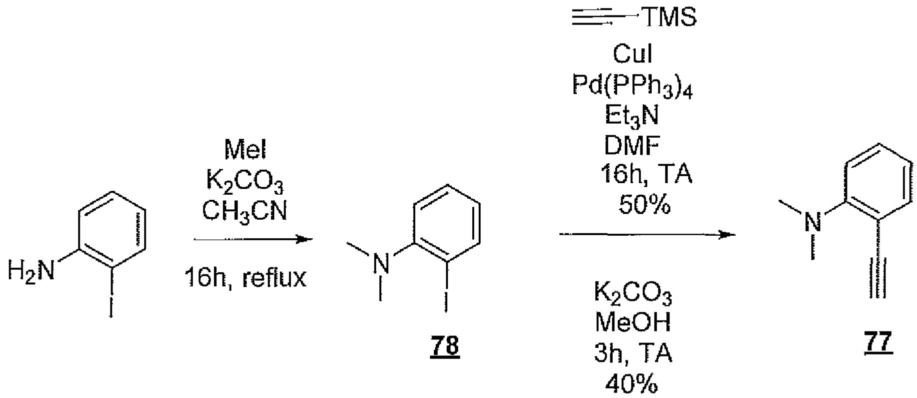


Synthèse du iodé **78** :

500 mg de 2-iodoanaline (2.28 mmol, 1.0 éq.) est solubilisé dans 10 mL de $CH_3CN$. Ensuite, 0.425 mL de iodométhane (6.84 mmol, 3.0 éq.) et 787 mg de $K_2CO_3$ (5.7 mmol, 2.5 éq.) sont additionnées successivement. Après 16h à reflux, le mélange réactionnel est refroidi. 20 mL d'$H_2O$ sont ajoutés et la phase organique est extraite avec de l'acétate d'éthyle (3 x 15 mL). Ensuite, les phases organiques sont réunies, séchées et filtrées sur coton. Après évaporation sous pression réduite, le brut est purifié par colonne de chromatographie flash sur gel de silice avec comme éluant (acétate d'éthyle/éther de pétrole = 2/48) pour obtenir 434 mg du iodé **78** avec 2% de monoalkylé.

Synthèse du 2-ethynvl-*N,N*-dimethylaniline **77** :

Du catalyseur $[Pd(PPh_3)_4]$ (12 mg, 0.018 mmol, 1.0 % mol) a été ajouté à une solution dégazée de dérivé iodé **78**, (434 mg, 1.75 mmol, 1.0 éq.), d'éthynyltrimethylsilane (0.365 mL, 2.6 mmol, 1.5 éq.), de CuI (10 mg, 0.052 mmol, 3 % mol) et $Et_3N$ (0.240 mL, 1.75 mmol, 1.0 éq.) dans 1.5 mL de DMF. Le mélange est agité à température ambiante pendant 16 h sous atmosphère d'argon. Ensuite, le mélange est concentré sous pression réduite, avant d'être purifiées par colonne de chromatographie flash sur gel de silice avec comme éluant (acétate d'éthyle/éther de pétrole = 1/49) pour obtenir 186 mg de l'alcyne intermédiaire sous forme d'une huile incolore avec un rendement 50%. Celui-ci est mis en réaction avec du $K_2CO_3$ (177 mg, 1.28 mmol, 1.5 éq.) dans 15 mL de méthanol. Au bout de 3h d'agitation à température ambiante. Le solvant est évaporé. Ensuite, le brut est solubilisé dans 10mL d'acétate d'éthyle. Puis la phase organique est lavé avec de la saumure (3 x 10 mL). Ensuite la phase organique est séchée, filtrée sur coton et évaporée sous pression réduite. L'alcyne 77 est obtenu avec un rendement de 64% sans purification. **RMN $^1$H** (250 MHz, $CDCl_3$) $\delta$ 7.43 (m, 1H), 7.29 - 7.13 (m, 1H), 6.95 - 6.76 (m, 2H), 3.43 (s, 1H), 2.90 (s, 6H). Numéro CAS : 219605-52-4.

**Exemple 34.2 2-((5-fluoro-1*H*-pyrrolo[2,3-*b*]pyridin-2-yl)ethynyl)-*N,N*-dimethylaniline 76**

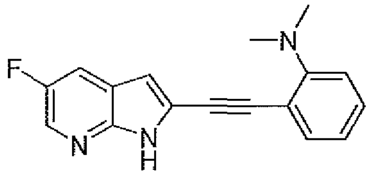

$C_{17}H_{14}FN_3$
M W: 279,32 gmol-1

Le composé a été préparé selon la **procédure C2** et purifié par colonne de chromatographie flash sur gel de silice (acétate d'éthyle/éther de pétrole = 10/90). Solide blanc (27%), **Pf** 146-148°C, **Rf** = 0.30 (acétate d'éthyle/éther

de pétrole = 15/85). **Infrarouge** (ν, $cm^{-1}$, neat) 3058, 2837, 2789, 2203, 1588, 1533, 1504, 1487, 1451, 1430, 1397, 1345, 1322, 1291, 1272, 1189, 1113, 1046, 983, 943. **RMN $^1$H** (400 MHz, $CDCl_3$, 20°C) δ 10.73 (s, 1H), 8.32 (t, *J* = 2.3 Hz, 1H), 7.59 (ddd, *J* = 28.4, 8.2, 2.3 Hz, 2H), 7.38 - 7.29 (m, 1H), 7.04 - 6.92 (m, 2H), 6.75 (d, *J* = 2.0 Hz, 1H), 3.04 (s, 6H). **RMN $^{13}$C** (101 MHz, $CDCl_3$ 20°C) δ 155.9 (d, *J* = 241.8 Hz, Cq), 155.2 (Cq), 145.1 (Cq), 134.3 (CH) 132.4 (d, *J* = 29.2 Hz, CH), 130.1 (CH), 122.6 (Cq), 120.7 (d, J = 7.3 Hz, Cq), 120.6 (CH) 117.1 (CH), 114.8 (Cq), 114.2 (d, *J* = 20.8 Hz, CH), 105.8 (d, *J* = 4.5 Hz, CH), 93.8 (Cq), 86.2 (Cq), 43.6. (2 x $CH_3$). **RMN $^{19}$F** (376 MHz, $CDCl_3$, 20°C) δ. -138.5. HRMS (+ESI) calculée pour $C_{17}7H_{14}FN_3$ (M+H$^+$) : 280.1244, trouvée: 280.1245.

**Exemple 35 1-[4-[2-(5-fluoro-1*H*-pyrrolo[2,3-*b*]pyridin-2-yl)ethynyl]phenyl]pyrrolidin-2-one 79**

Le 1-[4-[2-(5S-fluoro-1*H*-pyrrolo[2,3-*b*]pyridin-2-yl)ethynyl]phenyl]pyrrolidin-2-one a été synthétisé en 3 étapes. Dans un premier temps, l'alcyne correspondant **80** a été préparé en 3 étapes et puis nous avons effectué la réaction de Sonogashira avec l'azaindole pour obtenir le composé final.

**Exemple 35.1 Synthèse du 1-(4-ethynylphenyl)pyrrolidin-2-one 80 :**

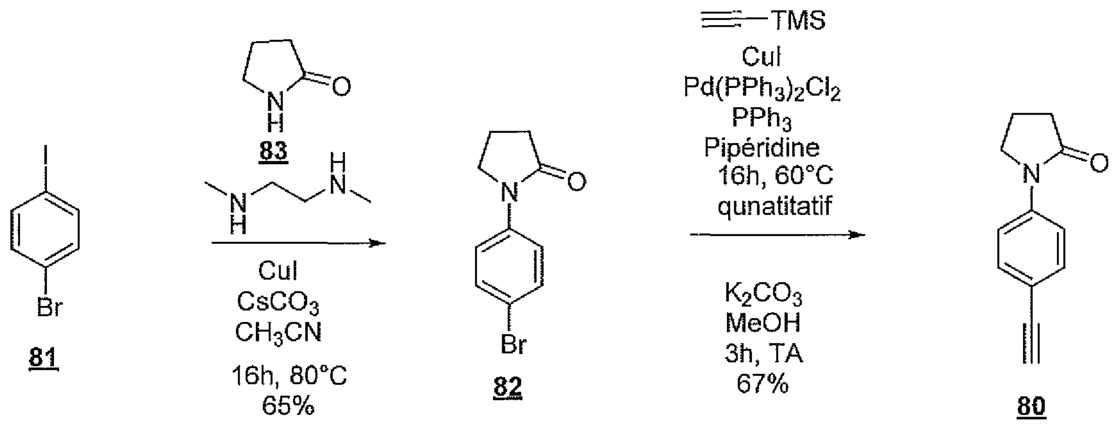


81 83 82 80

Synthèse du bromé **82** :

Du CuI (12 mg, 0.282 mmol, 20 % mol) a été ajouté à une solution dégazée de dérivé iodé **81,** (200 mg, 0.707 mmol, 1.0 éq.), d'amine **83** (0.056 mL, 0.742 mmol, 1.5 éq.), de *N,N'*-dimethylethylenediamine (0.026 mL, 0.282 mmol, 20 % mol) et $CsCO_3$ (459 mg, 1.41 mmol, 2.0 éq.) dans 3.0 mL de $CH_3CN$. Le mélange est agité à 80°C pendant 16 h sous atmosphère d'argon. Ensuite, le mélange est refroidi et filtré sur Celite®. Le filtrat est évaporé sous pression réduite avant d'être purifiées par colonne de chromatographie flash sur gel de silice avec comme éluant (acétate d'éthyle/éther de pétrole = 25/75) pour obtenir 110 mg de du bromé **82** sous forme d'une huile incolore avec un rendement 65%. **RMN $^1$H** (250 MHz, $CDCl_3$, 20°C) δ 7.57 - 7.37 (m, 4H), 3.81 (t, *J* = 7.0 Hz, 2H), 2.58 (t, *J* = 8.1 Hz, 2H), 2.14 (m, 2H). Numéro CAS : 7661-32-7.

Synthèse du 1-(4-ethynylphenyl)pyrrolidin-2-one **80** :

Sous argon, $Pd(PPh_3)_4$ (24 mg, 0.021 mmol, 0.1 éq.) et CuI (4mg, 0.021 mmol, 0.01 éq.) sont ajoutés à une solution de $Et_3N$ dégazée (1 mL) contenant l'éthynyltrimethylsilane (0.058 mL, 0.416 mmol, 2.0 éq.), le dérivé bromé **82** (50 mg, 0.21 mmol, 1.0 éq.). Au bout de 16h à 60°C, le solvant est évaporé sous pression réduite. Ensuite, le mélange réactionnel est purifié par colonne de chromatographie flash sur gel de silice. Puis, 56 mg de l'alcyne intermédiaire est mis en réaction avec $K_2CO_3$ (45 mg, 0.217 mmol, 1.5 éq.) dans 5 mL de méthanol. Au bout de 3h à température ambiante, le solvant est évaporé sous pression réduite. Le brut est solubilisé dans 10 mL d'acétate d'éthyle. Puis la phase organique est lavée avec de la saumure (3 x 10 mL). Ensuite la phase organique est séchée avec $MgSO_4$, filtrée sur coton et évaporée sous pression réduite pour conduire à l'alcyne **80** avec un rendement de 67% sous forme d'un solide blanc. **Pf** 136-138°C, **Rf** = 0.5 (acétate d'éthyle/éther de pétrole = 50/50). **Infrarouge** (ν, $cm^{-1}$, neat) 3292, 3031, 2915, 2099, 1605, 1514, 1494, 1451, 1395, 1355, 1233, 1177, 1100, 1046, 1003, 912, 883, 847, 815. **RMN $^1$H** (250 MHz, $CDCl_3$, 20°C) δ 7.60 (d, *J* = 9.0 Hz, 2H), 7.48 (d, *J* = 9.0 Hz, 2H), 3.86 (dd, *J* = 7.4, 6.7 Hz, 2H), 3.33 (s, 1H), 2.69 - 2.55 (m, 2H), 2.25 - 2.08 (m, 2H). **RMN $^{13}$C** (63 MHz, $CDCl_3$ 20°C) δ 174.8 (Cq), 140.3 (Cq), 133.2 (2 x CH), 119.7 (2 x CH),

118.2 (Cq), 83.4 (Cq), 77.3 (CH), 46.9 ($CH_2$), 33.27 ($CH_2$), 18.37 ($CH_2$). HRMS (+ESI) calculée pour $C_{12}H_{12}NO$ (M+H+) : 186.0913, trouvée: 186.0913.

**Exemple 35.2 1-[4-[2-(5-fluoro-1*H*-pyrrolo[2,3-*b*]pyridin-2-yl)ethynyl]phenyl]pyrrolidin-2-one 79**

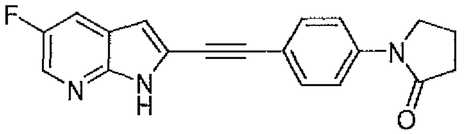

$C_{19}H_{14}FN_3O$
M W: 319,34 gmol-1

Le composé a été préparé selon la **procédure C2** et purifié par colonne de chromatographie flash sur gel de silice (acétate d'éthyle/éther de pétrole = 10/90). Solide blanc (27%), **Pf** 256-258°C, **Rf** = 0.30 (acétate d'éthyle/éther de pétrole = 15/85). **Infrarouge** (v, $cm^{-1}$, neat) 3058, 2837, 2789, 2203, 1588, 1533, 1504, 1487, 1451, 1430, 1397, 1345, 1322, 1291, 1272, 1189, 1113, 1046, 983, 943. **RMN $^1$H** (400 MHz, DMSO-$d_6$, 20°C) δ 12.37 (s, 1H), 8.27 (s, 1H), 7.85 (d, *J* = 9.8 Hz, 1H), 7.79 (d, *J* = 7.4 Hz, 2H), 7.60 (d, *J* = 7.4 Hz, 2H), 6.81 (s, 1H), 3.87 (m, 2H), 2.54 (m, 2H), 2.07 (m, 2H). **RMN $^{13}$C** (101 MHz, DMSO-$d_6$, 20°C) δ 174.8 (Cq), 155.7 (d, *J* = 240 Hz, Cq), 145.6 (Cq), 140.8 (Cq), 133.3 (d, *J* = 28.9 Hz, CH), 132.4 (2 x CH), 121.9 (Cq), 120.2 (d, *J* = 7.5 Hz, Cq), 119.5 (2 x CH), 116.6 (Cq), 114.1 (d, *J* = 20.8 Hz, CH), 106.5 (d, *J* = 3.5 Hz, Cq), 93.8 (Cq), 81.8 (Cq), 48.4 ($CH_2$), 32.9 ($CH_2$), 17.8 ($CH_2$). **RMN $^{19}$F** (376 MHz, DMSO-$d_6$, 20°C) δ. -138.5. HRMS (+ESI) calculée pour $C_{19}H_{14}FN_3O$ (M+H+) : 320.1192, trouvée: 320.1193.

**Exemple 36. 2-[2-(2-fluorophenyl)ethynyl]-1*H*-pyrrolo[2,3-*b*]pyridine 84**

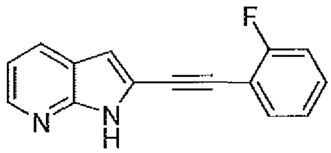

$C_{15}H_9FN_2$
M W: 236,25 gmol-1

Le composé a été préparé selon la **procédure C2** et purifié par colonne de chromatographie flash sur gel de silice (acétate d'éthyle/éther de pétrole = 10/90). Solide jaunâtre (32%), **Pf** 210-212°C, **Rf** = 0.20 (acétate d'éthyle/éther de pétrole = 15/85). **Infrarouge** (v, cm-1, neat) 3058, 2837, 2789, 2203, 1588, 1533, 1504, 1487, 1451, 1430, 1397, 1345, 1322, 1291, 1272, 1189, 1113, 1046, 983, 943. **RMN $^1$H** (400 MHz, $CDCl_3$, 20°C) δ 11.55 (s, 1H), 8.46 (s, 1H), 7.95 (d, *J* = 7.9 Hz, 1H), 7.59 (t, *J* = 7.3 Hz, 1H), 7.43 - 7.34 (m, 1H), 7.17 (m, 3H), 6.83 (s, 1H). **RMN $^{13}$C** (101 MHz, $CDCl_3$, 20°C) δ 163.7 (d, *J* = 252.5 Hz, Cq), 148.5 (Cq), 144.1 (CH), 133.3 (CH), 130.5 (d, *J* = 7.9 Hz, CH), 129.2 (CH), 124.1 (CH), 120.6 (Cq), 119.5 (Cq), 116.6 (CH), 115.7 (d, *J* = 20.5 Hz, CH), 111.3 (d, *J* = 15.2 Hz, Cq), 106.9 (CH), 86.8 (d, *J* = 15.2 Hz, Cq). **RMN $^{19}$F** (376 MHz, $CDCl_3$, 20°C) δ. -109.3. HRMS (+ESI) calculée pour $C_{15}H_9FN_2$ (M+H+) : 237.0822, trouvée: 237.0823.

**Exemple 37. 2-((4-(1-(3-fluoropropyl)piperidin-4-yl)phenyl)ethynyl)-1*H*-pyrrolo[2,3-*b*]pyridine 85**

Le 2-((4-(1-(3-fluoropropyl)piperidin-4-yl)phenyl)ethynyl)-1*H*-pyrrolo[2,3-*b*]pyridine a été synthétisé en 4 étapes. Dans un premier temps, l'alcyne correspondant **86** a été préparé en 3 étapes et puis nous avons effectué la réaction de Sonogashira avec l'azaindole pour obtenir le composé final.

**Exemple 37.1 Synthèse du 4-(4-ethynylphenyl)-1-(3-fluoropropyl)piperidine 86**

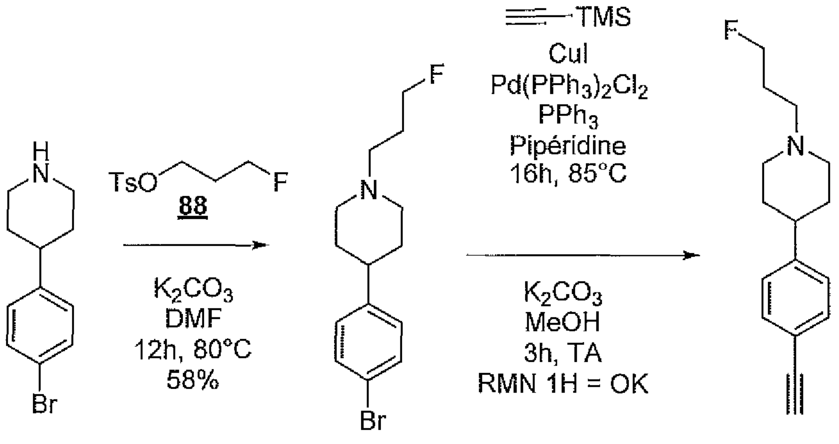


**87** **89** **86**

Synthèse du 4-(4-bromophenyl)-1-(3-fluoropropyl)piperidine **89**:

Le bromé **87** (176 mg, 0.635 mmol, 1.2 éq.) et le fluoré **88** (123 mg, 0.529 mmol, 1.0 éq.) sont mis en présence de $K_2CO_3$ (219 mg, 0.1.59 mmol, 3.0 éq.) dans 10 mL de $CH_3CN$. Au bout de 18 h à 90°C, le mélange réactionnel est refroidi à température ambiante et le solvant est évaporé sous pression réduite. Le brut est solubilisé dans 10 mL d'acétate d'éthyle. Puis la phase organique est lavée avec de la saumure (3 x 10 mL). Ensuite la phase organique est séchée, filtrée sur coton et évaporée sous pression réduite. Après une purification sur gel de silice avec comme éluant (acétate d'éthyle/éther de pétrole/$Et_3N$) conduit au bromé **89** avec un rendement de 58% sous forme d'une huile incolore. **Rf** = 0.2 (méthanol/dichlorométhane = 2/48). **Infrarouge** (v, $cm^{-1}$, neat) 3305, 2925, 1639, 1512, 1441, 1376, 1258, 1171, 1124, 1039, 997, 911, 835. **RMN $^1$H** (250 MHz, $CDCl_3$, 20°C) δ 7.41 (d, *J* = 8.4 Hz, 2H), 7.10 (d, *J* = 8.4 Hz, 2H), 4.62 (t, *J* = 6.0 Hz, 1H), 4.43 (t, *J* = 6.0 Hz, 1H), 3.23 - 2.93 (m, 2H), 2.59 - 2.43 (m, 2H), 2.24 - 1.60 (m, 10H). **RMN $^{13}$C** (63 MHz, $CDCl_3$ 20°C) δ 1453 (Cq), 131.4 (2 x CH), 128.6 (2 x CH), 119.7 (Cq), 82.7 (d, *J* = 164.3 Hz, $CH_2$), 54.7 (d, *J* = 5.4 Hz, $CH_2$), 54.3 ($CH_2$), 42.2 ($CH_2$), 33.4 ($CH_2$), 28.0 (d, *J* = 19.6 Hz, $CH_2$). **RMN $^{19}$F** (235 MHz, $CDCl_3$, 20°C) δ. -220. HRMS (+ESI) calculée pour $C_{14}H_{19}BrFN$ ($M+H^+$) : 300.0757, trouvée: 300.0757.

Synthèse du 4-(4-ethynylphenyl)-1-(3-fluoropropyl)piperidine **86** :

L'alcyne **86** a été préparé selon la **procédure E** et purifié par colonne de chromatographie flash sur gel de silice (méthanol/dichlorométhane = 2/48/) mais contenant 20% du bromé **89.**

**Exemple** 37.2 **2-((4-(1-(3-fluoropropyl)piperidin-4-yl)phenyl)ethynyl)-1*H*-pyrrolo[2,3-*b*]pyridine 85**

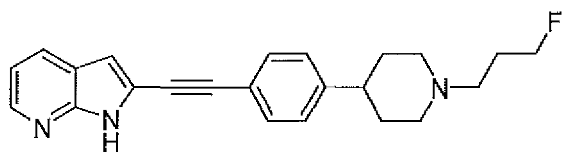

$C_{23}H_{24}FN_3$
M W: 361,46 gmol-1

Le composé a été préparé selon la **procédure C2** et purifié par colonne de chromatographie flash sur gel de silice (méthanol/dichlorométhane/$NH_4OH$ = 2/48/0.5). Solide blanc (55%), **Pf** 218-220°C, **Rf** = 0.20 (méthanol/dichlorométhane/$NH_4OH$ = 2/48/0.5). **Infrarouge** (v, $cm^{-1}$, neat) 3052, 2936, 2777, 2360, 1583, 1534, 1495, 1466, 1432, 1404, 1376, 1356, 1325, 1284, 1133, 1111, 1041, 999, 981, 938, 917, 828, 801, 763. **RMN $^1$H** (400 MHz, DMSO-$d_6$, 20°C) δ 12.19 (s, 1H), 8.28 (d, *J* = 4.6 Hz, 1H), 7.96 (d, *J* = 8.0 Hz, 1H), 7.51 (d, *J* = 7.8 Hz, 2H), 7.35 (d, *J* = 8.0 Hz, 2H), 7.10 (dd, *J* = 7.9, 4.7 Hz, 1H), 6.81 (d, *J* = 1.9 Hz, 1H), 4.56 (t, *J* = 6.0 Hz, 1H), 4.44 (t, *J* = 5.9 Hz, 1H), 2.98 (m, 2H), 2.50 (m, 2H), 2.41 (m, 2H), 2.02 (m, 2H), 1.92 - 1.59 (m, 6H). **RMN $^{13}$C** (101 MHz, DMSO-$d_6$, 20°C) 148.8 (Cq), 148.1 (Cq), 144.9 (CH), 131.8 (2 x CH), 128.8 (CH), 127.8 (2 x CH), 120.0 (Cq), 119.7 (CH), 119.5 (Cq), 116.8 (Cq), 106.6 (CH), 93.3 (Cq), 82.9 (d, *J* = 161.5 Hz, $CH_2$), 82.2 (Cq), 54.3 (d, *J* = 6.0 Hz, $CH_2$), 54.1 (2 x $CH_2$), 40.8 ($CH_2$), 33.2 (2 x $CH_2$), 28.0 (d, *J* = 19.1 Hz, $CH_2$). **RMN $^{19}$F** (376 MHz, DMSO-$d_6$, 20°C) δ. -218.3. HRMS (+ESI) calculée pour $C_{23}H_{24}FN_3$

(M+H+) : 361.2027, trouvée: 361.2026.

**Exemple 38. 5-fluoro-2-((4-(4-phenylpiperidin-1-yl)phenyl)ethynyl)-1*H*-pyrrolo[2,3-*b*]pyridine 90**

Le 5-fluoro-2-((4-(4-phenylpiperidin-1-yl)phenyl)ethynyl)-1*H*-pyrrolo[2,3-*b*]pyridine a été synthétisée en 4 étapes. Dans un premier temps, l'alcyne correspondant **91** a été préparé en 3 étapes et puis nous avons effectué la réaction de Sonogashira avec l'azaindole pour obtenir le composé final.

**Exemple 38.1 Synthèse de 1-(4-ethynylphenyl)-4-phenyl-piperidine 91**

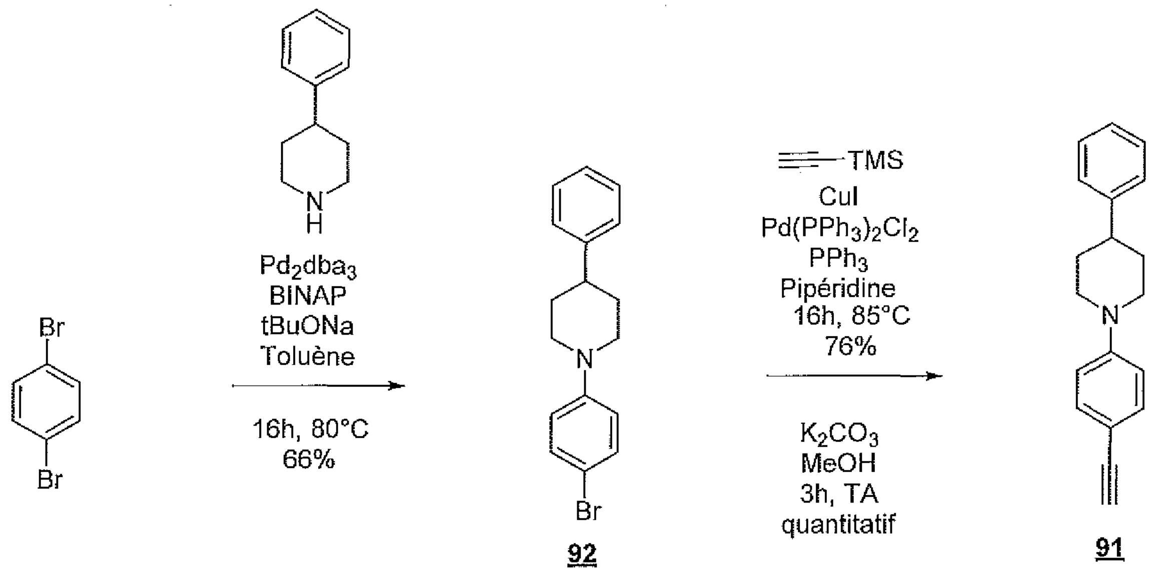


**92** **91**

Synthèse du Bromé **92** :

Le composé **92** a été préparé selon la **procédure D** et purifié par colonne de chromatographie flash sur gel de silice (acétate d'éthyle/éther de pétrole = 2/48). Solide blanc (97%). **RMN $^1$H** (250 MHz, $CDCl_3$) δ 7.41 - 7.20 (m, 6H), 6.92 - 6.83 (m, 2H), 3.84 - 3.74 (m, 2H), 2.84 (m, 2H), 2.68 (m, 1H), 2.03 - 1.83 (m, 4H). Numéro CAS : 303975-64-6.

Synthèse du 1-(4-emynylphenyl)-4-phenyl-piperidine **91**

L'alcyne **91** a été préparé selon la **procédure E** et purifié par colonne de chromatographie flash sur gel de silice (acétate d'éthyle/éther de pétrole = 2/48). Solide blanc (76%), **Pf** 136-138°C, **Rf =** 0.30 (acétate d'éthyle/éther de pétrole = 2/48). **Infrarouge** (v, cm-1, neat) 3157, 3072, 2893, 2205, 1605, 1541, 1511, 1432, 1273, 1224, 1183, 809, 763, 507. **RMN $^1$H** (400 MHz, $CDCl_3$, 20°C) δ 7.46 - 7.30 (m, 4H), 7.32 - 7.20 (m, 3H), 6.95 - 6.87 (m, 2H), 3.96 - 3.85 (m, 2H), 3.01 (s, 1H), 2.90 (m, 2H), 2.71 (m, 1H), 2.03 - 1.81 (m, 4H). **RMN $^{13}$C** (101 MHz, $CDCl_3$, 20°C) δ 151.5 (Cq), 145.8 (Cq), 133.2 (2 x CH), 128.5 (2 x CH), 126.8 (2 x CH), 126.4 (CH), 115.4 (2 x CH), 111.5 (Cq), 84.4 (Cq), 75.3 (CH), 49.5 (2 x $CH_2$), 42.5 (CH), 32.9 (2 x $CH_2$). HRMS (+ESI) calculée pour $C_{19}H_{19}N$ (M+H+) : 262.1590, trouvée: 292.1590.

**Exemple 38.2 5-fluoro-2-((4-(4-phenylpiperidin-1-yl)phenyl)ethynyl)-1*H*-pyrrolo[2,3-*b*]pyridine 90**

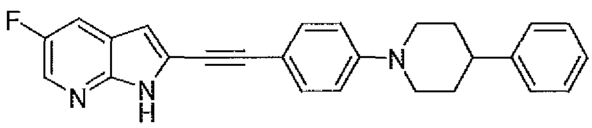

$C_{26}H_{22}FN_3$
M W: 395,48 gmol-1

Le composé a été préparé selon la **procédure C2** et purifié par colonne de chromatographie flash sur gel de silice (acétate d'éthyle/éther de pétrole = 20/80). Solide blanc (34%), **Pf** >260°C, **Rf** = 0.20 (acétate d'éthyle/éther de pétrole = 20/80). **Infrarouge** (v, cm-1, neat) 3117, 3063, 2917, 2813, 2734, 2205, 1600, 1586, 1534, 1503, 1462, 1386, 1341, 1292, 1214, 1191, 1154, 1101, 1011, 819. **RMN $^1$H** (400 MHz, DMSO-$d_6$, 20°C) δ 12.22 (s, 1H), 8.19 (m, 1H), 7.79 (m, 1H), 7.47 - 7.09 (m, 7H), 6.99 (d, J = 8.8 Hz, 2H), 6.68 (s, 1H), 3.95 (m, 2H), 2.80 (m, 3H), 1.90 - 1.55 (m, 4H). **RMN $^{19}$F** (376 MHz, DMSO-$d_6$, 20°C) δ. -138.5. HRMS (+ESI) calculée pour $C_{26}H_{22}FN_3$ (M+H+) : 396.1868, trouvée: 396.1869.

**Exemple 39.5-((1*H*-pyrrolo[2,3-*b*]pyridin-2-yl)ethynyl)-2-fluoro-*N,N*-dimethylaniline 93**

Le 5-((1*H*-pyrrolo[2,3-*b*]pyridin-2-yl)ethynyl)-2-fluoro-*N,N*-dimethylaniline a été synthétisé en 4 étapes. Dans un premier temps, l'alcyne correspondant **94** a été préparé en 3 étapes et puis nous avons effectué la réaction de Sonogashira avec l'azaindole pour obtenir le composé final.

**Exemple 39.1 Synthèse du 5-ethynyl-2-fluoro-*N,N*-dimethyl-aniline 94**

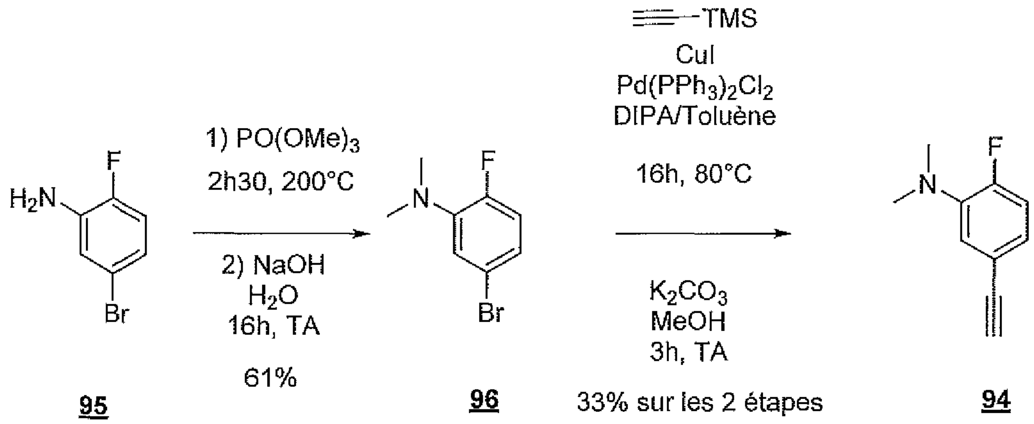


95 96 94

Synthèse du bromé **96** :

Le bromé **95** (1.0 g, 5.26 mmol, 1.0 éq.) est solubilisé dans 0.650 mL de triméthylphosphate (5.52 mmol, 1.05 éq.). Au bout de 2h30 à 200°C, le mélange est refroidi à température ambiante et 15 mL d'une solution de NaOH à 2M est ajouté. Au bout d'une nuit à température ambiante, la phase organique est extraite du dichlorométhane (3 x 15 mL). Ensuite, les phases organiques sont réunies, séchées et filtrées sur coton. Après évaporation sous pression réduite, le brut est purifié par colonne de chromatographie flash sur gel de silice avec comme éluant (acétate d'éthyle/éther de pétrole = 2/48) pour conduire au composé **96.** Huile incolore (61%). **RMN $^1$H** (250 MHz, $CDCl_3$) δ 7.01 - 6.77 (m, 3H), 2.84 (s, 6H). Numéro CAS : 1352214-46-0.

Synthèse du 5-ethynyl-2-fluoro-*N,N*-dimethylaniline **94** :

Du catalyseur [$Pd(PPh_3)_4$] (53 mg, 0.046 mmol, 5 % mol) a été ajouté à une solution dégazée de dérivé bromé **96**, (200 mg, 0.92 mmol, 1.0 éq.), d' éthynyltrimethylsilane (0.152 mL, 0.11 mmol, 1.2 éq.), de CuI (9 mg, 0.046 mmol, 5 % mol), dans un mélange de diisopropylamine/Toluène (1:3) à la concentration de 0.1 M. Le mélange est chauffé à 80°C pendant 16 h sous atmosphère d'argon. Après un retour à la température ambiante, le mélange est concentré sous pression réduite, avant d'être purifiées par colonne de chromatographie flash sur gel de silice avec comme éluant (acétate d'éthyle/éther de pétrole = 2/48) pour obtenir 172 mg de l'alcyne intermédiaire. Celui-ci est mis en réaction avec du $K_2CO_3$ (151 mg, 1.1 mmol, 1.5 éq.) dans le méthanol. Au bout de 3h d'agitation à température ambiante. Le solvant est évaporé. Ensuite, le brut est solubilisé dans 10 mL d'acétate d'éthyle. Puis la phase organique est lavée avec de la saumure (3 x 10 mL). Ensuite la phase organique est séchée avec du $MgSO_4$, filtrée sur coton et évaporée sous pression réduite. Le brut est purifié par colonne de chromatographie flash sur gel de silice avec comme éluant (acétate d'éthyle/éther de pétrole = 2/48) pour conduire à l'alcyne **94.** Huile incolore (33%). **Rf =** 0.30 (acétate d'éthyle/éther de pétrole = 15/85). **Infrarouge** (v, cm-1, neat) 3290, 2970, 2930, 2853, 2102, 1606, 1511, 1463, 1434, 1356, 1336, 1273, 1245, 1227, 1185, 1140, 1023, 935. **RMN $^1$H** (400 MHz, $CDCl_3$, 20°C) δ 7.12 - 6.81 (m, 3H), 3.00 (s, 1H),

2.84 (s, 6H). **RMN $^{13}$C** (101 MHz, $CDCl_3$, 20°C) δ 155.3 (d, *J* = 249.4 Hz, Cq), 140.7 (d, *J* = 9.4 Hz, Cq), 125.0 (d, *J* = 8.2 Hz, CH), 121.9 (d, *J* = 4.2 Hz, CH), 118.1 (d, *J* = 3.8 Hz, Cq), 116.3 (d, *J* = 22.2 Hz, CH), 83.4 (Cq), 76.1 (CH), 42.6 (d, *J* = 4.2 Hz, 2 x $CH_3$) **RMN $^{19}$F** (376 MHz, $CDCl_3$, 20°C) δ. -119.7. HRMS (+ESI) calculée pour C10H10FN (M+H+) : 164.0870, trouvée: 164.0872.

**Exemple 39.2 Synthèse du 5-((1*H*-pyrrolo[2,3-*b*]pyridin-2-yl)ethynyl)-2-fluoro-*N,N*-dimethylaniline 93**

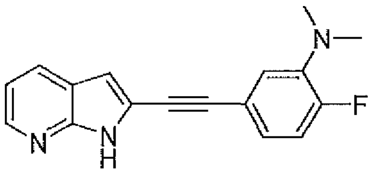

$C_{17}H_{14}FN_3$
M W: 279,32 gmol-1

Le composé a été préparé selon la **procédure C2** et purifié par colonne de chromatographie flash sur gel de silice (acétate d'éthyle/éther de pétrole = 45/55). Solide jaunâtre (79%), **Pf** 170-172°C, **Rf** = 0.20 (acétate d'éthyle/éther de pétrole = 30/70). **Infrarouge** (v, cm-1, neat) 3157, 3072, 2893, 2205, 1605, 1541, 1511, 1432, 1273, 1224, 1183, 809, 763, 507. **RMN $^{1}$H** (400 MHz, $CDCl_3$, 20°C) δ 10.84 (s, 1H), 8.43 - 8.31 (m, 1H), 7.93 (d, J = 7.9 Hz, 1H), 7.10 (d, J = 7.9 Hz, 2H), 7.05 - 6.93 (m, 1H), 6.76 (s, 1H), 2.90 (s, 6H). **RMN $^{13}$C** (101 MHz, $CDCl_3$, 20°C) δ 155.3 (d, *J* = 249.9 Hz, Cq), 148.4 (Cq), 143.9 (CH), 140.9 (d, *J* = 9.4 Hz, Cq), 128.9 (CH), 124.5 (d, *J* = 8.2 Hz, CH), 121.3 (d, *J* = 4.2 Hz, CH), 120.7 (Cq), 119.82 (Cq), 118.5 (Cq), 116.6 (CH), 116.5 (d, *J* = 22.2 Hz, CH), 106.4 (CH), 93.2 (Cq), 80.5 (Cq), 42.7 ($CH_3$), 42.6 ($CH_3$). **RMN $^{19}$F** (376 MHz, $CDCl_3$, 20°C) δ. -119.3. HRMS (+ESI) calculée pour $C_{17}H_{14}FN_3$ (M+H+) : 280.1244, trouvée: 280.1244.

**Exemple 40.2-((4-(4-((5-pyrrolo[2,3-*b*]pyridin-2 yl)ethynyl)phenoxy)phenyl)(methyl)amino)ethan-1-ol 97**

Le 2-((4-(4-((5-fluoro-1*H*-pyrrolo[2,3-*b*]pyridin-2-yl)ethynyl)phenoxy)phenyl)(methyl)amino)ethan-1-ol **97** a été synthétisée en 6 étapes. Dans un premier temps, l'alcyne correspondant **98** a été préparé en 4 étapes en partant du phényle éther **99** et puis nous avons effectué la réaction de Sonogashira avec l'azaindole suivie d'une réaction de déprotection avec du TBAF pour conduire au composé final.

**Exemple 40.1 Synthèse du *N*-[2-[tert-butyl(diphenyl)silyl]oxyethyl-4-(4-ethynylphenoxy)-*N*-methyl-aniline 98** :

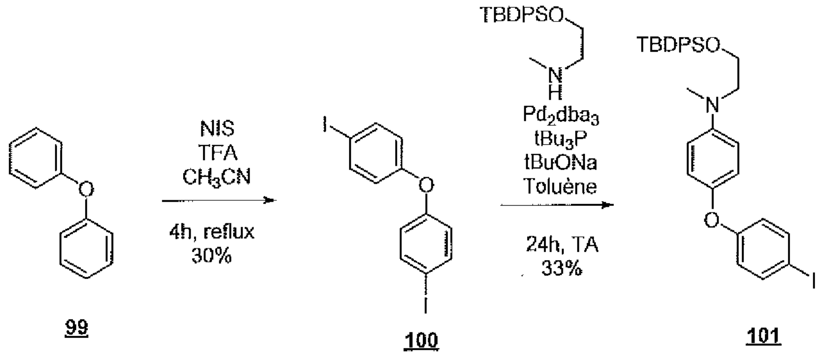


99 100 101

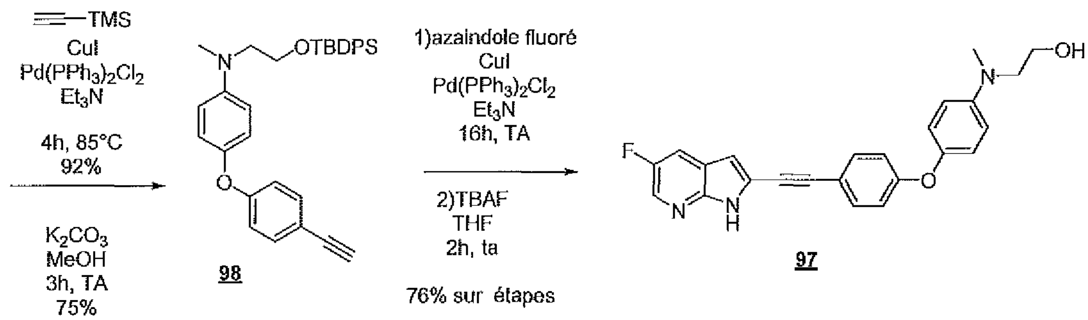


98 97

Synthèse du iodé **100** :

Sous argon, 200 mg de phenylether **99** (1.18 mmol, 1.0 éq.) est solubilisé dans 5 mL de $CH_3CN$. 528 mg de *N*-iodosuccinimide (2.35 mmol, 2.0 éq.) et 10 μL de TFA sont additionnés. Au bout de 4h à reflux, le mélange réactionnel est refroidi à température ambiante et 10 mL d'une solution de thiosulfate de soduim est ajoutée suivit de 10 mL de DCM. Puis la phase organique est lavée avec une solution de thiosulfate de soduim (3 x 10 mL). Ensuite la phase organique est séchée avec $MgSO_4$, filtrée sur coton et évaporée sous pression réduite. Le brut est trituré avec de l'éther diéthylique, suivit d'une filtration sous vide. Le iodé **100** est obtenu sous forme d'un solide blanc avec un rendement de 30%. **RMN $^1$H** (250 MHz, $CDCl_3$) δ 7.70 - 7.49 (m, 4H), 6.87 - 6.65 (m, 4H). Numéro CAS : 28896-49-3.

Synthèse du composé **101:**

Sous argon, $Pd_2dba_3$ (0.05 éq.) et $tBu_3P$ (0.075 mmol) sont ajoutés à une solution de toluène anhydre dégazée de concentration 0.3M contenant l'amine (1.0 éq.), le iodé **100** (1.0 éq.) et *t*-BuONa (1.2 éq.). Au bout de 24h à température ambiante, le solvant est évaporé sous pression réduite. Ensuite, le mélange réactionnel est concentré sous pression réduite, avant d'être purifiées par colonne de chromatographie flash sur gel de silice. Le composé **101** est obtenu sous forme d'une huile incolore avec un rendement de 33%, **Rf =** 0.20 (acétate d'éthyle/éther de pétrole = 1/49). **Infrarouge** (ν, cm-1, neat) 2928, 2856, 1610, 1508, 1477, 1426, 1390, 1273, 1232, 1163, 1105, 1056, 1003, 928, 866, 818. 781. **RMN $^1$H** (400 MHz, $CDCl_3$, 20°C) δ 7.65 (dt, *J* = 7.8, 1.3 Hz, 4H), 7.53 (d, *J* = 8.5 Hz, 2H), 7.45 - 7.33 (m, 6H), 6.85 (d, *J* = 9.0 Hz, 2H), 6.72 - 6.61 (m, 2H), 6.55 (d, *J* = 9.0 Hz, 2H), 3.81 (t, *J* = 6.2 Hz, 2H), 3.47 (t, *J* = 6.2 Hz, 2H), 2.93 (s, 3H), 1.04 (s, 9H). **RMN $^{13}$C** (101 MHz, $CDCl_3$, 20°C) δ 159.4 (Cq), 146.5 (Cq), 145.7 (Cq), 138.3 (2 x CH), 135.6 (4 x CH), 133.4 (Cq), 129.7 (4 x CH), 127.7 (2 x CH), 121.2 (2 x CH), 119.1 (2 x CH), 112.8 (2 x CH), 84.0 (Cq), 61.1 ($CH_2$), 54.9 ($CH_2$), 39.3 ($CH_3$), 26.8 (3 x $CH_3$). HRMS (+ESI) calculée pour $C_{31}H_{34}INO_2Si$ (M+H+) : 608.1476, trouvée: 608.1474.

Synthèse N-(2-((*tert*-butyldiphenylsilyl)oxy)ethyl)-4-(4-ethynylphenoxy-*N*-methylaniline **98** :

Du catalyseur [$Pd(PPh_3)_2Cl_2$] (2.8 mg, 0.004 mmol, 0.03éq.) a été ajouté à une solution dégazée du iodé **101** (80 mg, 0.131 mmol, 1.0 éq.), ethynyltrimethylsilane (0.027 mL, 0.2 mmol, 1.5 éq.), de CuI (1 mg, 0.004 mmol, 0.03 éq.), dans de la $Et_3N$ à la concentration de 0.3 M. Le mélange est agité à 85°C ambiante pendant 4 h sous atmosphère d'argon. Ensuite, le mélange réactionnel est concentré sous pression réduite, avant d'être purifiées par colonne de chromatographie flash sur gel de silice avec comme éluant (acétate d'éthyle/éther de pétrole = 20/80) pour obtenir 70 mg de l'intermédiaire qui mis en réaction avec du $K_2CO_3$ (25 mg, 0.181 mmol, 1.5 éq.) dans 6 mL d'un mélange DCM/méthanol (1/1). Au bout de 3h d'agitation à température ambiante, la solution est filtrée sur coton. Après évaporation à sec, le brut est purifié par colonne de chromatographie flash sur gel de silice avec comme éluant (acétate d'éthyle/éther

de pétrole = 30/70) pour obtenir l'alcyne **98** sous forme d'un solide marron avec un rendement de 69% sur 2 étapes. **Pf** 170-172°C, **Rf** = 0.20 (acétate d'éthyle/éther de pétrole = 30/70). **Infrarouge** (v, cm-1, neat) 3284, 3070, 2929, 2856, 1610, 1513, 1495, 1471, 1427, 1360, 1280, 1234, 1160, 1103, 985, 927, 869. **RMN $^1$H** (250 MHz, $CDCl_3$, 20°C) δ 7.65 - 7.56 (m, 4H), 7.41 - 7.28 (m, 8H), 6.82 (m, 4H), 6.62 - 6.45 (m, 2H), 3.87 - 3.64 (t, *J* = 6.2 Hz, 2H), 3.44 (t, *J* = 6.2 Hz, 2H), 2.97 (s, 1H), 2.91 (s, 3H), 1.01 (s, 9H). **RMN $^{13}$C** (63 MHz, $CDCl_3$, 20°C) δ 160.2 (2 x Cq), 147.2 (Cq), 146.2 (Cq), 136.3 (4 x CH), 134.2 (2 x CH), 134.1 (Cq), 130.4 (2 x CH), 128.4 (4 x CH), 122.0 (2 x CH), 117.2 (2 x CH), 115.8 (Cq), 113.5 (2 x CH), 77.9 (Cq) 76.6 (CH), 61.8 ($CH_2$), 55.5 ($CH_2$), 39.9 (Cq), 27.5 (3 x $CH_3$), 19.7 ($CH_3$). HRMS (+ESI) calculée pour $C_{33}H_{35}NO_2Si$ (M+H+): 506.2009, trouvée: 506.2009.

**Exemple 40.2 2-((4-(4-((5-fluoro-1*H*-pyrrolo[2,3-*b*]pyridin-2-yl)ethynyl)phenoxy)phenyl)(methyl)amino)ethan-1-ol 97**

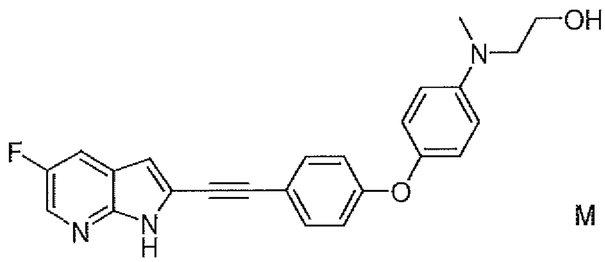

$C_{24}H_{20}FN_3O_2$
MW: 401,44 gmol-1

M

Le composé a été préparé selon la **procédure C2 suivie de la procédure F** et purifié par colonne de chromatographie flash sur gel de silice (méthanol/dichlorométhane = 2/48). Solide jaunâtre (76% sur 2 étapes) **Pf** 172-174°C, **Rf** = 0.20 (méthanol/dichlorométhane = 2/48). **Infrarouge** (v, cm-1, neat) 3122, 2925, 1662, 1610, 1510, 1494, 1436, 1399, 1349, 1296, 1246, 1165, 1120, 1048, 984, 872, 839. **RMN $^1$H** (400 MHz, DMSO-$d_6$, 20°C) δ 12.34 (s, 1H), 8.26 (s, 1H), 7.83 (m, 1H), 7.57 (m, 2H), 7.03 - 6.86 (m, 4H), 6.75 (m, 3H), 4.72 - 4.60 (t, *J* = 5.9 Hz, 1H), 3.56 (q, *J* = 5.9 Hz, 2H), 3.39 (t, *J* = 6.1 Hz, 2H), 2.93 (s, 3H). **RMN $^{13}$C** (101 MHz, DMSO-$d_6$ 20°C) δ 155.3 (d, *J* = 239.2 Hz, Cq), 147.9 (Cq), 147.2 (Cq), 145.6 (Cq), 144.9 (Cq), 133.7 (2 x CH), 132.8 (d, *J* = 20.9 Hz, CH) 122.1 (Cq), 121.9 (2 x CH), 120.2 (d, *J* = 6.2 Hz, Cq), 117.0 (2 x CH), 114.9 (Cq), 114.0 (d, *J* = 20.2 Hz, CH), 113.3 (2 x CH), 106.4 (d, *J* = 4.0 Hz, CH), 93.7 (Cq), 81.2 (Cq), 58.6 ($CH_2$), 55.1 ($CH_2$), 39.2 ($CH_3$). **RMN $^{19}$F** (376 MHz, DMSO-$d_6$, 20°C) δ. -138.4. HRMS (+ESI) calculée pour $C_{24}H_{20}FN_3O_2$ (M+H+) : 402.1612, trouvée: 402.1611.

**Exemple 41. 4-((1*H*-pyrrolo[2,3-*b*]pyridin-2-yl)ethynyl)-2-fluoro-*N,N*-dimethylaniline 103**

Le 4-((1*H*-pyrrolo[2,3-*b*]pyridin-2-yl)ethynyl)-2-fluoro-*N,N*-dimethylaniline a été synthétisée en 4 étapes. Dans un premier temps, l'alcyne correspondant **104** a été préparé en 3 étapes à partir de l'aldéhyde **105** et puis nous avons effectué la réaction de Sonogashira avec l'azaindole pour obtenir le composé final.

**Exemple 41.1 Synthèse 5-ethynyl-2-fluoro-*N,N*-dimethylaniline 104 :**

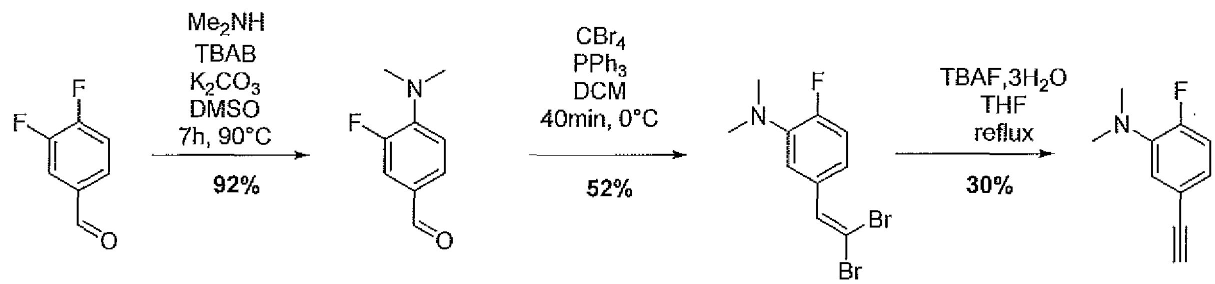


105 106 107 104

Synthèse de l'aldéhyde **106** :

Sous argon, l'aldéhyde **105** (500 mg, 3.52 mmol, 1.0 éq.) est solubilisé dans 3 mL de DMSO. Ensuite, TBAB (567 mg, 1.76 mmol, 0.5 éq.), $K_2CO_3$ (486 mg, 3.52 mmol, 1.0 éq.) et de la diméthylamine (C = 2M, 8.8 mL, 17.6 mmol, 5.0 éq.) sont ajoutés successivement. Au bout de 7h à 90°C, 10 mL d'une solution saturée de $NaHCO_3$ est additionnés. La phase organique est extraite avec de l'acétate d'éthyle (3 x 10mL) puis elles sont réunies, séchées avec $MgSO_4$ et filtrées sur coton. Après évaporation sous pression réduite, le brut est purifié par colonne de chromatographie flash sur gel de silice avec comme éluant (acétate d'éthyle/éther de pétrole = 30/70) pour obtenir à l'aldéhyde **106** sous forme d'une huile incolore avec un rendement de 92%. **RMN $^1$H** (250 MHz, $CDCl_3$) δ 9.72 (s, 1H), 7.57 - 7.38 (m, 2H), 6.78 (m, 1H), 3.02 (s, 6H). Numéro CAS : 1021240-69-6.

Synthèse du 5-ethynyl-2-fluoro-*N,N*-dimethyl-aniline **104** :

Sous argon, $CBr_4$ (198 mg, 0.6 mmol, 1.0 éq.) est solubilisé dans 5 mL de DCM. A 0°C, $PPh_3$ (315 mg, 1.2 mmol, 2.0 éq.) est ajouté en 1 portion. Au bout de 10 minutes, l'aldéhyde **106** (100 mg, 0.6 mmol, 1.0 éq.) est solubilisé dans 5 mL de DCM puis ajouté goutte à goutte. Au bout de 40 minutes à 0°C, 10 mL de $H_2O$ est ajouté. La phase organique est extraite avec de l'acétate d'éthyle (3x10mL) puis elles sont réunies, séchées avec $MgSO_4$ et filtrées sur coton. Après évaporation sous pression réduite, le brut est purifié par colonne de chromatographie flash sur gel de silice avec comme éluant (acétate d'éthyle/éther de pétrole = 10/40) pour obtenir le composé **107** sous forme d'un solide blanchâtre avec un rendement de 52%. Ensuite, il est mis en réaction avec du TBAF,$3H_2O$ (780 mg, 2.47 mmol, 8.0 éq.) dans un mélange THF/$H_2O$ (2 mL/0.1 mL). Au bout de 7h à 90°C, le mélange est refroidi à température ambiante et 5 mL d'H2O est ajouté. La phase organique est extraite avec de l'acétate d'éthyle (3x10mL) puis elles sont réunies, séchées avec $MgSO_4$ et filtrées sur coton. Après évaporation sous pression réduite, le brut est purifié par colonne de chromatographie flash sur gel de silice avec comme éluant (acétate d'éthyle/éther de pétrole = 1/99) pour obtenir à l'alcyne **104** sous forme d'une huile jaune avec un rendement de 30%. **Rf** = 0.50 (acétate d'éthyle/éther de pétrole = 1/99). **Infrarouge** (v, cm-1, neat) 3290, 2970, 2930,2853, 2102, 1606, 1511, 1463, 1434, 1356, 1336, 1273, 1245, 1227, 1185, 1140, 1023, 935. **RMN $^1$H** (400 MHz, $CDCl_3$, 20°C) δ 7.22 - 7.09 (m, 2H), 6.81 - 6.69 (m, 1H), 3.00 (s, 1H), 2.89 (s, 6H). **RMN $^{13}$C** (101 MHz, $CDCl_3$, 20°C) δ 153.5 (d, *J* = 245.3 Hz, Cq), 141.4 (d, *J* = 8.4 Hz, Cq) 128.6 (d, *J* = 3.0 Hz, CH) 119.7 (d, *J* = 23.1 Hz, CH), 117.4 (d, *J* = 4.5 Hz, CH), 113.3 (d, *J* = 9.2 Hz, Cq), 83.0 (d, *J* = 2.7 Hz, Cq), 76.23 (CH), 42.4 ($CH_3$), 42.4 ($CH_3$). **RMN $^{19}$F** (376 MHz, $CDCl_3$, 20°C) δ. -122.4. HRMS (+ESI) calculée pour C10H10FN (M+H+) : 164.0870, trouvée: 164.0872.

**Exemple 41.2 4-((1*H*-pyrrolo[2,3-*b*]pyridin-2-yl)ethynyl)-2-fluoro-*N,N*-dimethylaniline 103**

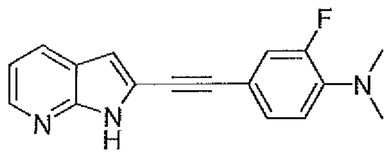

$C_{17}H_{14}FN_3$
M W: 279,32 gmol-1

Le composé a été préparé selon la **procédure C2** et purifié par colonne de chromatographie flash sur gel de silice (acétate d'éthyle/éther de pétrole = 45/55). Solide jaunâtre (79%), **Pf** 180-182°C, **Rf** = 0.20 (acétate d'éthyle/éther de pétrole = 30/70). **Infrarouge** (v, cm-1, neat) 3157, 3072, 2893, 2205, 1605, 1541, 1511, 1432, 1273, 1224, 1183, 809, 763, 507. **RMN $^1$H** (400 MHz, DMSO-$d_6$, 20°C) δ 12.15 (s, 1H), 8.27 (s, 1H), 7.95 (d, *J* = 7.8 Hz, 1H), 7.46 - 7.20 (m, 2H), 7.20 - 7.03 (m, 1H), 6.96 (t, *J* = 9.0 Hz, 1H), 6.78 (s, 1H), 2.89 (s, 6H). **RMN $^{13}$C** (101 MHz, DMSO-$d_6$, 20°C) δ 152.9 (d, *J* = 243.9 Hz, Cq), 148.5 (Cq), 144.5 (CH), 141.2 (d, *J* = 8.0 Hz, Cq), 128.6 (m, 2 x CH), 120.2 (Cq), 119.8 (Cq), 119.6 (d, *J* = 22.8 Hz, CH), 118.2 (d, *J* = 4.7 Hz, CH), 116.6 (CH), 112.2 (d, *J* = 9.7 Hz, Cq), 106.0 (CH), 92.5 (Cq), 81.5 (Cq), 42.2($CH_3$), 42.1 ($CH_3$). **RMN $^{19}$F** (376 MHz, DMSO-$d_6$, 20°C) δ . -122.1. HRMS (+ESI) calculée pour C17H14FN3 (M+H+): 280.1244, trouvée: 280.1245.

**Exemple 42: 2-((4-(1*H*-pyrazol-1-yl)phenyl)ethynyl)-5-ftuoro-1*H*-pyrrolo[2,3-*b*]pyridine 108**

Le 2-((4-(1*H*-pyrazol-1-yl)phenyl)ethynyl)-5-fluoro-1*H*-pyrrolo[2,3-*b*]pyridine a été synthétisée en 4 étapes. Dans un premier temps, l'alcyne correspondant **109** a été préparé en 3 étapes à partir du composé **110** et puis nous avons effectué la réaction de Sonogashira avec l'azaindole pour obtenir le composé final.

**Exemple 42.1 Synthèse 1-(4-ethynylpheny)-1*H*-pyrazole 109:**

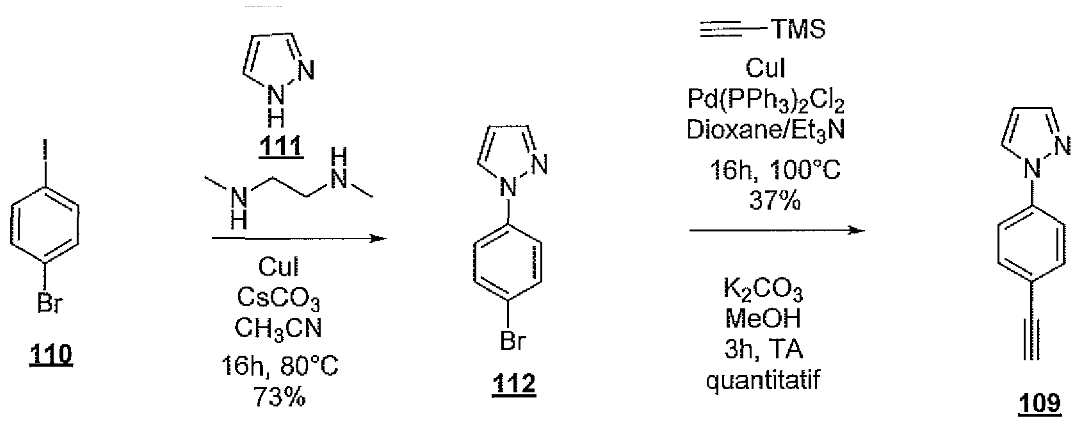


Synthèse du bromé 112:

Du CuI (27 mg, 0.141 mmol, 20 % mol) a été ajouté à une solution dégazée de dérivé iodé **110**, (200 mg, 0.707 mmol, 1.0 éq.), de amine **111** (50.5 mg, 0.742 mmol, 1.05 éq.), de *N,N'*-dimethylethylenediamine (0.026 mL, 0.282 mmol, 20 % mol) et $CsCO_3$ (459 mg, 1.41 mmol, 2.0 éq.) dans 3.0 mL de $CH_3CN$. Le mélange est agité à 80°C pendant 16 h sous atmosphère d'argon. Ensuite, le mélange est refroidi et filtré sur Celite®. Le filtrat est évaporé sous pression réduite avant d'être purifié par colonne de chromatographie flash sur gel de silice avec comme éluant (acétate d'éthyle/éther de pétrole = 25/75) pour obtenir 115 mg de du bromé **112** sous forme d'une huile incolore avec un rendement 73%. **RMN $^1$H** (250 MHz, $CDCl_3$, 20°C) δ 7.86 (m, 1H), 7.69 (m, 1H), 7.55 (m, 4H), 6.45 (m, 1H). Numéro CAS : 13788-92-6.

Synthèse du 1-(4-ethynylphenyl)-1*H*-pyrazole **109:**

Sous argon, $Pd(PPh_3)_2Cl_2$ (6 mg, 0.009 mmol, 0.04 éq.) et CuI (2 mg, 0.009 mmol, 0.04 éq.) sont ajoutés à une solution de $Et_3N$/Dioxane dégazée (1.5 mL (2/1)) contenant l'éthynyltrimethylsilane (0.155 mL, 1.12 mmol, 5.0 éq.), le dérivé bromé **112** (50 mg, 0.224 mmol, 1.0 éq.). Au bout de 16h à 100°C, le solvant est évaporé sous pression réduite. Ensuite, le mélange réactionnel est purifié par colonne de chromatographie flash sur gel de silice. Puis, 20 mg d'alcyne intermédiaire est mis en réaction avec $K_2CO_3$ (17 mg, 0.125 mmol, 1.5 éq.) dans 3 mL de méthanol. Au bout de 3h à température ambiante, le solvant est évaporé sous pression réduite. Le brut est solubilisé dans 10 mL d'acétate d'éthyle. Puis la phase organique est lavée avec de la saumure (3 x 10 mL). Ensuite la phase organique est séchée avec $MgSO_4$, filtrée sur coton et évaporée sous pression réduite pour conduire à l'alcyne **109** sous forme d'un solide blanc avec un rendement de 37% sur 2 étapes. **Pf** 54-56°C, **Rf =** 0.5 (acétate d'éthyle/éther de pétrole = 2/48). **Infrarouge** (v, cm-1, neat) 3273, 3232, 3150, 2918, 1606, 1521, 1506, 1390, 1335, 1312, 1248, 1205, 1193, 1205, 1193, 1118, 1045, 1028, 934. **RMN $^1$H** (250 MHz, $CDCl_3$, 20°C) δ 7.90 (dd, *J* = 2.5, 0.6 Hz, 1H), 7.71 (dd, *J* = 1.8, 0.6 Hz, 1H), 7.65 (d, *J*= 8.9 Hz, 2H), 7.55 (d, *J* = 8.9 Hz, 2H), 6.45 (dd, *J* = 2.5, 1.8 Hz, 1H), 3.09 (s, 1H). **RMN $^{13}$C** (63 MHz, $CDCl_3$ 20°C) δ 141.5 (CH), 140.1 (Cq), 133.3 (2 x CH), 126.7 (CH), 120.0 (Cq), 118.7 (2 x CH), 108.1 (CH), 82.9 (Cq), 77.9 (CH). HRMS (+ESI) calculée pour $C_{11}H_9N_2$ (M+H+) : 169.0760, trouvée: 169.0763.

**Exemple 42.2 2-((4-(1*H*-pyrazol-1-yl)phenyl)ethynyl)-5-fluoro-1*H*-pyrrolo[2,3-*b*]pyridine 108**

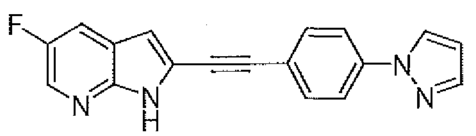

$C_{10}H_{11}FN_4$
M W: 302,31 gmol-1

Le composé a été préparé selon la **procédure C2** et purifié par colonne de chromatographie flash sur gel de silice (acétate d'éthyle/éther de pétrole = 10/40). Solide jaunâtre (51%), **Pf** >260°C, **Rf =** 0.1 (acétate d'éthyle/éther de

pétrole = 10/40). **Infrarouge** (ν, cm-1, neat) 3157, 3072, 2893, 2205, 1605, 1541, 1511, 1432, 1273, 1224, 1183, 809, 763, 507. **RMN $^1$H** (400 MHz, DMSO-$d_6$, 20°C) δ 12.41 (s, 1H), 8.60 (d, *J* = 2.6 Hz, 1H), 8.36 - 8.21 (m, 1H), 7.96 (d, *J* = 8.7 Hz, 1H), 7.90 - 7.79 (m, 2H), 7.71 (d, *J* = 8.7 Hz, 2H), 6.84 (d, *J* = 1.9 Hz, 1H), 6.5 (s, 1H). **RMN $^{13}$C** (101 MHz, DMSO-$d_6$, 20°C) δ 155.7 (d, *J* = 240.5 Hz, Cq), 145.7 (Cq), 142.1 (CH), 140.3 (Cq), 133.3 (d, *J* = 29.0 Hz, CH), 133.1 (2 x CH), 128.5 (CH), 121.6 (Cq), 120.1 (d, *J* = 7.5 Hz, Cq), 119.3 (Cq), 118.9 (2 x CH), 114.2 (d, *J* = 20.7 Hz, CH), 108.9 (CH), 106.8 (d, *J* = 4.6 Hz, CH), 93.2 (Cq), 82.7 (Cq). **RMN $^{19}$F** (376 MHz, DMSO-$d_6$, 20°C) δ. -122.1. HRMS (+ESI) calculée pour C18H11FN4 (M+H+) : 303.1040, trouvée: 303.1039.

**Exemple 43.2-[2-[4-(4-fluoro-1-piperidyl)phenyl]ethyny]-1*H*-pyrrolo[2,3-*b*]pyridine 113**

Le 2-[2-[4-(4-fluoro-1-piperidyl)phenyl]ethynyl]-1*H*-pyrrolo[2,3-*b*]pyridine **113** a été synthétisée en 4 étapes. Dans un premier temps, l'alcyne correspondant **114** a été préparé en 3 étapes à partir dibromobenzène et puis nous avons effectué la réaction de Sonogashira avec l'azaindole pour obtenir le composé final.

**Exemple 43.1 Synthèse du 1-(4-ethynylphenyl)-4-fluoropiperidine 114**

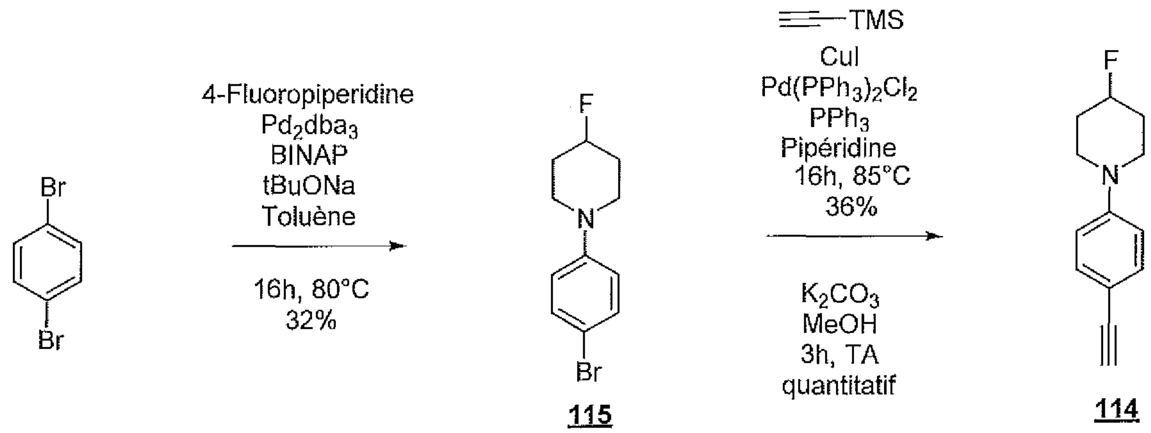


**115** **114**

Synthèse du bromé **115** :

Le composé **115** a été préparé selon la **procédure D** et purifié par colonne de chromatographie flash sur gel de silice (acétate d'éthyle/éther de pétrole = 1/49). Huile incolore (32%). **RMN $^1$R** (250 MHz, $CDCl_3$) δ 7.33 (d, *J* = 8.9 Hz, 2H), 6.80 (d, *J* = 8.9 Hz, 2H), 4.97 - 4.61 (m, 1H), 3.41 - 3.22 (m, 2H), 3.25 - 3.08 (m, 2H), 2.14 - 1.86 (m, 4H). Numéro CAS : 1423130-86-2.

Synthèse du 1-(4-ethynylphenyl)-4-fluoro-piperidine **114** :

L'alcyne **114** a été préparé selon la **procédure E** et purifié par colonne de chromatographie flash sur gel de silice (acétate d'éthyle/éther de pétrole = 2/48). Huile incolore (36% sur les deux étapes). **Infrarouge** (ν, cm$^{-1}$, neat) 3291, 2962, 2930, 2853, 2102, 1606, 1511, 1463, 1434, 1373, 1356, 1336, 1273, 1245, 1227, 1185, 1141, 1093, 1023, 935. **RMN $^1$H** (400 MHz, $CDCl_3$,20°C) δ 7.40 (d, *J* = 8.8 Hz, 2H), 6.87 (d, *J* = 8.8 Hz, 2H), 4.85 (m, 1H), 3.51 - 3.37 (m, 2H), 3.34 - 3.20 (m, 2H), 3.01 (s, 1H), 2.15 - 1.88 (m, 4H). **RMN $^{13}$C** (101 MHz, $CDCl_3$, 20°C) δ 150.9 (Cq), 133.3 (2 x CH), 115.4 (2 x CH), 111.9 (Cq), 88.1 (d, *J* = 171.3 Hz, CH), 84.2 (Cq), 75.4 (CH), 44.9 ($CH_2$), 44.9 ($CH_2$), 30.9 ($CH_2$), 30.7 ($CH_2$). **RMN $^{19}$F** (376 MHz, $CDCl_3$, 20°C) δ. -181.4. HRMS (+ESI) calculée pour C13H14FN (M+H+) : 204.1183, trouvée: 204.1181.

**Exemple 43.2 2-[2-[4-(4-fluoro-1-piperidyl)phenyl]ethynyl]-1*H*-pyrrolo[2,3-*b*]pyridine 113**

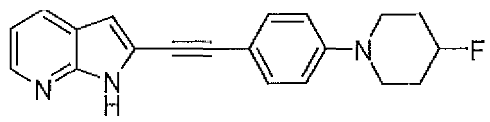

$C_{20}H_{18}FN_3$
MW: 319,38 gmol-1

Le composé a été préparé selon la **procédure C1** et purifié par colonne de chromatographie flash sur gel de silice (acétate d'éthyle/éther de pétrole = 30/70). Solide jaune (quantitatif), **Pf** >260°C, $\boldsymbol{R_f}$ = 0.20 (acétate d'éthyle/éther de pétrole = 30/70). **Infrarouge** (v, $cm^{-1}$, neat) 3113, 3057, 2955, 2932, 2819, 2207, 1600, 1584, 1533, 1505, 1405, 1370, 1354, 1278, 1224, 1189, 1027, 816. **RMN $^1$H** (250 MHz, DMSO-$d_6$, 20°C) δ 12.05 (s, 1H), 8.21 (s, 1H), 7.91 (d, *J* = 7.8 Hz, 1H), 7.38 (d, *J* = 8.6 Hz, 2H), 7.09 (m, 1H), 6.98 (d, *J* = 8.6 Hz, 2H), 6.69 (s, 1H), 4.99 - 4.76 (m, 2H), 3.49 (dt, *J* = 13.5, 5.4 Hz, 2H), 3.33 (dt, *J* = 13.5, 5.4 Hz, 2H), 2.02 (m, 4H). **RMN $^{13}$C DEPT** (63 MHz, DMSO-$d_6$, 20°C) δ 144.4 (CH), 132.9 (2 x CH), 128.5 (CH), 116.7 (CH), 115.3 (2 x CH), 105.8 (CH), 88.8 (d, *J* = 169.2 Hz, CH), 44.4 (d, *J* = 6.8 Hz, 2 x $CH_2$), 30.8 (d, *J* = 19.1 Hz, 2 x $CH_2$). **RMN $^{19}$F** (235 MHz, $CDCl_3$, 20°C) δ. -181.5. **HRMS (+ESI)** calculée pour $C_{20}H_{18}FN_3$ (M+H$^+$): 320.1157, trouvée: 320.1557.

**Exemple 44. 4'-((5-fluoro-1*H*-pyrrolo[2,3-*b*]pyridin-2-yl)ethynyl)-*N,N*-dimethyl-[1,1'-biphenyl]-4-amine 116**

Le 4'-((5-fluoro-1*H*-pyrrolo[2,3-*b*]pyridin-2-y))ethynyl)-*N,N*-dimethyl-[1,1'-biphenyl]-4-amine a été synthétisée en 4 étapes. Dans un premier temps, l'alcyne correspondant **117** a été préparé en 3 étapes à partir du composé **119** et puis nous avons effectué la réaction de Sonogashira avec l'azaindole pour obtenir le composé final.

**Exemple 44.1 Synthèse du 4'-ethynyl-*N,N*-dimethyl-[1,1'-biphenyl]-4-amine 117 :**

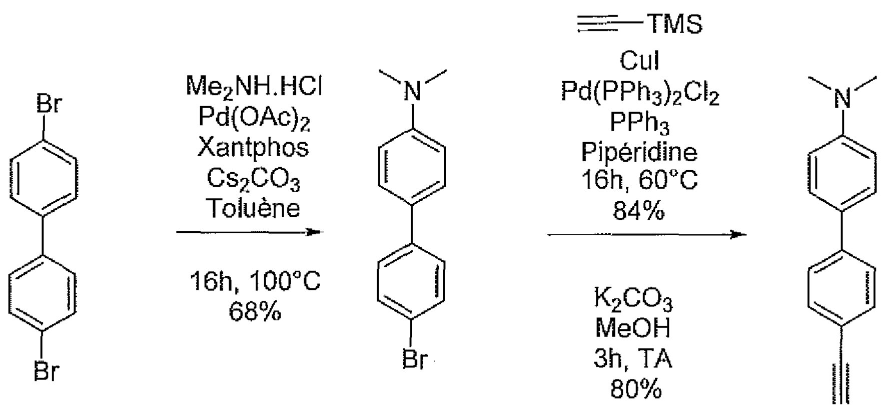


**119** **118** **117**

Synthèse du bromé **118** :

Sous argon, $Pd(OAc)_2$ (0.05 éq.) et Xantphos (0.067 mmol) sont ajoutés à une solution de toluène anhydre dégazée de concentration 0.3M contenant la diméthylamine chlorhydrate (1.0 éq.), le bromé **119** (2.0 éq.) et $Cs_2CO_3$ (3.0 éq.). Au bout de 16h à 100°C, le solvant est évaporé sous pression réduite. Ensuite, le mélange réactionnel est concentré sous pression réduite, avant d'être purifié par colonne de chromatographie flash sur gel de silice pour conduire au bromé **118** sous forme d'un solide blanc avec un rendement de 68%. **RMN $^1$H** (250 MHz, $CDCl_3$) δ 7.51 - 7.34 (m, 6H), 6.76 (d, *J* = 8.9 Hz, 2H), 2.97 (s, 6H). Numéro CAS : 92194-03-1.

Synthèse de 4-(4-ethynylphenyl)-*N,N*-dimethyl-aniline **117** :

L'alcyne **117** a été préparé selon la **procédure E** et à une température de 60°C et purifié par colonne de chromatographie flash sur gel de silice (acétate d'éthyle/éther de pétrole = 2/48). Huile incolore (67% sur les deux étapes). **RMN $^1$H** (250 MHz, $CDCl_3$) δ 7.56 - 7.34 (m, 6H), 6.84 - 6.67 (m, 2H), 3.31 (s, 1H), 2.97 (s, 6H). Numéro CAS : 1190376-40-9.

**Exemple 44.2 4'-((5-fluoro-1*H*-pyrrolo[2,3-*b*]pyridin-2-yl)ethynyl)-*N,N*-dimethyl-[1,1'-biphenyl]-4-amine 116**

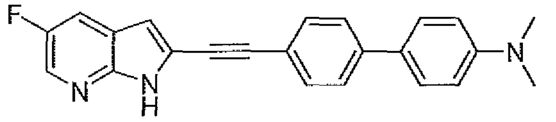

$C_{23}H_{18}FN_3$
M W: 355,41 gmol-1

Le composé a été préparé selon la **procédure C2** et purifié par colonne de chromatographie flash sur gel de silice (acétate d'éthyle/éther de pétrole = 30/70). Solide jaune (quantitatif), **Pf** >260°C, $\boldsymbol{R_f}$ **=** 0.20 (acétate d'éthyle/éther de pétrole = 30/70). **Infrarouge** (ν, $cm^{-1}$, neat) 3428, 3157, 3072, 2893, 2205, 1605, 1541, 1511, 1432, 1273, 1224, 1183, 809, 763, 507. **RMN $^1$H** (250 MHz, DMSO-$d_6$, 20°C) δ 12.38 (s, 1H), 8.28 (s, 1H), 7.86 (dd, *J* = 9.4, 2.7 Hz, 1H), 7.71 (d, *J* = 8.1 Hz, 2H), 7.63 - 7.53 (m, 4H), 6.86 - 6.75 (m, 3H), 2.97 (s, 6H). **RMN $^{13}$C DEPT** (63 MHz, DMSO-$d_6$ 20°C) δ 133.1 (d, *J* = 29 Hz, CH), 132.3 (2 x CH), 127.6 (2 x CH), 126.0 (2 x CH), 114.1 (d, *J* = 20.8 Hz, CH), 113.03 (2 x CH), 106.6 (d, *J* = 4.8 Hz, CH), 40.9 (2 x CH3). **RMN $^{19}$F** (235 MHz, DMSO-$d_6$, 20°C) δ. -138.5. **HRMS** (+ESI) calculée pour $C_{23}H_{18}FN_3$ ($M+H^+$) : 356.1557, trouvée: 356.1554.

**Exemple 45. (5-((5-fluoro-1*H*-pyrrolo[2,3-*b*]pyridin-2-yl)ethynyl)furan-2-yl)methanol 120**

Le (5-((5-fluoro-1*H*-pyrrolo[2,3-*b*]pyridin-2-yl)ethynyl)furan-2-yl)methanol a été synthétisée en 4 étapes. Dans un premier temps, l'alcyne correspondant **121** a été préparé en 3 étapes à partir du composé **122** et puis nous avons effectué la réaction de Sonogashira avec l'azaindole pour obtenir le composé final.

**Exemple 45.1 Préparation du (5-ethynylfuran-2-yl)methanol 121**

L'alcyne **121** a été préparé en trois étapes en partant du 2-bromo-2-furaldehyde **122.** La première étape a été décrite dans l'Organometallics, (vol. 24, n°4, 2005, p. 693) et pour la dernière étape a été trouvée dans un brevet (US2010/63041 A1 p.30)

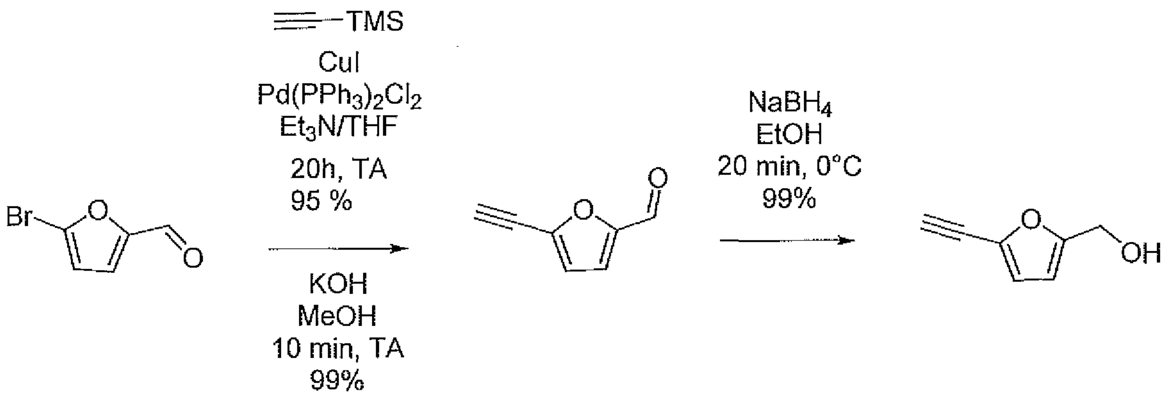


**122** **123** **121**

Synthèse de l'aldéhyde 123 :

Le composé **123** a été préparé selon la **procédure G.** Huile marron (99%), $\boldsymbol{R_f}$ = 0.5 (acétate d'éthyle/éther de pétrole = 2/98). Numéro CAS : 153026-71-2.

Synthèse du (5-ethynylfuran-2-yl)methanol **121:**

100 mg d'aldéhyde **123** (0.83 mmol, 1.0 éq.) est mis en solution avec du $NaBH_4$ (14 mg, 0.37 mmol, 0.45 éq.) dans 2.5 mL d'Ethanol. Après 20 min à 0°C, 10 mL d'$H_2O$ est ajoutée puis la phase organique est extraite avec de l'acétate d'éthyle (3 x 10 mL). Les phases organiques sont réunies, séchées sur $MgSO_4$, filtrées sur coton puis évaporées sous pression réduite. L'alcyne **121** est obtenu sous forme d'une huile marron avec un rendement de 99%. **RMN $^1$H**

(250 MHz, $CDCl_3$, 20 °C) δ 6.47 (dd, *J* = 3.3, 0.7 Hz, 1H), 6.15 (dd, *J* = 3.3, 0.7 Hz, 1H), 4.45 (s, 2H,), 3.32 (s, 1H). Numéro CAS : 153026-89-2.

**Exemple 45.2 (5-((5-fluoro-1*H*-pyrrolo[2,3-*b*]pyridin-2-yl)ethynyl)furan-2-yl)methanol 120**

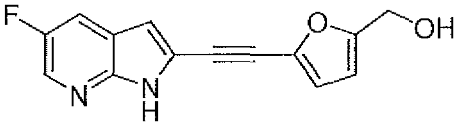

$C_{14}H_9FN_2O_2$
M W: 256,24 gmol-1

Le composé a été obtenu selon la procédure **C2** et purifié par colonne de chromatographie flash sur gel de silice (méthanol/dichlorométhane /$NH_4OH$ = 4/94/2). Solide jaune (30%). **Pf** : 216 °C. **Rf** = 0.42 (méthanol/dichlorométhane / $NH_4OH$ = 4/94/2). **Infrarouge** (v, $cm^{-1}$, neat) 3200, 3059, 2975, 2884, 2206, 1586, 1506, 1401, 1299, 1277, 1158, 1112, 1022, 982, 873. **RMN $^1$H** (250 MHz, DMSO-$d_6$, 20°C) δ 12.46 (s, 1H), 8.41 - 8.18 (m, 1H), 7.87 (dd, *J* = 9.3, 2.7 Hz, 1H), 6.94 (d, *J* = 3.4 Hz, 1H), 6.88 (d, *J* = 1.9 Hz, 1H), 6.45 (d, *J* = 3.3 Hz, 1H), 5.39 (t, *J* = 5.8 Hz, 1H), 4.40 (d, *J* = 5.8 Hz, 2H). **RMN $^{13}$C** (63 MHz, DMSO-$d_6$, 20°C) δ 159.9 (d, *J* = 249.9 Hz, Cq), 146.1 (Cq), 145.1 (Cq), 134.6 (CH), 133.8 (Cq), 133.6 (Cq), 120.5 (Cq), 118.2 (CH), 114.2 (d, *J* = 20.6 Hz, CH), 109.0 (CH), 107.5 (d, *J* = 4.5 Hz, CH), 87.8 (Cq), 84.4 (Cq), 58.5 ($CH_2$). **RMN $^{19}$F** (235 MHz, DMSO-$d_6$, 20°C) δ - 138.16 . **HRMS (+ESI)** calculée pour $C_{14}H_9FN_2O_2$ ($M+H^+$) : 257.0720, trouvée: 257.0722.

**Exemple 46. 2-((5-((4-fluoropiperidin-1-yl)methyl)thiophen-2-yl)ethynyl)-1*H*-pyrrolo[2,3-*b*]pyridine 124**

Le 2-((5-((4-fluoropiperidin-1-yl)methyl)thiophen-2-yl)ethynyl)-1*H*-pyrrolo[2,3-*b*]pyridine a été synthétisée en 4 étapes. Dans un premier temps, l'alcyne correspondant **125** a été préparé en 3 étapes à partir du composé **126** et puis nous avons effectué la réaction de Sonogashira avec l'azaindole pour obtenir le composé final.

**Exemple 46.1 Synthèse du 1-((5-ethynylthiophen-2-yl)methyl)-4-fluoropiperidine 125:**

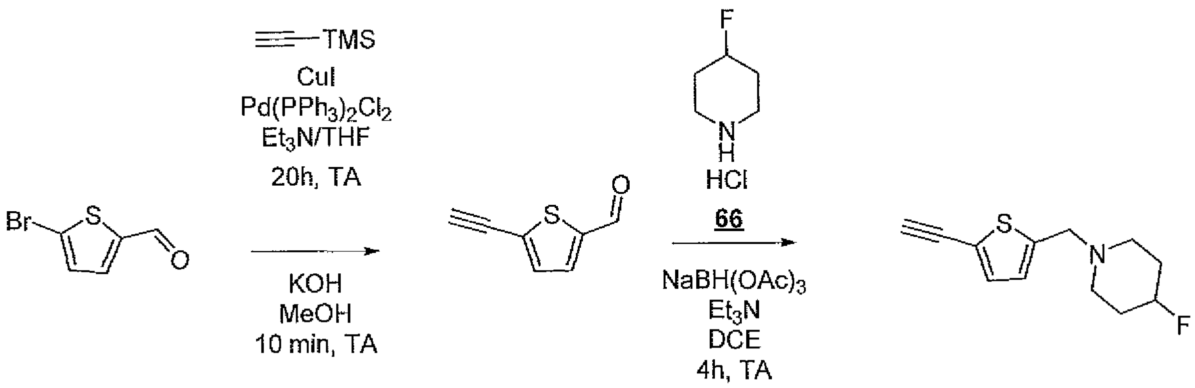


126 127 125

Synthèse du 1-((5-ethynylthiophen-2-yl)methyl)-4-fluoropiperidine **125:**

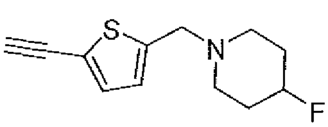

$C_{12}H_{14}FNS$
M W: 223,31gmol-1

Le composé a été préparé selon la **procédure H** et purifié par colonne de chromatographie flash sur gel de silice (acétate d'éthyle/éther de pétrole = 10/90). Huile marron (79 %). **$R_f$ =** 0.45 (acétate d'éthyle/éther de pétrole = 10/90). **Infrarouge** (v, $cm^{-1}$, neat) 3287, 2949, 1358, 1035, 808, 576, 546, 525. **RMN $^1$H** (250 MHz, $CDCl_3$, 20 °C) $\delta$ 7.09 (dd, *J* = 1.3 Hz, 1H), 6.66-6.78 (m, 1H), 4.48-4.82 (m, 1H), 3.58-3.70 (m, 2H), 3.18-3.37 (m, 1H), 2.74 (m, 4H), 1.68-1.96 (m, 4H). **RMN $^{13}$C** (63 MHz, $CDCl_3$, 20 °C) $\delta$153.4 (Cq), 135.7(Cq), 116.9 (Cq), 109.8(Cq), 88.1 (d, *J* = 170.6 Hz, CH), 81.8 (CH) , 74.1 (Cq), 54.80 ($CH_2$), 49.1 (d, *J* = 5.7 Hz, 2 x $CH_2$), 31.3 (d, *J* = 19.7 Hz, 2 x $CH_2$). **RMN $F^{19}$** (235 MHz, $CDCl_3$, 20°C) $\delta$ -181. **HRMS (+ESI):** calculée pour $C_{12}H_{14}FNS$ 223.31, trouvée : 223.09.

**Exemple 46.2 2-((5-((4-fluoropiperidin-1-yl)methyl)thiophen-2-yl)ethynyl)-1*H*-oyrrolo[2,3-*b*]pyridine 124**

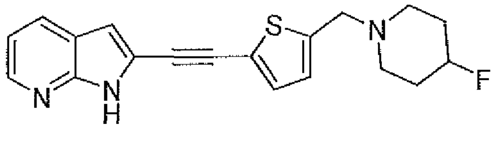

$C_{19}H_{18}FN_3S$
M W :339,43 gmol-1

Le composé a été synthétisé selon la procédure **H** et purifié par colonne de chromatographie flash sur gel de silice (DCM/MeOH/$NH_4OH$ = 94/4/2). Solide jaunâtre (60 %). **Pf** : 148 °C. ***$R_F$*** = 0.60 (DCM/MeOH/$NH_4OH$ = 94/4/2). **Infrarouge** (v, $cm^{-1}$, neat) 1283, 1123, 1101, 986, 926, 806, 767, 622, 566, 526. **RMN $^1$H** (250 MHz, $CDCl_3$, 20°C) $\delta$ 10.86 (s, 1H), 8.50 - 8.31 (m, 1H), 7.95 (d, *J* = 7.8 Hz, 1H), 7.23 (d, *J* = 3.6 Hz, 1H), 7.14 (dd, *J* = 7.8, 4.7 Hz, 1H), 6.87 (d, *J* = 3.6 Hz, 1H), 6.78 (s, 1H), 4.88 - 4.51 (m, 1H), 3.74 (s, 2H), 2.59 (ddt, *J* = 24.7, 11.0, 4.6 Hz, 4H), 1.94 (ddt, *J* = 24.7, 11.0, 4.6 Hz, 4H). **RMN $^{13}$C** (63 MHz, $CDCl_3$, 20°C) $\delta$ 148.4 (Cq), 145.5(Cq), 144.1(Cq), 132.41(Cq), 129.1 (Cq), 125.7 (Cq), 121.5 (d, *J* = 99.3 Hz, Cq), 120.5 (Cq), 119.5 (Cq), 116.5 (CH), 106.6 (CH), 87.16 (CH), 84.9 (CH), 57.3 ($CH_2$), 49.2 (d, *J* = 5.7 Hz, $CH_2$), 49.2 ($CH_2$), 31.5 (d, *J* = 19.5 Hz, $CH_2$), 31.3 ($CH_2$). **RMN $^{19}$F** (235 MHz, $CDCl_3$, 20°C) $\delta$ -135,79 (m, 1F). **.HRMS (+ESI)** calculée pour $C_{19}H_{18}FN_3S$ (M+$H^+$) : 340.1278, trouvée: 340.1273.

**Exemple 47. 2-((5-((4-fluoropiperidin-1-yl)methyl)furan-2-yl)ethynyl)-1*H*-pyrrolo[2,3-*b*]pyridine 128**

Le 2-((5-((4-fluoropiperidin-1-yl)methyl)furan-2-yl)ethynyl)-1*H*-pyrrolo[2,3-*b*]pyridine a été synthétisé en 4 étapes. Dans un premier temps, l'alcyne correspondant 130 a été préparé en 3 étapes à partir du composé 122 et puis nous avons effectué la réaction de Sonogashira avec l'azaindole pour obtenir le composé final.

**Exemple 47.1 Synthèse du 1-((5-ethynylfuran-2-yl)methyl)-4-fluoropiperidine 130:**

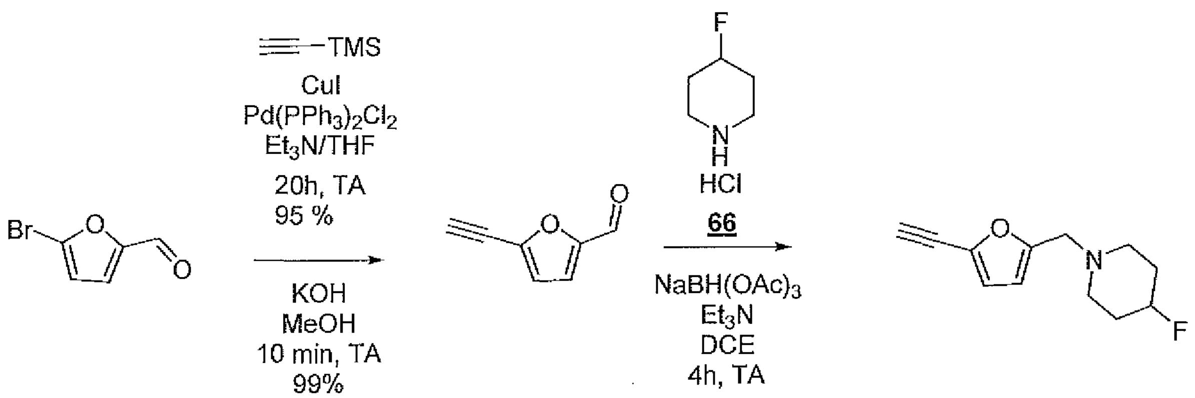


122 123 130

Synthèse du 1-((5-ethynylfuran-2-yl)methyl)-4fluoropiperidine **130**

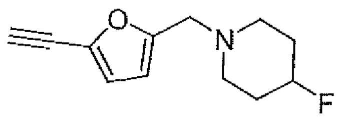

$C_{12}H_{14}FNO$
M W: 207,25 gmol-1

Le composé a été préparé selon la **procédure H** et purifié par colonne de chromatographie flash sur gel de silice (méthanol/dichlorométhane = 2/98). Huile marron (79 %). $\mathbf{R_f}$ = 0.45 (méthanol/dichlorométhane = 2/98). **Infrarouge** (ν, $cm^{-1}$, neat) 3280, 1425, 1332, 1203, 1018, 795, 537, 520, 506. **RMN $^1$H** (250 MHz, $CDCl_3$, 20°C) δ 6.49 (d, 1H, *J* = 3.3 Hz), 6.05-6.17 (m, 1H), 4.42-4.73 (m, 1H), 3.41-3.50 (m, 2H), 3.29-3.37 (m, 1H), 2.22-2.61 (m, 4H), 1.64-1.87(m, 4H). **RMN $^{13}$C** (63 MHz, $CDCl_3$, 20°C) δ 153.4 (Cq), 135.7(Cq), 116.9 (Cq), 109.8(Cq), 88.1 (d, *J* = 170.6 Hz, CH), 81.8 (CH), 74.1 (Cq), 54.8 ($CH_2$), 49.1 (d, *J*= 5.7 Hz, 2 x $CH_2$), 31.3 (d, *J* = 19.7 Hz, 2 x $CH_2$). **HRMS (+ESI):** calculée pour $C_{12}H_{14}FNO$ ($M+H^+$) : 208.11321, trouvée : 208.11327.

**Exemple 47.2 Synthèse du 2-((5-((4-fluoropiperidin-1-yl)methyl)furan-2-yl)ethynyl)-1*H*-pyrrolo[2,3-*b*]pyridine 128**

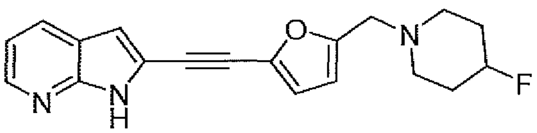

$C_{19}H_{18}FN_3O$
M W: 323,37 gmol-1

Le composé a été synthétisé selon la procédure **C2** et purifié par colonne de chromatographie flash sur gel de silice (méthanol/dichlorométhane/ $NH_4OH$ = 4/94/2). Solide jaunâtre (77%). **Pf** : 193°C. $\mathbf{R_f}$ = 0.60 (méthanol/dichlorométhane/ $NH_4OH$ = 4/94/2). **Infrarouge** (ν, $cm^{-1}$, neat) 3122, 3053, 2937, 2816, 1584, 1434, 1405, 1352, 1325, 1300, 1132, 1021, 975, 918, 820, 768. **RMN $^1$H** (250 MHz, $CDCl_3$, 20°C) δ 10.97 (s, 1H), 8.42 (s, 1H), 7.95 (d, *J* = 7.9 Hz, 1H), 7.13 (dd, *J* = 7.8, 4.5 Hz, 1H), 6.81 (s, 1H), 6.72 (d, *J* = 3.4 Hz, 1H), 6.31 (d, *J* = 3.3 Hz, 1H), 4.84-4.47 (m, 1H), 3.63 (s, 2H), 2.60 (ddt, *J* = 59.6, 11.5, 5.6 Hz, 4H), 1.96 (ddt, *J* = 24.6, 10.8, 4.7 Hz, 4H). **RMN $^{13}$C** (63 MHz, $CDCl_3$, 20°C) δ 154.1(Cq), 148.4 (Cq), 144.3 (CH), 136.0 (Cq), 129.2 (CH), 120.4 (Cq), 118.3 (Cq), 117.1 (CH), 116.6 (CH), 110.2 (CH), 107.1 (CH), 88.9 (d, *J* = 170.6 Hz, CH), 85.72 (Cq), 83.7 (Cq), 54.9 ($CH_2$), 49.1 (d, *J* = 5.5 Hz, 2x $CH_2$), 31.3 (d, *J* = 19.7 Hz, 2x $CH_2$). **RMN $^{19}$F** (235 MHz, $CDCl_3$, 20°C) δ -136,58 (m, 1F). .**HRMS (+ESI)** calculée pour $C_{19}H_{18}FN_3O$ ($M+H^+$): 340.1278, trouvée: 340.1273.

**Exemple 48. 1-(5-((5-fluoro-1H-pyrrolo[2,3-b]pyridin-2-yl)ethynyl)furan-2-yl)-*N,N*-dimethylmethanamine 131**

Le 1-(5-((5-fluoro-1*H*-pyrrolo[2,3-*b*]pyridin-2-yl)ethynyl)furan-2-yl)-*N,N*-dimethylmethanamine **131** a été synthétisé en 4 étapes. Dans un premier temps, l'alcyne correspondant **132** a été préparé en 3 étapes à partir du composé **122** et puis nous avons effectué la réaction de Sonogashira avec l'azaindole pour obtenir le composé final.

**Exemple 48.1 Synthèse du ((5-ethynylfuran-2-yl)methyl)dimethylamine 132**

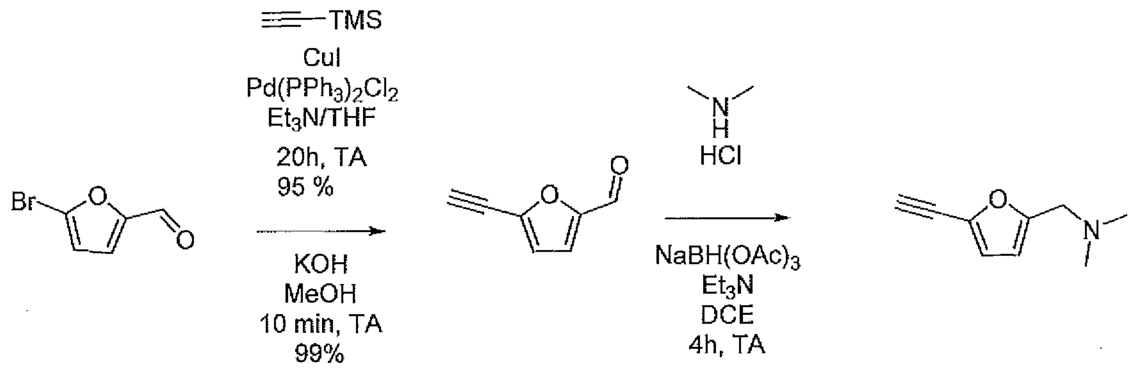


122 123 131

Synthèse du 1-(5-ethynylfuran-2-yl)-*N,N*-dimethylmethanamine **131**

Le composé a été préparé selon la **procédure H** et purifié par colonne de chromatographie flash sur gel de silice (méthanol/dichlorométhane = 5/95). Huile marron (79 %). $\mathbf{R_f}$= 0.58 (méthanol/dichlorométhane = 10/90). **Infrarouge** (v, $cm^{-1}$, neat) : 3292, 2945,2822, 2101, 1602, 1571, 1495, 1326, 1281, 1134, 1017, 992, 972, 936, 841, 792.. **RMN $^1$H** (400 MHz, $CDCl_3$, 20°C) δ 6.55 (d, *J* = 3.3 Hz, 1H), 6.16 (d, *J* = 3.3 Hz, 1H), 3.50 (s, 2H), 3.35 (s, 1H, CH), 2.24 (d, *J* = 4.4 Hz, 6H). **RMN $^{13}$C** (101 MHz, $CDCl_3$, 20°C) : δ 153.9 (Cq), 135.8 (Cq), 117.1 (CH), 109.7 (CH), 81.9 (Cq), 74.2 (CH), 55.7 ($CH_2$), 44.9 (3 x $CH_3$). **HRMS (+ESI)** calculée pour $C_9H_{11}NO$ (M+$H^+$): 150.0913, trouvée: 150.0915.

**Exemple 48.2 1-(5-((5-fluoro-1*H*-pyrrolo[2,3-*b*]pyridin-2-yl)ethynyl)furan-2-yl)-N,N-dimethylmethanamine 131**

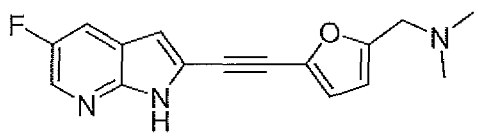

$C_{16}H_{14}FN_3O$
M W: 283,31 gmol-1

Le composé a été synthétisé selon la procédure C2 et purifié par colonne de chromatographie flash sur gel de silice (méthanol/dichlorométhane/ $NH_4OH$ = 4/94/2). Solide jaunâtre (14 %). $\mathbf{R_f}$=0.43 (méthanol/dichlorométhane/ $NH_4OH$ = 4/94/2). **Pf :** 144 °C. **Infrarouge** (v, $cm^{-1}$, neat) 2980, 2761, 2202, 1585, 1403, 1358, 1296, 1258, 1223, 1144, 1022, 980, 845, 769, 513. **RMN $^1$H** (400 MHz, DMSO-$d_6$, 20°C) δ 12.45 (s, 1H), 8.30 (s, 1H), 7.87 (d, *J* = 8.9 Hz, 1H), 7.02 - 6.79 (m, 2H), 6.45 (d, *J* = 3.8 Hz, 1H), 3.48 (s, 2H), 2.17 (s, 6H). **RMN $^{13}$C** (101 MHz, DMSO-$d_6$, 20°C) δ 155.9 (d, *J* = 249.9 Hz, Cq), 155.8 (Cq), 145.7 (Cq), 134.9 (Cq), 133.7 (d, *J* = 29.6 Hz, CH), 120.7 (Cq), 119.9 (d, *J* = 7.5 Hz, Cq), 118.4 (CH), 114.44 (d, *J* = 21.2 Hz, CH), 110.8 (CH), 107.4 (d, *J* = 4.6 Hz, CH), 86.3 (Cq), 83.9 (Cq), 55.40 ($CH_2$), 45.0 (2 x $CH_3$). **RMN $^{19}$F** (376 MHz, DMSO-$d_6$, 20°C) δ. -138.2. **HRMS** (+ESI): calculée pour $C_{16}H_{14}FN_3O$ (284.119367 g/mol), trouvée (284.119244 g/mol).

**Exemple 49.1-(5-((5-fluoro-1*H*-pyrrolo[2,3-*b*]pyridin-2-yl)ethynyl)thiophen-2-yl)-*N,N*-dimethylmethanamine 133**

Le 1-(5-((5-fluoro-1*H*-pyrrolo[2,3-*b*]pyridin-2-yl)ethynyl)thiophen-2-yl)-*N,N*-dimethylmethanamine **133** a été synthétisé en 4 étapes. Dans un premier temps, l'alcyne correspondant **134** a été préparé en 3 étapes à partir du composé **126** et puis nous avons effectué la réaction de Sonogashira avec l'azaindole pour obtenir le composé final.

**Exemple 49.1 Synthèse du 1-(5-ethynylthiophen-2-yl)-*N,N*-dimethylmethanamine 134**

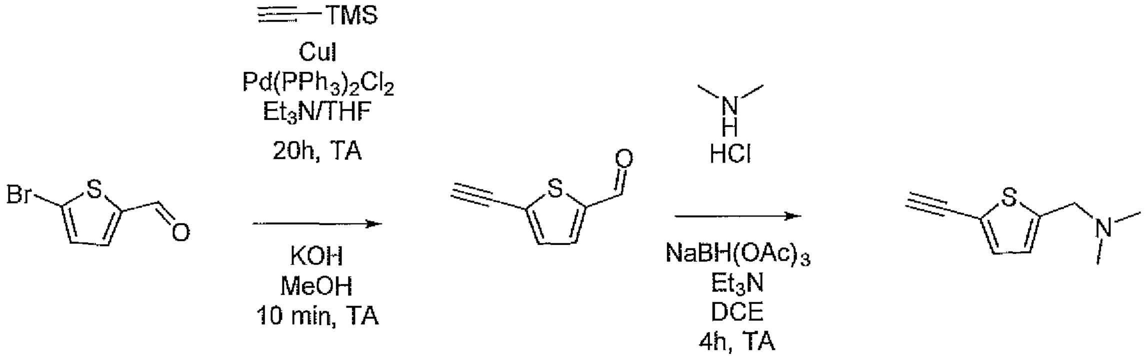


**126** **127** **134**

Synthèse du 1-(5-ethynylthiophen-2-yl)-N,N-dimethylmethanamine **134**

Le composé a été préparé selon la **procédure H** et purifié par colonne de chromatographie flash sur gel de silice (méthanol/dichlorométhane = 5/95). Huile marron (74%). $\mathbf{R_f}$ = 0.58 (méthanol/dichlorométhane = 10/90). **RMN $^1$H** (250 MHz, $CDCl_3$, 20°C) δ 6.68 (m, 1H), 6.55 (m, 1H), 3.60 (s, 2H) 3.26 (s, 1H), 2.14-2.24 (m, 6H). Numéro reaxys: 10655856.

**Exemple 49.2 1-(5-((5-fluoro-1*H*-pyrrolo[2,3-*b*]pyridin-2-yl)ethyn-yl)thiophen-2-yl)-*N,N*-dimethylmethanamine 133**

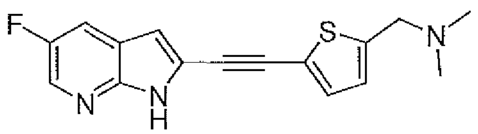

$C_{16}H_{14}FN_3S$
M W: 299,36 gmol-1

Le composé a été synthétisé selon la procédure **C2** et purifié par colonne de chromatographie flash sur gel de silice (méthanol/dichlorométhane/$NH_4OH$ = 4/94/2). Solide jaunâtre (14.5 %). **Pf** : 162 °C $\mathbf{R_f}$ = 0.72 (méthanol/dichlorométhane/ $NH_4OH$ = 4/94/2).**Infrarouge** (v, cm$^{-1}$, neat) 2980, 2761, 2202, 1585, 1503, 1403, 358, 1296, 1258, 1223, 1022, 980, 845, 769, 513. **RMN $^1$H** (250 MHz, $CDCl_3$, 20°C) δ 10.27 (d, *J* = 6.4 Hz, 1H), 8.15-8.30 (m, 1H), 7.57 (dd, *J* = 8.7, 2.8 Hz, 1H), 7.19 (d, *J* = 3.7 Hz, 1H), 6.82 (d, *J* = 3.8 Hz, 1H), 6.70 (d, *J* = 2.1 Hz, 1H), 3.63 (s, 2H), 2.29 (d, *J* = 3.4 Hz, 6H). **RMN $^{13}$C** (63 MHz, $CDCl_3$ 20°C) 155.9 (d, *J* = 249.9 Hz, Cq), 146.1 (Cq), 145.1 (Cq), 133.0 (Cq), 132.8 (CH), 125.9 (CH), 121.7 - 119.6 (m, 2 x Cq et CH), 114.2 (d, *J* = 20.6 Hz, CH), 106.5 (d, *J* = 4.5 Hz, CH), 87.8 (Cq), 84.4 (Cq), 58.5 ($CH_2$), 45.1 (2 x $CH_3$). **RMN $^{19}$F** (235 MHz, $CDCl_3$, 20°C) δ -185,73. **HRMS (+ESI)** calculée pour $C_{16}H_{14}FN_3S$ ($M+H^+$): 300.09652, trouvée: 300.09653.

**Exemple 50. Procédure L : (Désylilation sur les alcynes)**

L'alcyne silylé (1.0 éq.) est mis en réaction avec $K_2CO_3$ (1.5 éq.) dans le méthanol (0.03M). Au bout de 3h à température ambiante, le solvant est évaporé sous pression réduite. Le brut est solubilisé dans 10 mL d'acétate d'éthyle. Puis la phase organique est lavée avec de la saumure (3 X 10 mL). Ensuite la phase organique est séchée avec $MgSO_4$, filtrée sur coton et évaporée sous pression réduite. Pour finir, le brut est purifié par colonne de chromatographie flash sur gel de silice avec comme éluant (acétate d'éthyle/éther de pétrole) pour conduire à l'alcyne vrai.

**Exemple 51. Procédure M : (Méthylation)**

Le dérivé alcool (1.0 éq.) est solubilisé dans du THF. Du NaH (3.5 éq.) et du $CH_3I$ (1.7 éq.) sont ajoutés à 0°C. Au bout de 2 h à température ambiante le mélange est remis à 0°C puis une solution saturée de $NH_4Cl$ est ajoutée. Le mélange est extrait au dichlorométhane (3 x 10 mL). Les phases organiques sont réunies, séchées sur $MgSO_4$, filtrées sur coton puis évaporées à sec.

**Exemple 52 : 2-((4'-((5-fluoro-1*H*-pyrrolo[2,3-*b*]pyridin-2-yl)ethynyl)-[1,1'-biphenyl]-4-yl)(methyl)amino)ethan-1-ol 134 :**

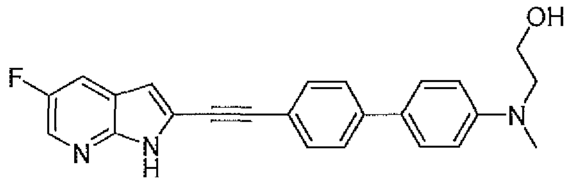

$C_{24}H_{20}FN_3O$
M W: 385,44 gmol-1

2-((4'-((5-fluoro-1*H*-pyrrolo[2,3-*b*]pyridin-2-yl)ethynyl)-[1,1'-biphenyl]-4-yl)(methyl)amino)ethan-1-ol

Le 2-((4'-((5-fluoro-1*H*-pyrrolo[2,3-*b*]pyridin-2-yl)éthynyl)-[1,1'-biphényl]-4-yl)(méthyl) amino)éthan-1-ol a été synthétisé en 5 étapes. Dans un premier temps, l'alcyne correspondant a été préparé en 3 étapes puis engagé dans une réaction de Sonogashira avec l'azaindole et pour finir, une réaction de déprotection permet d'obtenir le composé final.

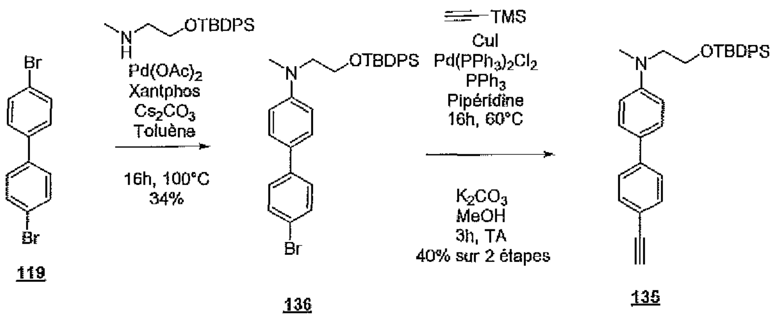


119 136 135

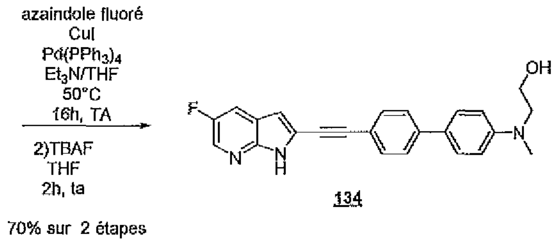


134

**Exemple 52.1** Synthèse de la 4'-bromo-N-(2-((tert-butyldrphenylsilyl)oxy)ethyl)-N-methyl-[1,1'-biphenyl]-4-amine **136** :

Sous argon, le $Pd(OAc)_2$ (0.05 éq.) et du Xantphos (0.067 mmol) sont ajoutés à une solution de toluène anhydre dégazée de concentration 0.3M contenant l'amine (1.0 éq.), le bromé **119** (2.0 éq.) et du $Cs_2CO_3$ (3.0 éq.). Au bout de 16h à 100°C, le solvant est évaporé sous pression réduite. Ensuite, le mélange réactionnel est concentré sous pression réduite, avant d'être purifié par colonne de chromatographie flash sur gel de silice pour conduire au bromé **136** sous forme d'un solide amorphe blanc avec un rendement de 34%, **Rf** = 0.20 (acétate d'éthyle/éther de pétrole = 1/99), **Infrarouge** (v, $cm^{-1}$, neat) 3284, 3070, 2929, 2856, 1610, 1513, 1495, 1471, 1427, 1360, 1280, 1234, 1160, 1103, 985, 927, 869. **RMN $^1$H** (400 MHz, $CDCl_3$, 20°C) $\delta$ 7.65 (d, *J* = 7.2 Hz, 4H), 7.50 (d, *J* = 8.5 Hz, 2H), 7.45 - 7.32 (m, 10H), 6.62 (d, *J* = 8.5 Hz, 2H), 3.83 (t, *J* = 6.2 Hz, 2H), 3.52 (t, *J* = 6.2 Hz, 2H), 2.99 (s, 3H), 1.05 (s, 9H). **RMN $^{13}$C** (101 MHz, $CDCl_3$, 20°C) $\delta$ 148.6 (Cq), 140.0 (Cq), 135.4 (4 x CH), 133.3 (2 x Cq), 131.5 (2 x CH), 129.5 (2 x CH), 127.5 (6 x CH), 127.3 (2 x CH), 127.0 (Cq), 119.6 (Cq), 111.9 (2 x CH), 60.9 ($CH_2$), 54.3 ($CH_2$), 39.0 ($CH_3$), 26.7 (3 x $CH_3$), 18.9 (Cq). **HRMS** (+ESI) calculée pour $C_{31}H_{34}BrNOSi$ ($M+H^+$): 544.1665, trouvée: 544.1663.

**Exemple 52.2** Synthèse du *N*-(2-((tert-butyldiphenylsilyl)oxy)ethyl)-4'-ethynyl-N-methyl-[1,1'-biphonyl]-4-amine **135** :

L'alcyne **135** a été préparé selon la **procédure I1** et à une température de 60°C et purifié par colonne de chromatographie flash sur gel de silice (acétate d'éthyle/éther de pétrole = 2/48). Huile jaune (40% sur les deux étapes), $\boldsymbol{R_f}$ = 0.20 (acétate d'éthyle/éther de pétrole = 2/48). **Infrarouge** (v, $cm^{-1}$, neat) 3277, 2928, 2855, 1609, 1599, 1532,

1493, 1471, 1426, 1371, 1357, 1292, 1240, 1210, 1194, 1134, 1104, 1087, 1006, 996, 982. **RMN $^1$H** (400 MHz, $CDCl_3$, 20°C) δ 7.65 (d, *J* = 7.2 Hz, 4H), 7.50 (s1, 2H), 7.45 - 7.32 (m, 8H), 6.62 (d, *J* = 8.5 Hz, 2H), 3.83 (t, *J* = 6.2 Hz, 2H), 3.52 (t, *J* = 6.2 Hz, 2H), 3.10 (s, 1H), 2.99 (s, 3H), 1.05 (s, 9H). **RMN** $^{13}$C (101 MHz, $CDCl_3$, 20°C) δ 148.6 (Cq), 140.0 (Cq), 135.6 (4 x CH), 133.4 (2 x Cq), 132.5 (2 x CH), 129.7 (2 x CH), 127.7 (4 x CH), 127.6 (2 x CH), 127.4 (Cq), 125.8 (2 x CH), 119.6 (Cq), 112.0 (2 x CH), 84.0 (Cq), 77.1 (CH), 61.0 ($CH_2$), 54.4 ($CH_2$), 39.1 ($CH_3$), 26.8 (3 x $CH_3$), 19.1 (Cq). **HRMS** (+ESI) calculée pour $C_{33}H_{35}NOSi$ ($M+H^+$): 490.2560, trouvée: 490.2552.

**Exemple 52.3** Préparation du 2-((4'-((5-fluoro-1*H*-pyrrolo[2,3-*b*]pyridin-2-yl)ethynyl)-[1,1'-biphenyl]-4-yl)(mathyl)amino)ethan-1-ol **134** :

Le composé a été préparé selon la **procédure C1 suivie de la procédure F** et purifié par colonne de chromatographie flash sur gel de silice (méthanol/dichlorométhane = 2/48). Solide jaunâtre (70% sur 2 étapes) **Pf** 172-174°C, **Rf** = 0.20 (méthanol/dichlorométhane = 2/48). **Infrarouge** (υ, $cm^{-1}$, neat) 3122, 2925, 1662, 1610, 1510, 1494, 1436, 1399, 1349, 1296, 1246, 1165, 1120, 1048, 984, 872, 839. **RMN $^1$H** (400 MHz, DMSO-$d_6$, 20°C) δ 12.38 (s, 1H), 8.28 (s, 1H), 7.86 (dd, *J* = 2 et 9 Hz, 1H), 7.70 (m, 2H), 7.58 (m, 4H), 6.81 (m, 3H), 4.70 (t, *J* = 7.3 Hz, 1H), 3.57 (m, 2H), 3.46 (d, *J* = 7.3 Hz, 2H), 2.99 (s, 3H). **RMN $^{13}$C** (101 MHz, DMSO-$d_6$ 20°C) δ 155.3 (d, *J* = 239.2 Hz, Cq), 147.9 (Cq), 145.6 (Cq), 144.9 (Cq), 133.2 (Cq), 132.8 (d, *J* = 20.9 Hz, CH), 132.3 (2 x CH), 127.7 (2 x CH), 125.9 (2 x CH) 125.8 (Cq), 121.9 (Cq), 120.2 (d, *J* = 6.2 Hz, Cq), 118.8 (Cq), 114.0 (d, *J* = 20.2 Hz, CH), 112.5 (2 x CH), 106.6 (d, *J* = 4.0 Hz, CH), 94.2 (Cq), 82.4 (Cq), 58.6 ($CH_2$), 54.5 ($CH_2$), 39.1 ($CH_3$). **RMN $^{19}$F** (376 MHz, DMSO-$d_6$, 20°C) δ. -138.5.

**Exemple 53 : 4'-((1*H*-pyrrolo[2,3-b]pyridin-2-yl)ethynyl)-*N*-(2-fluoroetbyl)-*N*-methyl-[1,1'-biphenyl]-4-amine 137** :

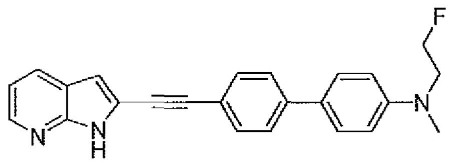

4'-((1*H*-pyrrolo[2,3-*b*]pyridin-2-yl)ethynyl)-*N*-(2-fluoroethyl)-*N*-methyl-[1,1'-biphenyl]-4-amine

Le 4'-((1*H*-pyrrolo[2,3-*b*]pyridin-2-yl)éthynyl)-*N*-(2-fluoroéthyl)-*N*-méthyl-[1,1'-biphényl]-4-amine137 a été synthétisé en 7 étapes. Dans un premier temps, l'alcyne correspondant a été préparé en 3 étapes puis engagé dans une réaction de Sonogashira avec l'azaindole et une réaction de déprotection. Ensuite, une réaction de fluoration avec le DAST suivie d'une réaction de déprotection de l'azaindole permet de conduire au composé attendu.

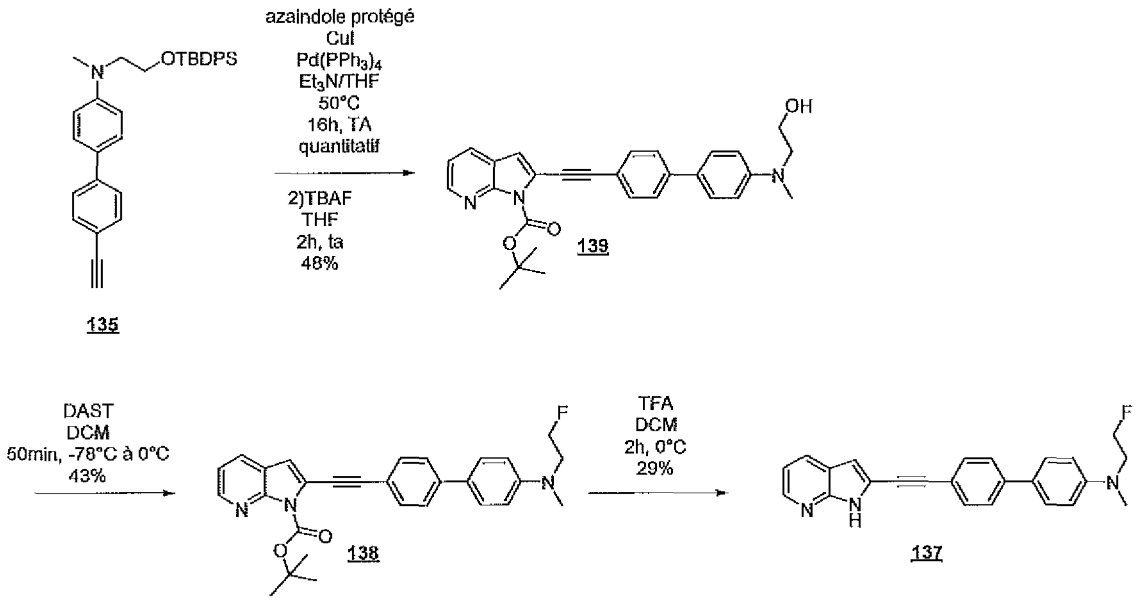

135 139 138 137

**Exemple 53.1** Préparation du tert-butyl 2-((4'-((2-hydroxyethyl)(methyl)amino)-1,1'-biphenyl]-4-yl)ethynyl)-1*H*-pyrrolo[2,3-*b*]pyrdine-1-carboxylate **139** :

Le composé a été préparé selon la **procédure C1 suivie de la procédure F** et purifié par colonne de chromatographie flash sur gel de silice (méthanol/dichlorométhane = 2/48). Solide jaunâtre (48% sur 2 étapes) **Pf** 20b-202°C, **Rf** = 0.20 (méthanol/dichlorométhane = 2/48). **Infrarouge** (υ, $cm^{-1}$, neat) 2979, 2204, 1742, 1605, 1573, 1544, 1517, 1355, 1306, 1253, 1189, 1154, 1115, 1089, 1041, 979, 813, 774, 527, 510. **RMN $^1$H** (400 MHz, $CDCl_3$, 20°C) δ 8.57 (m, 1H), 7.87 (d, *J* = 7.3 Hz, 1H), 7.57 (m, 6H), 7.28 (s, 1H), 7.22 (m, 1H), 6.89 (m, 3H), 3.88 (m, 2H), 3.57 (t, *J* = 5.7 Hz, 2H), 3.07 (s, 3H), 1.73 (s, 9H). **RMN $^{13}$C** (101 MHz, $CDCl_3$, 20°C) δ 149.6 (Cq), 148.6 (Cq), 147.9 (Cq), 146.1 (CH), 141.4 (Cq), 131.9 (2 x CH), 128.6 (CH), 128.4 (Cq), 127.8 (2 x CH), 126.1 (2 x CH), 121.4 (Cq), 121.3 (Cq), 120.0 (Cq), 118.9 (CH), 113.0 (2 x CH), 112.5 (CH), 105.3 (Cq), 95.9 (Cq), 84.8 (Cq), 81.9 (Cq), 60.2 ($CH_2$), 55.1 ($CH_2$), 38.9 ($CH_3$), 28.2 (3 x $CH_3$). **HRMS** (+ESI) calculée pour $C_{29}H_{29}N_3O_3$ ($M+H^+$) : 468.2281, trouvée: 468.2287.

**Exemple 53.2** Préparation du 4'-((1*H*-pyrrolo[2,3-*b*]pyridin-2-yl)ethynyl)-*N*-(2-fluoroethyl)-*N*-methyl-[1,1'-biphenyl]-4-amine **138** :

A une solution contenant du dérivé **139** (0.035 g, 0.075 mmol, 1.0 éq.) dans le dichlorométhane (5 mL) et refroidie à -78°C, est additionné du DAST (0.012 mL, 0.089 mmol, 1.2 éq.) goutte-à-goutte. Ensuite, le milieu réactionnel est mis à 0°C et au bout de 50 minutes 10 mL d'$H_2O$ est ajouté. La phase aqueuse est extraite par du dichlorométhane (2 fois 10 mL), les phases organiques réunies sont séchées sur $MgSO_4$ , filtrées et concentrées sous pression réduite. Le composé est purifié par colonne de chromatographie flash sur gel de silice (méthanol/dichlorométhane = 2/98). Solide blanc (22%), **Pf** >260°C, $\boldsymbol{R_f}$ = 0.8 (méthanol/dichlorométhane = 4/96). **Infrarouge** (v, $cm^{-1}$, neat) 2924, 1740, 1629, 1597, 1495, 1473, 156, 1400, 1359, 1337, 1301, 1250, 1209, 1153, 1140, 1108, 1086, 1043, 976, 906, 873. **RMN $^1$H** (250 MHz, $CDCl_3$, 20°C) δ 8.52 (dd, *J* = 4.8, 1.6 Hz, 1H), 7.82 (dd, *J* = 7.9, 1.6 Hz, 1H), 7.51 (d, *J* = 8.8 Hz, 6H), 7.18 (dd, *J* = 7.9, 4.8 Hz, 3H), 6.86 (s, 1H), 6.78 (d, *J* = 8.8 Hz, 2H), 4.62 (dt, *J* = 47.1, 5.2 Hz, 2H), 3.69 (dt, *J* = 24.3, 5.2 Hz, 2H), 3.06 (s, 3H), 1.68 (s, H). **RMN $^{13}$C** (63 MHz, $CDCl_3$, 20°C) δ 148.65 (Cq), 147.48 (Cq), 146.1 (CH), 131.9 (2 x CH), 128.6 (CH), 128.31 (Cq), 128.18 (Cq), 127.8 (2 x CH), 126.0 (2 x CH), 121.43 (Cq), 121.31 (Cq), 120.0 (Cq), 118.9 (CH), 112.6 (CH), 112.5 (2 x CH), 96.1 (Cq), 84.8 (Cq), 81.8 (d, *J* = 170.3 Hz, $CH_2$), 80.0 (Cq), 52.6 (d, *J* = 21.2 Hz, $CH_2$), 39.1 ($CH_3$), 28.2 (3 x $CH_3$). **$^{19}$F NMR** (235 MHz, $CDCl_3$, 20°C) δ -222.2. **HRMS (+ESI)** calculée pour $C_{23}H_{25}FN_3O_2$ ($M+H^+$) : 394.1925, trouvée: 394.1925.

**Exemple 53.3** Préparation du 4'-((1*H*-perolo[2,3-*b*]pyridin-2-yl)ethynyl)-*N*-(2-fluoroethyl)-*N*-methyl-[1,1'-biphenyl]-4-amine **137** :

Le composé **137** a été préparé selon la **procédure F** et purifié par colonne de chromatographie flash sur gel de silice (acétate d'éthyle/éther de pétrole = 20/80). Solide jaune (29%), **Pf** >260°C, $\boldsymbol{R_f}$ = 0.28 (acétate d'éthyle/éther de pétrole = 20/80). **Infrarouge** (v, $cm^{-1}$, neat) 2882, 2357, 1607, 1595, 1522, 1491, 1431, 1403, 1372, 1355, 1324, 1280, 1247, 1203, 1134, 1112, 1038, 977, 916. **RMN $^1$H** (400 MHz, DMSO-$d_6$, 20°C) δ 12.21 (s, 1H), 8.29 (s, 1H), 7.97 (d, *J* = 7.9 Hz, 1H), 7.70 (d, *J* = 8.0 Hz, 2H), 7.60 (d, *J* = 8.0 Hz, 3H), 7.11 (dd, *J* = 7.9, 4.7 Hz, 1H), 6.84 (m, 3H), 4.62 (d, *J* = 47.7, 4.7 Hz, 2H), 3.72 (d, *J* = 26.4, 4.7 Hz, 2H), 3.00 (s, 3H). **RMN $^{13}$C** (101 MHz, DMSO-$d_6$ 20°C) δ 13C NMR (101 MHz, DMSO) δ 149.3 (Cq), 148.8 (Cq), 144.8 (CH), 141.2 (Cq), 132.3 (2 x CH), 128.8 (CH), 127,7 (2 x CH), 126.6 (Cq), 126.0 (2 x CH), 125.9 (Cq), 120.0 (Cq), 119.6 (Cq), 119.2 (Cq), 116.8 (CH), 112.9 (2 x CH), 112.5 (Cq), 106.6 (CH), 93.57 (Cq), 82.9 (Cq), 82.8 (d, *J* = 170.3 Hz, $CH_2$), 52.1 (d, *J* = 21.2 Hz, $CH_2$), 38.94 ($CH_3$). **RMN $^{19}$F** (376 MHz, DMSO-$d_6$, 20°C) δ. -221.2. **HRMS** (+ESI) calculée pour $C_{24}H_{20}FN_3$ ($M+H^+$) : 370.1714, trouvée: 370.1707.

**Exemple 54 : 3-((1*H*-pyrrolo[2,3-*b*]pyridin-2-yl)ethynyl)-N-(2-fluoroethyl)-N-methylaniline 140 :**

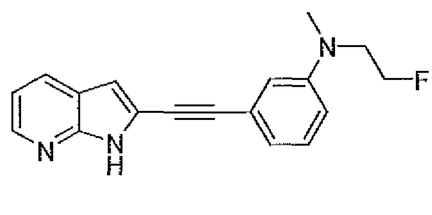

3-((1*H*-pyrrolo[2,3-*b*]pyridin-2-yl)ethynyl)-*N*-(2-fluoroethyl)-*N*-methylanilene

Le 3-(1*H*-pyrrolo[2,3-*b*]pyridin-2-yl)éthynyl)-*N*-(2-fluoroéthyl)-*N*-méthiylaniline a été synthétisé en 4 étapes à

partir du dibromobenzene. Dans un premier temps, l'alcyne correspondant **142** a été préparé en 3 étapes puis la réaction de Sonogashira avec l'azaindole protégé permet d'obtenir l'intermédiaire **143.** Ensuite, la fonction alcool est libérée à l'aide TBAF et pour finir le composé **140** est obtenu après 3 réactions successives (tosylation, SN et déprotection).

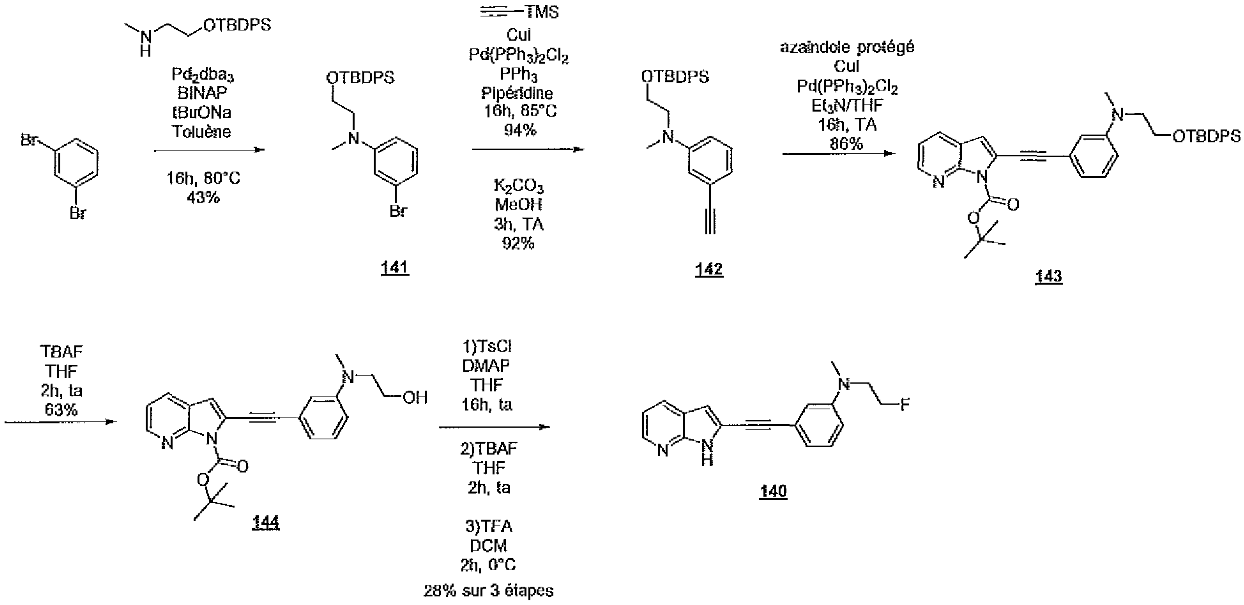


141 142 143 144 140

**Exemple 54.1** Synthèse de la 3-bromo-*N*-(2-((*tert*-butyldiphenylsilyl)oxy)ethyl)-*N*-methylaniline **141** :

Le composé **141** a été préparé selon la **procédure H** et purifié par colonne de chromatographie flash sur gel de silice (acétate d'éthyle/éther de pétrole = 1/49). Huile incolore (43%). **Infrarouge** (ν, $cm^{-1}$, neat) 3370, 2929, 2856, 1592, 1496, 1471, 1426, 1370, 1239, 1210, 1189, 1104, 926. **RMN $^1$H** (250 MHz, $CDCl_3$, 20°C) δ 7.60 (d, *J* = 6.5 Hz, 4H), 7.50 - 7.26 (m, 6H), 6.95 (t, *J* = 8.1 Hz, 1H), 6.73 (m, 2H), 6.44 (d, *J* = 7.3 Hz, 1H), 3.76 (t, *J* = 6.0 Hz, 2H), 3.43 (t, *J* = 6.0 Hz, 2H), 2.88 (s, 3H), 1.01 (s, 9H). **RMN $^{13}$C** (63 MHz, $CDCl_3$, 20°C) δ 150.4 (Cq), 135.6 (4 x CH), 133.3 (2 x Cq), 130.2 (CH), 129.7 (CH), 127.7 (5 x CH), 123.41 (Cq), 118.6 (CH), 114.2 (CH), 110.4 (CH), 61.1 ($CH_2$), 54.4 ($CH_2$), 39.0 ($CH_3$), 26.79 (2 x $CH_3$), 19.06 (Cq).. **HRMS** (+ESI) calculée pour $C_{25}H_{30}BrNOSi$ ($M+H^+$) : 468.1352, trouvée: 468.1356.

**Exemple 54.2** Synthèse de la *N*-(2-((*tert*-butyldiphenylsilyl)ox)ethyl)-3-ethynyl-*N*-methylaniline **142** :

L'alcyne **142** a été préparé selon la **procédure I1** et purifié par colonne de chromatographie flash sur gel de silice (acétate d'éthyle/éther de pétrole = 2/48). Huile incolore (86% sur les deux étapes). **Infrarouge** (ν, $cm^{-1}$, neat) 3288, 2929, 2856, 1594, 1570, 1494, 1471, 1427, 1360, 1105, 1003, 822. **RMN $^1$H** (250 MHz, $CDCl_3$, 20°C) δ 7.62 (d, *J*= 6.5 Hz, 4H), 7.50 - 7.26 (m, 6H), 7.08 (t, *J* = 8.1 Hz, 1H), 6.78 (m, 2H), 6.54 (d, *J* = 7.3 Hz, 1H), 3.79 (t, *J* = 6.0 Hz, 2H), 3.47 (t, *J* = 6.0 Hz, 2H), 2.97 (s, 1H), 2.90 (s, 3H), 1.01 (s, 9H). **RMN $^{13}$C** (63 MHz, $CDCl_3$, 20°C) δ 150.4 (Cq), 135.6 (4 x CH), 133.4 (2 x Cq), 129.9 (CH), 129.0 (CH), 127.7 (5 x CH), 122.4 (Cq), 119.8 (CH), 115.2 (CH), 112.6 (CH), 84.9 (Cq) 75.8 (CH), 61.1 ($CH_2$), 54.4 ($CH_2$), 39.0 ($CH_3$), 26.79 (2 x $CH_3$), 19.06 (Cq).. **HRMS** (+ESI) calculée pour $C_{25}H_{30}BrNOSi$ ($M+H^+$) : 468.1352, trouvée: 468.1356.

**Exemple 54.3** Préparation du *tert*-butyl 2-((3-((2-((*tert*-butyldiphenylsilyl)oxy)ethyl)(methyl)amino) phenyl)ethynyl)-1H-pyrrolo[2,3-*b*]pyridine-1-carboxylate **143** :

Le composé **137** a été préparé selon la **procédure C2** et purifié par colonne de chromatographie flash sur gel de silice (acétate d'éthyle/éther de pétrole = 20/80). Huile jaune (86%), ***R*$_f$** = 0.20 (acétate d'éthyle/éther de pétrole = 20/80). **Infrarouge** (ν, $cm^{-1}$, neat) 2986, 2360, 2198, 1749, 1605, 1544, 1514, 1407, 1348, 1306, 1253, 1187, 1170, 1114, 1092, 1010, 972, 900, 804, 776, 663, 556, 508 . **RMN** $^1$H (400 MHz, $CDCl_3$, 20°C) δ 8.57 (d, *J* = 3.4 Hz, 1H), 7.86 (d, *J* = 8.1 Hz, 1H), 7.66 (d, *J* = 7.8 Hz, 4H), 7.39 (m, 6H), 7.19 (m, 2H), 6.87 (m, 3H), 6.62 (d, *J* = 7.8 Hz, 1H), 3.84 (t, *J* = 5.9 Hz, 2H), 3.54 (t, *J* = 5.9 Hz, 2H), 2.99 (s, 3H), 1.67 (s, 9H), 1.06 (s, 9H). **RMN $^{13}$C** (101 MHz, $CDCl_3$, 20°C) δ 148.9 (Cq), 148.6 (Cq), 147.9 (Cq), 146.1 (CH), 135.6 (4 x CH), 133.4 (Cq), 129.7 (CH), 129.2 (CH), 128.6 (CH), 127.7 (5 x CH), 123.0 (Cq), 121.4 (Cq), 119.2 (CH), 118.9 (CH), 114.5 (CH), 112.7 (CH), 112.4 (CH), 96.8 (Cq), 84.7 (Cq),

80.4 (Cq), 61.2 ($CH_2$), 54.4 ($CH_2$), 39.1 ($CH_3$), 28.2 (3 x $CH_3$), 26.8 (3 x $CH_3$), 19.1 (Cq). **HRMS** (+ESI) calculée pour $C_{39}H_{43}N_3O_3Si$ (M+H$^+$) : 630.3146, trouvée: 630.3136

**Exemple 54.4** Préparation du *tert*-butyl 2-((3-((2-hydroxyethyl)(methyl)amino)phenyl)ethynyl)-1*H*-pyrrolo[2,3-*b*]pyridine-1-carboxylate **144** :

Le composé **144** a été préparé selon la **procédure J** et purifié par colonne de chromatographie flash sur gel de silice (méthanol/dichlorométhane = 4/96). Huile jaune (63%), $R_f$ = 0.20 (méthanol/dichlorométhane = 4/96). **Infrarouge** (v, cm$^{-1}$, neat) 3386, 2980, 2926, 2206, 1747, 1603, 1546, 1517, 1473, 1408, 1384, 1343, 1305, 1249, 1194, 1154, 1117, 1085, 980, 847, 831, 814, 776, 746, 643, 525. **RMN $^1$H** (400 MHz, $CDCl_3$, 20°C) δ 8.54 (d, *J*=4.3 Hz, 1H), 7.82 (s, 1H), 7.25 - 7.16 (m, 2H), 6.99 - 6.77 (m, 4H), 3.84 (t, *J*= 5.7 Hz, 2H), 3.50 (t, *J*= 5.7 Hz, 2H), 3.00 (s, 3H), 1.69 (s, 9H). **RMN $^{13}$C** (101 MHz, $CDCl_3$, 20°C) δ 148.8 (Cq), 148.6 (Cq), 147.9 (Cq), 146.1 (CH), 129.3 (CH), 128.6 (CH), 121.4 (Cq), 121.2 (Cq), 120.2 (CH), 118.9 (CH), 115.4 (CH), 113.9 (CH), 112.9 (CH), 112.4 (CH), 96.4 (Cq), 84.7 (Cq), 80.7 (Cq), 60.1 ($CH_2$), 55.2 ($CH_2$), 38.9 ($CH_3$), 28.2 (3 x $CH_3$). **HRMS** (+ESI) calculée pour $C_{23}H_{25}N_3O_3$ (M+H$^+$): 392.1968, trouvée: 392.1967.

**Exemple 54.5** Préparation de la 3-((1*H*-pyrrolo[2,3-*b*]pyridin-2-yl)ethynyl)-*N*-(2-fluoroethyl)-*N*-methylaniline 140 :

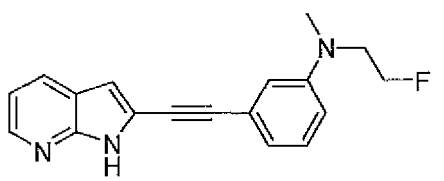

$C_{18}H_{16}FN_3$
M W: 293,35 gmol-1

3-((1*H*-pyrrolo[2,3-*b*]pyridin-2-yl)ethynyl)-*N*-(2-fluoroethyl)-*N*-methylaniline

A 0°C, TsCl (0.056 g, 0.294 mmol, 1.2 éq.), la triéthylamine (0.05 mL, 0.36 mmol, 1.5 éq.) et la 4-DMAP (0.003 g, 0.024 mmol, 0,1 éq.) ont été ajoutée à une solution de **144** (0.96 g, 0.245 mmol, 1.0 éq.) dissous dans du DCM (5 mL). Le mélange est agité à température ambiante durant une nuit, puis le mélange est neutralisé avec 10 mL d'eau. Ensuite, la phase aqueuse est extraite avec du dichlorométhane (2 fois 10 mL). Les phases organiques réunies ont été séchées sur $MgSO_4$, filtrées et concentrées sous pression réduite. Le brut est purifié par colonne de chromatographie flash sur gel de silice pour conduire à 73 mg de composé tosylé avec un rendement de 55%. Ensuite, le produit est solubilisé dans 5 mL de THF puis l'addition de 0.94 mL de TBAF (C = 1M, 0.94 mmol, 7.0 éq.) est réalisée goutte à goutte. Au bout de 24h à TA, 10 mL de $H_2O$ sont ajoutés et la phase aqueuse est extraite avec du dichlorométhane (2 fois 10 mL). Les phases organiques réunies ont été séchées sur $MgSO_4$, filtéres et concentrées sous pression réduite. Pour finir, le brut est solubilisé dans du dichlorométhane (0.05 M). A 0°C, du TFA est additionné goutte-à-goutte (rapport TFA/dichlorométhane = 1/2). L'agitation à 0°C est effectuée avec un suivi CCM. A la fin de la réaction, le mélange réactionnel est évaporé sous pression réduite. Le brut est repris dans un mélange DCM/NaOH (1M). Sous agitation, la phase aqueuse est extraite avec du dichlorométhane (2 fois 10 mL). Les phases organiques réunies ont été séchées sur $MgSO_4$, filtéres et concentrées sous pression réduite, avant de purifier le brut réactionnel par colonne de chromatographie flash sur gel de silice (méthanol/dichlorométhane = 4/96). Solide jaune (28%), $R_f$ = 0.15 (méthanol/dichlorométhane = 4/96). **Infrarouge** (v, cm$^{-1}$, neat) 3386, 2980, 2926, 2206, 1747, 1603, 1546, 1517, 1473, 1408, 1384, 1343, 1305, 1249, 1194, 1154, 1117, 1085, 980, 847, 831, 814, 776, 746, 643, 525. **RMN $^1$H** (250 MHz, DMSO-$d_6$, 20°C) δ 12.13 (s, 1H), 8.23 (d, *J* = 4.9 Hz, 1H), 7.91 (d, *J* = 7.9 Hz, 1H), 7.20 (t, *J* = 7.9 Hz, 1H), 7.14 - 7.02 (m, 1H), 6.89 - 6.70 (m, 4H), 4.56 (dt, *J* = 47.6, 5.0 Hz, 2H), 3.65 (dt, *J* = 26.4, 5.0 Hz, 2H), 2.93 (s, 3H). **RMN $^{13}$C** (101 MHz, DMSO-$d_6$, 20°C) δ 148.8 (Cq), 148.6 (Cq), 147.9 (Cq), 144.8 (CH), 129.9 (CH), 128.8 (CH), 121.4 (Cq), 121.2 (Cq), 119.5 (CH), 116.8 (CH), 114.6 (CH), 113.7 (CH), 106.5 (CH), 94.5 (Cq), 82.9 (Cq), 82.8 (d, *J* = 170.3 Hz, $CH_2$), 52.1 (d, *J* = 21.2 Hz, $CH_2$), 38.6 ($CH_3$). **RMN $^{19}$F** (376 MHz, DMSO-$d_6$, 20°C) δ -221.03. **HRMS** (+ESI) calculée pour $C_{18}H_{16}FN_3$ (M+H$^+$) : 294.1401, trouvée: 294.1401.

**Exemple 55 : 2-((3-((5-fluoro-1*H*-pyrrolo[2,3-*b*]pyridin-2-yl)ethynyl)phenyl)(methyl) amino)ethan-1-ol 145:**

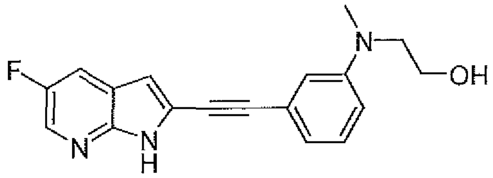

2-((3-((5-fluoro-1*H*-pyrrolo[2,3-*b*]pyridin-2-yl)ethynyl)phenyl)(methyl)amino)ethan-1-ol

Le 2-((3-((5-fluaro-1*H*-pyrrolo[2,3-*b*]pyridin-2-yl)éthynyl)phényl)(méthyl)amino)éthan-1-ol a été synthétisé en 2 étapes à partir de l'alcyne **142.** Dans un premier temps, une réaction de Sonogashira est effectuée entre avec l'azaindole fluoré et alcyne **142.** Pour finir, une réaction de déprotection à l'aide TBAF est réalisée pour conduire au composé final.

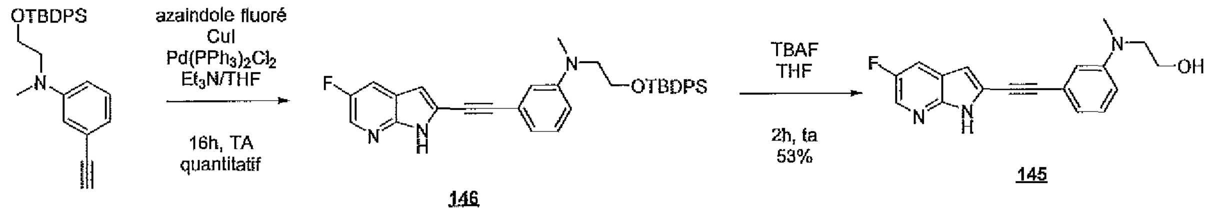


142 146 145

Le composé **145** a été préparé selon la **procédure** C suivie de la **procédure J** et purifié par colonne de chromatographie flash sur gel de silice (méthanol/dichlorométhane = 4/96). Solide jaune (53%), $R_f$ = 0.10 (méthanol/dichlorométhane = 2/98). Pf = 158-160°C. **Infrarouge** (v, $cm^{-1}$, neat) 2861, 2809, 1593, 1567, 1489, 1426, 1398, 1365, 1346, 1295, 1221, 1148, 1123, 1052, 1006, 987, 929, 892. **RMN $^1$H** (400 MHz, DMSO-$d_6$, 20°C) δ 12.35 (s, 1H), 8.27 (s, 1H), 7.85 (m, 1H), 7.27 - 7.18 (m, 1H), 6.83 (m, 4H), 4.70 (s, 1H), 3.61 - 3.52 (m, 2H), 3.46 - 3.38 (m, 2H), 2.96 (s, 3H). **RMN $^{13}$C** (101 MHz, $CDCl_3$, 20°C) δ 154.5 (d, *J* = 239.2 Hz, Cq), 149.6 (Cq), 145.6 (Cq), 132.7 (d, *J* = 29.0 Hz, CH), 129.9 (CH), 122.3 (2 x Cq), 120.1 (d, *J* = 7.2 Hz, Cq), 118.9 (CH), 114.8 (CH), 114.1 (d, *J* = 20.8 Hz, CH), 113.9 (CH), 106.5 (d, *J* = 4.0 Hz, CH), 95.0 (Cq), 81.0 (Cq), 58.5 ($CH_2$), 54.5 ($CH_2$), 39.4 ($CH_3$). **RMN $^{19}$F** (376 MHz, DMSO-$d_6$, 20°C) δ. -138.5, **HRMS** (+ESI) calculée pour $C_{18}H_{16}FN_3O$ (M+H$^+$): 310.1350 ; trouvée: 310.1349.

**Exemple 56 : *N1*-(4-((5-fluoro-1*H*-pyrrolo[2,3-*b*]pyridin-2-yl)ethynyl)phenyl)-*N1*,*N2*,*N2*-trimethylethane-1,2-diamine 147 :**

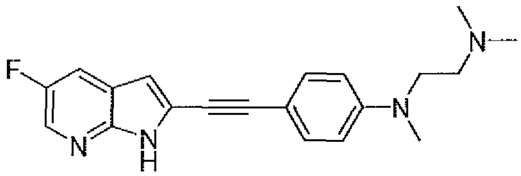

*N*1-(4-((5-fluoro-1*H*-pyrrolo[2,3-*b*]pyridin-2-yl)ethynyl)phenyl)-*N*1,*N*2,*N*2-trimethylethane-1,2-diamine

La *N1*-(4-((5-fluoro-1*H*-pyrrolo[2,3-*b*]pyridin-2-yl)éthynyl)phényl)-*N1*,*N2*,*N2*-triméthyléthane-1,2-diamine **147** a été synthétisée en 4 étapes. Dans un premier temps, l'alcyne **148** a été préparé en 3 étapes à partir dibromobenzène puis la réaction de Sonogashira avec l'azaindole permet d'obtenir le composé final.

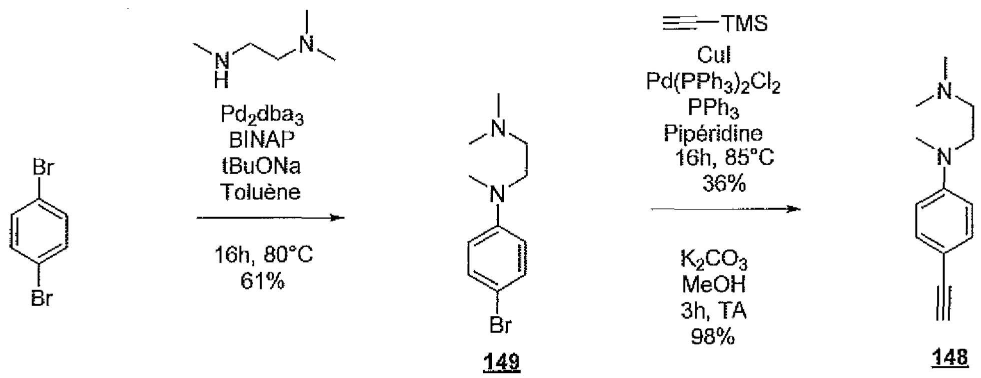


**149** **148**

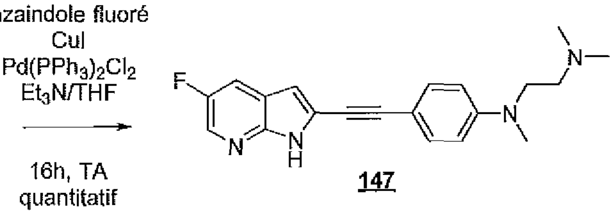


**148** **147**

**Exemple 56.1** Synthèse de la *N$^1$*-(4-bromophenyl)-*N$^1$,N$^2$,N$^2$*-trimethylethane-1,2-diamine **149** :

Le composé **149** a été préparé selon la **procédure H** et purifié par colonne de chromatographie flash sur gel de silice (acétate d'éthyle/éther de pétrole = 1/49). Huile orange (61%). **Infrarouge** (v, cm$^{-1}$, neat) 3291, 2962, 2930, 2853, 2102, 1606, 1511, 1463, 1434, 1373, 1356, 1336, 1273, 1245, 1227, 1185, 1141, 1093, 1023, 935. **RMN $^1$H** (250 MHz, CDCl$_3$ 20°C) δ 7.25 (d, *J* = 8.7 Hz, 2H), 6.54 (d, *J* = 8.7 Hz, 2H), 3.39 (m, 2H), 2.89 (s, 3H), 2.53 - 2.35 (m, 2H), 2.25 (s, 6H). **RMN $^{13}$C** (63 MHz, CDCl$_3$,20°C) δ 148.1 (Cq), 131.8 (2 x CH), 113.7 (2 x CH), 108.0 (Cq), 55.8 (CH$_2$), 51.2 (CH$_2$), 45.9 (2 x CH$_3$), 38.6 (CH$_3$). **HRMS** (+ESI) calculée pour $C_{11}H_{17}BrN_2$ (M+H$^+$) : 257.0647, trouvée: 257.0646.

**Exemple 56.2** Synthèse de la *N$^1$*-(4-ethynylphenyl)-*N$^1$,N$^2$,N$^2$*-trimethylethanecm-1,2-diamine **148**:

L'alcyne **148** a été préparé selon la **procédure I1** et purifié par colonne de chromatographie flash sur gel de silice (acétate d'éthyle/éther de pétrole = 2/48). Huile jaunâtre (35% sur les deux étapes).

**Infrarouge** (v, cm$^{-1}$, neat) 2931, 2819, 2769, 2098, 1606, 1515, 1455, 133, 1324, 1300, 1250, 1215, 1175, 1113. **RMN $^1$H** (400 MHz, CDCl$_3$, 20°C) δ 7.37 (d, *J* = 8.5 Hz, 2H), 6.63 (d, *J* = 8.5 Hz, 2H), 3.39 (m, 2H), 2.90 (s, 4H), 2.55 - 2.41 (m, 2H), 2.31 (s, 6H). **RMN $^{13}$C** (101 MHz, CDCl$_3$, 20°C) δ 149.1 (Cq), 133.4 (2 x CH), 111.4 (2 x CH), 108.6 (Cq), 84.8 (Cq), 74.7 (CH), 55.9 (CH$_2$), 50.9 (CH$_2$), 45.9 (2 x CH$_3$), 38.5 (CH$_3$).

**Exemple 56.3** Synthèse de la *N$^1$*-(4-((5-fluoro-1H-pyrrolo[2,3-*b*]pyridin-2-yl)ethynyl)phenyl)-*N$^1$,N$^2$,N$^2$*-trimethylethanecm-1,2-diamine **147:**

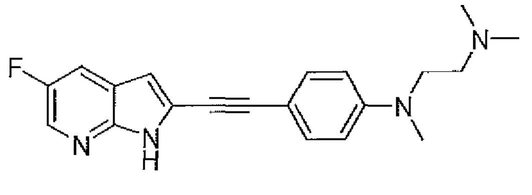

$C_{20}H_{21}FN_4$
M W: 336,41 gmol-1

*N*$^1$-(4-((5-fluoro-1*H*-pyrrolo[2,3-*b*]pyridin-2-yl)ethynyl)phenyl)-*N$^1$,N$^2$,N$^2$*-trimethylethane-1,2-diamine Le composé a été préparé selon la **procédure C2** et purifié par colonne de chromatographie flash sur gel de silice (méthanol/dichlorométhane/ammoniaque = 4/96/1). Solide jaune (80%), **Pf :** 220-222°C, ***R*$_f$** = 0.15 (méthanol/dichlorométhane/ = 4/96). **Infrarouge** (v, cm$^{-1}$, neat) 2775, 2360, 2342, 1738, 1604, 1583, 1530, 1503, 1462, 1427, 1382, 1295, 1260, 1219, 1186, 1168, 1155, 1118, 1104, 1063, 1040, 1016, 981, 971, 957, 885. **RMN $^1$H** (400 MHz, DMSO-*d*$_6$, 20°C) δ

12.23 (s, 1H), 8.23 (m, 1H), 7.80 (dd, *J* = 9.5, 2.5 Hz, 1H), 7.38 (d, *J* = 8.6 Hz, 2H), 6.76 - 6.67 (m, 3H), 3.49 (t, *J* = 7.1 Hz, 2H), 2.97 (s, 3H), 2.43 (t, *J* = 7.1 Hz, 2H), 2.23 (s, 6H). **RMN $^{13}$C** (101 MHz, DMSO-$d_6$, 20°C) δ 155.8 (d, *J* = 239.4 Hz, Cq), 149.7 (Cq), 145.6 (Cq), 133.1 (2 x CH), 132.1 (d, *J* = 27.4 Hz, CH), 122.9 (Cq), 120.3 (d, *J* = 7.5 Hz, Cq), 113.7 (2 x CH), 112.0 (d, *J* = 20.8 Hz, CH), 107.4 (Cq), 105.4 (d, *J* = 4.0 Hz, CH), 95.5 (Cq), 80.1 (Cq), 55.4 ($CH_2$), 49.9 ($CH_2$), 45.9 (2 x $CH_3$), 38.6 ($CH_3$). **RMN $^{19}$F** (376 MHz, $CDCl_3$, 20°C) δ. -138.4. **HRMS (+ESI)** calculée pour $C_{20}H_{21}FN_4$ (M+H$^+$): 337.1823, trouvée: 337.1821.

**Exemple 57 : 8-(4-((5-fluoro-1*H*-pyrrolo[2,3-*b*]pyridin-2-yl)ethynyl)phenyl)-8-azaspiro[4.5]decane 150 :**

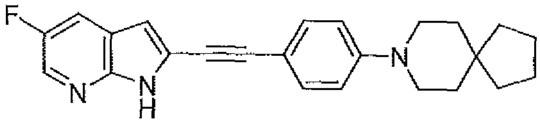

8-(4-((5-fluoro-1*H*-pyrrolo[2,3-b]pyridin-2-yl)ethynyl)phenyl)-8-azaspiro[4.5]decane

Le 8-(4-((5-fluoro-1*H*-pyrrolo[2,3-*b*]pyridin-2-yl)éthynyl)phényl)-8-azaspiro[4.5]decane **150** a été synthétisé en 3 étapes. Dans un premier temps, l'alcyne correspondant **151** a été préparé à partir du bromé **152** puis nous avons effectué la réaction de Sonogashira avec l'azaindole pour obtenir le composé final.

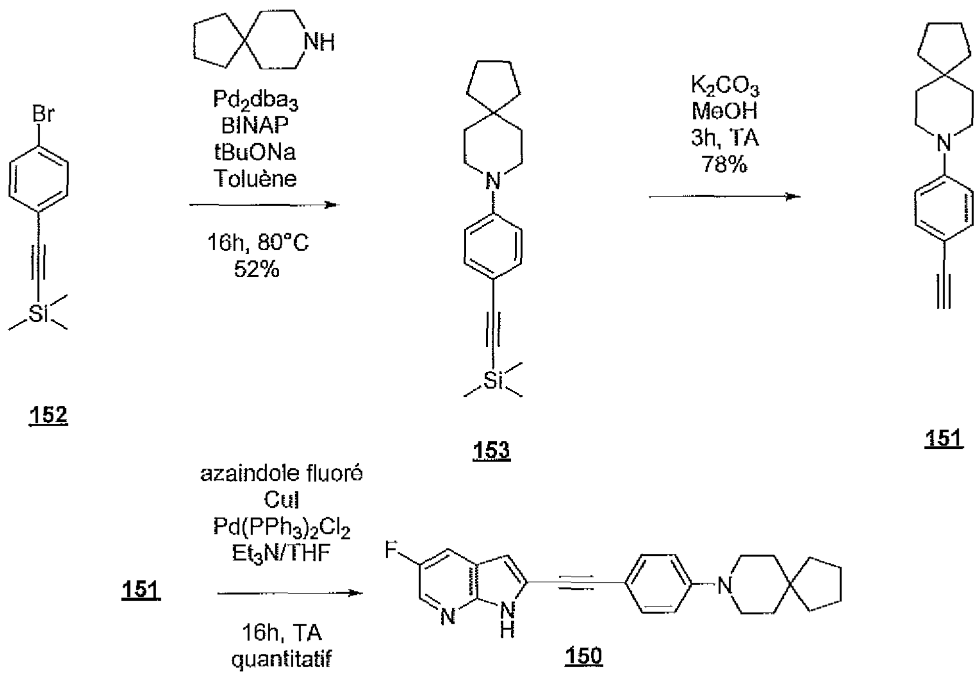


[2]**Exemple 57.1** Synthèse du 8-(4-ethynylphenyl)-8-azaspiro[4.5]decane **151** :

Le composé **151** a été préparé selon la **procédure Dure H**suivie de la **procédure L** et purifié par colonne de chromatographie flash sur gel de silice (acétate d'éthyle/éther de pétrole = 1/49). Solide amorphe jaune pâle (41% sur 2 étapes). **Infrarouge** (v, cm$^{-1}$, neat) 3302, 2915, 2849, 2096, 1729, 1678, 1602, 1554, 1508, 1463, 1448, 1389, 1345, 1309, 1248, 1193, 1137, 1067, 998, 951, 899, 858. **RMN $^1$H** (400 MHz, $CDCl_3$,20°C) δ 7.38 (d, *J* = 8.7 Hz, 2H), 6.85 (d, *J* = 8.7 Hz, 2H), 3.29 -3.20 (m, 4H), 2.99 (s, 1H), 1.72 - 1.58 (m, 8H), 1.49 (m, 4H). **RMN $^{13}$C** (101 MHz, $CDCl_3$, 20°C) δ 151.6 (Cq), 133.2 (2 x CH), 114.9 (2 x CH), 110.9 (Cq), 84.5 (Cq), 75.1 (Cq), 46.3 (2 x $CH_2$), 40.7 (Cq), 37.7 (2 x $CH_2$), 37.0 (2 x $CH_2$), 24.3 (2 x $CH_2$). **HRMS** (+ESI) calculée pour $C_{17}H_{21}N$(M+H$^+$) : 240.1745, trouvée: 240.1746.

**Exemple 57.2** Synthèse du 8-(4-((5-fluoro-1*H*-pyrrolo[2,3-*b*]pyridin-2-yl)ethynyl)phenyl)-8-azaspiro[4.5]decane **150:**

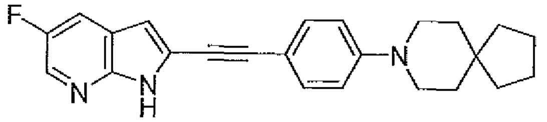

$C_{24}H_{24}FN_3$
M W: 373,48 gmol-1

8-(4-((5-fluoro-1*H*-pyrrolo[2,3-*b*]pyridin-2-yl)ethynyl)phenyl)-8-azaspiro[4.5]decane

Le composé a été préparé selon la **procédure C2** et purifié par colonne de chromatographie flash sur gel de silice (méthanol/dichlorométhane = 2/98). Solide jaune (40%), **Pf**>260°C, $R_f$ = 0.22 (méthanol/dichlorométhane = 2/98). **Infrarouge** (v, $cm^{-1}$, neat) 3119, 3058, 2921, 2827, 2200, 1601, 1585, 1533, 1504, 1462, 1385, 1344, 1287, 1243, 1226, 1186, 1155, 1138, 1107, 998, 984, 952, 898, 878. **RMN $^1$H** (400 MHz, DMSO-$d_6$, 20°C) δ 12.26 (s, 1H), 8.24 (s, 1H), 7.81 (m, 1H), 7.39 (d, *J* = 8.9 Hz, 2H), 6.97 (d, *J* = 8.9 Hz, 2H), 6.72 (s, 1H), 3.34 - 3.24 (m, 4H), 1.61 (s, 4H), 1.54 - 1.40 (m, 8H).**RMN $^{13}$C** (101 MHz, DMSO-$d_6$, 20°C) δ 155.6 (d, *J* = 239.4 Hz, Cq), 150.6 (Cq), 144.7 (Cq), 132.0 (2 x CH), 131.6 (d, *J* = 27.4 Hz, CH), 121.8 (Cq), 119.4 (d, *J* = 7.5 Hz, Cq), 114.1 (2 x CH), 112.8 (d, *J* = 20.8 Hz, CH), 108.6 (Cq), 104.8 (d, *J* = 4.0 Hz, CH), 94.2 (Cq), 79.4 (Cq), 44.63 (2 x $CH_2$), 40.1 (Cq), 36.7 (2 x $CH_2$), 35.9 (2 x $CH_2$), 24.4 (2 x $CH_2$). **RMN $^{19}$F** (376 MHz, $CDCl_3$, 20°C) δ. -138.4. **HRMS (+ESI)** calculée pour $C_{24}H_{24}FN_3$ (M+H$^+$): 374.2027, trouvée: 374.2027.

**Exemple 58 4-(4-((5-fluoro-1*H*-pyrrolo[2,3-*b*]pyridin-2-yl)ethynyl)phenoxy)-*N,N*-dimethylaniline 154 :**

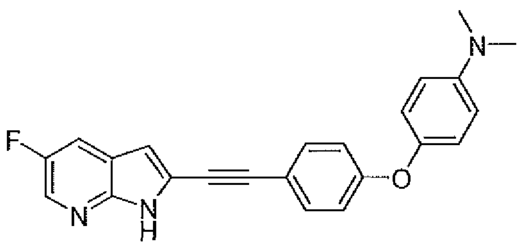

4-(4-((5-fluoro-1*H*-pyrrolo[2,3-b]pyridin-2-yl)ethynyl)phenoxy)-*N,N*-dimethylaniline

La 4-(4-((5-fluoro-1*H*-pyrrolo[2,3-*b*]pyridin-2-yl)éthynyl)phenoxy)-*N,N*-diméthylaniline **154** a été synthétisée en 5 étapes. Dans un premier temps, l'alcyne **155** a été préparé en 4 étapes en partant du phényle éther **99** puis la réaction de Sonogashira a été mise en œuvre avec l'azaindole fluoré pour conduire au composé final.

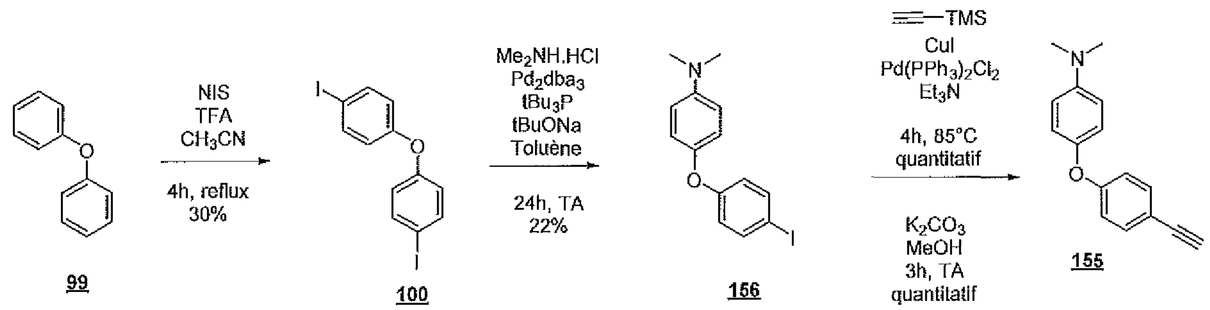


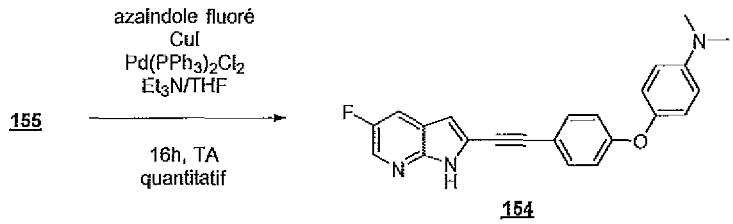

**Exemple 58.1** Synthèse de la 4-(4-iodophenoxy)-*N,N*-dimethylaniline **156** :

Sous argon, le $Pd_2dba_3$ (0.05 éq.) et la *i*$Bu_3P$ (0.075 mmol) sont ajoutés à une solution de toluène anhydre dégazée de concentration 0.3M contenant l'anime (1.0 éq.), le iodé **100** (1.0 éq.) et *t*-BuONa (1.2 éq.). Au bout de 24h à température ambiante, le solvant est évaporé sous pression réduite. Ensuite, le mélange réactionnel est concentré sous pression réduite, avant d'être purifié par colonne de chromatographie flash sur gel de silice. Le composé **156** est obtenu sous forme d'une huile incolore avec un rendement de 22%, **Rf =** 0.20 (acétate d'éthyle/éther de pétrole = 1/49). **Infrarouge** (ν, $cm^{-1}$, neat) 3044, 2844, 2793, 1578, 1508, 1475, 1439, 1395, 1344, 1276, 1223, 1163, 1125, 1056, 1001, 947. **RMN $^1$H** (250 MHz, $CDCl_3$, 20°C) δ 7.52 (d, *J* = 8.9 Hz, 2H), 6.91 (d, *J* = 8.9 Hz, 2H), 6.74 - 6.62 (m, 4H), 2.91 (s, 6H). **RMN $^{13}$C DEPT** (63 MHz, $CDCl_3$, 20°C) δ 138.5 (2 x CH), 121.2 (2 x CH), 119.4 (2 x CH), 114.1 (2 x CH), 41.4 (2 x $CH_3$). **HRMS** (+ESI) calculée pour $C_{14}H_{14}INO$ (M+H$^+$) : 340.0192, trouvée: 340.0191.

**Exemple 58.2** Synthèsede la 4-(-ethynylphenoxy-*N,N*-dimethylaniline **155** :

Du catalyseur $[Pd(PPh_3)_2Cl_2]$ (1.5 mg, 0.002 mmol, 0.03 éq.) a été ajouté à une solution dégazée du iodé **156** (80 mg, 0.131 mmol, 1.0 éq.), éthynyltriméthylsilane (0.016 mL, 0.1 mmol, 1.5 éq.), de CuI (1 mg, 0.002 mmol, 0.03 éq.), dans de la $Et_3N$ à la concentration de 0.3 M. Le mélange est agité à 85°C pendant 4 h sous atmosphère d'argon. Ensuite, le mélange réactionnel est concentré sous pression réduite, avant d'être purifié par colonne de chromatographie flash sur gel de silice avec comme éluant (acétate d'éthyle/éther de pétrole = 20/80) pour obtenir 28 mg de l'intermédiaire qui mis en réaction avec du $K_2CO_3$ (19 mg, 0.140 mmol, 1.5 éq.) dans 4 mL d'un mélange DCM/méthanol (1/1). Au bout de 3h d'agitation à température ambiante, la solution est filtrée sur coton. Après évaporation à sec, le brut est purifié par colonne de chromatographie flash sur gel de silice avec comme éluant (acétate d'éthyle/éther de pétrole = 4/96) pour obtenir l'alcyne **155** sous forme d'une huile incolore avec un rendement quantitatif sur 2 étapes. **Rf =** 0.20 (acétate d'éthyle/éther de pétrole = 4/96). **Infrarouge** (ν, $cm^{-1}$, neat) 3284, 3070, 2929, 2856, 1610, 1513, 1495, 1471, 1427, 1360, 1280, 1234, 1160, 1103, 985, 927, 869. **RMN $^1$H** (400 MHz, $CDCl_3$, 20°C) δ 7.40 (d, *J* = 8.6 Hz, 2H), 6.96 (d, *J* = 9.0 Hz, 2H), 6.85 (d, *J* = 8.6 Hz, 2H), 6.74 (d, *J* = 9.0 Hz, 2H), 3.00 (s, 1H), 2.94 (s, 6H). **RMN $^{13}$C** (101 MHz, $CDCl_3$, 20°C) δ 159.8 (Cq), 148.0 (Cq), 146.3 (Cq), 133.6 (2 x CH), 121.3 (2 x CH), 116.7 (2 x CH), 115.3 (Cq), 113.8 (2 x CH), 83.5 (Cq), 77.3 (CH), , 41.1 (2 x $CH_3$). **HRMS** (+ESI) calculée pour $C_{16}H_{15}NO$ (M+Na+): 280.1308, trouvée: 280.1312.

**Exemple 58.3** Synthèse de la 4-(4-((5-fluoro-1*H*-pyrrolo[2,3-*b*]pyridin-2-yl)ethynyl)phenoxy)-*N,N*-dimethylaniline **154** :

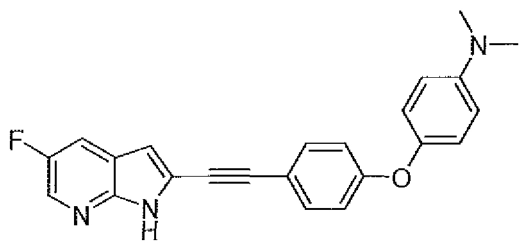

$C_{23}H_{18}FN_3O$
M W: 371,42 gmol-1

4-(4-((5-fluoro-1H-pyrrolo[2,3-*b*]pyridin-2-yl)ethynyl)phenoxy)-*N,N*-dimethylaniline

Le composé a été préparé selon la **procédure C2** et purifié par colonne de chromatographie flash sur gel de silice (méthanol/dichlorométhane = 2/98). Solide jaune (31%), **Pf :** 228-230°C, ***R*$_f$** = 0.22 (méthanol/dichlorométhane = 2/98). **Infrarouge** (ν, $cm^{-1}$, neat) 3119, 3060, 2970, 2850, 2799, 1586, 1515, 1504, 1493, 1455, 1443, 1427, 1396, 1347, 1293, 1277, 1250, 1222, 1162, 1126, 1102, 1060, 985, 946, 893. **RMN $^1$H** (250 MHz, DMSO-$d_6$, 20°C) δ 12.30 (s, 1H), 8.22 (s, 1H), 7.79 (m, 1H), 7.50 (d, *J* = 8.4 Hz, 2H), 6.92 (2 x d, *J* = 8.4 Hz, 4H), 6.75 (d, *J* = 7.7 Hz, 3H), 2.86 (s, 6H). **RMN $^{13}$C DEPT** (63 MHz, DMSO-$d_6$, 20°C) δ 133.7 (2 x CH), 132.9 (d, *J* = 27.4 Hz, CH), 121.7 (2 x CH), 117.1 (2 x CH), 114.2 (2 x CH), 113.8 (d, *J* = 20.8 Hz, CH), 106.4 (d, *J* = 4.0 Hz, CH), 41.00 (2 x $CH_3$). **RMN $^{19}$F** (235 MHz, $CDCl_3$, 20°C) δ. -138.5. **HRMS (+ESI)** calculée pour $C_{23}H_{18}FN_3O$ (M+H$^+$): 372.1506, trouvée: 372.1505.

**Exemple 59 : 5-fluoro-2-((4-(4-(methylsulfonyl)piperazin-1-yl)phenyl)ethynyl)-1*H*-pyrrolo[2,3 *b*]pyridine 157 :**

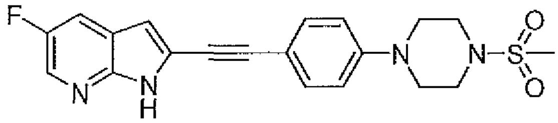

La 5-fluoro-2-((4-(4-(méthylsulfonyl)piperazin-1-yl)phényl)éthynyl)-1H-pyrrolo[2,3-*b*]pyridine **157** a été synthétisée en 3 étapes. Dans un premier temps, l'alcyne **158** a été préparé en 2 étapes à partir du bromé **152** puis la réaction de Sonogashira avec l'azaindole pemret d'obtenir le composé final.

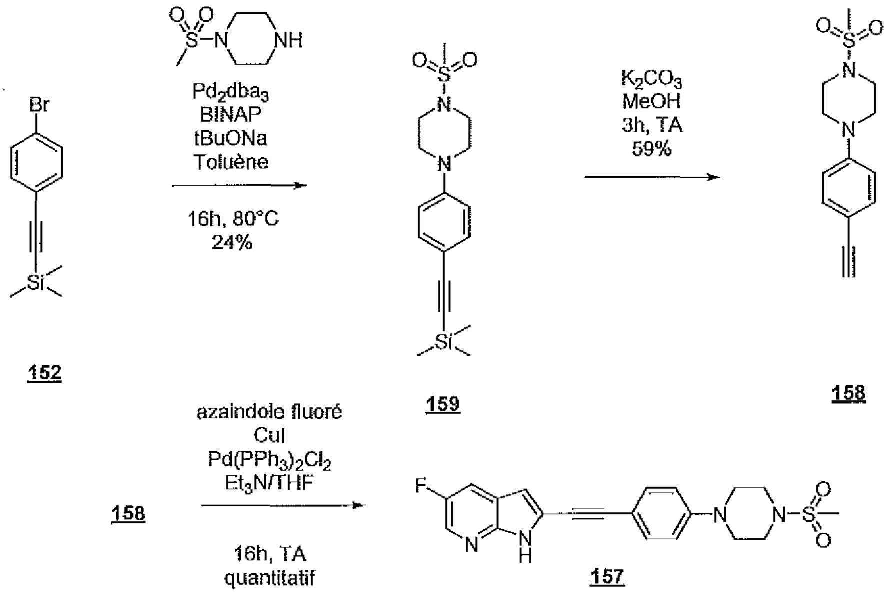


**Exemple 59.1** Synthèse de la 1-(4-ethynylphenyl)-4-(methylsulfonyl)piperazine **158** :

Le composé **158** a été préparé selon la **procédure H** suivie de la **procédure L** et purifié par colonne de chromatographie flash sur gel de silice (acétate d'éthyle/éther de pétrole = 30/70). Solide blanc (14% sur 2 étapes), **Pf :** 208-210°C, $R_f$ = 0.5 (acétate d'éthyle/éther de pétrole = 30/70). **Infrarouge** (v, $cm^{-1}$, neat) 3245, 2928, 2836, 1605, 1508, 1448, 1389, 1374, 1340, 1329, 1319, 1286, 1263, 1237, 1204, 1179, 1156, 1139, 1114, 1065, 1049, 948, 908. **RMN $^1$H** (400 MHz, $CDCl_3$, 20°C) $\delta$ 7.39 (d, *J* = 8.7 Hz, 2H), 6.82 (d, *J* = 8.7 Hz, 2H), 3.34 (s1, 8H), 2.98 (s, 1H), 2.80 (s, 3H). **RMN $^{13}$C** (101 MHz, $CDCl_3$,20°C) $\delta$ 150.5 (Cq), 133.3 (2 x CH), 115.9 (2 x CH), 113.5 (Cq), 83.8 (Cq), 75.9 (Cq), 48.5 (2 x $CH_2$), 45.6 (2 x $CH_2$), 34.5 ($CH_3$). **HRMS** (+ESI) calculée pour $C_{13}H_{16}N_2O_2S$ ($M+H^+$) : 265.1006, trouvée: 265.1005.

**Exemple 59.2** Synthèse de la 5-fluoro-2-((4-(4-(methylsulfonyl)piperazin-1-yl)phenyl)ethynyl)-1*H*-pyrrolo[2,3-*b*]pyridine **157** :

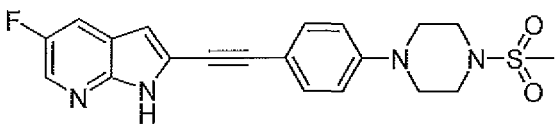

$C_{20}H_{19}FN_4O_2S$
M W 398,46 gmol-1

Le composé a été préparé selon la **procédure C2** et purifié par colonne de chromatographie flash sur gel de silice (acétate d'éthyle/éther de pétrole = 30/70). Solide jaune (33%), **Pf**>260°C, $R_f$ = 0.33 (acétate d'éthyle/éther de pétrole = 30/70). **Infrarouge** (v, $cm^{-1}$, neat) 2971, 2901, 2361, 1729, 1606, 1559, 1541, 1507, 1448, 1393, 1373, 1323, 1291, 1259, 1223, 1186, 1174, 1132, 115, 1088, 1066, 1012, 937, 833, 817, 803. **RMN $^1$H** (400 MHz, DMSO-$d_6$, 20°C) $\delta$ 12.28 (s, 1H), 8.25 (s, 1H), 7.83 (d, *J* = 9.1 Hz, 1H), 7.45 (d, *J* = 8.5 Hz, 2H), 7.04 (d, *J* = 8.7 Hz, 2H), 6.75 (s, 1H), 3.38 (d, *J* = 5.0 Hz, 4H), 3.25 (d, *J* = 5.0 Hz, 4H), 2.93 (s, 3H). **RMN $^{13}$C** (101 MHz, DMSO-$d_6$, 20°C) $\delta$ 155.6 (d, *J* = 239.4 Hz, Cq), 150.9 (Cq), 145,6 (Cq), 133.0 (2 x CH), 132.6 (d, *J* = 27.4 Hz, CH), 122.5 (Cq), 120.3 (d, *J* = 7.5 Hz, Cq),

115.7 (2 x CH), 113.9 (d, $J$ = 20.8 Hz, CH), 111.3 (Cq), 105.9 (d, $J$ = 4.0 Hz, CH), 94.7 (Cq), 80.7 (Cq), 47.4 (2 x $CH_2$), 45.6 (2 x $CH_2$), 34.4 ($CH_3$). **RMN $^{19}$F** (376 MHz, DMSO-$d_6$, 20°C) δ. -138.6. **HRMS (+ESI)** calculée pour $C_{20}H_{19}FN_4O_2S$ ($M+H^+$): 399.1285, trouvée: 399.1285.

**Exemple 60 : 2-((4-((5-fluoro-1*H*-pyrrolo[2,3-*b*]pyridin-2-yl)ethynyl)phenyl)(methyl) amino)ethan-1-ol 160** :

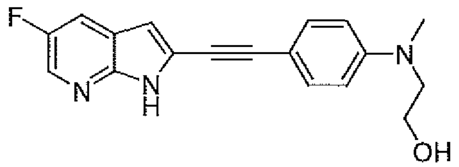

2-((4-((5-fluoro-1*H*-pyrrolo[2,3-*b*]pyridin-2-yl)ethynyl)phenyl)(methyl)amino)ethan-1-ol

Le 2-((4-((5-fluoro-1*H*-pyrrolo[2,3-*b*]pyridin-2-yl)éthynyl)phényl)(méthyl)amino)éthan-1-ol **160** a été synthétisé en 4 étapes. Dans un premier temps, l'alcyne **161** a été préparé en 2 étapes à partir du bromé **152** puis la réaction de Sonogashira avec l'azaindole suivie d'une déprotection permet d' obtenir le composé final.

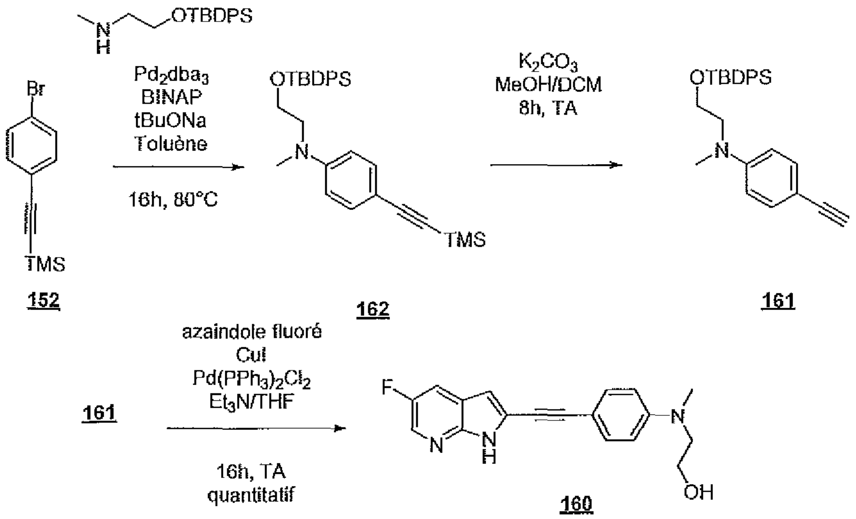

152 162 161
161 160

**Exemple 60.1** Synthèse de la *N*-(2-((tert-butyldiphenylsilyl)oxy)ethyl)-4-ethynyl-*N*-methylaniline **161** :

Le composé **161** a été préparé selon la **procédure H** suivie de la **procédure L** et purifié par colonne de chromatographie flash sur gel de silice (acétate d'éthyle/éther de pétrole = 10/90). Huile jaune (44% sur 2 étapes). ***R*$_f$** = 0.4 (acétate d'éthyle/éther de pétrole = 10/90). **Infrarouge** (v, $cm^{-1}$, neat) 3291, 2962, 2930, 2853, 2102, 1606, 1511, 1463, 1434, 1373, 1356, 1336, 1273, 1245, 1227, 1185, 1141, 1093, 1023, 935. **RMN $^{1}$H** (400 MHz, $CDCl_3$,20°C) δ 7.68 (d, $J$ = 6.8 Hz, 3H), 7.40 (td, $J$ = 12.3, 11.8, 6.0 Hz, 7H), 6.49 (d, $J$ = 8.8 Hz, 2H), 3.83 (t, $J$ = 6.2 Hz, 2H), 3.52 (d, $J$ = 6.1 Hz, 2H), 2.98 (d, $J$ = 5.3 Hz, 3H), 1.09 (s, 9H).

**Exemple 60.2** Synthèse de la N-(2-((*tert*-butyldiphenylsilyl)oxy)ethyl)-4-((5-fluoro-1*H*-pyrrolo[2,3-*b*]pyridin-2-yl)ethynyl)-*N*-methylaniline **163** :

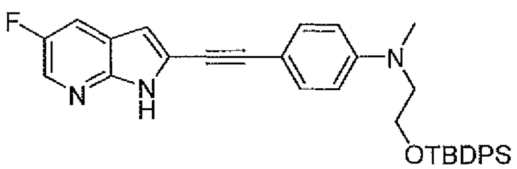

Le composé **163** a été préparé selon la **procédure C2** et purifié par colonne de chromatographie flash sur gel de silice (acétate d'éthyle/éther de pétrole = 10/90). Solide jaune (78%), ***R*f** = 0.10 (acétate d'éthyle/éther de pétrole = 10/90). **Pf** = 182-184°C. **Infrarouge** (ν, $cm^{-1}$, neat) 2922, 2852, 2198, 2008, 1605, 1584, 1537, 1504, 1470, 1426, 1377, 1349, 1294, 1233, 1188, 1154, 1107, 1007, 976, 871. **RMN $^1$H** (400 MHz, $CDCl_3$, 20°C) δ 8.23 (s, 1H), 7.66 (d, *J* = 7.5 Hz, 4H), 7.59 (d, *J* = 8.5 Hz, 1H), 7.46 (t, *J* = 7.5 Hz, 2H), 7.39 (t, *J* = 7.5 Hz, 6H), 6.68 (s, 1H), 6.55 (d, *J* = 8.5 Hz, 2H), 3.84 (t, *J* = 6.2 Hz, 2H), 3.54 (t, *J* = 6.2 Hz, 2H), 3.01 (s, 3H), 1.07 (s, 9H). **RMN $^{13}$C DEPT** (101 MHz, $CDCl_3$, 20°C) δ133.0 (4 x CH),132.8 (2 x CH), 132.3 (d, *J* = 27.4 Hz, CH), 129.8 (2 x CH), 127.8 (4 x CH), 113.6 (d, *J* = 20.8 Hz, CH), 111.5 (2 x CH), 106.4 (d, *J* = 4.0 Hz, CH), 60.7 ($CH_2$), 54.2 ($CH_2$), 39.2 ($CH_3$), 26.8 (3 x $CH_3$). **RMN $^{19}$F** (376 MHz, $CDCl_3$, 20°C) δ. -138.54, **HRMS (+ESI)** calculée pour $C_{34}H_{34}FN_3OSi$ ($M+H^+$) : 548.2527 ; trouvée: 548.2528.

**Exemple 60.3** Synthèse du 2-((4-((5-fluoro-1*H*-pyrrolo[2,3-*b*]pyridin-2-yl)ethynyl)phenyl)(methyl) amino)ethan-1-ol **160** :

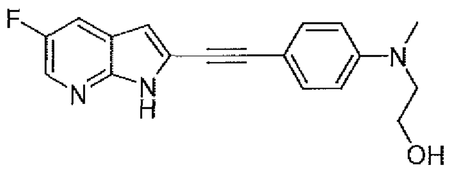

$C_{18}H_{16}FN_3O$
M W: 309,34 gmol-1

2-((4-((5-fluoro-1H-pyrrolo[2,3-*b*]pyridin-2-yl)ethynyl)phenyl)(methyl)amino)ethan-1-ol

Le composé **160** a été préparé selon la **procédure E** et purifié par colonne de chromatographie flash sur gel de silice (méthanol/dichlorométhane = 4/96). solide jaune (45%), ***R*f** = 0.10 (méthanol/dichlorométhane = 2/98). **Pf** = 192-194°C. **Infrarouge** (ν, $cm^{-1}$, neat) 3126, 2921, 2852, 2198, 1729, 1603, 1585, 1540, 1507, 1449, 1432, 1372, 1321, 1287, 1260, 1241, 1219, 1185, 1153, 1120, 1100, 1078, 1052, 978, 930, 917, 889, 892, 832. **RMN $^1$H** (400 MHz, DMSO-*d*$_6$, 20°C) δ 12.22 (s, 1H), 8.22 (s, 1H), 7.80 (m, 1H), 7.37 (d, *J* = 8.5 Hz, 2H), 6.80 - 6.60 (m, 3H), 4.71 (s, 1H), 3.56 (m, 2H), 3.50 - 3.39 (m, 2H), 2.99 (s, 3H). **RMN $^{13}$C** (101 MHz, DMSO-d6, 20°C) δ 155.6 (d, *J* = 239.4 Hz, Cq), 150.0 (Cq), 145.6 (Cq), 133.0 (2 x CH), 132.6 (d, *J* = 27.4 Hz, CH), 123.3 (Cq), 120.3 (d, *J* = 7.5 Hz, Cq), 113.4 (d, *J* = 20.8 Hz, CH), 112.0 (2 x CH), 107.2 (Cq), 106.3 (d, *J* = 4.0 Hz, CH), 95.6 (Cq), 80.6 (Cq), 58.5 ($CH_2$), 54.3 ($CH_2$), 39.2 ($CH_3$). **RMN $^{19}$F** (376 MHz, DMSO-*d*$_6$, 20°C) δ. - 138.4, **HRMS (+ESI)** calculée pour $C_{18}H_{16}FN_3O$ ($M+H^+$): 310.1350 ; trouvée: 310.1349.

**Exemple 61 : 3-((4-((5-fluoro-1*H*-pyrrolo[2,3-*b*]pyridin-2-yl)ethynyl)phenyl)(methyl) amino)propan-1-ol 164** :

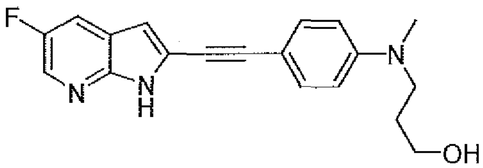

3-((4-((5-fluoro-1*H*-pyrrolo[2,3-*b*]pyridin-2-yl)ethynyl)phenyl)(methyl)amino)propan-1-ol

Le 3-((4-((5-fluoro-1*H*-pyrrolo[2,3-*b*]pyridin-2-yl)éthynyl)phényl)(méthyl)amino)propan-1-ol **164** a été synthétisé en 4 étapes. Dans un premier temps, l'alcyne **165** a été préparé en 3 étapes à partir du bromé **152** puis la réaction de Sonogashira avec l'azaindole suivie d'une déprotection fournit le composé final.

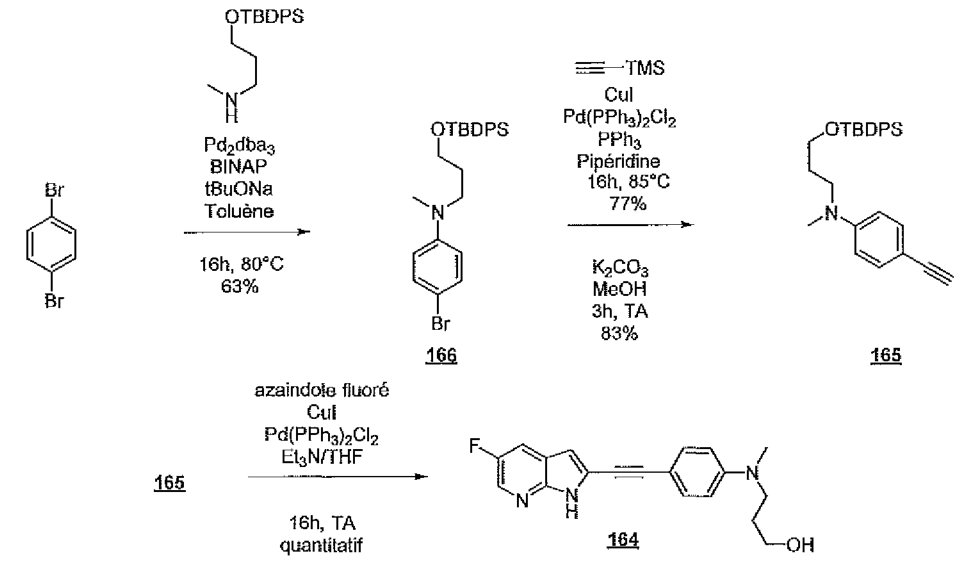


**Exemple 61.1** Synthèse de la 4-bromo-*N*-(3-((*tert*-butyldiphenylsilyl)oxy)propyl)-*N*-methylaniline **166** :

Le composé **166** a été préparé selon la **procédure H** et purifié par colonne de chromatographie flash sur gel de silice (acétate d'éthyle/éther de pétrole = 2/48). Huile orange (64%). $\boldsymbol{R_f}$ = 0.55 (acétate d'éthyle/éther de pétrole = 2/48). **Infrarouge** (ν, $cm^{-1}$, neat) 33070, 2929, 2856, 1592, 1496, 1471, 1426, 1370, 1239, 1210, 1189, 1104, 926. **RMN $^1$H** (400 MHz, $CDCl_3$, 20°C) δ 7.69 (d, *J* = 7.2 Hz, 4H), 7.42 (m, 6H), 7.29 (m, 2H), 6.62 (d, *J* = 8.5 Hz, 2H), 3.73 (t, *J* = 7.3 Hz, 2H), 3.49 (t, *J* = 7.3 Hz, 2H), 2.91 (s, 3H), 1.79 (p, *J* = 6.5 Hz, 2H), 1.11 (s, 9H). **RMN $^{13}$C** (101 MHz, $CDCl_3$, 20°C) δ 148.3 (Cq), 135.6 (4 x CH), 133.7 (2 x Cq), 131.7 (2 x CH), 129.7 (2 x CH), 127.7 (4 x CH), 113.8 (2 x CH), 107.7 (Cq) 61.3 ($CH_2$), 49.4 ($CH_2$), 38.3 ($CH_3$), 29.4 ($CH_2$), 26.9 (3 x $CH_3$), 19.2 (Cq). **HRMS (+ESI)** calculée pour $C_{26}H_{32}BrNOSi$ (M+) : 482.1509, trouvée: 482.1509.

**Exemple 61.2** Synthèse de la *N*-(3-((*tert*-butyldiphenylsilyl)oxy)propyl)-4-ethynyl-*N*-methylaniline **165** :

L'alcyne **165** a été préparé selon la **procédure I1** et purifié par colonne de chromatographie flash sur gel de silice (acétate d'éthyle/éther de pétrole = 2/48). Huile jaunâtre (35% sur les deux étapes). $\boldsymbol{R_f}$ = 0.50 (acétate d'éthyle/éther de pétrole = 2/48). **Infrarouge** (ν, $cm^{-1}$, neat) 3288, 2929, 2856, 1594, 1570, 1494, 1471, 1427, 1360, 1105, 1003, 822. **RMN $^1$H** (250 MHz, $CDCl_3$,20°C) δ 7.70 - 7.60 (m, 4H), 7.48 - 7.30 (m, 8H), 6.63 (d, *J* = 8.9 Hz, 2H), 3.70 (t, *J* = 5.7 Hz, 2H), 3.58 - 3.47 (m, 2H), 2.97 (s, 1H), 2.93 (s, 3H), 1.78 (s, 2H), 1.08 (s, 9H). **RMN $^{13}$C** (101 MHz, $CDCl_3$,20°C) δ 13C NMR (63 MHz, CDC13) δ 149.3 (Cq), 135.6 (4 x CH), 133.7 (2 x Cq), 133.3 (2 x CH), 129.7 (2 x CH), 127.7 (4 x CH), 111.4 (CH), 108.2 (Cq) 84.9 (Cq), 74.6 (CH), 61.2 ($CH_2$) 49.1 ($CH_2$), 38.2 ($CH_3$), 29.6 ($CH_2$), 26.9 (3 x $CH_3$), 19.2 (Cq). **HRMS (+ESI)** calculée pour $C_{28}H_{33}N_2OSi$ (M+H$^+$): 428.2404, trouvée: 428.2399.

**Exemple 61.3** Synthèse du 3-((4-((5-fluoro-1*H*-pyrrolo[2,3-*b*]pyridin-2-yl)ethynyl)phenyl)(methyl) amino)propan-1-ol **164** :

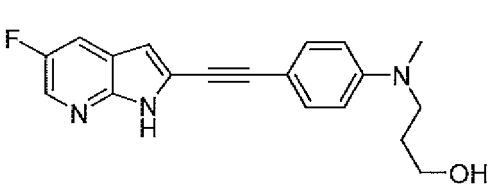

$C_{19}H_{18}FN_3O$
M W: 323,37 gmol-1

3-((4-((5-fluoro-1H-pyrrolo[2,3-*b*]pyridin-2-yl)ethynyl)phenyl)(methyl)amino)propan-1-ol

Le composé **164** a été préparé selon la **procédure C2** suivie de la **procédure E** et purifié par colonne de chromatographie flash sur gel de silice (méthanol/dichlorométhane = 4/96). Solide jaune (48% sur 2 étapes), $\boldsymbol{R_f}$ = 0.10 (méthanol/dichlorométhane = 2/98). **Pf** = 192-194°C. **Infrarouge** (ν, $^{-1}$, neat) 3126, 2921, 2852, 2198, 1729, 1603, 1585, 1540, 1507, 1449, 1432, 1372, 1321, 1287, 1260, 1241, 1219, 1185, 1153, 1120, 1100, 1078, 1052, 978, 930, 917, 889,

892, 832. **RMN $^1$H** (400 MHz, DMSO-$d_6$, 20°C) δ 12.23 (s, 1H), 8.22 (s, 1H), 7.80 (d, *J* = 9.5 Hz, 1H), 7.37 (d, *J* = 8.5 Hz, 2H), 6.77 - 6.67 (m, 3H), 4.58 - 4.51 (m, 1H), 3.51 - 3.39 (m, 4H), 2.95 (s, 3H), 1.68 (p, *J* = 6.5 Hz, 2H). **RMN $^{13}$C** (101 MHz, DMSO-d6, 20°C) δ 155.6 (d, *J* = 239.4 Hz, Cq), 150.0 (Cq), 145.6 (Cq), 133.0 (2 x CH), 132.6 (d, *J* = 27.4 Hz, CH), 123.3 (Cq), 120.3 (d, *J* = 7.5 Hz, Cq), 113.4 (d, *J* = 20.8 Hz, CH), 112.0 (2 x CH), 107.2 (Cq), 106.3 (d, *J* = 4.0 Hz, CH), 95.7 (Cq), 80.0 (Cq), 58.7 ($CH_2$), 48.9 ($CH_2$), 39.2 ($CH_3$), 29.9 ($CH_2$). **RMN $^{19}$F** (376 MHz, $CDCl_3$, 20°C) δ. -138.54. **HRMS** (+ESI) calculée pour $C_{19}H_{18}FN_3O$ (M+H$^+$) : 324.1505 ; trouvée: 324.1504.

**Exemple 62 (1-(2-((4-((5-fluoro-1*H*-pyrrolo[2,3-*b*]pyridin-2-yl)ethynyl)phenyl)(methyl)amino)ethyl)-1*H*-1,2,3-triazol-4-yl)methanol 167:**

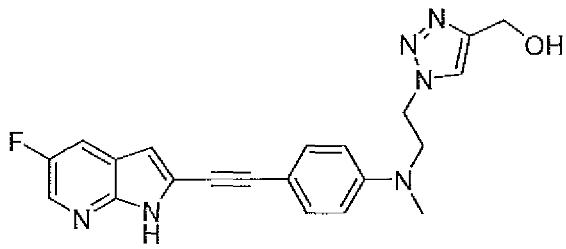

(1-(2-((4-((5-fluoro-1*H*-pyrrolo[2,3-*b*]pyridin-2-yl)ethynyl)phenyl)(methyl)amino)ethyl)-1*H*-1,2,3-triazol-4-yl)methanol

Le (1-(2-((4-((5-fluoro-1*H*-pyrrolo[2,3-*b*]pyridin-2-yl)éthynyl)phényl)(méthyl)amino)éthyl)-1*H*-1,2,3-triazol-4-yl)méthanol **167** a été synthétisé en 10 étapes. Dans un premier temps, l'alcyne correspondant **168** a été préparé en 8 étapes à partir du 1,4-dibromobenzène et puis la réaction de Sonogashira avec l'azaindole suivie d'une réaction de déprotection pemret d'obtenir le composé final.

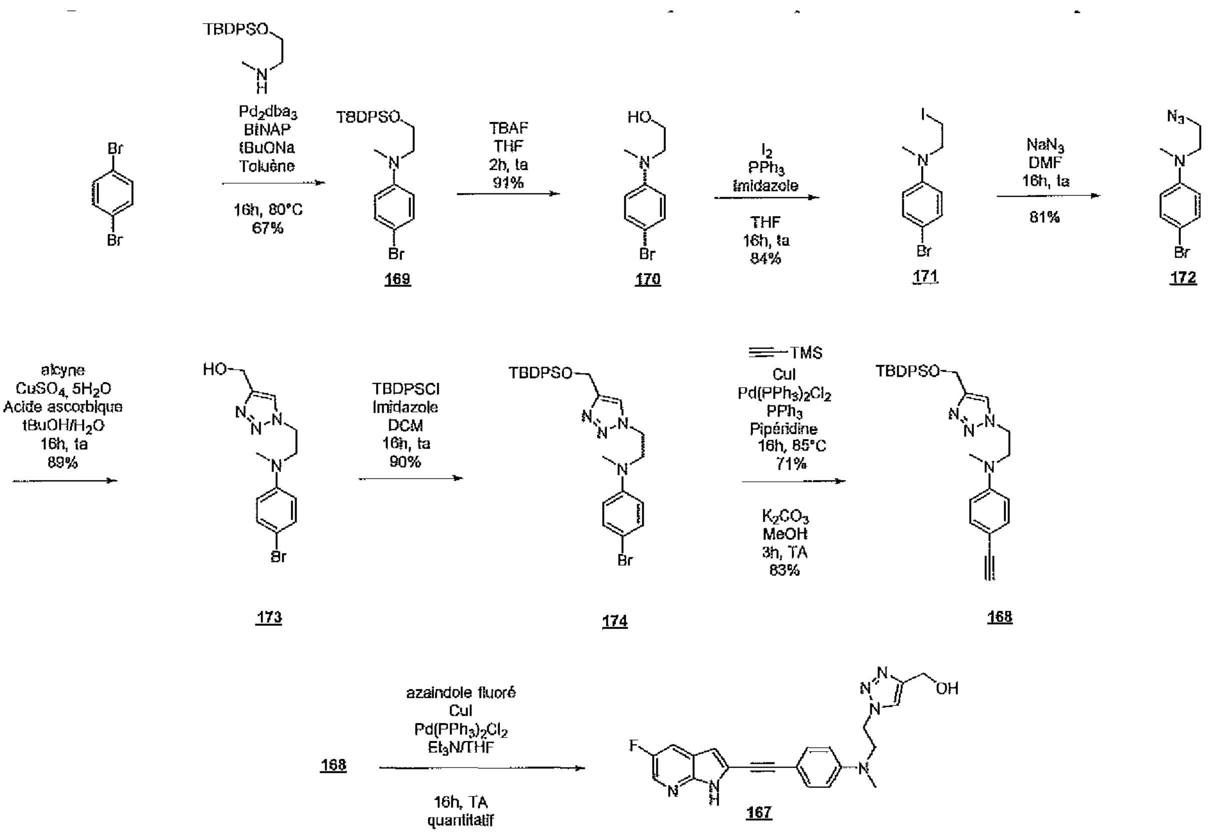


Exemple **62.1** Synthèse de la 4-bromo-*N*-(2-((*tert*-butyldiphenylsilyl)oxy)ethyl)-*N*-methylaniline **169** :

Le composé **169** a été préparé selon la **procédure H** et purifié par colonne de chromatographie flash sur gel

de silice (acétate d'éthyle/éther de pétrole = 5/45). Huile orange (67%). $\boldsymbol{R_f}$ = 0.65 (acétate d'éthyle/éther de pétrole = 1/49). **Infrarouge** (ν, $cm^{-1}$, neat) 2929, 2855, 1588, 1496, 1471, 1427, 1356, 1314, 1253, 1218, 1190, 1141, 1105, 1079, 1045, 997, 936. **RMN $^1$H** (400 MHz, $CDCl_3$, 20°C) δ 7.69 (d, *J* = 7.2 Hz, 4H), 7.42 (m, 6H), 7.29 (m, 2H), 6.62 (d, *J* = 8.5 Hz, 2H), 3.73 (t, *J* = 7.3 Hz, 2H), 3.49 (t, *J* = 7.3 Hz, 2H), 2.91 (s, 3H), 1.11 (s, 9H). **RMN $^{13}$C** (101 MHz, $CDCl_3$, 20°C) δ 148.3 (Cq), 135.6 (4 x CH), 133.7 (2 x Cq), 131.7 (2 x CH), 129.7 (2 x CH), 127.7 (4 x CH), 113.8 (2 x CH), 107.7 (Cq) 60.8 ($CH_2$), 54.5 ($CH_2$), 38.3 ($CH_3$), 26.9 (3 x $CH_3$), 19.2 (Cq). **HRMS (+ESI)** calculée pour $C_{25}H_{30}BrNOSi$ (M+) : 468.1352, trouvée: 468.1353.

**Exemple 62.2** Synthèse du 2-((4-bromophenyl)(methyl)amino)ethan-1-ol **170** :

Le composé **170** a été préparé selon la **procédure E** et purifié par colonne de chromatographie flash sur gel de silice (méthanol/dichlorométhane = 4/96). Huile incolore (91%). **RMN $^1$H** (400 MHz, $CDCl_3$) δ 7.30 (d, *J* = 8.3 Hz, 2H), 6.75 - 6.53 (d, *J* = 8.3 Hz, 2H), 3.80 (m, 2H), 3.45 (m, 2H), 2.94 (s, 3H). Numéro CAS : 252949-11-4.

**Exemple 62.3** Synthèse de la 4-bromo-*N*-(2-iodoethyl)-*N*-methylaniline **171** :

Sous argon, 400 mg de 2-((4-bromophényl)(méthyl)amino)éthan-1-ol **170** (1.74 mmol, 1.0 éq.) sont solubilisés dans 18 mL de THF. A 0°C, 190 mg d'imidazole (2.80 mmol, 1.6 éq.), 616 mg de triphénylphosphine (2.35 mmol, 1.35 éq.) et 574 mg d'iode (2.26 mmol, 1.3 éq.) sont ajoutés. Au bout de 5 minutes à 0°C, le mélange réactionnel est mis à température ambiante. Après 16h d'agitation, 30 mL d'une solution de thiosulfate saturé est additionnée. La phase organique est extraite avec de l'acétate d'éthyle (3 x 30 mL). Les phases organiques sont réunies et lavées avec une solution de NaCl saturée puis séchées sur $MgSO_4$, filtrées sur coton et évaporées à sec. Le brut est purifié par colonne de chromatographie flash sur gel de silice (acétate d'éthyle/éther de pétrole = 2/98) pour conduire au composé **171** (497 mg, 84%) sous forme d'une huile incolore. $\boldsymbol{R_f}$ = 0.9 (acétate d'éthyle/éther de pétrole = 20/80). **Infrarouge** (ν, $cm^{-1}$, neat) 2873, 2820, 1588, 1492, 1427, 1369, 1339, 1311, 1289, 1269, 1216, 1169, 1109, 1089, 971. **RMN $^1$H** (400 MHz, $CDCl_3$, 20°C) δ 7.31 (d, *J* = 9.0 Hz, 2H), 6.56 (d, *J* = 9.0 Hz, 2H), 3.73 - 3.65 (m, 2H), 3.23 - 3.17 (m, 2H), 2.96 (s, 3H). **RMN $^{13}$C** (101 MHz, $CDCl_3$, 20°C) δ 146.9 (Cq), 132.1 (2 x CH), 113.7 (2 x CH), 109.0 (Cq), 55.5 ($CH_2$), 38.4 ($CH_3$), 1.8 ($CH_2$). **HRMS (+ESI)** calculée pour $C_9H_{11}IBrN$ ($M+H^+$) : 341.9172, trouvée: 341.9170.

**Exemple 62.4** Synthèse de la *N*-(2-azidoethyl)-4-bromo-*N*-methylaniline **172** :

Sous argon, 460 mg de 4-bromo-*N*-(2-iodoéthyl)-*N*-méthylaniline **171** (1.35 mmol, 1.0 éq.) sont solubilisés dans 5 mL de DMF. Ensuite, 102 mg d'azoture de 1.62 mmol, 1.2 éq.) sont ajoutés. Après 16h d'agitation à température ambiante, 30 mL d'une $H_2O$ sont additionnés. La phase organique est extraite avec de l'acétate d'éthyle (3 x 30 mL). Les phases organiques sont réunies et lavées avec une solution de NaCl saturée puis séchées sur $MgSO_4$, filtrées sur coton et évaporées à sec. Le brut est purifié par colonne de chromatographie flash sur gel de silice (acétate d'éthyle/éther de pétrole = 4/96) pour conduire au composé **171** (280 mg, 81%) sous forme d'une huile incolore. $\boldsymbol{R_f}$ = 0.85 (acétate d'éthyle/éther de pétrole = 10/90). **Infrarouge** (ν, $cm^{-1}$, neat) 3292, 2940, 2870, 1594, 1570, 1494, 1480, 1435, 1370, 1155, 1003, 822. **RMN $^1$H** (400 MHz, $CDCl_3$, 20°C) δ 7.34 (d, *J* = 9.0 Hz, 2H), 6.62 (d, *J* = 9.0 Hz, 2H), 3.54 (t, *J* = 5.8 Hz, 2H), 3.47 (t, *J* = 5.8 Hz, 2H), 3.00 (s, 3H). **RMN $^{13}$C** (101 MHz, $CDCl_3$, 20°C) δ 147.6 (Cq), 132.0 (2 x CH), 113.9 (2 x CH), 109.0 (Cq), 55.5 ($CH_2$), 48.7 ($CH_2$), 38.9 ($CH_3$). **HRMS (+ESI)** calculée pour $C_9H_{11}BrN_4$ (M) : 255.0239, trouvée: 255.0238.

**Exemple 62.5** Synthèse du (1-(2-((4-bromophenyl)(methyl)amino)ethyl)-1*H*-1,2,3-triazol-4-yl)methanol **173** :

Sous argon, 240 mg de *N*-(2-azidoéthyl)-4-bromo-*N*-méthylaniline **170** (0.94 mmol, 1.0 éq.) sont solubilisés dans 15 mL d'un mélange *t*-butanol/eau (2/1). A température ambiante, 0.055 mL d'alcool propargylique (0.94, 1.0 éq.), 25 mg de $CuSO_4$,$5H_2O$ (0.094 mmol, 0.1 éq.) et 19 mg d'ascorbate de sodium (0.094 mmol, 0.1 éq.) sont ajoutés. Après 16h d'agitation, 30 mL d'une eau sont additionnés. La phase organique est extraite avec du dichlorométhane (3 x 30 mL). Les phases organiques sont réunies et lavées avec une solution de NaCl saturée puis séchées sur $MgSO_4$, filtrées sur coton et évaporées à sec. Le brut est purifié par colonne de chromatographie flash sur gel de silice (méthanol/dichlorométhane = 4/96) pour conduire au composé **173** (260 mg, 89%) sous forme d'une huile incolore. $\boldsymbol{R_f}$ = 0 (acétate d'éthyle/éther de pétrole = 20/80). **Infrarouge** (ν, $cm^{-1}$, neat) 3297, 2873, 1588, 1494, 1449, 1430, 1355, 1314, 1255, 1215, 1191, 1141, 1113, 1079, 1038, 1009, 959. **RMN $^1$H** (250 MHz, $CDCl_3$, 20°C) δ 7.33 (m, 3H), 6.48 (d, *J* = 9.0 Hz, 2H), 6.5 (s, 2H), 3.73 - 3.65 (m, 2H), 3.23 - 3.17 (m, 2H), 2.76 (s, 3H). **RMN $^{13}$C** (63 MHz, $CDCl_3$, 20°C) δ 147.9 (Cq), 147.1 (Cq), 132.1 (2 x CH), 122.6 (CH), 113.7 (2 x CH), 109.0 (Cq), 56.5 ($CH_2$), 53.0 ($CH_2$), 47.5 ($CH_2$), 38.8 ($CH_3$).

**Exemple 62.6** Synthèse de la 4-bromo-*N*-(2-(4-(((*tert*-butyldiphenylsilyl)oxy)methyl)-1*H*-1,2,3-triazol-1-yl)ethyl)-*N*-methylaniline **174 :**

Sous argon, le (1-(2-((4-bromophényl)(méthyl)amino)éthyl)-1*H*-1,2,3-triazol-4-yl)méthanol **173** (735 mg, 2.35 mmol, 1.0 éq.) est solubilisé dans 50 mL de DCM. A 0°C, 0.7 mL de TBDPSCl (2.82 mmol, 1.2 éq.), et 192 mg d'imidazole (2.80 mmol, 1.2 éq.) sont ajoutés. Au bout de 5 minutes à 0°C, le mélange réactionnel est mis à température ambiante. Après 16h d'agitation, 30 mL d'eau est additionné. La phase organique est extraite avec du dichlorométhane (3 x 30 mL). Les phases organiques sont réunies et lavées avec une solution de NaCl saturée puis séchées sur $MgSO_4$, filtrées sur coton et évaporées à sec. Le brut est purifié par colonne de chromatographie flash sur gel de silice (acétate d'éthyle/éther de pétrole = 20/80) pour conduire au composé **174** (1.2 g, 90%) sous forme d'une huile incolore. $R_f$ = 0.25 (acétate d'éthyle/éther de pétrole = 20/80). **Infrarouge** (ν, $cm^{-1}$, neat) 2929, 2855, 1588, 1496, 1471, 1427, 1356, 1314, 1253, 1218, 1190, 1141, 1105, 1079, 1045, 997, 936. **RMN $^1$H** (250 MHz, $CDCl_3$, 20°C) δ 7.65 (d, *J* = 6.5 Hz, 4H), 7.48 - 7.27 (m, 8H), 7.19 (s, 1H), 6.49 (d, *J* = 9.0 Hz, 2H), 4.85 (s, 2H), 4.51 (t, *J* = 6.1 Hz, 2H), 3.81 (t, *J* = 6.1 Hz, 2H), 2.73 (s, 3H), 1.05 (s, 9H). **RMN $^{13}$C** (63 MHz, $CDCl_3$, 20°C) δ 148.4 (Cq), 147.0 (Cq), 135.5 (4 x CH), 133.2 (2 x Cq), 132.1 (2 x CH), 129.8 (2 x CH), 127.8 (4 x CH), 122.6 (CH), 113.8 (2 x CH), 109.4 (Cq), 58.53 ($CH_2$), 53.0 ($CH_2$), 47.3 ($CH_2$), 38.8 ($CH_3$), 26.8 (3 x $CH_3$), 19.2 (Cq). **HRMS** (+ESI) calculée pour $C_{28}H_{33}BrN_4OSi$ (M+H+) : 551.1660, trouvée: 551.1664.

**Exemple 62.7** Synthèse de la *N*-(2-(4-(((*tert*-butyldiphenylsilyl)oxy)methyl)-1*H*-1,2,3-triazol-1-yl)ethyl)-4-ethynyl-*N*-methylaniline **168** :

L'alcyne **168** a été préparé selon la **procédure I2** et purifié par colonne de chromatographie flash sur gel de silice (acétate d'éthyle/éther de pétrole = 30/70). Huile marron (38% sur les deux étapes). $R_f$ = 0.55 (acétate d'éthyle/éther de pétrole = 40/60). **Infrarouge** (ν, $cm^{-1}$, neat) 3280, 2928, 2930, 2855, 2098, 1606, 1514, 1459, 1426, 1375, 1355, 1273, 1256, 1220, 1174, 1141. **RMN $^1$H** (250 MHz, $CDCl_3$, 20°C) δ 7.65 (d, *J* = 6.5 Hz, 4H), 7.48 - 7.27 (m, 8H), 7.18 (s, 1H), 6.55 (d, *J* = 9.0 Hz, 2H), 4.85 (s, 2H), 4.50 (t, *J* = 6.1 Hz, 2H), 3.86 (t, *J* = 6.1 Hz, 2H), 2.76 (s, 1H), 2.76 (s, 3H), 1.05 (s, 9H). **RMN $^{13}$C DEPT** (63 MHz, $CDCl_3$, 20°C) δ 135.5 (4 x CH), 133.6 (2 x CH), 129.8 (2 x CH), 127.8 (4 x CH), 122.6 (CH), 111.6 (2 x CH), 75.3 (CH), 58.5 ($CH_2$), 52.7 ($CH_2$), 47.3 ($CH_2$), 38.8 ($CH_3$), 26.8 (3 x $CH_3$), 19.2 (Cq). **HRMS** (+ESI) calculée pour $C_{30}H_{34}N_4OS$ (M+H+) : 495.2573, trouvée: 495.2575.

**Exemple 62.8** Synthèse du (1-(2-((4-((5-fluoro-1*H*-pyrrolo[2,3-*b*]pyridin-2-yl)ethynyl)phenyl)(methyl)amino)ethyl)-1*H*-1,2,3-triazol-4-yl)methanol **167 :**

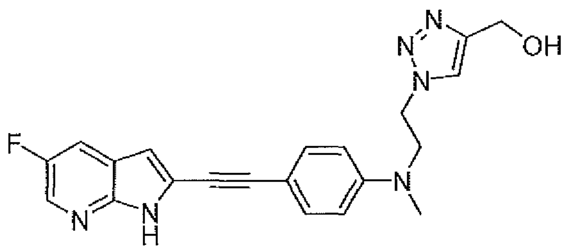

$C_{21}H_{19}FN_6O$
M W : 390,42 gmol-1

(1-(2-((4-((5-fluoro-1*H*-pyrrolo[2,3-*b*]pyridin-2-yl)ethynyl)phenyl)(methyl)amino)ethyl)-1*H*-1,2,3-triazol-4-yl)methanol

Le composé **167** a été préparé selon **la procédure C2** suivie de la **procédure E** et purifié par colonne de chromatographie flash sur gel de silice (méthanol/dichlorométhane = 4/96). Solide jaune (74% sur 2 étapes), $R_f$ = 0.30 (méthanol/dichlorométhane = 4/96). **Pf** = 222-224°C. **Infrarouge** (ν, $cm^{-1}$, neat) 3311, 3121, 2901, 2202, 1605, 1585, 1540, 1508, 1475, 1448, 1431, 1378, 1357, 1323, 1291, 1257, 1231, 1185, 1156, 1109, 1073, 1052, 1036, 1012, 983, 965, 937, 869. **RMN $^1$H** (400 MHz, DMSO-$d_6$, 20°C) δ 12.22 (s, 1H), 8.22 (s, 1H), 7.99 (s, 1H), 7.80 (dd, J = 8.8, 2.8 Hz, 1H), 7.37 (d, *J* = 8.8 Hz, 2H), 6.72 (m, 3H), 5.14 (t, *J* = 5.6 Hz, 1H), 4.52 (m, 4H), 3.85 (t, *J* = 6.2 Hz, 2H), 2.79 (s, 3H). **RMN $^{13}$C** (63 MHz, DMSO-d6, 20°C) δ 155.6 (d, *J* = 239.4 Hz, Cq), 149.3 (Cq), 148.6 (Cq), 145.63 (Cq), 133.0 (2 x CH), 132.6 (d, *J* = 27.4 Hz, CH), 123.6 (CH), 122.9 (Cq), 120.4 (d, *J* = 7.5 Hz, Cq), 113.7 (d, *J* = 20.8 Hz, CH), 112.2 (2 x CH), 108.7 (Cq), 105.9 (d, *J* = 4.0 Hz, CH), 95.3 (Cq), 80.0 (Cq), 55.5 ($CH_2$), 52.01 ($CH_2$), 47.1 ($CH_2$), 39.2 ($CH_3$). **RMN** $^{19}$F (376 MHz, $CDCl_3$, 20°C) δ. -138.54. **HRMS** (+ESI) calculée pour $C_{21}H_{19}FN_6O$ (M+H+) : 391.1675, trouvée: 391.1677.

**Exemple 63 : 4-((3-((dimethylamino)methyl)-1*H*-pyrrolo[2,3-*b*]pyridin-2-yl)ethynyl)-*N*-(2-fluoroethyl)-*N*-methylaniline 175 :**

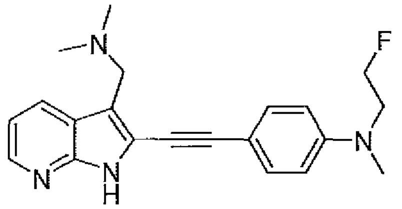

4-((3-((dimethylamino)methyl)-1*H*-pyrrolo[2,3-*b*]pyridin-2-yl)ethynyl)-*N*-(2-fluoroethyl)-*N*-methylaniline

La 4-((3-((diméthylamino)méthyl)-1*H*-pyrrolo[2,3-*b*]pyridin-2-yl)éthynyl)-*N*-(2-fluoroéthyl)-*N*-méthylaniline **175** a été synthétisée en 2 étapes. Dans un premier temps, l'azaindole **176** est obtenu par une réaction de Mannich suivie par une réaction de Sonogoshira pour obtenir le composé final attendu.

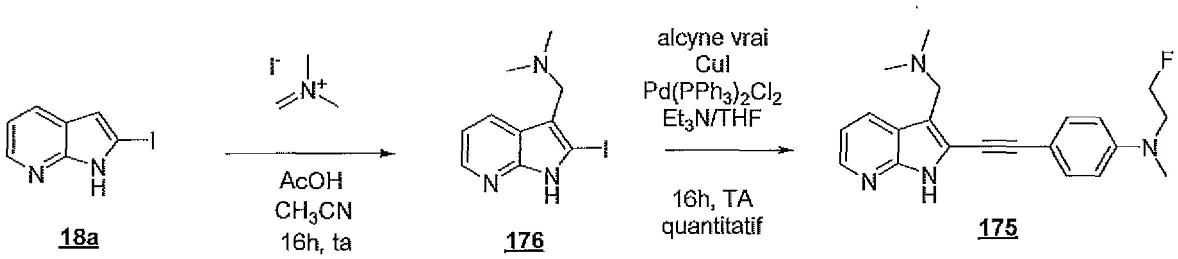


**18a** **176** **175**

**Exemple 63.1** Synthèse de la 1-(2-iodo-1*H*-pyrrolo[2,3-*b*]pyridin-3-yl)-*N,N*-dimethylmethanamine **176** :

Dans un vial, la 2-iodo-1*H*-pyrrolo[2,3-*b*]pyridine **18a** (200 mg, 0.82 mmol, 1 éq.) est solubilisée dans 8 mL d'acétonitrile. Ensuite, 0.4 mL d'acide acétique suivi de 182 mg de sel d'Eschenmoser (0.98 mmol, 1.2 éq.) sont ajoutés. Après le scellage du vial, le mélange réactionnel est agité pendant 20h à température ambiante. 15 mL d'une solution de potasse à 2M est ajouté doucement puis la phase organique est extraite avec de l'acétate d'éthyle (3 x 20 mL). Les phases organiques sont réunies puis séchées sur $MgSO_4$, filtrées sur coton et évaporées à sec. Pour finir, le brut est trituré au pentane puis filtration sous vide pour conduire au composé **174** (190 mg, 77%) sous forme d'un solide jaune. $R_f$ = 0.10 (méthanol/dichlorométhane = 2/98). **Pf** = 142-144°C. **Infrarouge** (ν, $cm^{-1}$, neat) 2957, 2932, 2851, 2812, 2768, 1603, 1580, 1514, 1489, 1448, 1407, 1369, 1354, 1328, 1287, 1273, 1248, 1206, 1167, 1147, 1121, 1094, 1036, 1004, 978, 905, 842. **RMN $^1$H** (400 MHz, $CDCl_3$, 20°C) δ 13.08 (si, 1H), 8.41 (dd, *J* = 4.9, 1.4 Hz, 1H), 8.09 (dd, *J* = 7.9, 1.4 Hz, 1H), 7.11 (dd, *J* = 7.9, 4.8 Hz, 1H), 3.60 (s 2H), 2.33 (s, 6H). **RMN $^{13}$C** (101 MHz, $CDCl_3$, 20°C) δ 151.0 (Cq), 142.0 (CH), 127.4 (CH), 121.3 (Cq), 116.7 (Cq), 115.8 (CH), 82.9 (Cq) 56.1 ($CH_2$), 45.4 (2 x $CH_3$). **HRMS (+ESI)** calculée pour $C_{10}H_{12}IN_3$ ($M+H^+$): 302.0148, trouvée: 302.0148.

**Exemple 63.2** Synthèse de la 4-((3-((dimethylamino)methyl)-1*H*-pyrolo[2,3-*b*]pyridin-2-yl)ethynyl)-*N*-(2-fluoroethyl)-*N*-methylaniline **175** :

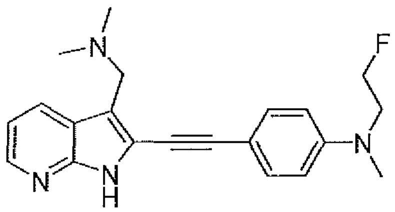

$C_{21}H_{23}FN_4$
M W: 350,44 gmol-1

4-((3-((dimethylamino)methyl)-1*H*-pyrrolo[2,3-*b*]pyridin-2-yl)ethynyl)-*N*-(2-fluoroethyl)-*N*-methylaniline

Le composé **175** a été préparé selon la **procédure C2** et purifié par colonne de chromatographie flash sur gel

de silice (méthanol/dichlorométhane/ammoniaque = 4/96/1). Solide marron (20%), $R_f$ = 0.15 (méthanol/dichlorométhane = 4/96). **Pf** = 168-170°C. **Infrarouge** (ν, $cm^{-1}$, neat) 2969, 2800, 2755, 2358, 1738, 1601, 1547, 1538,1512, 1505, 1494, 1470, 1463, 1455, 1428, 1393, 1374, 1349, 1290, 1264, 1216, 1187, 1164, 1142, 1045, 1020, 1004, 979, 951. **HRMS** (+ESI) calculée pour $C_{21}H_{23}FN_4$ $(M+H^+)$ : 351.1980 ; trouvée: 351.1979

**Exemple 63.3** Synthèse de la 4-((3-((dimethylamino)methyl)-6-fluoro-1*H*-pyrrolo[2,3-*b*]pyridin-2-yl)ethynyl)-*N,N*-dimethylaniline **177 :**

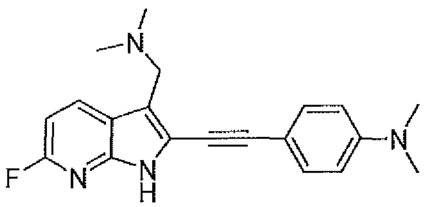

$C_{20}H_{21}FN_4$
M W: 336,41 gmol-1

4-((3-((dimethylamino)methyl)-6-fluoro-1*H*-pyrrolo[2,3-*b*]pyridin-2-yl)ethynyl)-*N,N*-dimethylaniline

Le composé **177** a été préparé selon la **procédure C2** et purifié par colonne de chromatographie flash sur gel de silice (méthanol/dichlorométhane/ammoniaque = 4/96/1). Solide verdâtre (36%), $R_f$ = 0.25 (méthanol/dichlorométhane = 4/96). **Pf =** 182-184°C. **Infrarouge** (ν, $cm^{-1}$, neat) 3157, 3072, 2893, 2205, 1605, 1541, 1511, 1432, 1273, 1224, 1183, 809, 763, 507. **RMN $^1$H** (400 MHz, $CDCl_3$, 20°C) 9.42 (s, 1H), 8.09 (t, *J* = 8.0 Hz, 1H), 7.45 (d, *J* = 8.7 Hz, 2H), 6.71 (m, 3H), 3.76 (s, 2H), 3.03 (s, 6H), 2.35 (s, 6H). **RMN $^{13}$C** (101 MHz, DMSO-d6, 20°C) δ 161.0 (d, *J* = 238.0 Hz, Cq), 150.5 (Cq), 144.7 (d, *J* = 18.3 Hz, CH), 132.7 (2 x CH), 132.5 (d, *J* = 9.3 Hz, Cq)), 119.40 (d, *J* = 4.4 Hz, Cq), 118.13 (d, *J* = 2.7 Hz, Cq), 116.2 (Cq), 111.8 (2 x CH), 108.8 (Cq), 101.82 (d, *J* = 37.8 Hz, CH), 97.7 (Cq), 78.0 (Cq), 54.1 ($CH_2$), 45.4 (2 x $CH_3$), 40.2 (2 x $CH_3$). **RMN $^{19}$F** (376 MHz, DMSO-d6, 20°C) δ. -76.0. **HRMS (+ESI)** calculée pour $C_{20}H_{21}FN_4$ $(M+H^+)$ : 337.1823 ; trouvée: 337.1822.

**Exemple 64: 4-((3-((dimethylamino)methyl)-5-fluoro-1*H*-pyrrolo[2,3-*b*]pyridin-2-yl)ethynyl)-*N,N*-dimethylaniline 179 :**

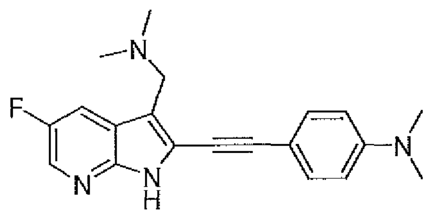

4-((3-((dimethylamino)methyl)-5-fluoro-1*H*-pyrrolo[2,3-*b*]pyridin-2-yl)ethynyl)-*N,N*-dimethylaniline

La 4-((3-((diméthylamino)méthyl)-5-fluoro-1*H*-pyrrolo[2,3-*b*]pyridin-2-yl)éthynyl)-*N,N*-diméthylaniline **179** a été synthétisée en 2 étapes. Dans un premier temps, l'azaindole **180** est obtenu par une réaction de Mannich suivi par une réaction de Sonogoshira pour obtenir le composé final attendu.

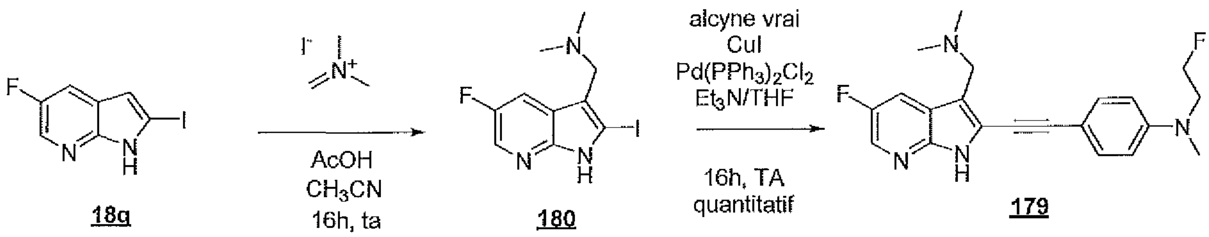


18q 180 179

Exemple 64.1 Synthèse de la 1-(5-fluoro-2-iodo-1*H*-pyrrolo[2,3-*b*]pyridin-3-yl)-*N,N*-dimethylmethanamine **180** :

Dans un vial, le 5-fluoro-2-iodo-1*H*-pyrrolo[2,3-*b*]pyridine **18g** (200 mg, 0.82 mmol, 1 éq.) est solubilisé dans 8 mL d'acétonitrile. Ensuite, 0.4 mL d'acide acétique suivi de 182 mg de sel d'Eschenmoser (0.98 mmol, 1.2 éq.) sont ajoutés. Après le scellage du vial, le mélange réactionnel est agité pendant 20h à température ambiante. 15 mL d'une solution de potasse à 2M est ajouté doucement puis la phase organique est extraite avec de l'acétate d'éthyle (3 x 20 mL). Les phases organiques sont réunies puis séchées sur $MgSO_4$, filtrées sur coton et évaporées à sec. Pour finir, le brut est trituré au pentane puis filtration sous vide pour conduire au composé **180** (180 mg, 77%) sous forme d'un solide brun, $\boldsymbol{R_f}$ = 0.10 (méthanol/dichlorométhane = 2/98). **Pf** = 180-182°C. **Infrarouge** (ν, $cm^{-1}$, neat) 2934, 2857, 2818, 2773, 2703, 1491, 152, 1395, 1368, 1333, 1322, 1295, 1261, 1250, 1218, 1183, 1168, 1146, 1068, 1006, 951, 871, 842. **RMN $^1$H** (250 MHz, $CDCl_3$, 20°C) δ 8.21 (s, 1H), 7.77 (dd, J = 8.9, 2.4 Hz, 1H), 3.50 (s, 2H), 2.26 (s, 6H). **RMN $^{13}$C** (250 MHz, $CDCl_3$, 20°C) δ 155.34 (d, *J* = 242.4 Hz, Cq), 147.8 (Cq), 130.6 (d, *J* = 30.2 Hz, CH), 121.02 (d, *J* = 7.0 Hz, Cq), 117.10 (d, *J* = 4.2 Hz, Cq), 113.27 (d, *J* = 21.0 Hz, CH), 84.4 (Cq) 56.3 ($CH_2$), 45.4 (2 x $CH_3$). **RMN $^{19}$F** (235 MHz, $CDCl_3$, 20°C) δ. -138.0. **HRMS (+ESI)** calculée pour $C_{10}H_{12}FIN_3$ ($M+H^+$) : 320.0054, trouvée: 320.0054.

Exemple 64.2 Synthèse de la 4-((3-((dimethylamino)methyl)-5-fluoro-1*H*-pyrrolo[2,3-*b*]pyridin-2-yl)ethynyl)-*N,N*-dimethylaniline **179** :

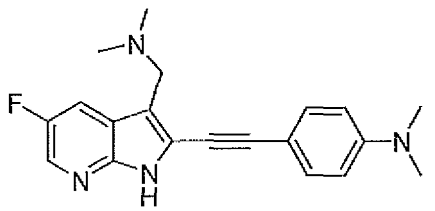

$C_{20}H_{21}FN_4$
M W: 336,41 gmol-1

Le composé **177** a été préparé selon **la procédure C2** et purifié par colonne de chromatographie flash sur gel de silice (méthanol/dichlorométhane/ammoniaque = 4/96/1). Solide marron (28%), $\boldsymbol{R_f}$ = 0.25 (méthanol/dichlorométhane = 4/96). **Pf** = 188-190°C. **Infrarouge** (ν, $cm^{-1}$, neat) 2933, 2875, 2851, 2766, 2766, 2708, 1604, 1587, 1574, 1539, 1515, 1494, 1462, 1455, 1445, 1397, 1368, 1355, 1334, 1307, 1283, 1224, 1166, 1126, 1116, 1090, 1017, 982, 949, 906, 863507. **RMN $^1$H** (250 MHz, $CDCl_3$, 20°C) δ 9.89 (s, 1H), 8.20 (m, 1H), 7.75 (dd, *J* = 8.9, 2.7 Hz, 1H), 7.44 (d, *J* = 8.9 Hz, 2H), 6.68 (d, *J* = 8.9 Hz, 2H), 3.72 (s, 2H), 3.02 (s, 6H), 2.32 (s, 6H).**RMN $^{19}$F** (376 MHz, $CDCl_3$, 20°C) δ. -138.0. **HRMS** (+ESI) calculée pour $C_{20}H_{21}FN_4$ ($M+H^+$) : 337.1823 ; trouvée: 337.1822.

**Exemple 65 : *N*-(2-fluoroethyl)-*N*-methyl-4-((3-methyl-1*H*-pyrrolo[2,3-*b*]pyridin-2-yl)ethynyl)aniline 181** :

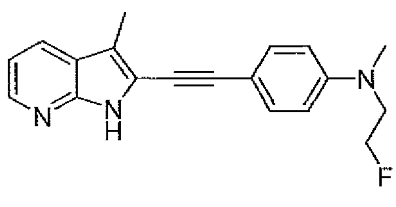

*N*-(2-fluoroethyl)-*N*-methyl-4-((3-methyl-1*H*-pyrrolo[2,3-*b*]pyridin-2-yl)ethynyl)aniline

La *N*-(2-fluoroéthyl)-*N*-méthyl-4-((3-méthyl-1*H*-pyrrolo[2,3-*b*]pyridin-2-yl)éthynyl)aniline **179** a été synthétisée en 6 étapes. Dans un premier temps, l'azaindole **182** est obtenu en 5 étapes successives en partant de 7-azaindole et la dernière étape consiste en une réaction de Sonogoshira avec l'alcyne pour obtenir le composé final attendu.

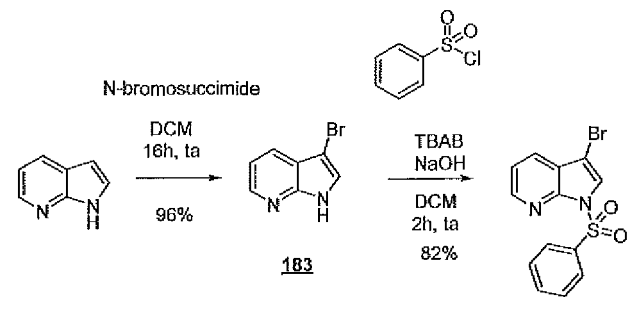


183

184

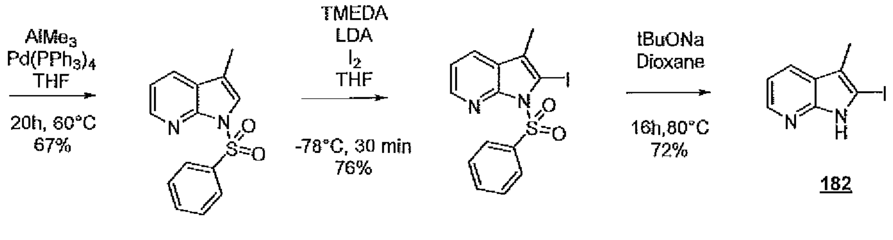


185

186

182

**Exemple 65.1** Synthèse de la 3-bromo-1*H*-pyrrolo[2,3-*b*]pyridine **183** :

A température ambiante, *N*-bromosuccimide (1.65 g, 9.31 mmol, 1.1 éq.) est additionné sur le 7-azaindole (1 g, 8.46 mmol, 1 éq.) dissous dans 20 mL de DCM. Au bout de 16h d'agitation à temperature, une solution aqueuse saturée en $NaHCO_3$ (20 mL) est ajoutée. La phase aqueuse est extraite avec du dichlorométhane (2 fois). Ensuite, les phases organiques réunies ont été séchées sur $MgSO_4$ et concentrées sous pression réduite. Le brut est purifié par colonne de chromatographie flash sur gel de silice (acétate d'éthyle/ether de pétrole = 1/1) pour conduire au composé **183** (1.6 g, 96%) sous la forme d'un solide brun. **RMN $^1$H** (400 MHz, DMSO-$d_6$, 20°C) δ 11.45 (br, 1H), 8.38 (dd, *J* = 5.1, 1.2 Hz, 1H), 8.06 (dd, *J* = 7.8, 1.2 Hz, 1H), 7.48 (s, 1H), 7.28 (dd, *J* = 7.8, 5.1 Hz, 1H). Numéro CAS : 74420-15-8.

**Exemple 65.2** Synthèse de la 3-bromo-1-(phenylsulfonyl)-1*H*-pyrrolo[2,3-*b*]pyridine **184:**

Sous argon, 1 g de 3-bromo-1*H*-pyrrolo[2,3-*b*]pyridine **184** (5.07 mmol, 1.0 éq.) est solubilisé dans 30 mL de DCM. A température ambiante, 49 mg de TBAB (0.15 mmol, 0.03 éq.) et 608 mg d'iode (15.21 mmol, 3.0 éq.) sont ajoutés. Ensuite, le melange réactionnel est placé à 0°C puis 0.81 mL de chlorure de benzosulfonyle est additionné goutte à goutte. Après 2h d'agitation à température ambiante, 30 mL d'eau sont ajoutés. La phase organique est extraite avec du dichlorométhane (3 x 30 mL). Les phases organiques sont réunies et lavées avec une solution de NaCl saturée puis séchées sur $MgSO_4$, filtrées sur coton et évaporées à sec. Le brut est trituré à l'éther diéthylique pour conduire au composé **184** (1.4 g, 82%) sous forme d'une solide orange. **RMN $^1$H** (400 MHz, $CDCl_3$, 20°C) δ 8.48 (dd, *J* = 4.8, 1.5 Hz, 1H), 8.24-8.17 (m, 2H), 7.82 (dd, *J* = 8.0, 1.5 Hz, 1H), 7.79 (s, 1H), 7.60 (dd, *J* = 7.5 Hz, 1H), 7.54-7.46 (m, 2H), 7.27 (m, 1H). Numéro CAS : 880769-95-9.

**Exemple 65.3** Synthèse de la 3-methyl-1-(phenylsulfonyl)-1*H*-pyrrolo[2,3-*b*]pyridine **185** :

La 3-bromo-1-(phénylsulfonyl)-1*H*-pyrrolo[2,3-*b*]pyridine **184** (500 mg, 1.48 mmol) est dissoute dans le THF (10 mL) sous argon. Après un dégazage de 10 minutes, le $Pd(PPh_3)_4$ (85 mg, 0.074 mmol, 0.05 éq.) est ensuite additionné et celle-ci est suivie de l'addition d'une solution de triméthylaluminium dans le toluène (2M, 1.48 mL, 2.96 mmol, 2.0 éq.) goutte à goutte. Ensuite, le milieu est chauffé à 60°C pendant 20h. Après refroidissement, 30 mL d'une solution de $NaHCO_3$ saturée sont ajoutés goutte à goutte. La phase organique est extraite avec de l'acétate d'éthyle (3 x 30 mL). Les phases organiques sont réunies et lavées avec une solution de NaCl saturée puis séchées sur $MgSO_4$, filtrées sur coton et évaporées à sec. Le brut est purifié par colonne de chromatographie flash sur gel de silice (acétate d'éthyle/éther de pétrole = 20/80) pour conduire au composé **185** (270 g, 67%) sous la forme d'un solide jaunâtre. **RMN $^1$H** (400 MHz, $CDCl_3$, 20°C) δ 11.45 (br, 1H), 8.38 (dd, *J* = 5.1, 1.2 Hz, 1H), 8.06 (dd, *J* = 7.8, 1.2 Hz, 1H), 7.48 (s, 1H), 7.28 (dd, *J* = 7.8, 5.1 Hz, 1H), 2.32 (s, 3H). Numéro Reaxys : 28959361.

**Exemple 65.4** Synthèse de la 2-iodo-3-methyl-1-(phenylsulfonyl)-1*H*-pyrrolo[2,3-*b*]pyridine **186** :

Sous atmosphère d'argon, à une solution de 3-méthyl-1-(phénylsulfonyl)-1*H*-pyrrolo[2,3-*b*]pyridine **185** (270 mg, 0.99 mmol, 1.0 éq.) et de TMEDA (0.163 mL, 1.09 mmol, 1.1 éq.) dans du THF (0.1M), refroidie à -78°C, a été additionnée une solution de LDA (2M dans du THF, 0.6 mL, 1.19 mmol, 1,2 éq.) goutte-à-goutte. Après 30 min, une solution d'iode (502.5 mg, 1.98 mmol, 2.0 éq.) dans du THF (0.5M) a été ajoutée lentement *via* une cannule au milieu réactionnel. Au bout de 30 min d'agitation à -78°C, 20 mL d'eau sont additonnés et le milieu réactionnel est remis à température ambiante doucement. Les phases organiques sont extraites avec de l'acétate d'éthyle (3 fois 20 mL), réunies, séchées sur $MgSO_4$, filtéres puis concentrée sous pression réduite. Le brut est purifié par colonne de chromatographie flash sur gel de silice (acétate d'éthyle/éther de pétrole = 20/80) pour conduire au composé **186** (300 g, 76%) sous la forme d'un solide jaunâtre. $\boldsymbol{R_f}$ **=** 0.10 (acétate d'éthyle/éther de pétrole = 20/80). **Pf =** 178-180°C. **Infrarouge** (ν, $cm^{-1}$, neat) 2955, 2172, 2137, 2104, 2025, 1966, 1618, 1586, 1562, 1501, 1479, 1435, 1383, 1337, 1277, 1248, 1190, 1157, 1119, 1071, 1001, 973, 962, 933. **RMN $^1$H** (250 MHz, $CDCl_3$, 20°C) δ 8.37 (d, *J* = 4.3 Hz, 1H), 8.14 (d, *J* = 7.3 Hz, 2H), 7.68 (dd, *J* = 8.7, 0.8 Hz, 1H), 7.57 - 7.35 (m, 3H), 7.12 (dd, *J* = 7.8, 4.8 Hz, 1H), 2.22 (s, 3H). **RMN $^{13}$C** (63 MHz, $CDCl_3$, 20°C) δ δ 150.5 (Cq) 144.7 (CH), 138.8 (Cq), 133.9 (CH), 128.9 (2 x CH), 127.9 (2 x CH), 126.8 (CH), 125.2 (Cq), 123.3 (Cq), 119.1 (CH), 80.0 (Cq), 13.5 ($CH_3$). **HRMS** (+ESI) calculée pour $C_{14}H_{11}IN_2O_2S$ ($M+H^+$) : 398.9658, trouvée: 398.9657.

**Exemple 65.5** Synthèse de la 2-iodo-3-methyl-1*H*-pyrrolo[2,3-*b*]pyridine **182 :**

Le 2-iodo-3-méthyl-1-(phénylsulfonyl)-1*H*-pyrrolo[2,3-*b*]pyridine **186** (125 mg, 0.314 mmol, 1.0 éq.) est solubilisé dans 7 mL de dioxane. 45 mg de *tert*-butoxide de sodium (0.47 mmol, 1.5 éq.) sont ajoutéd. Après 16h à 80°C, le milieu réactionnel est refroidi à température et le solvant est évaporé sous pression réduite. Le résidu est dissous dans de 30 mL l'acétate d'éthyle et 30 mL d'eau. Ensuite, la phase organique est extraite avec de l'acétate d'éthyle (3 fois). Les phases organiques réunies ont été séchées sur $MgSO_4$ et concentrées sous pression réduite. Le brut est purifié par colonne de chromatographie flash sur gel de silice (acétate d'éthyle/éther de pétrole = 20/80) pour conduire au composé **182** (58 mg, 72%) sous la forme d'un solide jaunâtre. $\boldsymbol{R_f}$ = 0.25 (acétate d'éthyle/éther de pétrole = 20/80). **Pf** = 188-190°C. **Infrarouge** (ν, $cm^{-1}$, neat) 2707, 1580, 1455, 1405, 1381, 1336, 1325, 1281, 1205, 1130, 903, 784. **RMN $^1$H** (400 MHz, DMSO-$d_6$, 20°C) δ 11.93 (s, 1H), 8.13 (dd, *J* = 4.6, 0.8 Hz, 1H), 7.87 (dd, *J* = 7.5, 0.8 Hz, 1H), 7.01 (dd, *J* = 7.5, 4.6 Hz, 1H), 2.19 (s, 3H). **RMN $^{13}$C** (250 MHz, DMSO-$d_6$, 20°C) δ 151.0 (Cq), 142.9 (CH), 126.0 (CH), 120.5 (Cq), 115.7 (CH), 114.9 (Cq), 83.4 (Cq), 11.9 ($CH_3$). **HRMS (+ESI)** calculée pour $C_8H_7IN_2$ ($M+H^+$) :, trouvée:

**Exemple 65.6** Synthèse de la *N*-(2-fluoroethyl)-*N*-methyl-4-((3-methyl-1*H*-pyrrolo[2,3-*b*]pyridin-2-yl)ethynyl)aniline **181 :**

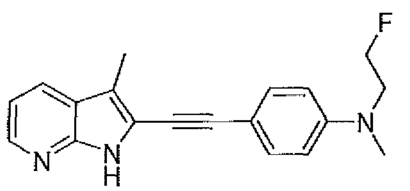

$C_{19}H_{18}FN_3$
M W: 307,37 gmol-1

Le composé **181** a été préparé selon la **procédure C2** et purifié par colonne de chromatographie flash sur gel de silice (acétate d'éthyle/éther de pétrole = 20/80). Solide jaunâtre (28%), $\boldsymbol{R_f}$ = 0.25 (acétate d'éthyle/éther de pétrole = 20/80). **Pf** = 212-214°C. **Infrarouge** (ν, $cm^{-1}$, neat) 3033, 2910, 2777, 2358, 2341, 2196, 1603, 1581, 1539, 1513, 1446, 1434, 1407, 1368, 1352, 1284, 1266, 1233, 1212, 1180, 1132, 1076, 1036, 976. **RMN $^1$H** (400 MHz, DMSO-$d_6$, 20°C) δ 11.75 (sl, 1H), 8.29 (si, 1H), 7.91 (d, *J* = 7.8 Hz, 1H), 7.60 (sl, 1H), 7.39 (d, *J* = 8.6 Hz, 2H), 7.07 (sl, 1H), 6.79 (d, *J* = 8.6 Hz, 2H), 4.61 (dt, *J* = 47.6, 5.0 Hz, 2H), 3.73 (dt, *J*= 26.4, 5.0 Hz, 2H), 3.32 (s, 3H), 2.35 (s, 3H). **RMN $^{13}$C** (101 MHz, DMSO-$d_6$, 20°C) δ 149.5 (Cq), 148.4 (Cq), 144.3 (Cq), 132.9 (2 x CH), 131.9 (Cq), 129.1 (Cq), 127.0 (CH), 118.1 (Cq), 114.3 (CH), 112.4 (2 x CH),108.7 (CH), 97.4 (Cq), 83.2 (d, *J* = 161.5 Hz, $CH_2$) 79.6 (Cq), 51.8 (d, *J* = 19.8 Hz, $CH_2$), 38.9 ($CH_3$), 9.9 ($CH_3$). **RMN $^{19}$F** (376 MHz, DMSO-$d_6$, 20°C) δ. -221.3. **HRMS (+ESI)** calculée pour $C_{19}H_{18}FN_3$ ($M+H^+$) : 308.1557 ; trouvée: 308.1558.

**Exemple 66**: ***N*-(2-fluoroethyl)-*N*-methyl-4-((6-morpholino-1*H*-pyrrolo[2,3-*b*]pyridin-2-yl)ethynyl)aniline 187 :**

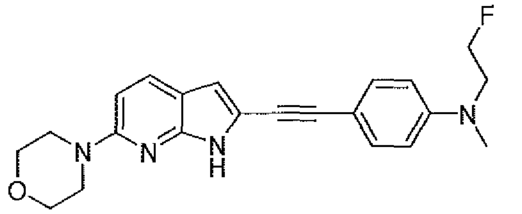

*N*-(2-fluoroethyl)-*N*-methyl-4-((6-morpholino-1*H*-pyrrolo[2,3-*b*]pyridin-2-yl)ethynyl)aniline

La *N*-(2-fluoroéthyl)-*N*-méthyl-4-((6-morpholino-1*H*-pyrrolo[2,3-*b*]pyridin-2-yl)éthynyl)aniline **187** a été synthétisée en 4 étapes. Dans un premier temps, l'alcyne **188** est obtenu en 2 étapes successives en partant de la 6-bromo-2-iodo-1*H*-pyrrolo[2,3-*b*]pyridine **18j** et ensuite une réaction de Buchwald avec une amine. Pour finir, une réaction de déprotection conduit au composé final attendu.

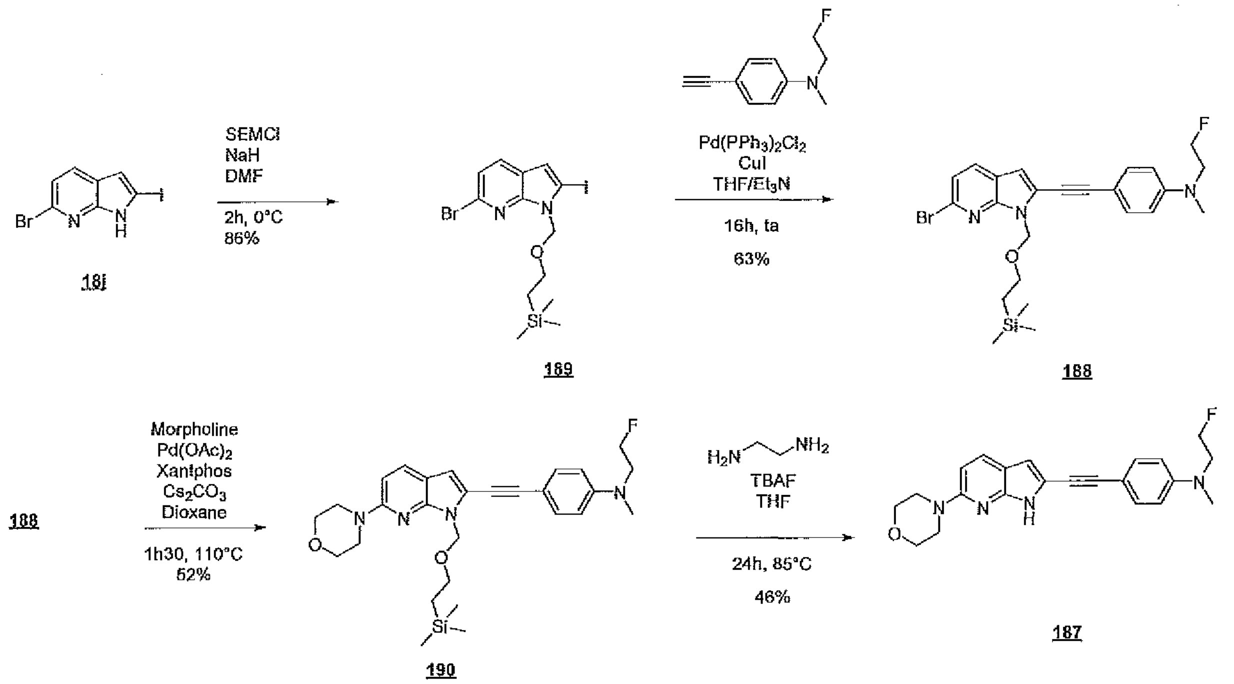


**Exemple 66.1** Synthèse de la 6-bromo-2-iodo-1-((2-(trimethylsilyl)ethoxy)methyl)-1*H*-pyrrolo[2,3-*b*]pyridine **189 :**

Sous argon, la 6-bromo-2-iodo-1*H*-pyrrolo[2,3-*b*]pyridine **18j** (500 mg, 1.55 mmol, 1 éq.) est solubilisée dans 10 mL de DMF. A 0°C, NaH (50% dans l'huile minérale, 89 mg, 1.86 mmol, 1.2 éq.) est ajouté par portion. Après 1h d'agitation, SEMCl (0.33 mL, 1.86 mmol, 1.2 éq.) est additionné goutte à goutte. Au bout de 2h de réaction, 10 mL d'eau sont ajoutés doucement. Les phases organiques sont extraites avec de l'acétate d'éthyle (3 x 10 mL). Les phases organiques réunies ont été lavées au NaCl, séchées sur $MgSO_4$ et concentrées sous pression réduite. Le brut est purifié par colonne de chromatographie flash sur gel de silice (acétate d'éthyle/éther de pétrole = 20/80) pour conduire au composé **189** (603 g, 86%) sous la forme d'une huile incolore. $R_f$ = 0.9 (acétate d'éthyle/éther de pétrole = 20/80). **Infrarouge** (ν, $cm^{-1}$, neat) 2949, 1568, 1463, 1448, 1418, 1394, 1376, 1310, 1259, 1246, 1217, 1202, 1119, 1110, 1076, 992, 969, 943, 915, 855. **RMN $^1$H** (400 MHz, $CDCl_3$, 20°C) δ 7.65 (d, *J* = 8.1 Hz, 1H), 7.20 (d, *J* = 8.1 Hz, 1H), 6.82 (s, 1H), 5.68 (s, 2H), 3.63 - 3.54 (m, 2H), 0.98 - 0.88 (m, 2H), 0.0 (s, 9H). **RMN $^{13}$C** (101 MHz, $CDCl_3$, 20°C) δ 148.5 (Cq), 135.1 (Cq), 129.2 (CH), 120.6 (CH), 112.2 (CH), 91.6 (Cq), 83.8 (Cq), 73.1 ($CH_2$), 66.4 ($CH_2$), 17.8 ($CH_2$), -1.46 (3 x $CH_3$). **HRMS (+ESI)** calculée pour $C_{13}H_{18}BrIN_2OSi$ ($M+H^+$) : 452.9489 trouvée: 452.9487.

**Exemple 66.2** Synthèse de la 4-((6-bromo-1-((2-(trimethylsilyl)ethoxy)methyl)-1*H*-pyrrolo[2,3-*b*]pyridin-2-yl)ethynyl)-*N*-(2-fluoroethyl)-*N*-methylaniline **188** :

Le composé a été préparé selon la **procédure C2** et purifié par colonne de chromatographie flash sur gel de silice (acétate d'éthyle/éther de pétrole = 10/90). Solide jaunâtre (63%) **Pf** 106-108°C, **Rf** = 0.7 (dichlorométhane).

**Infrarouge** (υ, $cm^{-1}$, neat) 2891, 2198, 1607, 1557, 1538, 1510, 1447, 1417, 1360, 1293, 1266, 1243, 1223, 1186, 1130, 1114, 1072, 1044, 979, 933, 895. **RMN $^1$H** (400 MHz, $CDCl_3$, 20°C) δ 7.68 (d, *J* = 7.9 Hz, 1H), 7.44 (d, *J* = 8.7 Hz, 2H), 7.25 (d, *J* = 7.9 Hz, 1H), 6.76 - 6.62 (m, 3H), 5.77 (s, 2H), 4.62 (dt, *J* = 47.1, 5.1 Hz, 2H), 3.78 - 3.61 (m, 4H), 3.07 (s, 3H), 1.01 - 0.91 (m, 2H), 0.00 (s, 9H). **RMN $^{13}$C** (101 MHz, $CDCl_3$, 20°C) δ 149.2 (Cq), 147.7 (Cq), 135.9 (Cq), 133.0 (2 x CH), 130.3 (CH), 123.6 (Cq), 120.7 (CH), 118.9 (Cq), 111.7 (2 x $CH_2$), 109.2 (Cq), 105.8 (CH), 98.0 (Cq), 81.60 (d, *J* = 170.2 Hz, $CH_2$), 78.2 (Cq), 71.1 ($CH_2$), 66.6 ($CH_2$), 52.35 (d, *J* = 21.1 Hz, $CH_2$), 39.0 ($CH_3$), 17.8 ($CH_2$), -0.00 (3 x $CH_3$). **RMN $^{19}$F** (376 MHz, DMSO-$d_6$, 20°C) δ. -222.3. **HRMS (+ESI)** calculée pour $C_{24}H_{29}BrFN_3OSi$ ($M+H^+$) : 502.1320, trouvée: 502.1318.

**Exemple 66.3** Synthèse de la *N*-(2-fluoroethyl)-*N*-methyl-4-((6-morpholino-1-((2-(trimethylsilyl)ethoxy)methyl)-1*H*-pyrrolo[2,3-*b*]pyridin-2-yl)ethynyl)aniline **190** :

Sous argon, le $Pd(OAc)_2$ (0.05 éq.) et le Xantphos (0.075 mmol) sont ajoutés à une solution de dioxane anhydre dégazée de concentration 0.05 M contenant le 4-((6-bromo-1-((2-(triméthylsilyl)éthoxy)méthyl)-1*H*-pyrrolo[2,3-*b*]pyridin-2-yl)éthynyl)-*N*-(2-fluoroéthyl)-*N*-méthylaniline **188** (1.0 éq.), la morpholine (1.0 éq.) et $CsCO_3$ (1.2 éq.). Au bout de 1h30 à 110°C, le solvant est évaporé sous pression réduite. Ensuite, le mélange réactionnel est concentré sous pression réduite, avant d'être purifié par colonne de chromatographie flash sur gel de silice (acétate d'éthyle/éther de pétrole = 20/80) pour conduire au composé **190** (21 mg, 52%) sous la forme d'un solide verdâtre. $R_f$ = 0.1 (dichlorométhane). **Pf :** 110-112°C. **Infrarouge** (v, $cm^{-1}$, neat) 2952, 2890, 2851, 1602, 1568, 1538, 1507, 1489, 1443, 1431, 1403, 1374, 1338, 1324, 1293, 1246; 1229, 1190, 1156, 1120, 1104, 1071, 1046, 980, 941, 907. **RMN $^1$H** (400 MHz, $CDCl_3$, 20°C) δ 8.19 (s, 1H), 7.49 - 7.37 (m, 3H), 6.75 - 6.60 (m, 3H), 5.77 (s, 2H), 4.62 (dt, *J* = 47.1, 5.1 Hz, 2H), 3.98 - 3.87 (m, 4H), 3.76 - 3.63 (m, 4H), 3.19 - 3.12 (m, 3H), 3.07 (s, 3H), 0.92 (m 2H), -0.08 (s, 9H). **RMN $^{13}$C** (101 MHz, $CDCl_3$, 20°C) δ 154.8 (Cq), 149.0 (Cq), 144.2 (Cq), 143.5 (Cq), 137.7 (CH), 132.9 (2 x CH), 123.8 (Cq), 120.2 (Cq), 115.6 (CH), 111.8 (2 x CH), 109.5 (Cq), 105.2 (CH), 97.5 (Cq), 81.60 (d, *J* = 170.2 Hz, $CH_2$), 78.7 (Cq), 71.2 ($CH_2$), 67.0 (2 x $CH_2$), 66.2 ($CH_2$), 52.5 (d, *J* = 21.1 Hz, $CH_2$), 51.6 (2 x $CH_2$), 39.0 ($CH_3$), 17.8 ($CH_2$), -0.00 (3 x $CH_3$). **HRMS (+ESI)** calculée pour $C_{28}H3_7FN_4O_2Si$ ($M+H^+$) : 509.2739, trouvée: 509.2743

**Exemple 66.4** Synthèse de la *N*-(2-fluoroethyl)-*N*-methyl-4-((6-morpholino-1*H*-pyrrolo[2,3-*b*]pyridin-2-yl)ethynyl)aniline **187:**

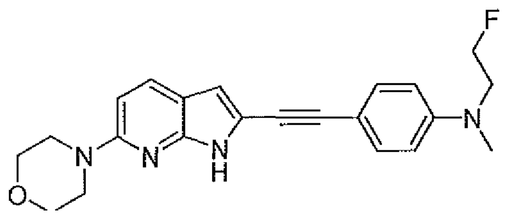

$C_{22}H_{23}FN_4O$
M W: 378,45 gmol-1

A température ambiante et sous argon, une solution de TBAF dans le THF (C = 1 M, 0.19 mL, 0.19 mmol, 5.0 éq.) est ajoutée goutte à goutte au milieu réactionnel contenant la *N*-(2-fluoroéthyl)-*N*-méthyl-4-((6-morpholino-1-((2-(triméthylsilyl)éthoxy)méthyl)-1*H*-pyrrolo [2,3-*b*]pyridin-2-yl)éthynyl)aniline **190** (20mg, 0.04 mmol, 1.0 éq.) et l'éthylènediamine (0.004 mL, 0.06 mmol, 1.2 éq.) diluée dans 2 mL de THF. Au bout de 24 h à 80°C, le mélange réactionnel est refroidi et 10 mL d'eau suivis de 10 mL d'acétate d'éthyle sont ajoutés successivement. Les phases organiques sont extraites 3 fois avec de l'acétate d'éthyle. Puis, elles sont réunies, séchées avec du $MgSO_4$, filtrées sur coton et évaporées sous pression réduite. Pour finir, le brut est purifié par colonne de chromatographie flash sur gel de silice avec comme éluant (acétate d'éthyle/éther de pétrole = 20/80) pour conduire au composé final (7 mg, 46%) sous la forme un solide verdâtre. $R_f$ **=** 0.5 (acétate d'éthyle/éther de pétrole = 20/80). **Pf** >260°C. **Infrarouge** (v, $cm^{-1}$, neat) 3289, 2918, 2851, 1604, 1573, 1539, 1506, 1449, 1424, 1374, 1293, 1257, 1230, 1184, 1136, 1108, 1067, 1055, 1038, 992, 900. RMN **$^1$H** (400 MHz, $CDCl_3$, 20°C) δ 8.39 (s, 1H), 7.71 (d, *J* = 8.8 Hz, 1H), 7.42 (m, 2H), 6.69 (d, *J* = 8.5 Hz, 1H), 6.59 (s, 1H), 4.69 (m, 1H), 4.57 (m, 1H), 3.87 (m, 4H), 3.74 (m, 1H), 3.68 (m, 1H), 3.55 (m, 4H), 3.08 (s, 3H). **RMN $^{13}$C DEPT** (101 MHz, $CDCl_3$, 20°C) δ 131.4 (2 x CH), 130.3 (CH), 111.6 (2 x CH), 106.3 (CH), 101.9 (CH), 81.60 (d, *J* = 170.2 Hz, $CH_2$), 78.7 (Cq), 65.0 (2 x $CH_2$), 52.5 (d, *J* = 21.1 Hz, $CH_2$), 46.4 (2 x $CH_2$), 39.0 ($CH_3$). **HRMS (+ESI)** calculée pour $C_{22}H_{23}FN_4O$ ($M+H^+$): 379.1924, trouvée: 379.1928

**Exemple 67 : 1-(6-bromo-2-((4-(dimethylamino)phenyl)ethynyl)-1*H*-pyrrolo[2,3-*b*]pyridin-1-yl)ethan-1-one 191 :**

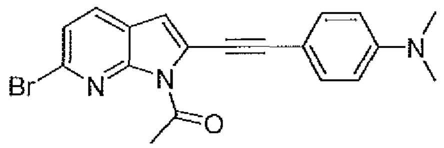

1-(6-bromo-2-((4-(dimethylamino)phenyl)ethynyl)-1*H*-pyrrolo[2,3-*b*]pyridin-1-yl)ethan-1-one

Le 1-(6-bromo-2-((4-(diméthylamino)phenyl)éthynyl)-1*H*-pyrrolo[2,3-*b*]pyridin-1-yl)éthan-1-one **191** a été synthétisée en 2 étapes. Dans un premier temps, le 6-bromo-2-iodo-1*H*-pyrrolo[2,3-b]pyridine **18j** est protégé avec un groupement protecteur de type acétate et pour finir une réaction de Sonogoshira pour conduire au composé final. Ensuite, des tests de radiomarquage au fluor 18 pourront être effectués sur la position 6 du noyau azaindolique pour donner après déprotection le composé **22f** radiomarqué.

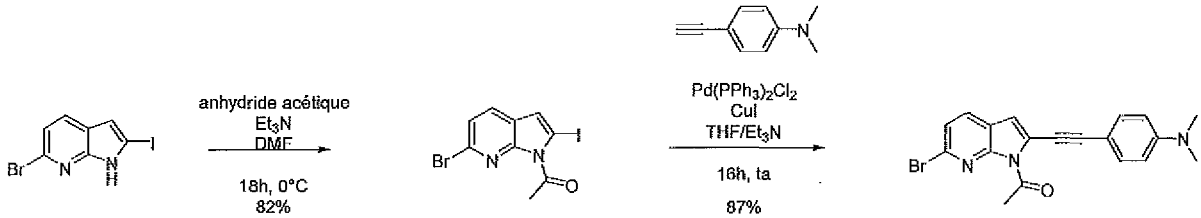


18j

192

191

**Exemple 67.1** Synthèse de la 1-(6-bromo-2-iodo-1*H*-1-pyrrolo[2,3-*b*]pyridin-1-yl)ethan-1-one **192** :

Sous argon, le 6-bromo-2-iodo-1*H*-pyrrolo[2,3-*b*]pyridine **18j** (200 mg, 0.62 mmol, 1 éq.) est solubilisé dans 5 mL de DMF. L'anhydride acétique (0.088 mL, 0.93 mmol, 1.5 éq.) et la triéthylamine (0.129 mL, 0.93 mmol, 1.5 éq) sont ajoutés successivement. Après 18h d'agitation à température, 10 mL d'eau sont ajoutés doucement. Les phases organiques sont extraites avec de l'acétate d'éthyle (3 x 10 mL). Les phases organiques réunies ont été lavées au NaCl, séchées sur $MgSO_4$, filtrées et concentrées sous pression réduite. Le brut est purifié par colonne de chromatographie flash sur gel de silice (dichlorométhane/éther de pétrole = 20/40) pour conduire au composé **192** (186 mg, 82%) sous la forme d'un solide blanc. $R_f$ = 0.6 (dichlorométhane). **Pf :** 126-128°C. **Infrarouge** (v, $cm^{-1}$, neat) 1718, 1590, 1558, 1489, 1429, 1404, 1368, 1294, 1254, 1218, 1205, 1134, 1113, 1090, 1025, 991, 905, 829, 807.. **RMN $^1$H** (400 MHz, $CDCl_3$, 20°C) $\delta$ 7.62 (d, *J* = 8.1 Hz, 1H), 7.34 (d, *J* = 8.1 Hz, 1H), 6.99 (s, 1H), 3.05 (s, 3H). **RMN $^{13}$C DEPT** (250 MHz, $CDCl_3$, 20°C) $\delta$ 129.2 (CH), 122.9 (CH), 119.7 (CH), 27.9 ($CH_3$). **HRMS (+ESI)** calculée pour $C_9H_6BrIN_2O$ ($M+Na^+$) : 386.8600, trouvée: 386.8599

**Exemple 67.2** Synthèse de la 1-(6-bromo-2-((4-(dimethylamino)phenyl)ethynyl)-1*H*-pyrrolo[23-*b*]pyridin-1-yl)ethan-1-one **191** :

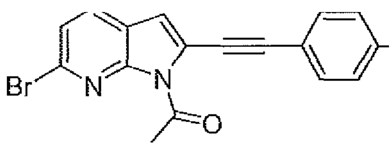

$C_{19}H_{16}BrN_3O$
M W: 382,26 gmol-1

Le composé a été préparé selon la **procédure C2** et purifié par colonne de chromatographie flash sur gel de silice (dichlorométhane/éther de pétrole =50/50). Solide jaunâtre (87%) **Pf** 194-196°C, **Rf** = 0.5 (dichlorométhane/éther de pétrole = 40/60). **Infrarouge** ($\upsilon$, $cm^{-1}$, neat) 2918, 2852, 2188, 1728, 1604, 1561, 1541, 1508, 1448, 1431, 1393, 1364, 1322, 1305, 1290, 1258, 1221, 1185, 1131, 1099, 1067, 1037, 1010, 936, 900. **RMN $^1$H** (250 MHz, $CDCl_3$, 20°C) $\delta$ 7.64 (d, *J* = 8.0 Hz, 1H), 7.45 (d, *J* = 8.9 Hz, 2H), 7.33 (*d*, J = 8.0 Hz, 1H), 6.77 (s, 1H), 6.63 (d, *J* = 8.9 Hz, 2H), 3.03

(s, 3H), 2.98 (s, 6H). **RMN $^{13}C$** (63 MHz, $CDCl_3$, 20°C) δ 169.0 (Cq), 150.5 (Cq), 146.6 (Cq),.135.5 (Cq), 133.0 (2 x CH), 130.2 (CH), 123.0 (CH), 122.7 (Cq), 120.9 (Cq), 111.7 (2 x CH), 111.2 (CH), 109.0 (Cq), 98.9 (Cq), 79.8 (Cq), 40.1 (2 x $CH_3$), 27.4 ($CH_3$). **HRMS (+ESI)** calculée pour $C_{19}H_{16}BrN_3O$ ($M+Na^+$) : 404.0368, trouvée: 404.0369.

**Exemple 68 : *N*-(2-fluoroethyl)-*N*-methyl-4-((5-morpholino-1*H*-pyrrolo[2,3-*b*]pyridin-2-yl)ethynyl)aniline 193** :

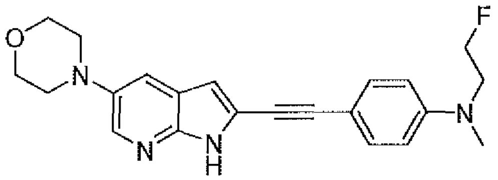

*N*-(2-fluoroethyl)-*N*-methyl-4-((5-morpholino-1*H*-pyrrolo[2,3-*b*]pyridin-2-yl)ethynyl)aniline

La *N*-(2-fluoroéthyl)-*N*-méthyl-4-((6-morpholino-1*H*-pyrrolo[2,3-*b*]pyridin-2-yl)éthynyl) aniline **193** a été synthétisée en 4 étapes. Dans un premier temps, l'alcyne **194** est obtenu en 2 étapes successive en partant du 5-bromo-2-iodo-1*H*-pyrrolo[2,3-*b*]pyridine **18k** puis une réaction de Buchwald avec une amine est réalisée. Pour finir, une réaction de déprotection conduit au composé final attendu.

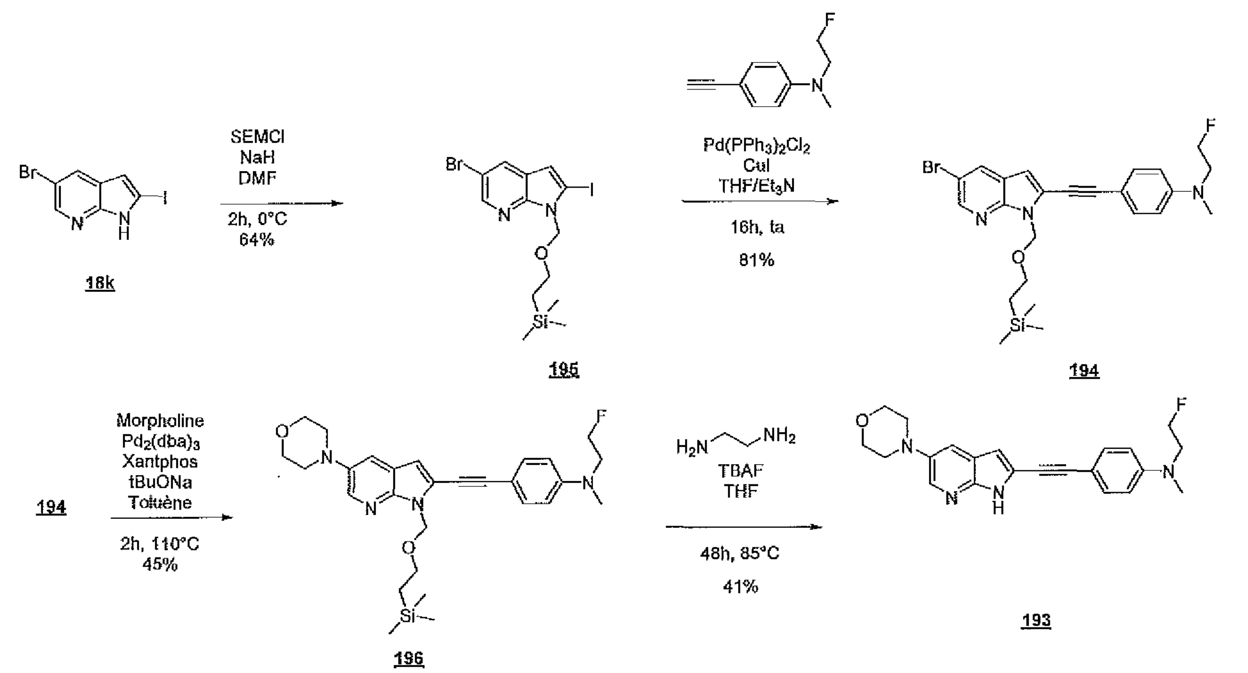

18k 195 194 194 196 193

**Exemple 68.1** Synthèse de la 5-bromo-2-iodo-1-((2-(trimethylsilyl)ethoxyimethyl)-1*H*-pyrrolo[2,3-*b*]pyridine **195** :

Sous argon, le 5-bronio-2-iodo-1*H*-pyrrolo[2,3-*b*]pyridine **18k** (594 mg, 1.84 mmol, 1 éq.) est solubilisé dans 10 mL de DMF. A 0°C, NaH (50% dans l'huile minérale, 105 mg, 2.21 mmol, 1.2 éq.) est ajouté par portion. Après 1h d'agitation, du SEMCl (0.39 mL, 2.21 mmol, 1.2 éq.) est additionné goutte à goutte. Au bout de 2h de réaction, 10 mL d'eau sont ajoutés doucement. Les phases organiques sont extraites avec de l'acétate d'éthyle (3 x 10 mL). Les phases organiques réunies ont été lavées au NaCl, séchées sur $MgSO_4$, filtrées et concentrées sous pression réduite. Le brut est purifié par colonne de chromatographie flash sur gel de silice (acétate d'éthyle/éther de pétrole = 5/95) pour conduire au composé **195** (534 g, 64%) sous la forme d'une huile incolore. $R_f$ = 0.9 (acétate d'éthyle/éther de pétrole = 5/95). **Infrarouge** (ν, $cm^{-1}$, neat) 2949, 1568, 1463, 1448, 1418, 1394, 1376, 1310, 1259, 1246, 1217, 1202, 1119, 1110, 1076, 992, 969, 943, 915, 855. **RMN $^1H$** (400 MHz, $CDCl_3$, 20°C) δ 8.27 (d, *J* = 2.3 Hz, 1H), 7.93 (d, *J* = 2.3 Hz, 1H), 6.77 (s, 1H), 5.68 (s, 2H), 3.63 - 3.54 (m, 2H), 0.98 - 0.88 (m, 2H), 0.00 (s, 9H). **RMN $^{13}C$** (101 MHz, $CDCl_3$, 20°C) δ 147.6 (Cq), 143.6 (CH), 129.1 (CH), 123.3 (Cq), 112.7 (Cq), 111.3 (CH), 91.6 (Cq), 85.8 (Cq), 73.1 ($CH_2$), 66.4 ($CH_2$), 17.8 ($CH_2$), -1.46 (3 x $CH_3$). **HRMS (+ESI)** calculée pour $C_{13}H_{18}BrIN_2OSi$ ($M+H^+$) : 452.9489 trouvée: 452.9487.

**Exemple 68.2** Synthèse de la 4-((5-bromo-1-((2-(trimethylsilvl)ethoxy)methyl)-1*H*-pyrrolo[2,3-*b*]pyridin-2-yl)ethynyl)-*N*-(2-fluoroethyl)-N-methylaniline **194** :

Le composé a été préparé selon la **procédure C2** et purifié par colonne de chromatographie flash sur gel de silice (acétate d'éthyle/éther de pétrole = 10/90). Solide jaunâtre (63%). **Pf** 84-86°C, **Rf =** 0.25 (acétate d'éthyle/éther de pétrole = 20/80). **Infrarouge** (υ, $cm^{-1}$, neat) 2891, 2198, 1607, 1557, 1538, 1510, 1447, 1417, 1360, 1293, 1266, 1243, 1223, 1186, 1130, 1114, 1072, 1044, 979, 933, 895. **RMN $^1$H** (400 MHz, $CDCl_3$, 20°C) δ 8.37 (d, *J* = 2.3 Hz, 1H), 7.97 (d, *J* = 2.3 Hz, 2H), 7.44 (d, *J* = 7.9 Hz, 1H), 6.76 - 6.62 (m, 3H), 5.78 (s, 2H), 4.62 (dt, *J* = 47.1, 5.1 Hz, 2H), 3.78 - 3.61 (m, 4H), 3.00 (s, 3H), 1.01 - 0.91 (m, 2H), 0.00 (s, 9H). **RMN $^{13}$C** (101 MHz, $CDCl_3$, 20°C) δ 149.2 (Cq), 146.6 (Cq), 144.5 (CH), 133.0 (2 x CH), 130.2 (CH), 124.9 (Cq), 121.8 (Cq), 112.8 (Cq), 112.8 (2 x $CH_2$), 109.0 (Cq), 1054.9 (CH), 98.0 (Cq), 81.60 (d, *J* = 170.2 Hz, $CH_2$), 78.2 (Cq), 71.1 ($CH_2$), 66.6 ($CH_2$), 52.35 (d, *J* = 21.1 Hz, $CH_2$), 39.0 ($CH_3$), 17.8 ($CH_2$), -0.00 (3 x $CH_3$). **RMN $^{19}$F** (376 MHz, DMSO-$d_6$, 20°C) δ. -222.3. **HRMS (+ESI)** calculée pour $C_{24}H_{29}BrFN_3OSi$ (M+H$^+$): 502.1320, trouvée: 502.1318.

**Exemple 68.3** Synthèse du *N*-(2-fluoroethyl)-*N*-methyl-4-((5-morpholino-1-((2-(trimethylsilyl)ethoxy)methyl)-1*H*-pyrrolo[2,3-*b*]pyridin-2-yl)ethynyl)aniline **196:**

Sous argon, le $Pd_2(dba)_3$ (2 mg, 0.002 mmol, 0.02 éq.) et le Xantphos (4 mg, 0.006 mmol, 0.06 éq.) sont ajoutés à une solution de toluène anhydre dégazée de concentration 0.1 M contenant le 4-((5-bromo-1-((2-(triméthylsilyl)éthoxy)méthyl)-1*H*-pyrrolo[2,3-*b*]pyridin-2-yl)éthynyl)-*N*-(2-fluoroéthyl)-N-méthylaniline **188** (50 mg, 0.1 mmol, 1.0 éq.), la morpholine (0.012 mL, 0.12 mmol, 1.2 éq.) et le *t*BuONa (14 mg, 0.15 mmol, 1.5 éq.). Au bout de 2h à 110°C, le solvant est évaporé sous pression réduite. Ensuite, le mélange réactionnel est concentré sous pression réduite, avant d'être purifié par colonne de chromatographie flash sur gel de silice (acétate d'éthyle/éther de pétrole = 20/80) pour conduire au composé **196** (23 mg, 45%) sous la forme d'une huile verdâtre. $R_f$ = 0.25 (acétate d'éthyle/éther de pétrole = 20/80). **Infrarouge** (v, $cm^{-1}$, neat) 2952, 2890, 2851, 1602, 1568, 1538, 1507, 1489, 1443, 1431, 1403, 1374, 1338, 1324, 1293, 1246, 1229, 1190, 1156, 1120, 1104, 1071, 1046, 980, 941, 907. **RMN $^1$H** (400 MHz, $CDCl_3$, 20°C) δ 8.19 (s, 1H), 7.43 (d, *J* = 8.6 Hz, 2H), 7.39 (s, 1H), 6.72 - 6.61 (m, 3H), 5.77 (s, 2H), 4.62 (dt, *J* = 47.2, 5.1 Hz, 2H), 3.94 - 3.88 (m, 4H), 3.73 (m, 4H), 3.14 (m, 4H), 3.07 (s, 3H), 0.97 - 0.89 (m, 2H), -0.08 (s, 9H). **RMN $^{13}$C** (101MHz, $CDCl_3$, 20°C) δ 148.9 (Cq), 144.00 (Cq), 143.4 (Cq), 137.5 (CH), 132.8 (2 x CH), 123,6 (Cq), 120.0 (Cq), 115.5 (CH), 111.6 (2 x $CH_2$), 109.3 (Cq), 105.0 (CH), 97.3 (Cq), 81.60 (d, *J* = 170.2 Hz, $CH_2$), 78.2 (Cq), 71.0 ($CH_2$), 66.9 (2 x $CH_2$), 66.1 ($CH_2$), 52.3 (d, *J* = 21.1 Hz, $CH_2$), 52.1 (2 x $CH_2$), 38.9 ($CH_3$), 17.7 ($CH_2$), -0.00 (3 x $CH_3$). **RMN $^{19}$F** (376 MHz, $CDCl_3$, 20°C) δ. -222.0 **HRMS (+ESI)** calculée pour $C_{28}H3_7FN_4O_2Si$ (M+H$^+$) : 509.2739, trouvée: 509.2743

**Exemple 68.4** Synthèse de la *N*-(2-fluoroethyl)-*N*-methyl-4-((5-morpholino-1*H*-pyrrolo[2,3-*b*]pyridin-2-yl)ethynyl)aniline **193:**

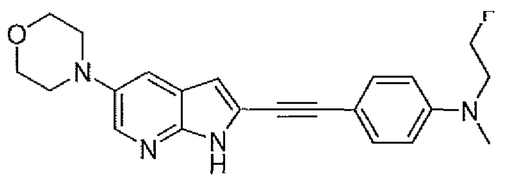

$C_{22}H_{23}FN_4O$
M W: 378,45 gmol-1

A température ambiante et sous argon, une solution de TBAF dans le THF (C = 1 M, 0.23 mL, 0.23 mmol, 5.0 éq.) est ajouté goutte à goutte au milieu réactionnel contenant la *N*-(2-fluoroéthyl)-*N*-méthyl-4-((6-morpholino-1-((2-(triméthylsilyl)éthoxy)méthyl)-1*H*-pyrrolo[2,3-*b*]pyridin-2-yl)éthynyl)aniline **190** (23mg, 0.045 mmol, 1.0 éq.) et l'éthylènediamine (0.005 mL, 0.07 mmol, 1.2 éq.) diluée dans 2 mL de THF. Au bout de 48 h à 80°C, le mélange réactionnel est refroidi et 10 mL d'eau puis 10 mL d'acétate d'éthyle sont ajoutés successivement. Les phases organiques sont extraites 3 fois avec de l'acétate d'éthyle (30 mL), réunies, séchées avec du $MgSO_4$, filtrées sur coton et évaporées sous pression réduite. Pour finir, le brut est purifié par colonne de chromatographie flash sur gel de silice avec comme éluant (acétate d'éthyle/éther de pétrole = 30/70) pour conduire au composé final (7 mg, 41%) sous la forme un solide verdâtre. $R_f$ = 0.1 (acétate d'éthyle/éther de pétrole = 30/70). **Pf**>260°C. **Infrarouge** (v, $cm^{-1}$, neat) 3289, 2918, 2851, 1604, 1573, 1539, 1506, 1449, 1424, 1374, 1293, 1257, 1230, 1184, 1136, 1108, 1067, 1055, 1038, 992, 900 . **RMN $^1$H** (400 MHz, $CDCl_3$, 20°C) δ 9.01 (s, 1H), 8.15 (s, 1H), 7.45 (m, 3H), 6.67 (m, 3H), 4.62 (m, 2H), 3.93 (m, 4H), 3.70 (m, 2H), 3.16 (m, 4H), 3.07 (s, 3H). **RMN $^{13}$C** (101 MHz, $CDCl_3$, 20°C) δ 149.1 (Cq), 143.9 (Cq), 143.8 (Cq), 137.3 (CH), 132.9 (2 x CH),

123.4 (Cq), 121.7 (Cq), 116.0 (CH), 111.8 (2 x $CH_2$), 109.3 (Cq), 105.4 (CH), 94.8 (Cq), 81.60 (d, *J* = 170.2 Hz, $CH_2$), 78.2 (Cq), 67.1 (2 x $CH_2$), 52.3 (d, *J* = 21.1 Hz, $CH_2$), 51.6 (2 x $CH_2$), 38.9 ($CH_3$). **RMN $^{19}$F** (376 MHz, $CDCl_3$, 20°C) δ. **-222.HRMS (+ESI)** calculée pour $C_8H_7IN_2$ (M+$H^+$) : 379.1929, trouvée: 379.1928

**Exemple 69 : 4-(2-((4-((2-fluoroethyl)(methyl)amino)phenyl)ethynyl)-1-((2-(trimethylsilyl)ethoxy)methyl)-1*H*-pyrrolo[2,3-*b*]pyridin-5-yl)benzonitrile 198 :**

La 4-((5-bromo-1-((2-(triméthylsilyl)éthoxy)méthyl)-1*H*-pyrrolo[2,3-*b*]pyridin-2-yl)éthynyl)-*N*-(2-fluoroéthyl)-N-méthylaniline **198** est obtenu au départ de **194** par une réaction de Suzuki avec l'acide boronique correspondant. Une déprotection du dérivé silylé pourra donner le produit final déprotégé **197.**

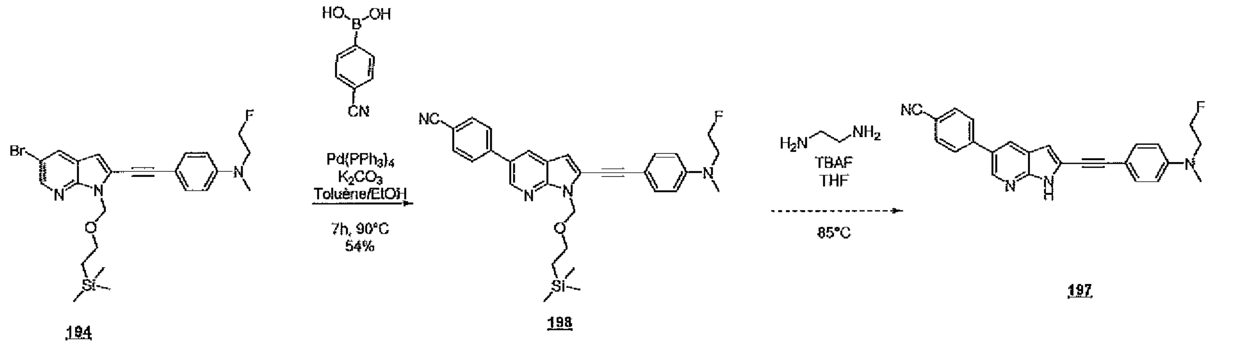


194 198 197

Sous argon, la 4-((5-bromo-1-((2-(triméthylsilyl)éthoxy)méthyl)-1*H*-pyrrolo[2,3-*b*]pyridin-2-yl)éthynyl)-*N*-(2-fluoroéthyl)-*N*-méthylaniline **194** (25 mg, 0.05 mmol, 1 éq.) est dissoute dans un mélange Toluène/EtOH (1/1) puis l'addition de l'acide 4-cyanophénylboronique (9 mg, 0.06 mmol, 1.2 éq.) et de $K_2CO_3$ (21 mg, 0.15 mmol, 3 éq.) est réalisée. Ensuite, le milieu réactionnel est dégazé pendant 10 minutes avant d'ajouter du [Pd($PPh_3$)$_4$] (5.8 mg, 0.005 mmol, 0.1 éq.). Le mélange réactionnel est chauffé à 90°C pendant 7 h. Après refroidissement, les solvants sont évaporés sous pression réduite. Pour finir, le brut est purifié par colonne de chromatographie flash sur gel de silice avec comme éluant (acétate d'éthyle/éther de pétrole = 20/80) pour conduire au composé final (14 mg, 54%) sous la forme un solide jaunâtre. $R_f$ = 0.2 (dichlorométhane). **Pf :** 158-160°C. **Infrarouge** (v, $cm^{-1}$, neat) 2949, 2224, 2197, 1606, 1541, 1514, 1474, 1450, 1422, 1377, 1353, 1317, 1291, 1261, 1247, 1215, 1196, 1182, 1135, 1114, 1097, 1066, 1047, 982, 941, 897, 857. **RMN $^1$H** (400 MHz, $CDCl_3$, 20°C) 8.59 (d, *J* = 1.6 Hz, 1H), 8.04 (d, *J* = 1.6 Hz, 1H),), 7.75 (m, 4H), 7.45 (d, *J* = 8.1 Hz, 2H), 6.79 (s, 1H), 6.69 (d, *J* = 8.1 Hz, 2H), 5.85 (s, 2H), 4.63 (dt, *J* = 47.2, 5.1 Hz, 2H), 3.71 (m, 4H), 3.08 (s, 3H), 0.97 - 0.89 (m, 2H), -0.08 (s, 9H). **RMN $^{13}$C** (101 MHz, $CDCl_3$, 20°C) δ 149.2 (Cq), 148.3 (Cq), 144.1 (Cq), 143.2 (CH), 133.0 (2 x CH), 132.8 (2 x CH), 128.7 (Cq), 127.8 (2 x CH), 126.8 (CH), 124.8 (Cq), 120.0 (Cq), 118.8 (Cq), 111.8 (2 x CH), 110.7 (Cq), 109.1 (Cq), 105.9 (CH), 98.4 (Cq), 81.60 (d, *J* = 170.2 Hz, $CH_2$), 78.3 (Cq), 71.0 ($CH_2$), 66.5 ($CH_2$), 52.3 (d, *J* = 21.1 Hz, $CH_2$), 38.9 ($CH_3$), 17.8 ($CH_2$), -0.00 (3 x $CH_3$). **HRMS (+ESI)** calculée pour $C_{31}H_{33}FN_4OSi$ (M+$H^+$) : 398.9658, trouvée: 398.9657

**Exemple 70: *N,N*-dimethyl-4-((5-(4,4,5,5-tetramethyl-1,3,2-dioxaborolan-2-yl)-1*H*-pyrrolo[2,3-*b*]pyridin-2-yl)ethynyl)aniline 199 :**

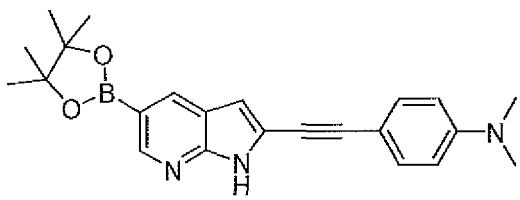

La *N,N*-diméthyl-4-((5-(4,4,5,5-tetraméthyl-1,3,2-dioxaborolan-2-yl)-1*H*-pyrrolo[2,3-*b*]pyridin-2-yl)éthynyl)aniline **193** a été synthétisée en 4 étapes. Dans un premier temps, l'alcyne **201** est obtenu en 2 étapes successives en partant du 5-bromo-2-iodo-1*H*-pyrrolo[2,3-*b*]pyridine **18k** puis une réaction Sonogashira avec l'alcyne correspondant est réalisée et enfin . une réaction de déprotection conduit au composé final attendu. Celui-ci sera un précurseur en vue

d'une utilisation pour radiomarquage.

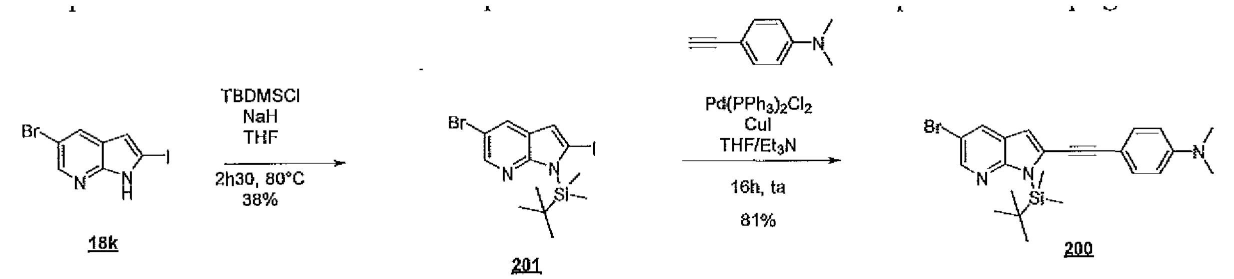


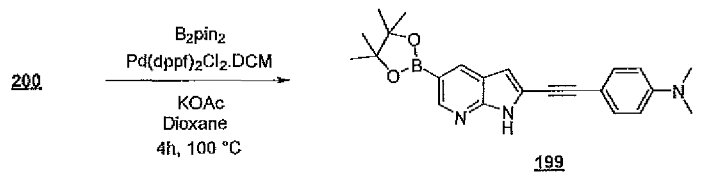


**Exemple 70.1** Synthèse de la 5-bromo-1-(*tert*-butyldimethylsilyl)-2-iodo-1*H*-pyrrolo[2,3-*b*]pyridine **201** :

Sous argon, la 5-bromo-2-iodo-1*H*-pyrrolo[2,3-*b*]pyridine **18k** (230 mg, 0.71 mmol, 1 éq.) est solubilisée dans 10 mL de THF. A 0°C, du NaH (50% dans l'huile minérale, 41 mg, 0.86 mmol, 1.2 éq.) est ajouté par portion. Après 30h d'agitation à température, le mélange est remis à 0°C et le TBDMSCl (129 mg, 0.86 mmol, 1.2 éq.) est additionné par portion. Ensuite, le mélange est mis à 80 °C pendant 2h30. Après refroidissement à 0°C, 10 mL d'eau sont ajoutés doucement et les phases organiques sont extraites avec de l'acétate d'éthyle (3 x 10 mL). Les phases organiques réunies ont été lavées au NaCl, séchées sur $MgSO_4$, filtrées et concentrées sous pression réduite. Le brut est purifié par colonne de chromatographie flash sur gel de silice (acétate d'éthyle/éther de pétrole = 5/95) pour conduire au composé **201** (117 g, 38%) sous la forme d'une huile incolore. $R_f$ = 0.9 (acétate d'éthyle/éther de pétrole = 5/95). **Infrarouge** (ν, $cm^{-1}$, neat) 2951, 2925, 2854, 1587, 1541, 1484, 1460, 1444, 1353, 1339, 1307, 1253, 1215, 1175, 1114, 1066, 997, 917, 846. **RMN $^1$H** (250 MHz, $CDCl_3$, 20°C) δ 8.16 (d, *J* = 2.3 Hz, 1H), 7.82 (d, *J* = 2.3 Hz, 1H), 6.80 (s, 1H), 0.94 (s, 9H), 0.81 (s, 6H). **RMN $^{13}$C DEPT** (63 MHz, $CDCl_3$, 20°C) δ 142.2 (CH), 127.7 (CH), 116,7 (CH), 27.1 (3 x $CH_2$), -0.65 (2 x $CH_3$). **HRMS (+ESI)** calculée pour $C_{13}H_{18}BrIN_2Si$ ($M+H^+$) : 436.9540 trouvée: 436.9538.

**Exemple 70.2** Synthèse de la 4-((5-bromo-1-(tert-butyldimethylsilyl)-1*H*-pyrrolo[2,3-*b*]pyridin-2-yl)ethynyl)-*N,N*-dimethylaniline **200** :

Le composé a été préparé selon la **procédure C2** et purifié par colonne de chromatographie flash sur gel de silice (dichlorométhane/éther de pétrole = 20/80). Solide amorphe jaunâtre (62%), **Rf** = 0.5 (acétate d'éthyle/éther de pétrole = 20/80). **Infrarouge** (υ, $cm^{-1}$, neat) 2952, 2890, 2851, 1603, 1568, 1538, 1507, 1489, 1443, 1431, 1403, 1374, 1338, 1324, 1293, 1246, 1229, 1191, 1157, 1120, 1104, 1071, 1046, 980, 942, 907. **RMN $^1$H** (400 MHz, $CDCl_3$, 20°C) δ 8.28 (d, *J* = 2.3 Hz, 1H), 7.91 (d, *J* = 2.3 Hz, 2H), 6.76 - 6.62 (m, 3H), 3.00 (s, 6H), 0.99 (s, 9H), 0.82 (s, 6H). **RMN $^{13}$C DEPT** (101 MHz, $CDCl_3$, 20°C) δ 143.4 (CH), 132.4 (2 x CH), 128.9 (CH), 111.9 (2 x $CH_2$), 109.7 (CH), 40.2 (2 x $CH_3$), 26.7 (3 x $CH_3$), -1.58 (2 x $CH_3$). **HRMS (+ESI)** calculée pour $C_2H_{28}BrN_3Si$ ($M+H^+$) : 454.1308, trouvée: 454.1309.

**Exemple 70.3** Synthèse de la *N,N*-dimethyl-4-((5-(4,4,5,5-tetramethyl-1,3,1-dioxaborolan-2-yl)-1*H*-pyrrolo[2,3-*b*]pyridin-2-yl)ethynyl)aniline **199** :

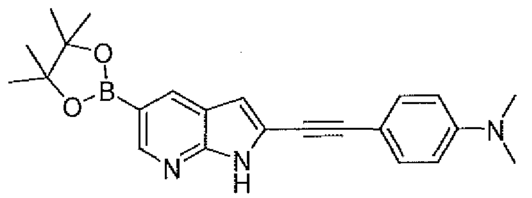

$C_{23}H_{26}BN_3O_2$
M W/ 387,29 gmol-1

Sous argon, la 4-((5-bromo-1-(tert-butyldiméthylsilyl)-1*H*-pyrrolo[2,3-*b*]pyridin-2-yl)éthynyl)-*N,N*-diméthylaniline **200** (50 mg, 0.11 mmol, 1.0 éq.) est dissoute dans le dioxane (1 mL) puis l'addition du bis(pinacolato)diboron (56 mg, 0.22 mmol, 2.0 éq.) et de KOAc (32 mg, 0.33 mmol, 3.0 éq.) est réalisée. Ensuite, le milieu réactionnel est dégazé pendant 10 minutes avant d'ajouter du $Pd(dppf)_2Cl_2$.DCM(9.0 mg, 0.011 mmol, 0.1 éq.). Le mélange réactionnel est chauffé à 100°C pendant 4 h. Après refroidissement, le solvant est évaporé sous pression réduite. Pour finir, le brut est purifié par colonne de chromatographie flash sur gel de silice avec comme éluant (dichlorométhane/éther de pétrole = 30/70) pour conduire à l'intermédiaire. Ensuite, le brut est directement solubilisé dans 5 mL de THF. A 0°C, une solution de TBAF dans le THF (C = 1 M, 0.05 mL, 0.05 mmol, 1.2 éq.) est ajouté goutte à goutte. Au bout de 40 minutes à 0°C, 10 mL d'eau et 10 mL d"acétate d'éthyle sont ajoutés successivement. Les phases organiques sont extraites 3 fois avec de l'acétate d'éthyle, réunies, séchées avec du $MgSO_4$, filtrées sur coton et évaporées sous pression réduite. Le brut est purifié par colonne de chromatographie flash sur gel de silice (acétate d'éthyle/éther de pétrole = 20/80) pour conduire au composé **199** (10 mg, 64%) sous la forme d'un solide jaune. $R_f$ = 0.1 (acétate d'éthyle/éther de pétrole = 10/90). **Pf** >260°C. **Infrarouge** (ν, $cm^{-1}$, neat) 3291, 2962, 2930, 2853, 2102, 1606, 1511, 1463, 1434, 1373, 1356, 1336, 1273, 1245, 1227, 1185, 1141, 1093, 1023, 935. **RMN $^1$H** (400 MHz, DMSO-$d_6$, 20°C) δ 12.20 (s, 1H), 8.46 (s, 1H), 8.18 (s, 1H), 7.39 (d, *J* = 8.6 Hz, 2H), 6.74 (m, 3H), 2.97 (s, 6H), 1.32 (s, 12H). **RMN $^{13}$C** (63 MHz, $CDCl_3$, 20°C) δ 150.9 (Cq), 150.3 (Cq), 149.8 (Cq), 135.2 (CH), 133.0 (2 x CH), 120.9 (Cq), 120.9 (Cq), 119.9 (Cq), 112.5 (2 x CH), 110.7 (Cq), 107.9 (Cq), 106.0 (CH), 95.4 (Cq), 84.1 (2 x Cq), 80.6 (Cq), 40.2 (2 x $CH_3$), 25.2 (4 x $CH_3$). **HRMS (+ESI)** calculée pour $C_{23}H_{26}BN_3O_2$ ($M+H^+$): 388.2190, trouvée: 388.2193.

**Exemple 71 : *N,N*-dimethyl-4-((4-(4,4,5,5-tetramethyl-1,3,2-dioxaborolan-2-yl)1*H*-pyrrolo[2,3-*b*]pyridin-2-yl)ethynyl)aniline 202** :

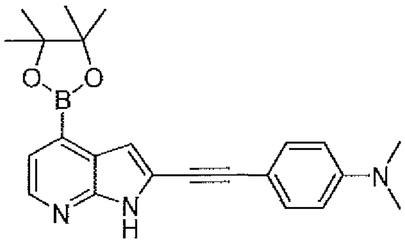

La *N,N*-diméthyl-4-((4-(4,4,5,5-tetraméthyl-1,3,2-dioxaborolan-2-yl)-1*H*-pyrrolo[2,3-b]pyridin-2-yl)éthynyl)aniline **202** a été synthétisée en 6 étapes. Dans un premier temps, l'alcyne **204** est obtenu en 5 étapes successives en partant de la 4-bromo-1*H*-pyrrolo[2,3-*b*]pyridine puis une réaction Sonogashira avec l'alcyne correspondant est réalisée et enfin une réaction de déprotection conduit au composé final attendu. Les produits **207** et **202** sont des précurseurs en vue du radiomarquage.

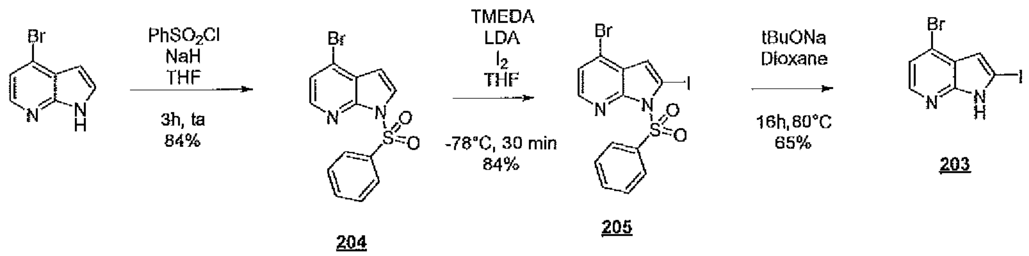


204

205

203

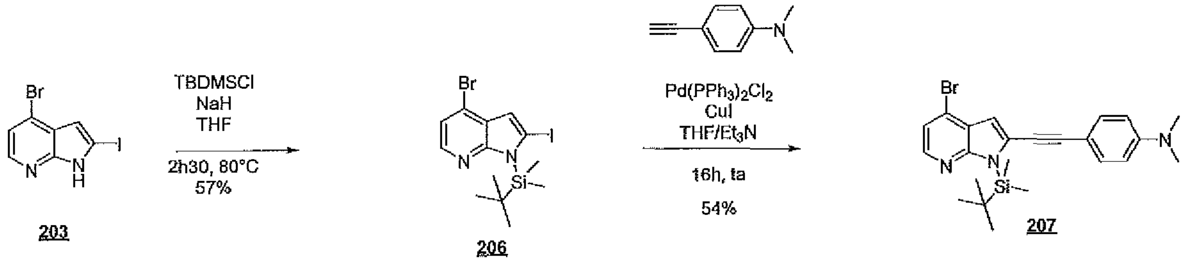


203

206

207

204

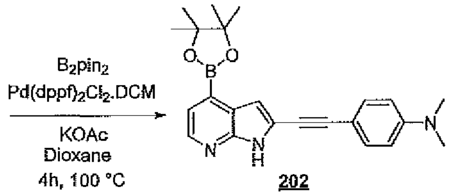


202

**Exemple 71.1** Synthèse de la 4-bromo-1-(phenylsulfonyl)-1*H*-pyrrolo[2,3-*b*]pyridine **205** :

A une solution de NaH (1.46 g, 30.4 mmol, 1.2 éq.) dans du THF sous argon et à 0°C a été additionnée lentement une solution de 4-bromo-7-azaindole (5g, 25.4 mmol, 1.0 éq.) dans du THF (1 M). Le mélange est agité à température ambiante pendant 30 min. De retour à 0°C, le chlorure de benzènesulfonyle (3.5 mL, 27.9 mmol, 1.1 éq.) a été ajouté goutte-à-goutte. Après 3h d'agitation à température ambiante, le mélange a été versé dans un becher rempli avec de la glace. Une fois le milieu revenu à la température ambiante, les phases ont été séparées. La phase aqueuse a été extraite à l'acétate d'éthyle. Les phases organiques réunies ont été lavées au NaCl, séchées sur $MgSO_4$ et concentrées sous pression réduite. Le brut est trituré avec du pentane et filtration sous vide pour obtenir le composé 205 (7.2 g, 84%) sous la forme d'un solide brun. **RMN $^1$H** (400 MHz, $CDCl_3$, 20°C) δ 8.18 (d, *J* = 7.8 Hz, 3H), 7.52 (m, 4H), 7.30 (m, 1H), 7.02 (s, 1H). Numéro CAS : 889939-25-7

**Exemple 71.2** Synthèse de la 4-bromo-2-iodo-1-(phenylsulfonyl)-1*H*-pyrrolo[2,3-*b*]pyridine **205** :

Sous atmosphère d'argon, à une solution de 4-bromo-1-(phénylsulfonyl)-1*H*-pyrrolo[2,3-*b*]pyridine 205 (7 g, 0.99 mmol, 1.0 éq.) et de TMEDA (3.42 mL, 22.8 mmol, 1.1 éq.) dans du THF (0.1M), refroidie à -78°C, a été additionnée une solution de LDA (2M dans du THF, 12.5 mL, 24.8 mmol, 1,2 éq.) goutte-à-goutte. Après 30 min, une solution d'iode (11.5 g, 45.5 mmol, 2.2 éq.) dans THF (0.5M) a été ajoutée lentement *via* une cannule au milieu réactionnel. Au bout de 30 min d'agitation à -78°C, 20 mL d'eau sont additonnés et le milieu réactionnel est remis à température ambiante doucement. Les phases organiques sont extraites avec de l'acétate d'éthyle (3 fois 20 mL), réunies, séchées sur $MgSO_4$, filtrées puis concentrée sous pression réduite. Le brut est purifié par colonne de chromatographie flash sur gel de silice (acétate d'éthyle/éther de pétrole = 20/80) pour conduire au composé 205 (8.1 g, 76%) sous la forme d'un solide brun. $R_f$ = 0.10 (acétate d'éthyle/éther de pétrole = 20/80). **Pf** = 144-146°C. **Infrarouge** (ν, $cm^{-1}$, neat) 3383, 2359, 1635, 1588, 1567, 1485, 1449, 1430, 1370, 1333, 1275, 1235, 1207, 1175, 1129, 1114, 1087, 1011, 918, 826, 755, 723, 697, 684, 623, 585, 557, 537, 502. **RMN $^1$H** (250 MHz, $CDCl_3$, 20°C) δ 8.19 (t, *J* = 6.5 Hz, 3H), 7.65 - 7.44 (m, 3H), 7.32 (d, *J* = 5.5 Hz, 1H), 7.05 (s, 1H). **RMN $^{13}$C** (63 MHz, $CDCl_3$, 20°C) δ 149.1 (Cq), 144.7 (CH), 138.3 (Cq), 134.4 (CH), 129.1 (2 x CH), 128.2 (2 x CH), 125.3 (2 x Cq), 123.7 (Cq), 122.5 (CH), 119.6 (CH). **HRMS (+ESI)** calculée pour $C_{13}H_9BrIN_2O_2S$ ($M+H^+$) : 464.8587, trouvée: 464.8581.

**Exemple 71.3** Synthèse de la 4-bromo-2-iodo-1*H*-pyrrolo[2,3-*b*]pyridine **203** :

La 4-bromo-2-iodo-1-(phénylsulfonyl)-1*H*-pyrrolo[2,3-*b*]pyridine 205 (8.1 g, 17.5 mmol, 1.0 éq.) est solubilisée dans 250 mL de dioxane puis 2.6 g de *tert*-butoxide de sodium (26.3 mmol, 1.5 éq.) sont ajoutés. Après 16h à 80°C, le

milieu réactionnel est refroidi à température et le solvant est évaporé sous pression réduite. Le résidu est dissous dans de 100 mL d'acétate d'éthyle et 100 mL d'eau. Ensuite, la phase organique est extraite avec de l'acétate d'éthyle (3 fois 20 mL). Les phases organiques réunies ont été séchées sur $MgSO_4$ et concentrées sous pression réduite. Le brut est trituré avec du dichlorométhane puis une filtration sous vide permet de conduire au composé **203** (58 g, 72%) sous la forme d'un solide jaunâtre. $R_f$ = 0.25 (acétate d'éthyle/éther de pétrole = 20/80). **Pf** >260°C. **Infrarouge** (v, $cm^{-1}$, neat) 3898, 3851, 3819, 3800, 3749, 3742, 3731, 3687, 3674, 3668, 3646, 3627, 3565, 3099, 3019, 2930, 2844, 2798, 2588, 2216, 1912, 1595, 1567, 1473, 1406, 1382, 1330, 1282, 1263, 1206, 1120, 1098, 964, 953, 920, 813, 751, 670, 621, 603, 536, 524, 517, 506. **RMN $^1$H** (250 MHz, DMSO-$d_6$, 20°C) δ 12.60 (s, 1H), 7.97 (d, *J* = 5.2 Hz, 1H), 7.26 (d, *J* = 5.2 Hz, 1H), 6.62 (s, 1H). **RMN $^{13}$C** (63 MHz, DMSO-$d_6$, 20°C) δ 150.7 (Cq), 143.5 (CH), 123.2 (Cq), 121.9 (Cq), 119.3 (CH), 109.4 (CH), 83.2 (Cq). **HRMS (+ESI)** calculée pour $C_7H_4BrIN_2$ $(M+H^+)$ : 322.8675, trouvée: 322.8673.

**Exemple 71.4** Synthèse de la 4-bromo-1-(*tert*-butyldimethylsilyl)-2-iodo-1*H*-pyrrolo[2,3-*b*]pyridine **206 :**

Sous argon, la 4-bromo-2-iodo-1*H*-pyrrolo[2,3-*b*]pyridine **203** (386 mg, 1.19 mmol, 1.0 éq.) est solubilisée dans 20 mL de THF. A 0°C, du NaH (50% dans l'huile minérale, 68 mg, 1.43 mmol, 1.2 éq.) est ajouté par portion. Après 30 minutes d'agitation à température, le mélange est remis à 0°C et le TBDMSCl (215 mg, 1.43 mmol, 1.2 éq.) est additionné par portion. Ensuite, le mélange est mis à 80 °C pendant de 2h30. Après refroidissement à 0°C, 10 mL d'eau sont ajoutés doucement et les phases organiques sont extraites avec de l'acétate d'éthyle (3 x 10 mL). Les phases organiques réunies ont été lavées au NaCl, séchées sur $MgSO_4$, filtrées et concentrées sous pression réduite. Le brut est purifié par colonne de chromatographie flash sur gel de silice (acétate d'éthyle/éther de pétrole = 5/95) pour conduire au composé **205** (300 g, 57%) sous la forme d'une huile incolore. $R_f$ = 0.9 (acétate d'éthyle/éther de pétrole = 5/95). **Infrarouge** (v, $cm^{-1}$, neat) 2951, 2925, 2854, 1587, 1541, 1484, 1460, 1444, 1353, 1339, 1307, 1253, 1215, 1175, 1114, 1066, 997, 917, 846. **RMN $^1$H** (400 MHz, $CDCl_3$, 20°C) δ 7.98 (d, *J* = 5.0 Hz, 1H), 7.18 (d, *J* = 5.0 Hz, 1H), 6.98 (s, 1H), 0.99 (s, 9H), 0.87 (s, 6H). **RMN $^{13}$C** (101 MHz, $CDCl_3$, 20°C) δ 156.0 (Cq), 141.9 (CH), 125.2 (Cq), 121.7 (Cq), 119.5 (CH), 117.3 (CH), 84.6 (Cq), 29.7 (3 x $CH_3$), 27.1 (Cq), 0.7 (2 x $CH_3$). **HRMS (+ESI)** calculée pour $C_{13}H_{18}BrIN_2Si$ $(M+H^+)$ : 436.9540 trouvée: 436.9538.

Synthèse de la 4-((4-bromo-1-(*tert*-butyldimethylsilyl)-1*H*-pyrrolo[2,3-*b*]pyridin-2-yl)ethynyl)-*N,N*-dimethylaniline **207** :

Le composé a été préparé selon la **procédure C2** et purifié par colonne de chromatographie flash sur gel de silice (dichlorométhane/éther de pétrole = 20/80). Solide amorphe jaunâtre (54%), **Rf** = 0.5 (acétate d'éthyle/éther de pétrole = 20/80). **Pf** : 120-122°C. **Infrarouge** (υ, $cm^{-1}$, neat) 2952, 2890, 2851, 1603, 1568, 1538, 1507, 1489, 1443, 1431, 1403, 1374, 1338, 1324, 1293, 1246, 1229, 1191, 1157, 1120, 1104, 1071, 1046, 980, 942, 907. **RMN $^1$H** (250 MHz, $CDCl_3$, 20°C) δ 8.02 (d, *J* = 5.2 Hz, 1H), 7.46 (d, *J* = 8.9 Hz, 2H), 7.17 (d, *J* = 5.2 Hz, 2H), 6.84 (s, 1H), 6.66 (d, *J* = 8.9 Hz, 2H), 2.99 (s, 6H), 0.95 (s, 9H), 0.79 (s, 6H). **RMN $^{13}$C** (63 MHz, $CDCl_3$, 20°C) δ 154.2 (Cq), 150.4 (Cq), 143.0 (CH), 132.4 (2 x CH), 127.2 (Cq), 123.6 (Cq), 123.1 (Cq), 119.5 (CH), 111.9 (2 x CH), 110.3 (CH), 109.3 (Cq), 96.8 (Cq), 82.3 (Cq), 40.2 (2 x $CH_3$), 26.8 (3 x $CH_3$), 19.9 (Cq), -1.5 (3 x $CH_3$). **HRMS** (+**ESI**) calculée pour $C_2H_{28}BrN_3Si$ $(M+H^+)$ : 454.1308, trouvée: 454.1309.

**Exemple 71.5** Synthèse de la *N,N*-dimethyl-4-((4-(4,4,5,5-tetramethylem-1,3,2-dioxaborolan-2-yl)-1*H*-pyrrolo[2,3-*b*]pyridin-2-yl)ethynyl)aniline **202** :

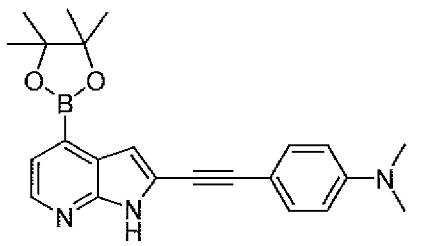

$C_{23}H_{26}BN_3O_2$
M W: 387,29 gmol-1

Sous argon, la 4-((4-bromo-1-(tert-butyldiméthylsilyl)-1*H*-pyrrolo[2,3-*b*]pyridin-2-yl)éthynyl)-*N,N*-diméthylaniline **207** (100 mg, 0.22 mmol, 1.0 éq.) est dissoute dans le dioxane (1 mL) puis l'addition du bis(pinacolato)diboron (112 mg, 0.44 mmol, 2.0 éq.) et de KOAc (64 mg, 0.66 mmol, 3.0 éq.) sont réalisées. Ensuite, le milieu réactionnel est dégazé pendant 10 minutes avant d'ajouter du $Pd(dppf)_2Cl_2$.DCM (18 mg, 0.022 mmol, 0.1 éq.). Le mélange réactionnel est chauffé à 100°C pendant 4 h. Après refroidissement, le solvant est évaporé sous pression réduite. Pour finir, le brut est

purifié par colonne de chromatographie flash sur gel de silice avec comme éluant (dichlorométhane/éther de pétrole = 20/80) pour conduire à l'intermédiaire mais avec 10% de bis(pinacolato)diboron). Ensuite, le brut est solubilisé dans 5 mL de THF. A 0°C, une solution de TBAF dans le THF (C = 1 M, 0.09 mL, 0.09 mmol, 1.2 éq.) est ajouté goutte à goutte. Au bout de 1h à 0°C, 10 mL d'eau et 10 mL d'acétate d'éthyle sont ajoutés successivement. Les phases organiques sont extraites 3 fois avec de l'acétate d'éthyle (30 mL). Réunies, elles sont séchées avec du $MgSO_4$, filtrées sur coton et évaporées sous pression réduite. Le brut est purifié par colonne de chromatographie flash sur gel de silice (acétate d'éthyle/éther de pétrole = 20/80) pour conduire au composé **199** (20 mg, 24% sur les 2 étapes) sous la forme d'un solide jaune. $R_f$ = 0.1 (acétate d'éthyle/éther de pétrole = 10/90). **Pf**>260°C. **Infrarouge** (ν, $cm^{-1}$, neat) 3291, 2962, 2930, 2853, 2102, 1606, 1511, 1463, 1434, 1373, 1356, 1336, 1273, 1245, 1227, 1185, 1141, 1093, 1023, 935. **RMN $^1$H** (250 MHz, DMSO-$d_6$, 20°C) 12.06 (s, 1H), 8.21 (m, 1H), 7.33 (m, 3H), 6.84 (s, 1H), 6.71 (d, $J$ = 8.7 Hz, 2H), 2.93 (s, 6H), 1.31 (s, 12H). **RMN $^{13}$C** (63 MHz, DMSO-$d_6$, 20°C) δ 150.7 (Cq), 148.4 (2 x Cq), 143.5 (CH), 132.9 (2 x CH), 124.4 (Cq), 122.2 (CH), 121.4 (Cq), 112.4 (2 x CH), 107.9 (Cq), 107.0 (CH), 95.6 (Cq), 84.4 (Cq), 80.5 (2 x Cq), 40.7 (2 x $CH_3$), 25.2 (4 x $CH_3$). **HRMS** (+ESI) calculée pour $C_{23}H_{26}BN_3O_2$ ($M+H^+$) : 388.2190, trouvée: 388.2193.

**Exemple 72: 5-Fluoro-2-((5-(methoxymethyl)furan-2-yl)ethynyl)-1*H*-pyrrolo[2,3-*b*]pyridine 215:**

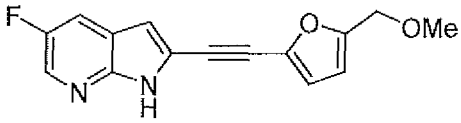

**215**

La 5-Fluoro-2-((5-(methoxymethyl)furan-2-yl)ethynyl)-1H-pyrrolo[2,3-b]pyridine **215** a été synthétisée en 2 étapes par une réaction de méthylation de l'alcyne vrai 212 suivie d'une réaction de Songashira au départ de d'halogenoazaindole.

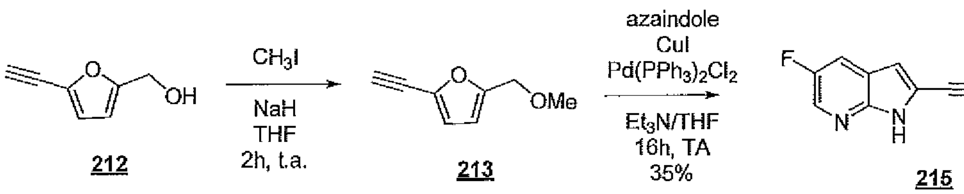


**212** **213** **215**

**Exemple 72.1** Synthèse du 2-ethynyl-5-(methoxymethyl)furane **213** :

Le composé **213** a été préparé selon la **procédure M** à partir du **212** et isolé sous forme d'une huile marron (90%). $R_f$= 0.25 (éther de pétrole/ acétate d'éthyle = 9/1). **Infrarouge** (ν, $cm^{-1}$, neat) 3517, 3165, 2896, 2179, 1654, 1560, 1370, 1111, 957, 770. **RMN $^1$H** (250 MHz, $CDCl_3$, 20 °C) δ 6.58 (m, 1H), 6.45 - 6.28 (d, $J$ = 0.6 Hz, 1H), 4.35 (d, $J$ = 0.6 Hz, 2H), 3.36 (s, 3H), 3.23 (s, 1H). **RMN $^{13}$C** (63 MHz, $CDCl_3$, 20°C) δ 170.4 (Cq), 129.8 (CH), 114.6 ($CH_2$), 109.4 (Cq), 102.1 (CH), 78.9 (CH), 76.8 (Cq), 61.3 ($CH_3$). **HRMS (+ESI)** calculée pour $C_{14}H_9FN_2O_2$ ($M+H^+$): 137,1738 trouvée: 137,1278.

**Exemple 72.2** Synthèse de la 5-Fluoro-2-((5-(methoxymethyl)furan-2-yl)ethynyl)-1*H*-pyrrolo[2,3-*b*]pyridine **215** :

Le composé **215** a été synthétisé selon la **procédure C2** et purifié par colonne de chromatographie flash sur gel de silice (acétate d'éthyle/éther de pétrole = 1/9). Le composé a été obtenu sous forme d'un solide jaune (35%). **Pf**: 162-164 °C. $R_f$ = 0.31 (acétate d'éthyle/éther de pétrole = 1/9). **Infrarouge** (ν, $cm^{-1}$, neat) 3117, 3059, 2981, 2890, 2727, 1584, 1505, 1433, 1374, 1360, 1346, 1297, 1251, 1179, 1157, 1087, 1020, 981, 935, 963, 876, 855, 758, 628. RMN **$^1$H** (400 MHz, DMSO-$d_6$, 20°C) δ 12.43 (s, 1H), 8.26 (m, 1H), 7.83 (m, 1H), 6.97 - 6.85 (m, 2H), 6.56 (m, 1H), 4.35 (s, 2H), 3.23 (s, 3H). **RMN $^{13}$C** (101 MHz, DMSO-$d_6$, 20°C) δ 154.7 (Cq), 145.7 (Cq), 135.5 (Cq), 133.6 (CH), 120.7 (Cq), 119.7 (d, $J$ = 47.8 Hz, CH), 117.57 (Cq), 114.5 (d, $J$ = 23.6 Hz, CH), 114.2 (Cq), 111.6 (CH), 107.6(CH), 86.3 (Cq), 83.8 (Cq), 65.7 ($CH_2$), 58.4 ($CH_3$). **RMN $^{19}$F** (376 MHz, DMSO-$d_6$, 20°C) δ -138.1 (m, 1F). **HRMS (+ESI)** calculée pour $C_{14}H_9FN_2O_2$ ($M+H^+$): 271,087732, trouvée: 271,087812.

**Exemple 73 : 5-(2-(5-Fluoro-1*H*-pyrrolo[2,3-*b*]pyridin-2-yl)ethynyl)furan-2-yl)methanol 221 :**

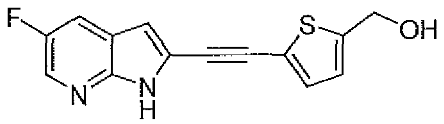

**221**

$C_{14}H_{10}N_2OS$
M.W.: 254,31g.mol$^{-1}$

Le 5-(2-(5-Fluoro-1*H*-pyrrolo[2,3-*b*]pyridin-2-yl)ethynyl)furan-2-yl)methanol **221** a été synthétisé en 1 étape par une réaction de Songashira au départ de l'alcyne vrai.

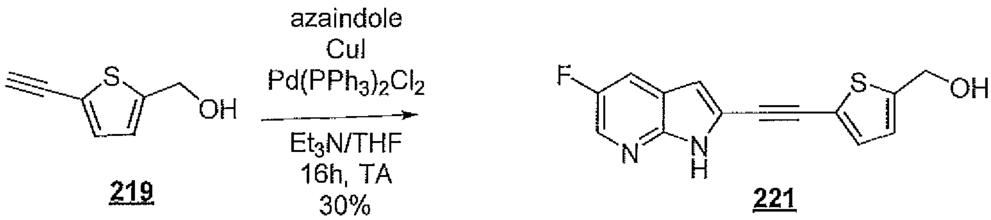


**219** **221**

Le composé **221** a été préparé selon la **procédure C2** et purifié par colonne de chromatographie flash sur gel de silice (méthanol/dichlorométhane/ammoniaque = 4/94/2). Le composé a été obtenu sous forme d'un solide jaune (30%). **Pf** : 240-242°C. **$R_f$**= 0.42 (méthanol/dichlorométhane/ammoniaque = 4/94/2). **Infrarouge** (v, cm$^{-1}$, neat) 3136, 2220, 2186, 2021, 1993, 1966, 1589, 1504, 1408, 1300, 1237, 1208, 1142, 1008, 985, 870, 800, 768, 611. **RMN $^1$H** (400 MHz, DMSO-$d_6$, 20°C) δ 12.38 (s, 1H), 8.28 (d, *J* = 2.2 Hz, 1H), 7.85 (m, 1H), 7.35 (m, 1H), 6.98 (d, *J* = 2.2 Hz, 1H), 6.84 (d, *J* = 2.2 Hz, 1H), 4.71 - 4.52 (m, 2H), 3.17 (m, 1H). **RMN $^{13}$C** (101 MHz, DMSO-$d_6$, 20°C) δ 151.1 (Cq), 145.6 (Cq), 133.7 (Cq), 133.4 (m, *J* = 29.3 Hz, 2 x CH), 124.8 (CH), 121.3 (Cq), 119.7 (Cq), 114.1 (d, *J* = 20.6 Hz, CH), 106.9 (d, *J* = 4.5 Hz, CH), 87.4 (Cq), 85.4 (Cq), 58.8 ($CH_2$), 31.1 (Cq). **RMN $^{19}$F** (376 MHz, DMSO-$d_6$, 20°C) δ -138.3 (m, 1F). **HRMS (+ESI)** calculée pour $C_{14}H_9FN_2O_2$ (M+H$^+$) : 273,0492 trouvée: 273,0489.

**Exemple 74 : 5-Fluoro-2-((5-(methoxymethyl)furan-2-yl)ethynyl)-1*H*-pyrrolo[2,3-*b*]pyridine 222** :

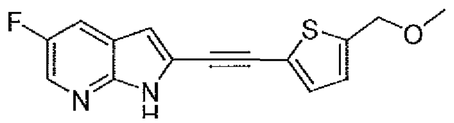

$C_{15}H_{11}FN_2OS$
M W: 286,32 gmol-1

La 5-Fluoro-2-((5-(methoxymethyl)furan-2-yl)ethynyl)-1*H*-pyrrolo[2,3-*b*]pyridine **222** a été synthétisé en 2 étapes par une réaction de méthylation de l'alcool primaire suivi d'une réaction de Songashira au départ de l'alcyne vrai et d'azaindole correspondant halogéné.

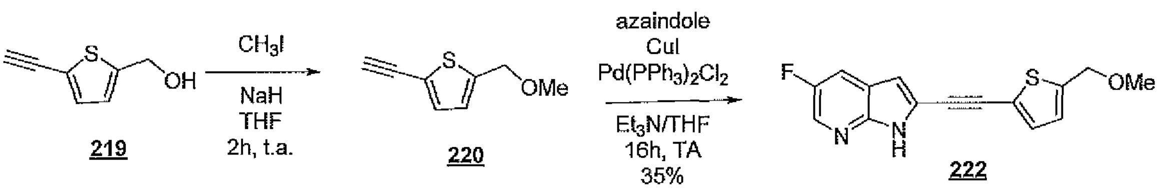


**219** **220** **222**

**Exemple 74.1** Synthèse du 2-Ethynyl-5-(methoxymethyl)thiophene **220:**

Le composé **220** a été préparé selon la **procédure M** et sous forme d'une huile marron (90%). $\mathbf{R_f}$= 0.25 (éther de pétrole/ acétate d'éthyle = 9/1). **Infrarouge** (v, $cm^{-1}$, neat) 3400, 3250, 2980, 2052, 1620, 1433, 1269, 1087, 922, 831. **RMN $^1$H** (250 MHz, $CDCl_3$, 20 °C) δ 7.16 - 7.05 (m, 1H), 6.84 - 6.74 (m, 1H), 4.58 - 4.45 (m, 2H), 3.35 (s, 3H), 3.30 (s, 1H). **RMN $^{13}$C** (63 MHz, $CDCl_3$, 20°C) δ 167.1 (Cq), 131.9 (CH), 117.1 ($CH_2$), 108.2 (Cq), 103.8 (CH), 79.1 (CH), 77.1 (Cq), 60.2 ($CH_3$). **HRMS (+ESI)** calculée pour $C_{14}H_9FN_2O_2$ ($M+H^+$): 153,08976 trouvée: 153,09459.

**Exemple 74.2** Synthèse de la 5-Fluoro-2-((5-(methoxymethyl)thiophen-2-yl)ethynyl)-1*H*-pyrrolo[2,3-*b*]pyridine 222 :

Le composé **222** a été synthétisé selon la **procédure C2** et purifié par colonne de chromatographie flash sur gel de silice (acétate d'éthyle/éther de pétrole = 1/9). Le composé a été obtenu sous forme d'un solide jaune (35%). **Pf** : 154-156 °C. $\mathbf{R_f}$= 0.31 (acétate d'éthyle/éther de pétrole = 1/9).

**Infrarouge** (v, $cm^{-1}$, neat) 3114, 3047, 2929, 2855, 2203, 1587, 1523, 1503, 1471, 1427, 1394, 1375, 1295, 1242, 1219, 1175, 1142, 1087, 1007, 980, 950, 799, 769, 643, 566. **RMN $^1$H** (400 MHz, $CDCl_3$, 20°C) δ 10.95 (s, 1H), 8.29 (s, 1H), 7.64 (m, 1H), 7.32 - 7.19 (m, 2H), 6.96 (m, 1H), 6.76 (s, 1H), 4.64 (s, 3H), 3.44 (s, 4H). **RMN $^{13}$C** (101 MHz, $CDCl_3$, 20°C) δ 157.1 (Cq), 154.6 (Cq), 145.1 (Cq), 144.3 (Cq), 132.8 (Cq), 132.6 (d, *J* = 16.2 Hz, CH), 126.3 (CH), 121.8 (d, *J* = 47.8 Hz, CH), 120.6 (Cq), 114.4 (d, *J* = 20.9 Hz, CH), 106.5 (d, *J* = 4.5 Hz, CH), 87.4 (Cq), 84.8 (Cq), 69.1($CH_2$), 58.1($CH_3$). **RMN $^{19}$F** (376 MHz, DMSO-$d_6$, 20°C) δ -137.3 (m, 1F). **HRMS** (+**ESI**) calculée pour $C_{14}H_9FN_2O_2$ ($M+H^+$): 287,06488 trouvée: 287,06469.

**Exemple 75 : 4-({[4-(2-{5-Fluoro-1*H*-pyrrolo[2,3-*b*]pyridin-2-yl}ethynyl)phenyl] methoxy}methyl)piperidine 223 :**

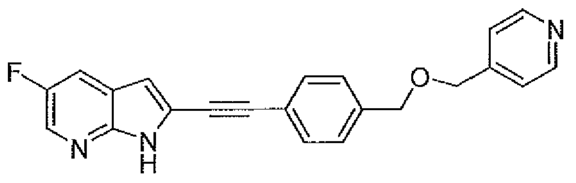

$C_{22}H_{16}FN_3O$
M W: 357,39 gmol-1

La 4-({[4-(2-{5-Fluoro-1*H*-pyrrolo[2,3-*b*]pyridin-2-yl}ethynyl)phenyl]methoxy}methyl)piperidine **223** a été synthétisé en 1 étape par uune réaction de Songashira au départ de l'alcyne vrai et d'azaindole correspondant halogéné.

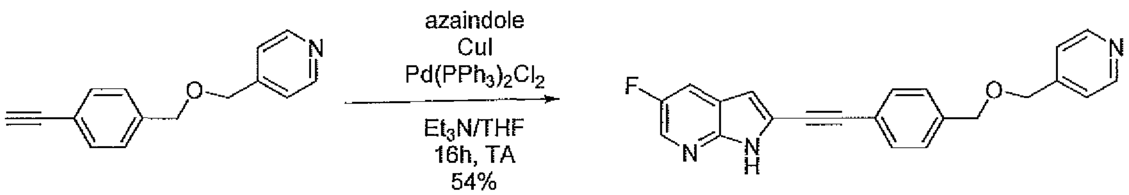


Le composé **223** a été synthétisé selon la **procédure C2** à partir de la 4-{[(4-Ethynylphenyl)methoxy]methyl}pyridine et purifié par colonne de chromatographie flash sur gel de silice (acétate d'éthyle/éther de pétrole = 3/7). Le composé a été obtenu sous forme d'un solide jaune (54%). **Pf**: >260°C. $\mathbf{R_f}$= 0.29 (acétate d'éthyle/éther de pétrole = 3/7). **Infrarouge** (v, $cm^{-1}$, neat) 2678, 1610, 1535, 1497, 1397, 1296, 1150, 1085, 1028, 880, 808, 760, 737, 591, 563. **RMN $^1$H** (400 MHz, DMSO-$d_6$, 20°C) 12.40 (s1, 1H), 8.60 (s, 2H), 8.28 (m, 1H), 7.86 (m, 1H), 7.60 (d, *J* = 7.8 Hz, 2H), 7.49 (d, *J* = 7.8 Hz, 2H), 7.40 (s1, 2H), 6.84 (d, *J* = 2.0 Hz, 1H), 4.64 (2 s, 4H). RMN **$^{13}$C** (101 MHz, DMSO-$d_6$, 20°C) δ 156.8 (Cq), 154.34 (Cq), 150.1 (Cq), 147.7 (Cq), 145.6 (Cq), 139.9 (Cq), 133.4 (CH), 133.13 (CH), 131.8 (2 x CH), 128.3 (2 x CH), 121.6 (CH), 120.78 (CH), 120.1 (d, *J* = 7.5 Hz, Cq), 114.3 (CH), 114.1 (CH), 106.7 (d, *J* = 4.6 Hz, CH), 93.6 (Cq), 82.2 (Cq), 71.8 ($CH_2$), 70.4 ($CH_2$) **RMN $^{19}$F** (376 MHz, DMSO-$d_6$, 20°C) δ -137.8 (m, 1F). **HRMS (+ESI)** calculée pour $C_{14}H_9FN_2O_2$ ($M+H^+$): 258,135027, trouvée: 258,135169.

**Exemple 76 : 2-[2-(4-Fluorophenyl)ethynyl]-1*H*-pyrrolo[2,3-*b*]pyridine 224** :

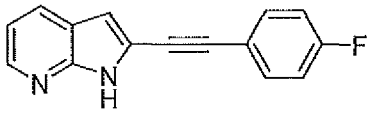

$C_{15}H_9FN_2$
M W: 236,25 gmol-1

La 2-[2-(4-Fluorophenyl)ethynyl]-1*H*-pyrrolo[2,3-*b*]pyridine **224** a été synthétisée en 1 étape par une réaction de Songashira au départ de l'alcyne vrai.

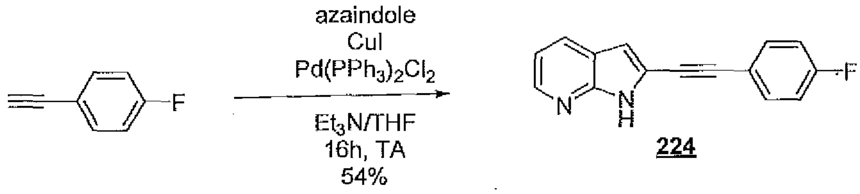


224

Le composé **224** a été synthétisé selon la **procédure C2** à partir du 1-ethynyl-4-fluoro-benzene et purifié par colonne de chromatographie flash sur gel de silice (acétate d'éthyle/éther de pétrole = 3/7). Le composé a été obtenu sous forme d'un solide jaune (81%). **Pf**: >260°C. $\mathbf{R_f}$= 0.29 (acétate d'éthyle/éther de pétrole = 3/7). **Infrarouge** (v, $cm^{-1}$, neat) 3117, 3056, 2979, 2877, 2817, 2703, 1536, 1397, 1359, 1228, 1197, 1155, 1020, 979, 935, 918, 833, 760, 652, 597. **RMN $^1$H** (400 MHz, DMSO-$d_6$, 20°C) δ 12.23 (s, 1H), 8.29 (dd, *J* = 4.6, 1.6 Hz, 1H), 7.97 (dd, *J* = 7.9, 1.6 Hz, 1H), 7.71 - 7.61 (m, 2H), 7.37 - 7.27 (m, 2H), 7.11 (dd, *J* = 7.9, 4.6 Hz, 1H), 6.83 (d, *J* = 1.6 Hz, 1H). **RMN $^{13}$C** (101 MHz, DMSO-$d_6$, 20°C) δ 163.9 (Cq), 161.4 (Cq), 148.8 (CH), 144.9 (CH), 134.1 (d, *J* = 8.7 Hz, CH), 128.9 (CH), 119.9 (Cq), 119.2 (Cq), 118.59 (d, *J* = 3.4 Hz, CH), 116.87 (d, *J* = 5.9 Hz, CH), 116.6 (CH), 106.8 (CH), 99.9 (Cq), 92.1 (Cq), 82.4 (Cq). **RMN $^{19}$F** (376 MHz, DMSO-$d_6$, 20°C) δ -109.7 (m, 1F). **HRMS (+ESI)** calculée pour $C_{14}H_9FN_2O_2$ (M+H$^+$) : 237,082253, trouvée: 237,082117.

**Exemple 77 : 2-((1*H*-Indol-4-yl)ethynyl)-5-fluoro-1*H*-pyrrolo[2,3-*b*]pyridine 225:**

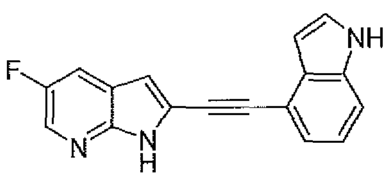

$C_{17}H_{10}FN_3$

M W: 275,29 gmol-1

La 2-((1*H*-Indol-4-yl)ethynyl)-5-fluoro-1*H*-pyrrolo[2,3-*b*]pyridine **225** a été synthétisée en 1 étape par une réaction de Songashira au départ de l'alcyne vrai.

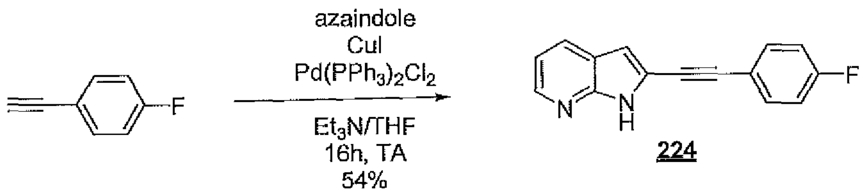


224

Le composé **225** a été synthétisé selon la **procédure A** à partir du 4-Ethynyl-1*H*-indole et purifié par colonne de chromatographie flash sur gel de silice (acétate d'éthyle/éther de pétrole = 3/7). Le composé a été obtenu sous forme d'un solide jaune (15%). **Pf**: >260°C. $\mathbf{R_f}$= 0.29 (acétate d'éthyle/éther de pétrole = 3/7). **Infrarouge** (v, $cm^{-1}$, neat) 3400, 3111, 3054, 2980, 2792, 2205, 1732, 1607, 1582, 1531, 1499, 1462, 1416, 1394, 1344, 1310, 1259, 1227, 1196, 1138, 1099, 1066, 985, 887, 803, 726, 604, 556. **RMN $^1$H** (400 MHz, DMSO-$d_6$, 20°C) δ 12.30 (s1, 1H), 11.38 (sl, 1H), 8.25 (s, 1H), 7.83 (dt, *J* = 6.5 Hz, 2H), 7.46 (dd, *J* = 6.5 Hz, 2H), 7.30 (d, *J* = 1.6 Hz, 1H), 6.77 (d, *J* = 1.6 Hz, 1H), 6.53 - 6.48 (m, 1H). **RMN $^{13}$C** (101 MHz, DMSO-$d_6$, 20°C) 145.6 (Cq), 136.4 (Cq), 132.6 (CH), 132.4 (CH), 128.1 (Cq), 127.4 (CH),

124.76 (CH), 124.33 (CH), 122.7 (Cq), 120.34 (Cq), 113.8 (d, $J$ = 20.9 Hz, CH), 112.5 (Cq), 111.8 (CH), 105.8 (CH), 102.1 (CH), 96.1 (Cq), 79.5 (Cq). **RMN $^{19}$F** (376 MHz, DMSO-$d_6$, 20°C) $\delta$ -138.7 (m, 1F). **HRMS (+ESI)** calculée pour $C_{14}H_9FN_2O_2$ (M+H$^+$) : 276,093152, trouvée: 276,093345.

## Revendications

**1.** Composé de formule II

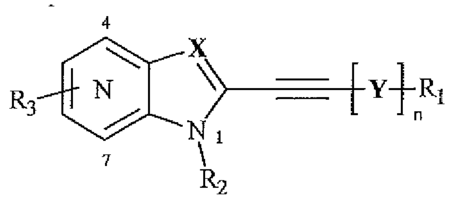

(II)

- **X étant choisi parmi** N ou C-$R_4$
- **Y étant choisi parmi** un alcène ou un alcyne, un aryle ou un hétéroaryle, un alcane comprenant de 1 à 7 atomes de carbone, n étant un entier égal à 0 ou 1 ;
- **$R_1$ étant choisi parmi** un aryle ou hétéroaryle choisis parmi les groupes phényle, pyrimidine, pyridine, thiophène, furane, triazole, oxazole, (aza)indole, (aza)indoline, (aza)benzimidazole éventuellement substitués sur une ou plusieurs positions par un groupe choisi parmi :

  **Halogène, $NO_2$,** CN, **diméthyltriazène, triméthylammonium, aryliodonium,**
  **$NR^aR^b$, $SO_2NR^aR^b$, $CONR^aR^b$, $NR^cSO_2NR^aR^b$, $NR^cCONR^aR^b$, $NR^aCOR^b$;**
  $R^a$, $R^b$ et $R^c$ représentent indépendamment les uns des autres un H, un ($C_1$-$C_7$)alkyle, un aryle, un hétéroaryle, un ($C_3$-$C_7$)carbocyclyle, un ($C_1$-$C_7$)alkyle-aryle, ($C_1$-$C_7$)alkyle-hétéroaryle, ou $R^a$ et $R^b$ forment ensemble un ($C_3$-$C_7$)hétérocyclyle ;
  **$Sn(Alkyle)_3$, Alkyle étant choisi parmi méthyle ou *n*-butyle** ;
  **$B(OH)_2$, B(pinacol)** ;
  **$R^d$, $CH_2R^d$;**
  $R^d$ représente un H, un ($C_1$-$C_7$)alkyle, un aryle, un hétéroaryle, un ($C_3$-$C_7$)hétérocyclyle, un ($C_3$-$C_7$)carbocyclyle, un ($C_1$-$C_7$)alkyle-aryle, un ($C_1$-$C_7$)alkyle-hétéroaryle, un ($C_1$-$C_7$)alkyle-($C_3$-$C_7$)hétérocyclyle, un $[(C_1\text{-}C_7)\text{Alkyle}]_n$-Z ou un $[(C_1\text{-}C_7)\text{Alkyle-Z}]_n$ Z étant un hétéroatome choisi parmi N, O ou S, en particulier choisi parmi $NR^aR^b$ ou $R^e$ et n étant un entier compris de 1 à 7 ;
  **$OR^e$, OAc, OTs, OTf, $SR^e$, $SO_2R^e$, $COR^e$, $NR^aSO_2R^e$, $NHCOOR^e$;**
  $R^e$ représente un H, un ($C_1$-$C_7$)alkyle, un aryle, un hétéroaryle, un ($C_3$-$C_7$)hétérocyclyle, un ($C_3$-$C_7$)carbocyclyle, un ($C_1$-$C_7$)alkyle-aryle, un ($C_1$-$C_7$)alkyle-hétéroaryle ou un ($C_1$-$C_7$)alkyle-($C_3$-$C_7$)hétérocyclyle ;

- **$R_2$ étant choisi parmi :**
  H, $SO_2Ph$, $COR^e$, $NR^cCONR^aR^b$, $COOR^e$, OH, $R^d$ ;
- **$R_3$ étant choisi parmi :**

  Halogène, $NO_2$, CN, diméthyltriazène, triméthylammonium, aryliodonium,
  $NR^aR^b$, $SO_2NR^aR^b$, $CONR^aR^b$, $NR^cSO_2NR^aR^b$, $NR^cCONR^aR^b$, $NR^aCOR^b$;
  $Sn(Alkyle)_3$, Alkyle étant choisi parmi méthyle ou *n*-butyle ;
  $B(OH)_2$, B(pinacol) ;
  $R^d$, $CH_2R^d$,
  $OR^e$, OAc, OTs, OTf, O(hétéroaryle) notamment O(HOBt), $SR^e$, $SO_2R^e$, $COR^e$, $NR^aSO_2R^e$, $NHCOOR^e$;

- **$R_4$ étant choisi parmi :**
  Halogène, $CH_2NR^aR^b$, $R^a$, $COOR^e$, CHO, $CH_2OR^e$ ;

dans lequel au moins l'un des substituants $R_1$, $R_2$, $R_3$ ou $R_4$ comprend un radioélément choisi parmi $^{18}$F, $^{11}$C, $^{123}$I et $^{124}$I.

**2.** Composé selon la revendication 1, de formule générale II-1 :

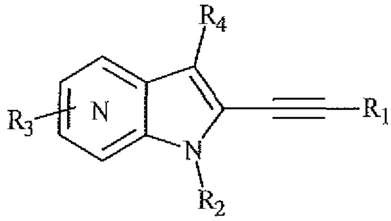

(II-1)

$R_1$, $R_2$, $R_3$ et $R_4$ étant tels que définis précédemment dans la formule II ;
dans lequel au moins l'un des substituants $R_1$, $R_2$, $R_3$ ou $R_4$ comprend un radioélément choisi parmi $^{18}F$, $^{11}C$, $^{123}I$ et $^{124}I$.

**3.** Composé selon l'une des revendications 1 à 2, ledit composé étant de formule générale II-1 :

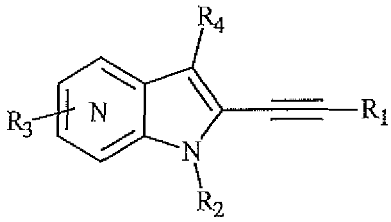

(II-1)

$R_2$, $R_3$ et $R_4$ étant tels que définis précédemment dans la formule II;
$R_1$ étant éventuellement marqué et choisi parmi

- 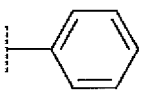 ;

- 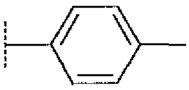 ;

- 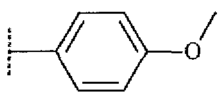 ;

- 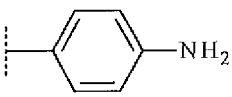 ou 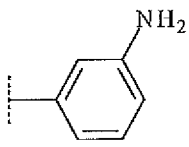 ;

- 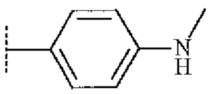 ;

- 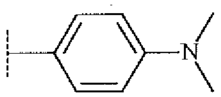 ou 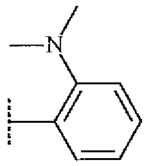 ou 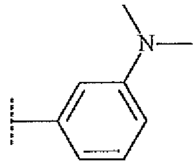 ;

- 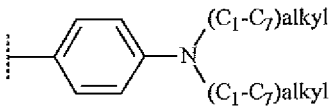 ou 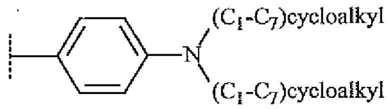 ,

notamment

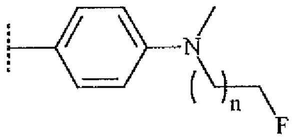 ,

n étant compris de 0 à 6 ;

- 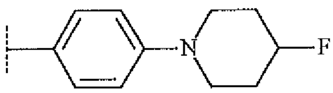 ;

- 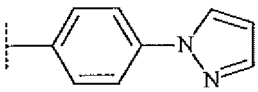 ;

- 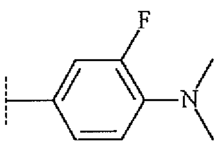 ou 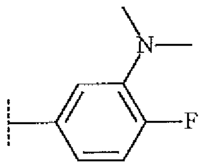 ;

- 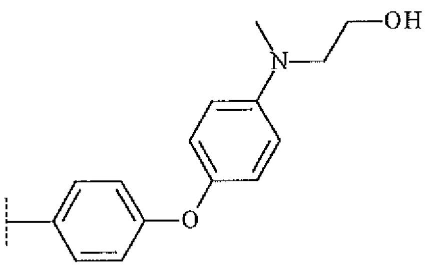 ; 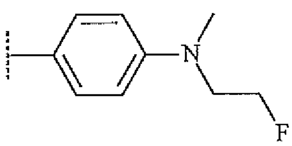 ;

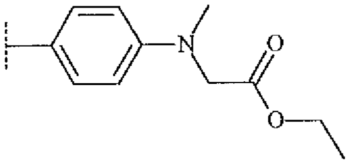
;

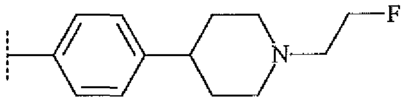
;

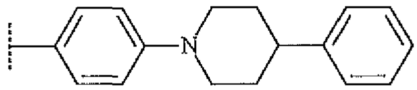
;

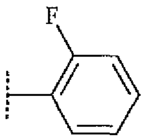
;
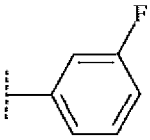
;
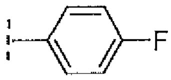
;

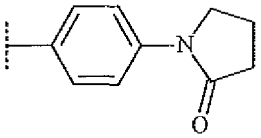
;

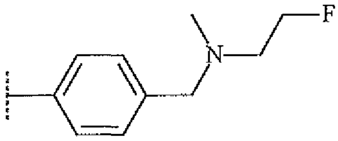
;

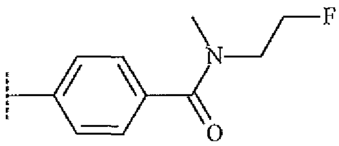
;

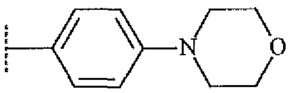
;

- 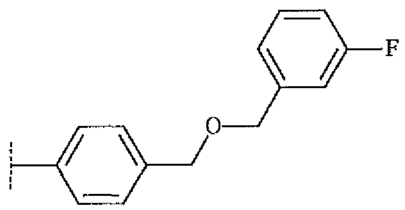 ;

- 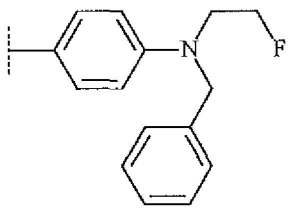 ;

- 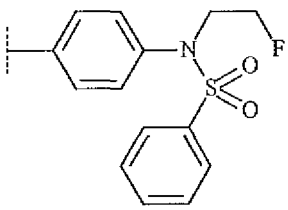 ;

- 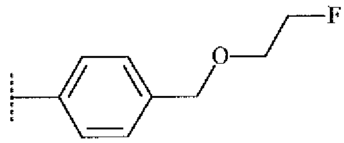 ;

- 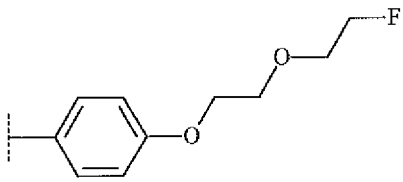 ;

- 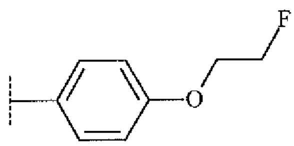 ;

- 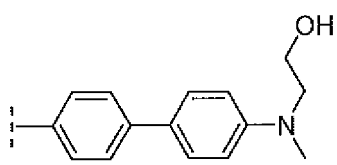 ;

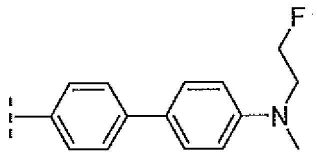 ;

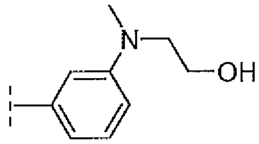 ;

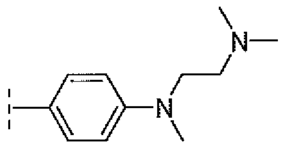 ;

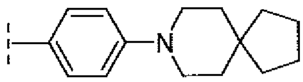 ;

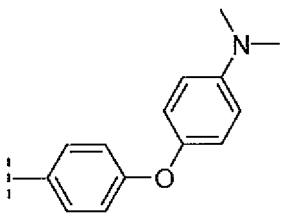 ;

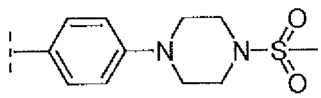 ;

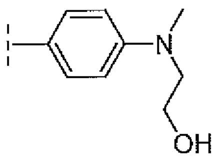 ;

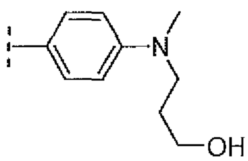 ;

- 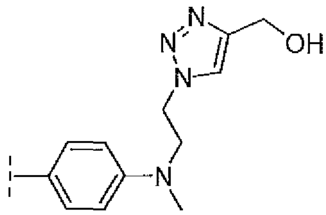 ;

- 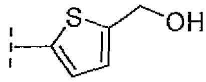 ;

- 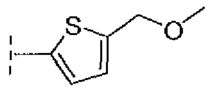 ;

- 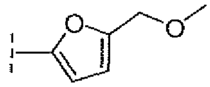 ;

- 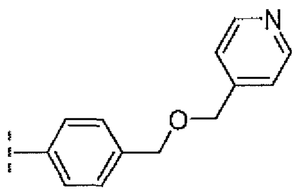 ;

- 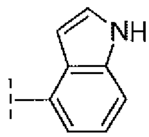 .

**4.** Composé selon l'une des revendications 1 à 3, ledit composé étant de formule générale II-1 :

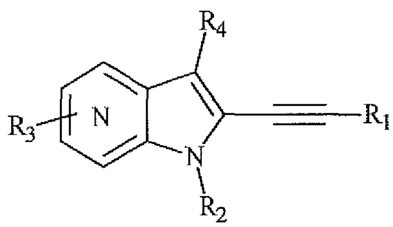

(II-1)

$R_1$, $R_3$ et $R_4$ étant tels que définis précédemment dans la formule II ;
$R_2$ étant éventuellement marqué et choisi parmi

- H;
- $(CH_2CH_2O)_n$-$CH_2CH_2F$, n étant un entier égal à 0, 1 ou 2 ;
- $SO_2Ph$ ;
- $COO^tBu$ ;
- $CH_2OCH_3$ ;
- $CH_2O(CH_2)_2OCH_3$ ;

- $COCH_3$;
-

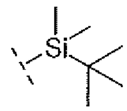

.

**5.** Composé selon l'une des revendications 1 à 4, ledit composé étant de formule générale II-1 :

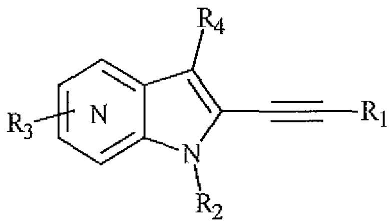

(II-1)

$R_1$, $R_2$ et $R_4$ étant tels que définis précédemment dans la formule II ;
$R_3$ étant éventuellement marqué et choisi parmi

- 6-Cl ;
- 6-F ;
- 5-F ;
- 4-F ;
- 6-(2-thiényle) ;
- 6-(3-thiényle) ;
- 6-(6-fluoro-3-pyridyle) ;
- 5-OMe ;
- 6-morpholino ;
- 5-morpholino ;
- 6-Br ;
- 4-Br ;
- 4-Bpin ;
- 5-Bpin.

**6.** Composé selon l'une des revendications 1 à 5, ledit composé étant de formule générale II-1 :

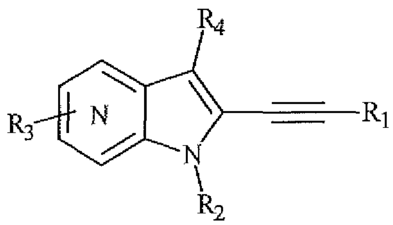

(II-1)

$R_1$, $R_2$ et $R_3$ étant tels que définis précédemment dans la formule II ;
$R_4$ étant choisi parmi

- H;

$(CH_2)_n$-N-alkyl, n étant un entier égal à 0, 1 ou 2.

**7.** Composé selon l'une des revendications 1 à 6, ledit composé étant de formule générale II-1a :

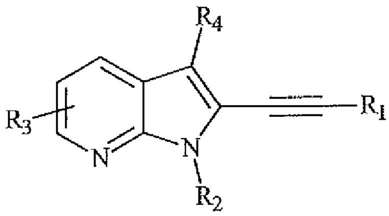

(II-1a)

$R_2$, $R_3$ et $R_4$ étant tels que définis précédemment dans la formule II ;
$R_1$ étant éventuellement marqué et choisi parmi

- 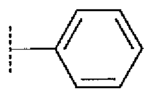 ;
- 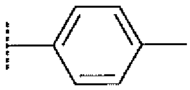 ;
- 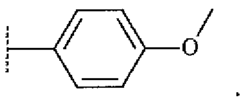 ;
- 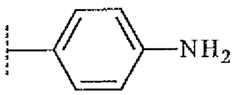 ou 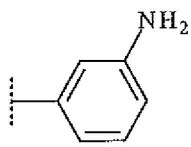 ;
- 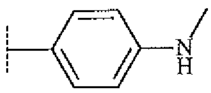 ;
- 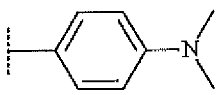 ou 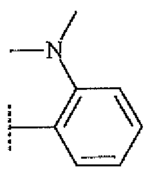 ou 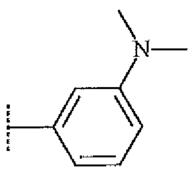 ;
- 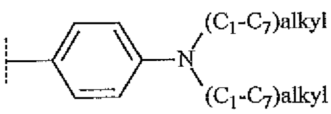 ou 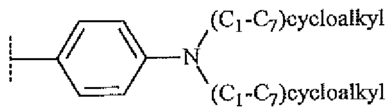 ,

notamment

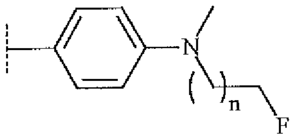,

n étant compris de 0 à 6 ;

- 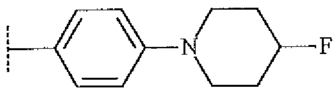 ;

- 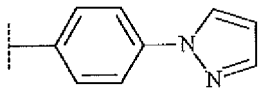 ;

- 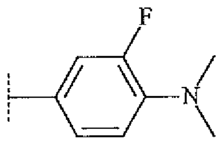 ou 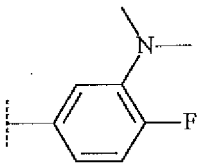 ;

- 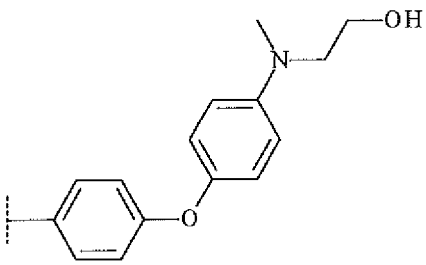 ; 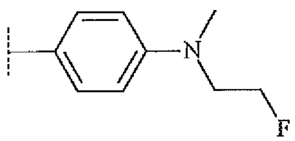 ;

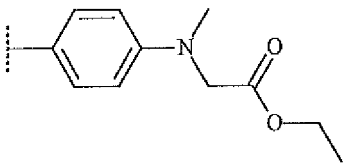 ;

- 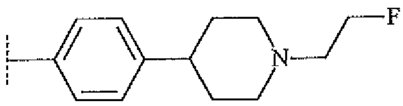 ;

- 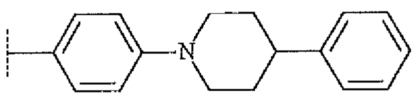 ;

- 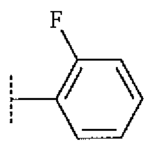 ; 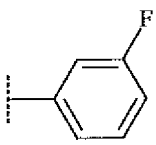 ; 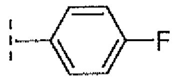 ;
- 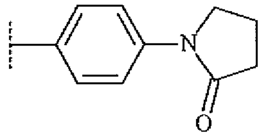 ;
- 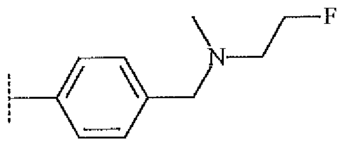 ;
- 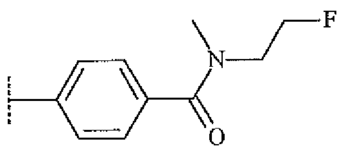 ;
- 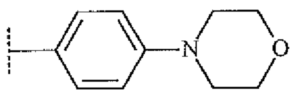 ;
- 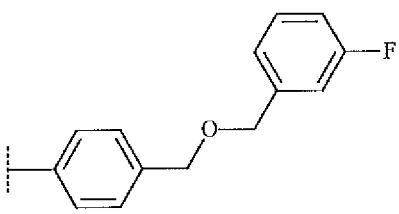 ;
- 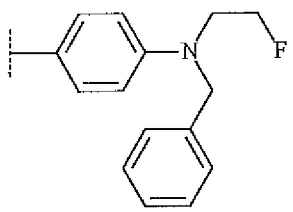 ;

- 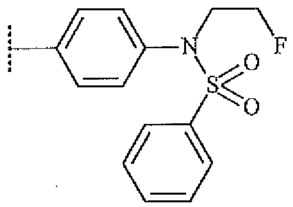 ;
- 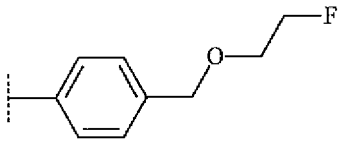 ;
- 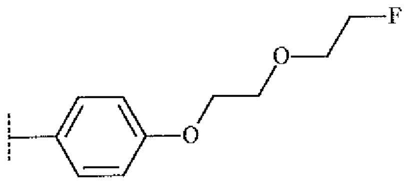 ;
- 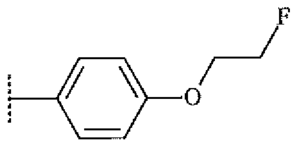 ;
- 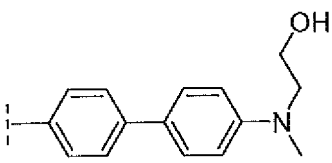 ;
- 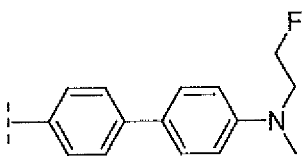 ;
- 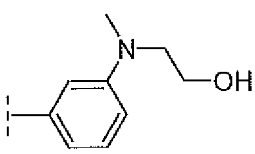 ;
- 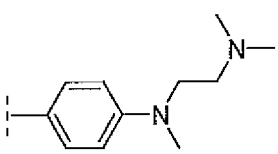 ;

- 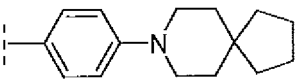 ;

- 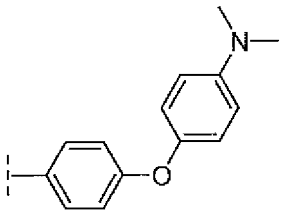 ;

- 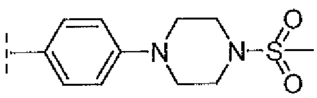 ;

- 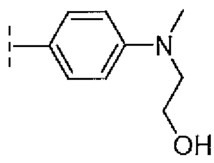 ;

- 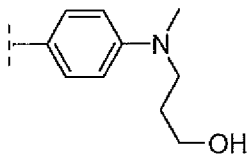 ;

- 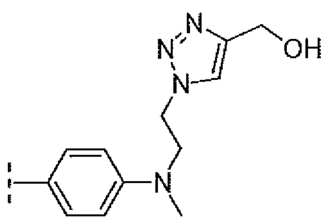 ;

- 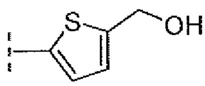 ;

- 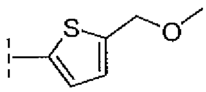 ;

- 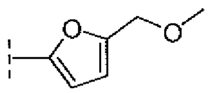 ;

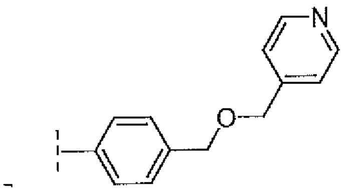

;

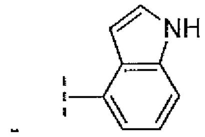

.

8. Composé selon l'une des revendications 1 à 7, ledit composé étant de formule générale II-2 :

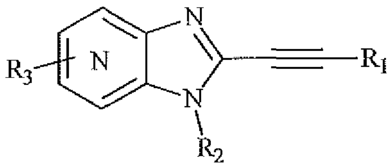

(II-2)

$R_1$, $R_2$ et $R_3$ étant tels que définis précédemment dans la formule II ;
dans lequel au moins l'un des substituants $R_1$, $R_2$ ou $R_3$ comprend un radioélément choisi parmi $^{18}F$, $^{11}C$, $^{123}I$ et $^{124}I$.

9. Composé selon l'une des revendications 1 à 8, ledit composé de formule II étant choisi parmi les composés de formule suivante :

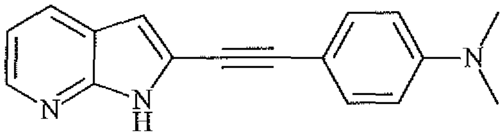

**22a**

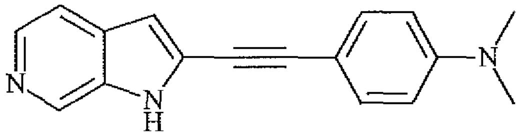

**22b**

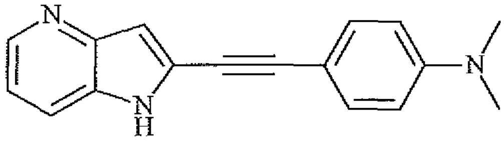

**22c**

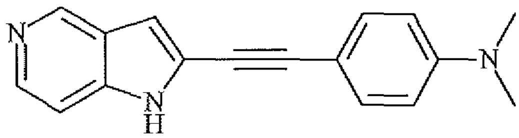

**22d**

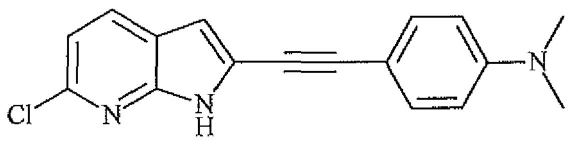

22e

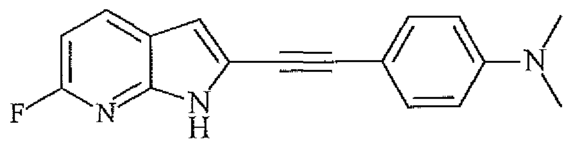

22f

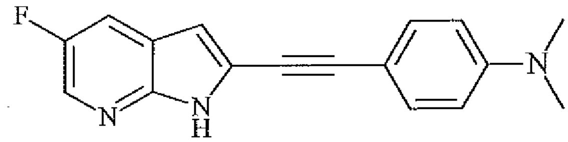

22g

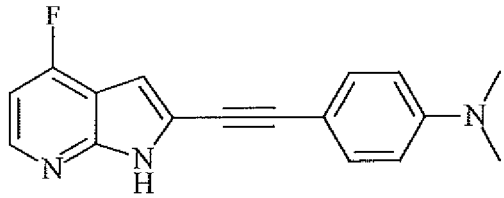

22h

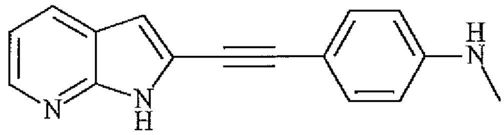

22i

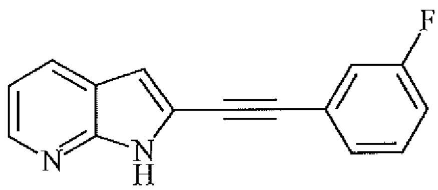

22k

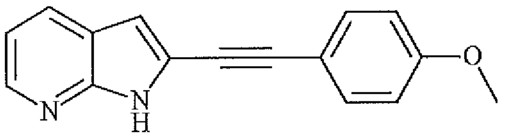

22m

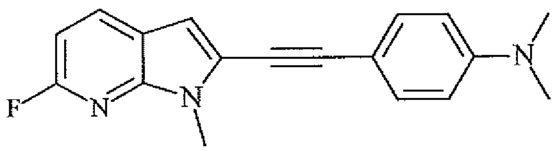

22n

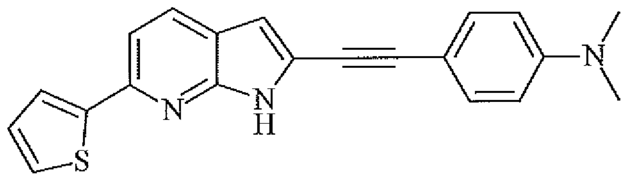

25a

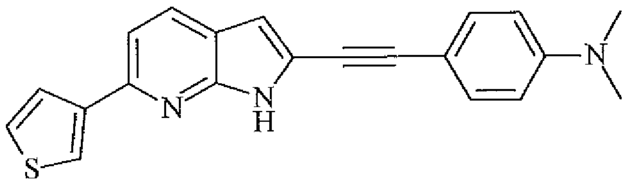

25b

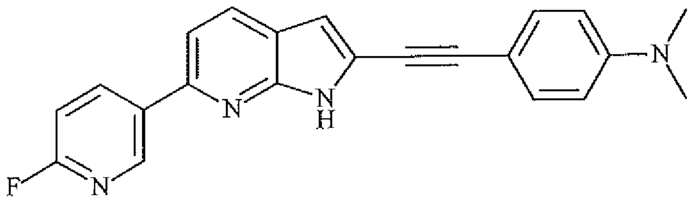

25c

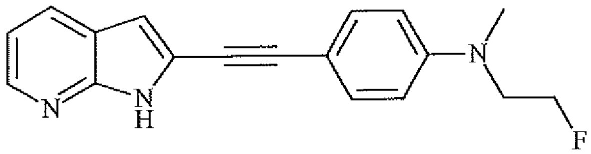

28

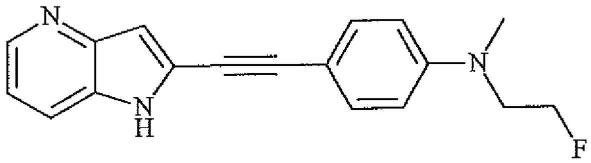

35

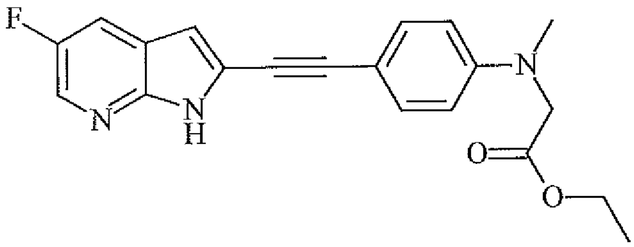

37

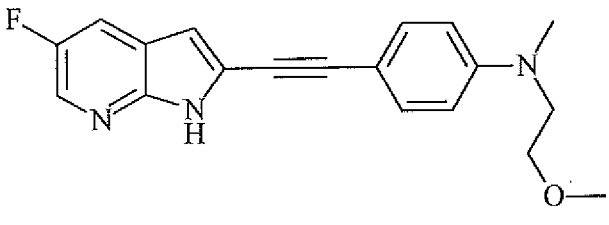

38

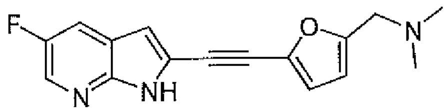

**131**

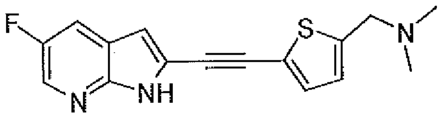

**133**

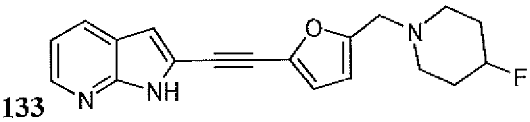

**128**

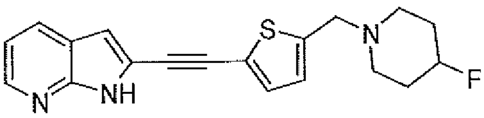

**124**

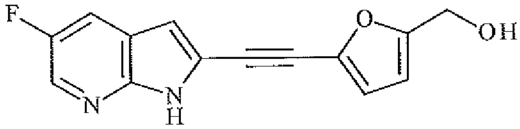

**120**

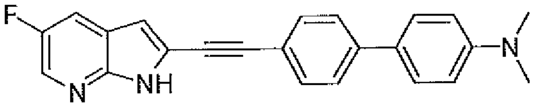

**116**

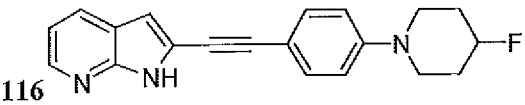

**113**

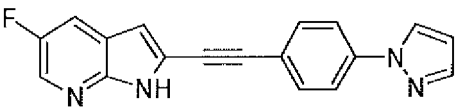

**108**

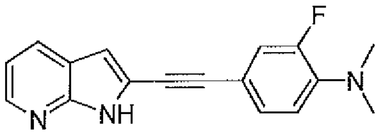

**103**

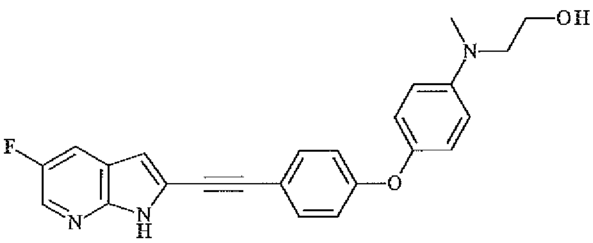

**97**

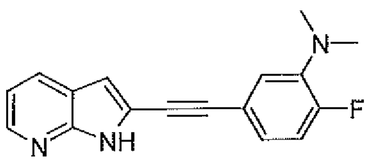

**93**

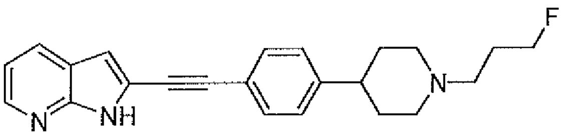

**85**

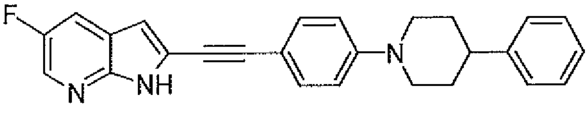

**90**

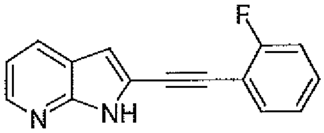

**84**

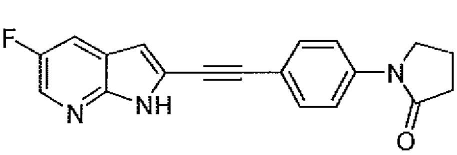

**79**

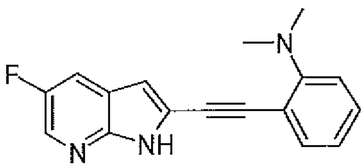

**76**

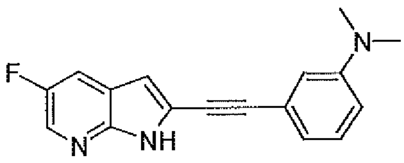 74

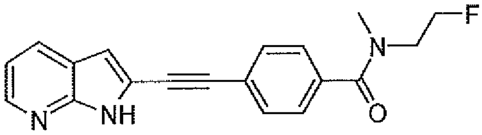 71

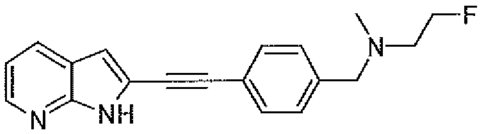 67

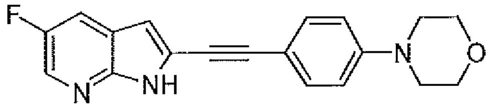 64

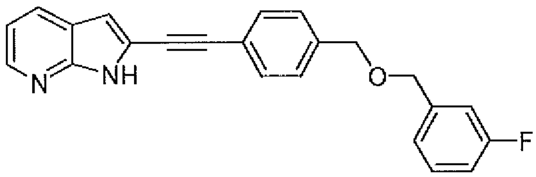 61

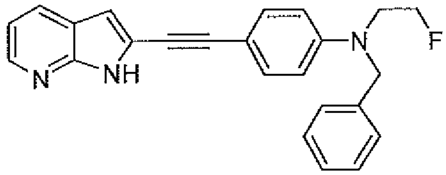 52

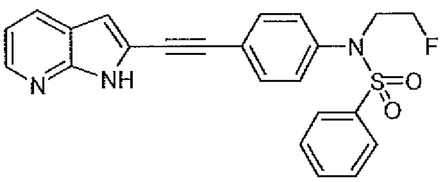 57

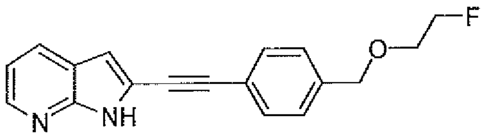 44

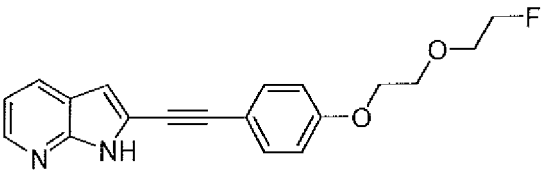 48

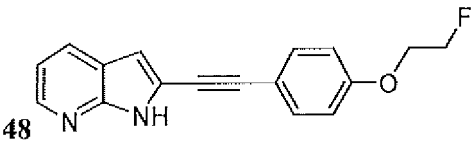 43

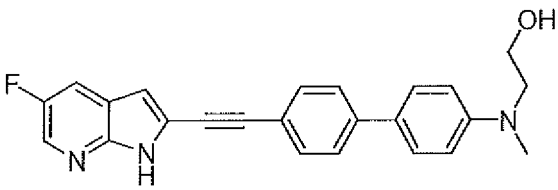

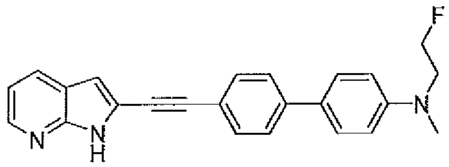

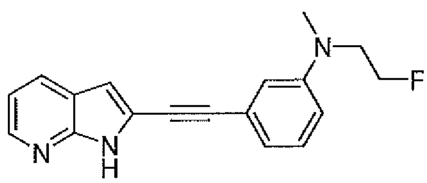

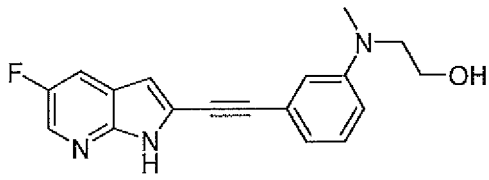

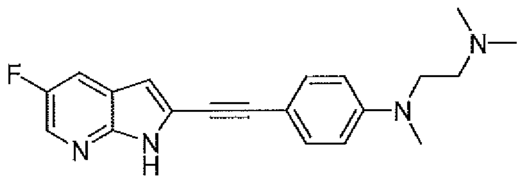

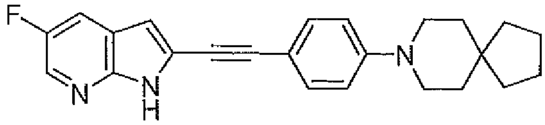

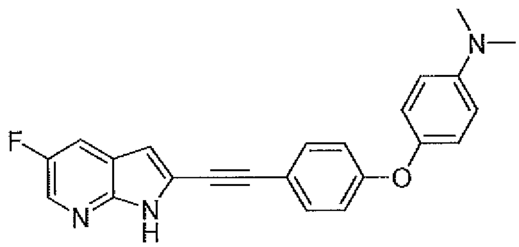

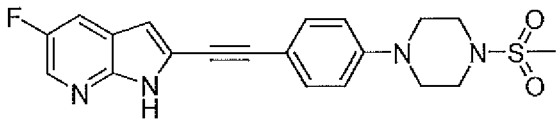

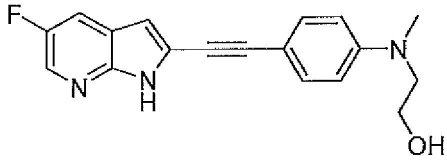

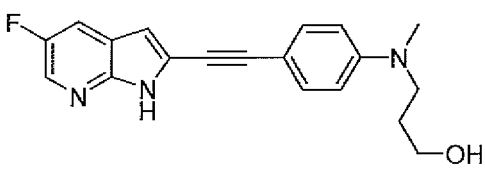

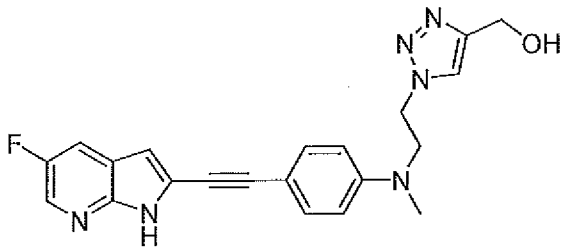

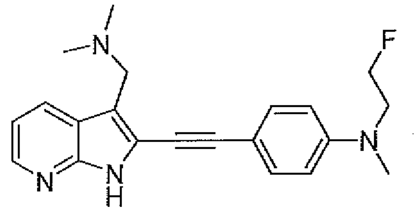

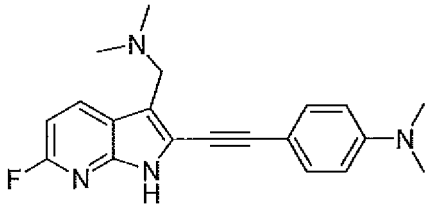

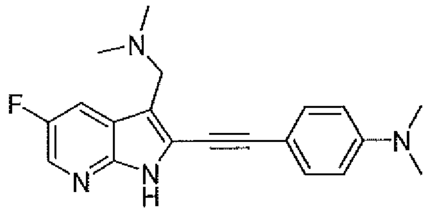

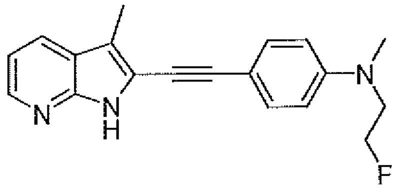

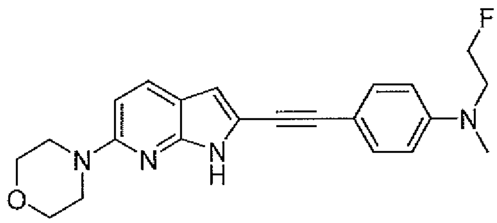

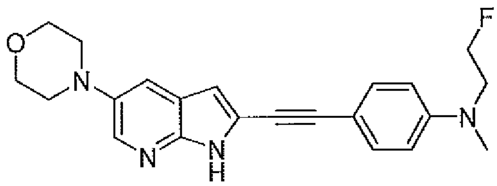

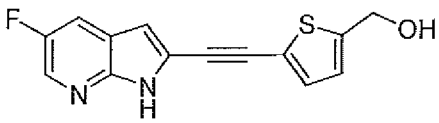

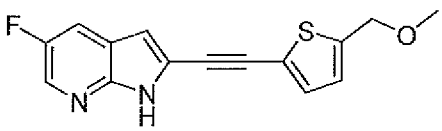

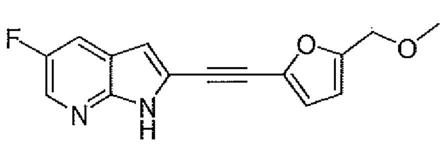

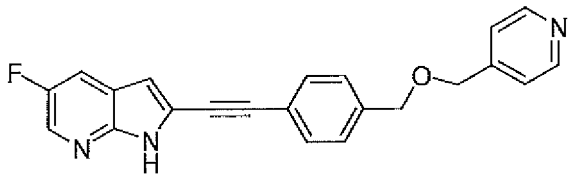

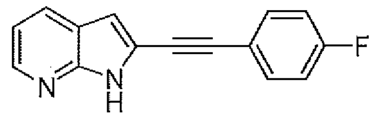

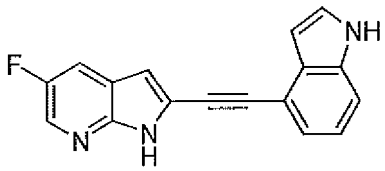

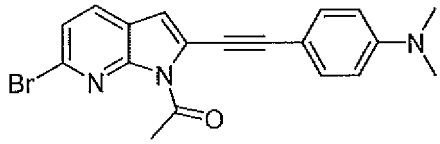

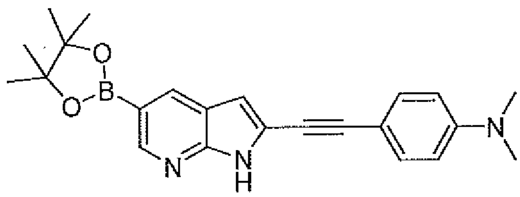

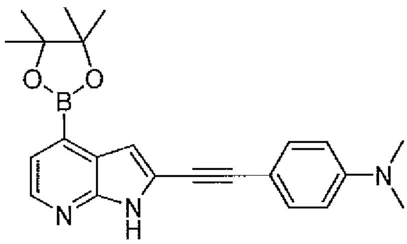

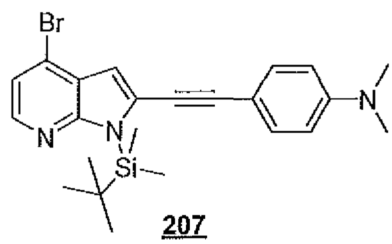

**207**

à l'état radiomarqué ;
notamment, ledit composé de formule II étant :

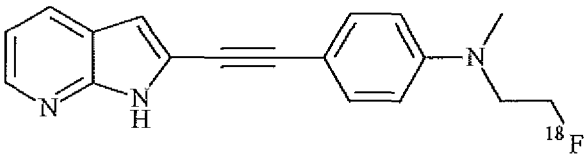

$[^{18}F]$-28

**10.** Utilisation d'un composé de formule III :

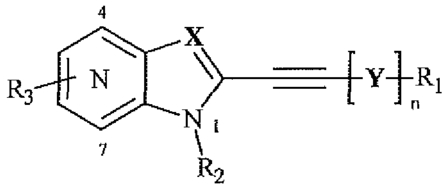

(III)

- X étant choisi parmi N ou C-$R_4$
- Y étant choisi parmi un alcène ou un alcyne, un aryle ou un hétéroaryle, un alcane comprenant de 1 à 7 atomes de carbone, n étant un entier égal à 0 ou 1 ;
- $R_1$ étant choisi parmi un aryle ou hétéroaryle choisis parmi les groupes phényle, pyrimidine, pyridine, thiophène, furane, triazole, oxazole, (aza)indole, (aza)indoline, (aza)benzimidazole éventuellement substitués sur une ou plusieurs positions par un groupe choisi parmi :

  Halogène, $NO_2$, CN, diméthyltriazène, triméthylammonium, aryliodonium, $NR^aR^b$, $SO_2NR^aR^b$, $CONR^aR^b$, $NR^cSO_2NR^aR^b$, $NR^cCONR^aR^b$, $NR^aCOR^b$;
  $R^a$, $R^b$ et $R^c$ représentent indépendamment les uns des autres un H, un ($C_1$-$C_7$)alkyle, un aryle, un hétéroaryle, un ($C_3$-$C_7$)carbocyclyle, un ($C_1$-$C_7$)alkyle-aryle, ($C_1$-$C_7$)alkyle-hétéroaryle, ou $R^a$ et $R^b$ forment ensemble un ($C_3$-$C_7$)hétérocyclyle ;
  Sn(Alkyle)$_3$, Alkyle étant choisi parmi méthyle ou *n*-butyle ;
  $B(OH)_2$, B(pinacol) ;
  $R^d$, $CH_2R^d$ ;
  $R^d$ représente un H, un ($C_1$-$C_7$)alkyle, un aryle, un hétéroaryle, un ($C_3$-$C_7$)hétérocyclyle, un ($C_3$-$C_7$)carbocyclyle, un ($C_1$-$C_7$)alkyle-aryle, un ($C_1$-$C_7$)alkyle-hétéroaryle, un ($C_1$-$C_7$)alkyle-($C_3$-$C_7$)hétérocyclyle, un [($C_1$-$C_7$)Alkyle]$_n$-Z ou un [($C_1$-$C_7$)Alkyle-Z]$_n$ Z étant un hétéroatome choisi parmi N, O ou S, en particulier choisi parmi $NR^aR^b$ ou $OR^e$, et n étant un entier compris de 1 à 7 ;
  $OR^e$, OAc, OTs, OTf, $SR^e$, $SO_2R^e$, $COR^e$, $NR^aSO_2R^e$, $NHCOOR^e$;
  $R^e$ représente un H, un ($C_1$-$C_7$)alkyle, un aryle, un hétéroaryle, un ($C_3$-$C_7$)hétérocyclyle, un ($C_3$-$C_7$)carbocyclyle, un ($C_1$-$C_7$)alkyle-aryle, un ($C_1$-$C_7$)alkyle-hétéroaryle ou un ($C_1$-$C_7$)alkyle-($C_3$-$C_7$)hétérocyclyle ;

- $R_2$ étant choisi parmi :
  H, $SO_2Ph$, $COR^e$, $NR^cCONR^aR^b$, $COOR^e$, OH, $R^d$ ;
- $R_3$ étant choisi parmi :

  Halogène, $NO_2$, CN, diméthyltriazène, triméthylammonium, aryliodonium, $NR^aR^b$, $SO_2NR^aR^b$, $CONR^aR^b$, $NR^cSO_2NR^aR^b$, $NR^cCONR^aR^b$, $NR^aCOR^b$;
  Sn(Alkyle)$_3$, Alkyle étant choisi parmi méthyle ou *n*-butyle ;
  $B(OH)_2$, B(pinacol) ;
  $R^d$, $CH_2R^d$,
  $OR^e$, OAc, OTs, OTf, O(hétéroaryle) notamment O(HOBt), $SR^e$, $SO_2R^e$, $COR^e$, $NR^aSO_2R^e$, $NHCOOR^e$;

- $R_4$ étant choisi parmi :
  Halogène, $CH_2NR^aR^b$, $R^a$, $COOR^e$, CHO, $CH_2OR^e$ ;

dans lequel aucun des substituants $R_1$, $R_2$, $R_3$ et $R_4$ ne comprend de radioélément, pour la préparation de composés de formule II, II-1, II-1a ou II-2 selon l'une des revendications 1 à 9.

**11.** Composé de formule II, II-1, II-1a ou II-2 selon l'une des revendications 1 à 9 dans lequel au moins l'un des

substituants $R_1$, $R_2$, $R_3$ ou $R_4$ comprend un radioélément choisi parmi $^{18}F$, $^{11}C$, $^{123}I$ et $^{124}I$, pour son utilisation dans un procédé de diagnostic ou d'imagerie in vivo d'un sujet atteint d'une maladie neuro-dégénérative, dans lequel ledit procédé de diagnostic ou d'imagerie comprend l'administration dudit composé.

**12.** Composé de formule II, II-1, II-1a ou II-2 pour son utilisation selon la revendication 11, dans un procédé de diagnostic ou d'imagerie in vivo d'un sujet atteint d'une maladie neuro-dégénérative, dans lequel le procédé d'imagerie in vivo est la tomographie par émission de positrons ou la tomographie d'émission monophotonique.

**13.** Composé de formule II, II-1, II-1a ou II-2 pour son utilisation selon l'une des revendications 11 ou 12, dans un procédé de diagnostic ou d'imagerie in vivo d'un sujet atteint d'une maladie neuro-dégénérative, ladite maladie neuro-dégénérative étant une amyloïdopathie, une alpha-synucléinopathie ou une tauopathie.

**Patentansprüche**

**1.** Verbindung der Formel II,

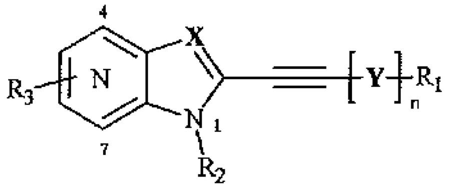

(II)

- **wobei X ausgewählt ist aus** N oder C-$R_4$
- **wobei Y ausgewählt ist aus** einem Alken oder Alkin, Aryl oder Heteroaryl, einem Alkan mit 1 bis 7 Kohlenstoffatomen, wobei n eine ganze Zahl gleich 0 oder 1 ist;
- **wobei $R_1$ ausgewählt ist aus** einem Aryl oder Heteroaryl, ausgewählt aus Phenyl-, Pyrimidin-, Pyridin-, Thiophen-, Furan-, Triazol-, Oxazol-, (Aza)Indol-, (Aza)Indolin-, (Aza)Benzimidazolgruppen, die optional in einer oder mehreren Stellen durch eine Gruppe substituiert sind, die ausgewählt ist aus:

**Halogen, $NO_2$, CN, Dimethyltriazol, Trimethylammonium, Aryliodonium, $NR^aR^b$, $SO_2NR^a R^b$, $CONR^aR^b$, $NR^cSO_2NR^aR^b$, $NR^cCONR^aR^b$, $NR^aCOR^b$;**
wobei $R^a$, $R^b$ und $R^c$ unabhängig voneinander für ein H, ein ($C_1$-$C_7$)-Alkyl, ein Aryl, ein Heteroaryl, ein ($C_3$-$C_7$)-Carbocyciyl, ein ($C_1$-$C_7$)-Alkyl-Aryl, ein ($C_1$-$C_7$)-Alkylheteroaryl stehen oder wobei $R^a$ und $R^b$ zusammen ein ($C_3$-$C_7$)-Heterocyclyl bilden;
**$Sn(Alkyl)_3$, wobei Alkyl ausgewählt** ist **aus Methyl oder *n*-Butyl;**
**$B(OH)_2$, B(Pinakol);**
**$R^d$, $CH_2R^d$;**
wobei $R^d$ für ein H, ein ($C_1$-$C_7$)-Alkyl, ein Aryl, ein Heteroaryl, ein ($C_3$-$C_7$)-Heterocyclyl, ein ($C_3$-$C_7$)-Carbocyclyl, ein ($C_1$-$C_7$)-Alkyl-Aryl, ein ($C_1$-$C_7$)-Alkyl-Heteroaryl, ein ($C_1$-$C_7$)-Alkyl-($C_3$-$C_7$)-Heterocyclyl, ein $[(C_1\text{-}C_7)\text{-Alkyl}]_n$-Z oder ein $[(C_1\text{-}C_7)\text{-Alkyl-Z}]_n$ steht, wobei Z ein Heteroatom ist, ausgewählt aus N, O oder S, insbesondere ausgewählt aus $NR^aR^b$ oder $R^e$, und n eine ganze Zahl von 1 bis 7 ist;
**$OR^6$, OAc, OTs, OTf, $SR^e$, $SO_2R^e$, $COR^6$, $NR^aSO_2R^e$, $NHCOOR^e$;**
wobei $R^e$ für ein H, ein ($C_1$-$C_7$)-Alkyl, ein Aryl, ein Heteroaryl, ein ($C_3$-$C_7$)-Heterocyclyl, ein ($C_3$-$C_7$)-Carbocyclyl, ein ($C_1$-$C_7$)-Alkyl-Aryl, ein ($C_1$-$C_7$)-Alkyl-Heteroaryl oder ein ($C_1$-$C_7$)-Alkyl-($C_3$-$C_7$)-Heterocyclyl steht;

- **wobei $R_2$ ausgewählt ist aus:**
H, $SO_2Ph$, $COR^e$, $NR^cCONR^aR^b$, $COOR^e$, OH, $R^d$;
- **wobei $R_3$ ausgewählt ist aus:**

Halogen, $NO_2$, CN, Dimethyltriazol, Trimethylammonium, Aryliodonium, $NR^aR^b$, $SO_2NR^aR^b$, $CONR^aR^b$, $NR^cSO_2NR^aR^b$, $NR^cCONR^aR^b$, $NR^aCOR^b$;
$Sn(Alkyl)_3$, wobei Alkyl ausgewählt ist aus Methyl oder *n*-Butyl;
$B(OH)_2$, B(Pinakol);
$R^d$, $CH_2R^d$, $OR^e$, OAc, OTs, OTf, O(Heteroaryl), insbesondere O(HOBt), $SR^e$, $SO_2R^e$, $COR^e$, $NR^aSO_2R^e$, $NHCOOR^e$;

- **wobei $R^4$ ausgewählt ist aus:**

  Halogen, $CH_2NR^aR^b$, $R^a$, $COOR^e$, CHO, $CH_2OR^e$;
  wobei mindestens einer der Substituenten $R_1$ $R_2$, $R_3$ oder $R_4$ ein aus $^{18}F$, $^{11}C$, $^{123}I$ und $^{124}I$ ausgewähltes Radioelement umfasst.

**2.** Verbindung nach Anspruch 1 der allgemeinen Formel II-1:

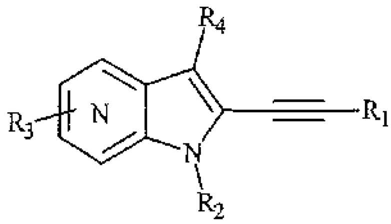

(II-1)

wobei $R_1$, $R_2$, $R_3$ und $R_4$ wie oben in Formel II definiert sind;
wobei mindestens einer der Substituenten $R_1$ $R_2$, $R_3$ oder $R_4$ ein aus $^{18}F$, $^{11}C$, $^{123}I$ und $^{124}I$ ausgewähltes Radioelement umfasst.

**3.** Verbindung nach einem der Ansprüche 1 bis 2, wobei die Verbindung die allgemeine Formel II-1 hat:

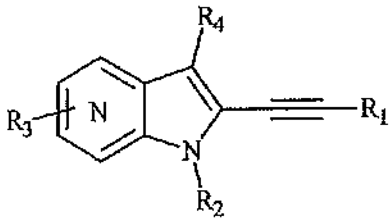

(II-1)

wobei $R_2$, $R_3$ und $R_4$ wie oben in Formel II definiert sind;
wobei $R_1$ optional markiert und ausgewählt ist aus:

- 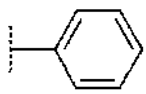 ;
- 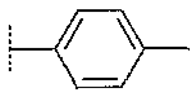 ;
- 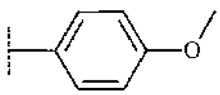 ;
- 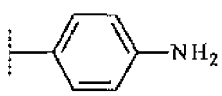 ou 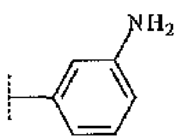 ;

- 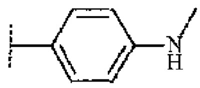 ;
- 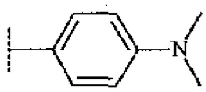 ou 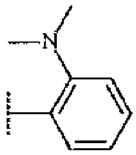 ou 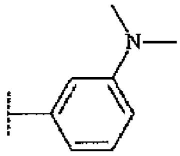 ;
- 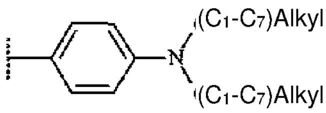 , 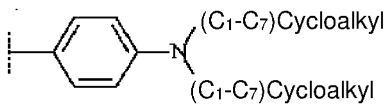 .

insbesondere

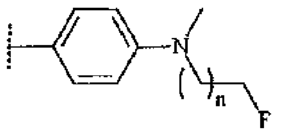 ,

wobei n von 0 bis 6 beträgt;

- 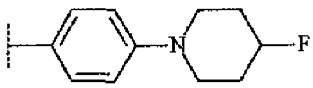 ;
- 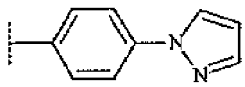 ;
- 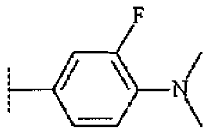 ou 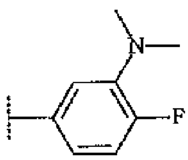 ;
- 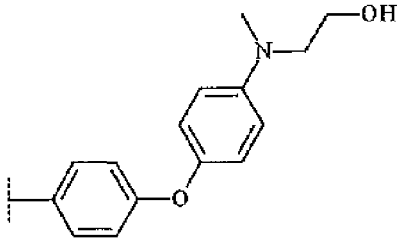 ; 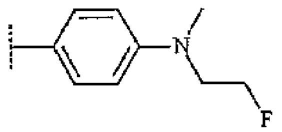 ;

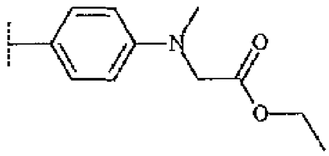
;

- 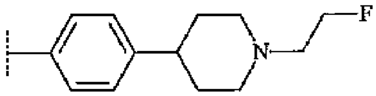 ;

- 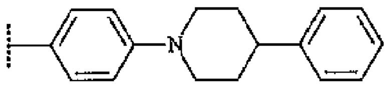 ;

- 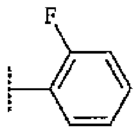 ; 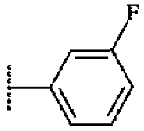 ; 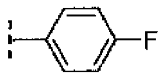 ;

- 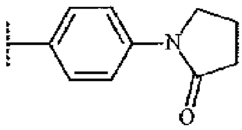 ;

- 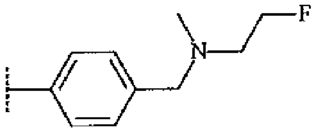 ;

- 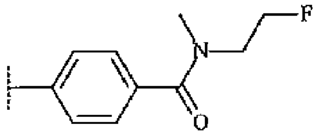 ;

- 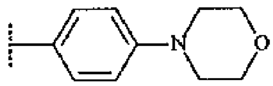 ;

- 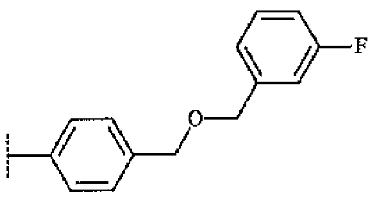 ;

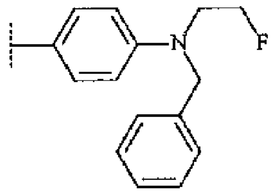
;

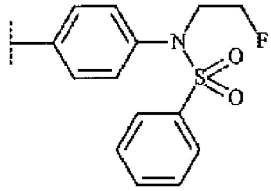
;

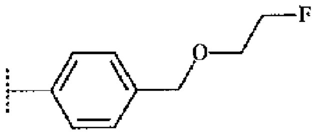
;

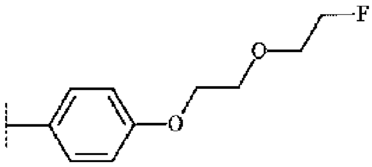
;

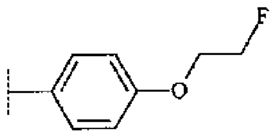
;

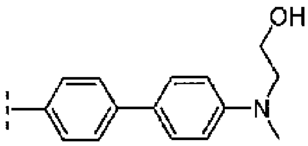
;

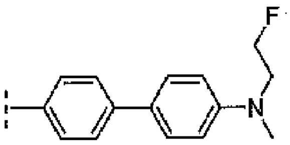
;

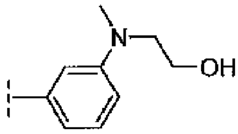
;

- 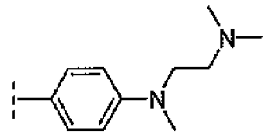 ;

- 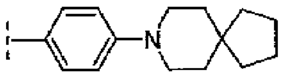 ;

- 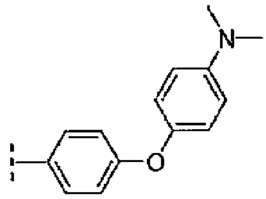 ;

- 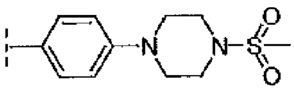 ;

- 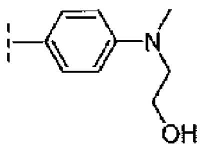 ;

- 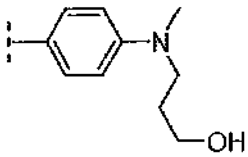 ;

- 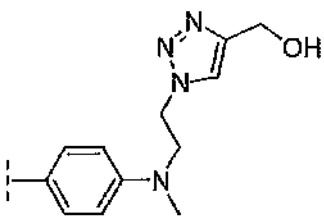 ;

- 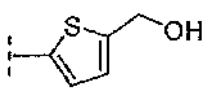 ;

- 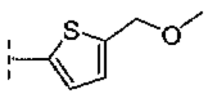 ;

- 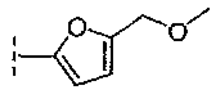 ;

- 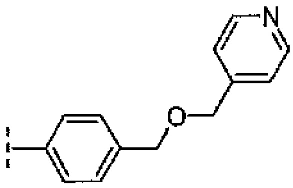 ;

- 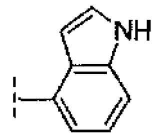 .

**4.** Verbindung nach einem der Ansprüche 1 bis 3, wobei die Verbindung die allgemeine Formel II-1 hat:

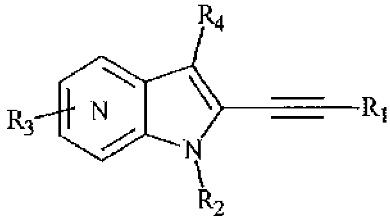

(II-1)

wobei $R_1$, $R_3$ und $R_4$ wie oben in Formel II definiert sind;
wobei $R_2$ optional markiert und ausgewählt ist aus:

- H;
- $(CH_2CH_2O)_n$-$CH_2CH_2F$, wobei n eine ganze Zahl gleich 0, 1 oder 2 ist;
- $SO_2Ph$;
- $COO^tBu$;
- $CH_2OCH_3$;
- $CH_2O(CH_2)_2OCH_3$;
- $COCH_3$;
- 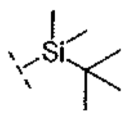 .

**5.** Verbindung nach einem der Ansprüche 1 bis 4, wobei die Verbindung die allgemeine Formel II-1 hat:

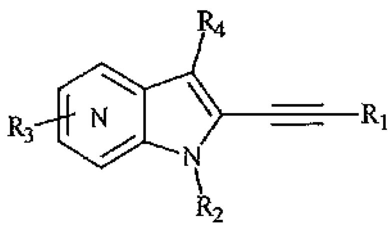

(II-1)

wobei $R_1$, $R_2$ und $R_4$ wie oben in Formel II definiert sind;
wobei $R_3$ optional markiert und ausgewählt ist aus:

- 6-Cl;
- 6-F;
- 5-F;
- 4-F;
- 6-(2-Thienyl);
- 6-(3-Thienyl);
- 6-(6-Fluor-3-pyridyl);
- 5-OMe;
- 6-Morpholino;
- 5-Morpholino;
- 6-Br;
- 4-Br;
- 4-Bpin;
- 5-Bpin.

**6.** Verbindung nach einem der Ansprüche 1 bis 5, wobei die Verbindung die allgemeine Formel II-1 hat:

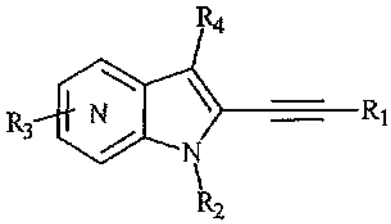

(II-1)

wobei $R_1$, $R_2$ und $R_3$ wie oben in Formel II definiert sind;
wobei $R_4$ ausgewählt ist aus:

- H;

$(CH_2)_n$-N-Alkyl, wobei n eine ganze Zahl gleich 0, 1 oder 2 ist.

**7.** Verbindung nach einem der Ansprüche 1 bis 6, wobei die Verbindung die allgemeine Formel II-1a hat:

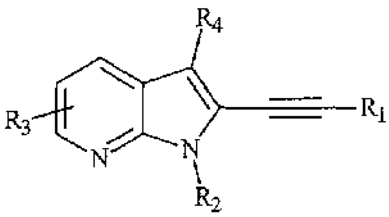

(II-1a)

wobei $R_2$, $R_3$ und $R_4$ wie oben in Formel II definiert sind;
wobei $R_1$ optional markiert und ausgewählt ist aus:

- 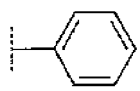 ;

- 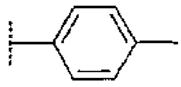 ;

- 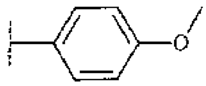 ;

- 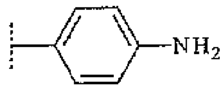 ou 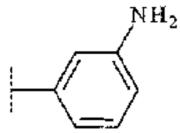 ;

- 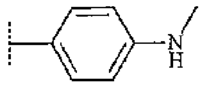 ;

- 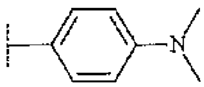 ou 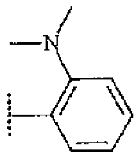 ou 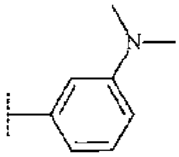 ;

- 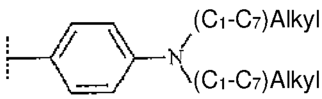 ou 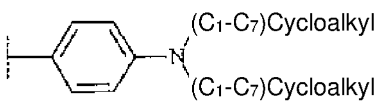 ,

insbesondere

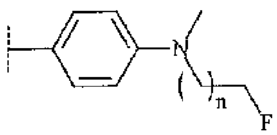 ,

wobei n von 0 bis 6 beträgt;

- 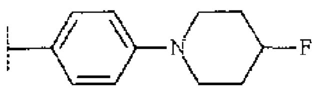 ;

- 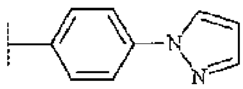 ;

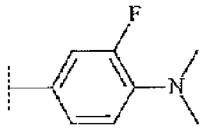 ou 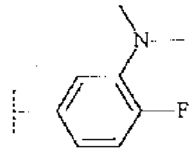 ;

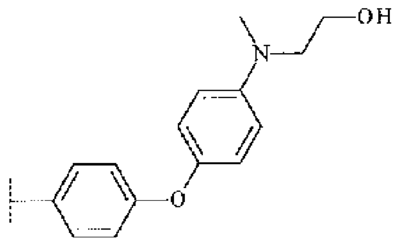 ; 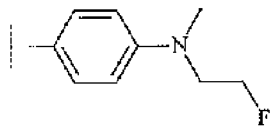 ;

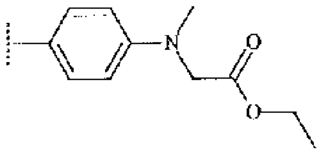 ;

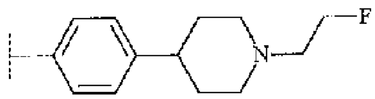 ;

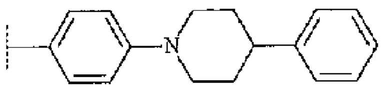 ;

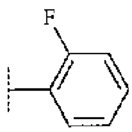 ; 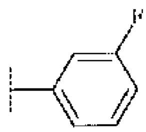 ; 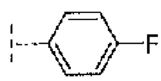 ;

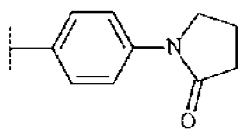 ;

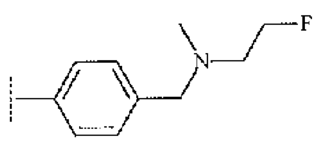 ;

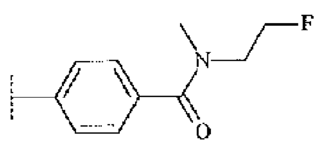 ;

- 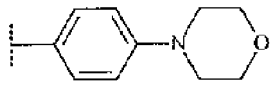 ;

- 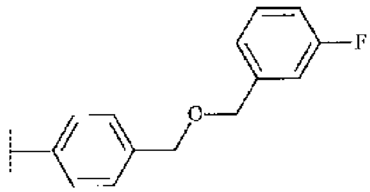 ;

- 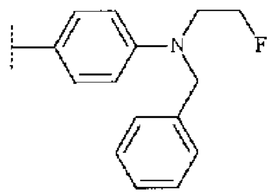 ;

- 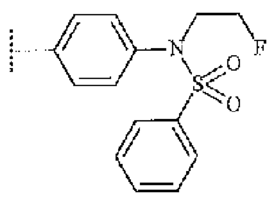 ;

- 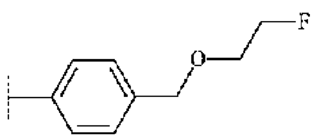 ;

- 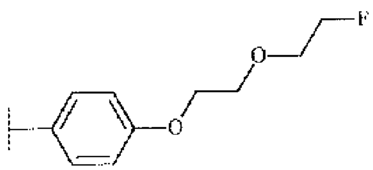 ;

- 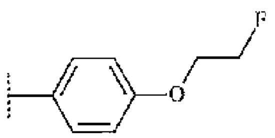 ;

- 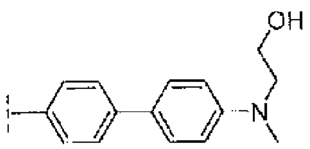 ;

- 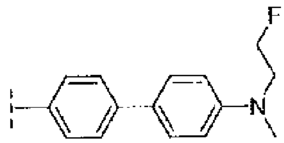 ;

- 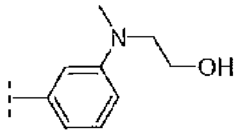 ;

- 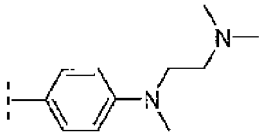 ;

- 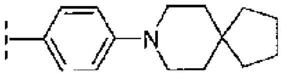 ;

- 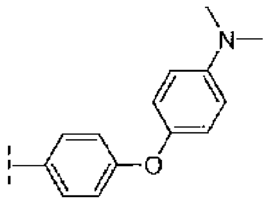 ;

- 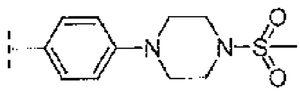 ;

- 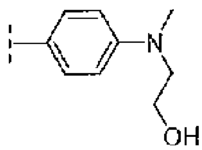 ;

- 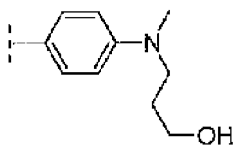 ;

- 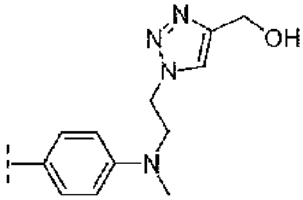 ;

- 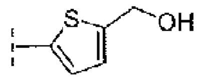 ;

- 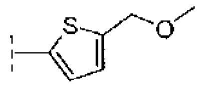 ;

- 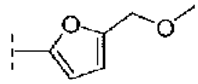 ;

- 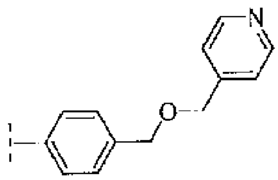 ;

- 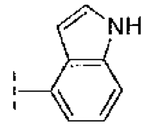 .

**8.** Verbindung nach einem der Ansprüche 1 bis 7, wobei die Verbindung die allgemeine Formel II-2 hat:

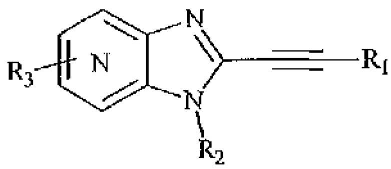

(II-2)

wobei $R_1$, $R_2$ und $R_3$ wie oben in Formel II definiert sind;
wobei mindestens einer der Substituenten $R_1$ $R_2$, oder $R_3$ ein aus $^{18}F$, $^{11}C$, $^{123}I$ und $^{124}I$ ausgewähltes Radioelement umfasst.

**9.** Verbindung nach einem der Ansprüche 1 bis 8, wobei die Verbindung der Formel II ausgewählt ist aus Verbindungen der folgenden Formel:

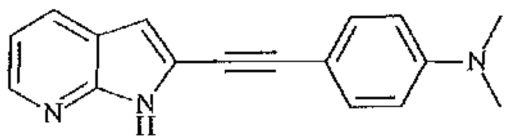

**22a**

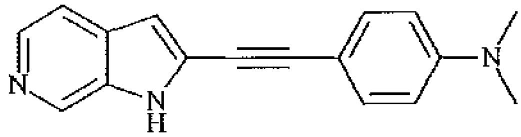

**22b**

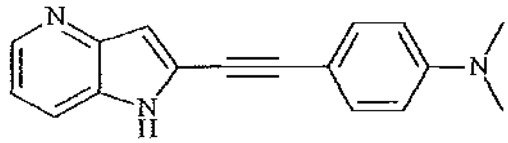

22c

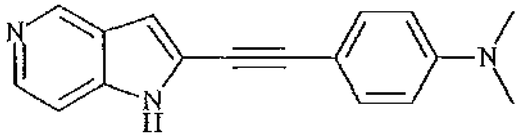

22d

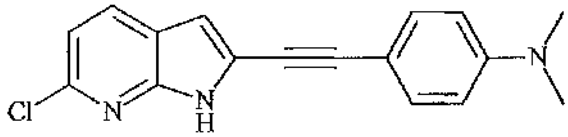

22e

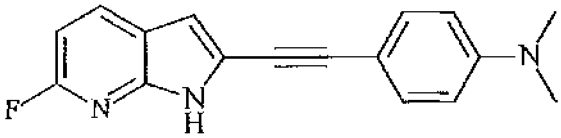

22f

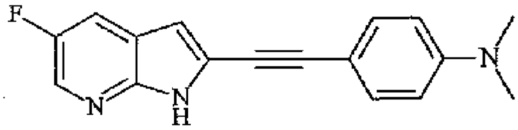

22g

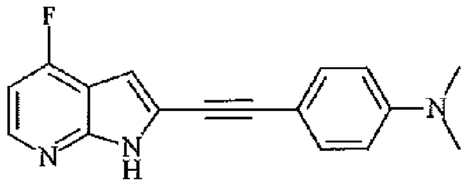

22h

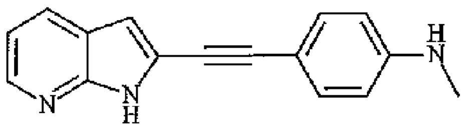

22i

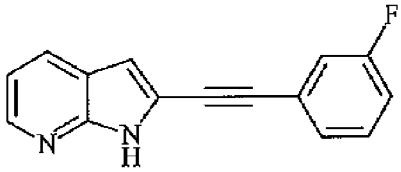

22k

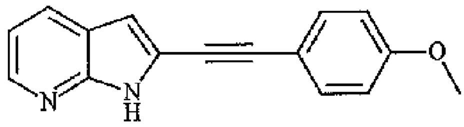

22m

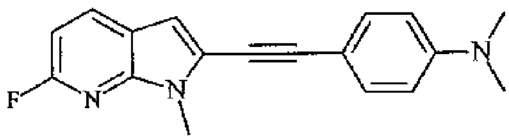

22n

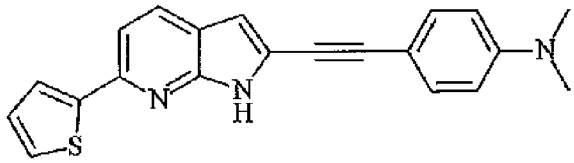

25a

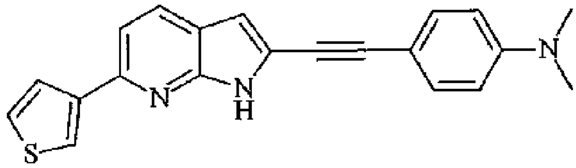

25b

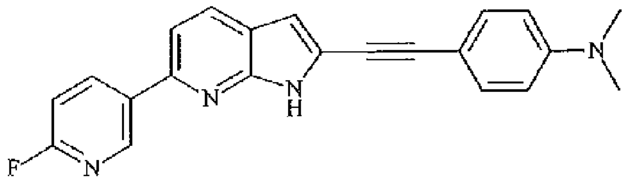

25c

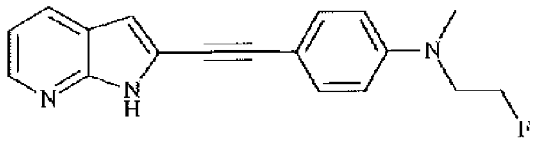

28

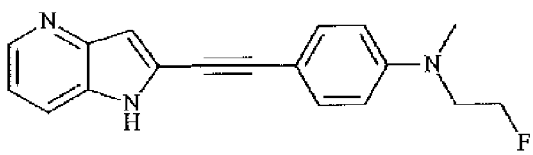

35

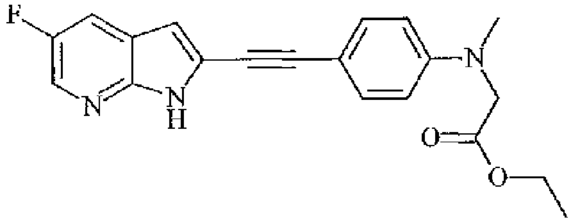

37

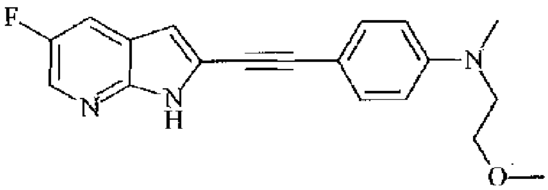
38

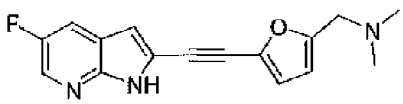
131

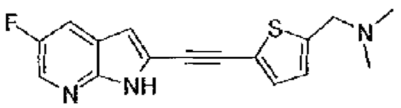
133

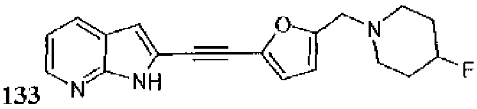
128

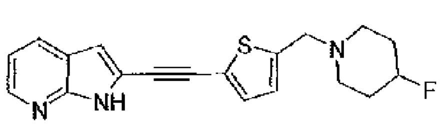
124

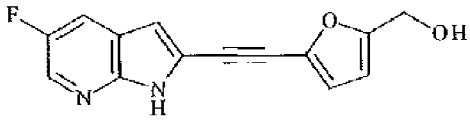
120

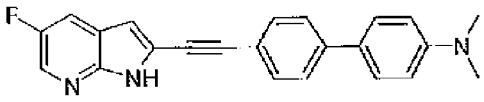
116

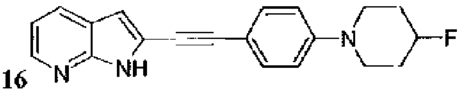
113

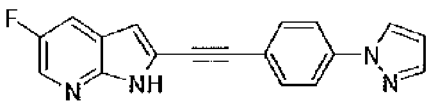
108

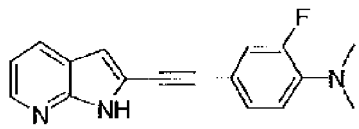
103

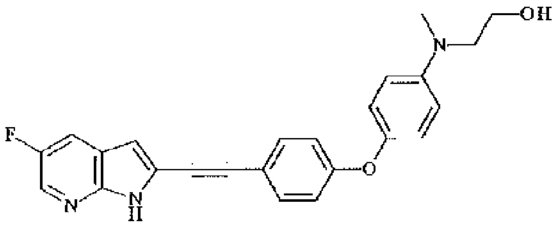
97

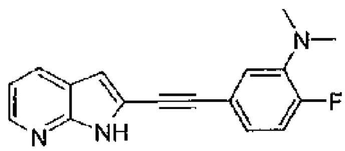
93

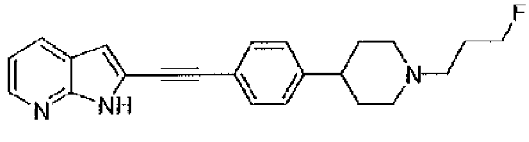
85

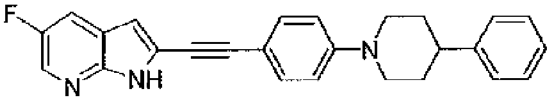
90

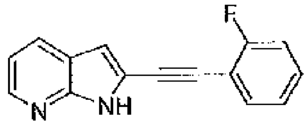
84

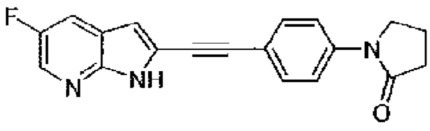
79

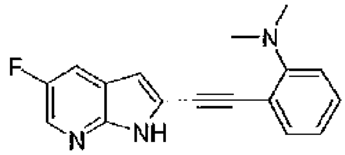
76

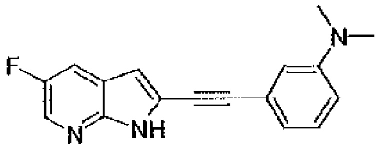
74

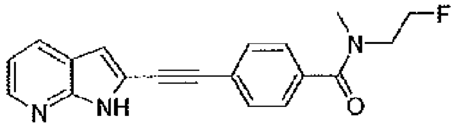
71

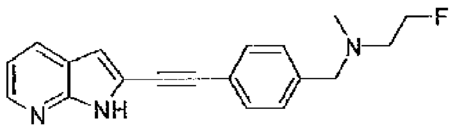
67

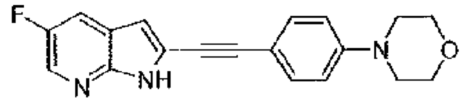
64

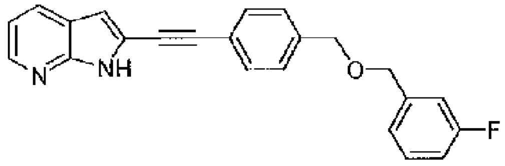
61

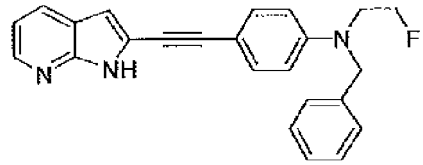
52

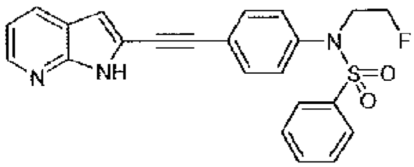
57

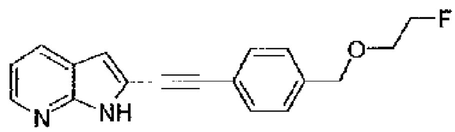
44

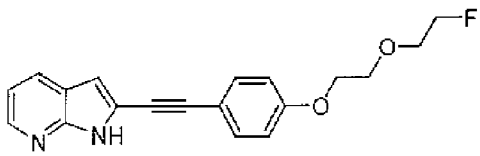
48

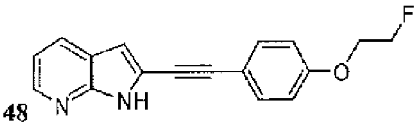
43

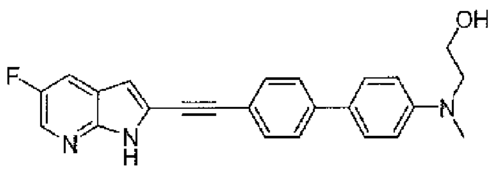

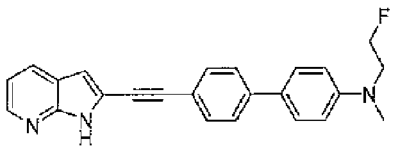

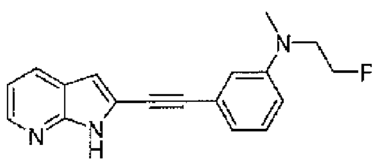

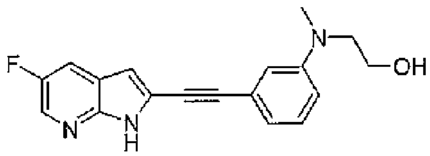

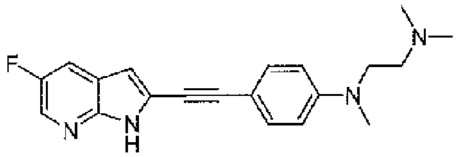

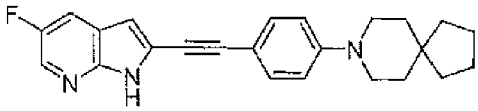

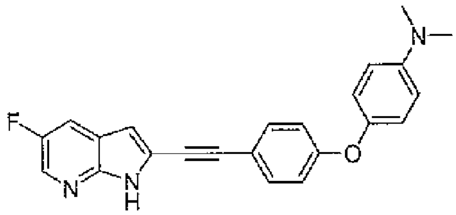

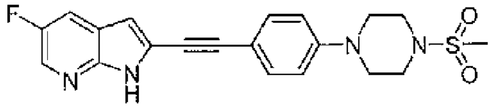

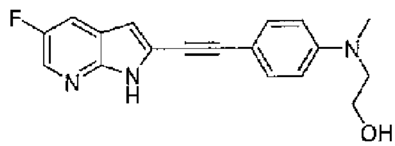

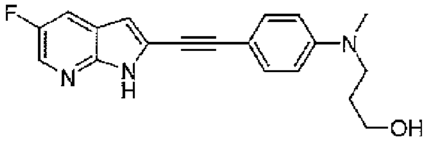

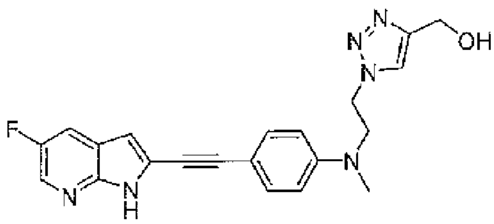

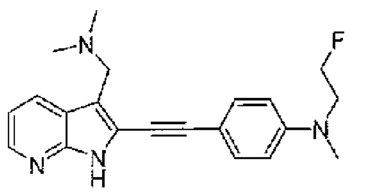

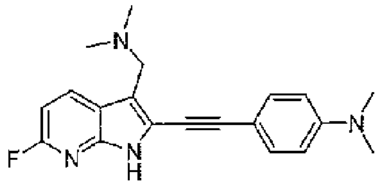

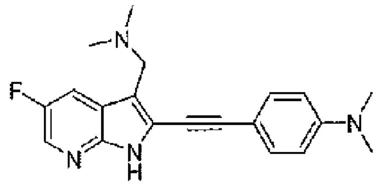

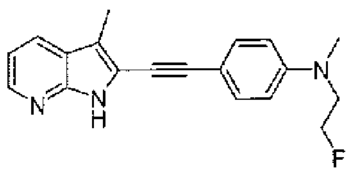

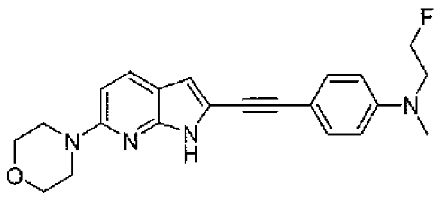

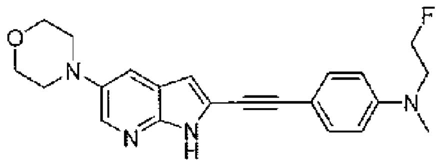

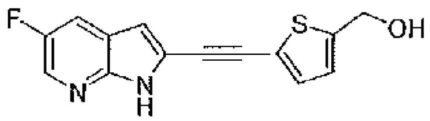

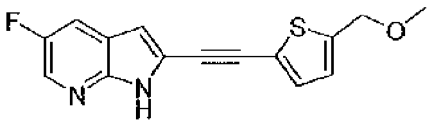

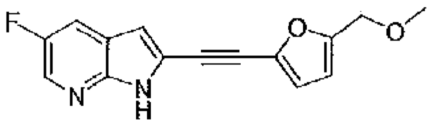

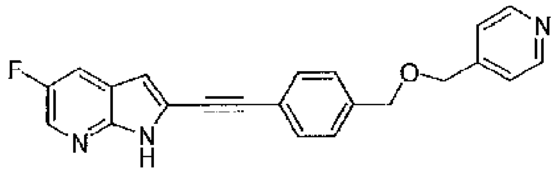

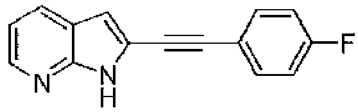

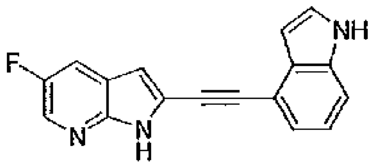

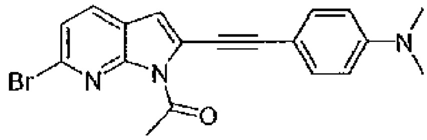

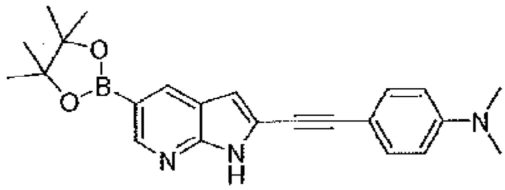

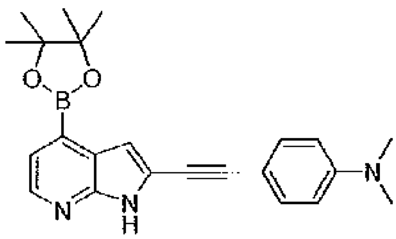

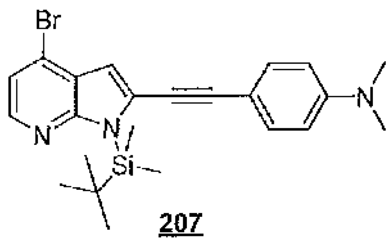

207

im radioaktiv markierten Zustand;
wobei insbesondere die Verbindung der Formel II folgende ist:

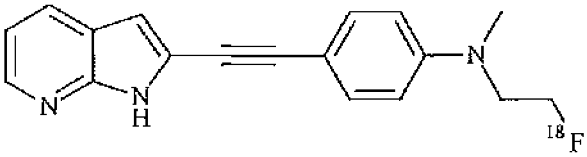

[$^{18}$F]-28

**10.** Verwendung einer Verbindung der Formel III:

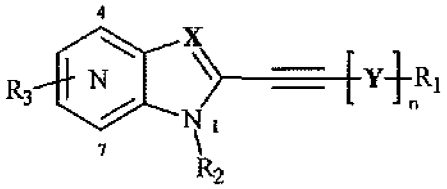

(III)

- wobei X ausgewählt ist aus N oder C-$R_4$
- wobei Y ausgewählt ist aus einem Alken oder einem Alkin, einem Aryl oder einem Heteroaryl, einem Alkan mit 1 bis 7 Kohlenstoffatomen, wobei n eine ganze Zahl gleich 0 oder 1 ist;
- wobei $R_1$ ausgewählt ist aus einem Aryl oder Heteroaryl, ausgewählt aus Phenyl-, Pyrimidin-, Pyridin-, Thiophen-, Furan-, Triazol-, Oxazol-, (Aza)Indol-, (Aza)Indolin-, (Aza)Benzimidazolgruppen, die optional in einer oder mehreren Stellen durch eine Gruppe substituiert sind, die ausgewählt ist aus:

Halogen, $NO_2$, CN, Dimethyltriazol, Trimethylammonium, Aryliodonium, $NR^aR^b$, $SO_2NR^aR^b$, $CONR^aR^b$, $NR^cSO_2NR^aR^b$, $NR^cCONR^aR^b$, $NR^aCOR^b$;
wobei $R^a$, $R^b$ und $R^c$ unabhängig voneinander für ein H, ein ($C_1$-$C_7$)-Alkyl, ein Aryl, ein Heteroaryl, ein ($C_3$-$C_7$)-Carbocyclyl, ein ($C_1$-$C_7$)-Alkyl-Aryl, ein ($C_1$-$C_7$)-Alkyl-Heteroaryl stehen oder wobei $R^a$ und $R^b$ zusammen ein ($C_3$-$C_7$)-Heterocyclyl bilden;
$Sn(Alkyl)_3$, wobei Alkyl ausgewählt ist aus Methyl oder *n*-Butyl;
$B(OH)_2$, B(Pinakol);
$R^d$, $CH_2R^d$;
wobei $R^d$ für ein H, ein ($C_1$-$C_7$)-Alkyl, ein Aryl, ein Heteroaryl, ein ($C_3$-$C_7$)-Heterocyclyl, ein ($C_3$-$C_7$)-Carbocyclyl, ein ($C_1$-$C_7$)-Alkyl-Aryl, ein ($C_1$-$C_7$)-Alkyl-Heteroaryl, ein ($C_1$-$C_7$)-Alkyl-($C_3$-$C_7$)-Heterocyclyl, ein $[(C_1\text{-}C_7)\text{-Alkyl}]_n$-Z oder ein $[(C_1\text{-}C_7)\text{-Alkyl-Z}]_n$ steht, wobei Z ein Heteroatom ist, ausgewählt aus N, O oder S, insbesondere ausgewählt aus $NR^aR^b$ oder $OR^e$, und n eine ganze Zahl von 1 bis 7 ist;
$OR^e$, OAc, OTs, OTf, $SR^e$, $SO_2R^e$, $COR^e$, $NR^aSO_2R^e$, $NHCOOR^e$;
wobei $R^e$ für ein H, ein ($C_1$-$C_7$)-Alkyl, ein Aryl, ein Heteroaryl, ein ($C_3$-$C_7$)-Heterocyclyl, ein ($C_3$-$C_7$)-Carbocyclyl, ein ($C_1$-$C_7$)-Alkyl-Aryl, ein ($C_1$-$C_7$)-Alkyl-Heteroaryl oder ein ($C_1$-$C_7$)-Alkyl-($C_3$-$C_7$)-Heterocyclyl steht;

- wobei $R_2$ ausgewählt ist aus:
H, $SO_2Ph$, $COR^e$, $NR^cCONR^aR^b$, $COOR^e$, OH, $R^d$;
- wobei $R_3$ ausgewählt ist aus:

Halogen, $NO_2$, CN, Dimethyltriazol, Trimethylammonium, Aryliodonium, $NR^aR^b$, $SO_2NR^aR^b$, $CONR^aR^b$, $NR^cSO_2NR^aR^b$, $NR^cCONR^aR^b$, $NR^aCOR^b$;
$Sn(Alkyl)_3$, wobei Alkyl ausgewählt ist aus Methyl oder *n*-Butyl;
$B(OH)_2$, B(Pinakol);
$R^d$, $CH_2R^d$, $OR^e$, OAc, OTs, OTf, O(Heteroaryl), insbesondere O(HOBt), $SR^e$, $SO_2R^e$, $COR^e$, $NR^aSO_2R^e$, $NHCOOR^e$;

- wobei $R^4$ ausgewählt ist aus:

Halogen, $CH_2NR^aR^b$, $R^a$, $COOR^e$, CHO, $CH_2OR^e$;
wobei keiner der Substituenten $R_1$, $R_2$, $R_3$ und $R^4$ ein Radioelement umfasst, zur Herstellung von Verbindungen der Formel II, II-1, II-1a oder II-2 nach einem der Ansprüche 1 bis 9.

**11.** Verbindung der Formel II, II-1, II-1a oder II-2 nach einem der Ansprüche 1 bis 9, wobei mindestens einer der Substituenten $R_1$, $R_2$, $R_3$ oder $R^4$ ein aus $^{18}F$, $^{11}C$, $^{123}I$ und $^{124}I$ ausgewähltes Radioelement umfasst, zur Verwendung in einem Verfahren zur in-vivo-Diagnose oder -Bildgebung eines Patienten mit einer neurodegenerativen Erkrankung, wobei das Verfahren zur Diagnose oder Bildgebung das Verabreichen der Verbindung umfasst.

**12.** Verbindung der Formel II, II-1, II-1a oder II-2 zur Verwendung nach Anspruch 11 in einem Verfahren zur in-vivo-Diagnose oder -Bildgebung eines Patienten mit einer neurodegenerativen Erkrankung, wobei das Verfahren zur in-vivo-Bildgebung die Positronenemissionstomographie oder Einzelphotonenemissionstomographie ist.

**13.** Verbindung der Formel II, II-1, II-1a oder II-2 zur Verwendung gemäß einem der Ansprüche 11 oder 12 in einem

Verfahren zur in-vivo-Diagnose oder -Bildgebung eines Patienten mit einer neurodegenerativen Krankheit, wobei die neurodegenerative Krankheit Amyloidopathie, Alpha-Synucleinopathie oder Tauopathie ist.

## Claims

**1.** Compound of formula II

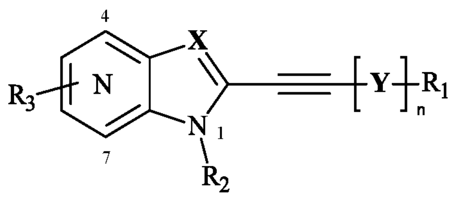

(II)

- **X being selected from** N or C-$R_4$
- **Y being selected from** an alkene or an alkyne, an aryl or a heteroaryl, an alkane comprising from 1 to 7 carbon atoms, n being an integer equal to 0 or 1;
- **$R_1$ being selected from** aryl or heteroaryl selected from phenyl, pyrimidine, pyridine, thiophene, furan, triazole, oxazole, (aza)indole, (aza)indoline, (aza)benzimidazole optionally substituted at one or more positions with a group selected from:

**Halogen, $NO_2$, CN, dimethyltriazene, trimethylammonium, aryliodonium,**
**$NR^aR^b$, $SO_2NR^aR^b$, $CONR^aR^b$, $NR^cSO_2NR^aR^b$, $NR^cCONR^aR^b$, $NR^aCOR^b$;**
$R^a$, $R^b$ and $R^c$ are independently of each other H, ($C_1$-$C_7$) alkyl, aryl, heteroaryl, ($C_3$-$C_7$) carbocyclyl, ($C_1$-$C_7$) alkyl-aryl, ($C_1$-$C_7$) alkyl-heteroaryl, or $R^a$ and $R^b$ together form a ($C_3$-$C_7$) heterocyclyl;
**$Sn(Alkyl)_3$, Alkyl is selected from methyl or n-butyl;**
**$B(OH)_2$, B(pinacol);**
**$R^d$, $CH_2R^d$;**
$R^d$ represents H, ($C_1$-$C_7$)alkyl, aryl, heteroaryl, ($C_3$-$C_7$) heterocyclyl, ($C_3$-$C_7$) carbocyclyle, ($C_1$-$C_7$)alkyl-aryl, ($C_1$-$C_7$)alkyl-heteroaryl, ($C_1$-$C_7$) alkyl-($C_3$-$C_7$)heterocyclyl, $[(C_1$-$C_7)$ alkyl$]_n$-Z or a $[(C_1$-$C_7)$ alkyl-Z$]_n$ Z being a heteroatom selected from N, O or S, in particular selected from $NR^aR^b$ or $R^e$, and n being an integer comprised from 1 to 7;
**$OR^e$, OAc, OTs, OTf, $SR^e$, $SO_2R^e$, $COR^e$, $NR^eSO_2R^e$, $NHCOOR^e$;**
$R^e$ is H, ($C_1$-$C_7$)alkyl, aryl, heteroaryl, ($C_3$-$C_7$)heterocyclyl, ($C_3$-$C_7$)carbocyclyl, ($C_1$-$C_7$)alkyl-aryl, ($C_1$-$C_7$)alkyl-heteroaryl or ($C_1$-$C_7$) alkyl-($C_3$-$C_7$) heterocyclyl;

- **$R_2$ being selected from:**
H, $SO_2Ph$, $COR^e$, $NR^cCONR^aR^b$, $COOR^e$, OH, $R^d$;
- **$R_3$ being selected from:**

Halogen, $NO_2$, CN, dimethyltriazene, trimethylammonium, aryliodonium,
$NR^aR^b$, $SO_2NR^aR^b$, $CONR^aR^b$, $NR^cSO_2NR^aR^b$, $NR^cCONR^aR^b$, $NR^aCOR^b$;
$Sn(Alkyl)_3$, Alkyl being selected from methyl or *n*-butyl;
$B(OH)_2$, B (pinacol);
$R^d$, $CH_2R^d$;
$OR^e$, OAc, OTs, OTf, O(heteroaryl), especially O(HOBt), $SR^e$, $SO_2R^e$, $COR^e$, $NR^aSO_2R^e$, $NHCOOR^e$;

- **$R_4$ being selected from:**

Halogen, $CH_2NR^aR^b$, $R^a$, $COOR^e$, CHO, $CH_2OR^e$;
wherein at least one of the substituents $R_1$, $R_2$ $R_3$ or $R_4$ comprises a radioelement selected from $^{18}F$, $^{11}C$, $^{123}I$ and $^{124}I$.

**2.** A compound according to claim 1 of the general formula II-1:

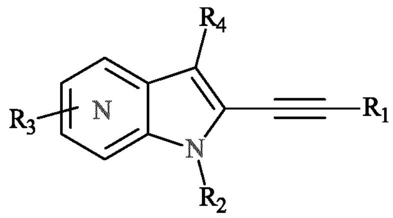

(II-1)

$R_1$, $R_2$, $R_3$ and $R_4$ being as previously defined in formula II; wherein at least one of the substituents $R_1$, $R_2$, $R_3$ or $R_4$ comprises a radioelement selected from $^{18}F$, $^{11}C$, $^{123}I$ and $^{124}I$.

**3.** Compound according to any of claims 1 to 2, said compound being of general formula II-1:

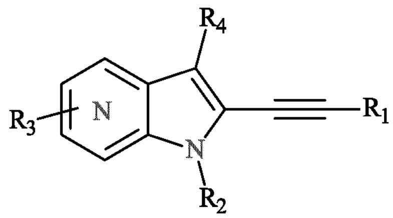

(II-1)

$R_2$, $R_3$ and $R_4$ being as previously defined in formula II;
$R_1$ being optionally labeled and selected from

- 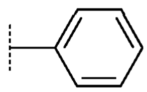 ; 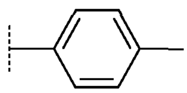 ; 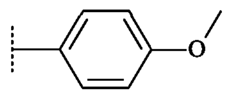 ; 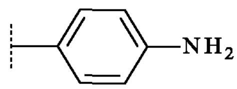

or

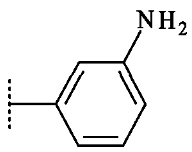 ; 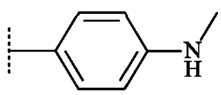 ; 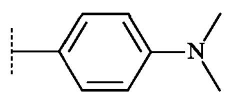 or 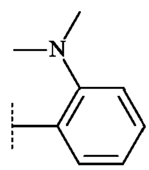

or

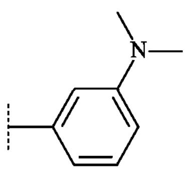 ; 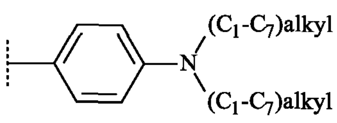 or 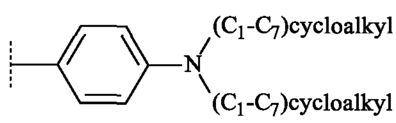 ,

particularly

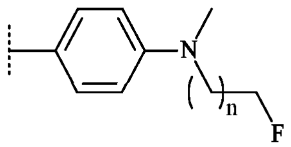
,

n being comprised from 0 to 6;

- 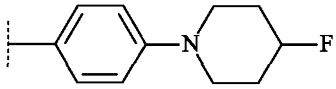 ; 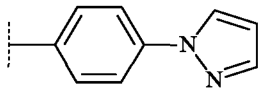 ; 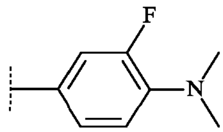

or

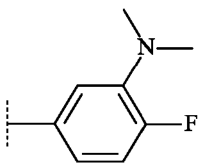 ; 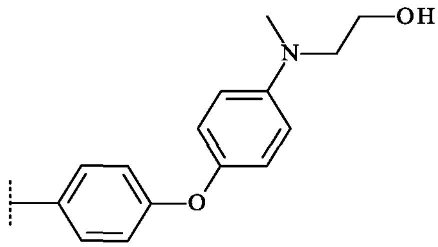 ;

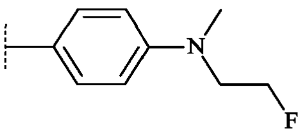 ; 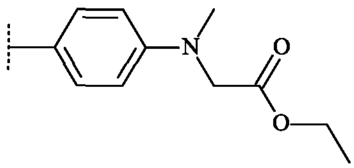 ;

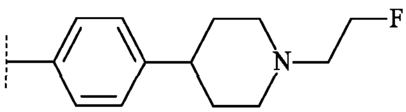 ; 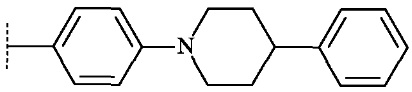 ;

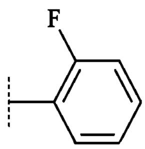 ; 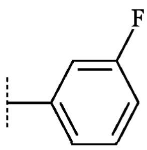 ; 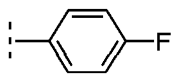 ;

- 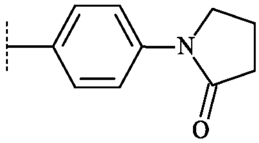 ; 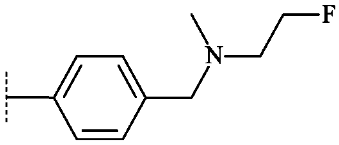 ; 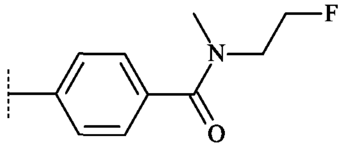 ;

- 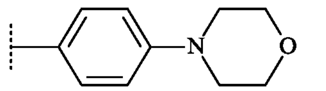 ; 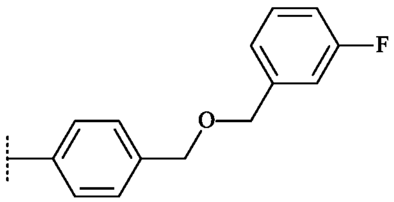 ;

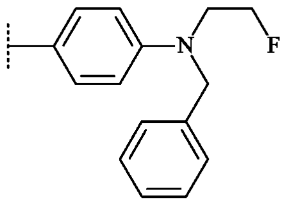 ; 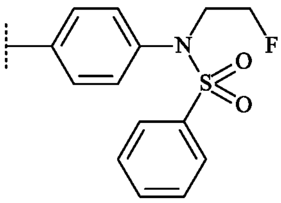 ; 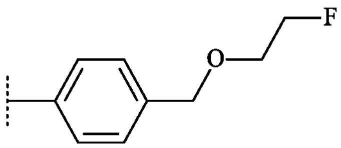 ;

- 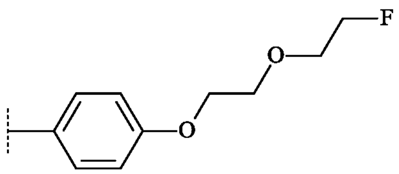 ; 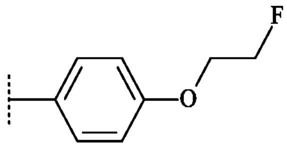 ;

- 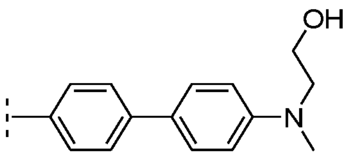 ; 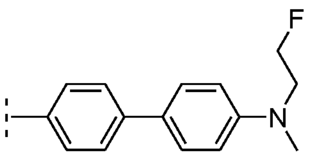 ; 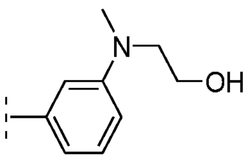 ;

- 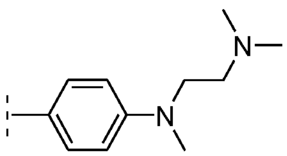 ; 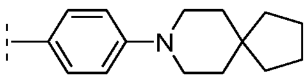 ; 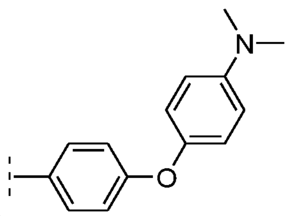 ;

- 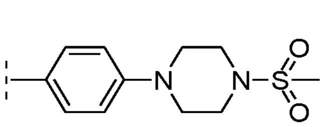 ; 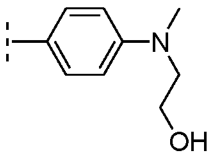 ; 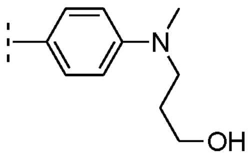 ;

- 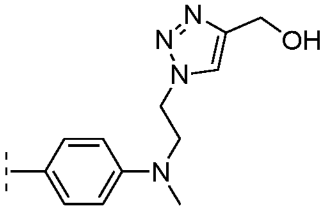 ; 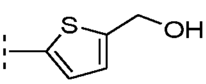 ; 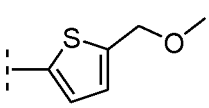 ; 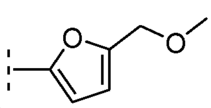 ;

- 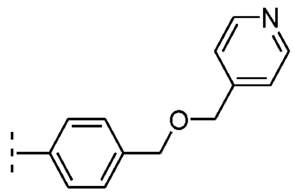 ; 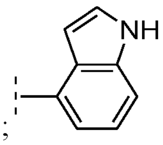 .

4. Compound according to any of claims 1 to 3, said compound being of general formula II-1:

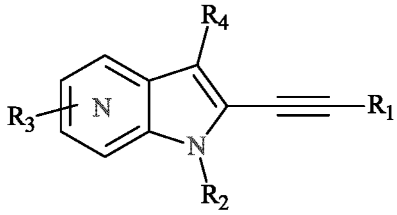

(II-1)

$R_1$, $R_3$ and $R_4$ being as previously defined in formula II;
$R_2$ being optionally labeled and selected from

- H;
- $(CH_2CH_2O)_n$-$CH_2CH_2F$, n being an integer equal to 0, 1 or 2;
  - $SO_2Ph$;
  - $COO^tBu$;
  - $CH_2OCH_3$;
  - $CH_2O(CH_2)_2OCH_3$;
  - $COCH_3$;
  - 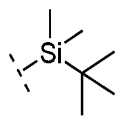 .

5. Compound according to any of claims 1 to 4, said compound being of general formula II-1:

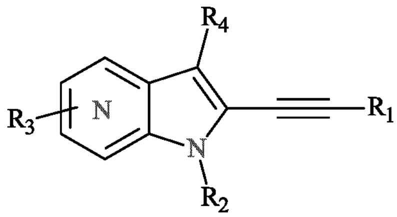

(II-1)

$R_1$, $R_2$ and $R_4$ being as previously defined in formula II;
$R_3$ is optionally labeled and selected from

- 6-Cl;
- 6-F;
- 5-F;
- 4-F;
- 6- (2-thienyl);
- 6- (3-thienyl);

- 6- (6-fluoro-3-pyridyl);
- 5-OMe;
- 6-morpholino;
- 5-morpholino;
- 6-Br;
- 4-Br;
- 4-Bpin;
- 5-Bpin.

**6.** Compound according to any of claims 1 to 5, said compound being of general formula II-1:

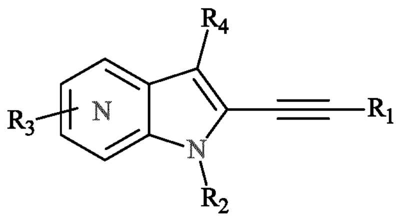

(II-1)

$R_1$, $R_2$ et $R_3$ being as previously defined in formula II;
$R_4$ is selected from

- H;
$(CH_2)_n$-N-alkyl, n being an integer equal to 0, 1 or 2.

**7.** Compound according to any of claims 1 to 6, said compound being of general formula II-1a:

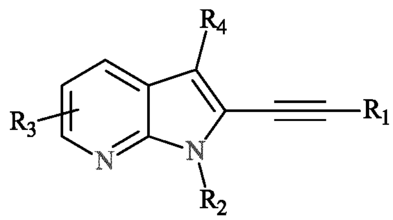

(II-1a)

$R_2$, $R_3$ and $R_4$ being as previously defined in formula II;
$R_1$ being optionally labeled and is selected from

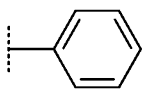 ; 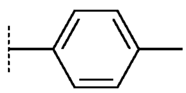 ; 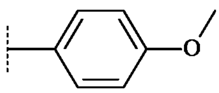 ; 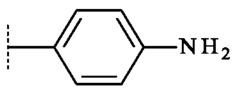

or

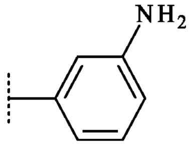 ; 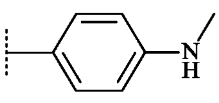 ; 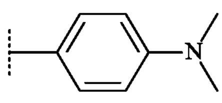 or 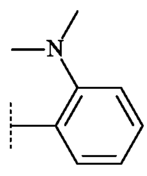

or

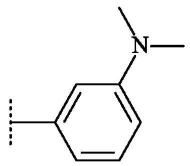 ; 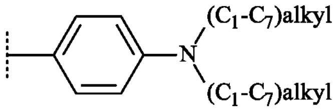 or 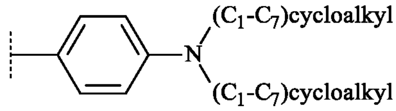 ,

particularly

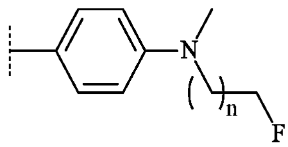 ,

n being from 0 to 6

- 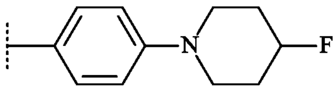 ; 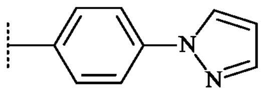 ; 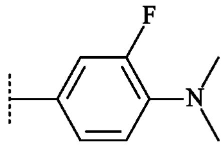 ;

or

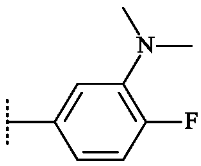 ; 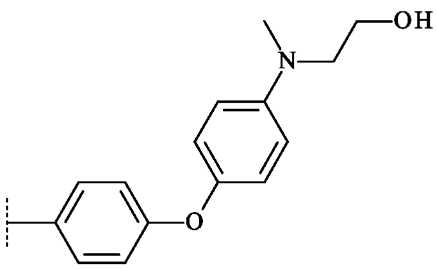 ; 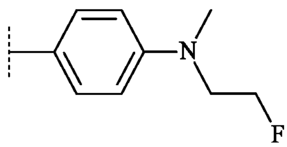 ;

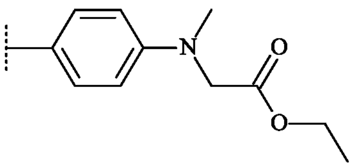 ; 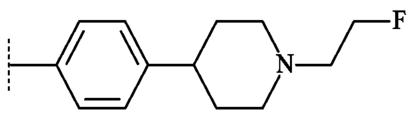 ;

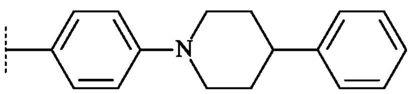 ; 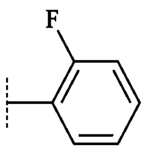 ; 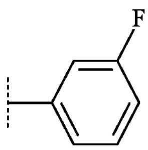 ; 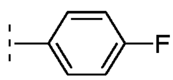 ;

- 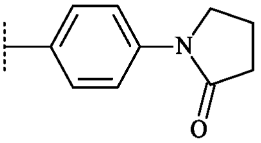 ; 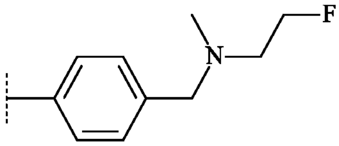 ; 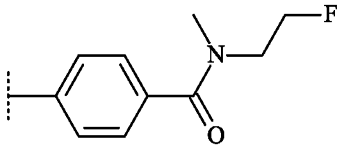 ;

- 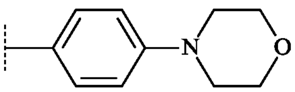 ; 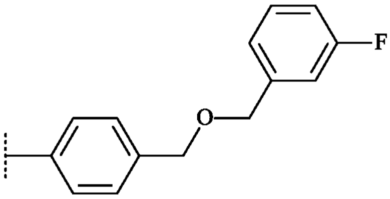 ; 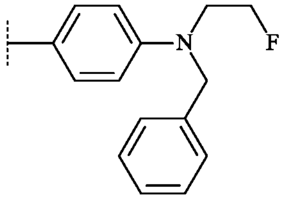 ;

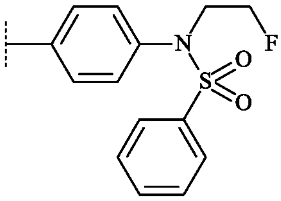 ; 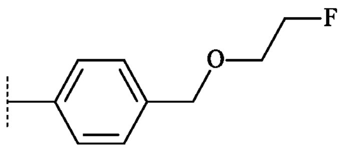 ;

- 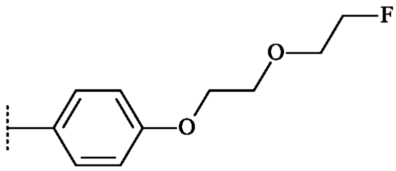 ; 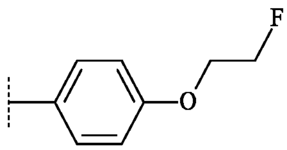 ;

- 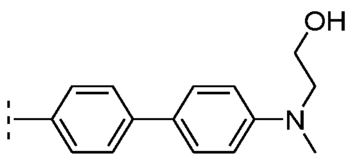 ; 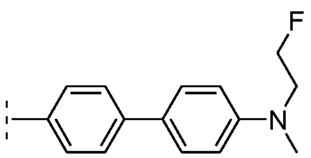 ; 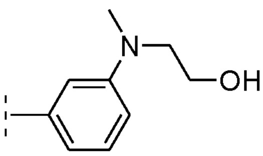 ;

- 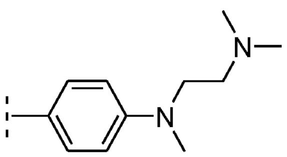 ; 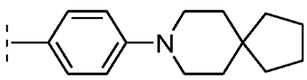 ; 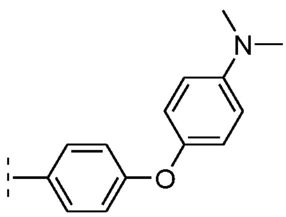 ;

- 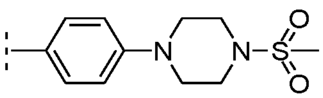 ; 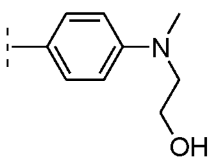 ; 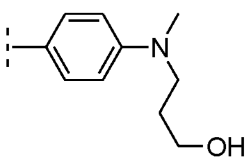 ;

- 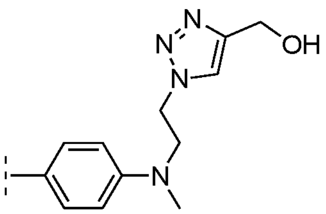 ; 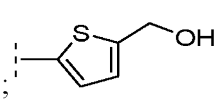 ; 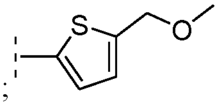 ; 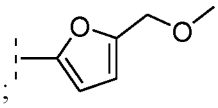 ;

- 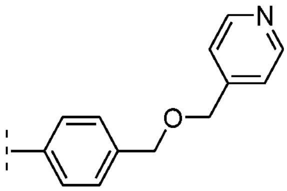 ; 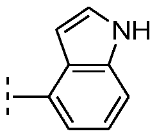 .

**8.** Compound according to any of claims 1 to 7, said compound being of general formula II-2

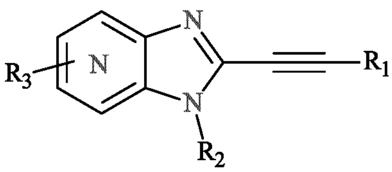

(II-2)

$R_1$, $R_2$ or $R_3$ being as defined above in formula II;
wherein at least one of the substituents $R_1$, $R_2$ or $R_3$ comprises a radioelement selected from $^{18}F$, $^{11}C$, $^{123}I$ and $^{124}I$.

**9.** Compound according to any of claims 1 to 8, said compound of formula II being chosen from compounds of the following formulas:

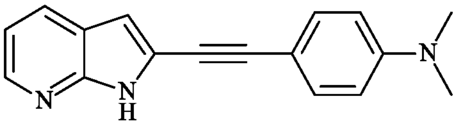

**22a**

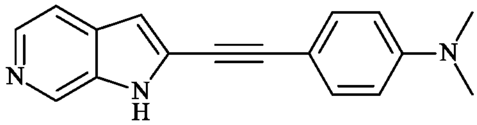

**22b**

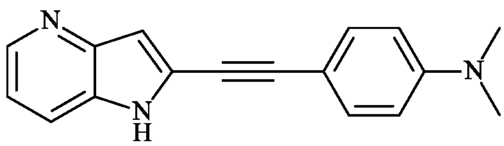

**22c**

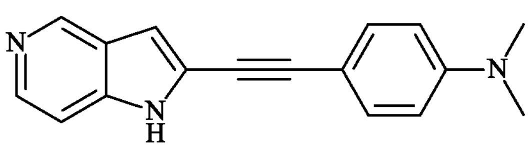

**22d**

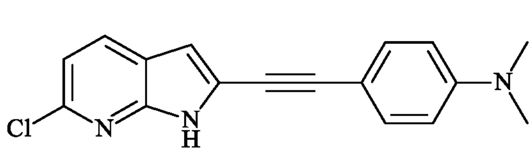

**22e**

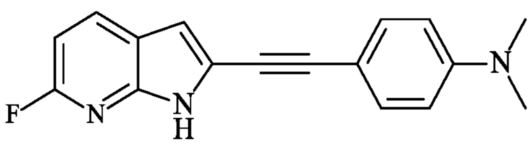

**22f**

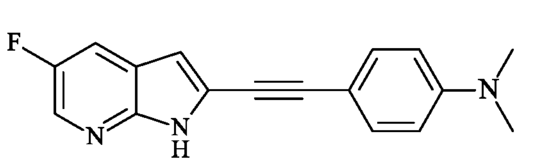

22g

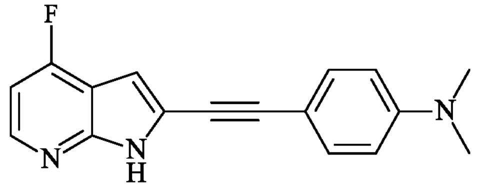

22h

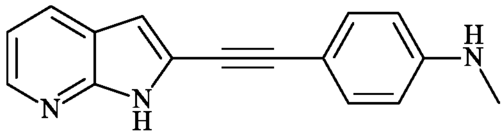

22i

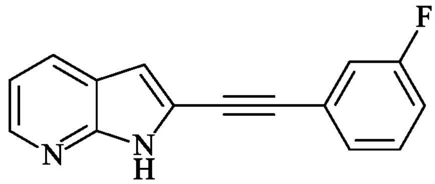

22k

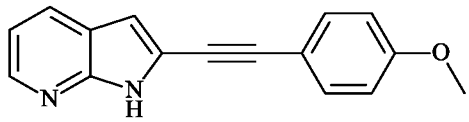

22m

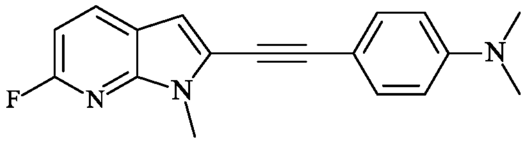

22n

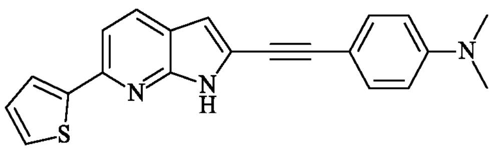

25a

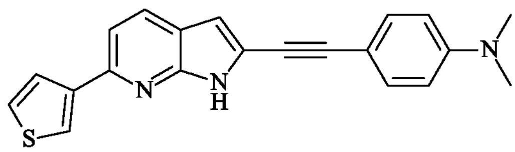

25b

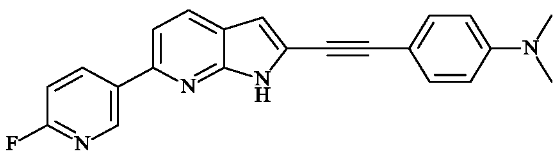

25c

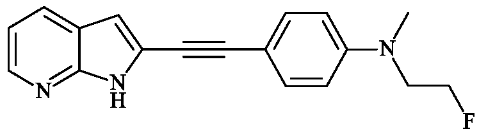

28

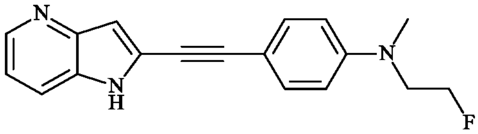

35

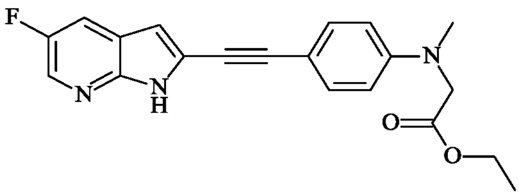

37

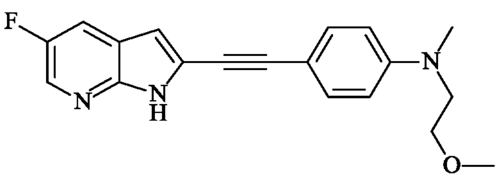

38

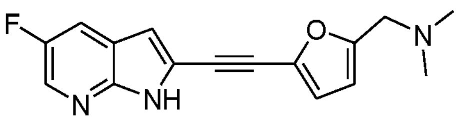

131

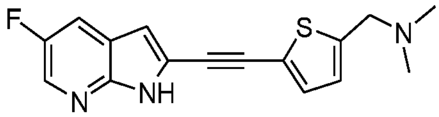
133

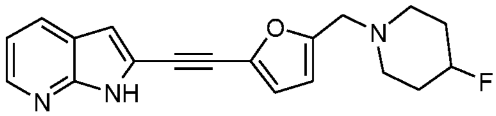
128

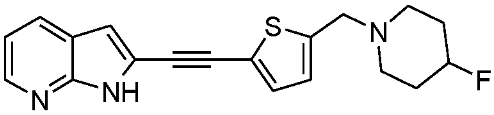
124

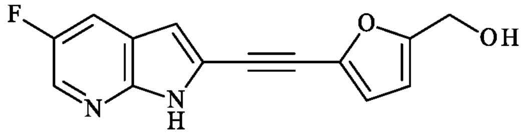
120

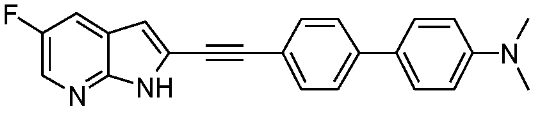
116

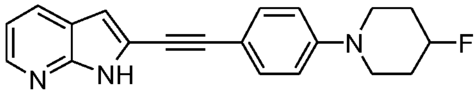
113

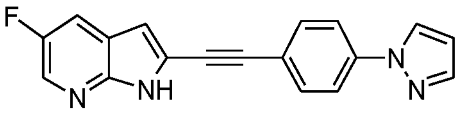
108

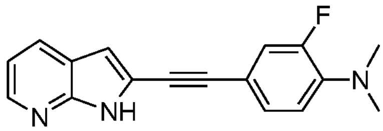
103

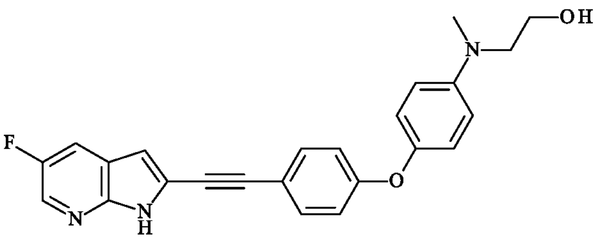
97

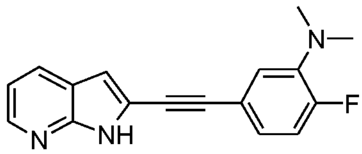
93

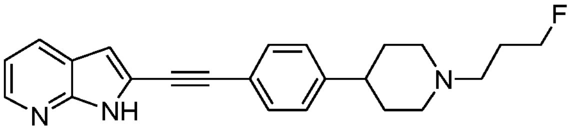
85

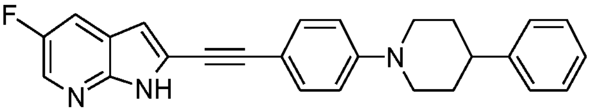
90

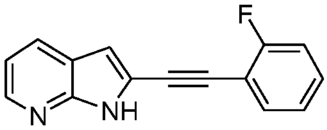
84

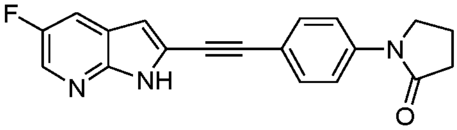
79

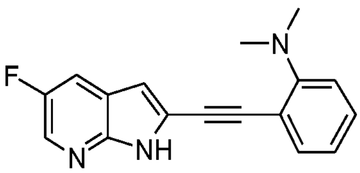
76

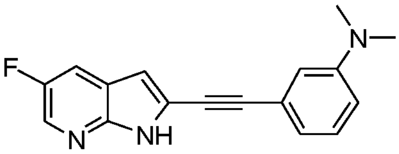
74

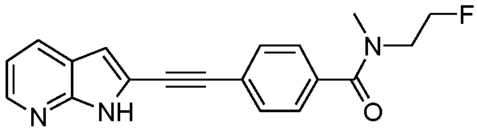
71

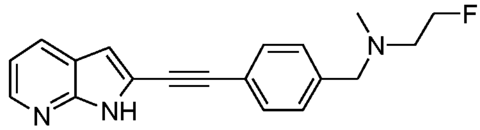
67

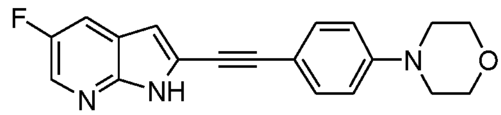
64

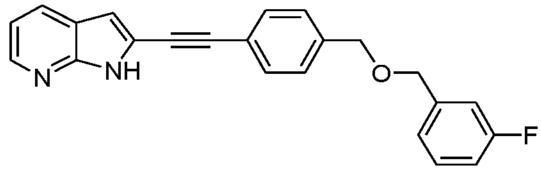
61

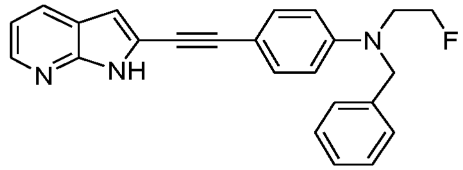
52

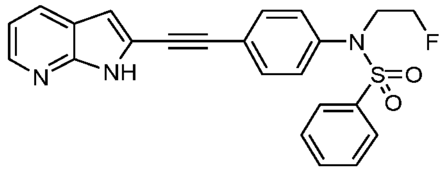
57

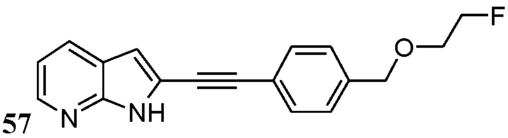
44

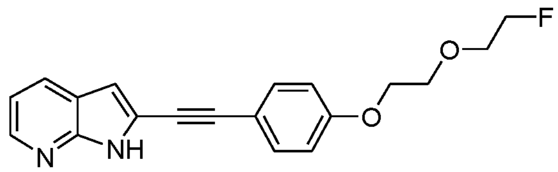
48

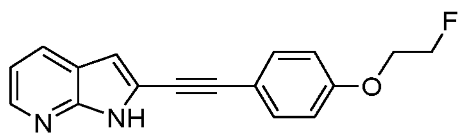
43

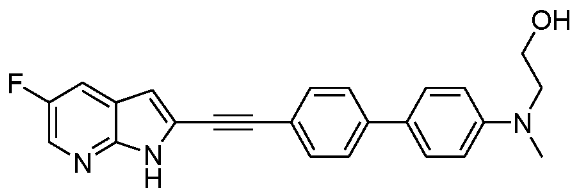

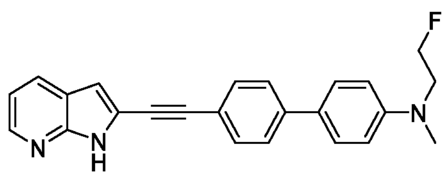

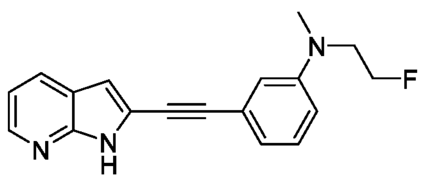

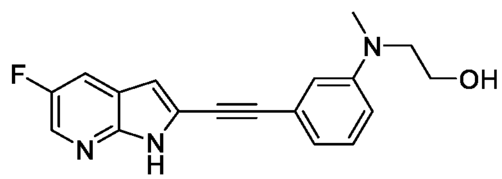

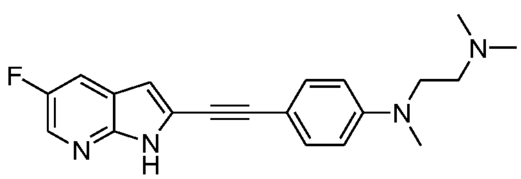

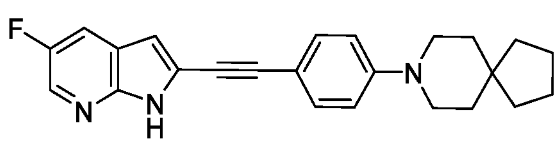

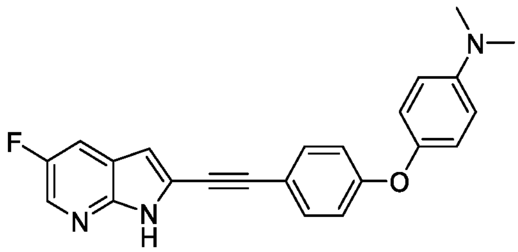

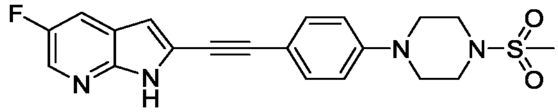

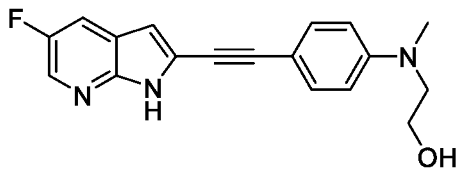

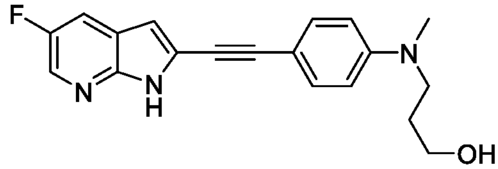

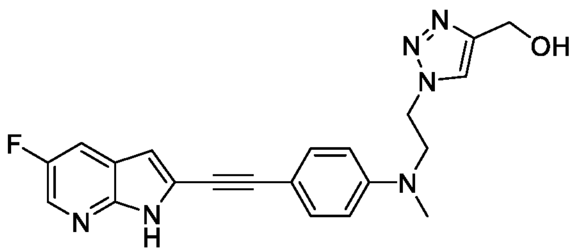

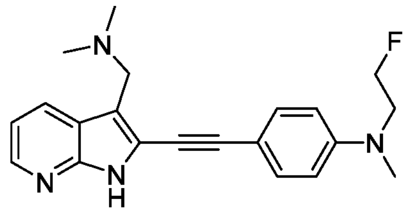

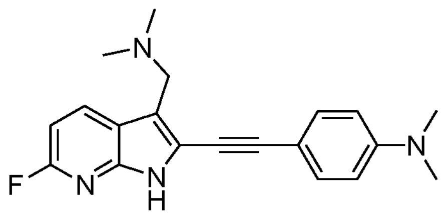

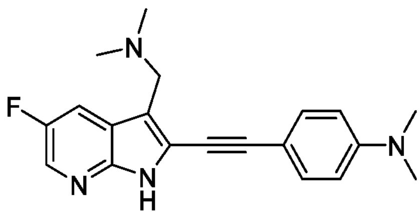

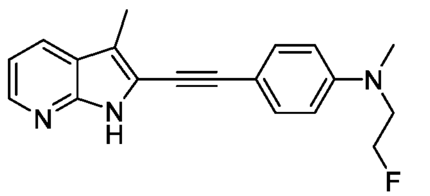

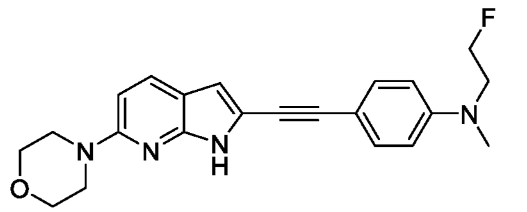

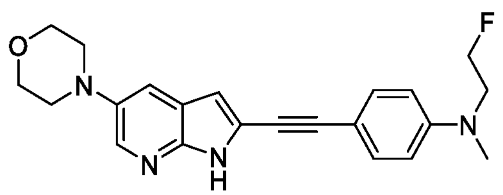

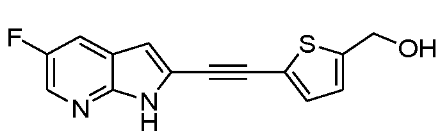

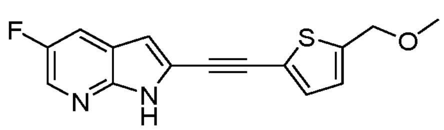

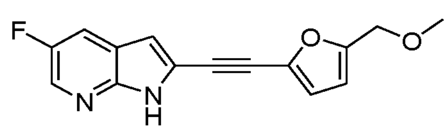

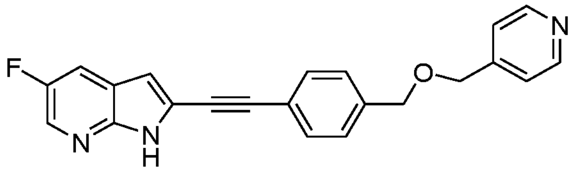

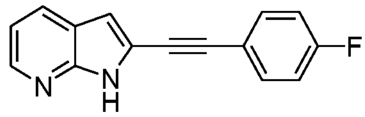

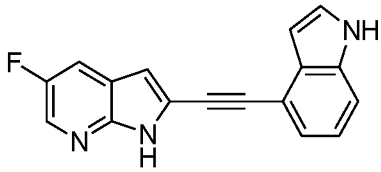

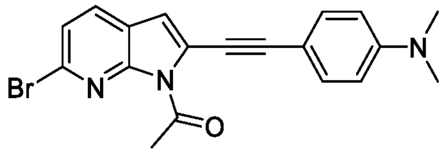

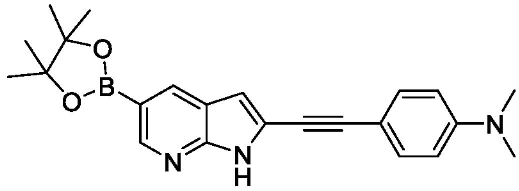

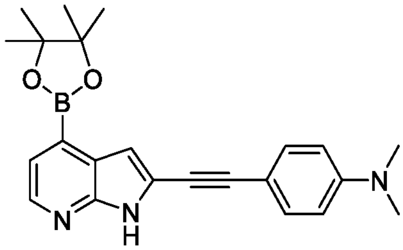

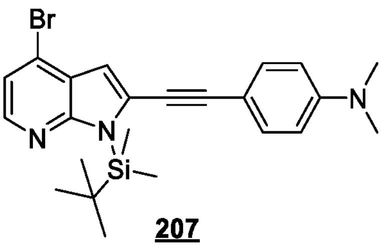

**207**

in a radio labelled state,
particularly, said compound of formula II being

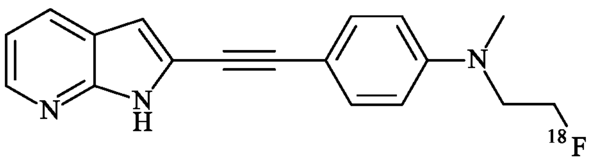

**[18F]-28**

**10.** Use of a compound of formula III:

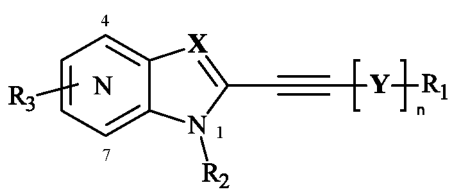

(III)

- X being selected from N or C-$R_4$
- Y being selected from an alkane, alkene or alkyne comprising from 1 to 7 carbon atoms, or aryl or heteroaryle, n being an integer equal to 0 or 1;
- $R_1$ being selected from aryl or heteroaryle selected from phenyl, pyrimidine, pyridine, thiophene, furan, triazole, oxazole, (aza)indole, (aza)indoline, (aza)benzimidazole optionally substituted at one or more positions

with a group selected from:

Halogen, $NO_2$, CN, dimethyltriazene,
$NR^aR^b$, $SO_2NR^aR^b$, $CONR^aR^b$, $NR^cSO_2NR^aR^b$, $NR^cCONR^aR^b$, $NR^aCOR^b$;
$R^a$, $R^b$ and $R^c$ are independently of each other H, ($C_1$-$C_7$) alkyl, aryl, heteroaryl, ($C_3$-$C_7$)carbocyclyl, ($C_1$-$C_7$) alkyl-aryl ($C_1$-$C_7$)alkyl- heteroaryl, or $R^a$ and $R^b$ together form a ($C_3$-$C_7$) heterocyclyl;
$R^d$, $CH_2$ $R^d$;
$R^d$ is H, ($C_1$-$C_7$)alkyl, aryl, heteroaryl, ($C_3$-$C_7$)heterocyclyl, ($C_3$-$C_7$)carbocyclyl, ($C_1$-$C_7$) alkyle-aryl, ($C_1$-$C_7$) alkyl-heteroaryl, ($C_1$-$C_7$) alkyl-($C_3$-$C_7$) heterocyclyl, $[(C_1\text{-}C_7)\text{Alkyle}]_n$-Z or a $[(C_1\text{-}C_7)\text{Alkyle-Z}]_n$ Z being a heteroatom selected from N, O or S, in particular selected from $NR^aR^c$ or $R^e$ and n being an integer comprised from 1 to 7;
$OR^e$, $SR^e$, $SO_2R^e$, $COR^e$, $NR^aSO_2R^e$, $NHCOOR^e$;
$R^e$ is H, ($C_1$-$C_7$) alkyl, aryl, heteroaryl, ($C_3$-$C_7$)heterocyclyl, ($C_3$-$C_7$)carbocyclyl, ($C_1$-$C_7$) alkyl aryl, ($C_1$-$C_7$) alkyl-heteroaryl, or ($C_1$-$C_7$)alkyl- ($C_3$-$C_7$) heterocyclyl;

- $R_2$ being selected from:

H, $SO_2Ph$, $COR^e$, $NR^cCONR^aR^b$, $COOR^e$, OH, $R^d$;

- $R_3$ being selected from:

Halogen, $NO_2$, CN, dimethyltriazene,
$NR^aR^b$, $SO_2NR^aR^b$, $CONR^aR^b$, $NR^cSO_2NR^aR^b$, $NR^cCONR^aR^b$, $NR^aCOR^b$;
$R^d$, $CH_2R^d$,
$OR^e$, $SR^e$, $SO_2R^e$, $COR^e$, $NR^aSO_2R^e$, $NHCOOR^e$;

- $R^4$ being selected from:

Halogen, $CH_2NR^aR^b$, $R^a$, $COOR^e$, CHO, $CH_2OR^e$;
wherein none of the substituents R, $R_2$, $R_3$ or $R^4$ comprises a radioelement,
for the preparation of compounds of formula II, II-1, II-1 or II-2 according to any of claims 1 to 9.

**11.** Compound of formula II, II-1, II1a or II-2 according to any of claims 1 to 9, Π wherein at least one of the substituents $R_1$, $R_2$, $R_3$ or $R^4$ comprises a radioelement selected from $^{18}F$, $^{11}C$, $^{123}I$ and $^{124}I$, for its use in a method for diagnosing or in vivo imaging a subject having a neurodegenerative disease, wherein said method for diagnosing or in vivo imaging comprises administering said compound.

**12.** Compound of formula II, II-1, II1a or II-2 for tis use according to claim 11, in a method for diagnosing or in vivo imaging a subject having a neurodegenerative disease, wherein the in vivo imaging method is positron emission tomography or single photon emission tomography.

**13.** Compound of formula II, II-1, II1a or II-2 for tis use according to any of claims 11 or 12, in a method for diagnosing or in vivo imaging a subject having a neurodegenerative disease, wherein said neurodegenerative disease is amyloidopathy, alpha-synucleinopathy or tauopathy.

**FIG. 1A**

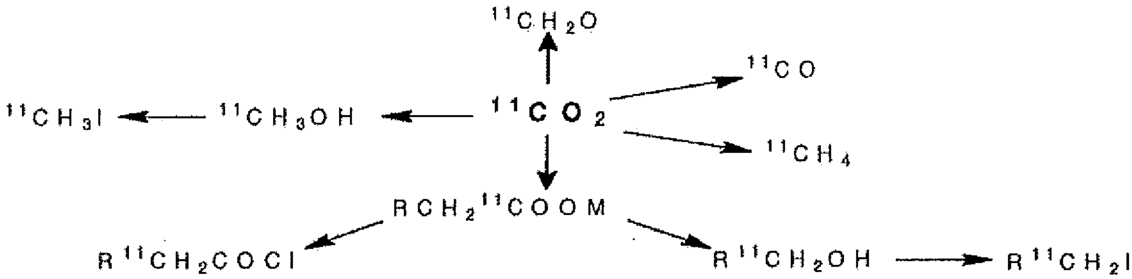

**FIG. 1B**

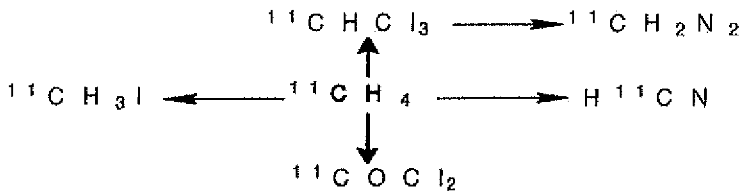

**FIG. 2**

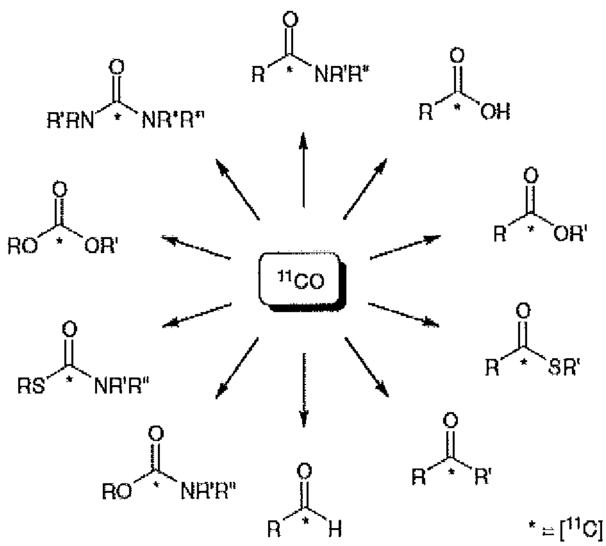

FIG. 3

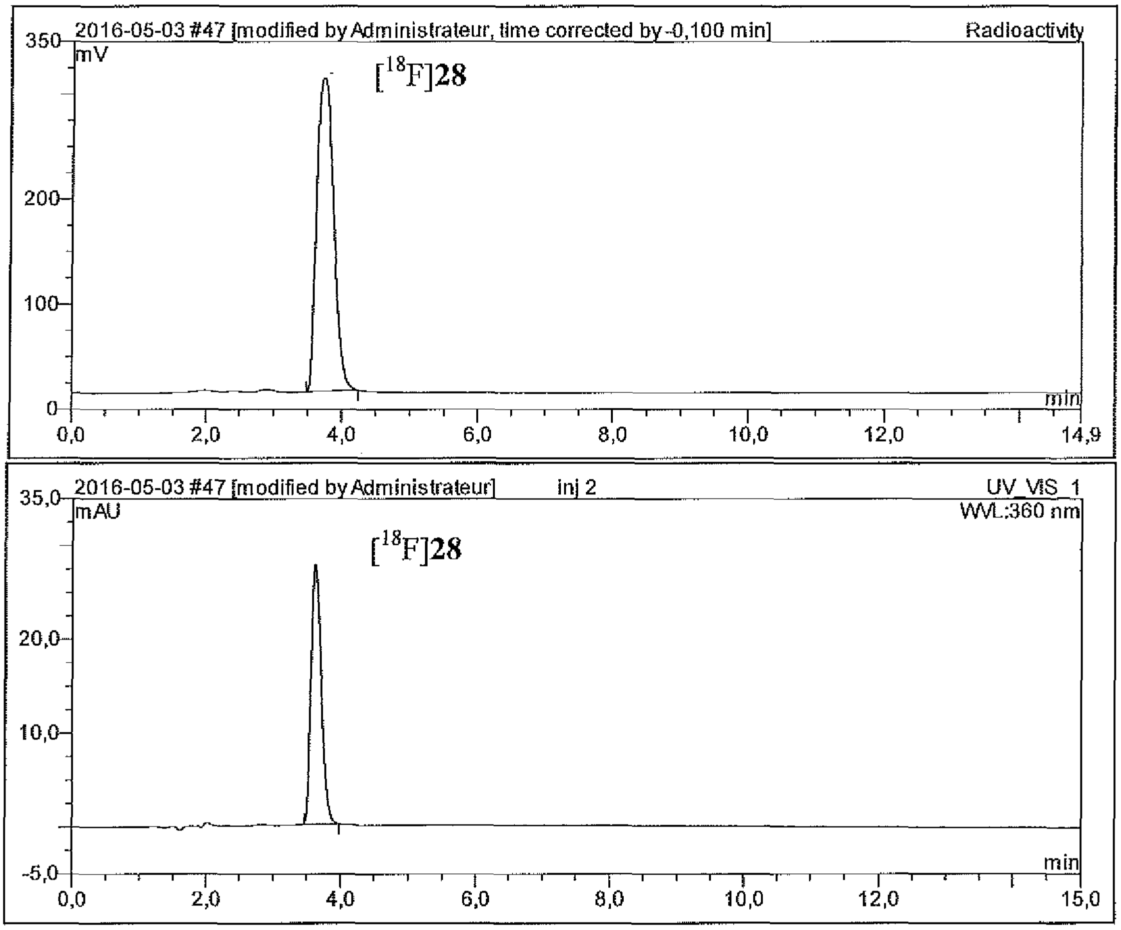
2016-05-03 #47 [modified by Administrateur, time corrected by -0,100 min]
Radioactivity
mV
[18F]28
350
200
100
0
min
0,0
2,0
4,0
6,0
8,0
10,0
12,0
14,9
2016-05-03 #47 [modified by Administrateur]
inj 2
UV_VIS_1
WVL:360 nm
mAU
[18F]28
35,0
20,0
10,0
-5,0
min
15,0

FIG. 4

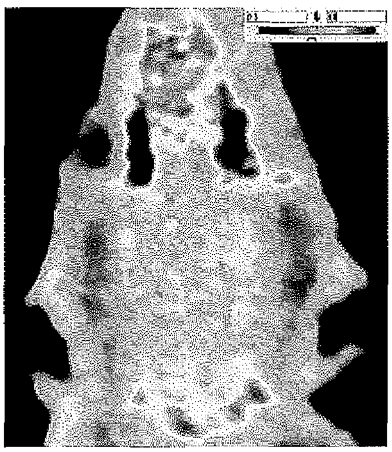

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2008003736A1 A
- US 2004031188 W
- US 2005097129 W
- US 20110144105 A
- US 20080009514 A1
- US 20070129364 A1
- US 201063041 A1


### Littérature non-brevet citée dans la description


- **E. DESARBRE ; S. COUDRET ; C. MEHEUST ; J.-Y. MEROUR.** *Tetrahedron,* 1997, vol. 53 (10), 3637-3648
- **B. JOSEPH ; H. DA COSTA ; J.-Y. MEROUR ; S. LEONCE.** *Tetrahedron,* 2000, vol. 56, 3189-3196
- **S. MINAKATA ; M. KOMATSU ; Y. OHSHIRO.** *Synthesis,* 1992, vol. 7, 661-663
- **S.-F. LIU ; Q. WU ; H. L. SCHMIDER ; H. AZIZ ; N.-X. HU ; Z. POPOVIC ; S. WANG.** *J. Am. Chem. Soc.,* 2000, vol. 122, 3671-3678
- **M. LEFOIX ; J.-P. DAILLANT ; S. ROUTIER ; J.-Y. MÉROUR ; I. GILLAIZEAU ; G. COUDERT.** *Synthesis,* 2005, vol. 20, 3581-3588
- **Y.-S. TUNG ; M. S. COUMAR ; Y.-S. WU ; H.-Y. SHIAO ; J.-Y. CHANG ; J.-P. LIOU ; P. SHUKLA ; C.-W. CHANG ; C.-Y. CHANG ; C.-C. KUO.** *J Med. Chem.,* 2011, vol. 54, 3076-3080
- **A. R. STOIT ; A. P. DEN HARTOG ; H. MONS ; S. VAN SCHAIK ; N. BARKHUIJSEN ; C. STROOMER ; H. K. A. C. COOLEN ; J. H. REINDERS ; T. J. P. ADOLFS ; M. VAN DER NEUT.** *Bioorg. Med. Chem. Lett.,* 2008, vol. 18, 188-193
- **N. LIU ; Y WANG ; G. HUANG ; C. JI ; W. FAN ; H. LI ; Y. CHENG ; H. TIAN.** *Bioorg. Chem.,* 2016, vol. 65, 146-158
- **GHEE M. ; MELKI R. ; MICHOT N. ; MALLET J.** PA700, the regulatory complex of the 26S proteasome, interferes with α-synuclein assembly. *FEBS J.,* 2005, vol. 272, 4023-4033
- **WALSH DM ; THULIN E ; MINOGUE AM ; GUSTAVSSON N ; PANG E ; TEPLOW DB ; LINSE S.** A facile method for expression and purification of the Alzheimer's disease-associated amyloid beta-peptide. *FEBS J.,* 2009, vol. 276, 1266-1281
- **UDENFRIEND S ; STEIN S ; BÖHLEN P ; DAIRMAN W ; LEIMGRUBER W ; WEIGELE M.** Fluorescamine: a reagent for assay of amino acids, peptides, proteins, and primary amines in the picomolar range. *Science,* 1972, vol. 178, 871-872
- **BARGHORN S ; BIERNAT J ; MANDELKOW E.** *Methods Mol Biol.,* 2005, vol. 299, 35-51
- **LEVINE, H 3RD.** Thioflavine T interaction with synthetic Alzheimer's disease beta-amyloid peptides: détection of amyloid aggregation in solution. *Protein Sci.,* 1993, vol. 2, 404-410
- **CHENG Y ; PRUSOFF WH.** Relationship between the inhibition constant (K1) and the concentration of inhibitor which causes 50 per cent inhibition (150) of an enzymatic reaction. *Biochem Pharmacol,* 1973, vol. 22, 3099-3108
- Fluorescence; Solution Studies. **BELL, J.E.** Spectrometry in Biochemistry. CRC Press, Inc, 1981, vol. I, 155-194
- **MAIA et al.** *Synapse,* 2012, vol. 66, 573-83
- **SÉRRIÈRE et al.** *Nucl. Med. Biol.,* 2014, vol. 41, 103-13
- **SÉRRIÈRE et al.** *Neurobiol. Aging,* 2015, vol. 36, 1639-52
- **HIROSE et al.** *Chemical Communications,* 2009, vol. 39, 5832-5834
- **OUACH et al.** *European Journal of Medicinal Chemistry,* 2016, vol. 107, 153-164
- *CHEMICAL ABSTRACTS,* 1562413-13-1
- **HERBERT.** *Organic Letters,* 2013, vol. 15 (13), 3334-3337
- *CHEMICAL ABSTRACTS,* 1118567-11-5
- *CHEMICAL ABSTRACTS,* 122974-04-3
- **SAKAI et al.** *Macromolecules,* 2012, vol. 45 (20), 8221-8227, 7
- *CHEMICAL ABSTRACTS,* 383147-75-9
- *CHEMICAL ABSTRACTS,* 30483-75-1
- *CHEMICAL ABSTRACTS,* 41876-72-6
- **FANG.** *Journal of Chemical Research,* 2015, vol. 39 (8), 487-491
- *CHEMICAL ABSTRACTS,* 52324-05-7
- *CHEMICAL ABSTRACTS,* 219605-52-4
- *CHEMICAL ABSTRACTS,* 7661-32-7
- *CHEMICAL ABSTRACTS,* 303975-64-6
- *CHEMICAL ABSTRACTS,* 1352214-46-0
- *CHEMICAL ABSTRACTS,* 28896-49-3

- *CHEMICAL ABSTRACTS,* 1021240-69-6
- *CHEMICAL ABSTRACTS,* 13788-92-6
- *CHEMICAL ABSTRACTS,* 1423130-86-2
- *CHEMICAL ABSTRACTS,* 92194-03-1
- *CHEMICAL ABSTRACTS,* 1190376-40-9
- *Organometallics,* 2005, vol. 24 (4), 693
- *CHEMICAL ABSTRACTS,* 153026-71-2
- *CHEMICAL ABSTRACTS,* 153026-89-2
- *CHEMICAL ABSTRACTS,* 252949-11-4
- *CHEMICAL ABSTRACTS,* 74420-15-8
- *CHEMICAL ABSTRACTS,* 880769-95-9
- *CHEMICAL ABSTRACTS,* 889939-25-7